(19) Europäisches Patentamt
European Patent Office
Office européen des brevets

(11) EP 1 585 541 B1

(12) **EUROPEAN PATENT SPECIFICATION**

(45) Date of publication and mention of the grant of the patent:
**14.11.2007 Bulletin 2007/46**

(21) Application number: **03767488.4**

(22) Date of filing: **22.12.2003**

(51) Int Cl.:
***A61K 38/28*** *(2006.01)* ***C07D 249/18*** *(2006.01)*
***C07D 277/34*** *(2006.01)* ***A61K 31/4192*** *(2006.01)*
***A61K 31/416*** *(2006.01)* ***C07D 417/02*** *(2006.01)*
***C07D 413/02*** *(2006.01)* ***C07K 5/00*** *(2006.01)*
***C07K 7/00*** *(2006.01)* ***C07K 14/62*** *(2006.01)*

(86) International application number:
**PCT/DK2003/000931**

(87) International publication number:
**WO 2004/056347 (08.07.2004 Gazette 2004/28)**

(54) **PHARMACEUTICAL COMPOSITIONS COMPRISING INSULIN AND LEGENDS OF INSULIN HEXAMER**

PHARMAZEUTISCHE ZUSAMMENSETZUNGEN MIT INSULIN UND INSULIN-HEXAMER-LIGANDEN

COMPOSITIONS PHARMACEUTIQUES CONTENANT DE L'INSULINE ET DES LIGANDS DE L'HEXAMÈRE D'INSULINE

(84) Designated Contracting States:
**AT BE BG CH CY CZ DE DK EE ES FI FR GB GR HU IE IT LI LU MC NL PT RO SE SI SK TR**

(30) Priority: **20.12.2002 DK 200201991**
**10.01.2003 US 439382 P**

(43) Date of publication of application:
**19.10.2005 Bulletin 2005/42**

(73) Proprietor: **NOVO NORDISK A/S**
**2880 Bagsvaerd (DK)**

(72) Inventors:
- **KAARSHOLM, Niels, Christian**
  **DK-2720 Vanlose (DK)**
- **MADSEN, Peter**
  **DK-2880 Bagsvard (DK)**
- **SCHLEIN, Morten**
  **DK-2300 Copenhagen S (DK)**
- **OLSEN, Helle, Birk**
  **DK-3450 Allerod (DK)**
- **HAVELUND, Svend**
  **DK-2880 Bagsvard (DK)**
- **STEENSGAARD, Dorte, Bjerre**
  **DK- 2100-Kobenhavn (DK)**
- **LUDVIGSEN, Svend**
  **DK-3540 Lynge (DK)**
- **JAKOBSEN, Palle**
  **DK-3500 Varlose (DK)**
- **PETERSEN, Anders, Klarskov**
  **DK-2850 Narum (DK)**
- **SCHLUCKEBIER, Gerd**
  **DK-2200 Kobenhavn N (DK)**

(74) Representative: **Madsen, Inger Margrethe Schelde**
**Novo Nordisk A/S,**
**Corporate Patents,**
**Novo Alle**
**2880 Bagsvaerd (DK)**

(56) References cited:
**EP-A- 1 396 272** **WO-A-03/027081**
**US-A- 4 476 118** **US-A- 5 830 999**
**US-A1- 2003 229 120**

- **FERRARI D ET AL: "Raman signatures of ligand binding and allosteric conformation change in hexameric insulin." BIOPOLYMERS. UNITED STATES 2001, vol. 62, no. 5, 2001, pages 249-260, XP002280143 ISSN: 0006-3525**
- **HUANG S T ET AL: "Carboxylate ions are strong allosteric ligands for the HisB10 sites of the R-state insulin hexamer." BIOCHEMISTRY. UNITED STATES 12 AUG 1997, vol. 36, no. 32, 12 August 1997 (1997-08-12), pages 9878-9888, XP002280144 ISSN: 0006-2960**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 585 541 B1

- BRADER M L ET AL: "Characterization of the R-state insulin hexamer and its derivatives. The hexamer is stabilized by heterotropic ligand binding interactions." BIOCHEMISTRY. UNITED STATES 9 JUL 1991, vol. 30, no. 27, 9 July 1991 (1991-07-09), pages 6636-6645, XP002280145 ISSN: 0006-2960

## Description

### FIELD OF THE INVENTION

This invention relates to insulin compositions stabilised by adding ligands for the $His^{B10}$ $Zn^{2+}$ sites of the R-state insulin hexamer, as well as methods for preparation and use of such preparations.

### BACKGROUND OF THE INVENTION

Diabetes is a general term for disorders in man having excessive urine excretion as in diabetes mellitus and diabetes insipidus. Diabetes mellitus is a metabolic disorder in which the ability to utilize glucose is partly or completely lost.
Since the discovery of insulin in the 1920's, continuous strides have been made to improve the treatment of diabetes mellitus. To help avoid extreme glycaemia levels, diabetic patients often practice multiple injection therapy, whereby insulin is administered with each meal. Many diabetic patients are treated with multiple daily insulin injections in a regimen comprising one or two daily injections of a protracted insulin composition to cover the basal requirement, supplemented by bolus injections of rapid acting insulin to cover the meal-related requirements.
Insulin compositions having a protracted profile of action are well known in the art. Thus, one main type of such insulin compositions comprises injectable aqueous suspensions of insulin crystals or amorphous insulin. Typically, the insulin in these compositions is provided in the form of protamine insulin, zinc insulin or protamine zinc insulin
Soluble, rapid acting insulin compositions usually comprise insulin, insulin analogue or insulin derivative together with zinc ion, phenolic preservative, isotonicity agent, and a buffer substance. In addition, the preparation may optionally contain some salts and/or surfactants. Such preparations contain insulin in the form of an R-state hexamer.

*Insulin Allostery.*

The insulin hexamer is an allosteric protein that exhibits both positive and negative cooperativity and half-of-the-sites reactivity in ligand binding. This allosteric behaviour consists of two interrelated allosteric transitions designated $L^A{}_0$ and $L^B{}_0$, three inter-converting allosteric conformation states (eq. 1),

$$T_6 \overset{L^A{}_0}{\leftrightarrow} T_3R_3 \overset{L^B{}_0}{\leftrightarrow} R_6 \qquad \text{equation (1)}$$

designated $T_6$, $T_3R_3$, and $R_6$ and two classes of allosteric ligand binding sites designated as the phenolic pockets and the $His^{B10}$ anion sites. These allosteric sites are associated only with insulin subunits in the R conformation.

*Insulin Hexamer Structures and Ligand Binding.*

The T- to R-transition of the insulin hexamer involves transformation of the first nine residues of the B chain from an extended conformation in the T-state to an alpha-helical conformation in the R-state. This coil-to-helix transition causes the N-terminal residue, $Phe^{B1}$, to undergo an ~ 30 Å change in position. This conformational change creates hydrophobic pockets (the phenolic pockets) at the subunit interfaces (three in $T_3R_3$, and six in $R_6$), and the new B-chain helices form 3-helix bundles (one in $T_3R_3$ and two in $R_6$) with the bundle axis aligned along the hexamer three-fold symmetry axis. The $His^{B10}$ $Zn^{2+}$ in each $R_3$ unit is forced to change coordination geometry from octahedral to either tetrahedral (monodentate ligands) or pentahedral (bidentate ligands). Formation of the helix bundle creates a narrow hydrophobic tunnel in each $R_3$ unit that extends from the surface ~12 Å down to the $His^{B10}$ metal ion. This tunnel and the $His^{B10}$ $Zn^{2+}$ ion form the anion binding site. Ligands for the $His^{B10}$ $Zn^{2+}$ sites of the R-state insulin hexamer have been disclosed in US 5830999.

*Hexamer Ligand Binding and Stability of Insulin compositions.*

The *in vivo* role of the T to R transition is unknown. However, the addition of allosteric ligands (e.g. phenol and chloride ion) to insulin compositions is widely used. Hexamerization is driven by coordination of $Zn^{2+}$ at the $His^{B10}$ sites to give $T_6$. Following subcutaneous injection, some dilution of the depot will take place over time and the ligands of soluble hexamers most likely diffuse away from the protein relatively rapidly. This is probably due to one or more phenomena including the binding of $Zn^{2+}$ by surrounding tissue and albumin, the relatively larger space available for

diffusion of the hydrophobic phenolic preservatives, and the generally larger diffusion coefficients characteristic of the smaller sized molecules.

Insulin compositions are usually stored for extended periods of time e.g. in vials or cartridges. Furthermore, insulin pumps are becoming more widely used, which places an additional demand on the chemical and physical stability of the insulin composition due to the elevated temperatures and physical stress these preparations are exposed to. There is thus a need for insulin compositions that are more physically and chemically stable. It has been found that stabilising $Zn^{2+}$-site ligands may be added to insulin compositions to improve these properties.

## SUMMARY OF THE INVENTION

The present invention provides pharmaceutical compositions comprising insulin and novel ligands for the $His^{B10}$ $Zn^{2+}$ sites of the R-state insulin hexamer. The ligands belong to different subclasses of compounds, e.g. benzotriazoles, 3-hydroxy 2-napthoic acids, salicylic acids, tetrazoles, thiazolidinediones, 5-mercaptotetrazoles, or 4-cyano-1,2,3-triazoles. The insulin may be rapid-acting. The insulin may be selected from human insulin, or an analogue or derivative thereof. The formulation may also comprise a phenolic compound, an isotonicity agent, and buffer.

## DESCRIPTION OF THE DRAWINGS

Figures 1-8 show ThT assays of various combinations of insulin formulations and ligands of the invention.
Figure 9 shows disappearance rate of various combinations of insulin formulations and ligands of the invention from the subcutaneous depot following injection in pigs.
Figures 10-14 show reverse phase chromatography of various combinations of insulin formulations and ligands of the invention.

## DEFINITIONS

The following is a detailed definition of the terms used to describe the invention:

"Halogen" designates an atom selected from the group consisting of F, Cl, Br and I.

The term "alkyl" as used herein represents a saturated, branched or straight hydrocarbon group having the indicated number of carbon atoms. Representative examples include, but are not limited to, methyl, ethyl, n-propyl, isopropyl, butyl, isobutyl, *sec*-butyl, *tert*-butyl, n-pentyl, isopentyl, neopentyl, *tert*-pentyl, n-hexyl, isohexyl and the like.
The term "alkylene" as used herein represents a saturated, branched or straight bivalent hydrocarbon group having the indicated number of carbon atoms. Representative examples include, but are not limited to, methylene, 1,2-ethylene, 1,3-propylene, 1,2-propylene, 1,4-butylene, 1,5-pentylene, 1,6-hexylene, and the like.
The term "alkenyl" as used herein represents a branched or straight hydrocarbon group having the indicated number of carbon atoms and at least one double bond. Examples of such groups include, but are not limited to, vinyl, 1-propenyl, 2-propenyl, iso-propenyl, 1,3-butadienyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 2-hexenyl, 3-hexenyl, 2,4-hexadienyl, 5-hexenyl and the like.
The term "alkynyl" as used herein represents a branched or straight hydrocarbon group having the indicated number of carbon atoms and at least one triple bond. Examples of such groups include, but are not limited to, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 2,4-hexadiynyl and the like.
The term "alkoxy" as used herein refers to the radical -O- alkyl, wherein alkyl is as defined above. Representative examples are methoxy, ethoxy, n-propoxy, isopropoxy, butoxy, *sec*-butoxy, *tert*-butoxy, pentoxy, isopentoxy, hexoxy, isohexoxy and the like.
The term "cycloalkyl" as used herein represents a saturated, carbocyclic group having the indicated number of carbon atoms. Representative examples are cyclopropyl, cyclobutyl, cyclopentyl, cyclohexyl, cycloheptyl, cyclooctyl and the like.
The term "cycloalkenyl" as used herein represents a non-aromatic, carbocyclic group having the indicated number of carbon atoms containing one or two double bonds. Representative examples are 1-cyclopentenyl, 2-cyclopentenyl, 3-cyclopentenyl, 1-cyclohexenyl, 2-cyclohexenyl, 3-cyclohexenyl, 2-cycloheptenyl, 3-cycloheptenyl, 2-cyclooctenyl, 1,4-cyclooctadienyl and the like.
The term "heterocyclyl" as used herein represents a non-aromatic 3 to 10 membered ring containing one or more heteroatoms selected from nitrogen, oxygen and sulphur and optionally containing one or two double bonds. Representative examples are pyrrolidinyl, piperidyl, piperazinyl, morpholinyl, thiomorpholinyl, aziridinyl, tetrahydrofuranyl and

the like.
The term "aryl" as used herein is intended to include carbocyclic, aromatic ring systems such as 6 membered monocyclic and 9 to 14 membered bi- and tricyclic, carbocyclic, aromatic ring systems. Representative examples are phenyl, biphenylyl, naphthyl, anthracenyl, phenanthrenyl, fluorenyl, indenyl, azulenyl and the like. Aryl is also intended to include the partially hydrogenated derivatives of the ring systems enumerated above. Non-limiting examples of such partially hydrogenated derivatives are 1,2,3,4-tetrahydronaphthyl, 1,4-dihydronaphthyl and the like.

The term "arylene" as used herein is intended to include divalent, carbocyclic, aromatic ring systems such as 6 membered monocyclic and 9 to 14 membered bi- and tricyclic, divalent, carbocyclic, aromatic ring systems. Representative examples are phenylene, biphenylylene, naphthylene, anthracenylene, phenanthrenylene, fluorenylene, indenylene, azulenylene and the like. Arylene is also intended to include the partially hydrogenated derivatives of the ring systems enumerated above. Non-limiting examples of such partially hydrogenated derivatives are 1,2,3,4-tetrahydronaphthylene, 1,4-dihydronaphthylene and the like.

The term "aryloxy" as used herein denotes a group -O-aryl, wherein aryl is as defined above.

The term "aroyl" as used herein denotes a group -C(O)-aryl, wherein aryl is as defined above.

The term "heteroaryl" as used herein is intended to include aromatic, heterocyclic ring systems containing one or more heteroatoms selected from nitrogen, oxygen and sulphur such as 5 to 7 membered monocyclic and 8 to 14 membered bi- and tricyclic aromatic, heterocyclic ring systems containing one or more heteroatoms selected from nitrogen, oxygen and sulphur. Representative examples are furyl, thienyl, pyrrolyl, pyrazolyl, 3-oxopyrazolyl, oxazolyl, thiazolyl, imidazolyl, isoxazolyl, isothiazolyl, 1,2,3-triazolyl, 1,2,4-triazolyl, pyranyl, pyridyl, pyridazinyl, pyrimidinyl, pyrazinyl, 1,2,3-triazinyl, 1,2,4-triazinyl, 1,3,5- triazinyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, tetrazolyl, thiadiazinyl, indolyl, isoindolyl, benzofuryl, benzothienyl, indazolyl, benzimidazolyl, benzthiazolyl, benzisothiazolyl, benzoxazolyl, benzisoxazolyl, purinyl, quinazolinyl, quinolizinyl, quinolinyl, isoquinolinyl, quinoxalinyl, naphthyridinyl, pteridinyl, carbazolyl, azepinyl, diazepinyl, acridinyl, thiazolidinyl, 2-thiooxothiazolidinyl and the like. Heteroaryl is also intended to include the partially hydrogenated derivatives of the ring systems enumerated above. Non-limiting examples of such partially hydrogenated derivatives are 2,3-dihydrobenzofuranyl, pyrrolinyl, pyrazolinyl, indolinyl, oxazolidinyl, oxazolinyl, oxazepinyl and the like.

The term "heteroarylene" as used herein is intended to include divalent, aromatic, heterocyclic ring systems containing one or more heteroatoms selected from nitrogen, oxygen and sulphur such as 5 to 7 membered monocyclic and 8 to 14 membered bi- and tricyclic aromatic, heterocyclic ring systems containing one or more heteroatoms selected from nitrogen, oxygen and sulphur. Representative examples are furylene, thienylene, pyrrolylene, oxazolylene, thiazolylene, imidazolylene, isoxazolylene, isothiazolylene, 1,2,3-triazolylene, 1,2,4-triazolylene, pyranylene, pyridylene, pyridazinylene, pyrimidinylene, pyrazinylene, 1,2,3-triazinylene, 1,2,4-triazinylene, 1,3,5- triazinylene, 1,2,3-oxadiazolylene, 1,2,4-oxadiazolylene, 1,2,5-oxadiazolylene, 1,3,4-oxadiazolylene, 1,2,3-thiadiazolylene, 1,2,4-thiadiazolylene, 1,2,5-thiadiazolylene, 1,3,4-thiadiazolylene, tetrazolylene, thiadiazinylene, indolylene, isoindolylene, benzofurylene, benzothienylene, indazolylene, benzimidazolylene, benzthiazolylene, benzisothiazolylene, benzoxazolylene, benzisoxazolylene, purinylene, quinazolinylene, quinolizinylene, quinolinylene, isoquinolinylene, quinoxalinylene, naphthyridinylene, pteridinylene, carbazolylene, azepinylene, diazepinylene, acridinylene and the like. Heteroaryl is also intended to include the partially hydrogenated derivatives of the ring systems enumerated above. Non-limiting examples of such partially hydrogenated derivatives are 2,3-dihydrobenzofuranylene, pyrrolinylene, pyrazolinylene, indolinylene, oxazolidinylene, oxazolinylene, oxazepinylene and the like.

The term "ArG1" as used herein is intended to include an aryl or arylene radical as applicable, where aryl or arylene are as defined above but limited to phenyl, biphenylyl, naphthyl, anthracenyl, phenanthrenyl, fluorenyl, indenyl, and azulenyl as well as the corrresponding divalent radicals.

The term "ArG2" as used herein is intended to include an aryl or arylene radical as applicable, where aryl or arylene are as defined above but limited to phenyl, biphenylyl, naphthyl, fluorenyl, and indenyl, as well as the corrresponding divalent radicals.

The term "Het1" as used herein is intended to include a heteroaryl or heteroarylene radical as applicable, where heteroaryl or heteroarylene are as defined above but limited to furyl, thienyl, pyrrolyl, pyrazolyl, 3-oxopyrazolyl, oxazolyl, thiazolyl, imidazolyl, isoxazolyl, isothiazolyl, 1,2,3-triazolyl, 1,2,4-triazolyl, pyranyl, pyridyl, pyridazinyl, pyrimidinyl, pyrazinyl, 1,2,3-triazinyl, 1,2,4-triazinyl, 1,3,5- triazinyl, 1,2,3-oxadiazolyl, 1,2,4-oxadiazolyl, - 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, tetrazolyl, thiadiazinyl, indolyl, isoindolyl, benzofuryl, benzothienyl, indazolyl, benzimidazolyl, benzthiazolyl, benzisothiazolyl, benzoxazolyl, benzisoxazolyl, purinyl, quinazolinyl, quinolizinyl, quinolinyl, isoquinolinyl, quinoxalinyl, naphthyridinyl, pteridinyl, carbazolyl, azepinyl, diazepinyl, acridinyl, thiazolidinyl, 2-thiooxothiazolidinyl, as well as the corrresponding divalent radicals.

The term "Het2" as used herein is intended to include a heteroaryl or heteroarylene radical as applicable, where heteroaryl or heteroarylene are as defined above but limited to furyl, thienyl, pyrrolyl, pyrazolyl, 3-oxopyrazolyl, oxazolyl, thiazolyl, imidazolyl, isoxazolyl, isothiazolyl, 1,2,3-triazolyl, 1,2,4-triazolyl, pyranyl, pyridyl, pyridazinyl, pyrimidinyl, pyrazinyl, 1,2,3-triazinyl, 1,2,4-triazinyl, 1,3,5- triazinyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, tetrazolyl, thiadiazinyl, indolyl, isoindolyl, benzofuryl, benzothienyl, benzimidazolyl, benzthiazolyl, benzisothiazolyl, benzoxazolyl, benzisoxazolyl, quinolinyl, isoquinolinyl, quinoxalinyl, carbazolyl, thiazolidinyl, 2-thiooxothiazolidinyl, as well as the corrresponding divalent radicals.
The term "Het3" as used herein is intended to include a heteroaryl or heteroarylene radical as applicable, where heteroaryl or heteroarylene are as defined above but limited to furyl, thienyl, pyrrolyl, pyrazolyl, 3-oxopyrazolyl, oxazolyl, thiazolyl, imidazolyl, isoxazolyl, isothiazolyl, 1,2,3-triazolyl, 1,2,4-triazolyl, pyridyl, tetrazolyl, indolyl, isoindolyl, benzofuryl, benzothienyl, benzimidazolyl, benzthiazolyl, benzisothiazolyl, benzoxazolyl, benzisoxazolyl, quinolyl, isoquinolyl, quinoxalinyl, carbazolyl, thiazolidinyl, 2-thiooxothiazolidinyl, as well as the corrresponding divalent radicals.
"Aryl-$C_1$-$C_6$-alkyl", "heteroaryl-$C_1$-$C_6$-alkyl", "aryl-$C_2$-$C_6$-alkenyl" etc. is intended to mean $C_1$-$C_6$-alkyl or $C_2$-$C_6$-alkenyl as defined above, substituted with an aryl or heteroaryl as defined above, for example:

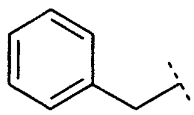 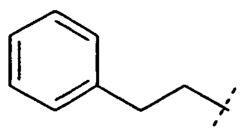 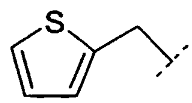 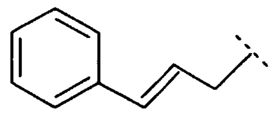

The term "optionally substituted" as used herein means that the groups in question are either unsubstituted or substituted with one or more of the substituents specified. When the groups in question are substituted with more than one substituent the substituents may be the same or different.
Certain of the above defined terms may occur more than once in the structural formulae, and upon such occurrence each term shall be defined independently of the other.
Furthermore, when using the terms "independently are" and "independently selected from" it should be understood that the groups in question may be the same or different.
The term "substituted with one or more substituents" as used herein is intended to include one to four substituents, such as one to three substitents, one to two substituents, or even one substituent.
The term "treatment" as used herein means the management and care of a patient for the purpose of combating a disease, disorder or condition. The term is intended to include the delaying of the progression of the disease, disorder or condition, the alleviation or relief of symptoms and complications, and/or the cure or elimination of the disease, disorder or condition. The patient to be treated is preferably a mammal, in particular a human being. When in the specification or claims mention is made of groups of compounds such as benzotriazoles, 3-hydroxy 2-napthoic acids, salicylic acids, tetrazoles, thiazolidinediones, 5-mercaptotetrazoles, or 4-cyano-1,2,3-triazoles, these groups of compounds are intended to include also derivatives of the compounds from which the groups take their name.
The term "insulin" as used herein refers to human insulin as well as derivatives ans analogues hereof as defined below.
The term "human insulin" as used herein refers to insulin naturally produced in the human body or recombinantly produced insulin identical thereto. Recombinant human insulin may be produced in any suitable host cell, for example the host cells may be bacterial, fungal (including yeast), insect, animal or plant cells.
The term "insulin derivative" as used herein (and related terms) refers to human insulin or an analogue thereof in which at least one organic substituent is bound to one or more of the amino acids.
By the term "analogue of human insulin" as used herein (and related terms) is meant human insulin in which one or more amino acids have been deleted and/or replaced by other amino acids, including non-codeable amino acids, or human insulin comprising additional amino acids, i.e. more than 51 amino acids, such that the resulting analogue possesses insulin activity.
Rapid acting insulin is intended to mean human insulin, insulin analogues or insulin derivatives having an onset of action after injection or any other form of administration faster or equal to that of soluble and neutral formulations of human insulin.
The term "phenolic compound" or similar expressions as used herein refers to a compound in which a hydroxyl group is bound directly to a benzene or substituted benzene ring. Examples of such compounds include, but are not limited to, phenol, o-cresol, m-cresol, p-cresol, chloro-cresol, thymol, and 7-hydroxyindole.

## DESCRIPTION OF THE INVENTION

The present invention is based on the discovery that the two known ligand binding sites of the R-state insulin hexamer can be used to obtain an insulin composition having improved physical and chemical stability.
The basic concept underlying the present invention involves reversible attachment of a ligand to the $His^{B10}$ $Zn^{2+}$ site of the R-state hexamer. The anions currently used in insulin compositions as allosteric ligands for the R-state hexamers (notably chloride ion) bind only weakly to the $His^{B10}$ anion site.
The $His^{B10}$ $Zn^{2+}$ site consists of a tunnel or cavity with a triangular-shaped cross-section that extends ~12 Å from the

surface of the hexamer down to the $His^{B10}$ $Zn^{2+}$ ion. The diameter of the tunnel varies along its length and, depending on the nature of the ligand occupying the site, the opening can be capped over by the $Asn^{B3}$ and $Phe^{B1}$ side chains. The walls of the tunnel are made up of the side chains of the amino acid residues along one face each of the three alpha-helices. The side chains from each helix that make up the lining of the tunnel are $Phe^{B1}$, $Asn^{B3}$, and $Leu^{B6}$. Therefore, except for the zinc ion, which is coordinated to three $His^{B10}$ residues and is positioned at the bottom of the tunnel, the site is principally hydrophobic. Depending on the ligand structure, it may be possible for substituents on the ligand to make H-bonding interactions with $Asn^{B3}$ and with the peptide linkage to $Cys^{B7}$.

In one aspect the invention provides a pharmaceutical composition comprising insulin and a zinc-binding ligand which reversibly binds to a HisB10 Zn2+ site of an insulin hexamer,
wherein the ligand is selected from the group consisting of benzotriazoles, 3-hydroxy 2-napthoic acids, salicylic acids, tetrazoles, thiazolidinediones, 5-mercaptotetrazoles, or 4-cyano-1,2,3-triazoles, or any enantiomer, diastereomer, including a racemic mixture, tautomer as well as a salt thereof with a pharmaceutically acceptable acid or base.

In one embodiment the invention provides a pharmaceutical composition wherein the zinc-binding ligand is

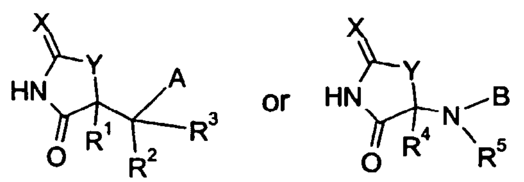

wherein

X is =O, =S or =NH

Y is -S-, -O- or -NH-

$R^1$ and $R^4$ are independently selected from hydrogen or $C_1$-$C_6$-alkyl,

$R^2$ is hydrogen or $C_1$-$C_6$-alkyl or aryl, $R^1$ and $R^2$ may optionally be combined to form a double bond,

$R^3$ and $R^5$ are independently selected from hydrogen, halogen, aryl, $C_1$-$C_6$-alkyl, or -C(O)$NR^{11}R^{12}$,

A and B are independently selected from $C_1$-$C_6$-alkyl, aryl, aryl-$C_1$-$C_6$-alkyl, aryl-$C_2$-$C_6$-alkenyl or heteroaryl, wherein the alkyl or alkenyl is optionally substituted with one or more substituents independently selected from $R^6$ and the aryl or heteroaryl is optionally substituted with up to four substituents $R^7$, $R^8$, $R^9$, and $R^{10}$,

A and $R^3$ may be connected through one or two valence bonds, B and $R^5$ may be connected through one or two valence bonds,

$R^6$ is independently selected from halogen, -CN, -$CF_3$, -$OCF_3$, aryl, -COOH and -$NH_2$,

$R^7$, $R^8$, $R^9$ and $R^{10}$ are independently selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -$S(O)_2CF_3$, -OS$(O)_2CF_3$, -$SCF_3$, -$NO_2$, -$OR^{11}$, -$NR^{11}R^{12}$, -$SR^{11}$, -$NR^{11}S(O)_2R^{12}$, -$S(O)_2NR^{11}R^{12}$, -S(O)$NR^{11}R^{12}$, -S(O)$R^{11}$, -$S(O)_2R^{11}$, -OS$(O)_2$ $R^{11}$, -C(O)$NR^{11}R^{12}$, -OC(O)$NR^{11}R^{12}$, -$NR^{11}$C(O)$R^{12}$, -$CH_2$C(O)$NR^{11}R^{12}$, -O$C_1$-$C_6$-alkyl-C(O)$NR^{11}R^{12}$, -$CH_2OR^{11}$, -$CH_2$OC(O)$R^{11}$, -$CH_2NR^{11}R^{12}$, -OC(O)$R^{11}$, -O$C_1$-$C_{15}$-alkyl-C(O)$OR^{11}$, -O$C_1$-$C_6$-alkyl-$OR^{11}$, -S$C_1$-$C_6$-alkyl-C(O)$OR^{11}$, -$C_2$-$C_6$-alkenyl-C(=O)$OR^{11}$, -$NR^{11}$-C(=O)-$C_1$-$C_6$-alkyl-C(=O)$OR^{11}$, -$NR^{11}$-C(=O)-$C_1$-$C_6$-alkenyl-C(=O)$OR^{11}$ , -C(O)$OR^{11}$, C(O)$R^{11}$, or -$C_2$-$C_6$-alkenyl-C(=O)$R^{11}$, =O, or -$C_2$-$C_6$-alkenyl-C(=O)-$NR^{11}R^{12}$,

- $C_1$-$C_6$-alkyl, $C_2$-$C_6$-alkenyl or $C_2$-$C_6$-alkynyl, each of which may optionally be substituted with one or more substituents independently selected from $R^{13}$,

- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl, heteroaryl-

$C_2$-$C_6$-alkynyl, or $C_3$-$C_6$ cycloalkyl,

of which each cyclic moiety may optionally be substituted with one or more substituents independently selected from $R^{14}$,

$R^{11}$ and $R^{12}$ are independently selected from hydrogen, OH, $C_1$-$C_{20}$-alkyl, aryl-$C_1$-$C_6$-alkyl or aryl, wherein the alkyl groups may optionally be substituted with one or more substituents independently selected from $R^{15}$ , and the aryl groups may optionally be substituted one or more substituents independently selected from $R^{16}$; $R^{11}$ and $R^{12}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{13}$ is independently selected from halogen, -CN, -$CF_3$, -$OCF_3$, -$OR^{11}$, -C(O)$OR^{11}$, -$NR^{11}R^{12}$, and -C(O)$NR^{11}R^{12}$,

$R^{14}$ is independently selected from halogen, -C(O)$OR^{11}$, -$CH_2C(O)OR^{11}$, -$CH_2OR^{11}$, -CN, - $CF_3$, -$OCF_3$, -$N0_2$, -$OR^{11}$, -$NR^{11}R^{12}$, $S(O)_2R^{11}$, aryl and $C_1$-$C_6$-alkyl,

$R^{15}$ is independently selected from halogen, -CN, -$CF_3$, -$OCF_3$, -$OC_1$-$C_6$-alkyl, -C(O)$OC_1$-$C_6$-alkyl, -COOH and -$NH_2$,

$R^{16}$ is independently selected from halogen, -C(O)$OC_1$-$C_6$-alkyl, -COOH, -CN, -$CF_3$, -$OCF_3$, - $N0_2$, -OH, -$OC_1$-$C_6$-alkyl, -$NH_2$, C(=O) or $C_1$-$C_6$-alkyl, or any enantiomer, diastereomer, including a racemic mixture, tautomer as well as a salt thereof with a pharmaceutically acceptable acid or base.

In another embodiment the invention provides a pharmaceutical composition wherein X is =O or =S.
In another embodiment the invention provides a pharmaceutical composition wherein X is =O.
In another embodiment the invention provides a pharmaceutical composition wherein X is =S.
In another embodiment the invention provides a pharmaceutical composition wherein Y is -O- or -S-.
In another embodiment the invention provides a pharmaceutical composition wherein Y is -O-.
In another embodiment the invention provides a pharmaceutical composition wherein Y is -S-.
In another embodiment the invention provides a pharmaceutical composition wherein A is aryl optionally substituted with up to four substituents, $R^7$, $R^8$, $R^9$, and $R^{10}$ which may be the same or different.
In another embodiment the invention provides a pharmaceutical composition wherein A is selected from ArG1 optionally substituted with up to four substituents, $R^7$, $R^8$, $R^9$, and $R^{10}$ which may be the same or different.
In another embodiment the invention provides a pharmaceutical composition wherein A is phenyl or naphtyl optionally substituted with up to four substituents, $R^7$, $R^8$, $R^9$, and $R^{10}$ which may be the same or different.
In another embodiment the invention provides a pharmaceutical composition wherein A is

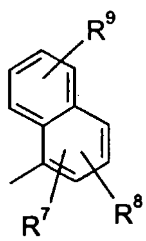

or

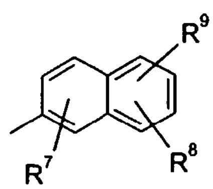

In another embodiment the invention provides a pharmaceutical composition wherein A is phenyl.
In another embodiment the invention provides a pharmaceutical composition wherein A is heteroaryl optionally substituted with up to four substituents, $R^7$, $R^8$, $R^9$, and $R^{10}$ which may be the same or different.
In another embodiment the invention provides a pharmaceutical composition wherein A is selected from Het1 optionally substituted with up to four substituents, $R^7$, $R^8$, $R^9$, and $R^{10}$ which may be the same or different.
In another embodiment the invention provides a pharmaceutical composition wherein A is selected from Het2 optionally substituted with up to four substituents, $R^7$, $R^8$, $R^9$, and $R^{10}$ which may be the same or different.
In another embodiment the invention provides a pharmaceutical composition wherein A is selected from Het3 optionally substituted with up to four substituents, $R^7$, $R^8$, $R^9$, and $R^{10}$ which may be the same or different.
In another embodiment the invention provides a pharmaceutical composition wherein A is selected from the group consisting of indolyl, benzofuranyl, quinolyl, furyl, thienyl, or pyrrolyl,

wherein each heteroaryl may optionally substituted with up to four substituents, $R^7$, $R^8$, $R^9$, and $R^{10}$ which may be the same or different.

In another embodiment the invention provides a pharmaceutical composition wherein A is benzofuranyl optionally substituted with up to four substituents $R^7$, $R^8$, $R^9$, and $R^{10}$ which may be the same or different.

In another embodiment the invention provides a pharmaceutical composition wherein A is

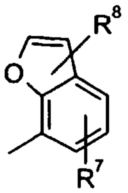

or

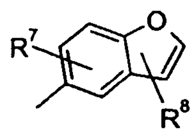

or

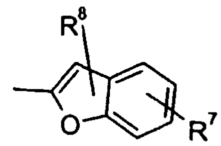

In another embodiment the invention provides a pharmaceutical composition wherein A is carbazolyl optionally substituted with up to four substituents $R^7$, $R^8$, $R^9$, and $R^{10}$ which may be the same or different.

In another embodiment the invention provides a pharmaceutical composition wherein A is

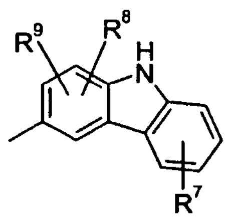

In another embodiment the invention provides a pharmaceutical composition wherein A is quinolyl optionally substituted with up to four substituents $R^7$, $R^8$, $R^9$, and $R^{10}$ which may be the same or different.

In another embodiment the invention provides a pharmaceutical composition wherein A is

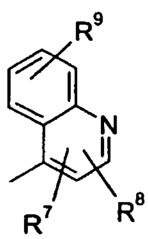

or

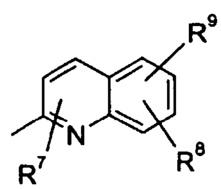

In another embodiment the invention provides a pharmaceutical composition wherein A is indolyl optionally substituted with up to four substituents $R^7$, $R^8$, $R^9$, and $R^{10}$ which may be the same or different.

In another embodiment the invention provides a pharmaceutical composition wherein A is

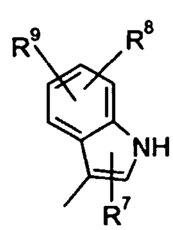

or

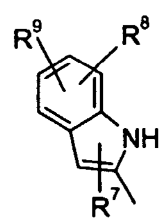

In another embodiment the invention provides a pharmaceutical composition wherein $R^1$ is hydrogen.

In another embodiment the invention provides a pharmaceutical composition wherein $R^2$ is hydrogen.

In another embodiment the invention provides a pharmaceutical composition wherein $R^1$ and $R^2$ are combined to form a double bond.

In another embodiment the invention provides a pharmaceutical composition wherein $R^3$ is $C_1$-$C_6$-alkyl, halogen, or C (O)$NR^{16}R^{17}$.

In another embodiment the invention provides a pharmaceutical composition wherein $R^3$ is $C_1$-$C_6$-alkyl or C(O)$NR^{16}R^{17}$.

In another embodiment the invention provides a pharmaceutical composition wherein $R^3$ is methyl.

In another embodiment the invention provides a pharmaceutical composition wherein B is phenyl optionally substituted with up to four substituents, $R^7$, $R^6$, $R^9$, and $R^{10}$ which may be the same or different.

In another embodiment the invention provides a pharmaceutical composition wherein $R^4$ is hydrogen.

In another embodiment the invention provides a pharmaceutical composition wherein $R^5$ is hydrogen.

In another embodiment the invention provides a pharmaceutical composition wherein $R^6$ is aryl.

In another embodiment the invention provides a pharmaceutical composition wherein $R^6$ is phenyl.

In another embodiment the invention provides a pharmaceutical composition wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-NR^{11}S(O)_2R^{12}$, $-S(O)_2NR^{11}R^{12}$, $-S(O)NR^{11}R^{12}$, $-S(O)R^{11}$, -S (O)$_2R^{11}$, $-OS(O)_2$ $R^{11}$, $-NR^{11}C(O)R^{12}$, $-CH_2OR^{11}$,$-CH_2OC(O)R^{11}$, $-CH_2NR^{11}R^{12}$, $-OC(O)R^{11}$, $-OC_1$-$C_6$-alkyl-C(O) $OR^{11}$, $-OC_1$-$C_6$-alkyl-C(O)$NR^{11}R^{12}$, $-OC_1$-$C_6$-alkyl-$OR^{11}$, $-SC_1$-$C_6$-alkyl-C(O)$OR^{11}$, $-C_2$-$C_6$-alkenyl-C(=O)$OR^{11}$, -C (O)$OR^{11}$, or $-C_2$-$C_6$-alkenyl-C(=O)$R^{11}$,
- $C_1$-$C_6$-alkyl, $C_2$-$C_6$-alkenyl or $C_2$-$C_6$-alkynyl, which may each optionally be substituted with one or more substituents independently selected from $R^{13}$
- aryl, aryloxy, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, wherein each of the cyclic moieties optionally may be substituted with one or more substituents independently selected from $R^{14}$

In another embodiment the invention provides a pharmaceutical composition wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2$ $R^{11}$, - $CH_2OC(O)R^{11}$, $-OC(O)R^{11}$, $-OC_1$-$C_6$-alkyl-C(O)$OR^{11}$, $-OC_1$-$C_6$-alkyl-$OR^{11}$, $-SC_1$-$C_6$-alkyl-C(O)$OR^{11}$, -C(O)$OR^{11}$, or-$C_2$-$C_6$-alkenyl-C(=O)$R^{11}$,
- $C_1$-$C_6$-alkyl or $C_1$-$C_6$-alkenyl which may each optionally be substituted with one or more substituents independently selected from $R^{13}$
- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$

In another embodiment the invention provides a pharmaceutical composition wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2$ $R^{11}$,$-CH_2OC(O)R^{11}$, $-OC(O)R^{11}$, $-OC_1$-$C_6$-alkyl-C(O)$OR^{11}$, $-OC_1$-$C_6$-alkyl-$OR^{11}$, $-SC_1$-$C_6$-alkyl-C(O)$OR^{11}$, -C(O)$OR^{11}$, or $-C_2$-$C_6$-alkenyl-C(=O)$R^{11}$,
- $C_1$-$C_6$-alkyl or $C_1$-$C_6$- which may each optionally be substituted with one or more substituents independently selected from $R^{13}$
- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$.

In another embodiment the invention provides a pharmaceutical composition wherein $R^7$, $R^8$, $R^9$ and $R^{10}$ are independently selected from

- hydrogen, halogen, $-OR^{11}$, $-OC_1$-$C_6$-alkyl-C(O)$OR^{11}$, or -C(O)$OR^{11}$,
- $C_1$-$C_6$-alkyl which may each optionally be substituted with one or more substituents independently selected from $R^{13}$

- aryl, aryloxy, aryl-$C_1$-$C_6$-alkoxy,

of which each of the cyclic moieties optionally may be substituted with one or more substituents independently selected from $R^{14}$.

In another embodiment the invention provides a pharmaceutical composition wherein $R^7$, $R^8$, $R^9$ and $R^{10}$ are independently selected from

- hydrogen, halogen, $-OR^{11}$, $-OC_1$-$C_6$-alkyl-C(O)$OR^{11}$, or $-C(O)OR^{11}$,
- $C_1$-$C_6$-alkyl which may optionally be substituted with one or more substituents independently selected from $R^{13}$
- phenyl, phenyloxy, phenyl-$C_1$-$C_6$-alkoxy, wherein each of the cyclic moieties optionally may be substituted with one or more substituents independently selected from $R^{14}$.

In another embodiment the invention provides a pharmaceutical composition wherein $R^{11}$ and $R^{12}$ are independently selected from hydrogen, $C_1$-$C_{20}$-alkyl, aryl or aryl-$C_1$-$C_6$-alkyl, wherein the alkyl groups may optionally be substituted with one or more substituents independently selected from $R^{15}$, and the aryl groups may optionally be substituted one or more substituents independently selected from $R^{16}$; $R^{11}$ and $R^{12}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds.

In another embodiment the invention provides a pharmaceutical composition wherein $R^{11}$ and $R^{12}$ are independently selected from hydrogen, $C_1$-$C_{20}$-alkyl, aryl or aryl-$C_1$-$C_6$-alkyl, wherein the alkyl groups may optionally be substituted with one or more substituents independently selected from $R^{15}$, and the aryl groups may optionally be substituted one or more substituents independently selected from $R^{16}$.

In another embodiment the invention provides a pharmaceutical composition wherein $R^{11}$ and $R^{12}$ are independently selected from phenyl or phenyl-$C_1$-$C_6$-alkyl.

In another embodiment the invention provides a pharmaceutical composition wherein one or both of $R^{11}$ and $R^{12}$ are methyl.

In another embodiment the invention provides a pharmaceutical composition wherein $R^{13}$ is independently selected from halogen, $CF_3$, $OR^{11}$ or $NR^{11}R^{12}$.

In another embodiment the invention provides a pharmaceutical composition wherein $R^{13}$ is independently selected from halogen or $OR^{11}$.

In another embodiment the invention provides a pharmaceutical composition wherein $R^{13}$ is $OR^{11}$.

In another embodiment the invention provides a pharmaceutical composition wherein $R^{14}$ is independently selected from halogen, $-C(O)OR^{11}$, -CN, $-CF_3$, $-OR^{11}$, $S(O)_2R^{11}$, and $C_1$-$C_6$-alkyl.

In another embodiment the invention provides a pharmaceutical composition wherein $R^{14}$ is independently selected from halogen, $-C(O)OR^{11}$, or $-OR^{11}$.

In another embodiment the invention provides a pharmaceutical composition wherein $R^{15}$ is independently selected from halogen, -CN, $-CF_3$, $-C(O)OC_1$-$C_6$-alkyl,and -COOH.

In another embodiment the invention provides a pharmaceutical composition wherein $R^{15}$ is independently selected from halogen or $-C(O)OC_1$-$C_6$-alkyl.

In another embodiment the invention provides a pharmaceutical composition wherein $R^{16}$ is independently selected from halogen, $-C(O)OC_1$-$C_6$-alkyl, -COOH, $-NO_2$, $-OC_1$-$C_6$-alkyl, $-NH_2$, C(=O) or $C_1$-$C_6$-alkyl.

In another embodiment the invention provides a pharmaceutical composition wherein $R^{16}$ is independently selected from halogen, $-C(O)OC_1$-$C_6$-alkyl, -COOH, $-NO_2$, or $C_1$-$C_6$-alkyl.

In another embodiment the invention provides a pharmaceutical composition wherein the zinc-binding ligand is

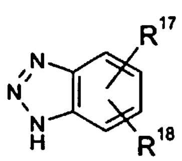 or 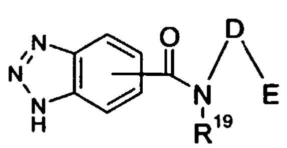 or 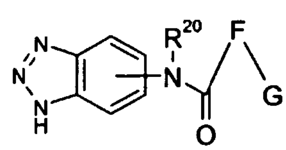

wherein

$R^{19}$ is hydrogen or $C_1$-$C_6$-alkyl,

$R^{20}$ is hydrogen or $C_1$-$C_6$-alkyl,

D and F are a valence bond or $C_1$-$C_6$-alkylene optionally substituted with one or more substituents independently selected from $R^{72}$,

$R^{72}$ is independently selected from hydroxy, $C_1$-$C_6$-alkyl, or aryl,

E is $C_1$-$C_6$-alkyl, aryl or heteroaryl, wherein the aryl or heteroaryl is optionally substituted with up to three substituents $R^{21}$, $R^{22}$ and $R^{23}$,

G is $C_1$-$C_6$-alkyl, aryl or heteroaryl, wherein the aryl or heteroaryl is optionally substituted with up to three substituents $R^{24}$, $R^{25}$ and $R^{26}$,

$R^{17}$ $R^{18}$, $R^{21}$, $R^{22}$, $R^{23}$, $R^{24}$, $R^{25}$ and $R^{26}$ are independently selected from

- hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, $-OR^{27}$, $-NR^{27}R^{28}$, $-SR^{27}$, $-NR^{27}S(O)_2R^{28}$, $-S(O)_2NR^{27}R^{28}$, $-S(O)NR^{27}R^{28}$, $-S(O)R^{27}$, $-S(O)_2R^{27}$, $-C(O)NR^{27}R^{28}$, $-OC(O)NR^{27}R^{28}$, $-NR^{27}C(O)R^{28}$, $-NR^{27}C(O)OR^{28}$, $-CH_2C(O)NR^{27}R^{28}$, $-OCH_2C(O)NR^{27}R^{28}$, $-CH_2OR^{27}$, $-CH_2NR^{27}R^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-NR^{27}$-C(=O)-$C_1$-$C_6$-alkyl-$C(=O)OR^{27}$, $-NR^{27}$-C(=O)-$C_1$-$C_6$-alkenyl-$C(=O)OR^{27}$, -C(=O)$NR^{27}$-$C_1$-$C_6$-alkyl-$C(=O)OR^{27}$, $-C_1$-$C_6$-alkyl-$C(=O)OR^{27}$, or $-C(O)OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$,

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$,

$R^{27}$ and $R^{28}$ are independently selected from hydrogen, $C_1$-$C_6$-alkyl, aryl-$C_1$-$C_6$-alkyl or aryl, or $R^{27}$ and $R^{28}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{29}$ is independently selected from halogen, -CN, $-CF_3$, $-OCF_3$, $-OR^{27}$, and $-NR^{27}R^{28}$,

$R^{30}$ is independently selected from halogen, $-C(O)OR^{27}$, -CN, $-CF_3$, $-OCF_3$, $-NO_2$, $-OR^{27}$, $-NR^{27}R^{28}$ and $C_1$-$C_6$-alkyl, or any enantiomer, diastereomer, including a racemic mixture, tautomer as well as a salt thereof with a pharmaceutically acceptable acid or base.

In another embodiment the invention provides a pharmaceutical composition wherein D is a valence bond.

In another embodiment the invention provides a pharmaceutical composition wherein D is $C_1$-$C_6$-alkylene optionally substituted with one or more hydroxy, $C_1$-$C_6$-alkyl, or aryl.

In another embodiment the invention provides a pharmaceutical composition wherein E is aryl or heteroaryl, wherein the aryl or heteroaryl is optionally substituted with up to three substituents independently selected from $R^{21}$, $R^{22}$ and $R^{23}$.

In another embodiment the invention provides a pharmaceutical composition wherein E is aryl optionally substituted with up to three substituents independently selected from $R^{21}$, $R^{22}$ and $R^{23}$.

In another embodiment the invention provides a pharmaceutical composition wherein E is selected from ArG1 and optionally substituted with up to three substituents independently selected from $R^{21}$, $R^{22}$ and $R^{23}$.

In another embodiment the invention provides a pharmaceutical composition wherein E is phenyl optionally substituted with up to three substituents independently selected from $R^{21}$, $R^{22}$ and $R^{23}$.

In another embodiment the invention provides a pharmaceutical composition wherein the zinc-binding ligand is

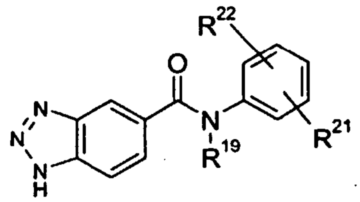

In another embodiment the invention provides a pharmaceutical composition wherein $R^{21}$, $R^{22}$ and $R^{23}$ are independently selected from

- hydrogen, halogen, $-CHF_2$, $-CF_3$, $-OCF_3$, $-OCHF_2$, $-OCH_2CF_3$, $-OCF_2CHF_2$, $-SCF_3$, - $NO_2$, $-OR^{27}$, $-NR^{27}R^{28}$, $-SR^{27}$, $-C(O)NR^{27}R^{28}$, $-OC(O)NR^{27}R^{28}$, $-NR^{27}C(O)R^{28}$, $-NR^{27}C(O)OR^{28}$, $-CH_2C(O)NR^{27}R^{28}$, $-OCH_2C(O)NR^{27}R^{28}$, $-CH_2OR^{27}$, $-CH_2NR^{27}R^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O) $OR^{27}$, $-NR^{27}$-C(=O)-$C_1$-$C_6$-alkyl-C(=O)$OR^{27}$, $-NR^{27}$-C(=O)-$C_1$-$C_6$-alkenyl-C(=O)$OR^{27}$-, -C(=O)$NR^{27}$-$C_1$-$C_6$-alkyl-C (=O)$OR^{27}$, -$C_1$-$C_6$-alkyl-C(=O)$OR^{27}$, or -C(O)$OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-C(O)$OR^{27}$, $-SC_1$-$C_6$-alkyl-C(O)$OR^{27}$, $-C_2$-$C_6$-alkenyl-C(=O)$OR^{27}$, -C(=O)$NR^{27}$-$C_1$-$C_6$-alkyl-C(=O)$OR^{27}$, -$C_1$-$C_6$-alkyl-C(=O)$OR^{27}$, or -C(O)$OR^{27}$,

- $C_1$-$C_6$-alkyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-C(O)$OR^{27}$, $-SC_1$-$C_6$-alkyl-C(O)$OR^{27}$, $-C_2$-$C_6$-alkenyl-C(=O)$OR^{27}$, -C(=O)$NR^{27}$-$C_1$-$C_6$-alkyl-C(=O)$OR^{27}$, -$C_1$-$C_6$-alkyl-C(=O)$OR^{27}$, or -C(O)$OR^{27}$,

- methyl, ethyl propyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-C(O)$OR^{27}$, $-SC_1$-$C_6$-alkyl-C(O)$OR^{27}$, $-C_2$-$C_6$-alkenyl-C(=O)$OR^{27}$, -C(=O)$NR^{27}$-$C_1$-$C_6$-alkyl-C(=O)$OR^{27}$, -$C_1$-$C_6$-alkyl-C(=O)$OR^{27}$, or -C(O)$OR^{27}$,

- methyl, ethyl propyl optionally substituted with one or more substituents independently selected from $R^{29}$

- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, -$OCF_3$, -$OR^{27}$, -$NR^{27}R^{28}$, -$SR^{27}$, -$NR^{27}C(O)R^{28}$, -$NR^{27}C(O)OR^{28}$, -$OC(O)R^{27}$, -$OC_1$-$C_6$-alkyl-C(O)$OR^{27}$, -$SC_1$-$C_6$-alkyl-C(O)$OR^{27}$, -$C_2$-$C_6$-alkenyl-C(=O)$OR^{27}$, -C(=O)$NR^{27}$-$C_1$-$C_6$-alkyl-C(=O)$OR^{27}$. -$C_1$-$C_6$-alkyl-C(=O)$OR^{27}$, or -C(O)$OR^{27}$,

- $C_1$-$C_6$-alkyl optionally substituted with one or more substituents independently selected from $R^{29}$

- phenyl, phenyloxy, phenyl-$C_1$-$C_6$-alkoxy, phenyl-$C_1$-$C_6$-alkyl,

of which the cyclic moieties optionally may be substituted with one or more substituents selected from $R^{30}$.

In another embodiment the invention provides a pharmaceutical composition wherein $R^{19}$ is hydrogen or methyl.
In another embodiment the invention provides a pharmaceutical composition wherein $R^{19}$ is hydrogen.
In another embodiment the invention provides a pharmaceutical composition wherein $R^{27}$ is Hydrogen, $C_1$-$C_6$-alkyl or aryl.
In another embodiment the invention provides a pharmaceutical composition wherein $R^{27}$ is hydrogen or $C_1$-$C_6$-alkyl.
In another embodiment the invention provides a pharmaceutical composition wherein $R^{28}$ is hydrogen or $C_1$-$C_6$-alkyl.
In another embodiment the invention provides a pharmaceutical composition wherein F is a valence bond.
In another embodiment the invention provides a pharmaceutical composition wherein F is $C_1$-$C_6$-alkylene optionally substituted with one or more hydroxy, $C_1$-$C_6$-alkyl, or aryl.
In another embodiment the invention provides a pharmaceutical composition wherein G is $C_1$-$C_6$-alkyl or aryl, wherein the aryl is optionally substituted with up to three substituents $R^{24}$, $R^{25}$ and $R^{26}$.
In another embodiment the invention provides a pharmaceutical composition wherein G is $C_1$-$C_6$-alkyl or ArG1, wherein the aryl is optionally substituted with up to three substituents $R^{24}$, $R^{25}$ and $R^{26}$.
In another embodiment the invention provides a pharmaceutical composition wherein G is $C_1$-$C_6$-alkyl.
In another embodiment the invention provides a pharmaceutical composition wherein G is phenyl optionally substituted with up to three substituents $R^{24}$, $R^{25}$ and $R^{26}$.
In another embodiment the invention provides a pharmaceutical composition wherein $R^{24}$, $R^{25}$ and $R^{26}$ are independently selected from

- hydrogen, halogen, -$CHF_2$, -$CF_3$, -$OCF_3$, -$OCHF_2$, -$OCH_2CF_3$, -$OCF_2CHF_2$, -$SCF_3$, - $NO_2$, -$OR^{27}$, -$NR^{27}R^{28}$, -$SR^{27}$, -C(O)$NR^{27}R^{28}$, -OC(O)$NR^{27}R^{28}$, -$NR^{27}C(O)R^{28}$, -$NR^{27}C(O)OR^{28}$, -$CH_2C(O)NR^{27}R^{28}$, -$OCH_2C(O)NR^{27}R^{28}$, -$CH_2OR^{27}$, -$CH_2NR^{27}R^{28}$, -OC(O)$R^{27}$, -$OC_1$-$C_6$-alkyl-C(O)$OR^{27}$, -$SC_1$-$C_6$-alkyl-C(O)$OR^{27}$, -$C_2$-$C_6$-alkenyl-C(=O)$OR^{27}$, -$NR^{27}$-C(=O)-$C_1$-$C_6$-alkyl-C(=O)$OR^{27}$, -$NR^{27}$-C(=O)-$C_1$-$C_6$-alkenyl-C(=O)$OR^{27}$-, -C(=O)$NR^{27}$-$C_1$-$C_6$-alkyl-C(=O)$OR^{27}$, -$C_1$-$C_6$-alkyl-C(=O)$OR^{27}$, or -C(O)$OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment the invention provides a pharmaceutical composition wherein $R^{24}$, $R^{25}$ and $R^{26}$ are independently selected from

- hydrogen, halogen, -$OCF_3$, -$OR^{27}$, -$NR^{27}R^{28}$, -$SR^{27}$, -$NR^{27}C(O)R^{28}$, -$NR^{27}C(O)OR^{28}$, -$OC(O)R^{27}$, -$OC_1$-$C_6$-alkyl-C(O)$OR^{27}$, -$SC_1$-$C_6$-alkyl-C(O)$OR^{27}$, -$C_2$-$C_6$-alkenyl-C(=O)$OR^{27}$, -C(=O)$NR^{27}$-$C_1$-$C_6$-alkyl-C(=O)$OR^{27}$, -$C_1$-$C_6$-alkyl-C(=O)$OR^{27}$, or -C(O)$OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment the invention provides a pharmaceutical composition wherein $R^{24}$, $R^{25}$ and $R^{26}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- $C_1$-$C_6$-alkyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- methyl, ethyl propyl optionally substituted with one or more substituents independently selected from $R^{29}$

- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- methyl, ethyl propyl optionally substituted with one or more substituents independently selected from $R^{29}$

- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment the invention provides a pharmaceutical composition wherein $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$alkyl-$C(=O)OR^{27}$. $-C_1$-$C_6$-alkyl-$C(=O)OR^{27}$, or $-C(O)OR^{27}$,
- methyl, ethyl propyl optionally substituted with one or more substituents independently selected from $R^{29}$
- ArG1, ArG1-O-, ArG1-$C_1$-$C_6$-alkoxy, ArG1-$C_1$-$C_6$-alkyl,

of which the cyclic moieties optionally may be substituted with one or more substituents selected from $R^{30}$.

In another embodiment the invention provides a pharmaceutical composition wherein $R^{20}$ is hydrogen or methyl.
In another embodiment the invention provides a pharmaceutical composition wherein $R^{20}$ is hydrogen.
In another embodiment the invention provides a pharmaceutical composition wherein $R^{27}$ is hydrogen, $C_1$-$C_6$-alkyl or aryl.
In another embodiment the invention provides a pharmaceutical composition wherein $R^{27}$ is hydrogen or $C_1$-$C_6$-alkyl or ArG1.
In another embodiment the invention provides a pharmaceutical composition wherein $R^{27}$ is hydrogen or $C_1$-$C_6$-alkyl.
In another embodiment the invention provides a pharmaceutical composition wherein $R^{28}$ is hydrogen or $C_1$-$C_6$-alkyl.
In another embodiment the invention provides a pharmaceutical composition wherein $R^{17}$ and $R^{18}$ are independently selected from

- hydrogen, halogen, -CN, $-CF_3$, $-OCF_3$, $-NO_2$, $-OR^{27}$, $-NR^{27}R^{28}$, $-SR^{27}$, $-S(O)R^{27}$, $-S(O)_2R^{27}$, $-C(O)NR^{27}R^{28}$,

$-CH_2OR^{27}$, $-OC(O)R^{27}$, $-OC_1-C_6$-alkyl-$C(O)OR^{27}$, $-SC_1-C_6$-alkyl-$C(O)OR^{27}$, or $-C(O)OR^{27}$,

- $C_1-C_6$-alkyl, $C_2-C_6$-alkenyl or $C_2-C_6$-alkynyl, optionally substituted with one or more substituents independently selected from $R^{29}$
- aryl, aryloxy, aroyl, aryl-$C_1-C_6$-alkoxy, aryl-$C_1-C_6$-alkyl, heteroaryl, heteroaryl-$C_1-C_6$-alkyl,

of which the cyclic moieties optionally may be substituted with one or more substituents selected from $R^{30}$.

In another embodiment the invention provides a pharmaceutical composition wherein $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$,
- $C_1-C_6$-alkyl optionally substituted with one or more substituents independently selected from $R^{29}$
- aryl, aryloxy, aroyl, aryl-$C_1-C_6$-alkoxy, aryl-$C_1-C_6$-alkyl, heteroaryl, heteroaryl-$C_1-C_6$-alkyl,

of which the cyclic moieties optionally may be substituted with one or more substituents selected from $R^{30}$.

In another embodiment the invention provides a pharmaceutical composition wherein $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$
- methyl, ethyl propyl optionally substituted with one or more substituents independently selected from $R^{29}$
- aryl, aryloxy, aroyl, aryl-$C_1-C_6$-alkoxy, aryl-$C_1-C_6$-alkyl, heteroaryl, heteroaryl-$C_1-C_6$-alkyl

of which the cyclic moieties optionally may be substituted with one or more substituents selected from $R^{30}$.

In another embodiment the invention provides a pharmaceutical composition wherein $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$
- methyl, ethyl propyl optionally substituted with one or more substituents independently selected from $R^{29}$
- ArG1, ArG1-O-, ArG1-C(O)-, ArG1-$C_1-C_6$-alkoxy, ArG1-$C_1-C_6$-alkyl, Het3, Het3-$C_1-C_6$-alkyl

of which the cyclic moieties optionally may be substituted with one or more substituents selected from $R^{30}$.

In another embodiment the invention provides a pharmaceutical composition wherein $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$
- $C_1-C_6$-alkyl optionally substituted with one or more substituents independently selected from $R^{29}$
- phenyl, phenyloxy, phenyl-$C_1-C_6$-alkoxy, phenyl-$C_1-C_6$-alkyl,

of which the cyclic moieties optionally may be substituted with one or more substituents selected from $R^{30}$.

In another embodiment the invention provides a pharmaceutical composition wherein $R^{27}$ is hydrogen or $C_1-C_6$-alkyl.
In another embodiment the invention provides a pharmaceutical composition wherein $R^{27}$ is hydrogen, methyl or ethyl.
In another embodiment the invention provides a pharmaceutical composition wherein $R^{28}$ is hydrogen or $C_1-C_6$-alkyl.
In another embodiment the invention provides a pharmaceutical composition wherein $R^{28}$ is hydrogen, methyl or ethyl.
In another embodiment the invention provides a pharmaceutical composition wherein $R^{72}$ is -OH or phenyl.

In another embodiment the invention provides a pharmaceutical composition wherein the zinc-binding ligand is

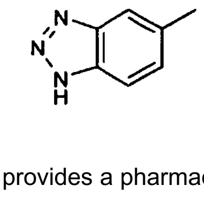

In another embodiment the invention provides a pharmaceutical composition wherein the zinc-binding ligand is of the form H-I-J

wherein H is

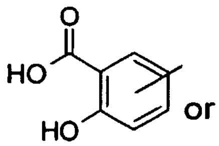 or 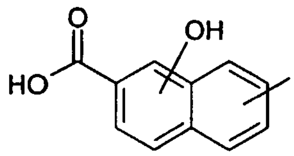 or 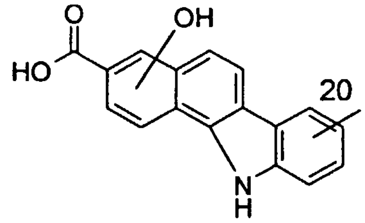

wherein the phenyl, naphthalene or benzocarbazole rings are optionally substituted with one or more substituents independently selected from $R^{31}$

I is selected from

- a valence bond,
- $-CH_2N(R^{32})-$ or $-SO_2N(R^{33})-$,
- 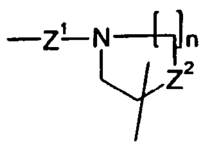

wherein $Z^1$ is $S(O)_2$ or $CH_2$, $Z^2$ is -NH-, -O-or -S-, and n is 1 or 2,

J is

- $C_1$-$C_6$-alkyl, $C_2$-$C_8$-alkenyl or $C_2$-$C_6$-alkynyl, which may each optionally be substituted with one or more substituents selected from $R^{34}$,
- Aryl, aryloxy, aryl-oxycarbonyl-, aroyl, aryl-$C_1$-$C_6$-alkoxy-, aryl-$C_1$-$C_6$-alkyl-, aryl-$C_2$-$C_6$-alkenyl-, aryl-$C_2$-$C_6$-alkynyl-, heteroaryl, heteroaryl-$C_1$-$C_6$-alkyl-, heteroaryl-$C_2$-$C_6$-alkenyl- or heteroaryl-$C_2$-$C_6$-alkynyl-, wherein the cyclic moieties are optionally substituted with one or more substituents selected from $R^{37}$,
- hydrogen,

$R^{31}$ is independently selected from hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, $-OR^{35}$, $-C(O)R^{35}$, $-NR^{35}R^{36}$, $-SR^{35}$, $-NR^{35}S(O)_2R^{36}$, $-S(O)_2NR^{35}R^{36}$, $-S(O)NR^{35}R^{36}$, $-S(O)R^{35}$, $-S(O)_2R^{35}$, $-C(O)NR^{35}R^{36}$, $-OC(O)NR^{35}R^{36}$, $-NR^{35}C(O)R^{36}$, $-CH_2C(O)NR^{35}R^{36}$, $-OCH_2C(O)NR^{35}R^{36}$, $-CH_2OR^{35}$, $-CH_2NR^{35}R^{36}$, $-OC(O)R^{35}$, $-OC_1$-$C_6$-alkyl-$C(O)OR^{35}$, $-SC_1$-$C_6$-alkyl-$C(O)OR^{35}$ $-C_2$-$C_6$-alkenyl-$C(=O)OR^{35}$, $-NR^{35}$-C(=O)-$C_1$-$C_6$-alkyl-$C(=O)OR^{35}$, $-NR^{35}$-C(=O)-$C_1$-$C_6$-alkenyl-$C(=O)OR^{35}$-, $C_1$-$C_6$-alkyl, $C_1$-$C_6$-alkanoyl or $-C(O)OR^{35}$,

$R^{32}$ and $R^{33}$ are independently selected from hydrogen, $C_1$-$C_6$-alkyl or $C_1$-$C_6$-alkanoyl,

$R^{34}$ is independently selected from halogen, -CN, $-CF_3$, $-OCF_3$, $-OR^{35}$, and $-NR^{35}R^{36}$,

$R^{35}$ and $R^{36}$ are independently selected from hydrogen, $C_1$-$C_6$-alkyl, aryl-$C_1$-$C_6$-alkyl or aryl, or $R^{35}$ and $R^{36}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{37}$ is independently selected from halogen, $-C(O)OR^{35}$, -C(O)H, -CN, $-CF_3$, $-OCF_3$, $-NO_2$,$-OR^{35}$, $-NR^{35}R^{36}$, $C_1$-$C_6$-alkyl or $C_1$-$C_6$-alkanoyl,

or any enantiomer, diastereomer, including a racemic mixture, tautomer as well as a salt thereof with a pharmaceutically

acceptable acid or base.

In another embodiment the invention provides a pharmaceutical composition wherein the zinc-binding ligand is of the form H-I-J, wherein H is

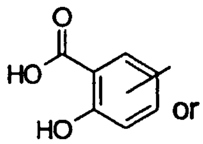 or 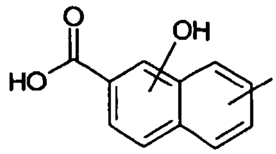 or 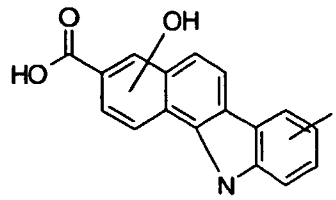

wherein the phenyl, naphthalene or benzocarbazole rings are optionally substituted with one or more substituents independently selected from $R^{31}$,

I is selected from

- a valence bond,
- $-CH_2N(R^{32})-$ or $-SO_2N(R^{33})-$,
- 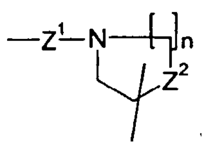

wherein $Z^1$ is $S(O)_2$ or $CH_2$, $Z^2$ is N,-O-or -S-, and n is 1 or 2,

J is

- $C_1$-$C_6$-alkyl, $C_2$-$C_6$-alkenyl or $C_2$-$C_6$-alkynyl, which may each optionally be substituted with one or more substituents selected from $R^{34}$,
- Aryl, aryloxy, aryl-oxycarbonyl-, aroyl, aryl-$C_1$-$C_6$-alkoxy-, aryl-$C_1$-$C_6$-alkyl-, aryl-$C_2$-$C_6$-alkenyl-, aryl-$C_2$-$C_6$-alkynyl-, heteroaryl, heteroaryl-$C_1$-$C_6$-alkyl-, heteroaryl-$C_2$-$C_6$-alkenyl- or heteroaryl-$C_2$-$C_6$-alkynyl-, wherein the cyclic moieties are optionally substituted with one or more substituents selected from $R^{37}$,
- hydrogen,

$R^{31}$ is independently selected from hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, $-OR^{35}$, $-C(O)R^{35}$, $-NR^{35}R^{36}$, $-SR^{35}$, $-NR^{35}S(O)_2R^{36}$, $-S(O)_2NR^{35}R^{36}$, $-S(O)NR^{35}R^{36}$, $-S(O)R^{35}$, $-S(O)_2R^{35}$, $-C(O)NR^{35}R^{36}$, $-OC(O)NR^{35}R^{36}$, $-NR^{35}C(O)R^{36}$, $-CH_2C(O)NR^{35}R^{36}$, $-OCH_2C(O)NR^{35}R^{36}$, $-CH_2OR^{35}$, $-CH_2NR^{35}R^{36}$, $-OC(O)R^{35}$, $-OC_1$-$C_6$-alkyl-$C(O)OR^{35}$, $-SC_1$-$C_6$-alkyl-$C(O)OR^{35}$ $-C_2$-$C_6$-alkenyl-$C(=O)OR^{35}$, $-NR^{35}$-C(=O)-$C_1$-$C_6$-alkyl-$C(=O)OR^{35}$, $-NR^{35}$-C(=O)-$C_1$-$C_6$-alkenyl-$C(=O)OR^{35}$-, $C_1$-$C_6$-alkyl, $C_1$-$C_6$-alkanoyl or -C (O) $OR^{35}$.

$R^{32}$ and $R^{33}$ are independently selected from hydrogen, $C_1$-$C_6$-alkyl or $C_1$-$C_6$-alkanoyl,

$R^{34}$ is independently selected from halogen, -CN, $-CF_3$, $-OCF_3$, $-OR^{35}$, and $-NR^{35}R^{36}$,

$R^{35}$ and $R^{36}$ are independently selected from hydrogen, $C_1$-$C_6$-alkyl, aryl-$C_1$-$C_6$-alkyl or aryl, or $R^{35}$ and $R^{36}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{37}$ is independently selected from halogen, $-C(O)OR^{35}$, -C(O)H, -CN, $-CF_3$, $-OCF_3$, $-NO_2$,$-OR^{35}$, $-NR^{35}R^{36}$, $C_1$-$C_6$-alkyl or $C_1$-$C_6$-alkanoyl,

or any enantiomer, diastereomer, including a racemic mixture, tautomer as well as a salt thereof with a pharmaceutically acceptable acid or base,
With the proviso that $R^{31}$ and J cannot both be hydrogen.

In another embodiment the invention provides a pharmaceutical composition wherein H is

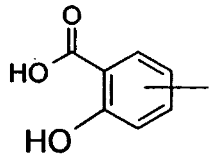

or

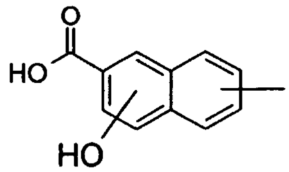

In another embodiment the invention provides a pharmaceutical composition wherein H is

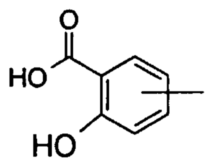

In another embodiment the invention provides a pharmaceutical composition wherein H is

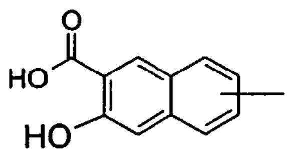

In another embodiment the invention provides a pharmaceutical composition wherein I is a valence bond, $-CH_2N(R^{32})-$, or $-SO_2N(R^{33})-$.
In another embodiment the invention provides a pharmaceutical composition wherein I is a valence bond.
In another embodiment the invention provides a pharmaceutical composition wherein J is

- hydrogen,
- $C_1$-$C_6$-alkyl, $C_2$-$C_6$-alkenyl or $C_2$-$C_6$-alkynyl,
  which may optionally be substituted with one or more substituents selected from halogen, -CN, $-CF_3$, $-OCF_3$, $-OR^{35}$, and $-NR^{35}R^{36}$,
- aryl, or heteroaryl, wherein the cyclic moieties are optionally substituted with one or more substituents independently selected from $R^{37}$.

In another embodiment the invention provides a pharmaceutical composition wherein J is

- hydrogen,
- aryl or heteroaryl, wherein the cyclic moieties are optionally substituted with one or more substituents independently selected from $R^{37}$. In another embodiment the invention provides a pharmaceutical composition wherein J is
- hydrogen,
- ArG1 or Het3, wherein the cyclic moieties are optionally substituted with one or more substituents independently selected from $R^{37}$.

In another embodiment the invention provides a pharmaceutical composition wherein J is

- hydrogen,
- phenyl or naphthyl optionally substituted with one or more substituents independently selected from $R^{37}$.

In another embodiment the invention provides a pharmaceutical composition wherein J is hydrogen.

In another embodiment the invention provides a pharmaceutical composition wherein $R^{32}$ and $R^{33}$ are independently selected from hydrogen or $C_1$-$C_6$-alkyl.

In another embodiment the invention provides a pharmaceutical composition wherein $R^{34}$ is hydrogen, halogen, -CN, -$CF_3$, -$OCF_3$, -$SCF_3$, -$NO_2$, -$OR^{35}$, -C(O)$R^{35}$, -$NR^{35}R^{36}$, -$SR^{35}$, -C(O)$NR^{35}R^{36}$, -OC(O)$NR^{35}R^{36}$, -$NR^{35}$C(O)$R^{36}$, -OC(O)$R^{35}$, -$OC_1$-$C_6$-alkyl-C(O)$OR^{35}$, -$SC_1$-$C_6$-alkyl-C(O)$OR^{35}$ or -C(O)$OR^{35}$.

In another embodiment the invention provides a pharmaceutical composition wherein $R^{34}$ is hydrogen, halogen, -$CF_3$, -$NO_2$, -$OR^{35}$, -$NR^{35}R^{36}$, -$SR^{35}$, -$NR^{35}$C(O)$R^{36}$, or -C(O)$OR^{35}$.

In another embodiment the invention provides a pharmaceutical composition wherein $R^{34}$ is hydrogen, halogen, -$CF_3$, -$NO_2$, -$OR^{35}$, -$NR^{35}R^{36}$, or -$NR^{35}$C(O)$R^{36}$.

In another embodiment the invention provides a pharmaceutical composition wherein $R^{34}$ is hydrogen, halogen, or -$OR^{35}$.

In another embodiment the invention provides a pharmaceutical composition wherein $R^{35}$ and $R^{36}$ are independently selected from hydrogen, $C_1$-$C_6$-alkyl, or aryl.

In another embodiment the invention provides a pharmaceutical composition wherein $R^{35}$ and $R^{36}$ are independently selected from hydrogen or $C_1$-$C_6$-alkyl.

In another embodiment the invention provides a pharmaceutical composition wherein $R^{37}$ is halogen, -C(O)$OR^{35}$, -CN, -$CF_3$, -$OR^{35}$, -$NR^{35}R^{36}$, $C_1$-$C_6$-alkyl or $C_1$-$C_6$-alkanoyl.

In another embodiment the invention provides a pharmaceutical composition wherein $R^{37}$ is halogen, -C(O)$OR^{35}$, -$OR^{35}$, -$NR^{35}R^{36}$, $C_1$-$C_6$-alkyl or $C_1$-$C_6$-alkanoyl.

In another embodiment the invention provides a pharmaceutical composition wherein $R^{37}$ is halogen, -C(O)$OR^{35}$ or -$OR^{35}$.

In another embodiment the invention provides a pharmaceutical composition wherein the zinc-binding ligand is

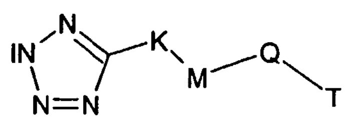

wherein K is a valence bond, $C_1$-$C_6$-alkylene, -NH-C(=O)-U-, -$C_1$-$C_6$-alkyl-S-, -$C_1$-$C_6$-alkyl-O-, -C(=O)-, or -C(=O)-NH-, wherein any $C_1$-$C_6$-alkyl moiety is optionally substituted with $R^{38}$,

U is a valence bond, $C_1$-$C_6$-alkenylene, -$C_1$-$C_6$-alkyl-O- or $C_1$-$C_6$-alkylene wherein any $C_1$-$C_6$-alkyl moiety is optionally substituted with $C_1$-$C_6$-alkyl,

$R^{38}$ is $C_1$-$C_6$-alkyl, aryl, wherein the alkyl or aryl moieties are optionally substituted with one or more substituents independently selected from $R^{39}$,

$R^{39}$ is independently selected from halogen, cyano, nitro, amino,

M is a valence bond, arylene or heteroarylene, wherein the aryl or heteroaryl moieties are optionally substituted with one or more substituents independently selected from $R^{40}$,

$R^{40}$ is selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -$S(O)_2CF_3$, -$OS(O)_2CF_3$, -$SCF_3$, -$N0_2$, -$OR^{41}$, -$NR^{41}R^{42}$, -$SR^{41}$, -$NR^{41}S(O)_2R^{42}$, -$S(O)_2NR^{41}R^{42}$, -S(O)$NR^{41}R^{42}$, -S(O)$R^{41}$, -$S(O)_2R^{41}$, -$OS(O)_2$ $R^{41}$, -C(O)$NR^{41}R^{42}$, -OC(O)$NR^{41}R^{42}$, -$NR^{41}$C(O)$R^{42}$, -$CH_2$C(O)$NR^{41}R^{42}$, -$OC_1$-$C_6$-alkyl-C(O)$NR^{41}R^{42}$, -$CH_2OR^{41}$, -$CH_2$OC(O)$R^{41}$, -$CH_2NR^{41}R^{42}$, -OC(O)$R^{41}$, -$OC_1$-$C_6$-alkyl-C(O)$OR^{41}$, -$OC_1$-$C_6$-alkyl-$OR^{41}$, -S-$C_1$-$C_6$-alkyl-C(O)$OR^{41}$, -$C_2$-$C_6$-alkenyl-C(=O)$OR^{41}$, -$NR^{41}$-C(=O)-$C_1$-$C_6$-alkyl-C(=O)$OR^{41}$, -$NR^{41}$-C(=O)-$C_1$-$C_6$-alkenyl-C(=O)$OR^{41}$, -C(O)$OR^{41}$, -$C_2$-$C_6$-alkenyl-C(=O)$R^{41}$, =O, -NH-C(=O)-O-$C_1$-$C_6$-alkyl, or -NH-C(=O)-C(=O)-O-$C_1$-$C_6$-alkyl,
- $C_1$-$C_6$-alkyl, $C_2$-$C_6$-alkenyl or $C_2$-$C_6$-alkynyl, which may each optionally be substituted with one or more substituents selected from $R^{43}$,
- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-

$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl, wherein the cyclic moieties optionally may be substituted with one or more substituents selected from $R^{44}$,

$R^{41}$ and $R^{42}$ are independently selected from hydrogen, -OH, $C_1$-$C_6$-alkyl, $C_1$-$C_6$-alkenyl, aryl-$C_1$-$C_6$-alkyl or aryl, wherein the alkyl moieties may optionally be substituted with one or more substituents independently selected from $R^{45}$, and the aryl moieties may optionally be substituted with one or more substituents independently selected from $R^{46}$; $R^{41}$ and $R^{42}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{43}$ is independently selected from halogen, -CN, $-CF_3$, $-OCF_3$, $-OR^{41}$, and $-NR^{41}R^{42}$

$R^{44}$ is independently selected from halogen, $-C(O)OR^{41}$, $-CH_2C(O)OR^{41}$, $-CH_2OR^{41}$, -CN, - $CF_3$, $-OCF_3$, $-NO_2$, $-OR^{41}$, $-NR^{41}R^{42}$ and $C_1$-$C_6$-alkyl,

$R^{45}$ is independently selected from halogen, -CN, $-CF_3$, $-OCF_3$, -O-$C_1$-$C_6$-alkyl, -C(O)-O-$C_1$-$C_6$-alkyl, -COOH and $-NH_2$,

$R^{46}$ is independently selected from halogen, -C(O)O$C_1$-$C_6$-alkyl, -COOH, -CN, $-CF_3$, $-OCF_3$, - $NO_2$, -OH, -O$C_1$-$C_6$-alkyl, $-NH_2$, C(=O) or $C_1$-$C_6$-alkyl,

Q is a valence bond, $C_1$-$C_6$-alkylene, -$C_1$-$C_6$-alkyl-O-, -$C_1$-$C_6$-alkyl-NH-, -NH-$C_1$-$C_6$-alkyl, -NH-C(=O)-, -C(=O)-NH-, -O-$C_1$-$C_6$-alkyl, -C(=O)-, or -$C_1$-$C_6$-alkyl-C(=O)-N($R^{47}$)- wherein the alkyl moieties are optionally substituted with one or more substituents independently selected from $R^{48}$,

$R^{47}$ and $R^{48}$ are independently selected from hydrogen, $C_1$-$C_6$-alkyl, aryl optionally substituted with one or more $R^{49}$,

$R^{49}$ is independently selected from halogen and -COOH,

T is

- hydrogen,
- $C_1$-$C_6$-alkyl, $C_2$-$C_6$-alkenyl , $C_2$-$C_6$-alkynyl, $C_1$-$C_6$-alkyloxy- carbonyl, wherein the alkyl, alkenyl and alkynyl moieties are optionally substituted with one or more substituents independently selected from $R^{50}$,
- aryl, aryloxy, aryloxy-carbonyl, aryl-$C_1$-$C_6$-alkyl, aroyl, aryl-$C_1$-$C_6$-alkoxy, aryl-$C_2$-$C_6$-alkenyl, aryl-$C_2$-$C_6$-alkyny-, heteroaryl, heteroaryl-$C_1$-$C_6$-alkyl, heteroaryl-$C_2$-$C_6$-alkenyl, heteroaryl-$C_2$-$C_6$-alkynyl,

wherein any alkyl, alkenyl , alkynyl, aryl and heteroaryl moiety is optionally substituted with one or more substituents independently selected from $R^{50}$,

$R^{50}$ is $C_1$-$C_6$-alkyl, $C_1$-$C_6$-alkoxy, aryl, aryloxy, aryl-$C_1$-$C_6$-alkoxy, -C(=O)-NH-$C_1$-$C_6$-alkyl-aryl, heteroaryl, heteroaryl-$C_1$-$C_6$-alkoxy, -$C_1$-$C_6$-alkyl-COOH, -O-$C_1$-$C_6$-alkyl-COOH, $-S(O)_2R^{51}$, -$C_2$-$C_6$-alkenyl-COOH, $-OR^{51}$, $-NO_2$, halogen, -COOH, $-CF_3$, -CN, =O, $-N(R^{51}R^{52})$, wherein the aryl or heteroaryl moieties are optionally substituted with one or more $R^{53}$,

$R^{51}$ and $R^{52}$ are independently selected from hydrogen and $C_1$-$C_6$-alkyl,

$R^{53}$ is independently selected from $C_1$-$C_6$-alkyl, $C_1$-$C_6$-alkoxy, -$C_1$-$C_6$-alkyl-COOH, -$C_2$-$C_6$-alkenyl-COOH, $-OR^{51}$, $-NO_2$, halogen, -COOH, $-CF_3$, -CN, or $-N(R^{51}R^{52})$,

or any enantiomer, diastereomer, including a racemic mixture, tautomer as well as a salt thereof with a pharmaceutically acceptable acid or base.

In another embodiment the invention provides a pharmaceutical composition wherein K is a valence bond, $C_1$-$C_6$-alkylene, -NH-C(=O)-U-, -$C_1$-$C_6$-alkyl-S-, -$C_1$-$C_6$-alkyl-O-, or -C(=O)-, wherein any $C_1$-$C_6$-alkyl moiety is optionally substituted with $R^{38}$.

In another embodiment the invention provides a pharmaceutical composition wherein K is a valence bond, $C_1$-$C_6$-alkylene, -NH-C(=O)-U-, -$C_1$-$C_6$-alkyl-S-, or -$C_1$-$C_6$-alkyl-O, wherein any $C_1$-$C_6$-alkyl moiety is optionally substi-

tuted with $R^{38}$.

In another embodiment the invention provides a pharmaceutical composition wherein K is a valence bond, $C_1$-$C_6$-alkylene, or -NH-C(=O)-U, wherein any $C_1$-$C_6$-alkyl moiety is optionally substituted with $R^{38}$.

In another embodiment the invention provides a pharmaceutical composition wherein K is a valence bond or $C_1$-$C_6$-alkylene, wherein any $C_1$-$C_6$-alkyl moiety is optionally substituted with $R^{38}$.

In another embodiment the invention provides a pharmaceutical composition wherein K is a valence bond or -NH-C(=O)-U.

In another embodiment the invention provides a pharmaceutical composition wherein K is a valence bond.

In another embodiment the invention provides a pharmaceutical composition wherein U is a valence bond or -$C_1$-$C_6$-alkyl-O-.

In another embodiment the invention provides a pharmaceutical composition wherein U is a valence bond.

In another embodiment the invention provides a pharmaceutical composition wherein M is arylene or heteroarylene, wherein the arylene or heteroarylene moieties are optionally substituted with one or more substituents independently selected from $R^{40}$.

In another embodiment the invention provides a pharmaceutical composition wherein M is ArG1 or Het1, wherein the arylene or heteroarylene moieties are optionally substituted with one or more substituents independently selected from $R^{40}$.

In another embodiment the invention provides a pharmaceutical composition wherein M is ArG1 or Het2, wherein the arylene or heteroarylene moieties are optionally substituted with one or more substituents independently selected from $R^{40}$.

In another embodiment the invention provides a pharmaceutical composition wherein M is ArG1 or Het3, wherein the arylene or heteroarylene moieties are optionally substituted with one or more substituents independently selected from $R^{40}$.

In another embodiment the invention provides a pharmaceutical composition wherein M is phenylene optionally substituted with one or more substituents independently selected from $R^{40}$.

In another embodiment the invention provides a pharmaceutical composition wherein M is indolylene optionally substituted with one or more substituents independently selected from $R^{40}$.

In another embodiment the invention provides a pharmaceutical composition wherein M is

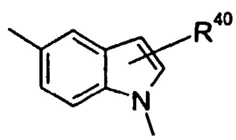

In another embodiment the invention provides a pharmaceutical composition wherein M is carbazolylene optionally substituted with one or more substituents independently selected from $R^{40}$.

In another embodiment the invention provides a pharmaceutical composition wherein M is

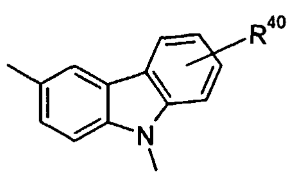

In another embodiment the invention provides a pharmaceutical composition wherein $R^{40}$ is selected from

- hydrogen, halogen, -CN, -$CF_3$, -$OCF_3$, -$NO_2$, -$OR^{41}$, -$NR^{41}R^{42}$, -$SR^{41}$, -$S(O)_2R^{41}$, -$NR^{41}C(O)R^{42}$, -$OC_1$-$C_6$-alkyl-C(O)$NR^{41}R^{42}$, -$C_2$-$C_6$-alkenyl-C(=O)$OR^{41}$, -C(O)$OR^{41}$, =O, -NH-C(=O)-O-$C_1$-$C_6$-alkyl, or -NH-C(=O)-C(=O)-O-$C_1$-$C_6$-alkyl,
  $C_1$-$C_6$-alkyl or $C_2$-$C_6$- alkenyl which may each optionally be substituted with one or more substituents independently selected from $R^{43}$,

- aryl, aryloxy, aryl-$C_1$-$C_6$-alkoxy, aryl-$C_1$-$C_6$-alkyl, aryl-$C_2$-$C_6$-alkenyl, heteroaryl, heteroaryl-$C_1$-$C_6$-alkyl, or heteroaryl-$C_2$-$C_6$-alkenyl, wherein the cyclic moieties optionally may be substituted with one or more substituents selected

from $R^{44}$.

In another embodiment the invention provides a pharmaceutical composition wherein $R^{40}$ is selected from

- hydrogen, halogen, -CN, $-CF_3$, $-OCF_3$, $-NO_2$, $-OR^{41}$, $-NR^{41}R^{42}$, $-SR^{41}$, $-S(O)_2R^{41}$, $-NR^{41}C(O)R^{42}$, $-OC_1$-$C_6$-alkyl-$C(O)NR^{41}R^{42}$, $-C_2$-$C_6$-alkenyl-$C(=O)OR^{41}$, $-C(O)OR^{41}$, =O, -NH-C(=O)-O-$C_1$-$C_6$-alkyl, or -NH-C(=O)-C(=O)-O-$C_1$-$C_6$-alkyl,
  $C_1$-$C_6$-alkyl or $C_2$-$C_6$- alkenyl which may each optionally be substituted with one or more substituents independently selected from $R^{43}$,
- ArG1, ArG1-O-, ArG1-$C_1$-$C_6$-alkoxy, ArG1-$C_1$-$C_6$-alkyl, ArG1-$C_2$-$C_6$-alkenyl, Het3, Het3-$C_1$-$C_6$-alkyl, or Het3-$C_2$-$C_6$-alkenyl, wherein the cyclic moieties optionally may be substituted with one or more substituents selected from $R^{44}$.

In another embodiment the invention provides a pharmaceutical composition wherein $R^{40}$ is selected from

- hydrogen, halogen, $-CF_3$, $-NO_2$, $-OR^{41}$, $-NR^{41}R^{42}$, $-C(O)OR^{41}$, =O, or $-NR^{41}C(O)R^{42}$,
- $C_1$-$C_6$-alkyl,
- ArG1.

In another embodiment the invention provides a pharmaceutical composition wherein $R^{40}$ is selected from

- Halogen, $-NO_2$, $-OR^{41}$, $-NR^{41}R^{42}$, $-C(O)OR^{41}$, or $-NR^{41}C(O)R^{42}$,
- Methyl,
- Phenyl.

In another embodiment the invention provides a pharmaceutical composition wherein $R^{41}$ and $R^{42}$ are independently selected from hydrogen, $C_1$-$C_6$-alkyl, or aryl, wherein the aryl moieties may optionally be substituted with halogen or -COOH.

In another embodiment the invention provides a pharmaceutical composition wherein $R^{41}$ and $R^{42}$ are independently selected from hydrogen, methyl, ethyl, or phenyl, wherein the phenyl moieties may optionally be substituted with halogen or -COOH.

In another embodiment the invention provides a pharmaceutical composition wherein Q is a valence bond, $C_1$-$C_6$-alkylene, $-C_1$-$C_6$-alkyl-O-, $-C_1$-$C_6$-alkyl-NH-, -NH-$C_1$-$C_6$-alkyl, -NH-C(=O)-, -C(=O)-NH-, -O-$C_1$-$C_6$-alkyl, -C(=O)-, or $-C_1$-$C_6$-alkyl-C(=O)-N($R^{47}$)- wherein the alkyl moieties are optionally substituted with one or more substituents independently selected from $R^{48}$.

In another embodiment the invention provides a pharmaceutical composition wherein Q is a valence bond, $-CH_2$-, $-CH_2$-$CH_2$-, $-CH_2$-O-, $-CH_2$-$CH_2$-O-, $-CH_2$-NH-, $-CH_2$-$CH_2$-NH-, -NH-$CH_2$-, -NH-$CH_2$-$CH_2$-, -NH-C(=O)-, -C(=O)-NH-, -O-$CH_2$-, -O-$CH_2$-$CH_2$-, or -C(=O)-.

In another embodiment the invention provides a pharmaceutical composition wherein $R^{47}$ and $R^{48}$ are independently selected from hydrogen, methyl and phenyl.

In another embodiment the invention provides a pharmaceutical composition wherein T is

- hydrogen,
- $C_1$-$C_6$-alkyl optionally substituted with one or more substituents independently selected from $R^{50}$,
- aryl, aryl-$C_1$-$C_6$-alkyl, heteroaryl, wherein the alkyl, aryl and heteroaryl moieties are optionally substituted with one or more substituents independently selected from $R^{50}$.

In another embodiment the invention provides a pharmaceutical composition wherein T is

- hydrogen,
- $C_1$-$C_6$-alkyl optionally substituted with one or more substituents independently selected from $R^{50}$,
- ArG1, ArG1-$C_1$-$C_6$-alkyl, Het3, wherein the alkyl, aryl and heteroaryl moieties are optionally substituted with one or more substituents independently selected from $R^{50}$.

In another embodiment the invention provides a pharmaceutical composition wherein T is

- hydrogen,

- $C_1$-$C_6$-alkyl, optionally substituted with one or more substituents independently selected from $R^{50}$,
- phenyl, phenyl-$C_1$-$C_6$-alkyl, wherein the alkyl and phenyl moieties are optionally substituted with one or more substituents independently selected from $R^{50}$.

In another embodiment the invention provides a pharmaceutical composition wherein $R^{50}$ is $C_1$-$C_6$-alkyl, $C_1$-$C_6$-alkoxy, aryl, aryloxy, aryl-$C_1$-$C_6$-alkoxy, -C(=O)-NH-$C_1$-$C_6$-alkyl-aryl, heteroaryl, -$C_1$-$C_6$-alkyl-COOH, -O-$C_1$-$C_6$-alkyl-COOH, -S(O)$_2$$R^{51}$, -$C_2$-$C_6$-alkenyl-COOH, -O$R^{51}$, -$NO_2$, halogen, -COOH, -$CF_3$, -CN, =O, -N($R^{51}R^{52}$), wherein the aryl or heteroaryl moieties are optionally substituted with one or more $R^{53}$.
In another embodiment the invention provides a pharmaceutical composition wherein $R^{50}$ is $C_1$-$C_6$-alkyl, $C_1$-$C_6$-alkoxy, aryl, aryloxy, aryl-$C_1$-$C_6$-alkoxy , -O$R^{51}$, -$NO_2$, halogen, -COOH, -$CF_3$, wherein any aryl moiety is optionally substituted with one or more $R^{53}$.
In another embodiment the invention provides a pharmaceutical composition wherein $R^{50}$ is $C_1$-$C_6$-alkyl, aryloxy, aryl-$C_1$-$C_6$-alkoxy, -O$R^{51}$, halogen, -COOH, -$CF_3$, wherein any aryl moiety is optionally substituted with one or more $R^{53}$.
In another embodiment the invention provides a pharmaceutical composition wherein $R^{50}$ is $C_1$-$C_6$-alkyl, ArG1-O-, ArG1-$C_1$-$C_6$-alkoxy, -O$R^{51}$, halogen, -COOH, -$CF_3$, wherein any aryl moiety is optionally substituted with one or more $R^{53}$.
In another embodiment the invention provides a pharmaceutical composition wherein $R^{50}$ is phenyl, methyl or ethyl.
In another embodiment the invention provides a pharmaceutical composition wherein $R^{50}$ is methyl or ethyl.
In another embodiment the invention provides a pharmaceutical composition wherein $R^{51}$ is methyl.
In another embodiment the invention provides a pharmaceutical composition wherein $R^{53}$ is $C_1$-$C_6$-alkyl, $C_1$-$C_6$-alkoxy, -O$R^{51}$, halogen,or -$CF_3$.
In another embodiment the invention provides a pharmaceutical composition wherein the zinc-binding ligand is

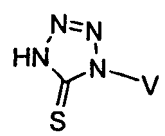

wherein V is $C_1$-$C_6$-alkyl, aryl, heteroaryl, aryl-$C_{1-6}$-alkyl- or aryl-$C_{2-6}$-alkenyl-, wherein the alkyl or alkenyl is optionally substituted with one or more substituents independently selected from $R^{54}$, and the aryl or heteroaryl is optionally substituted with one or more substituents independently selected from $R^{55}$,

$R^{54}$ is independently selected from halogen, -CN, -$CF_3$, -$OCF_3$, aryl, -COOH and -$NH_2$,

$R^{55}$ is independently selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -S(O)$_2$$CF_3$, -OS(O)$_2$$CF_3$, -$SCF_3$, -$NO_2$, -O$R^{56}$, -N$R^{56}R^{57}$, -S$R^{56}$, -N$R^{56}$S(O)$_2$$R^{57}$, -S(O)$_2$N$R^{56}R^{57}$, -S(O)N$R^{56}R^{57}$, -S(O)$R^{56}$, -S(O)$_2$$R^{56}$, -OS(O)$_2$ $R^{56}$, -C(O)N$R^{56}R^{57}$, -OC(O)N$R^{56}R^{57}$, -N$R^{56}$C(O)$R^{57}$, -$CH_2$C(O)N$R^{56}R^{57}$, -O$C_1$-$C_6$-alkyl-C(O)N$R^{56}R^{57}$, -$CH_2$O$R^{56}$, -$CH_2$OC(O)$R^{56}$, -$CH_2$N$R^{56}R^{57}$, -OC(O)$R^{56}$, -O$C_1$-$C_8$-alkyl-C(O)OR $^{56}$, -O$C_1$-$C_6$-alkyl-O$R^{56}$, -S$C_1$-$C_6$-alkyl-C(O)ORO$^{56}$, -$C_2$-$C_6$-alkenyl-C(=O)O$R^{56}$, -N$R^{56}$-C(=O)-$C_1$-$C_6$-alkyl-C(=O)O$R^{56}$, -N$R^{56}$-C(=O)-$C_1$-$C_6$-alkenyl-C(=O)O$R^{56}$ , -C(O)O$R^{56}$, or -$C_2$-$C_6$-alkenyl-C(=O)$R^{56}$,

- $C_1$-$C_6$-alkyl, $C_2$-$C_6$-alkenyl or $C_2$-$C_6$-alkynyl,
  which may optionally be substituted with one or more substituents selected from $R^{58}$,

- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{59}$,

$R^{56}$ and $R^{57}$ are independently selected from hydrogen, OH, $CF_3$, $C_1$-$C_{12}$-alkyl, aryl-$C_1$-$C_6$-alkyl, -C(=O)-$C_1$-$C_6$-alkyl or aryl, wherein the alkyl groups may optionally be substituted with one or more substituents independently selected from $R^{60}$, and the aryl groups may optionally be substituted with one or more substituents independently selected from $R^{61}$ ; $R^{56}$ and $R^{57}$ when attached to the same nitrogen atom may form a 3 to 8 membered heterocyclic ring with the said nitrogen atom, the heterocyclic ring optionally containing one or two further heteroatoms selected from

nitrogen, oxygen and sulphur, and optionally containing one or two double bonds,

$R^{58}$ is independently selected from halogen, -CN, $-CF_3$, $-OCF_3$, $-OR^{56}$, and $-NR^{56}R^{57}$,

$R^{59}$ is independently selected from halogen, $-C(O)OR^{56}$, $-CH_2C(O)OR^{56}$, $-CH_2OR^{56}$, -CN,$-CF_3$, $-OCF_3$, $-NO_2$, $-OR^{56}$, $-NR^{56}R^{57}$ and $C_1$-$C_6$-alkyl,

$R^{60}$ is independently selected from halogen, -CN, $-CF_3$, $-OCF_3$, $-OC_1$-$C_6$-alkyl, $-C(O)OC_1$-$C_6$-alkyl, $-C(=O)-R^{62}$, -COOH and $-NH_2$,

$R^{61}$ is independently selected from halogen, $-C(O)OC_1$-$C_6$-alkyl, -COOH, -CN, $-CF_3$, $-OCF_3$,$-NO_2$, -OH, $-OC_1$-$C_6$-alkyl, $-NH_2$, C(=O) or $C_1$-$C_6$-alkyl,

$R^{62}$ is $C_1$-$C_6$-alkyl, aryl optionally substituted with one or more substituents independently selected from halogen, or heteroaryl optionally substituted with one or more $C_1$-$C_6$-alkyl independently,

or any enantiomer, diastereomer, including a racemic mixture, tautomer as well as a salt thereof with a pharmaceutically acceptable acid or base.

In another embodiment the invention provides a pharmaceutical composition wherein V is aryl, heteroaryl, or aryl-$C_{1-6}$-alkyl-, wherein the alkyl is optionally substituted with one or more substituents independently selected $R^{54}$, and the aryl or heteroaryl is optionally substituted with one or more substituents independently selected from $R^{55}$.

In another embodiment the invention provides a pharmaceutical composition wherein V is aryl, Het1, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.

In another embodiment the invention provides a pharmaceutical composition wherein V is aryl, Het2, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.

In another embodiment the invention provides a pharmaceutical composition wherein V is aryl, Het3, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.

In another embodiment the invention provides a pharmaceutical composition wherein V is aryl optionally substituted with one or more substituents independently selected from $R^{55}$.

In another embodiment the invention provides a pharmaceutical composition wherein V is ArG1 optionally substituted with one or more substituents independently selected from $R^{55}$.

In another embodiment the invention provides a pharmaceutical composition wherein V is phenyl, naphthyl or anthranyl optionally substituted with one or more substituents independently selected from $R^{55}$.

In another embodiment the invention provides a pharmaceutical composition wherein V is phenyl optionally substituted with one or more substituents independently selected from $R^{55}$. In another embodiment the invention provides a pharmaceutical composition wherein $R^{55}$ is independently selected from

- halogen, $C_1$-$C_6$-alkyl, -CN, $-OCF_3$ ,$-CF_3$, $-NO_2$, $-OR^{56}$, $-NR^{56}R^{57}$, $-NR^{56}C(O)R^{57}$ $-SR^{56}$, $-OC_1$-$C_8$-alkyl-$C(O)OR^{56}$, or $-C(O)OR^{56}$,
- $C_1$-$C_6$-alkyl optionally substituted with one or more substituents independently selected from $R^{58}$
- aryl, aryl-$C_1$-$C_6$-alkyl, heteroaryl, or heteroaryl-$C_1$-$C_6$-alkyl

of which the cyclic moieties optionally may be substituted with one or more substituents independently selected from $R^{59}$.

In another embodiment the invention provides a pharmaceutical composition wherein $R^{55}$ is independently selected from

- halogen, $C_1$-$C_6$-alkyl, -CN, $-OCF_3$ ,$-CF_3$, $-NO_2$, $-OR^{56}$, $-NR^{56}R^{57}$, $-NR^{56}C(O)R^{57}$ $-SR^{56}$, $-OC_1$-$C_8$-alkyl-$C(O)OR^{56}$, or $-C(O)OR^{56}$
- $C_1$-$C_6$-alkyl optionally substituted with one or more substituents independently selected from $R^{58}$
- ArG1, ArG1-$C_1$-$C_6$-alkyl, Het3, or Het3-$C_1$-$C_6$-alkyl

of which the cyclic moieties optionally may be substituted with one or more substituents independently selected from $R^{59}$.

In another embodiment the invention provides a pharmaceutical composition wherein $R^{55}$ is independently selected from halogen, $-OR^{56}$, $-NR^{56}R^{57}$, $-C(O)OR^{56}$, $-OC_1$-$C_8$-alkyl-$C(O)OR^{56}$, $-NR^{56}C(O)R^{57}$ or $C_1$-$C_6$-alkyl.

In another embodiment the invention provides a pharmaceutical composition wherein $R^{55}$ is independently selected from halogen, $-OR^{56}$, $-NR^{56}R^{57}$, $-C(O)OR^{56}$, $-OC_1$-$C_8$-alkyl-$C(O)OR^{56}$, $-NR^{56}C(O)R^{57}$ , methyl or ethyl.

In another embodiment the invention provides a pharmaceutical composition wherein $R^{56}$ and $R^{57}$ are independently selected from hydrogen, $CF_3$, $C_1$-$C_{12}$-alkyl, or -C(=O)-$C_1$-$C_6$-alkyl; $R^{56}$ and $R^{57}$ when attached to the same nitrogen atom may form a 3 to 8 membered heterocyclic ring with the said nitrogen atom.
In another embodiment the invention provides a pharmaceutical composition wherein $R^{56}$ and $R^{57}$ are independently selected from hydrogen or $C_1$-$C_{12}$-alkyl, $R^{56}$ and $R^{57}$ when attached to the same nitrogen atom may form a 3 to 8 membered heterocyclic ring with the said nitrogen atom.
In another embodiment the invention provides a pharmaceutical composition wherein $R^{56}$ and $R^{57}$ are independently selected from hydrogen or methyl, ethyl, propyl butyl, $R^{56}$ and $R^{57}$ when attached to the same nitrogen atom may form a 3 to 8 membered heterocyclic ring with the said nitrogen atom.
In another embodiment the invention provides a pharmaceutical composition 1 wherein the zinc-binding ligand is

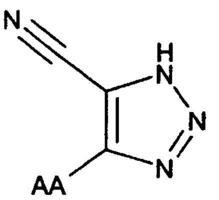

wherein AA is $C_1$-$C_6$-alkyl, aryl, heteroaryl, aryl-$C_{1-6}$-alkyl- or aryl-$C_{2-6}$-alkenyl-, wherein the alkyl or alkenyl is optionally substituted with one or more substituents independently selected from $R^{63}$, and the aryl or heteroaryl is optionally substituted with one or more substituents independently selected from $R^{64}$,

$R^{63}$ is independently selected from halogen, -CN, -$CF_3$, -$OCF_3$, aryl, -COOH and -$NH_2$,

$R^{64}$ is independently selected from

- hydrogen, halogen, -CN, -$CH_2CN$, -$CHF_2$, -$CF_3$, -$OCF_3$, -$OCHF_2$, -$OCH_2CF_3$, -$OCF_2CHF_2$, -$S(O)_2CF_3$, -OS$(O)_2CF_3$, -$SCF_3$, -$NO_2$, -$OR^{65}$, -$NR^{65}R^{66}$, -$SR^{65}$, -$NR^{65}S(O)_2R^{66}$, -$S(O)_2NR^{65}R^{66}$, -$S(O)NR^{65}R^{66}$, -$S(O)R^{65}$, -$S(O)_2R^{65}$, -$OS(O)_2$ $R^{65}$,- $C(O)NR^{65}R^{66}$, -$OC(O)NR^{65}R^{66}$, -$NR^{65}C(O)R^{66}$, $CH_2C(O)NR^{65}R^{66}$, -$OC_1$-$C_6$-alkyl-$C(O)NR^{65}R^{66}$, -$CH_2OR^{65}$, -$CH_2OC(O)R^{65}$, -$CH_2NR^{65}R^{66}$, -$OC(O)R^{65}$, -$OC_1$-$C_6$-alkyl-$C(O)OR^{65}$, -$OC_1$-$C_6$-alkyl-$OR^{65}$, -$SC_1$-$C_6$-alkyl-$C(O)OR^{65}$, -$C_2$-$C_6$-alkenyl-$C(=O)OR^{65}$, -$NR^{65}$-C(=O)-$C_1$-$C_6$-alkyl-C(=O)$OR^{61}$, -$NR^{65}$-C(=O)-$C_1$-$C_6$-alkenyl-$C(=O)OR^{65}$, -$C(O)OR^{65}$, or -$C_2$-$C_6$-alkenyl- C (=O) $R^{65}$,
- $C_1$-$C_6$-alkyl, $C_2$-$C_6$-alkenyl or $C_2$-$C_6$-alkynyl, each of which may optionally be substituted with one or more substituents selected from $R^{67}$,
- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{68}$,

$R^{65}$ and $R^{66}$ are independently selected from hydrogen, OH, $CF_3$, $C_1$-$C_{12}$-alkyl, aryl-$C_1$-$C_6$-alkyl, -C (=O)-$R^{69}$, aryl or heteroaryl, wherein the alkyl groups may optionally be substituted with one or more substituents selected from $R^{70}$, and the aryl and heteroaryl groups may optionally be substituted with one or more substituents independently selected from $R^{71}$; $R^{65}$ and $R^{66}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{67}$ is independently selected from halogen, -CN, -$CF_3$, -$OCF_3$, -$OR^{65}$, and -$NR^{65}R^{66}$,

$R^{68}$ is independently selected from halogen, -$C(O)OR^{65}$, -$CH_2C(O)OR^{65}$, -$CH_2OR^{65}$, -CN,-$CF_3$, -$OCF_3$, -$NO_2$, -$OR^{65}$, -$NR^{65}R^{66}$ and $C_1$-$C_6$-alkyl,

$R^{69}$ is independently selected from $C_1$-$C_6$-alkyl, aryl optionally substituted with one or more halogen, or heteroaryl

optionally substituted with one or more $C_1$-$C_6$-alkyl,

$R^{70}$ is independently selected from halogen, -CN, -$CF_3$, -$OCF_3$, -$OC_1$-$C_6$-alkyl, -C(O)$OC_1$-$C_6$-alkyl, -COOH and -$NH_2$,

$R^{71}$ is independently selected from halogen, -C(O)$OC_1$-$C_6$-alkyl, -COOH, -CN, -$CF_3$, -$OCF_3$, - $NO_2$, -OH, -$OC_1$-$C_6$-alkyl, -$NH_2$, C(=O) or $C_1$-$C_6$-alkyl,

or any enantiomer, diastereomer, including a racemic mixture, tautomer as well as a salt thereof with a pharmaceutically acceptable acid or base.

In another embodiment the invention provides a pharmaceutical composition wherein AA is aryl, heteroaryl or aryl-$C_{1-6}$-alkyl-, wherein the alkyl is optionally substituted with one or more $R^{63}$, and the aryl or heteroaryl is optionally substituted with one or more substituents independently selected from $R^{64}$.

In another embodiment the invention provides a pharmaceutical composition wherein AA is aryl or heteroaryl optionally substituted with one or more substituents independently selected from $R^{64}$.

In another embodiment the invention provides a pharmaceutical composition wherein AA is ArG1 or Het1 optionally substituted with one or more substituents independently selected from $R^{64}$.

In another embodiment the invention provides a pharmaceutical composition wherein AA is ArG1 or Het2 optionally substituted with one or more substituents independently selected from $R^{64}$.

In another embodiment the invention provides a pharmaceutical composition wherein AA is ArG1 or Het3 optionally substituted with one or more substituents independently selected from $R^{64}$.

In another embodiment the invention provides a pharmaceutical composition wherein AA is phenyl, naphtyl, anthryl, carbazolyl, thienyl, pyridyl, or benzodioxyl optionally substituted with one or more substituents independently selected from $R^{64}$.

In another embodiment the invention provides a pharmaceutical composition wherein AA is phenyl or naphtyl optionally substituted with one or more substituents independently selected from $R^{64}$.

In another embodiment the invention provides a pharmaceutical composition wherein $R^{64}$ is independently selected from hydrogen, halogen, -$CF_3$, -$OCF_3$, -$OR^{65}$, -$NR^{65}R^{66}$, $C_1$-$C_6$-alkyl, -OC(O)$R^{65}$, -$OC_1$-$C_6$-alkyl-C(O)$OR^{85}$, aryl-$C_2$-$C_6$-alkenyl, aryloxy or aryl, wherein $C_1$-$C_6$-alkyl is optionally substituted with one or more substituents independently selected from $R^{67}$, and the cyclic moieties optionally are substituted with one or more substituents independently selected from $R^{68}$.

In another embodiment the invention provides a pharmaceutical composition wherein $R^{64}$ is independently selected from halogen, -$CF_3$, -$OCF_3$, -$OR^{65}$, -$NR^{65}R^{66}$, methyl, ethyl, propyl, -OC(O)$R^{65}$, -$OCH_2$-C(O)$OR^{65}$, -$OCH_2$-$CH_2$-C(O)$OR^{65}$, phenoxy optionally substituted with one or more substituents independently selected from $R^{68}$.

In another embodiment the invention provides a pharmaceutical composition wherein $R^{65}$ and $R^{66}$ are independently selected from hydrogen, $CF_3$, $C_1$-$C_{12}$-alkyl, aryl, or heteroaryl optionally substituted with one or more substituents independently selected from $R^{71}$.

In another embodiment the invention provides a pharmaceutical composition wherein $R^{65}$ and $R^{66}$ are independently hydrogen, $C_1$-$C_{12}$-alkyl, aryl, or heteroaryl optionally substituted with one or more substituents independently selected from $R^{71}$.

In another embodiment the invention provides a pharmaceutical composition wherein $R^{65}$ and $R^{66}$ are independently hydrogen, methyl, ethyl, propyl, butyl, 2,2-dimethyl-propyl, ArG1 or Het1 optionally substituted with one or more substituents independently selected from $R^{71}$.

In another embodiment the invention provides a pharmaceutical composition wherein $R^{65}$ and $R^{66}$ are independently hydrogen, methyl, ethyl, propyl, butyl, 2,2-dimethyl-propyl, ArG1 or Het2 optionally substituted with one or more substituents independently selected from $R^{71}$.

In another embodiment the invention provides a pharmaceutical composition wherein $R^{65}$ and $R^{66}$ are independently hydrogen, methyl, ethyl, propyl, butyl, 2,2-dimethyl-propyl, ArG1 or Het3 optionally substituted with one or more substituents independently selected from $R^{71}$.

In another embodiment the invention provides a pharmaceutical composition wherein $R^{65}$ and $R^{66}$ are independently hydrogen, methyl, ethyl, propyl, butyl, 2,2-dimethyl-propyl, phenyl, naphtyl, thiadiazolyl optionally substituted with one or more $R^{71}$ independently; or isoxazolyl optionally substituted with one or more substituents independently selected from $R^{71}$.

In another embodiment the invention provides a pharmaceutical composition wherein $R^{71}$ is halogen or $C_1$-$C_6$-alkyl.

In another embodiment the invention provides a pharmaceutical composition wherein $R^{71}$ is halogen or methyl.

In another aspect the invention provides a pharmaceutical composition comprising insulin and a zinc-binding ligand which reversibly binds to a $His^{B10}$ $Zn^{2+}$ site of an insulin hexamer, wherein the ligand is selected from the group consisting of benzotriazoles, 3-hydroxy 2-napthoic acids, salicylic acids, tetrazoles, thiazolidinediones, 5-mercaptotetrazoles, or 4-cyano-1,2,3-triazoles, or any enantiomer, diastereomer, including a racemic mixture, tautomer as well as a

salt thereof with a pharmaceutically acceptable acid or base.

In one embodiment hereof the zinc-binding ligand is

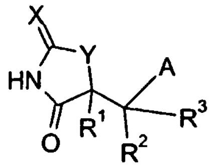 or 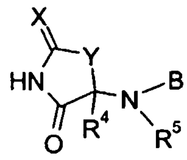 or 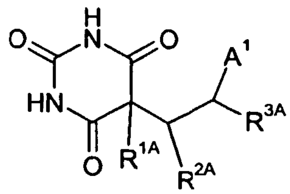

wherein

X is =O, =S or =NH

Y is -S-, -O- or -NH-

$R^1$, $R^{1A}$ and $R^4$ are independently selected from hydrogen or $C_1$-$C_6$-alkyl,

$R^2$ and $R^{2A}$ are hydrogen or $C_1$-$C_6$-alkyl or aryl, $R^1$ and $R^2$ may optionally be combined to form a double bond, $R^{1A}$ and $R^{2A}$ may optionally be combined to form a double bond,

$R^3$, $R^{3A}$ and $R^5$ are independently selected from hydrogen, halogen, aryl optionally substituted with one or more substituents independently selected from $R^{16}$, $C_1$-$C_6$-alkyl, or -C(O)$NR^{11}R^{12}$,

A, $A^1$ and B are independently selected from $C_1$-$C_6$-alkyl, aryl, aryl-$C_1$-$C_6$-alkyl, -$NR^{11}$-aryl, aryi-$C_2$-$C_6$-alkenyl or heteroaryl, wherein the alkyl or alkenyl is optionally substituted with one or more substituents independently selected from $R^6$ and the aryl or heteroaryl is optionally substituted with up to four substituents $R^7$, $R^8$, $R^9$, and $R^{10}$,

A and $R^3$ may be connected through one or two valence bonds, B and $R^5$ may be connected through one or two valence bonds,

$R^6$ is independently selected from halogen, -CN, -$CF_3$, -$OCF_3$, aryl, -COOH and -$NH_2$,

$R^7$, $R^8$, $R^9$ and $R^{10}$ are independently selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -S(O)$_2CF_3$, -OS(O)$_2CF_3$, -$SCF_3$, -$NO_2$, -$OR^{11}$, -$NR^{11}R^{12}$, -$SR^{11}$, -$NR^{11}$S(O)$_2R^{12}$, -S(O)$_2NR^{11}R^{12}$, -S(O)$NR^{11}R^{12}$, -S(O)$R^{11}$, -S(O)$_2R^{11}$, -OS(O)$_2$ $R^{11}$, -C(O)$NR^{11}R^{12}$, -OC(O)$NR^{11}R^{12}$, -$NR^{11}$C(O)$R^{12}$, -$CH_2$C(O)$NR^{11}R^{12}$, -O$C_1$-$C_6$-alkyl-C(O)$NR^{11}R^{12}$, -$CH_2OR^{11}$, -$CH_2$OC(O)$R^{11}$, -$CH_2NR^{11}R^{12}$, -OC(O)$R^{11}$, -O$C_1$-$C_{15}$-alkyl-C(O)$OR^{11}$, -O$C_1$-$C_6$-alkyl-$OR^{11}$, S$C_1$-$C_6$-alkyl-C(O)$OR^{11}$ -$C_2$-$C_6$-alkenyl-C(=O)$OR^{11}$, -$NR^{11}$-C(=O)-$C_1$-$C_6$-alkyl-C(=O)$OR^{11}$,- $NR^{11}$-C(=O)-$C_1$-$C_6$-alkenyl-C(=O)$OR^{11}$, -C(O)$OR^{11}$, C(O)$R^{11}$, or -$C_2$-$C_6$-alkenyl-C(=O)$R^{11}$, =O, or -$C_2$-$C_6$-alkenyl-C(=O)-$NR^{11}R^{12}$,
- $C_1$-$C_6$-alkyl, $C_2$-$C_6$-alkenyl or $C_2$-$C_6$-alkynyl, each of which may optionally be substituted with one or more substituents independently selected from $R^{13}$,
- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl, heteroaryl-$C_2$-$C_6$-alkynyl, or $C_3$-$C_6$ cycloalkyl,

of which each cyclic moiety may optionally be substituted with one or more substituents independently selected from $R^{14}$,

$R^{11}$ and $R^{12}$ are independently selected from hydrogen, OH, $C_1$-$C_{20}$-alkyl, aryl-$C_1$-$C_6$-alkyl or aryl, wherein the alkyl groups may optionally be substituted with one or more substituents independently selected from $R^{15}$, and the aryl groups may optionally be substituted one or more substituents independently selected from $R^{16}$; $R^{11}$ and $R^{12}$ when

attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{13}$ is independently selected from halogen, -CN, $-CF_3$, $-OCF_3$, $-OR^{11}$, $-C(O)OR^{11}$, $-NR^{11}R^{12}$, and $-C(O)NR^{11}R^{12}$,

$R^{14}$ is independently selected from halogen, $-C(O)OR^{11}$, $-CH_2C(O)OR^{11}$, $-CH_2OR^{11}$, -CN,$-CF_3$, $-OCF_3$, $-NO_2$, $-OR^{11}$, $-NR^{11}R^{12}$, $-NR^{11}C(O)R^{11}$, $-S(O)_2R^{11}$, aryl and $C_1$-$C_6$-alkyl,

$R^{15}$ is independently selected from halogen, -CN, $-CF_3$, =O, $-OCF_3$, $-OC_1$-$C_6$-alkyl, $-C(O)OC_1$-$C_6$-alkyl, -COOH and $-NH_2$,

$R^{16}$ is independently selected from halogen, $-C(O)OC_1$-$C_6$-alkyl, -COOH, -CN, $-CF_3$, $-OCF_3$,$-NO_2$, -OH, $-OC_1$-$C_6$-alkyl, $-NH_2$, C(=O) or $C_1$-$C_6$-alkyl, or any enantiomer, diastereomer, including a racemic mixture, tautomer as well as a salt thereof with a pharmaceutically acceptable acid or base.

In another embodiment hereof X is =O or =S.
In another embodiment hereof X is =O.
in another embodiment hereof X is =S.
in another embodiment hereof Y is -O- or -S-.
In another embodiment hereof Y is -O-.
In another embodiment hereof Y is -NH-.
In another embodiment hereof Y is -S-.
In another embodiment hereof A is aryl optionally substituted with up to four substituents, $R^7$, $R^8$, $R^9$, and $R^{10}$ which may be the same or different.
In another embodiment hereof A is selected from ArG1 optionally substituted with up to four substituents, $R^7$, $R^8$, $R^9$, and $R^{10}$ which may be the same or different.
In another embodiment hereof A is phenyl or naphtyl optionally substituted with up to four substituents, $R^7$, $R^8$, $R^9$, and $R^{10}$ which may be the same or different.
In another embodiment hereof A is

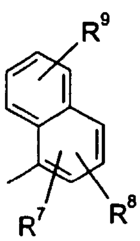

or

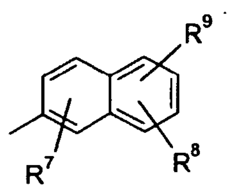

In another embodiment hereof A is phenyl.
In another embodiment hereof A is heteroaryl optionally substituted with up to four substituents, $R^7$, $R^8$, $R^9$, and $R^{10}$ which may be the same or different.
In another embodiment hereof A is selected from Het1 optionally substituted with up to four substituents, $R^7$, $R^8$, $R^9$, and $R^{10}$ which may be the same or different.
In another embodiment hereof A is selected from Het2 optionally substituted with up to four substituents, $R^7$, $R^8$, $R^9$, and $R^{10}$ which may be the same or different.
In another embodiment hereof A is selected from Het3 optionally substituted with up to four substituents, $R^7$, $R^8$, $R^9$, and $R^{10}$ which may be the same or different.
In another embodiment hereof A is selected from the group consisting of indolyl, benzofuranyl, quinolyl, furyl, thienyl, or pyrrolyl, wherein each heteroaryl may optionally substituted with up to four substituents, $R^7$, $R^8$, $R^9$, and $R^{10}$ which may be the same or different.
In another embodiment hereof A is benzofuranyl optionally substituted with up to four substituents $R^7$, $R^8$, $R^9$, and $R^{10}$ which may be the same or different.
In another embodiment hereof A is

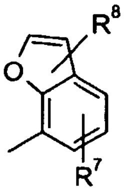 or 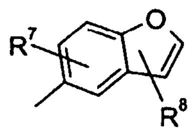 or 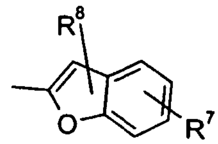

In another embodiment hereof A is carbazolyl optionally substituted with up to four substituents $R^7$, $R^8$, $R^9$, and $R^{10}$ which may be the same or different.

In another embodiment hereof A is

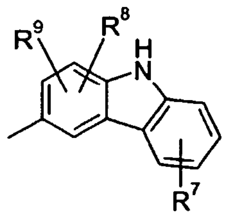

In another embodiment hereof A is quinolyl optionally substituted with up to four substituents $R^7$, $R^8$, $R^9$, and $R^{10}$ which may be the same or different.

In another embodiment hereof A is

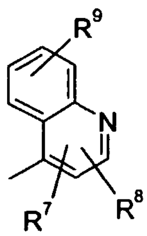 or 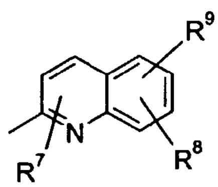

In another embodiment hereof A is indolyl optionally substituted with up to four substituents $R^7$, $R^8$, $R^9$, and $R^{10}$ which may be the same or different.

In another embodiment hereof A is

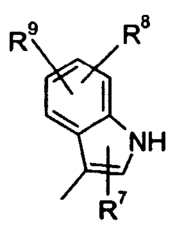 or 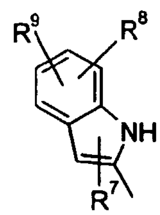

In another embodiment hereof $R^1$ is hydrogen.

In another embodiment hereof $R^2$ is hydrogen.

In another embodiment hereof $R^1$ and $R^2$ are combined to form a double bond.

In another embodiment hereof $R^3$ is $C_1$-$C_6$-alkyl, halogen, or $C(O)NR^{16}R^{17}$.

In another embodiment hereof $R^3$ is $C_1$-$C_6$-alkyl or $C(O)NR^{16}R^{17}$.

In another embodiment hereof $R^3$ is methyl.

In another embodiment hereof B is phenyl optionally substituted with up to four substituents, $R^7$, $R^8$, $R^9$, and $R^{10}$ which may be the same or different.

In another embodiment hereof $R^4$ is hydrogen.

In another embodiment hereof $R^5$ is hydrogen.
In another embodiment hereof $R^6$ is aryl.
In another embodiment hereof $R^6$ is phenyl.
In another embodiment hereof $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-NR^{11}S(O)_2R^{12}$, $-S(O)_2NR^{11}R^{12}$, $-S(O)NR^{11}R^{12}$, $-S(O)R^{11}$, $-S(O)_2R^{11}$, $-OS(O)_2 R^{11}$, $-NR^{11}C(O)R^{12}$, $-CH_2OR^{11}$, $- CH_2OC(O)R^{11}$, $-CH_2NR^{11}R^{12}$, $-OC(O)R^{11}$, $-OC_1$-$C_6$-alkyl-C(O)$OR^{11}$, $-OC_1$-$C_6$-alkyl-C(O)$NR^{11}R^{12}$, $-OC_1$-$C_6$-alkyl-$OR^{11}$, $-SC_1$-$C_6$-alkyl-C(O)$OR^{11}$, $-C_2$-$C_6$-alkenyl-C(=O)$OR^{11}$, -C(O)$OR^{11}$, or $-C_2$-$C_6$-alkenyl-C(=O)$R^{11}$,

- $C_1$-$C_6$-alkyl, $C_2$-$C_6$-alkenyl or $C_2$-$C_6$-alkynyl, which may each optionally be substituted with one or more substituents independently selected from $R^{13}$

- aryl, aryloxy, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, wherein each of the cyclic moieties optionally may be substituted with one or more substituents independently selected from $R^{14}$

In another embodiment hereof $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2 R^{11}$, $- CH_2OC(O)R^{11}$, $-OC(O)R^{11}$, $-OC_1$-$C_6$-alkyl-C(O)$OR^{11}$, $-OC_1$-$C_6$-alkyl-$OR^{11}$, $-SC_1$-$C_6$-alkyl-C(O)$OR^{11}$, -C(O)$OR^{11}$, or $-C_2$-$C_6$-alkenyl-C(=O)$R^{11}$,

- $C_1$-$C_6$-alkyl or $C_1$-$C_6$-alkenyl which may each optionally be substituted with one or more substituents independently selected from $R^{13}$

- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$

In another embodiment hereof $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2 R^{11}$, $- CH_2OC(O)R^{11}$, $-OC(O)R^{11}$, $-OC_1$-$C_6$-alkyl-C(O)$OR^{11}$, $-OC_1$-$C_6$-alkyl-$OR^{11}$, $-SC_1$-$C_6$-alkyl-C(O)$OR^{11}$, -C(O)$OR^{11}$, or $-C_2$-$C_6$-alkenyl-C(=O)$R^{11}$,

- $C_1$-$C_6$-alkyl or $C_1$-$C_6$- which may each optionally be substituted with one or more substituents independently selected from $R^{13}$

- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$.

In another embodiment hereof $R^7$, $R^8$, $R^9$ and $R^{10}$ are independently selected from

- hydrogen, halogen, $-OR^{11}$, $-OC_1$-$C_6$-alkyl-C(O)$OR^{11}$, or -C(O)$OR^{11}$,

- $C_1$-$C_6$-alkyl which may each optionally be substituted with one or more substituents independently selected from $R^{13}$

- aryl, aryloxy, aryl-$C_1$-$C_6$-alkoxy,

of which each of the cyclic moieties optionally may be substituted with one or more substituents independently selected from $R^{14}$.

In another embodiment hereof $R^7$, $R^8$, $R^9$ and $R^{10}$ are independently selected from

- hydrogen, halogen, $-OR^{11}$, $-OC_1$-$C_6$-alkyl-C(O)$OR^{11}$, or -C(O)$OR^{11}$,

- $C_1$-$C_6$-alkyl which may each optionally be substituted with one or more substituents independently selected from $R^{13}$

- ArG1, ArG1oxy, ArG1-$C_1$-$C_6$-alkoxy,

of which each of the cyclic moieties optionally may be substituted with one or more substituents independently selected from $R^{14}$.
In another embodiment hereof $R^7$, $R^8$, $R^9$ and $R^{10}$ are independently selected from

- hydrogen, halogen, $-OR^{11}$, $-OC_1-C_6$-alkyl-$C(O)OR^{11}$, or $-C(O)OR^{11}$,
- $C_1-C_6$-alkyl which may optionally be substituted with one or more substituents independently selected from $R^{13}$
- phenyl, phenyloxy, phenyl-$C_1-C_6$-alkoxy, wherein each of the cyclic moieties optionally may be substituted with one or more substituents independently selected from $R^{14}$.

In another embodiment hereof $R^{11}$ and $R^{12}$ are independently selected from hydrogen, $C_1-C_{20}$-alkyl, aryl or aryl-$C_1-C_6$-alkyl, wherein the alkyl groups may optionally be substituted with one or more substituents independently selected from $R^{15}$, and the aryl groups may optionally be substituted one or more substituents independently selected from $R^{16}$; $R^{11}$ and $R^{12}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds.
In another embodiment hereof $R^{11}$ and $R^{12}$ are independently selected from hydrogen, $C_1-C_{20}$-alkyl, aryl or aryl-$C_1-C_6$-alkyl, wherein the alkyl groups may optionally be substituted with one or more substituents independently selected from $R^{15}$, and the aryl groups may optionally be substituted one or more substituents independently selected from $R^{16}$.
In another embodiment hereof $R^{11}$ and $R^{12}$ are independently selected from phenyl or phenyl-$C_1-C_6$-alkyl.
In another embodiment hereof one or both of $R^{11}$ and $R^{12}$ are methyl.
In another embodiment hereof $R^{13}$ is independently selected from halogen, $CF_3$, $OR^{11}$ or $NR^{11}R^{12}$.
In another embodiment hereof $R^{13}$ is independently selected from halogen or $OR^{11}$.
In another embodiment hereof $R^{13}$ is $OR^{11}$.
In another embodiment hereof $R^{14}$ is independently selected from halogen, $-C(O)OR^{11}$, -CN,-$CF_3$, $-OR^{11}$, $S(O)_2R^{11}$, and $C_1-C_6$-alkyl.
In another embodiment hereof $R^{14}$ is independently selected from halogen, $-C(O)OR^{11}$, or-$OR^{11}$.
In another embodiment hereof $R^{15}$ is independently selected from halogen, -CN, -$CF_3$,-$C(O)OC_1-C_6$-alkyl,and -COOH.
in another embodiment hereof $R^{15}$ is independently selected from halogen or -$C(O)OC_1-C_6$-alkyl.
In another embodiment hereof $R^{16}$ is independently selected from halogen, -$C(O)OC_1-C_6$-alkyl, -COOH, $-NO_2$, $-OC_1-C_6$-alkyl, $-NH_2$, C(=O) or $C_1-C_6$-alkyl.
In another embodiment hereof $R^{16}$ is independently selected from halogen, -$C(O)OC_1-C_6$-alkyl, -COOH, $-NO_2$, or $C_1-C_6$-alkyl.
In another embodiment hereof the zinc-binding ligand is

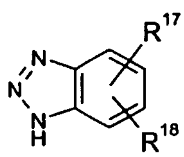 or 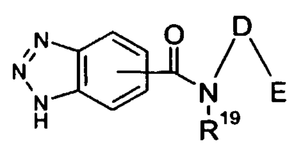 or 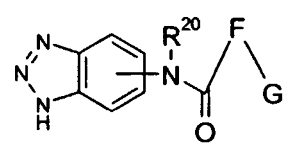

or 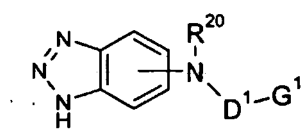

wherein

$R^{19}$ is hydrogen or $C_1-C_6$-alkyl,

$R^{20}$ is hydrogen or $C_1-C_6$-alkyl,

D, $D^1$ and F are a valence bond, $C_1-C_6$-alkylene or $C_1-C_6$-alkenylene optionally substituted with one or more substituents independently selected from $R^{72}$,

$R^{72}$ is independently selected from hydroxy, $C_1$-$C_6$-alkyl, or aryl,

E is $C_1$-$C_6$-alkyl, aryl or heteroaryl, wherein the aryl or heteroaryl is optionally substituted with up to three substituents $R^{21}$, $R^{22}$ and $R^{23}$,

G and $G^1$ are $C_1$-$C_6$-alkyl, aryl or heteroaryl, wherein the aryl or heteroaryl is optionally substituted with up to three substituents $R^{24}$, $R^{25}$ and $R^{26}$,

$R^{17}$, $R^{18}$, $R^{21}$, $R^{22}$, $R^{23}$, $R^{24}$, $R^{25}$ and $R^{26}$ are independently selected from

- hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, =O, $-OR^{27}$, $-NR^{27}R^{28}$, $-SR^{27}$, $-NR^{27}S(O)_2R^{28}$,- $S(O)_2NR^{27}R^{28}$, $-S(O)NR^{27}R^{28}$, $-S(O)R^{27}$, $-S(O)_2R^{27}$, $-C(O)NR^{27}R^{28}$, $-OC(O)NR^{27}R^{28}$, $-NR^{27}C(O)R^{28}$, $-NR^{27}C(O)OR^{28}$, $-CH_2C(O)NR^{27}R^{28}$, $-OCH_2C(O)NR^{27}R^{28}$, $-CH_2OR^{27}$, $-CH_2NR^{27}R^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-NR^{27}$-C(=O)-$C_1$-$C_6$-alkyl-$C(=O)OR^{27}$, $-NR^{27}$-C(=O)-$C_1$-$C_6$-alkenyl-$C(=O)OR^{27}$, -C(=O)$NR^{27}$-$C_1$-$C_6$-alkyl-$C(=O)OR^{27}$, $-C_1$-$C_6$-alkyl-$C(=O)OR^{27}$ or $-C(O)OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$,

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$,

$R^{27}$ and $R^{28}$ are independently selected from hydrogen, $C_1$-$C_6$-alkyl, aryl-$C_1$-$C_6$-alkyl or aryl, or $R^{27}$ and $R^{28}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{29}$ is independently selected from halogen, -CN, $-CF_3$, $-OCF_3$, $-OR^{27}$, and $-NR^{27}R^{28}$,

$R^{30}$ is independently selected from halogen, $-C(O)OR^{27}$, -CN, $-CF_3$, $-OCF_3$, $-NO_2$, $-OR^{27}$, $-NR^{27}R^{28}$ and $C_1$-$C_6$-alkyl, or any enantiomer, diastereomer, including a racemic mixture, tautomer as well as a salt thereof with a pharmaceutically acceptable acid or base.

In another embodiment hereof D is a valence bond.
In another embodiment hereof D is $C_1$-$C_6$-alkylene optionally substituted with one or more hydroxy, $C_1$-$C_6$-alkyl, or aryl.
In another embodiment hereof E is aryl or heteroaryl, wherein the aryl or heteroaryl is optionally substituted with up to three substituents independently selected from $R^{21}$, $R^{22}$ and $R^{23}$.
In another embodiment hereof E is aryl optionally substituted with up to three substituents independently selected from $R^{21}$, $R^{22}$ and $R^{23}$.
In another embodiment hereof E is selected from ArG1 and optionally substituted with up to three substituents independently selected from $R^{21}$, $R^{22}$ and $R^{23}$.
In another embodiment hereof E is phenyl optionally substituted with up to three substituents independently selected from $R^{21}$, $R^{22}$ and $R^{23}$.
In another embodiment hereof the zinc-binding ligand is

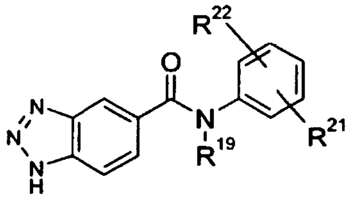

In another embodiment hereof $R^{21}$, $R^{22}$ and $R^{23}$ are independently selected from

- hydrogen, halogen, $-CHF_2$, $-CF_3$, $-OCF_3$, $-OCHF_2$, $-OCH_2CF_3$, $-OCF_2CHF_2$, $-SCF_3$, - $N0_2$, $-OR^{27}$, $-NR^{27}R^{28}$, $-SR^{27}$, $-C(O)NR^{27}R^{28}$, $-OC(O)NR^{27}R^{28}$, $-NR^{27}C(O)R^{28}$, $-NR^{27}C(O)OR^{28}$, $-CH_2C(O)NR^{27}R^{28}$, $-OCH_2C(O)NR^{27}R^{28}$, $-CH_2OR^{27}$, $-CH_2NR^{27}R^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O)$OR^{27}$, $-NR^{27}$-C(=O)-$C_1$-$C_6$-alkyl-C(=O)$OR^{27}$, $-NR^{27}$-C(=O)-$C_1$-$C_6$-alkenyl-C(=O)$OR^{27}$-, -C(=O)$NR^{27}$-$C_1$-$C_6$-alkyl-C(=O)$OR^{27}$, $-C_1$-$C_6$-alkyl-C(=O)$OR^{27}$, or -C(O)$OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O)$OR^{27}$, -C(=O)$NR^{27}$-$C_1$-$C_6$-alkyl-C(=O)$OR^{27}$, $-C_1$-$C_6$-alkyl-C(=O)$OR^{27}$, or -C(O)$OR^{27}$,

- $C_1$-$C_6$-alkyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O)$OR^{27}$, -C(=O)$NR^{27}$-$C_1$-$C_6$-alkyl-C(=O)$OR^{27}$, $-C_1$-$C_6$-alkyl-C(=O)$OR^{27}$, or -C(O)$OR^{27}$,

- methyl, ethyl propyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O)$OR^{27}$, -C(=O)$NR^{27}$-$C_1$-$C_6$-alkyl-C(=O)$OR^{27}$, $-C_1$-$C_6$-alkyl-C(=O)$OR^{27}$, or -C(O)$OR^{27}$,

- Methyl, ethyl propyl optionally substituted with one or more substituents independently selected from $R^{29}$

- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-C(=O)$OR^{27}$, -C(=O)$NR^{27}$-$C_1$-$C_6$-alkyl-C(=O)$OR^{27}$, $-C_1$-$C_6$-alkyl-C(=O)$OR^{27}$, or -C(O)$OR^{27}$,

- $C_1$-$C_6$-alkyl optionally substituted with one or more substituents independently selected from $R^{29}$

- phenyl, phenyloxy, phenyl-$C_1$-$C_6$-alkoxy, phenyl-$C_1$-$C_6$-alkyl,

of which the cyclic moieties optionally may be substituted with one or more substituents selected from $R^{30}$.

In another embodiment hereof $R^{19}$ is hydrogen or methyl.

In another embodiment hereof $R^{19}$ is hydrogen.
In another embodiment hereof $R^{27}$ is hydrogen, $C_1$-$C_6$-alkyl or aryl.
In another embodiment hereof $R^{27}$ is hydrogen or $C_1$-$C_6$-alkyl.
In another embodiment hereof $R^{28}$ is hydrogen or $C_1$-$C_6$-alkyl.
In another embodiment hereof F is a valence bond.
In another embodiment hereof F is $C_1$-$C_6$-alkylene optionally substituted with one or more hydroxy, $C_1$-$C_6$-alkyl, or aryl.
In another embodiment hereof G is $C_1$-$C_6$-alkyl or aryl, wherein the aryl is optionally substituted with up to three substituents $R^{24}$, $R^{25}$ and $R^{26}$.
In another embodiment hereof G is $C_1$-$C_6$-alkyl or ArG1, wherein the aryl is optionally substituted with up to three substituents $R^{24}$, $R^{25}$ and $R^{26}$.
In another embodiment hereof G is $C_1$-$C_6$-alkyl.
In another embodiment hereof G is phenyl optionally substituted with up to three substituents $R^{24}$, $R^{25}$ and $R^{26}$.
In another embodiment hereof $R^{24}$, $R^{25}$ and $R^{26}$ are independently selected from

- hydrogen, halogen, $-CHF_2$, $-CF_3$, $-OCF_3$, $-OCHF_2$, $-OCH_2CF_3$, $-OCF_2CHF_2$, $-SCF_3$,$-NO_2$, $-OR^{27}$, $-NR^{27}R^{28}$, $-SR^{27}$, $-C(O)NR^{27}R^{28}$, $-OC(O)NR^{27}R^{28}$, $-NR^{27}C(O)R^{28}$, $-NR^{27}C(O)OR^{28}$, $-CH_2C(O)NR^{27}R^{28}$, $-OCH_2C(O)NR^{27}R^{28}$, $-CH_2OR^{27}$, $-CH_2NR^{27}R^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-NR^{27}$-$C(=O)$-$C_1$-$C_6$-alkyl-$C(=O)OR^{27}$, $-NR^{27}$-$C(=O)$-$C_1$-$C_6$-alkenyl-$C(=O)OR^{27}$-, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment hereof $R^{24}$, $R^{25}$ and $R^{26}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- $C_1$-$C_6$-alkyl, $C_2$-$C_6$-alkenyl or $C_2$-$C_6$-alkynyl,
  which may optionally be substituted with one or more substituents independently selected from $R^{29}$

- aryl, aryloxy, aryloxycarbonyl, aroyl, aryl-$C_1$-$C_6$-alkoxy, aryl-$C_1$-$C_6$-alkyl, aryl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{30}$.

In another embodiment hereof $R^{24}$, $R^{25}$ and $R^{26}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- $C_1$-$C_6$-alkyl optionally substituted with one or more substituents independently selected from $R^{29}$

- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, $-OCF_3$, $-OR^{27}$, $-NR^{27}R^{28}$, $-SR^{27}$, $-NR^{27}C(O)R^{28}$, $-NR^{27}C(O)OR^{28}$, $-OC(O)R^{27}$, $-OC_1$-$C_6$-alkyl-$C(O)OR^{27}$, $-SC_1$-$C_6$-alkyl-$C(O)OR^{27}$, $-C_2$-$C_6$-alkenyl-$C(=O)OR^{27}$, $-C(=O)NR^{27}$-$C_1$-$C_6$-alkyl-$C(=O)OR^{27}$, $-C_1$-$C_6$-alkyl-$C(=O)OR^{27}$, or $-C(O)OR^{27}$,

- methyl, ethyl propyl optionally substituted with one or more substituents independently selected from $R^{29}$

- ArG1, ArG1-O-, ArG1-C(O)-, ArG1-$C_1$-$C_6$-alkoxy, $ArG_1$-$C_1$-$C_6$-alkyl, Het3, Het3-$C_1$-$C_6$-alkyl

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, -$OCF_3$, -$OR^{27}$, -$NR^{27}R^{28}$, -$SR^{27}$, -$NR^{27}C(O)R^{28}$, -$NR^{27}C(O)OR^{28}$, -$OC(O)R^{27}$, -$OC_1$-$C_6$-alkyl-$C(O)OR^{27}$, -$SC_1$-$C_6$-alkyl-$C(O)OR^{27}$, -$C_2$-$C_6$-alkenyl-$C(=O)OR^{27}$, -$C(=O)NR^{27}$-$C_1$-$C_6$-alkyl-$C(=O)OR^{27}$, -$C_1$-$C_6$-alkyl-$C(=O)OR^{27}$, or -$C(O)OR^{27}$,
- methyl, ethyl propyl optionally substituted with one or more substituents independently selected from $R^{29}$
- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment hereof $R^{21}$, $R^{22}$ and $R^{23}$ are independently selected from

- hydrogen, halogen, -$OCF_3$, -$OR^{27}$, -$NR^{27}R^{28}$, -$SR^{27}$, -$NR^{27}C(O)R^{28}$, -$NR^{27}C(O)OR^{28}$, -$OC(O)R^{27}$, -$OC_1$-$C_6$-alkyl-$C(O)OR^{27}$, -$SC_1$-$C_6$-alkyl-$C(O)OR^{27}$, -$C_2$-$C_6$-alkenyl-$C(=O)OR^{27}$, -$C(=O)NR^{27}$-$C_1$-$C_6$-alkyl-$C(=O)OR^{27}$, -$C_1$-$C_6$-alkyl-$C(=O)OR^{27}$, or -$C(O)OR^{27}$,
- methyl, ethyl propyl optionally substituted with one or more substituents independently selected from $R^{29}$
- ArG1, ArG1-O-, ArG1-$C_1$-$C_6$-alkoxy, ArG1-$C_1$-$C_6$-alkyl,

of which the cyclic moieties optionally may be substituted with one or more substituents selected from $R^{30}$.

In another embodiment hereof $R^{20}$ is hydrogen or methyl.
In another embodiment hereof $R^{20}$ is hydrogen.
In another embodiment hereof $R^{27}$ is hydrogen, $C_1$-$C_6$-alkyl or aryl.
In another embodiment hereof $R^{27}$ is hydrogen or $C_1$-$C_6$-alkyl or ArG1.
In another embodiment hereof $R^{27}$ is hydrogen or $C_1$-$C_6$-alkyl.
In another embodiment hereof $R^{28}$ is hydrogen or $C_1$-$C_6$-alkyl.
In another embodiment hereof $R^{17}$ and $R^{18}$ are independently selected from

- hydrogen, halogen, -CN, -$CF_3$, -$OCF_3$, -$NO_2$, -$OR^{27}$, -$NR^{27}R^{28}$, -$SR^{27}$, -$S(O)R^{27}$, -$S(O)_2R^{27}$, -$C(O)NR^{27}R^{28}$, -$CH_2OR^{27}$, -$OC(O)R^{27}$, -$OC_1$-$C_6$-alkyl-$C(O)OR^{27}$, -$SC_1$-$C_6$-alkyl-$C(O)OR^{27}$, or -$C(O)OR^{27}$,
- $C_1$-$C_6$-alkyl, $C_2$-$C_6$-alkenyl or $C_2$-$C_6$-alkynyl, optionally substituted with one or more substituents independently selected from $R^{29}$
- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment hereof $R^{17}$ and $R^{18}$ are independently selected from

- hydrogen, halogen, -CN, -$CF_3$, -$N0_2$, -$OR^{27}$, -$NR^{27}R^{28}$, or -$C(O)OR^{27}$,
- $C_1$-$C_6$-alkyl optionally substituted with one or more substituents independently selected from $R^{29}$
- aryl, aryloxy, aroyl, aryl-$C_1$-$C_6$-alkoxy, aryl-$C_1$-$C_6$-alkyl, heteroaryl, heteroaryl-$C_1$-$C_6$-alkyl,

of which the cyclic moieties optionally may be substituted with one or more substituents selected from $R^{30}$.

In another embodiment hereof $R^{17}$ and $R^{18}$ are independently selected from

- hydrogen, halogen, -CN, -$CF_3$, -$NO_2$, -$OR^{27}$, -$NR^{27}R^{28}$, or -$C(O)OR^{27}$
- methyl, ethyl propyl optionally substituted with one or more substituents independently selected from $R^{29}$
- aryl, aryloxy, aroyl, aryl-$C_1$-$C_6$-alkoxy, aryl-$C_1$-$C_6$-alkyl, heteroaryl, heteroaryl-$C_1$-$C_6$-alkyl

of which the cyclic moieties optionally may be substituted with one or more substituents selected from $R^{30}$.

In another embodiment hereof $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$
- methyl, ethyl propyl optionally substituted with one or more substituents independently selected from $R^{29}$
- ArG1, ArG1-O-, ArG1-C(O)-, ArG1-$C_1$-$C_6$-alkoxy, ArG1-$C_1$-$C_6$-alkyl, Het3, Het3-$C_1$-$C_6$-alkyl

of which the cyclic moieties optionally may be substituted with one or more substituents selected from $R^{30}$.

In another embodiment hereof $R^{17}$ and $R^{18}$ are independently selected from

- hydrogen, halogen, -CN, $-CF_3$, $-NO_2$, $-OR^{27}$, $-NR^{27}R^{28}$, or $-C(O)OR^{27}$
- $C_1$-$C_6$-alkyl optionally substituted with one or more substituents independently selected from $R^{29}$
- phenyl, phenyloxy, phenyl-$C_1$-$C_6$-alkoxy, phenyl-$C_1$-$C_6$-alkyl,

of which the cyclic moieties optionally may be substituted with one or more substituents selected from $R^{30}$.
In another embodiment hereof $R^{27}$ is hydrogen or $C_1$-$C_6$-alkyl.
In another embodiment hereof $R^{27}$ is hydrogen, methyl or ethyl.
In another embodiment hereof $R^{28}$ is hydrogen or $C_1$-$C_6$-alkyl.
In another embodiment hereof $R^{28}$ is hydrogen, methyl or ethyl.
In another embodiment hereof $R^{72}$ is -OH or phenyl.

In another embodiment hereof the zinc-binding ligand is

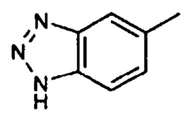

In another embodiment hereof the zinc-binding ligand is of the form H-I-J

wherein H is

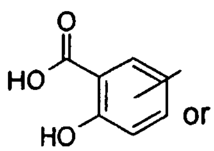

or

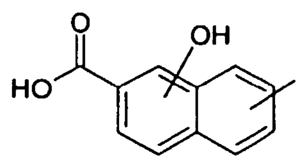

or

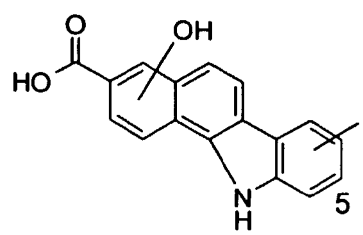

wherein the phenyl, naphthalene or benzocarbazole rings are optionally substituted with one or more substituents independently selected from $R^{31}$

I is selected from

- a valence bond,
- $-CH_2N(R^{32})$- or $-SO_2N(R^{33})$-,
- 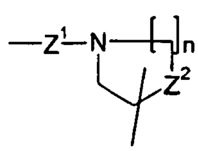

wherein $Z^1$ is $S(O)_2$ or $CH_2$, $Z^2$ is -NH-, -O-or -S-, and n is 1 or 2,

J is

- $C_1$-$C_6$-alkyl, $C_2$-$C_6$-alkenyl or $C_2$-$C_6$-alkynyl, which may each optionally be substituted with one or more substituents selected from $R^{34}$,
- Aryl, aryloxy, aryl-oxycarbonyl-, aroyl, aryl-$C_1$-$C_6$-alkoxy-, aryl-$C_1$-$C_6$-alkyl-, aryl-$C_2$-$C_6$-alkenyl-, aryl-$C_2$-$C_6$-alkynyl-, heteroaryl, heteroaryl-$C_1$-$C_6$-alkyl-, heteroaryl-$C_2$-$C_6$-alkenyl- or heteroaryl-$C_2$-$C_6$-alkynyl-, wherein the cyclic moieties are optionally substituted with one or more substituents selected from $R^{37}$,
- hydrogen,

$R^{31}$ is independently selected from hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, $-OR^{35}$, $-C(O)R^{35}$, $-NR^{35}R^{36}$, $-SR^{35}$, $-NR^{35}S(O)_2R^{36}$, $-S(O)_2NR^{35}R^{36}$, $-S(O)NR^{35}R^{36}$, $-S(O)R^{35}$, $-S(O)_2R^{35}$, $-C(O)NR^{35}R^{36}$, $-OC(O)NR^{35}R^{36}$, $-NR^{35}C(O)R^{36}$, $-CH_2C(O)NR^{35}R^{36}$, $-OCH_2C(O)NR^{35}R^{36}$, $-CH_2OR^{35}$, $-CH_2NR^{35}R^{36}$, $-OC(O)R^{35}$, $-OC_1$-$C_6$-alkyl-$C(O)OR^{35}$, $-SC_1$-$C_6$-alkyl-$C(O)OR^{35}$ $-C_2$-$C_6$-alkenyl-$C(=O)OR^{35}$, $-NR_{35}$-$C(=O)$-$C_1$-$C_6$-alkyl-$C(=O)OR^{35}$, $-NR^{35}$-$C(=O)$-$C_1$-$C_6$-alkenyl-$C(=O)OR^{35}$-, $C_1$-$C_6$-alkyl, $C_1$-$C_6$-alkanoyl or $-C(O)OR^{35}$,

$R^{32}$ and $R^{33}$ are independently selected from hydrogen, $C_1$-$C_6$-alkyl or $C_1$-$C_6$-alkanoyl,

$R^{34}$ is independently selected from halogen, -CN, $-CF_3$, $-OCF_3$, $-OR^{35}$, and $-NR^{35}R^{36}$,

$R^{35}$ and $R^{36}$ are independently selected from hydrogen, $C_1$-$C_6$-alkyl, aryl-$C_1$-$C_6$-alkyl or aryl, or $R^{35}$ and $R^{36}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{37}$ is independently selected from halogen, $-C(O)OR^{35}$, -C(O)H, -CN, $-CF_3$, $-OCF_3$, $-NO_2$, - $OR^{35}$, $-NR^{35}R^{36}$, $C_1$-$C_6$-alkyl or $C_1$-$C_6$-alkanoyl,

or any enantiomer, diastereomer, including a racemic mixture, tautomer as well as a salt thereof with a pharmaceutically acceptable acid or base.

In another embodiment hereof the zinc-binding ligand is of the form H-I-J, wherein H is wherein the phenyl, naphthalene or benzocarbazole rings are optionally substituted with one

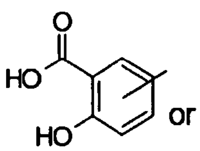 or 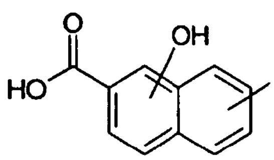 or 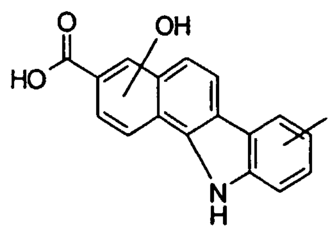

or more substituents independently selected from $R^{31}$,

I is selected from

- a valence bond,
- $-CH_2N(R^{32})$- or $-SO_2N(R^{33})$-,
- 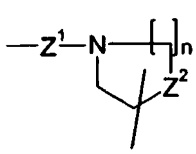

wherein $Z^1$ is $S(O)_2$ or $CH_2$, $Z^2$ is N,-O-or -S-, and n is 1 or 2,

J is

- $C_1$-$C_6$-alkyl, $C_2$-$C_6$-alkenyl or $C_2$-$C_6$-alkynyl, which may each optionally be substituted with one or more substituents selected from $R^{34}$,
- Aryl, aryloxy, aryl-oxycarbonyl-, aroyl, aryl-$C_1$-$C_6$-alkoxy-, aryl-$C_1$-$C_6$-alkyl-, aryl-$C_2$-$C_6$-alkenyl-, aryl-$C_2$-$C_6$-alkynyl-, heteroaryl, heteroaryl-$C_1$-$C_6$-alkyl-, heteroaryl-$C_2$-$C_6$-alkenyl- or heteroaryl-$C_2$-$C_6$-alkynyl-, wherein the cyclic moieties are optionally substituted with one or more substituents selected from $R^{37}$ ,
- hydrogen,

$R^{31}$ is independently selected from hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, $-SCF_3$, $-NO_2$, $-OR^{35}$, $-C(O)R^{35}$, $-NR^{35}R^{36}$, $-SR^{35}$, $-NR^{35}S(O)_2R^{36}$, $-S(O)_2NR^{35}R^{36}$, $-S(O)NR^{35}R^{36}$, $-S(O)R^{35}$, $-S(O)_2R^{35}$, $-C(O)NR^{35}R^{36}$, $-OC(O)NR^{35}R^{36}$, $-NR^{35}C(O)R^{36}$, $-CH_2C(O)NR^{35}R^{36}$, $-OCH_2C(O)NR^{35}R^{36}$, $-CH_2OR^{35}$, $-CH_2NR^{35}R^{36}$, $-OC(O)R^{35}$, $-OC_1$-$C_6$-alkyl-$C(O)OR^{35}$, $-SC_1$-$C_6$-alkyl-$C(O)OR^{35}$ $-C_2$-$C_6$-alkenyl-$C(=O)OR^{35}$, $-NR^{35}$-C(=O)-$C_1$-$C_6$-alkyl-$C(=O)OR^{35}$, $-NR^{35}$-C(=O)-$C_1$-$C_6$-alkenyl-$C(=O)OR^{35}$-, $C_1$-$C_6$-alkyl, $C_1$-$C_6$-alkanoyl or $-C(O)OR^{35}$.

$R^{32}$ and $R^{33}$are independently selected from hydrogen, $C_1$-$C_6$-alkyl or $C_1$-$C_6$-alkanoyl,

$R^{34}$ is independently selected from halogen, -CN, $-CF_3$, $-OCF_3$, $-OR^{35}$ , and $-NR^{35}R^{36}$,

$R^{35}$ and $R^{36}$ are independently selected from hydrogen, $C_1$-$C_6$-alkyl, aryl-$C_1$-$C_6$-alkyl or aryl, or $R^{35}$ and $R^{36}$ when attached to the same nitrogen atom together with the said nitrogen atom may form a 3 to 8 membered heterocyclic ring optionally containing one or two further heteroatoms selected from nitrogen, oxygen and sulphur, and optionally containing one or two double bonds,

$R^{37}$ is independently selected from halogen, $-C(O)OR^{35}$, -C(O)H, -CN, $-CF_3$, $-OCF_3$, $-NO_2$,$-OR^{35}$, $-NR^{35}R^{36}$, $C_1$-$C_6$-alkyl or $C_1$-$C_6$-alkanoyl,

or any enantiomer, diastereomer, including a racemic mixture, tautomer as well as a salt thereof with a pharmaceutically acceptable acid or base,
With the proviso that $R^{31}$ and J cannot both be hydrogen.

In another embodiment hereof H is

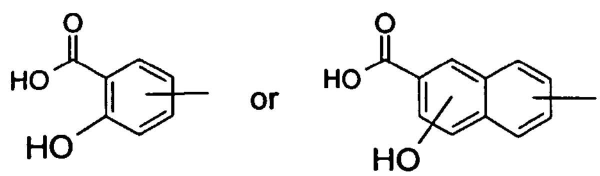

In another embodiment hereof H is

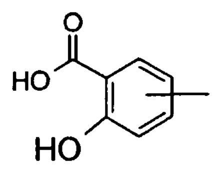

In another embodiment hereof H is

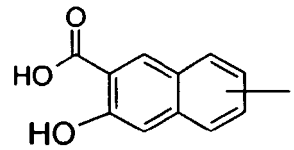

In another embodiment hereof I is a valence bond, $-CH_2N(R^{32})-$, or $-SO_2N(R^{33})-$.
In another embodiment hereof I is a valence bond.
In another embodiment hereof J is

- hydrogen,
- $C_1$-$C_6$-alkyl, $C_2$-$C_6$-alkenyl or $C_2$-$C_6$-alkynyl, which may optionally be substituted with one or more substituents selected from halogen, -CN, $-CF_3$, $-OCF_3$, $-OR^{35}$, and $-NR^{35}R^{36}$,
- aryl, or heteroaryl, wherein the cyclic moieties are optionally substituted with one or more substituents independently selected from $R^{37}$.

In another embodiment hereof J is

- hydrogen,
- aryl or heteroaryl, wherein the cyclic moieties are optionally substituted with one or more substituents independently selected from $R^{37}$.

In another embodiment hereof J is

- hydrogen,
- ArG1 or Het3, wherein the cyclic moieties are optionally substituted with one or more substituents independently selected from $R^{37}$.

In another embodiment hereof J is

- hydrogen,
- phenyl or naphthyl optionally substituted with one or more substituents independently selected from $R^{37}$.

In another embodiment hereof J is hydrogen.
In another embodiment hereof $R^{32}$ and $R^{33}$ are independently selected from hydrogen or $C_1$-$C_6$-alkyl.
In another embodiment hereof $R^{34}$ is hydrogen, halogen, -CN, $-CF_3$, $-OCF_3$, $-SCF_3$, $-NO_2$, $-OR^{35}$, $-C(O)R^{35}$, $-NR^{35}R^{36}$, $-SR^{35}$, $-C(O)NR^{35}R^{36}$, $-OC(O)NR^{35}R^{36}$, $-NR^{35}C(O)R^{36}$, $-OC(O)R^{35}$, $-OC_1$-$C_6$-alkyl-$C(O)OR^{35}$, $-SC_1$-$C_6$-alkyl-$C(O)OR^{35}$ or $-C(O)OR^{35}$.
In another embodiment hereof $R^{34}$ is hydrogen, halogen, $-CF_3$, $-NO_2$, $-OR^{35}$, $-NR^{35}R^{36}$ $-SR^{35}$, $-NR^{35}C(O)R^{36}$, or $-C(O)OR^{35}$.
In another embodiment hereof $R^{34}$ is hydrogen, halogen, $-CF_3$, $-NO_2$, $-OR^{35}$, $-NR^{35}R^{36}$, or $-NR^{35}C(O)R^{36}$.
In another embodiment hereof $R^{34}$ is hydrogen, halogen, or $-OR^{35}$.
In another embodiment hereof $R^{35}$ and $R^{36}$ are independently selected from hydrogen, $C_1$-$C_6$-alkyl, or aryl.
In another embodiment hereof $R^{35}$ and $R^{36}$ are independently selected from hydrogen or $C_1$-$C_6$-alkyl.
In another embodiment hereof $R^{37}$ is halogen, $-C(O)OR^{35}$, -CN, $-CF_3$, $-OR^{35}$, $-NR^{35}R^{36}$, $C_1$-$C_6$-alkyl or $C_1$-$C_6$-alkanoyl.
In another embodiment hereof $R^{37}$ is halogen, $-C(O)OR^{35}$, $-OR^{35}$, $-NR^{35}R^{36}$, $C_1$-$C_6$-alkyl or $C_1$-$C_6$-alkanoyl.
In another embodiment hereof $R^{37}$ is halogen, $-C(O)OR^{35}$ or $-OR^{35}$.
In another embodiment hereof the zinc-binding ligand is

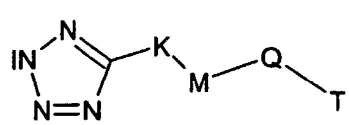

wherein K is a valence bond, $C_1$-$C_6$-alkylene, -NH-C(=O)-U-, -$C_1$-$C_6$-alkyl-S-, -$C_1$-$C_6$-alkyl-O-, -C(=O)-, or -C(=O)-NH-, wherein any $C_1$-$C_6$-alkyl moiety is optionally substituted with $R^{38}$,

U is a valence bond, $C_1$-$C_6$-alkenylene, -$C_1$-$C_6$-alkyl-O- or $C_1$-$C_6$-alkylene wherein any $C_1$-$C_6$-alkyl moiety is optionally substituted with $C_1$-$C_6$-alkyl,

$R^{38}$ is $C_1$-$C_6$-alkyl, aryl, wherein the alkyl or aryl moieties are optionally substituted with one or more substituents independently selected from $R^{39}$,

$R^{39}$ is independently selected from halogen, cyano, nitro, amino,

M is a valence bond, arylene or heteroarylene, wherein the aryl or heteroaryl moieties are optionally substituted with one or more substituents independently selected from $R^{40}$,

$R^{40}$ is selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -$S(O)_2CF_3$, -OS$(O)_2CF_3$, -$SCF_3$, -$NO_2$, -$OR^{41}$, -$NR^{41}R^{42}$, -$SR^{41}$, -$NR^{41}S(O)_2R^{42}$, -$S(O)_2NR^{41}R^{42}$, -$S(O)NR^{41}R^{42}$, -$S(O)R^{41}$, -$S(O)_2R^{41}$, -$OS(O)_2$ $R^{41}$, -$C(O)NR^{41}R^{42}$, -$OC(O)NR^{41}R^{42}$, -$NR^{41}C(O)R^{42}$, -$CH_2C(O)NR^{41}R^{42}$, -$OC_1$-$C_6$-alkyl-$C(O)NR^{41}R^{42}$, -$CH_2OR^{41}$, -$CH_2OC(O)R^{41}$, -$CH_2NR^{41}R^{42}$, -$OC(O)R^{41}$, -$OC_1$-$C_6$-alkyl-$C(O)OR^{41}$, -$OC_1$-$C_6$-alkyl-$OR^{41}$, -S-$C_1$-$C_6$-alkyl-$C(O)OR^{41}$, -$C_2$-$C_6$-alkenyl-$C(=O)OR^{41}$, -$NR^{41}$-C(=O)-$C_1$-$C_6$-alkyl-C(=O)$OR^{41}$, -$NR^{41}$-C(=O)-$C_1$-$C_6$-alkenyl-$C(=O)OR^{41}$, -$C(O)OR^{41}$, -$C_2$-$C_6$-alkenyl-$C(=O)R^{41}$, =O, -NH-C(=O)-O-$C_1$-$C_6$-alkyl, or -NH-C(=O)-C(=O)-O-$C_1$-$C_6$-alkyl,
- $C_1$-$C_6$-alkyl, $C_2$-$C_6$-alkenyl or $C_2$-$C_6$-alkynyl, which may each optionally be substituted with one or more substituents selected from $R^{43}$,
- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl, wherein the cyclic moieties optionally may be substituted with one or more substituents selected from $R^{44}$,

$R^{41}$ and $R^{42}$ are independently selected from hydrogen, -OH, $C_1$-$C_6$-alkyl, $C_1$-$C_6$-alkenyl, aryl-$C_1$-$C_6$-alkyl or aryl, wherein the alkyl moieties may optionally be substituted with one or more substituents independently selected from $R^{45}$, and the aryl moieties may optionally be substituted with one or more substituents independently selected from $R^{46}$; $R^{41}$ and $R^{42}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{43}$ is independently selected from halogen, -CN, -$CF_3$, -$OCF_3$, -$OR^{41}$, and -$NR^{41}R^{42}$

$R^{44}$ is independently selected from halogen, -$C(O)OR^{41}$, -$CH_2C(O)OR^{41}$, -$CH_2OR^{41}$, -CN, - $CF_3$, -$OCF_3$, -$NO_2$, -$OR^{41}$, -$NR^{41}R^{42}$ and $C_1$-$C_6$-alkyl,

$R^{45}$ is independently selected from halogen, -CN, -$CF_3$, -$OCF_3$, -O-$C_1$-$C_6$-alkyl, -C(O)-O-$C_1$-$C_6$-alkyl, -COOH and -$NH_2$,

$R^{46}$ is independently selected from halogen, -$C(O)OC_1$-$C_6$-alkyl, -COOH, -CN, -$CF_3$, -$OCF_3$, - $NO_2$, -OH, -$OC_1$-$C_6$-alkyl, -$NH_2$, C(=O) or $C_1$-$C_6$-alkyl,

Q is a valence bond, $C_1$-$C_6$-alkylene, -$C_1$-$C_6$-alkyl-O-, -$C_1$-$C_6$-alkyl-NH-, -NH-$C_1$-$C_6$-alkyl, -NH-C(=O)-, -C(=O)-NH-, -O-$C_1$-$C_6$-alkyl, -C(=O)-, or -$C_1$-$C_6$-alkyl-C(=O)-N($R^{47}$)- wherein the alkyl moieties are optionally substituted with one or more substituents independently selected from $R^{46}$,

$R^{47}$ and $R^{48}$ are independently selected from hydrogen, $C_1$-$C_6$-alkyl, aryl optionally substituted with one or more $R^{49}$,

$R^{49}$ is independently selected from halogen and -COOH,

T is

- hydrogen,
- $C_1$-$C_6$-alkyl, $C_2$-$C_6$-alkenyl, $C_2$-$C_6$-alkynyl, $C_1$-$C_6$-alkyloxy- carbonyl, wherein the alkyl, alkenyl and alkynyl moieties are optionally substituted with one or more substituents independently selected from $R^{50}$,
- aryl, aryloxy, aryloxy-carbonyl, aryl-$C_1$-$C_6$-alkyl, aroyl, aryl-$C_1$-$C_6$-alkoxy, aryl-$C_2$-$C_6$-alkenyl, aryl-$C_2$-$C_6$-alkyny-, heteroaryl, heteroaryl-$C_1$-$C_6$-alkyl, heteroaryl-$C_2$-$C_6$-alkenyl, heteroaryl-$C_2$-$C_6$-alkynyl,

wherein any alkyl, alkenyl , alkynyl, aryl and heteroaryl moiety is optionally substituted with one or more substituents independently selected from $R^{50}$,

$R^{50}$ is $C_1$-$C_6$-alkyl, $C_1$-$C_6$-alkoxy, aryl, aryloxy, aryl-$C_1$-$C_6$-alkoxy, -C(=O)-NH-$C_1$-$C_6$-alkyl-aryl, -C(=O)-$NR^{50A}$-$C_1$-$C_6$-alkyl, -C(=O)-NH-$(CH_2CH_2O)_m$$C_1$-$C_6$-alkyl-COOH, heteroaryl, heteroaryl-$C_1$-$C_8$-alkoxy, -$C_1$-$C_6$-alkyl-COOH, -O-$C_1$-$C_6$-alkyl-COOH, -$S(O)_2R^{51}$, -$C_2$-$C_6$-alkenyl-COOH, -$OR^{51}$, -$NO_2$, halogen, -COOH, -$CF_3$, -CN, =O, -$N(R^{51}R^{52})$, wherein m is 1, 2, 3 or 4, and wherein the aryl or heteroaryl moieties are optionally substituted with one or more $R^{53}$, and the alkyl moieties are optionally substituted with one or more $R^{50B}$.

$R^{50A}$ and $R^{50B}$ are independently selected from -C(O)O$C_1$-$C_6$-alkyl, -COOH, -$C_1$-$C_6$-alkyl-C(O)O$C_1$-$C_6$-alkyl, -$C_1$-$C_6$-alkyl-COOH, or $C_1$-$C_6$-alkyl,

$R^{51}$ and $R^{52}$ are independently selected from hydrogen and $C_1$-$C_6$-alkyl,

$R^{53}$ is independently selected from $C_1$-$C_6$-alkyl, $C_1$-$C_6$-alkoxy, -$C_1$-$C_6$-alkyl-COOH, -$C_2$-$C_6$-alkenyl-COOH, -$OR^{51}$, -$NO_2$, halogen, -COOH, -$CF_3$, -CN, or -$N(R^{51}R^{52})$,

or any enantiomer, diastereomer, including a racemic mixture, tautomer as well as a salt thereof with a pharmaceutically acceptable acid or base.
In another embodiment hereof K is a valence bond, $C_1$-$C_6$-alkylene, -NH-C(=O)-U-, -$C_1$-$C_6$-alkyl-S-, -$C_1$-$C_6$-alkyl-O-, or -C(=O)-, wherein any $C_1$-$C_6$-alkyl moiety is optionally substituted with $R^{38}$.
In another embodiment hereof K is a valence bond, $C_1$-$C_6$-alkylene, -NH-C(=O)-U-, -$C_1$-$C_6$-alkyl-S-, or -$C_1$-$C_6$-alkyl-O, wherein any $C_1$-$C_6$-alkyl moiety is optionally substituted with $R^{38}$.
In another embodiment hereof K is a valence bond, $C_1$-$C_6$-alkylene, or -NH-C(=O)-U, wherein any $C_1$-$C_6$-alkyl moiety is optionally substituted with $R^{38}$.
In another embodiment hereof K is a valence bond or $C_1$-$C_6$-alkylene, wherein any $C_1$-$C_6$-alkyl moiety is optionally substituted with $R^{38}$.
In another embodiment hereof K is a valence bond or -NH-C(=O)-U.
In another embodiment hereof K is a valence bond.
In another embodiment hereof U is a valence bond or -$C_1$-$C_6$-alkyl-O-.
In another embodiment hereof U is a valence bond.
In another embodiment hereof M is arylene or heteroarylene, wherein the arylene or heteroarylene moieties are optionally substituted with one or more substituents independently selected from $R^{40}$.
In another embodiment hereof M is ArG1 or Het1, wherein the arylene or heteroarylene moieties are optionally substituted with one or more substituents independently selected from $R^{40}$.
In another embodiment hereof M is ArG1 or Het2, wherein the arylene or heteroarylene moieties are optionally substituted with one or more substituents independently selected from $R^{40}$.
In another embodiment hereof M is ArG1 or Het3, wherein the arylene or heteroarylene moieties are optionally substituted with one or more substituents independently selected from $R^{40}$.
In another embodiment hereof M is phenylene optionally substituted with one or more substituents independently selected from $R^{40}$.
In another embodiment hereof M is indolylene optionally substituted with one or more substituents independently selected from $R^{40}$.
In another embodiment hereof M is

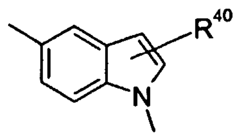

In another embodiment hereof M is carbazolylene optionally substituted with one or more substituents independently selected from $R^{40}$.
In another embodiment hereof M is

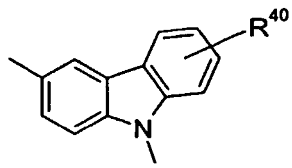

In another embodiment hereof $R^{40}$ is selected from

- hydrogen, halogen, -CN, $-CF_3$, $-OCF_3$, $-NO_2$, $-OR^{41}$, $-NR^{41}R^{42}$, $-SR^{41}$, $-S(O)_2R^{41}$, $-NR^{41}C(O)R^{42}$, $-OC_1$-$C_6$-alkyl-$C(O)NR^{41}R^{42}$, $-C_2$-$C_6$-alkenyl-$C(=O)OR^{41}$, $-C(O)OR^{41}$, =O, -NH-C(=O)-O-$C_1$-$C_6$-alkyl, or -NH-C(=O)-C(=O)-O-$C_1$-$C_6$-alkyl,
  $C_1$-$C_6$-alkyl or $C_2$-$C_6$- alkenyl which may each optionally be substituted with one or more substituents independently selected from $R^{43}$,

- aryl, aryloxy, aryl-$C_1$-$C_6$-alkoxy, aryl-$C_1$-$C_6$-alkyl, aryl-$C_2$-$C_6$-alkenyl, heteroaryl, heteroaryl-$C_1$-$C_6$-alkyl, or heteroaryl-$C_2$-$C_6$-alkenyl, wherein the cyclic moieties optionally may be substituted with one or more substituents selected from $R^{44}$.

In another embodiment hereof $R^{40}$ is selected from

- hydrogen, halogen, -CN, $-CF_3$, $-OCF_3$, $-NO_2$, $-OR^{41}$, $-NR^{41}R^{42}$, $-SR^{41}$, $-S(O)_2R^{41}$, $-NR^{41}C(O)R^{42}$, $-OC_1$-$C_6$-alkyl-$C(O)NR^{41}R^{42}$, $-C_2$-$C_6$-alkenyl-$C(=O)OR^{41}$, $-C(O)OR^{41}$, =O, -NH-C(=O)-O-$C_1$-$C_6$-alkyl, or -NH-C(=O)-C(=O)-O-$C_1$-$C_6$-alkyl,
  $C_1$-$C_6$-alkyl or $C_2$-$C_6$- alkenyl which may each optionally be substituted with one or more substituents independently selected from $R^{43}$,
- ArG1, ArG1-O-, ArG1-$C_1$-$C_6$-alkoxy, ArG1-$C_1$-$C_6$-alkyl, ArG1-$C_2$-$C_6$-alkenyl, Het3, Het3-$C_1$-$C_6$-alkyl, or Het3-$C_2$-$C_6$-alkenyl, wherein the cyclic moieties optionally may be substituted with one or more substituents selected from $R^{44}$.

In another embodiment hereof $R^{40}$ is selected from

- hydrogen, halogen, $-CF_3$, $-NO_2$, $-OR^{41}$, $-NR^{41}R^{42}$, $-C(O)OR^{41}$, =O, or-$NR^{41}C(O)R^{42}$,
- $C_1$-$C_6$-alkyl,
- ArG1.

In another embodiment hereof $R^{40}$ is hydrogen.
In another embodiment hereof $R^{40}$ is selected from

- Halogen, $-NO_2$, $-OR^{41}$, $-NR^{41}R^{42}$, $-C(O)OR^{41}$, or $-NR^{41}C(O)R^{42}$,
- Methyl,
- Phenyl.

In another embodiment hereof $R^{41}$ and $R^{42}$ are independently selected from hydrogen, $C_1$-$C_6$-alkyl, or aryl, wherein the aryl moieties may optionally be substituted with halogen or - COOH.
In another embodiment hereof $R^{41}$ and $R^{42}$ are independently selected from hydrogen, methyl, ethyl, or phenyl, wherein the phenyl moieties may optionally be substituted with halogen or -COOH.

In another embodiment hereof Q is a valence bond, $C_1$-$C_6$-alkylene, -$C_1$-$C_6$-alkyl-O-, -$C_1$-$C_6$-alkyl-NH-, -NH-$C_1$-$C_6$-alkyl, -NH-C(=O)-, -C(=O)-NH-, -O-$C_1$-$C_6$-alkyl, -C(=O)-, or -$C_1$-$C_6$-alkyl-C(=O)-N($R^{47}$)- wherein the alkyl moieties are optionally substituted with one or more substituents independently selected from $R^{48}$.

In another embodiment hereof Q is a valence bond, -$CH_2$-, -$CH_2$-$CH_2$-, -$CH_2$-O-, -$CH_2$-$CH_2$-O-, -$CH_2$-NH-, -$CH_2$-$CH_2$-NH-, -NH-$CH_2$-, -NH-$CH_2$-$CH_2$-, -NH-C(=O)-, -C(=O)-NH-, -O-$CH_2$-, -O-$CH_2$-$CH_2$-, or -C(=O)-.

In another embodiment hereof $R^{47}$ and $R^{48}$ are independently selected from hydrogen, methyl and phenyl.

In another embodiment hereof T is

- Hydrogen,
- $C_1$-$C_6$-alkyl optionally substituted with one or more substituents independently selected from $R^{50}$,
- aryl, aryl-$C_1$-$C_6$-alkyl, heteroaryl, wherein the alkyl, aryl and heteroaryl moieties are optionally substituted with one or more substituents independently selected from $R^{50}$.

In another embodiment hereof T is

- hydrogen,
- $C_1$-$C_6$-alkyl optionally substituted with one or more substituents independently selected from $R^{50}$,
- ArG1, ArG1-$C_1$-$C_6$-alkyl, Het3, wherein the alkyl, aryl and heteroaryl moieties are optionally substituted with one or more substituents independently selected from $R^{50}$.

In another embodiment hereof T is

- hydrogen,
- $C_1$-$C_6$-alkyl, optionally substituted with one or more substituents independently selected from $R^{50}$,
- phenyl, phenyl-$C_1$-$C_6$-alkyl, wherein the alkyl and phenyl moieties are optionally substituted with one or more substituents independently selected from $R^{50}$.

In another embodiment hereof T is phenyl substituted with $R^{50}$.

In another embodiment hereof $R^{50}$ is $C_1$-$C_6$-alkyl, $C_1$-$C_6$-alkoxy, aryl, aryloxy, aryl-$C_1$-$C_6$-alkoxy, -C(=O)-NH-$C_1$-$C_6$-alkyl-aryl, -C(=O)-$NR^{50A}$-$C_1$-$C_6$-alkyl, -C(=O)-NH-$(CH_2CH_2O)_m$$C_1$-$C_6$-alkyl-COOH, heteroaryl, -$C_1$-$C_6$-alkyl-COOH, -O-$C_1$-$C_6$-alkyl-COOH, -$S(O)_2R^{51}$, -$C_2$-$C_6$-alkenyl-COOH, -$OR^{51}$, -$N0_2$, halogen, -COOH, -$CF_3$, -CN, =O, -N($R^{51}R^{52}$), wherein the aryl or heteroaryl moieties are optionally substituted with one or more $R^{53}$.

In another embodiment hereof $R^{50}$ is $C_1$-$C_6$-alkyl, $C_1$-$C_6$-alkoxy, aryl, aryloxy, -C(=O)-$NR^{50A}$-$C_1$-$C_6$-alkyl, -C(=O)-NH-$(CH_2CH_2O)_m$$C_1$-$C_6$-alkyl-COOH, aryl-$C_1$-$C_6$-alkoxy , -$OR^{51}$, -$NO_2$, halogen, -COOH, -$CF_3$, wherein any aryl moiety is optionally substituted with one or more $R^{53}$.

In another embodiment hereof $R^{50}$ is $C_1$-$C_6$-alkyl, aryloxy, -C(=O)-$NR^{50A}$-$C_1$-$C_6$-alkyl, -C(=O)-NH-$(CH_2CH_2$-$O)_m$$C_1$-$C_6$-alkyl-COOH, aryl-$C_1$-$C_6$-alkoxy, -$OR^{51}$, halogen, -COOH, -$CF_3$, wherein any aryl moiety is optionally substituted with one or more $R^{53}$.

In another embodiment hereof $R^{50}$ is $C_1$-$C_6$-alkyl, ArG1-0-, -C(=O)-$NR^{50A}$-$C_1$-$C_6$-alkyl -C(=O)-NH-$(CH_2CH_2$-$O)_m$$C_1$-$C_6$-alkyl-COOH, ArG1-$C_1$-$C_6$-alkoxy, -$OR^{51}$, halogen, -COOH, -$CF_3$, wherein any aryl moiety is optionally substituted with one or more $R^{53}$.

In another embodiment hereof $R^{50}$ is -C(=O)-$NR^{50A}CH_2$, -C(=O)-NH-$(CH_2CH_2O)_2CH_2$l-COOH, or -C(=O)-$NR^{50A}CH_2CH_2$.

In another embodiment hereof $R^{50}$ is phenyl, methyl or ethyl.

In another embodiment hereof $R^{50}$ is methyl or ethyl.

In another embodiment hereof m is 1 or 2.

In another embodiment hereof $R^{51}$ is methyl.

In another embodiment hereof $R^{53}$ is $C_1$-$C_6$-alkyl, $C_1$-$C_6$-alkoxy, -$OR^{51}$, halogen,or -$CF_3$.

In another embodiment hereof $R^{50A}$ is -C(O)$OCH_3$, -C(O)$OCH_2CH_3$ -COOH, -$CH_2C(O)OCH_3$, -$CH_2C(O)OCH_2CH_3$, -$CH_2CH_2C(O)OCH_3$, -$CH_2CH_2C(O)OCH_2CH_3$, -$CH_2COOH$, methyl, or ethyl.

In another embodiment hereof $R^{50B}$ is -C(O)$OCH_3$, -C(O)$OCH_2CH_3$ -COOH, -$CH_2C(O)OCH_3$, -$CH_2C(O)OCH_2CH_3$, -$CH_2CH_2C(O)OCH_3$, -$CH_2CH_2C(O)OCH_2CH_3$, -$CH_2COOH$, methyl, or ethyl.

In another embodiment hereof the zinc-binding ligand is

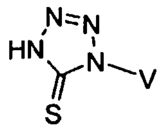

wherein V is $C_1$-$C_6$-alkyl, aryl, heteroaryl, aryl-$C_{1-6}$-alkyl- or aryl-$C_{2-6}$-alkenyl-, wherein the alkyl or alkenyl is optionally substituted with one or more substituents independently selected from $R^{54}$, and the aryl or heteroaryl is optionally substituted with one or more substituents independently selected from $R^{55}$,

$R^{54}$ is independently selected from halogen, -CN, -$CF_3$, -$OCF_3$, aryl, -COOH and -$NH_2$,

$R^{55}$ is independently selected from

- hydrogen, halogen, -CN, -$CH_2CN$, -$CHF_2$, -$CF_3$, -$OCF_3$, -$OCHF_2$, -$OCH_2CF_3$, -$OCF_2CHF_2$, -$S(O)_2CF_3$, -OS $(O)_2CF_3$, -$SCF_3$, -$NO_2$, -$OR^{56}$, -$NR^{56}R^{57}$, -$SR^{56}$, -$NR^{56}S(O)_2R^{57}$, -$S(O)_2NR^{56}R^{57}$, -$S(O)NR^{56}R^{57}$, -$S(O)R^{56}$, -$S(O)_2R^{56}$, -$OS(O)_2$ $R^{56}$, -$C(O)NR^{56}R^{57}$, -$OC(O)NR^{56}R^{57}$, -$NR^{56}C(O)R^{57}$, -$CH_2C(O)NR^{56}R^{57}$, -$OC_1$-$C_6$-alkyl-$C(O)NR^{56}R^{57}$, -$CH_2OR_{56}$, -$CH_2OC(O)R^{56}$, -$CH_2NR^{56}R^{57}$, -$OC(O)R^{56}$, -$OC_1$-$C_8$-alkyl-$C(O)OR^{56}$, -$OC_1$-$C_6$-alkyl-$OR^{56}$, -$SC_1$-$C_6$-alkyl-$C(O)OR^{56}$, -$C_2$-$C_6$-alkenyl-$C(=O)OR^{56}$, -$NR^{56}$-C(=O)-$C_1$-$C_6$-alkyl-C(=O) $OR^{56}$, -$NR^{56}$-C(=O)-$C_1$-$C_6$-alkenyl-$C(=O)OR^{58}$, -$C(O)OR^{56}$, or -$C_2$-$C_6$-alkenyl-$C(=O)R^{56}$,

- $C_1$-$C_6$-alkyl, $C_2$-$C_6$-alkenyl or $C_2$-$C_6$-alkynyl,
  which may optionally be substituted with one or more substituents selected from $R^{58}$,

- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl or heteroaryl-$C_2$-$C_6$-alkynyl,

of which the cyclic moieties optionally may be substituted with one or more substituents selected from $R^{59}$,

$R^{56}$ and $R^{57}$ are independently selected from hydrogen, OH, $CF_3$, $C_1$-$C_{12}$-alkyl, aryl-$C_1$-$C_6$-alkyl, -C(=O)-$C_1$-$C_6$-alkyl or aryl, wherein the alkyl groups may optionally be substituted with one or more substituents independently selected from $R^{60}$, and the aryl groups may optionally be substituted with one or more substituents independently selected from $R^{61}$; $R^{56}$ and $R^{57}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{58}$ is independently selected from halogen, -CN, -$CF_3$, -$OCF_3$, -$OR^{56}$, and -$NR^{56}R^{57}$,

$R^{59}$ is independently selected from halogen, -$C(O)OR^{56}$, $CH_2C(O)OR^{56}$ , -$CH_2OR^{56}$, -CN,-$CF_3$, -$OCF_3$, -$NO_2$, -$OR^{56}$, -$NR^{56}R^{57}$ and $C_1$-$C_6$-alkyl,

$R^{60}$ is independently selected from halogen, -CN, -$CF_3$, -$OCF_3$, -$OC_1$-$C_6$-alkyl, -$C(O)OC_1$-$C_6$-alkyl, -C(=O)-$R^{62}$, -COOH and -$NH_2$,

$R^{61}$ is independently selected from halogen, -$C(O)OC_1$-$C_6$-alkyl, -COOH, -CN, -$CF_3$, -$OCF_3$, - $NO_2$, -OH, -$OC_1$-$C_6$-alkyl, -$NH_2$, C(=O) or $C_1$-$C_6$-alkyl,

$R^{62}$ is $C_1$-$C_6$-alkyl, aryl optionally substituted with one or more substituents independently selected from halogen, or heteroaryl optionally substituted with one or more $C_1$-$C_6$-alkyl independently,
or any enantiomer, diastereomer, including a racemic mixture, tautomer as well as a salt thereof with a pharmaceutically acceptable acid or base.

In another embodiment hereof V is aryl, heteroaryl, or aryl-$C_{1-6}$-alkyl-, wherein the alkyl is optionally substituted with one or more substituents independently selected $R^{54}$, and the aryl or heteroaryl is optionally substituted with one or more substituents independently selected from $R^{55}$.

In another embodiment hereof V is aryl, Het1, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.
In another embodiment hereof V is aryl, Het2, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.
In another embodiment hereof V is aryl, Het3, or aryl-$C_{1-6}$-alkyl-, wherein the alkyl is optionally substituted with one or more substituents independently selected from $R^{54}$, and the aryl or heteroaryl moiety is optionally substituted with one or more substituents independently selected from $R^{55}$.
In another embodiment hereof V is aryl optionally substituted with one or more substituents independently selected from $R^{55}$.
In another embodiment hereof V is ArG1 optionally substituted with one or more substituents independently selected from $R^{55}$.
In another embodiment hereof V is phenyl, naphthyl or anthranyl optionally substituted with one or more substituents independently selected from $R^{55}$.
In another embodiment hereof V is phenyl optionally substituted with one or more substituents independently selected from $R^{55}$.
In another embodiment hereof $R^{55}$ is independently selected from

- halogen, $C_1$-$C_6$-alkyl, -CN, -$OCF_3$ ,-$CF_3$, -$NO_2$, -$OR^{56}$, -$NR^{56}R^{57}$, -$NR^{56}C(O)R^{57}$ -$SR^{56}$, -$OC_1$-$C_8$-alkyl-C(O)$OR^{56}$, or -C(O)$OR^{56}$,
- $C_1$-$C_6$-alkyl optionally substituted with one or more substituents independently selected from $R^{58}$
- aryl, aryl-$C_1$-$C_6$-alkyl, heteroaryl, or heteroaryl-$C_1$-$C_6$-alkyl

of which the cyclic moieties optionally may be substituted with one or more substituents independently selected from $R^{59}$.
In another embodiment hereof $R^{55}$ is independently selected from

- halogen, $C_1$-$C_6$-alkyl, -CN, -$OCF_3$ ,-$CF_3$, -$NO_2$, -$OR^{56}$, -$NR^{56}R^{57}$, -$NR^{56}C(O)R^{57}$ -$SR^{56}$, -$OC_1$-$C_6$-alkyl-C(O)$OR^{56}$, or -C(O)$OR^{56}$
- $C_1$-$C_6$-alkyl optionally substituted with one or more substituents independently selected from $R^{58}$
- ArG1, ArG1-$C_1$-$C_6$-alkyl, Het3, or Het3-$C_1$-$C_6$-alkyl

of which the cyclic moieties optionally may be substituted with one or more substituents independently selected from $R^{59}$.
In another embodiment hereof $R^{55}$ is independently selected from halogen, -$OR^{56}$, -$NR^{56}R^{57}$, -C(O)$OR^{56}$, -$OC_1$-$C_8$-alkyl-C(O)$OR^{56}$, -$NR^{56}C(O)R^{57}$ or $C_1$-$C_6$-alkyl.
In another embodiment hereof $R^{55}$ is independently selected from halogen, -$OR^{56}$, -$NR^{56}R^{57}$, -C(O)$OR^{56}$, -$OC_1$-$C_8$-alkyl-C(O)$OR^{56}$, -$NR^{56}C(O)R^{57}$, methyl or ethyl.
In another embodiment hereof $R^{56}$ and $R^{57}$ are independently selected from hydrogen, $CF_3$, $C_1$-$C_{12}$-alkyl, or -C(=O)-$C_1$-$C_6$-alkyl; $R^{56}$ and $R^{57}$ when attached to the same nitrogen atom may form a 3 to 8 membered heterocyclic ring with the said nitrogen atom.
In another embodiment hereof $R^{56}$ and $R^{57}$ are independently selected from hydrogen or $C_1$-$C_{12}$-alkyl, $R^{56}$ and $R^{57}$ when attached to the same nitrogen atom may form a 3 to 8 membered heterocyclic ring with the said nitrogen atom.
In another embodiment hereof $R^{56}$ and $R^{57}$ are independently selected from hydrogen or methyl, ethyl, propyl butyl, $R^{56}$ and $R^{57}$ when attached to the same nitrogen atom may form a 3 to 8 membered heterocyclic ring with the said nitrogen atom.
In another embodiment hereof the zinc-binding ligand is

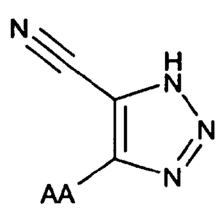

wherein AA is $C_1$-$C_6$-alkyl, aryl, heteroaryl, aryl-$C_{1-6}$-alkyl- or aryl-$C_{2-6}$-alkenyl-, wherein the alkyl or alkenyl is

optionally substituted with one or more substituents independently selected from $R^{63}$, and the aryl or heteroaryl is optionally substituted with one or more substituents independently selected from $R^{64}$,

$R^{63}$ is independently selected from halogen, -CN, $-CF_3$, $-OCF_3$, aryl, -COOH and $-NH_2$,

$R^{64}$ is independently selected from

- hydrogen, halogen, -CN, $-CH_2CN$, $-CHF_2$, $-CF_3$, $-OCF_3$, $-OCHF_2$, $-OCH_2CF_3$, $-OCF_2CHF_2$, $-S(O)_2CF_3$, -OS $(O)_2CF_3$, $-SCF_3$, $-NO_2$, $-OR^{65}$, $-NR^{65}R^{66}$, $-S_R{}^{65}$,- $NR^{65}S(O)_2R^{66}$, $-S(O)_2NR^{65}R^{66}$, $-S(O)NR^{65}R^{66}$, $-S(O)R^{65}$, -S $(O)_2R^{65}$, $-OS(O)_2$ $R^{65}$, $-C(O)NR^{65}R^{66}$, $-OC(O)NR^{65}R^{68}$, $-NR^{65}C(O)R^{66}$, $-CH_2C(O)NR^{65}R^{66}$, $-OC_1-C_6$-alkyl-C $(O)NR^{65}R^{66}$, $-CH_2O^{65}$, $-CH_2OC(O)R^{65}$, $-CH_2NR^{65}R^{66}$, $-OC(O)R^{65}$, $-OC_1-C_6$-alkyl-$C(O)OR^{65}$, $-OC_1-C_6$-alkyl-$OR^{65}$, $-SC_1-C_6$-alkyl-$C(O)OR^{65}$, $-C_2-C_6$-alkenyl-$C(=O)OR^{65}$, $-NR^{65}$-C(=O)-$C_1-C_6$-alkyl-$C(=O)OR^{65}$, $-NR^{65}$-C (=O)-$C_1-C_6$-alkenyl-$C(=O)OR^{65}$, $-C(O)OR^{65}$, or $-C_2-C_6$-alkenyl- C (=O) $R^{65}$,
- $C_1-C_6$-alkyl, $C_2-C_6$-alkenyl or $C_2-C_6$-alkynyl, each of which may optionally be substituted with one or more substituents selected from $R^{67}$,
- aryl, aryloxy, aryloxycarbonyl, aroyl, arylsulfanyl, aryl-$C_1-C_6$-alkoxy, aryl-$C_1-C_6$-alkyl, aryl-$C_2-C_6$-alkenyl, aroyl-$C_2-C_6$-alkenyl, aryl-$C_2-C_6$-alkynyl, heteroaryl, heteroaryl-$C_1-C_6$-alkyl, heteroaryl-$C_2-C_6$-alkenyl or heteroaryl-$C_2-C_6$-alkynyl,

of which the cyclic moieties optionally may be substituted with one or more substituents selected from $R^{68}$,

$R^{65}$ and $R^{66}$ are independently selected from hydrogen, OH, $CF_3$, $C_1-C_{12}$-alkyl, aryl-$C_1-C_6$-alkyl, -C (=O)-$R^{69}$, aryl or heteroaryl, wherein the alkyl groups may optionally be substituted with one or more substituents selected from $R^{70}$, and the aryl and heteroaryl groups may optionally be substituted with one or more substituents independently selected from $R^{71}$ ; $R^{65}$ and $R^{66}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,

$R^{67}$ is independently selected from halogen, -CN, $-CF_3$, $-OCF_3$, $-OR^{65}$, and $-NR^{65}R^{66}$,

$R^{68}$ is independently selected from halogen, $-C(O)OR^{65}$, $-CH_2C(O)OR^{65}$, $-CH_2OR^{65}$, -CN,$-CF_3$, $-OCF_3$, $-NO_2$, $-OR^{65}$, $-NR^{65}R^{66}$ and $C_1-C_6$-alkyl,

$R^{69}$ is independently selected from $C_1-C_6$-alkyl, aryl optionally substituted with one or more halogen, or heteroaryl optionally substituted with one or more $C_1-C_6$-alkyl,

$R^{70}$ is independently selected from halogen, -CN, $-CF_3$, $-OCF_3$, $-OC_1-C_6$-alkyl, $-C(O)OC_1-C_6$-alkyl, -COOH and $-NH_2$,

$R^{71}$ is independently selected from halogen, $-C(O)OC_1-C_6$-alkyl, -COOH, -CN, $-CF_3$, $-OCF_3$, - $NO_2$, -OH, $-OC_1-C_6$-alkyl, $-NH_2$, C(=O) or $C_1-C_6$-alkyl,

or any enantiomer, diastereomer, including a racemic mixture, tautomer as well as a salt thereof with a pharmaceutically acceptable acid or base.

In another embodiment hereof AA is aryl, heteroaryl or aryl-$C_{1-6}$-alkyl-, wherein the alkyl is optionally substituted with one or more $R^{63}$, and the aryl or heteroaryl is optionally substituted with one or more substituents independently selected from $R^{64}$.

In another embodiment hereof AA is aryl or heteroaryl optionally substituted with one or more substituents independently selected from $R^{64}$.

In another embodiment hereof AA is ArG1 or Het1 optionally substituted with one or more substituents independently selected from $R^{64}$.

In another embodiment hereof AA is ArG1 or Het2 optionally substituted with one or more substituents independently selected from $R^{64}$.

In another embodiment hereof AA is ArG1 or Het3 optionally substituted with one or more substituents independently selected from $R^{64}$.

In another embodiment hereof AA is phenyl, naphtyl, anthryl, carbazolyl, thienyl, pyridyl, or benzodioxyl optionally substituted with one or more substituents independently selected from $R^{64}$.

In another embodiment hereof AA is phenyl or naphtyl optionally substituted with one or more substituents independently selected from $R^{64}$.
In another embodiment hereof $R^{64}$ is independently selected from hydrogen, halogen, $-CF_3$, $-OCF_3$, $-OR^{65}$, $-NR^{65}R^{66}$, $C_1$-$C_6$-alkyl , $-OC(O)R^{65}$, $-OC_1$-$C_6$-alkyl-$C(O)OR^{65}$ , aryl-$C_2$-$C_6$-alkenyl, aryloxy or aryl, wherein $C_1$-$C_6$-alkyl is optionally substituted with one or more substituents independently selected from $R^{67}$, and the cyclic moieties optionally are substituted with one or more substituents independently selected from $R^{68}$.
In another embodiment hereof $R^{64}$ is independently selected from halogen, $-CF_3$, $-OCF_3$, $-OR^{65}$, $-NR^{65}R^{66}$, methyl, ethyl, propyl, $-OC(O)R^{65}$, $-OCH_2$-$C(O)OR^{65}$, $-OCH_2$-$CH_2$-$C(O)OR^{65}$, phenoxy optionally substituted with one or more substituents independently selected from $R^{68}$.
In another embodiment hereof $R^{65}$ and $R^{66}$ are independently selected from hydrogen, $CF_3$, $C_1$-$C_{12}$-alkyl, aryl, or heteroaryl optionally substituted with one or more substituents independently selected from $R^{71}$.
In another embodiment hereof $R^{65}$ and $R^{66}$ are independently hydrogen, $C_1$-$C_{12}$-alkyl, aryl, or heteroaryl optionally substituted with one or more substituents independently selected from $R^{71}$.
In another embodiment hereof $R^{65}$ and $R^{66}$ are independently hydrogen, methyl, ethyl, propyl, butyl, 2,2-dimethyl-propyl, ArG1 or Het1 optionally substituted with one or more substituents independently selected from $R^{71}$.
In another embodiment hereof $R^{65}$ and $R^{66}$ are independently hydrogen, methyl, ethyl, propyl, butyl, 2,2-dimethyl-propyl, ArG1 or Het2 optionally substituted with one or more substituents independently selected from $R^{71}$.
In another embodiment hereof $R^{65}$ and $R^{66}$ are independently hydrogen, methyl, ethyl, propyl, butyl, 2,2-dimethyl-propyl, ArG1 or Het3 optionally substituted with one or more substituents independently selected from $R^{71}$.
In another embodiment hereof $R^{65}$ and $R^{66}$ are independently hydrogen, methyl, ethyl, propyl, butyl, 2,2-dimethyl-propyl, phenyl, naphtyl, thiadiazolyl optionally substituted with one or more $R^{71}$ independently; or isoxazolyl optionally substituted with one or more substituents independently selected from $R^{71}$.
In another embodiment hereof $R^{71}$ is halogen or $C_1$-$C_6$-alkyl.
In another embodiment hereof $R^{71}$ is halogen or methyl.

The following aspects are also provided by the present invention, wherein the compounds of the invention may be any of the above described embodiments.

In one aspect the invention provides a pharmaceutical composition wherein the insulin is rapid acting insulin.
In another embodiment the invention provides a pharmaceutical composition wherein the insulin is selected from the group consisting of human insulin, an analogue thereof, a derivative thereof, and combinations of any of these.
In another embodiment the invention provides a pharmaceutical composition wherein the insulin is an analogue of human insulin selected from the group consisting of

i. An analogue wherein position B28 is Asp, Lys, Leu, Val, or Ala and position B29 is Lys or Pro; and
ii. des(B28-B30), des(B27) or des(B30) human insulin.

In another embodiment the invention provides a pharmaceutical composition wherein the insulin is an analogue of human insulin wherein position B28 is Asp or Lys, and position B29 is Lys or Pro.
In another embodiment the invention provides a pharmaceutical composition wherein the insulin is des(B30) human insulin.
In another embodiment the invention provides a pharmaceutical composition wherein the insulin is is an analogue of human insulin wherein position B3 is Lys and position B29 is Glu or Asp.
In another embodiment the invention provides a pharmaceutical composition wherein the insulin is a derivative of human insulin having one or more lipophilic substituents.
In another embodiment the invention provides a pharmaceutical composition wherein the insulin derivative is selected from the group consisting of B29-$N^{\varepsilon}$-myristoyl-des(B30) human insulin, B29-$N^{\varepsilon}$-palmitoyl-des(B30) human insulin, B29-$N^{\varepsilon}$-myristoyl human insulin, B29-$N^{\varepsilon}$-palmitoyl human insulin, B28-$N^{\varepsilon}$-myristoyl $Lys^{B28}$ $Pro^{B29}$ human insulin, B28-$N^{\varepsilon}$-palmitoyl $Lys^{B28}$ $Pro^{B29}$ human insulin, B30-$N^{\varepsilon}$-myristoyl-$Thr^{B29}Lys^{B30}$ human insulin, B30-$N^{\varepsilon}$-palmitoyl-$Th^{B29}Lys^{B30}$ human insulin, B29-$N^{\varepsilon}$-(N-palmitoyl-$\gamma$-glutamyl)-des(B30) human insulin, B29-$N^{\varepsilon}$-(N-lithocholyl-$\gamma$-glutamyl)-des(B30) human insulin, B29-$N^{\varepsilon}$-($\omega$-carboxyheptadecanoyl)-des(B30) human insulin and B29-$N^{\varepsilon}$-($\omega$-carboxyheptadecanoyl) human insulin.
In another embodiment the invention provides a pharmaceutical composition wherein the insulin derivative is B29-$N^{\varepsilon}$-myristoyl-des(B30) human insulin.
In another embodiment the invention provides a pharmaceutical composition comprising 2-6 moles $zinc^{2+}$ ions per mole insulin.
In another embodiment the invention provides a pharmaceutical composition comprising 2-3 moles $zinc^{2+}$ ions per mole insulin.

In another embodiment the invention provides a pharmaceutical composition further comprising at least 3 molecules of a phenolic compound per insulin hexamer.

In another embodiment the invention provides a pharmaceutical composition further comprising an isotonicity agent.
In another embodiment the invention provides a pharmaceutical composition further comprising a buffer substance.
A method of stabilising an insulin composition comprising adding a zinc-binding ligand to the insulin composition.

The claimed compositions can be used in a method of treating type 1 or type 2 diabetes comprising administering to a patient in need thereof a pharmaceutically effective dose of an insulin composition.

In one embodiment of the invention the concentration of added ligand for the zinc site is between 0.2 and 10 times that of zinc ion in the preparation. In another embodiment the concentration is between 0.5 and 5 times that of zinc ion. In another embodiment the ligand concentration is identical to that of zinc ion in the preparation.

The compounds of the present invention may be chiral, and it is intended that any enantiomers, as separated, pure or partially purified enantiomers or racemic mixtures thereof are included within the scope of the invention.
Furthermore, when a double bond or a fully or partially saturated ring system or more than one centre of asymmetry or a bond with restricted rotatability is present in the molecule diastereomers may be formed. It is intended that any diastereomers, as separated, pure or partially purified diastereomers or mixtures thereof are included within the scope of the invention.
Furthermore, some of the compounds of the present invention may exist in different tautomeric forms and it is intended that any tautomeric forms, which the compounds are able to form, are included within the scope of the present invention.
The present invention also encompasses pharmaceutically acceptable salts of the present compounds. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulphuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, picric, pyruvic, succinic, methanesulfonic, ethanesulfonic, tartaric, ascorbic, pamoic, , ethanedisulfonic, gluconic, citraconic, aspartic, stearic, palmitic, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium salts and the like. Examples of ammonium and alkylated ammonium salts include ammonium, methyl-, dimethyl-, trimethyl-, ethyl-, hydroxyethyl-, diethyl-, n-butyl-, *sec*-butyl-, *tert*-butyl-, tetramethylammonium salts and the like.
Also intended as pharmaceutically acceptable acid addition salts are the hydrates, which the present compounds, are able to form.
Furthermore, the pharmaceutically acceptable salts comprise basic amino acid salts such as lysine, arginine and ornithine.
The acid addition salts may be obtained as the direct products of compound synthesis. In the alternative, the free base may be dissolved in a suitable solvent containing the appropriate acid, and the salt isolated by evaporating the solvent or otherwise separating the salt and solvent.
The compounds of the present invention may form solvates with standard low molecular weight solvents using methods well known to the person skilled in the art. Such solvates are also contemplated as being within the scope of the present invention.

In one embodiment of the invention the stabilized preparations are used in connection with insulin pumps. The insulin pumps may be prefilled and disposable, or the insulin compositions may be supplied from a reservoir which is removable. Insulin pumps may be skin-mounted or carried, and the path of the insulin composition from the storage compartment of the pump to the patient may be more or less tortuous. The elevated temperature and increased physical stress the insulin composition is thus exposed to challenges the stability of the constituent insulin. Non-limiting examples of insulin pumps are disclosed in US 5,957,895, US 5,858,001, US 4,468,221, US 4,468,221, US 5,957,895, US 5,858,001, US 6,074,369, US 5,858,001, US 5,527,288, and US 6,074,369.

In another embodiment the stabilized preparations are used in connection with pen-like injection devices, which may be prefilled and disposable, or the insulin compositions may be supplied from a reservoir which is removable. Non-limiting examples of pen-like injection devices are FlexPen®, InnoLet®, InDuo™, Innovo®.

In a further embodiment stabilized preparations are used in connection with devices for pulmonary administration of aqueous insulin compositions, a non-limiting example of which is the AerX® device.

In one aspect of the invention, the ligands are added to rapid acting insulin. The resulting preparations have improved physical and chemical stability while still retaining a high rate of absorbtion from subcutaneous tissue.

The present invention also relates to pharmaceutical compositions for the treatment of diabetes in a patient in need of such a treatment comprising an R-state hexamer of insulin according to the invention together with a pharmaceutically acceptable carrier.
In one embodiment of the invention the insulin composition comprises 60 to 3000 nmol/ml of insulin.
In another embodiment of the invention the insulin composition comprises 240 to 1200 nmol/ml of insulin.

In another embodiment of the invention the insulin composition comprises about 600 nmol/ml of insulin.
Zinc ions may be present in an amount corresponding to 13 to 33 μg Zn/100 U insulin, more preferably 15 to 26 μg Zn/100 U insulin.
Insulin formulations of the invention are usually administered from multi-dose containers where a preservative effect is desired. Since phenolic preservatives also stabilize the R-state hexamer the formulations may contain up to 50 mM of phenolic molecules. Non-limiting examples of phenolic molecules are phenol, m-cresol, chloro-cresol, thymol, 7-hydroxyindole or any mixture thereof.
In one embodiment of the invention 0.5 to 4.0 mg/ml of phenolic compound may be employed. In another embodiment of the invention 0.6 to 4.0 mg/ml of m-cresol may be employed.
In another embodiment of the invention 0.5 to 4.0 mg/ml of phenol may be employed.
In another embodiment of the invention 1.4 to 4.0 mg/ml of phenol may be employed.
In another embodiment of the invention 0.5 to 4.0 mg/ml of a mixture of m-cresol or phenol may be employed.
In another embodiment of the invention 1.4 to 4.0 mg/ml of a mixture of m-cresol or phenol may be employed.
The pharmaceutical composition may further comprise a buffer substance, such as a TRIS, phosphate, glycine or glycylglycine (or another zwitterionic substance) buffer, an isotonicity agent, such as NaCl, glycerol, mannitol and/or lactose. Chloride would be used at moderate concentrations, in one embodiment of the invention up to 50 mM to avoid competition with the zinc-site ligands of the present invention. In another embodiment the chloride concentration would be from 3 to 20 mM.
The *in vivo* action of insulin may be modified by the addition of physiologically acceptable agents that increase the viscosity of the pharmaceutical composition. Thus, the pharmaceutical composition according to the invention may furthermore comprise an agent which increases the viscosity, such as polyethylene glycol, polypropylene glycol, copolymers thereof, dextrans and/or polylactides.

In one embodiment the insulin composition of the invention comprises between 0.0005 % by weight and 1 % by weight of a non-ionic or zwitter-ionic surfactant, for example tween 20 or Polox 188. A nonionic detergent can be added to stabilise insulin against fibrillation during storage and handling.
The insulin composition of the present invention may have a pH value in the range of 3.0 to 8.5, e.g. 7.4 to 7.9.

**EXAMPLES**

The following examples and general procedures refer to intermediate compounds and final products identified in the specification and in the synthesis schemes. The preparation of the compounds of the present invention is described in detail using the following examples, but the chemical reactions described are disclosed in terms of their general applicability to the preparation of compounds of the invention. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. All temperatures are set forth in degrees Celsius and unless otherwise indicated, all parts and percentages are by weight when referring to yields and all parts are by volume when referring to solvents and eluents.

**HPLC-MS (Method A)**

The following instrumentation was used:

- Hewlett Packard series 1100 G1312A Bin Pump
- Hewlett Packard series 1100 Column compartment
- Hewlett Packard series 1100 G13 15A DAD diode array detector
- Hewlett Packard series 1100 MSD

The instrument was controlled by HP Chemstation software.
The HPLC pump was connected to two eluent reservoirs containing:

A: 0.01% TFA in water
B: 0.01% TFA in acetonitrile

The analysis was performed at 40 °C by injecting an appropriate volume of the sample (preferably 1 μL) onto

the column, which was eluted with a gradient of acetonitrile.
The HPLC conditions, detector settings and mass spectrometer settings used are given in the following table.

| | |
|---|---|
| Column | Waters Xterra MS C-18 X 3 mm id |
| Gradient | 10% - 100% acetonitrile lineary during 7.5 min at 1.0 mL/min |
| Detection | UV: 210 nm (analog output from DAD) |
| MS | Ionisation mode: API-ES<br>Scan 100-1000 amu step 0.1 amu |

**HPLC-MS (Method B)**

The following instrumentation was used:

Sciex API 100 Single quadropole mass spectrometer
Perkin Elmer Series 200 Quard pump
Perkin Elmer Series 200 autosampler
Applied Biosystems 785A UV detector
Sedex 55 evaporative light scattering detector
A Valco column switch with a Valco actuator controlled by timed events from the pump.

The Sciex Sample control software running on a Macintosh PowerPC 7200 computer was used for the instrument control and data acquisition.
The HPLC pump was connected to four eluent reservoirs containing:

A: Acetonitrile
B: Water
C: 0.5% TFA in water
D: 0.02 M ammonium acetate

The requirements for samples are that they contain approximately 500 μg/mL of the compound to be analysed in an acceptable solvent such as methanol, ethanol, acetonitrile, THF, water and mixtures thereof. (High concentrations of strongly eluting solvents will interfere with the chromatography at low acetonitrile concentrations.)
The analysis was performed at room temperature by injecting 20 μL of the sample solution on the column, which was eluted with a gradient of acetonitrile in either 0.05% TFA or 0.002 M ammonium acetate. Depending on the analysis method varying elution conditions were used.
The eluate from the column was passed through a flow splitting T-connector, which passed approximately 20 μL/min through approx. 1 m. 75 μ fused silica capillary to the API interface of API 100 spectrometer.
The remaining 1.48 mL/min was passed through the UV detector and to the ELS detector.
During the LC-analysis the detection data were acquired concurrently from the mass spectrometer, the UV detector and the ELS detector.
The LC conditions, detector settings and mass spectrometer settings used for the different methods are given in the following table.

| | | |
|---|---|---|
| Column | YMC ODS-A 120Å s - 5μ 3 mm x 50 mm id | |
| Gradient | 5% - 90% acetonitrile in 0.05% TFA linearly during 7.5 min at 1.5 mL/min | |
| Detection | UV: 214 nm | ELS: 40 °C |
| MS | Experiment: Start: 100 amu Stop: 800 amu Step: 0.2 amu<br>Dwell: 0.571 msec<br>Method: Scan 284 times = 9.5 min | |

**HPLC-MS (Method C)** The following instrumentation is used:

- Hewlett Packard series 1100 G1312A Bin Pump

- Hewlett Packard series 1100 Column compartment
- Hewlett Packard series 1100 G1315A DAD diode array detector
- Hewlett Packard series 1100 MSD
- Sedere 75 Evaporative Light Scattering detector

The instrument is controlled by HP Chemstation software.
The HPLC pump is connected to two eluent reservoirs containing:

| | |
|---|---|
| A | 0.01% TFA in water |
| B | 0.01% TFA in acetonitrile |

The analysis is performed at 40 °C by injecting an appropriate volume of the sample (preferably 1 μl) onto the column which is eluted with a gradient of acetonitrile.
The HPLC conditions, detector settings and mass spectrometer settings used are given in the following table.

| | |
|---|---|
| Column | Waters Xterra MS C-18 X 3 mm id 5 μm |
| Gradient | 5%- 100% acetonitrile linear during 7.5 min at 1.5 ml/min |
| Detection | 210 nm (analogue output from DAD)<br>ELS (analogue output from ELS) |
| MS | ionisation mode API-ES Scan 100-1000 amu step 0.1 amu |

After the DAD the flow is divided yielding approximately 1 ml/min to the ELS and 0.5 ml/min to the MS.

**HPLC-MS (Method D)**

The following instrumentation was used:

Sciex API 150 Single Quadropole mass spectrometer
Hewlett Packard Series 1100 G1312A Bin pump
Gilson 215 micro injector
Hewlett Packard Series 1100 G1315A DAD diode array detector
Sedex 55 evaporative light scattering detector
A Valco column switch with a Valco actuator controlled by timed events from the pump.
The Sciex Sample control software running on a Macintosh Power G3 computer was used for the instrument control and data acquisition.

The HPLC pump was connected to two eluent reservoirs containing:

A: Acetonitrile containing 0.05% TFA
B: Water containing 0.05% TFA

The requirements for the samples are that they contain approximately 500 μg/ml of the compound to be analysed in an acceptable solvent such as methanol, ethanol, acetonitrile, THF, water and mixtures thereof. (High concentrations of strongly eluting solvents will interfere with the chromatography at low acetonitrile concentrations.)
The analysis was performed at room temperature by injecting 20 μl of the sample solution on the column, which was eluted with a gradient of acetonitrile in 0.05% TFA
The eluate from the column was passed through a flow splitting T-connector, which passed approximately 20 μl/min through approx. 1 m 75 μ fused silica capillary to the API interface of API 150 spectrometer.
The remaining 1.48 ml/min was passed through the UV detector and to the ELS detector. During the LC-analysis the detection data were acquired concurrently from the mass spectrometer, the UV detector and the ELS detector.
The LC conditions, detector settings and mass spectrometer settings used for the different methods are given in the following table.

| | |
|---|---|
| Column | Waters X-terra C18 5μ 3 mm x 50 mm id |

(continued)

| | | |
|---|---|---|
| Gradient | 5% - 90% acetonitrile in 0.05% TFA linearly during 7.5 min at 1.5 ml/min | |
| Detection | UV: 214 nm | ELS: 40 °C |
| MS | Experiment: Start: 100 amu Stop: 800 amu Step: 0.2 amu<br>Dwell: 0.571 msec<br>Method: Scan 284 times = 9.5 min | |

**EXAMPLES**

0102-0000-0273Example 1 HBOL

1*H*-Benzotriazole

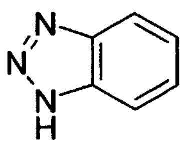

0102-0000-0274Example 2 HBOL

5,6-Dimethyl-1*H*-benzotriazole

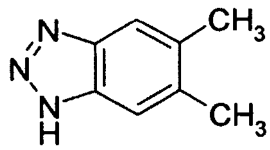

0102-0000-0275Example 3 HBOL

1*H*-Benzotriazole-5-carboxylic acid

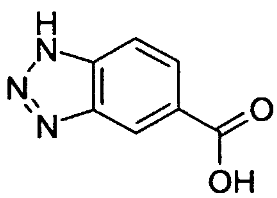

0102-0000-0280 Example 4 HBOL

4-Nitro-1*H*-benzotriazole

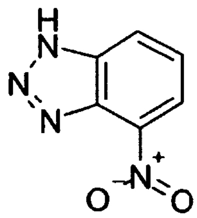

0102-0000-0284Example 5 HBOL

5-Amino-1*H*-benzotriazole

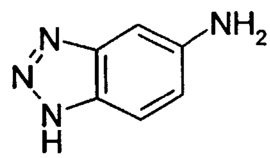

0102-0000-0287Example 6 HBOL

5-Chloro-1*H*-benzotriazole

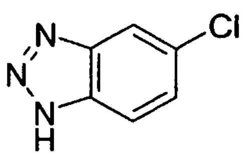

0102-0000-0286Example 7 HBOL

5-Nitro-1*H*-benzotriazole

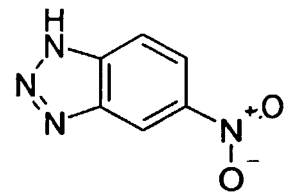

0102-0000-3015Example 8 PEM

4-[(1*H*-Benzotriazole-5-carbonyl)amino]benzoic acid

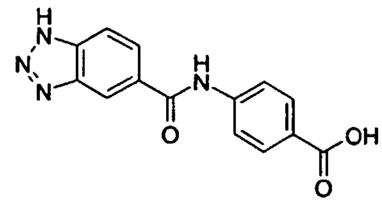

4-[(1*H*-Benzotriazole-5-carbonyl)amino]benzoic acid methyl ester (5.2 g, 17.6 mmol) was dissolved in THF (60 mL) and methanol (10 mL) was added followed by 1N sodium hydroxide (35 mL). The mixture was stirred at room temperature for 16 hours and then 1N hydrochloric acid (45 mL) was added. The mixture was added water (200 mL) and extracted with ethyl acetate (2 x 500 mL). The combined organic phases were evaporated *in vacuo* to afford 0.44 g of 4-[(1*H*-benzotriazole-5-carbonyl)amino]benzoic acid. By filtration of the aqueous phase a further crop of 4-[(1*H*-benzotriazole-5-carbonyl)amino]benzoic acid was isolated (0.52 g).
$^1$H-NMR (DMSO-$d_6$): δ 7.97 (4H, s), 8.03 (2H, m), 8.66 (1H, bs), 10.7 (1H, s), 12.6 (1H, bs); HPLC-MS (Method A): m/z: 283 (M+1); Rt = 1.85 min.

**General procedure (A) for preparation of compounds of general formula $I_1$:**

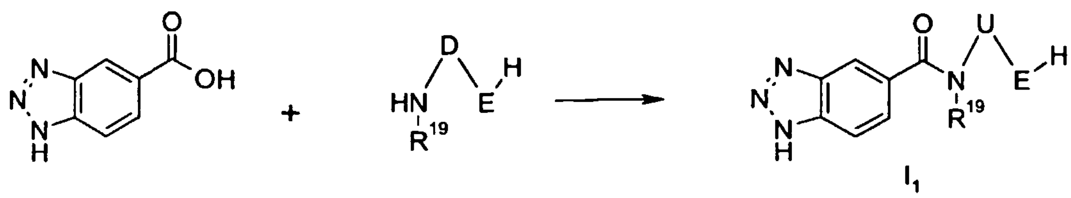

$I_1$

wherein D, E and $R^{19}$ are as defined above, and E is optionally substituted with up to three substituents $R^{21}$, $R^{22}$ and $R^{23}$ independently as defined above.

The carboxylic acid of 1H-benzotriazole-5-carboxylic acid is activated, ie the OH functionality is converted into a leaving group L (selected from eg fluorine, chlorine, bromine, iodine, 1-imidazolyl, 1,2,4-triazolyl, 1-benzotriazolyloxy, 1-(4-aza benzotriazolyl)oxy, pentafluorophenoxy, N-succinyloxy 3,4-dihydro-4-oxo-3-(1,2,3-benzotriazinyl)oxy, benzotriazole 5-COO, or any other leaving group known to act as a leaving group in acylation reactions. The activated benzotriazole-5-carboxylic acid is then reacted with $R^2$-$(CH_2)_n$-B' in the presence of a base. The base can be either absent (i.e. $R^2$-$(CH_2)_n$-B' acts as a base) or triethylamine, N-ethyl-N,N.-diisopropylamine, N-methylmorpholine, 2,6-lutidine, 2,2,6,6-tetramethylpiperidine, potassium carbonate, sodium carbonate, caesium carbonate or any other base known to be useful in acylation reactions. The reaction is performed in a solvent solvent such as THF, dioxane, toluene, dichloromethane, DMF, NMP or a mixture of two or more of these. The reaction is performed between 0 ˚C and 80 ˚C, preferably between 20 ˚C and 40 ˚C. When the acylation is complete, the product is isolated by extraction, filtration, chromatography or other methods known to those skilled in the art.

The general procedure (A) is further illustrated in the following example:

0102-0000-1020Example 9 (General Procedure (A))PEM

1*H*-Benzotriazole-5-carboxylic acid phenylamide

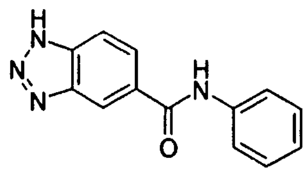

Benzotriazole-5-carboxylic acid (856 mg), HOAt (715 mg) and EDAC (1.00 g) were dissolved in DMF (17.5 mL) and the mixture was stirred at room temperature 1 hour. A 0.5 mL aliqot of this mixture was added to aniline (13.7 μL, 0.15 mmol) and the resulting mixture was vigorously shaken at room temperature for 16 hours. 1N hydrochloric acid (2 mL) and ethyl acetate (1 mL) were added and the mixture was vigorously shaken at room temperature for 2 hours. The organic phase was isolated and concentrated *in vacuo* to afford the title compound.
HPLC-MS (Method B): m/z: 239 (M+1); Rt = 3.93 min.
The compounds in the following examples were similarly made. Optionally, the compounds may be isolated by filtration or by chromatography.

0102-0000-1019Example 10 (General Procedure (A))PEM

1*H*-Benzotriazole-5-carboxylic acid (4-methoxyphenyl)amide

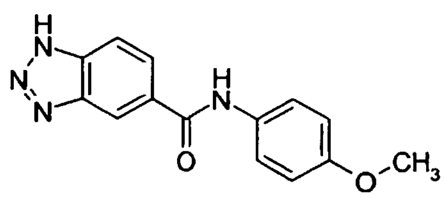

HPLC-MS (Method A): m/z: 269 (M+1) & 291 (M+23); Rt = 2.41 min
HPLC-MS (Method B): m/z: 239 (M+1); Rt = 3.93 min.

0102-0000-1021 Example 11 (General Procedure (A))PEM

{4-[(1*H*-Benzotriazole-5-carbonyl)amino]phenyl}carbamic acid *tert*-butyl ester

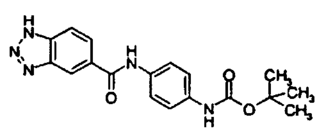

HPLC-MS (Method B): m/z: 354 (M+1); Rt = 4.58 min.

0102-0000-1022Example 12 (General Procedure (A))PEM

1*H*-Benzotriazole-5-carboxylic acid (4-acetylaminophenyl)amide

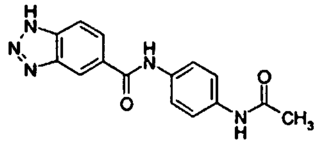

HPLC-MS (Method B): m/z: 296 (M+1); Rt = 3.32 min.

0102-0000-1023Example 13 (General Procedure (A))PEM

1*H*-Benzotriazole-5-carboxylic acid (3-fluorophenyl)amide

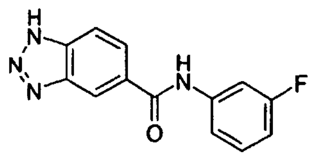

HPLC-MS (Method B): m/z: 257 (M+1); Rt = 4.33 min.

0102-0000-1024Example 14 (General Procedure (A))PEM

1*H*-Benzotriazole-5-carboxylic acid (2-chlorophenyl)amide

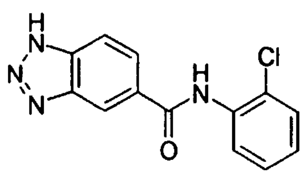

HPLC-MS (Method B): m/z: 273 (M+1); Rt = 4.18 min.

0102-0000-1025Example 15 (General Procedure (A))PEM

4-[(1*H*-Benzotriazole-5-carbonyl)amino]benzoic acid methyl ester

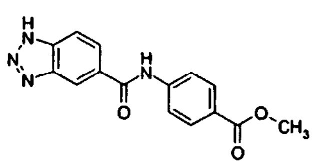

HPLC-MS (Method A):m/z: 297 (M+1); Rt : 2,60 min. HPLC-MS (Method B): m/z: 297 (M+1); Rt = 4.30 min.

0102-0000-1026Example 16 (General Procedure (A))PEM

1*H*-Benzotriazole-5-carboxylic acid (4-butylphenyl)amide

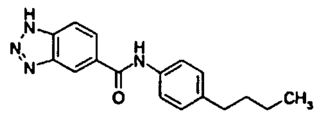

HPLC-MS (Method B): m/z: 295 (M+1); Rt = 5.80 min.

0102-0000-1027Example 17 (General Procedure (A))PEM

1*H*-Benzotriazole-5-carboxylic acid (1-phenylethyl)amide

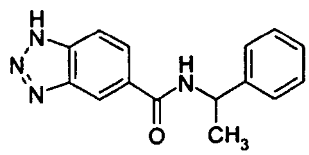

HPLC-MS (Method B): m/z: 267 (M+1); Rt = 4.08 min.

0102-0000-1028Example 18 (General Procedure (A))PEM

1*H*-Benzotriazole-5-carboxylic acid benzylamide

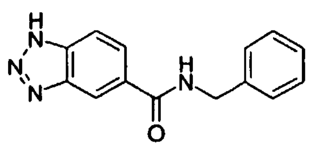

HPLC-MS (Method B): m/z: 253 (M+1); Rt = 3.88 min.

0102-0000-1029Example 19 (General Procedure (A))PEM

1*H*-Benzotriazole-5-carboxylic acid 4-chlorobenzylamide

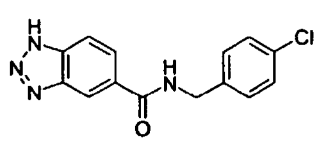

HPLC-MS (Method B): m/z: 287 (M+1); Rt = 4.40 min.

0102-0000-1030Example 20 (General Procedure (A))PEM

1*H*-Benzotriazole-5-carboxylic acid 2-chlorobenzylamide

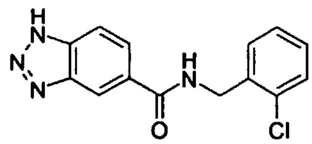

HPLC-MS (Method B): m/z: 287 (M+1); Rt = 4.25 min.

0102-0000-1031 Example 21 (General Procedure (A))PEM

1*H*-Benzotriazole-5-carboxylic acid 4-methoxybenzylamide

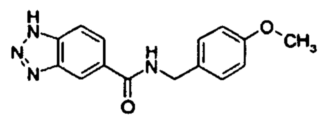

HPLC-MS (Method B): m/z: 283 (M+1); Rt = 3.93 min.

0102-0000-1032Example 22 (General Procedure (A))PEM

1*H*-Benzotriazole-5-carboxylic acid 3-methoxybenzylamide

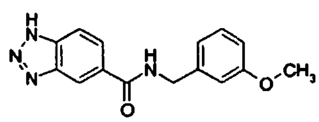

HPLC-MS (Method B): m/z: 283 (M+1); Rt = 3.97 min.

0102-0000-1033Example 23 (General Procedure (A))PEM

1*H*-Benzotriazole-5-carboxylic acid (1,2-diphenylethyl)amide

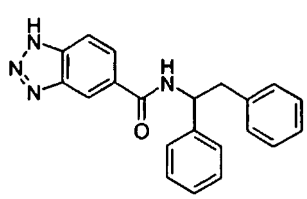

HPLC-MS (Method B): m/z: 343 (M+1); Rt = 5.05 min.

0102-0000-1034Example 24 (General Procedure (A))PEM

1*H*-Benzotriazole-5-carboxylic acid 3-bromobenzylamide

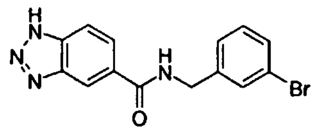

HPLC-MS (Method B): m/z: 331 (M+1); Rt = 4.45 min.

0102-0000-1035Example 25 (General Procedure (A))PEM

4-{[(1*H*-Benzotriazole-5-carbonyl)amino]methyl}benzoic acid

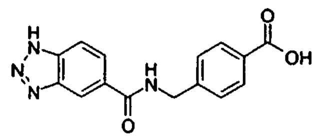

HPLC-MS (Method B): m/z: 297 (M+1); Rt = 3.35 min.

0102-0000-1036Example 26 (General Procedure (A))PEM

1*H*-Benzotriazole-5-carboxylic acid phenethylamide

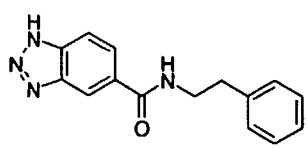

HPLC-MS (Method B): m/z: 267 (M+1); Rt = 4.08 min.

0102-0000-1037Example 27 (General Procedure (A))PEM

1*H*-Benzotriazole-5-carboxylic acid [2-(4-chlorophenyl)ethyl]amide

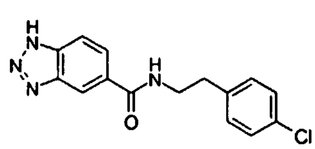

HPLC-MS (Method B): m/z: 301 (M+1); Rt = 4.50 min.

0102-0000-1038Example 28 (General Procedure (A))PEM

1*H*-Benzotriazole-5-carboxylic acid [2-(4-methoxyphenyl)ethyl]amide

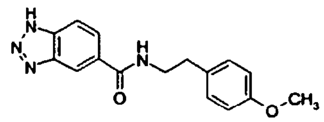

HPLC-MS (Method B): m/z: 297 (M+1); Rt = 4.15 min.

0102-0000-1039 Example 29 (General Procedure (A))PEM

1*H*-Benzotriazole-5-carboxylic acid [2-(3-methoxyphenyl)ethyl]amide

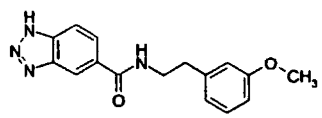

HPLC-MS (Method B): m/z: 297 (M+1); Rt = 4.13 min.

0102-0000-1040Example 30 (General Procedure (A))PEM

1*H*-Benzotriazole-5-carboxylic acid [2-(3-chlorophenyl)ethyl]amide

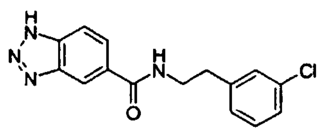

HPLC-MS (Method B): m/z: 301 (M+1); Rt = 4.55 min.

0102-0000-1041 Example 31 (General Procedure (A))PEM

1*H*-Benzotriazole-5-carboxylic acid (2,2-diphenylethyl)amide

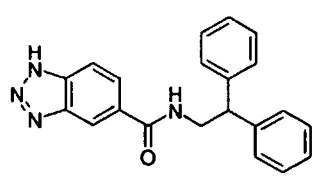

HPLC-MS (Method B): m/z: 343 (M+1); Rt = 5.00 min.

0102-0000-1042Example 32 (General Procedure (A))PEM

1*H*-Benzotriazole-5-carboxylic acid (3,4-dichlorophenyl)methylamide

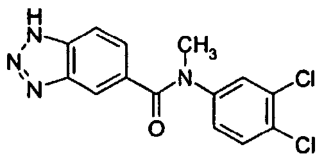

HPLC-MS (Method B): m/z: 321 (M+1); Rt = 4.67 min.

0102-0000-1043Example 33 (General Procedure (A))PEM

1*H*-Benzotriazole-5-carboxylic acid methylphenylamide

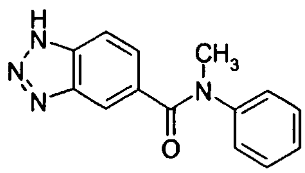

HPLC-MS (Method B): m/z: 253 (M+1); Rt = 3.82 min.

0102-0000-1044Example 34 (General Procedure (A))PEM

1*H*-Benzotriazole-5-carboxylic acid benzylmethylamide

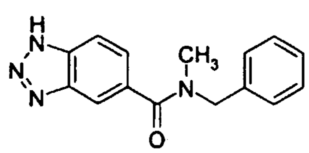

HPLC-MS (Method B): m/z: 267 (M+1); Rt = 4.05 min.

0102-0000-1045Example 35 (General Procedure (A))PEM

1*H*-Benzotriazole-5-carboxylic acid [2-(3-chloro-4-methoxyphenyl)ethyl]methyl-amide

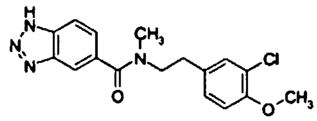

HPLC-MS (Method B): m/z: 345 (M+1); Rt = 4.37 min.

0102-0000-1046Example 36 (General Procedure (A))PEM

1*H*-Benzotriazole-5-carboxylic acid methylphenethylamide

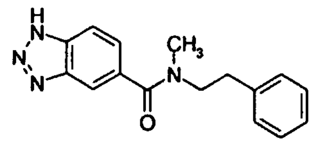

HPLC-MS (Method B): m/z: 281 (M+1); Rt = 4.15 min.

0102-0000-1047Example 37 (General Procedure (A))PEM

1*H*-Benzotriazole-5-carboxylic acid [2-(3,4-dimethoxyphenyl)ethyl]methylamide

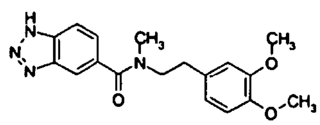

HPLC-MS (Method B): m/z: 341 (M+1); Rt = 3.78 min;

0102-0000-1048Example 38 (General Procedure (A))PEM

1*H*-Benzotriazole-5-carboxylic acid (2-hydroxy-2-phenylethyl)methylamide

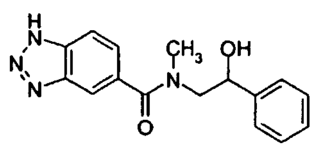

HPLC-MS (Method B): m/z: 297 (M+1); Rt = 3.48 min.

Example 39 (General procedure (A))

1*H*-Benzotriazole-5-carboxylic acid (3-bromophenyl)amide

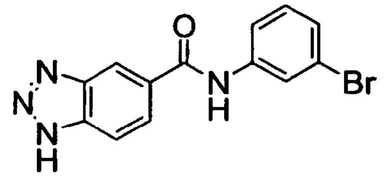

HPLC-MS (Method A): m/z: 317 (M+1); Rt = 3.19 min.

Example 40 (General procedure (A))

1*H*-Benzotriazole-5-carboxylic acid (4-bromophenyl)amide

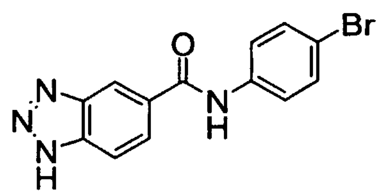

HPLC-MS (Method A): m/z: 317 (M+1); Rt = 3.18 min.

Example 41 (General procedure (A))

{4-[(1H-Benzotriazole-5-carbonyl)amino]benzoylamino}acetic acid

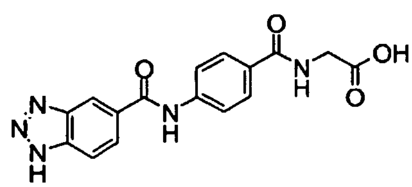

HPLC-MS (Method A): m/z: 340 (M+1); Rt = 1.71 min.

Example 42 (General procedure (A))

{4-[(1H-Benzotriazole-5-carbonyl)amino]phenyl}acetic acid

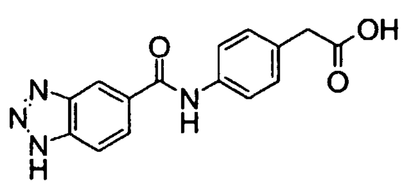

HPLC-MS (Method A): m/z: 297 (M+1); Rt = 2.02 min.

Example 43 (General procedure (A))

3-{4-[(1H-Benzotriazole-5-carbonyl)amino]phenyl}acrylic acid

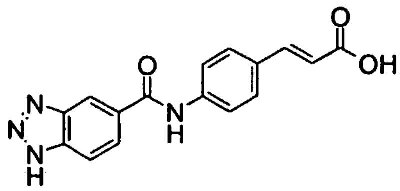

HPLC-MS (Method A): m/z: 309 (M+1); Rt = 3.19 min.

Example 44 (General procedure (A))

{3-[(1H-Benzotriazole-5-carbonyl)amino]phenyl}acetic acid

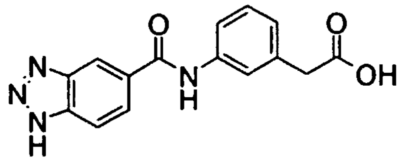

HPLC-MS (Method A): m/z: 297 (M+1); Rt = 2.10 min.

Example 45 (General procedure (A))

2-{4-[(1H-Benzotriazole-5-carbonyl)amino]phenoxy}-2-methylpropionic acid

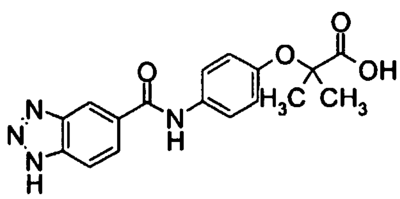

HPLC-MS (Method A): m/z: 341 (M+1); Rt = 2.42 min.

Example 46 (General procedure (A))

3-{4-[(1H-Benzotriazole-5-carbonyl)amino]benzoylamino}propionic acid

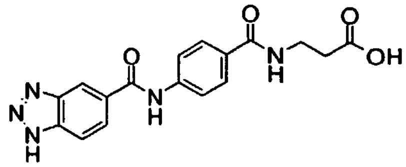

HPLC-MS (Method A): m/z: 354 (M+1); Rt = 1.78 min.

Example 47 (General procedure (A))

3-{4-[(1H-Benzotriazole-5-carbonyl)amino]phenyt}propionic acid

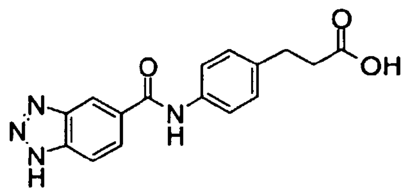

HPLC-MS (Method A): m/z: 311 (M+1); Rt = 2.20 min.

Example 48 (General procedure (A))

1H-Benzotriazole-5-carboxylic acid (4-benzyloxyphenyl)amide

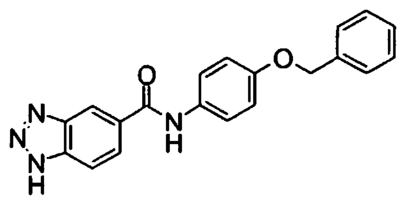

HPLC-MS (Method A): m/z: 345 (M+1); Rt = 3.60 min.

Example 49 (General procedure (A))

1H-Benzotriazole-5-carboxylic acid (3-chloro-4-methoxyphenyl)amide

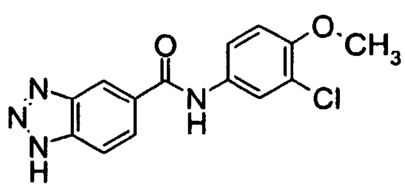

HPLC-MS (Method A): m/z: 303 (M+1); Rt = 2.88 min.

Example 50 (General procedure (A))

1H-Benzotriazole-5-carboxylic acid (4-phenoxyphenyl)amide

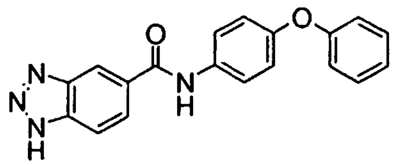

HPLC-MS (Method A): m/z: 331 (M+1); Rt = 3.62 min.

Example 51 (General procedure (A))

1H-Benzotriazole-5-carboxylic acid (4-butoxyphenyl)amide

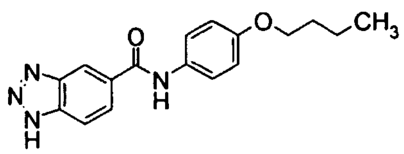

HPLC-MS (Method A): m/z: 311 (M+1); Rt = 3.59 min.

Example 52 (General procedure (A))

1H-Benzotriazole-5-carboxylic acid (3-bromo-4-trifluoromethoxyphenyl)amide

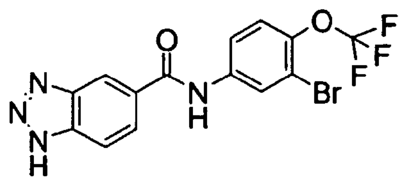

HPLC-MS (Method A): m/z: 402 (M+1); Rt = 3.93 min.

Example 53 (General procedure (A))

1H-Benzotriazole-5-carboxylic acid (3,5-dichloro-4-hydroxyphenyl)amide

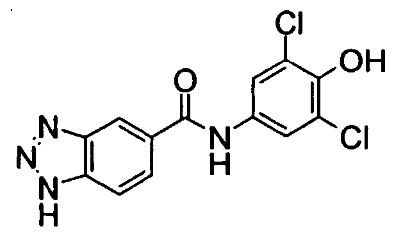

HPLC-MS (Method A): m/z: 323 (M+1); Rt = 2.57 min.

Example 54 (General procedure (A))

4-{[(1H-Benzotriazole-5-carbonyl)amino]methyl}benzoic acid

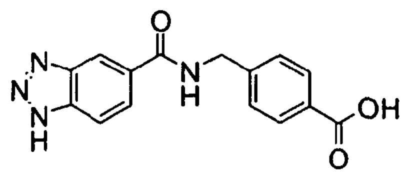

HPLC-MS (Method A): m/z: 297 (M+1); Rt = 1.86 min.

Example 55 (General procedure (A))

{4-[(1H-Benzotriazole-5-carbonyl)amino]phenylsulfanyl}acetic acid

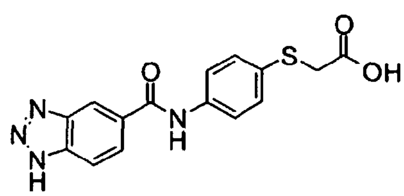

HPLC-MS (Method A): m/z: 329 (M+1); Rt = 2.34 min.

Example 56

N-(1*H*-Benzotriazol-5-yl)acetamide

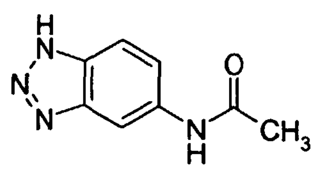

HPLC-MS (Method A): m/z: 177 (M+1); Rt = 0.84 min.

Example 57 (General Procedure (A))

1*H*-Benzotriazole-5-carboxylic acid 4-nitrobenzylamide

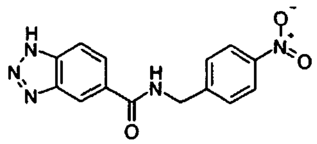

The following compound is prepared according to general procedure (N) as described below:

Example 58 (General procedure (N))

1*H*-Benzotriazole-5-carboxylic acid 4-chlorobenzylamide

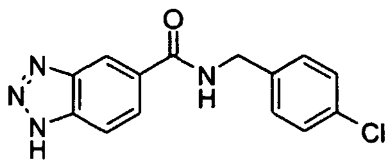

HPLC-MS (Method B): m/z: 287 (M+1); Rt = 4.40 min.

Example 59 2-[(1H-Benzotriazol-5-ylimino)methyl]-4,6-dichlorophenol

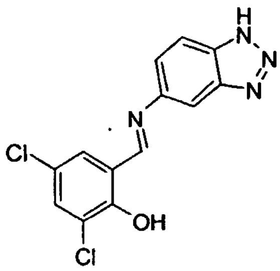

Example 60 Diethyl 2-[(1H-benzotriazol-6-ylamino)methylidene]malonate

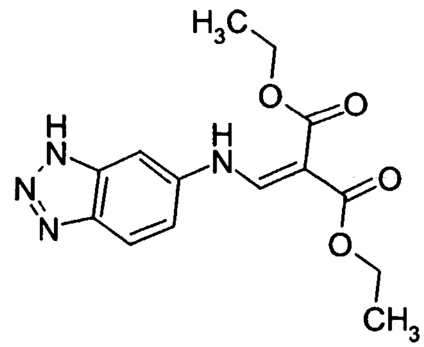

Example 61 N1-(1H-Benzotriazol-5-yl)-3-chlorobenzamide

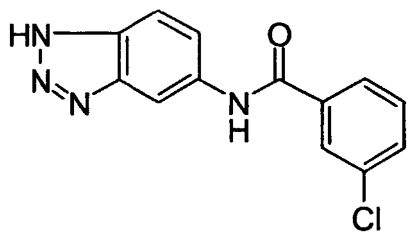

Example 62 N1-(1H-Benzotriazol-5-yl)-3,4,5-trimethoxybenzamide

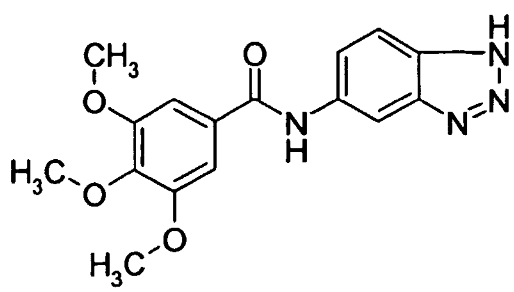

Example 63 N2-(1H-Benzotriazol-5-yl)-3-chlorobenzo[b]thiophene-2-carboxamide

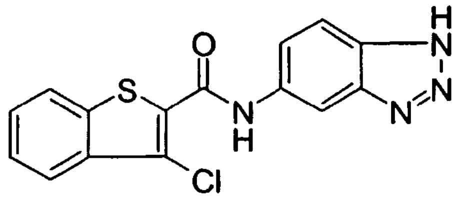

Example 64 6-Bromo-1H-benzotriazole

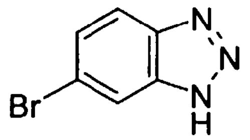

Example 65 2-[(1H-Benzotriazol-5-ylimino)methyl]-4-bromophenol

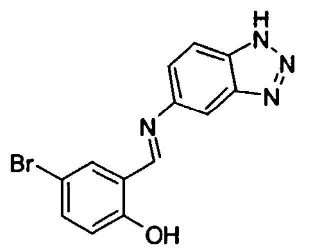

General procedure (B) for preparation of compounds of general formula $I_2$:

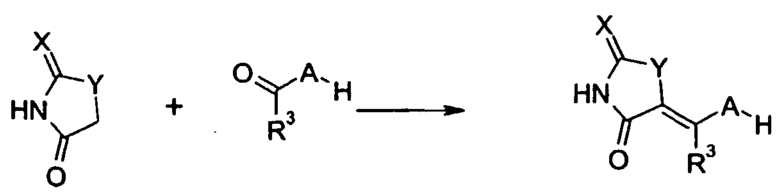

$I_2$

wherein X, Y, A and $R^3$ are as defined above and A is optionally substituted with up to four substituents $R^7$, $R^8$, $R^9$, and $R^{10}$ as defined above.

The chemistry is well known (eg Lohray et al., J. Med. Chem., 1999, 42, 2569-81) and is generally performed by reacting a carbonyl compound (aldehyde or ketone) with the heterocyclic ring (eg thiazolidine-2,4-dione (X = O; Y = S), rhodanine (X = Y = S) and hydantoin (X = O; Y = NH) in the presence of a base, such as sodium acetate, potassium acetate, ammonium acetate, piperidinium benzoate or an amine (eg piperidine, triethylamine and the like) in a solvent (eg acetic acid, ethanol, methanol, DMSO, DMF, NMP, toluene, benzene) or in a mixture of two or more of these solvents. The reaction is performed at room temperature or at elevated temperature, most often at or near the boiling point of the mixture. Optionally, azeotropic removal of the formed water can be done.

This general procedure (B) is further illustrated in the following example:

Example 66 (General procedure (B))

5-(3-Phenoxybenzylidene)thiazolidine-2,4-dione

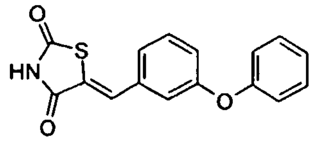

A solution of thiazolidine-2,4-dione (90%, 78 mg, 0.6 mmol) and ammonium acetate (92 mg, 1.2 mmol) in acetic acid (1 mL) was added to 3-phenoxybenzaldehyde (52μL, 0.6 mmol) and the resulting mixture was shaken at 115°C for 16 hours. After cooling, the mixture was concentrated *in vacuo* to afford the title compound.
HPLC-MS (Method A): m/z: 298 (M+1); Rt = 4.54 min.

The compounds in the following examples were similarly prepared. Optionally, the compounds can be further purified by filtration and washing with water, ethanol and / or heptane instead of concentration *in vacuo.* Also optionally the compounds can be purified by washing with ethanol, water and/or heptane, or by chromatography, such as preparative HPLC.

Example 67 (General procedure (B))

5-(4-Dimethylaminobenzylidene)thiazolidine-2,4-dione

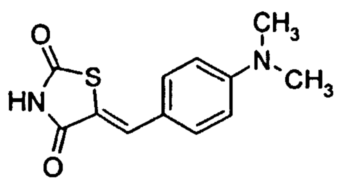

HPLC-MS (Method C): m/z: 249 (M+1); Rt = 4.90 min

Example 68 (General procedure (B))

5-Naphthalen-1-ylmethylenethiazolidine-2,4-dione

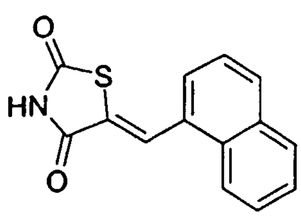

HPLC-MS (Method A): m/z: 256 (M+1); Rt = 4,16 min.

Example 69 (General procedure (B))

5-Benzylidene-thiazolidine-2,4-dione

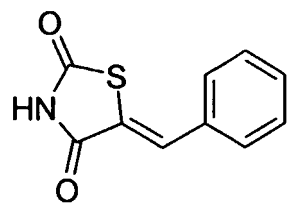

HPLC-MS (Method A): m/z: 206 (M+1); Rt = 4,87 min.

Example 70 (General procedure (B))

5-(4-Diethylaminobenzylidene)thiazolidine-2,4-dione

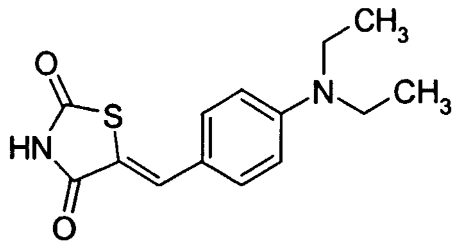

HPLC-MS (Method A): m/z: 277 (M+1); Rt = 4.73 min.

Example 71 (General procedure (B))

5-(4-Methoxy-benzylidene)-thiazolidine-2,4-dione

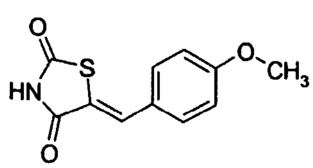

HPLC-MS (Method A): m/z: 263 (M+1); Rt = 4,90 min.

Example 72 (General procedure (B))

5-(4-Chloro-benzylidene)-thiazolidine-2,4-dione

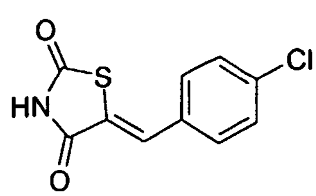

HPLC-MS (Method A): m/z: 240 (M+1); Rt = 5,53 min.

Example 73 (General procedure (B))

5-(4-Nitro-benzylidene)-thiazolidine-2,4-dione

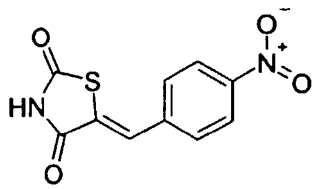

HPLC-MS (Method A): m/z: 251 (M+1); Rt = 4,87 min.

Example 74 (General procedure (B))

5-(4-Hydroxy-3-methoxy-benzylidene)-thiazolidine-2,4-dione

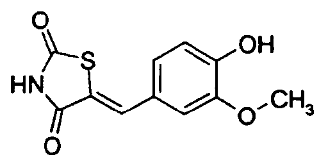

HPLC-MS (Method A): m/z: 252 (M+1); Rt = 4,07 min.

Example 75 (General procedure (B))

5-(4-Methylsulfanylbenzylidene)thiazolidine-2,4-dione

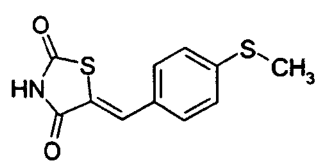

HPLC-MS (Method A): m/z: 252 (M+1); Rt = 5,43 min.

Example 76 (General procedure (B))

5-(2-Pentyloxybenzylidene)thiazolidine-2,4-dione

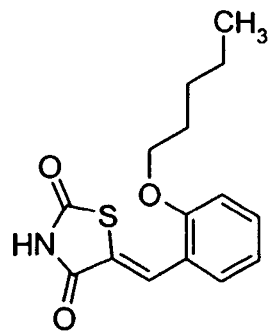

HPLC-MS (Method C): m/z: 292 (M+1); Rt = 4.75 min.
$^1$H NMR (DMSO-$d_6$): δ = 0.90 (3H, t), 1.39 (4H, m), 1.77 (2H, p), 4.08 (2H, t), 7.08 (1H, t), 7.14 (1H, d), 7.43 (2H, m), 8.03 (1H, s), 12.6 (1H, bs).

Example 77 (General procedure (B))

5-(3-Fluoro-4-methoxybenzylidene)thiazolidine-2,4-dione

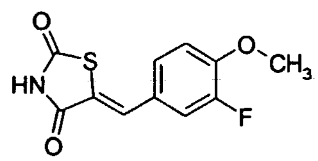

HPLC-MS (Method A): m/z: 354 (M+1); Rt = 4,97 min.

Example 78 (General procedure (B))

5-(4-tert-Butylbenzylidene)thiazolidine-2,4-dione

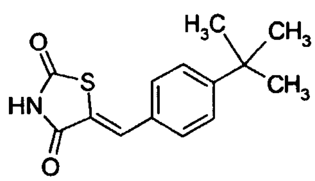

HPLC-MS (Method A): m/z: 262 (M+1); Rt = 6,70 min.

Example 79 (General procedure (B))

*N*-[4-(2,4-Dioxothiazolidin-5-ylidenemethyl)phenyl]acetamide

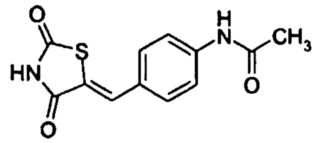

HPLC-MS (Method A): m/z: 263 (M+1); Rt = 3,90 min.

Example 80 (General procedure (B))

5-Biphenyl-4-ylmethylene-thiazolidine-2,4-dione

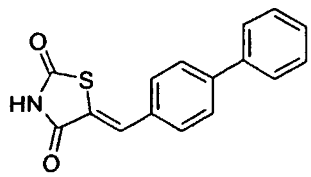

HPLC-MS (Method A): m/z: 282 (M+1); Rt = 4,52 min.

Example 81 (General procedure (B))

5-(4-Phenoxy-benzylidene)-thiazolidine-2,4-dione

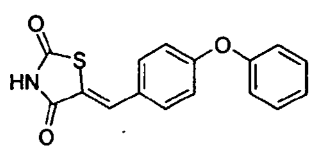

HPLC-MS (Method A): m/z: 298 (M+1); Rt = 6,50 min.

Example 82 (General procedure (B))

5-(3-Benzyloxybenzylidene)thiazolidine-2,4-dione

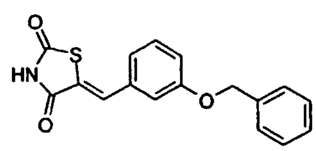

HPLC-MS (Method A): m/z: 312 (M+1); Rt = 6,37 min.

Example 83 (General procedure (B))

5-(3-p-Tolyloxybenzylidene)thiazolidine-2,4-dione

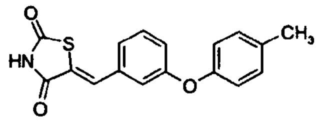

HPLC-MS (Method A): m/z: 312 (M+1); Rt = 6,87 min.

Example 84 (General procedure (B))

5-Naphthalen-2-ylmethylene-thiazolidine-2,4-dione

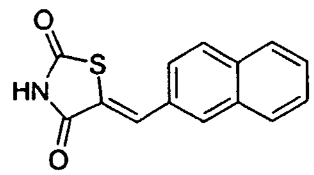

HPLC-MS (Method A): m/z: 256 (M+1); Rt = 4.15 min.

Example 85 (General procedure (B))

5-Benzo[1,3]dioxol-5-ylmethylenethiazolidine-2,4-dione

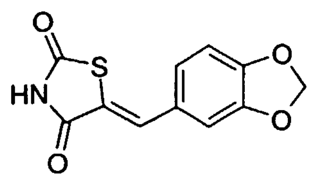

HPLC-MS (Method A): m/z: 250 (M+1), Rt = 3.18 min.

Example 86 (General procedure (B))

5-(4-Chlorobenzylidene)-2-thioxothiazolidin-4-one

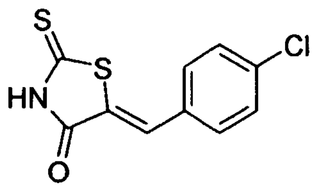

HPLC-MS (Method A): m/z: 256 (M+1); Rt = 4,51 min.

Example 87 (General procedure (B))

5-(4-Dimethylaminobenzylidene)-2-thioxothiazolidin-4-one

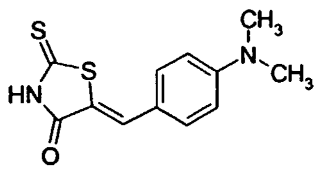

HPLC-MS (Method A): m/z: 265 (M+1); Rt = 5,66 min.

Example 88 (General procedure (B))

5-(4-Nitrobenrylidene)-2-thioxothiazolidin-4-one

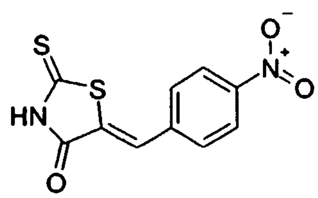

HPLC-MS (Method A): m/z: 267 (M+1); Rt = 3,94 min.

Example 89 (General procedure (B))

5-(4-Methylsulfanylbenzylidene)-2-thioxothiazolidin-4-one

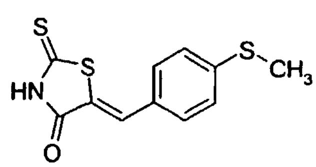

HPLC-MS (Method A): m/z: 268 (M+1); Rt = 6,39 min.

Example 90 (General procedure (B))

5-(3-Fluoro-4-methoxybenzylidene)-2-thioxothiazolidin-4-one

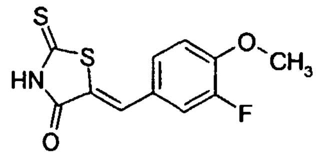

HPLC-MS (Method A): m/z: 270 (M+1); Rt = 5,52 min.

Example 91 (General procedure (B))

5-Naphthalen-2-ylmethylene-2-thioxothiazolidin-4-one

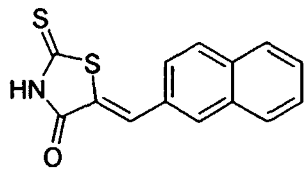

HPLC-MS (Method A): m/z: 272 (M+1); Rt = 6,75 min.

Example 92 (General procedure (B))

5-(4-Diethylaminobenzylidene)-2-thioxothiazolidin-4-one

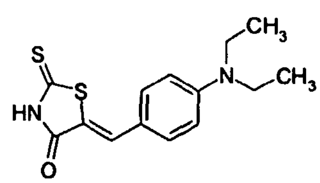

HPLC-MS (Method A): m/z: 293 (M+1); Rt = 5,99 min.

Example 93 (General procedure (B))

5-Biphenyl-4-ylmethylene-2-thioxothiazolidin-4-one

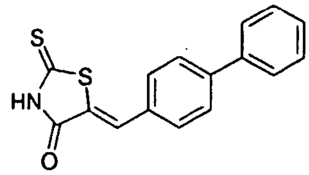

HPLC-MS (Method A): m/z: 298 (M+1); Rt = 7,03 min.

Example 94 (General procedure (B))

5-(3-Phenoxybenzylidene)-2-thioxothiazolidin-4-one

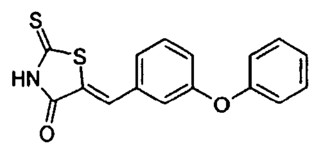

HPLC-MS (Method A): m/z: 314 (M+1); Rt = 6,89 min.

Example 95 (General procedure (B))

5-(3-Benzyloxybenzylidene)-2-thioxothiazolidin-4-one

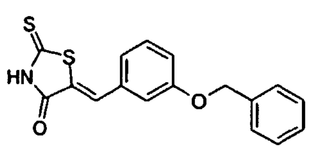

HPLC-MS (Method A): m/z: 328 (M+1); Rt = 6,95 min.

Example 96 (General procedure (B))

5-(4-Benzyloxybenzylidene)-2-thioxothiazolidin-4-one

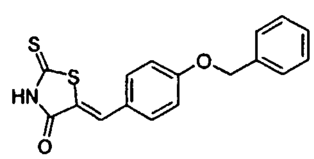

HPLC-MS (Method A): m/z: 328 (M+1); RT = 6,89 min.

Example 97 (General procedure (B))

5-Naphthalen-1-ylmethylene-2-thioxothiazolidin-4-one

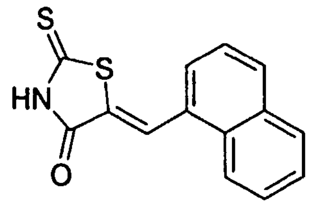

HPLC-MS (Method A): m/z: 272 (M+1); Rt = 6,43 min.

Example 98 (General procedure (B))

5-(3-Methoxybenzyl)thiazolidine-2,4-dione

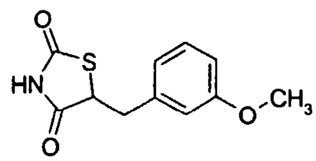

HPLC-MS (Method A): m/z: 236 (M+1); Rt = 3,05 min.

Example 99 (General procedure (D))

4-[2-Chloro-4-(2,4-dioxothiazolidin-5-ylidenemethyl)phenoxy]butyric acid ethyl ester

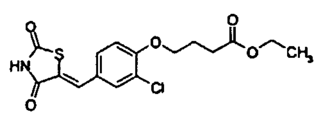

HPLC-MS (Method A): m/z: 392 (M+23), Rt = 4.32 min.

Example 100 (General procedure (D))

4-[2-Bromo-4-(2,4-dioxothiazolidin-5-ylidenemethyl)-phenoxy]-butyric acid

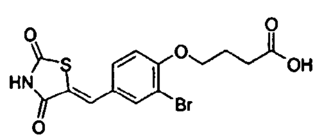

HPLC-MS (Method A): m/z: 410 (M+23); Rt = 3,35 min.

Example 101 (General procedure (B))

5-(3-Bromobenzylidene)thiazolidine-2,4-dione

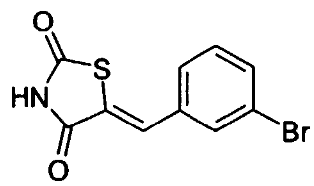

HPLC-MS (Method A): m/z: 285 (M+1); Rt = 4.01 min.

Example 102 (General procedure (B))

5-(4-Bromobenzylidene)thiazolidine-2,4-dione

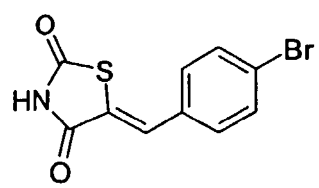

HPLC-MS (Method A): m/z: 285 (M+1); Rt = 4.05 min.

Example 103 (General procedure (B))

5-(3-Chlorobenzylidene)thiazolidine-2,4-dione

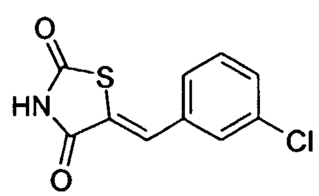

HPLC-MS (Method A): m/z: 240 (M+1); Rt = 3.91 min.

Example 104 (General procedure (B))

5-Thiophen-2-ylmethylenethiazolidine-2,4-dione

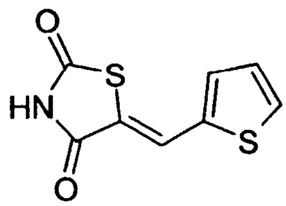

HPLC-MS (Method A): m/z: 212 (M+1); Rt = 3.09 min.

Example 105 (General procedure (B))

5-(4-Bromothiophen-2-ylmethylene)thiazolidine-2,4-dione

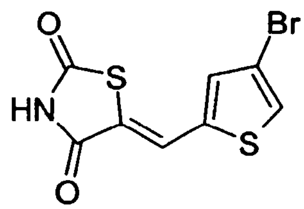

HPLC-MS (Method A): m/z: 291 (M+1); Rt = 3.85 min.

Example 106 (General procedure (B))

5-(3,5-Dichlorobenzylidene)thiazolidine-2,4-dione

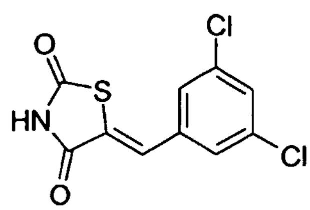

HPLC-MS (Method A): m/z: 274 (M+1); Rt = 4.52 min.

Example 107 (General procedure (B))

5-(1-Methyl-1H-indol-3-ylmethylene)thiazolidine-2,4-dione

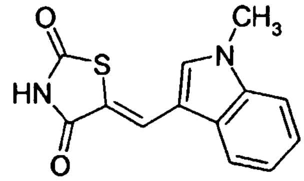

HPLC-MS (Method A): m/z: 259 (M+1); Rt = 3.55 min.

Example 108 (General procedure (B))

5-(1H-Indol-3-ylmethylene)thiazolidine-2,4-dione

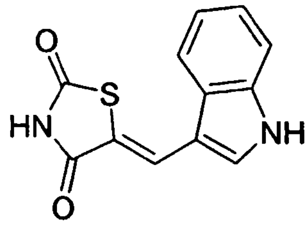

HPLC-MS (Method A): m/z: 245 (M+1); Rt = 2.73 min.

Example 109 (General procedure (B))

5-Fluoren-9-ylidenethiazolidine-2,4-dione

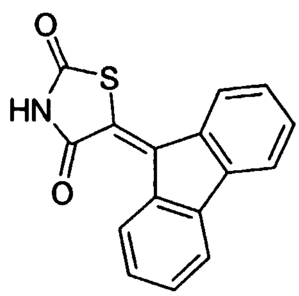

HPLC-MS (Method A): m/z: 280 (M+1); Rt = 4.34 min.

Example 110 (General procedure (B))

5-(1-Phenylethylidene)thiazolidine-2,4-dione

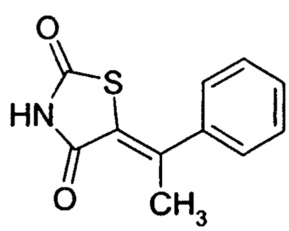

HPLC-MS (Method A): m/z: 220 (M+1); Rt = 3,38 min.

Example 111 (General procedure (B))

5-[1-(4-Methoxyphenyl)-ethylidene]-thiazolidine-2,4-dione

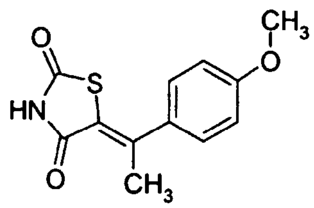

HPLC-MS (Method A): m/z: 250 (M+1); Rt = 3.55 min.

Example 112 (General procedure (B))

5-(1-Naphthalen-2-yl-ethylidene)-thiazolidine-2,4-dione

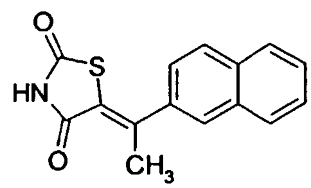

HPLC-MS (Method A): m/z: 270 (M+1); Rt = 4,30 min.

Example 113 (General procedure (B))

5-[1-(4-Bromophenyl)-ethylidene]-thiazolidine-2,4-dione

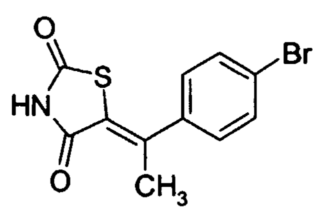

HPLC-MS (Method A): m/z: 300 (M+1); Rt = 4,18 min.

Example 114 (General procedure (B))

5-(2,2-Diphenylethylidene)-thiazolidine-2,4-dione

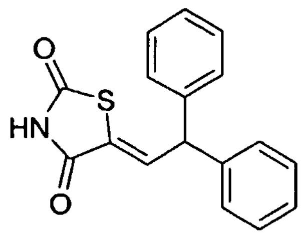

HPLC-MS (Method A): m/z: 296 (M+1); Rt = 4,49 min.

Example 115 (General procedure (B))

5-[1-(3-Methoxyphenyl)-ethylidene]-thiazolidine-2,4-dione

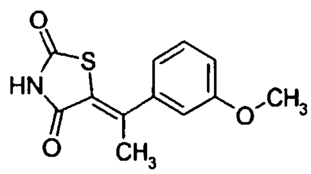

HPLC-MS (Method A): m/z: 250 (M+1); Rt = 3,60 min.

Example 116 (General procedure (B))

5-[1-(6-Methoxynaphthalen-2-yl)-ethylidene]-thiazolidine-2,4-dione

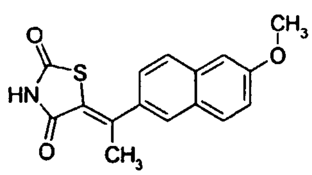

HPLC-MS (Method A): m/z: 300 (M+1); Rt = 4,26 min.

Example 117 (General procedure (B))

5-[1-(4-Phenoxyphenyl)-ethylidene]-thiazolidine-2,4-dione

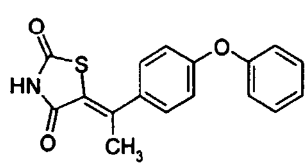

HPLC-MS (Method A): m/z: 312 (M+1); Rt = 4,68 min.

Example 118 (General procedure (B))

5-[1-(3-Fluoro-4-methoxyphenyl)ethylidene]thiazolidine-2,4-dione

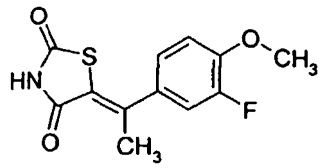

HPLC-MS (Method A): m/z: 268 (M+1); Rt = 3,58 min.

Example 119 (General procedure (B))

5-[1-(3-Bromophenyl)-ethylidene]-thiazolidine-2,4-dione

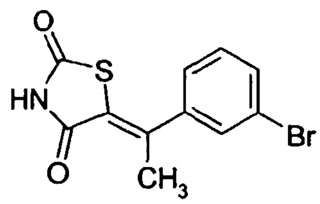

HPLC-MS (Method A): m/z: 300 (M+1); Rt = 4,13 min.

Example 120 (General procedure (B))

5-Anthracen-9-ylmethylenethiazolidine-2,4-dione

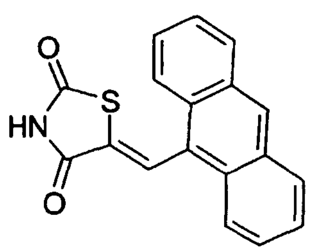

HPLC-MS (Method A): m/z: 306 (M+1); Rt = 4,64 min.

Example 121 (General procedure (B))

5-(2-Methoxynaphthalen-1-ylmethylene)-thiazolidine-2,4-dione

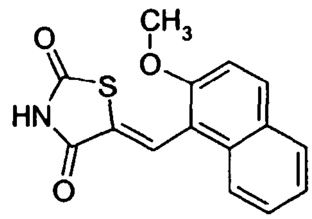

HPLC-MS (Method A): m/z: 286 (M+1); Rt = 4,02 min.

Example 122 (General procedure (B))

5-(4-Methoxynaphthalen-1-ylmethylene)-thiazolidine-2,4-dione

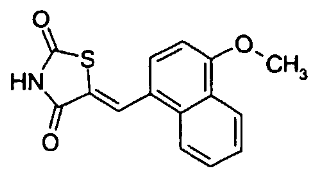

HPLC-MS (Method A): m/z: 286 (M+1); Rt = 4,31 min.

Example 123 (General procedure (B))

5-(4-Dimethylaminonaphthalen-1-ylmethylene)-thiazolidine-2,4-dione

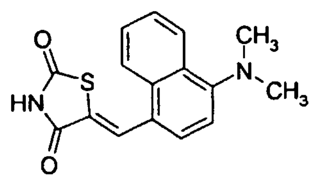

HPLC-MS (Method A): m/z: 299 (M+1); Rt = 4,22 min.

Example 124 (General procedure (B))

5-(4-Methylnaphthalen-1-ylmethylene)-thiazolidine-2,4-dione

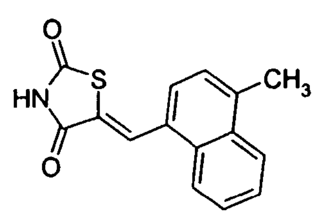

HPLC-MS (Method A): m/z: 270 (M+1); Rt = 4,47 min.

Example 125 (General procedure (B))

5-Pyridin-2-ylmethylene-thiazolidine-2,4-dione

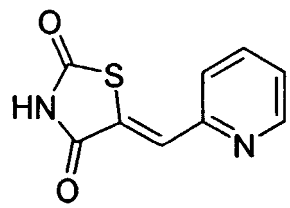

Example 126

5-Pyridin-2-ylmethyl-thiazolidine-2,4-dione

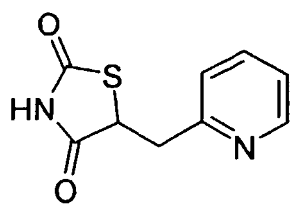

5-Pyridin-2-ylmethylene-thiazolidine-2,4-dione (5 g) in tetrahydrofuran (300 ml) was added 10% Pd/C (1 g) and the mixture was hydrogenated at ambient pressure for 16 hours. More 10% Pd/C (5 g) was added and the mixture was hydrogenated at 50 psi for 16 hours. After filtration and evaporation *in vacuo,* the residue was purified by column chromatography eluting with a mixture of ethyl acetate and heptane (1:1). This afforded the title compound (0.8 g, 16%) as a solid.
TLC: $R_f$ = 0.30 ($SiO_2$; EtOAc: heptane 1:1)

Example 127 (General procedure (B))

5-(1H-Imidazol-4-ylmethylene)-thiazolidine-2,4-dione

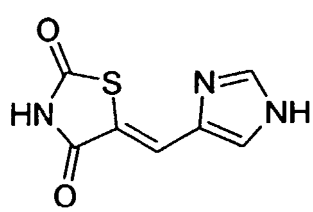

Example 128 (General procedure (B))

5-(4-Benzyloxy-benzylidene)-thiazolidine-2,4-dione

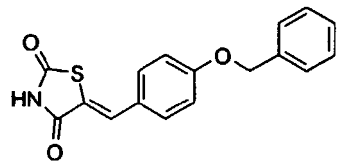

HPLC-MS (Method A): m/z: 6,43 min ; 99 % (2A)

Example 129 (General procedure (B))

5-[4-(4-Fluorobenzyloxy)benzylidene]-2-thioxothiazolidin-4-one

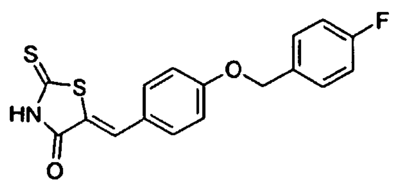

Example 130 (General procedure (B))

5-(4-Butoxybenzylidene)-2-thioxothiazolidin-4-one

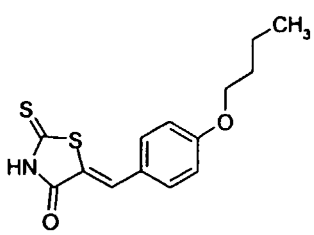

Example 131 (General procedure (B))

5-(3-Methoxybenzylidene)thiazolidine-2,4-dione

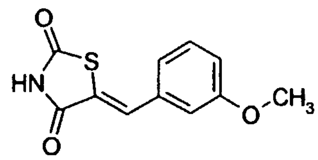

HPLC-MS (Method A): m/z: 236 (M+1); Rt = 4,97 min

Example 132 (General procedure (B))

5-(3-Methoxybenzylidene)imidazolidine-2,4-dione

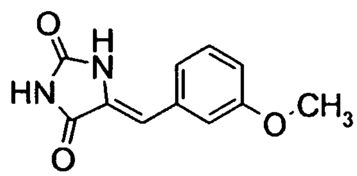

HPLC-MS (Method A): m/z: 219 (M+1); Rt = 2.43 min.

Example 133 (General procedure (B))

5-(4-Methoxybenzylidene)imidazolidine-2,4-dione

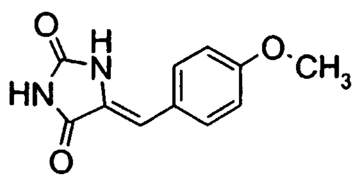

HPLC-MS (Method A): m/z: 219 (M+1); Rt = 2.38 min.

Example 134 (General procedure (B))

5-(2,3-Dichlorobenzylidene)thiazolidine-2,4-dione

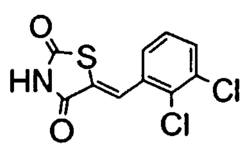

Example 135 (General procedure (B))

5-Benzofuran-7-ylmethylenethiazolidine-2,4-dione

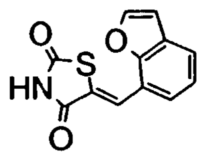

HPLC-MS (Method C): m/z: 247 (M+1); Rt = 4,57 min.

Example 136 (General procedure (B))

5-Benzo[1,3]dioxol-4-ylmethylenethiazolidine-2,4-dione

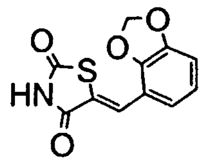

HPLC-MS (Method C): m/z: 250 (M+1); Rt = 4,00 min.

Example 137 (General procedure (B))

5-(4-Methoxy-2,3-dimethylbenzylidene)thiazolidine-2,4-dione

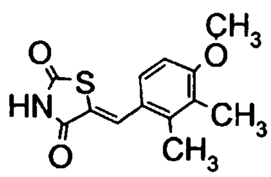

HPLC-MS (Method C): m/z: 264 (M+1); Rt = 5,05 min.

Example 138 (General procedure (B))

5-(2-Benzyloxy-3-methoxybenzylidene)thiazolidine-2,4-dione

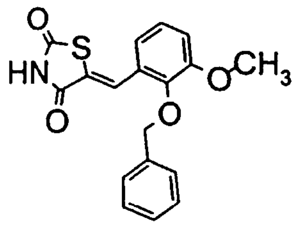

HPLC-MS (Method C): m/z: 342 (M+1); Rt = 5,14 min.

Example 139 (General procedure (B))

5-(2-Hydroxybenzylidene)thiazolidine-2,4-dione

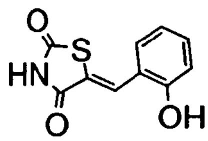

HPLC-MS (Method C): m/z: 222 (M+1); Rt = 3,67 min.

Example 140 (General procedure (B))

5-(2,4-Dichlorobenzylidene)thiazolidine-2,4-dione

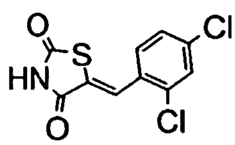

$^{1}H$-NMR (DMSO-$d_6$): 7.60 (2H, "s"), 7.78 (1H, s), 7.82 (1H, s).

Example 141 (General procedure (B))

5-(2-Chlorobenzylidene)thiazolidine-2,4-dione

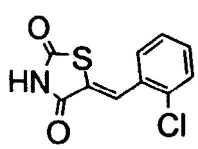

$^{1}H$-NMR (DMSO-$d_6$): 7.40 (1H, t), 7.46 (1H, t), 7.57 (1H, d), 7.62 (1H, d), 7.74 (1H, s).

Example 142 (General procedure (B))

5-(2-Bromobenzylidene)thiazolidine-2,4-dione

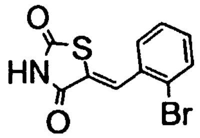

$^1$H-NMR (DMSO-$d_6$): 7.33 (1H, t), 7.52 (1H, t), 7.60 (1H, d), 7.71 (1H, s), 7.77 (1H, d).

Example 143 (General procedure (B))

5-(2,4-Dimethoxybenzylidene)thiazolidine-2,4-dione

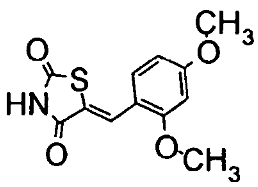

HPLC-MS (Method C): m/z: 266 (M+1) Rt = 4,40 min.

Example 144 (General procedure (B))

5-(2-Methoxybenzylidene)thiazolidine-2,4-dione

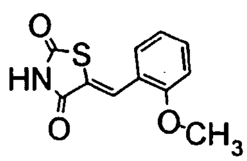

HPLC-MS (Method C): m/z: 236 (M+1); Rt = 4,17 min.

Example 145 (General procedure (B))

5-(2,6-Difluorobenzylidene)thiazolidine-2,4-dione

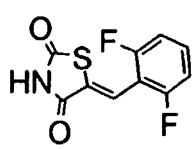

HPLC-MS (Method C): m/z: 242 (M+1); Rt = 4,30 min.

Example 146 (General procedure (B))

5-(2,4-Dimethylbenzylidene)thiazolidine-2,4-dione

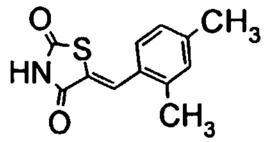

HPLC-MS (Method C): m/z: 234 (M+1); Rt = 5,00 min.

Example 147 (General procedure (B))

5-(2,4,6-Trimethoxybenzylidene)thiazolidine-2,4-dione

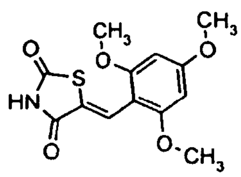

HPLC-MS (Method C): m/z: 296 (M+1); Rt = 4,27 min.

Example 148 (General procedure (B))

5-(4-Hydroxy-2-methoxybenzylidene)thiazolidine-2,4-dione

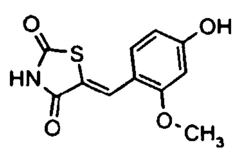

HPLC-MS (Method C): m/z: 252 (M+1); Rt = 3,64 min.

Example 149 (General procedure (B))

5-(4-Hydroxynaphthalen-1-ylmethylene)thiazolidine-2,4-dione

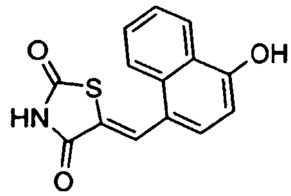

$^1$H-NMR (DMSO-$d_6$): δ = 7.04 (1H, d), 7.57 (2H, m), 7.67 (1H, t), 8.11 (1H, d), 8.25 (1H, d), 8.39 (1H, s) 11.1 (1H, s), 12.5 (1H, bs). HPLC-MS (Method C): m/z: 272 (M+1); Rt = 3.44 min.

Example 150 (General procedure (B))

5-(2-Trifluoromethoxybenzylidene)thiazolidine-2,4-dione

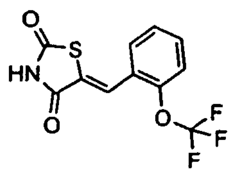

HPLC-MS (Method C): m/z: 290 (M+1); Rt = 4,94 min.

Example 151 (General procedure (B))

5-Biphenyl-2-ylmethylenethiazolidine-2,4-dione

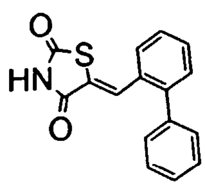

HPLC-MS (Method C): m/z: 282 (M+1); Rt = 5,17 min.

Example 152 (General procedure (B))

5-(2-Benzyloxybenzylidene)thiazolidine-2,4-dione

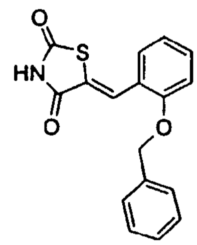

HPLC-MS (Method C): m/z: 312 (M+1); Rt = 5,40 min.

Example 153 (General procedure (B))

5-Adamantan-2-ylidenethiazolidine-2,4-dione

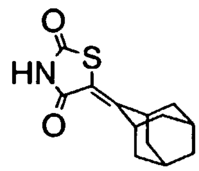

HPLC-MS (Method A): m/z: 250 (M+1); Rt = 4,30 min.

Example 154 (General Procedure (B))

5-[3-(4-Nitrophenyl)allylidene]thiazolidine-2,4-dione

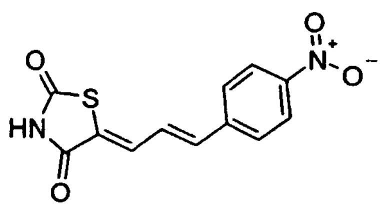

HPLC-MS (Method C): m/z: 277 (M+1); Rt = 3.63 min.

Example 155 (General Procedure (B))

5-[3-(2-Methoxyphenyl)allylidene]thiazolidine-2,4-dione

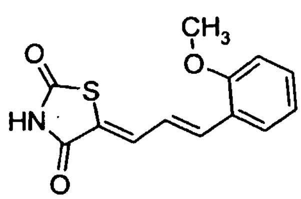

HPLC-MS (Method C): m/z: 262 (M+1); Rt = 3.81 min.

Example 156 (General Procedure (B))

5-[3-(4-Methoxyphenyl)allylidene]thiazolidine-2,4-dione

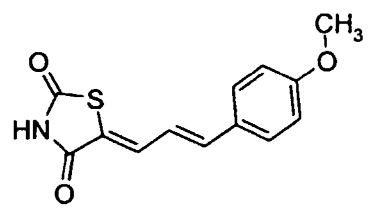

HPLC-MS (Method C): m/z: 262 (M+1); Rt = 3.67 min.

Example 157 (General procedure (B))

5-(4-Hydroxybenzylidene)thiazolidine-2,4-dione

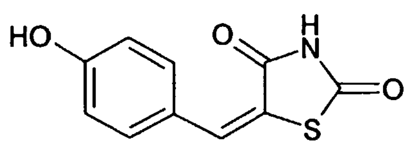

Example 158 (General procedure (B))

5-(4-Dimethylaminobenzylidene)pyrimidine-2,4,6-trione

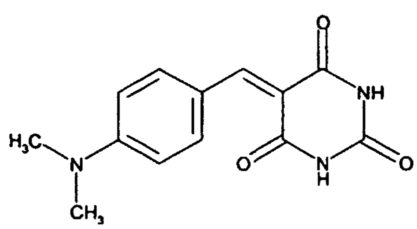

HPLC-MS (Method C): m/z = 260 (M+1) Rt = 2,16 min.

Example 159 (General procedure (B))

5-(9-Ethyl-9H-carbazol-2-ylmethylene)-pyrimidine-2,4,6-trione

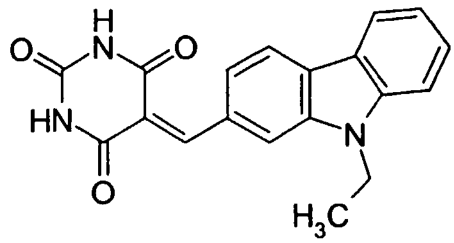

HPLC-MS (Method C): m/z = 334 (M+1); Rt = 3,55 min.

Example 160 (General procedure (B))

5-(4-Hexyloxynaphthalen-1-ylmethylene)thiazolidine-2,4-dione

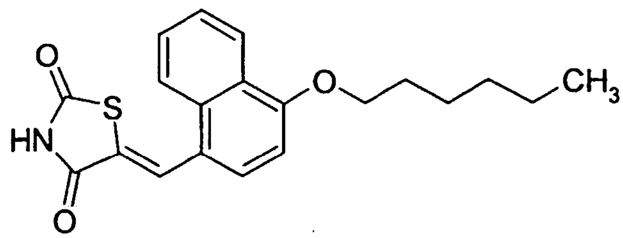

HPLC-MS (Method C): m/z = 356 (M+1); Rt = 5.75 min.

Example 161 (General procedure (B))

5-(4-Decyloxynaphthalen-1-ylmethylene)thiazolidine-2,4-dione

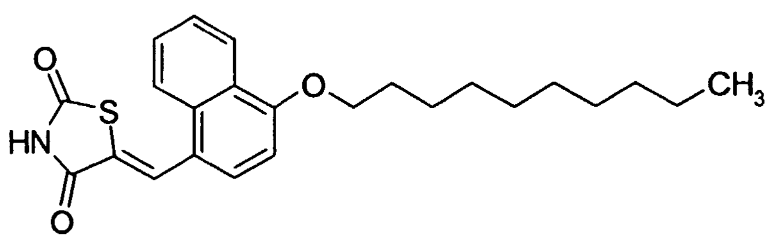

HPLC-MS (Method C): m/z = 412 (M+1); Rt = 6.44 min.

Example 162 (General procedure (B))

5-[4-(2-Aminoethoxy)-naphthalen-1-ylmethylene]-thiazolidine-2,4-dione

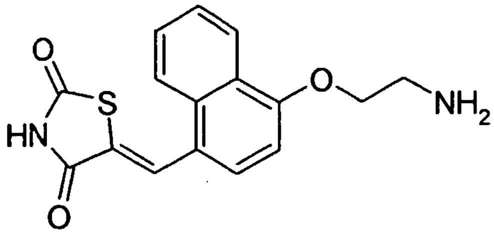

HPLC-MS (Method C): m/z = 315 (M+1); Rt = 3,24 min.

Example 163 (General procedure (B))

5-(2,4-Dimethyl-9H-carbazol-3-ylmethylene)-pyrimidine-2,4,6-trione

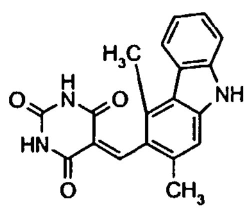

HPLC-MS (Method C): m/z = 334 (M+1); Rt = 3,14 min.

Example 164 (General procedure (B))

4-(4-Hydroxy-3-methoxybenzylidine)hydantoin

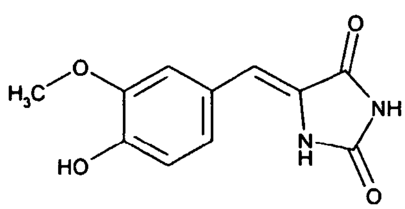

Example 165 (General procedure (B))

5-Benzylidenehydantoin

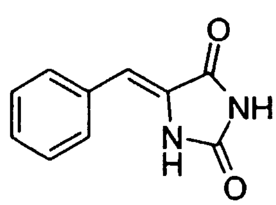

General procedure (C) for preparation of compounds of general formula $I_2$:

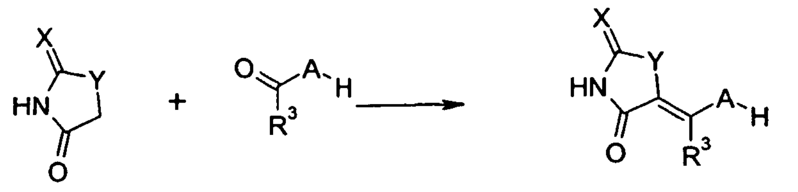

$I_2$

wherein X, Y, A, and $R^3$ are as defined above and A is optionally substituted with up to four substituents $R^7$, $R^8$, $R^9$, and $R^{10}$ as defined above.

This general procedure (C) is quite similar to general procedure (B) and is further illustrated in the following example:

Example 166 (General procedure (C))

5-(3,4-Dibromobenzylidene)thiazolidine-2,4-dione

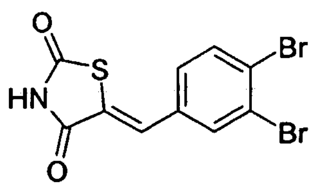

A mixture of thiazolidine-2,4-dione (90%, 65 mg, 0.5 mmol), 3,4-dibromobenzaldehyde (132 mg, 0.5 mmol), and piperidine (247 µL, 2.5 mmol) was shaken in acetic acid (2 mL) at 110 °C for 16 hours. After cooling, the mixture was concentrated to dryness *in vacuo* .
The resulting crude product was shaken with water, centrifuged, and the supernatant was discarded. Subsequently the residue was shaken with ethanol, centrifuged, the supernatant was discarded and the residue was further evaporated to dryness to afford the title compound.
$^1$H NMR (Acetone-$d_6$): $\delta_H$ 7.99 (d,1H), 7.90 (d,1H), 7.70 (s,1H), 7.54 (d,1H); HPLC-MS (Method A): m/z: 364 (M+1); Rt = 4.31 min.

The compounds in the following examples were similarly prepared. Optionally, the compounds can be further purified by filtration and washing with water instead of concentration *in vacuo.* Also optionally the compounds can be purified by washing with ethanol, water and/or heptane, or by preparative HPLC.

Example 167 (General procedure (C))

5-(4-Hydroxy-3-iodo-5-methoxybenzylidene)thiazolidine-2,4-dione

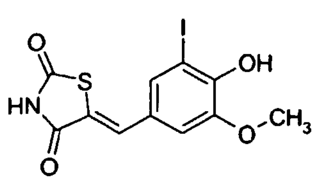

Mp = 256 ˚C; $^1$H NMR (DMSO-$d_6$) $\delta$ = 12.5 (s,broad,1H), 10.5 (s,broad,1H), 7.69 (s,1H), 7.51 (d,1H), 7.19 (d,1H)3.88 (s,3H), $^{13}$C NMR (DMSO-d$_6$) $\delta_c$ = 168.0,167.7,149.0,147.4,133.0, 131.2, 126.7, 121.2, 113.5, 85.5, 56.5; HPLC-MS (Method A): m/z: 378 (M+1); Rt = 3.21 min.

Example 168 (General procedure (C))

5-(4-Hydroxy-2,6-dimethylbenzylidene)thiazolidine-2,4-dione

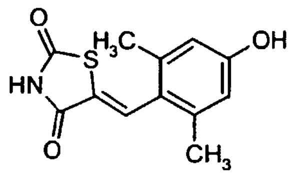

HPLC-MS (Method C): m/z: 250 (M+1); Rt.= 2.45 min.

Example 169 (General procedure (C))

4-[5-Bromo-6-(2,4-dioxothiazolidin-5-ylidenemethyl)-naphthalen-2-yloxymethyl]-benzoic acid

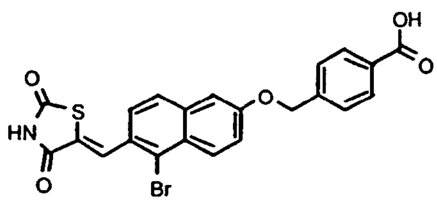

HPLC-MS (Method C): m/z: 506 (M+23); Rt.= 4.27 min.

Example 170 (General procedure (C))

5-(4-Bromo-2,6-dichlorobenzylidene)thiazolidine-2,4-dione

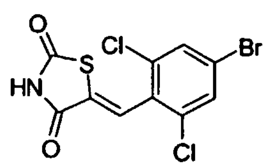

HPLC-MS (Method C): m/z: 354 (M+1); Rt.= 4.36 min.

Example 171 (General procedure (C))

5-(6-Hydroxy-2-naphthylmethylene) thiazolidine-2,4-dione

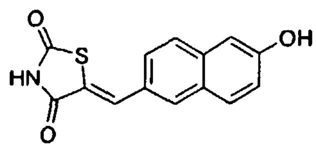

Mp 310-314 ˚C,$^1$H NMR (DMSO-$d_6$): $\delta_H$ = 12.5 (s,broad,1H), 8.06(d,1H), 7.90-7.78(m,2H),7.86 (s, 1H), 7.58 (dd,1 H), 7.20 7.12 (m,2H). $^{13}$C NMR (DMSO-$d_6$): $\delta_C$ = 166.2, 165.8 , 155.4, 133.3, 130.1, 129.1, 128.6, 125.4, 125.3, 125.1, 124.3, 120.0, 117.8, 106.8; HPLC-MS (Method A): m/z: 272 (M+1); Rt = 3.12 min.

Preparation of the starting material, 6-hydroxy-2-naphtalenecarbaldehyde:

6-Cyano-2-naphthalenecarbaldehyde (1.0 g, 5.9 mmol) was dissolved in dry hexane (15 mL) under nitrogen. The solution was cooled to -60 ˚C and a solution of diisobutyl aluminium hydride (DIBAH) (15 mL, 1M in hexane) was added dropwise. After the addition, the solution was left at room temperature overnight. Saturated ammonium chloride solution (20 mL) was added and the mixture was stirred at room temperature for 20 min, subsequently aqueous $H_2SO_4$ (10% solution, 15 mL) was added followed by water until all salt was dissolved. The resulting solution was extracted with ethyl acetate (3x), the combined organic phases were dried with $MgSO_4$, evaporated to dryness to afford 0.89 g of 6-hydroxy-2-naphtalenecarbaldehyde.
Mp.: 153.5-156.5 ˚C; HPLC-MS (Method A): m/z: 173 (M+1); Rt = 2.67 min; $^1$H NMR (DMSO-*$d_6$*)*:* $\delta_H$ = 10.32(s, 1H), 8.95 (d, 1H), 10.02 (s, 1H), 8.42 (s, broad, 1H), 8.01 (d, 1H), 7.82-7.78 (m,2H), 7.23-7.18 (m,2H).

Alternative preparation of 6-hydroxy-2-naphtalenecarbaldehyde:

To a stirred cooled mixture of 6-bromo-2-hydroxynaphthalene (25.3 g, 0.113 mol) in THF (600 mL) at -78 ˚C was added n-BuLi (2.5 M, 100 mL, 0.250 mol) dropwise. The mixture turned yellow and the temperature rose to -64 ˚C. After ca 5 min a suspension appeared. After addition, the mixture was maintained at -78 ˚C. After 20 minutes, a solution of DMF (28.9 mL, 0.373 mol) in THF (100 mL) was added over 20 minutes. After addition, the mixture was allowed to warm slowly to room temperature. After 1 hour, the mixture was poured in ice/water (200 mL). To the mixture citric acid was added to a pH of 5. The mixture was stirred for 0.5 hour. Ethyl acetate (200 mL) was added and the organic layer was separated and washed with brine (100 mL), dried over $Na_2SO_4$ and concentrated. To the residue was added heptane with 20% ethyl acetate (ca 50 mL) and the mixture was stirred for 1 hour. The mixture was filtered and the solid was washed with ethyl acetate and dried *in vacuo* to afford 16 g of the title compound.

Example 172 (General procedure (C))

5-(3-Iodo-4-methoxybenzylidene)thiazolidiene-2,4-dione

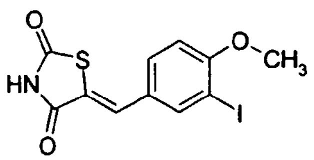

$^1$H NMR (DMSO-$d_6$): $\delta_H$ 12.55 (s,broad,1H), 8.02 (d,1H). 7.72 (s,1H). 7.61 (d.1H)7.18(d.1H). 3.88 (s,3H); $^{13}$C NMR (DMSO-$d_6$): $\delta_C$ 168.1, 167.7, 159.8, 141.5, 132.0, 130.8, 128.0, 122.1, 112.5, 87.5, 57.3. HPLC-MS (Method A): m/z: 362 (M+1); Rt = 4.08 min.

Preparation of the starting material, 3-iodo-4-methoxybenzaldehyde:

4-Methoxybenzaldehyde (0.5 g, 3.67 mmol) and silver trifluoroacetate (0.92 g, 4.19 mmol) were mixed in dichloromethane (25 mL). Iodine (1.19 g, 4.7 mmol) was added in small portions and the mixture was stirred overnight at room temperature under nitrogen. The mixture was subsequently filtered and the residue washed with DCM. The combined filtrates were treated with an acqueous sodium thiosulfate solution (1 M) until the colour disappeared. Subsequent extraction with dichloromethane (3 x 20 mL) followed by drying with $MgSO_4$ and evaporation *in vacuo* afforded 0.94 g of 3-iodo-4-methoxybenzaldehyde.
Mp 104-107 °C; HPLC-MS (Method A): m/z:263 (M+1); Rt = 3.56 min.;$^1$H NMR ($CDCl_3$): $\delta_H$ = 8.80 (s, 1H), 8.31 (d,1H), 7.85 (dd,1H) 6.92 (d,1H), 3.99 (s, 3H).

Example 173 (General procedure (C))

5-(1-Bromonaphthalen-2-ylmethylene)thiazolidine-2,4-dione

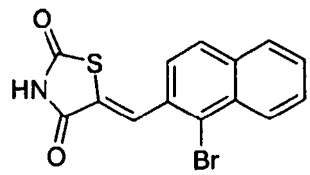

HPLC-MS (Method A): m/z: =336 (M+1); Rt = 4.46 min.

Example 174 (General procedure (C))

1-[5-(2,4-Dioxothiazolidin-5-ylidenemethyl)thiazol-2-yl]piperidine-4-carboxylic acid ethyl ester

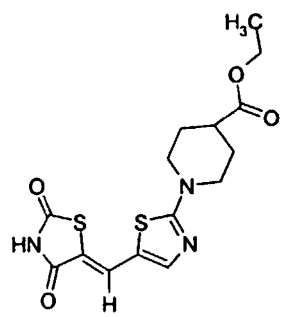

$^1$H NMR (DMSO-$d_6$): $\delta_H$ = 7.88 (s, 1H), 7.78 (s, 1H), 4.10 (q,2H), 4.0-3.8 (m,2H), 3.40-3.18 (m,2H), 2.75-2.60 (m, 1H), 2.04-1.88 (m,2H), 1.73-1.49 (m, 2H), 1.08 (t,3H); HPLC-MS (Method A): m/z: 368 (M+1); Rt = 3.41 min.

Example 175 (General procedure (C))

5-(2-Phenyl-[1,2,3]triazol-4-ylmethylene) thiazolidine-2,4-dione

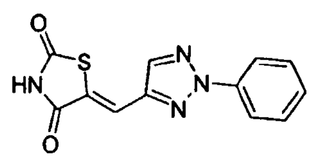

$^1$H NMR (DMSO-$d_6$): $\delta_H$ = 12.6 (s, broad, 1H), 8.46 (s, 1H), 8.08 (dd,2H), 7.82 (s, 1H), 7.70-7.45 (m, 3H). HPLC-MS (Method A): m/z: 273 (M+1); Rt = 3.76 min.

Example 176 (General procedure (C))

5-(Quinolin-4-ylmethylene)thiazolidine-2,4-dione

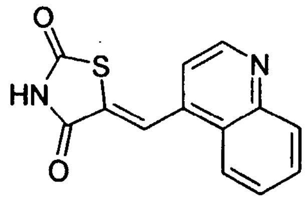

HPLC-MS (Method A): m/z: 257 (M+1); Rt = 2.40 min.

Example 177 (General procedure (C))

5-(6-Methylpyridin-2-ylmethylene)thiazolidine-2,4-dione

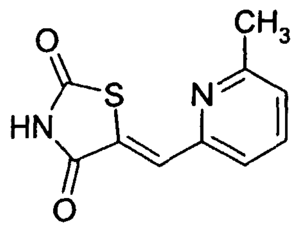

$^1$H NMR (DMSO-$d_6$): $\delta_H$ = 12.35 (s, broad, 1H), 7.82 (t, 1H), 7.78 (s, 1H), 7.65 (d, 1H), 7.18 (d, 1H), 2.52 (s, 3H); HPLC-MS (Method A): m/z: 221 (M+1); Rt = 3.03 min.

Example 178 (General procedure (C))

5-(2,4-dioxothiazolidin-5-ylidenemethyl)-furan-2-ylmethylacetate

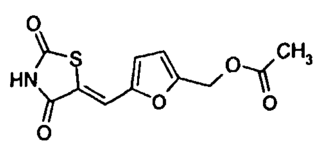

$^1$H NMR (DMSO-$d_6$): $\delta_H$ = 12.46 (s,broad,1H), 7.58 (s,1H), 7.05 (d,1H), 6.74 (s,1H), 5.13 (s,2H), 2.10 (s,3H). HPLC-MS (Method A): m/z: 208 (M-$CH_3COO$); Rt = 2.67 min.

Example 179 (General procedure (C))

5-(2,4-Dioxothiazolidin-5-ylidenemethyl)furan-2-sulfonic acid

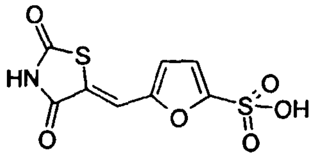

HPLC-MS (Method A): m/z:276 (M+1); Rt = 0.98 min.

Example 180 (General procedure (C))

5-(5-Benzyloxy-1H-pyrrolo[2,3-c]pyridin-3-ylmethylene)-thiazolidine-2,4-dione

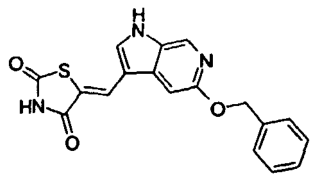

HPLC-MS (Method A): m/z: 352 (M+1); Rt = 3.01 min.

Example 181 (General procedure (C))

5-(Quinolin-2-ylmethylene)thiazolidine-2,4-dione

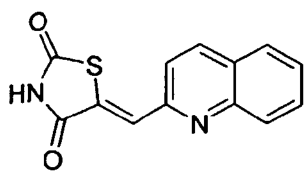

HPLC-MS (Method A): m/z: 257 (M+1); Rt = 3.40 min.

Example 182 (General procedure (C))

5-(2,4-Dioxothiazolidin-5-ylidenemethyl)thiophene-2-carboxylic acid

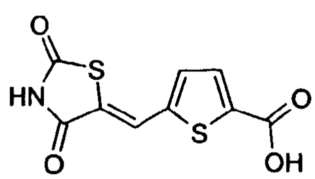

HPLC-MS (Method A): m/z: 256 (M+1); Rt = 1.96 min.

Example 183 (General procedure (C))

5-(2-Phenyl-1H-imidazol-4-ylmethylene)thiazolidine-2,4-dione

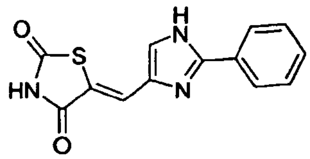

HPLC-MS (Method A): m/z: 272 (M+1); Rt = 2.89 min.

Example 184 (General procedure (C))

5-(4-Imidazol-1-yl-benzylidene)thiazolidine-2,4-dione

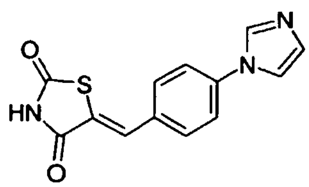

HPLC-MS (Method A): m/z: 272 (M+1); Rt = 1.38 min.

Example 185 (General procedure (C))

5-(9-Ethyl-9H-carbazol-3-ylmethylene)thiazolidine-2,4-dione

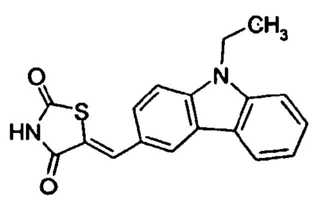

HPLC-MS (Method A): m/z: 323 (M+1); Rt = 4.52 min.

Example 186 (General procedure (C))

5-(1,4-Dimethyl-9H-carbazol-3-ylmethylene)thiazolidine-2,4-dione

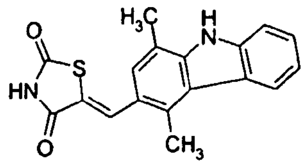

HPLC-MS (Method A): m/z: 323 (M+1); Rt = 4.35 min.

Example 187 (General procedure (C))

5-(2-Methyl-1H-indol-3-ylmethylene)thiazolidine-2,4-dione

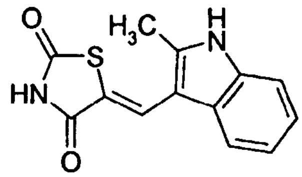

HPLC-MS (Method A): m/z: 259 (M+1); Rt = 3.24 min.

Example 188 (General procedure (C))

5-(2-Ethylindol-3-ylmethylene)thiazolidine-2,4-dione

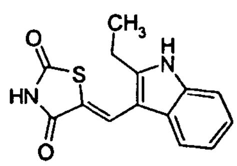

2-Methylindole (1.0 g, 7.6mmol) dissolved in diethyl ether (100 mL) under nitrogen was treated with n-Butyl lithium (2 M in pentane, 22.8 mmol) and potassium *tert*-butoxide (15.2 mmol) with stirring at RT for 30 min. The temperature was lowered to -70 C and methyl lodide (15.2 mmol) was added and the resulting mixture was stirred at -70 for 2 h. Then 5 drops of water was added and the mixture allowed to warm up to RT. Subsequently, the mixture was poured into water (300 mL), pH was adjusted to 6 by means of 1N hydrochloric acid and the mixture was extracted with diethyl ether. The organic phase was dried with $Na_2SO_4$ and evaporated to dryness. The residue was purified by column chromatography on silica gel using heptane/ether( 4/1) as eluent. This afforded 720 mg (69 %) of 2-ethylindole.
$^1$H NMR (DMSO-$d_6$): $\delta$ = 10.85 (1H,s); 7.39 (1H,d); 7.25 (1H,d); 6.98(1H,t); 6.90(1H,t); 6.10 (1H,s); 2.71 (2H,q); 1.28 (3H,t).

2-Ethylindole (0.5 g, 3.4mmol) dissolved in DMF (2 mL) was added to a cold (0 ˚C) premixed (30 minutes) mixture of DMF (1.15 mL) and phosphorous oxychloride (0.64 g, 4.16 mmol). After addition of 2-ethylindole, the mixture was heated to 40 ˚C for 1 h, water (5 mL) was added and the pH adjusted to 5 by means of 1 N sodium hydroxide.The mixture was subsequently extracted with diethyl ether, the organic phase isolated, dried with $MgSO_4$ and evaporated to dryness affording 2-ethylindole-3-carbaldehyde (300 mg ).
HPLC-MS (Method C): m/z:174 (M+1); Rt. =2.47 min.

2-Ethylindole-3-carbaldehyde (170 mg) was treated with thiazolidine-2,4-dione using the general procedure (C) to afford the title compound (50 mg).
HPLC-MS (Method C):m/z: 273 (M+1); Rt.= 3.26 min.

Example 189 (General procedure (C))

5-[2-(4-Bromophenylsulfanyl)-1-methyl-1H-indol-3-ylmethylene]thiazolidine-2,4-dione

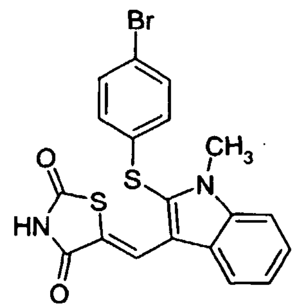

HPLC-MS (Method A): m/z: 447 (M+1); Rt = 5.25 min.

Example 190 (General procedure (C))

5-[2-(2,4-Dichlorobenzyloxy)-naphthalen-1-ylmethylene]thiazolidine-2,4-dione

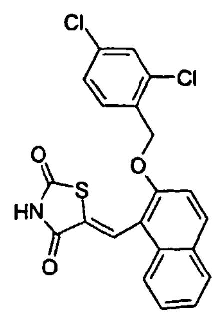

HPLC-MS (Method A): (anyone 1) m/z: 430 (M+1); Rt = 5.47 min.

Example 191 (General procedure (C))

5-{4-[3-(4-Bromophenyl)-3-oxopropenyl]-benzylidene}thiazolidine-2,4-dione

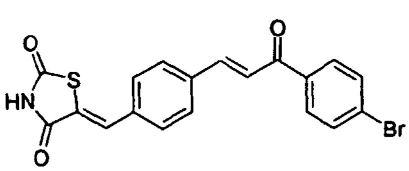

HPLC-MS (Method A): m/z: 416 (M+1); Rt = 5.02 min.

Example 192 (General procedure (C))

5-(4-Pyridin-2-ylbenzylidene)thiazolidine-2,4-dione

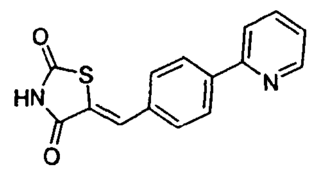

HPLC-MS (Method A): m/z: 283 (M+1), Rt = 2.97 min.

Example 193 (General procedure (C))

5-(3,4-Bisbenzyloxybenzylidene)thiazolidine-2,4-dione

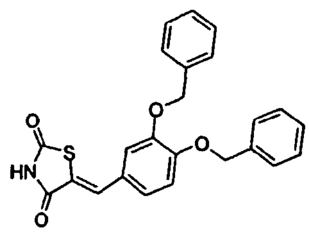

HPLC-MS (Method A): m/z: 418 (M+1); Rt = 5.13 min.

Example 194 (General procedure (C))

5-[4-(4-Nitrobenzyloxy)-benzylidene]thiazolidine-2,4-dione

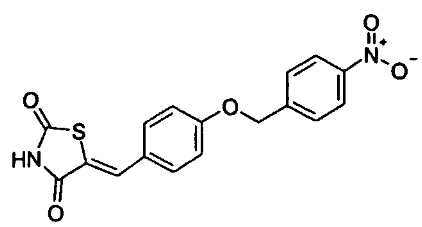

HPLC-MS (Method A): m/z: 357 (M+1); Rt = 4.45 min.

Example 195 (General procedure (C))

5-(2-Phenyl-1H-indol-3-ylmethylene)thiazolidine-2,4-dione

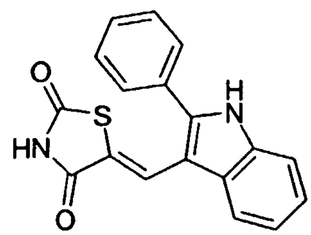

HPLC-MS (Method A): m/z: 321 (M+1); Rt = 3.93 min.

Example 196 (General procedure (C))

5-(5-Benzyloxy-1H-indol-3-ylmethylene)thiazolidine-2,4-dione

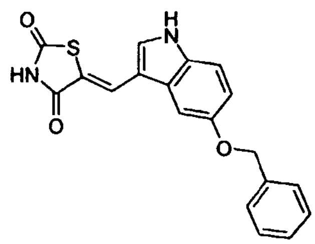

HPLC-MS (Method A): m/z: 351 (M+1); Rt = 4.18 min.

Example 197 (General procedure (C))

5-(4-Hydroxybenzylidene)thiazolidine-2,4-dione

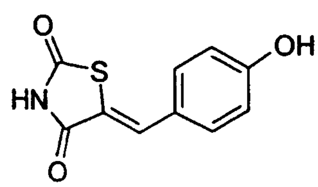

HPLC-MS (Method A): m/z: 222 (M+1); Rt = 2.42 min.

Example 198 (General procedure (C))

5-(1-Methyl-1H-indol-2-ylmethylene)thiazolidine-2,4-dione

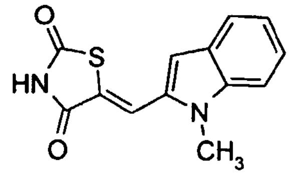

$^1$H NMR (DMSO-$d_6$): $\delta_H$ = 12.60 (s,broad,1H), 7.85 (s,1H), 7.68 (dd,1H), 7.55 (dd,1H), 7.38 (dt,1H), 7.11 (dt,1H) 6.84 (s,1H), 3.88 (s,3H); HPLC-MS (Method A): m/z: 259 (M+1); Rt = 4.00 min.

Example 199 (General procedure (C))

5-(5-Nitro-1H-indol-3-ylmethylene)thiazolidine-2,4-dione

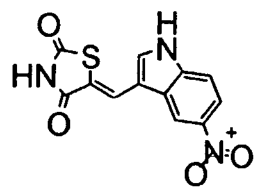

Mp 330-333 ˚C, $^1$H NMR (DMSO-$d_6$): $\delta_H$ = 12.62 (s, broad, 1H), 8.95 (d, 1H), 8.20 (s, 1H), 8.12 (dd, 1H), 7.98 (s, broad, 1H), 7.68 (d,1H); HPLC-MS (Method A): m/z: 290 (M+1); Rt = 3.18 min.

Example 200 (General procedure (C))

5-(6-Methoxynaphthalen-2-ylmethylene)thiazolidine-2,4-dione

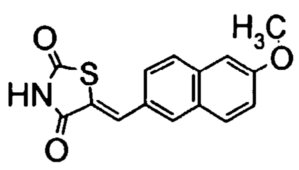

HPLC-MS (Method A): m/z: 286 (M+1); Rt = 4.27 min.

Example 201 (General procedure (C))

5-(3-Bromo-4-methoxybenzylidene)thiazolidine-2,4-dione

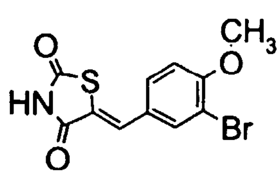

HPLC-MS (Method A): m/z: 314 (M+1), Rt = 3.96 min.

Example 202 (General procedure (C))

3-{(2-Cyanoethyl)-[4-(2,4-dioxothiazolidin-5-ylidenemethyl)phenyl]amino}propionitrile

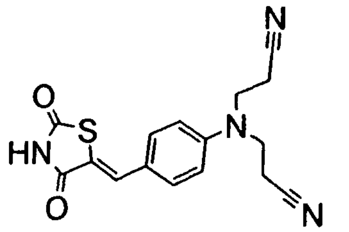

HPLC-MS (Method A): m/z: 327 (M+1); Rt = 2.90 min.

Example 203 (General procedure (C))

3-(2,4-Dioxothiazolidin-5-ylidenemethyl)indole-6-carboxylic acid methyl ester

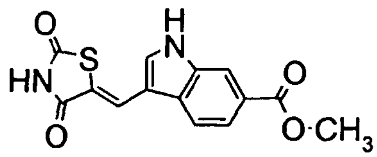

HPLC-MS (Method A): m/z: 303 (M+1); Rt = 3.22-3-90 min.

Example 204

3-(2,4-Dioxothiazolidin-5-ylidenemethyl)indole-6-carboxylic acid pentyl ester.

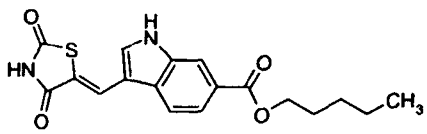

3-(2,4-Dioxothiazolidin-5-ylidenemethyl)indole-6-carboxylic acid methyl ester (example 203, 59 mg; 0.195mmol) was stirred in pentanol (20 mL) at 145 ˚C for 16 hours. The mixture was evaporated to dryness affording the title compound (69 mg).
HPLC-MS (Method C): m/z: 359 (M+1); Rt.= 4.25 min.

Example 205 (General procedure (C))

3-(2,4-Dioxothiazolidin-5-ylidenemethyl)indole-7-carboxylic acid

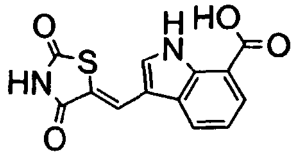

HPLC-MS (Method A): m/z: 289 (M+1); Rt = 2.67 min.

Example 206 (General procedure (C))

5-(1-Benzylindol-3-ylmethylene)thiazolidine-2,4-dione

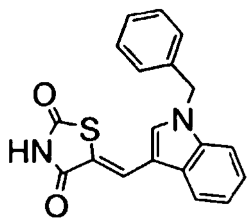

HPLC-MS (Method A): m/z: 335 (M+1); Rt = 4.55 min.

Example 207 (General procedure (C))

5-(1-Benzenesulfonylindol-3-ylmethylene)thiazolidine-2,4-dione

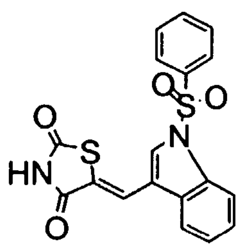

HPLC-MS (Method A): m/z: = 385 (M+1); Rt = 4.59 min.

Example 208 (General procedure (C))

5-(4-[1,2,3]Thiadiazol-4-ylbenzylidene)thiazolidine-2,4-dione

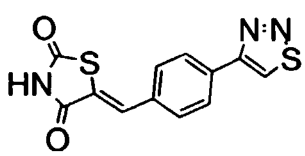

HPLC-MS (Method A): m/z: 290 (M+1); Rt = 3.45 min.

Example 209 (General procedure (C))

5-[4-(4-Nitrobenzyloxy)-benzylidene]thiazolidine-2,4-dione

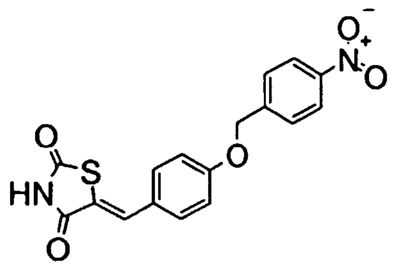

HPLC-MS (Method A): m/z: 357 (M+1); Rt = 4.42 min.

Example 210 (General procedure (C))

3-(2,4-Dioxothiazolidin-5-ylidenemethyl)indole-1-carboxylic acid ethyl ester

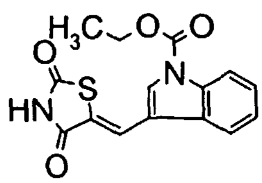

HPLC-MS (Method A): m/z: 317 (M+1); Rt = 4.35 min.

Example 211 (General procedure (C))

5-[2-(4-Pentylbenzoyl)-benzofuran-5-ylmethylene]thiazolidine-2,4-dione

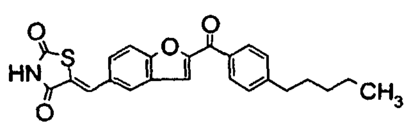

HPLC-MS (Method A): m/z: 420 (M+1); Rt = 5.92 min.

Example 212 (General procedure (C))

5-[1-(2-Fluorobenzyl)-4-nitroindol-3-ylmethylene]thiazolidine-2,4-dione

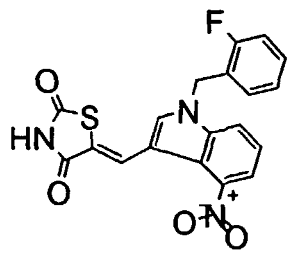

HPLC-MS (Method A): (Anyone 1) m/z: 398 (M+1); Rt = 4.42 min.

Example 213 (General procedure (C))

5-(4-Benzyloxyindol-3-ylmethylene)thiazolidine-2,4-dione

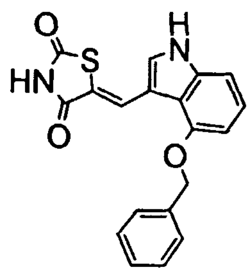

HPLC-MS (Method A): m/z: 351 (M+1); Rt = 3.95 min.

Example 214 (General procedure (C))

5-(4-Isobutylbenzylidene)-thiazolidine-2,4-dione

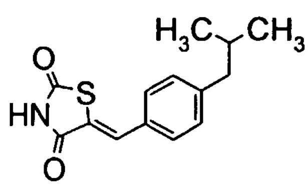

HPLC-MS (Method A): m/z: 262 (M+1); Rt = 4.97 min.

Example 215 (General procedure (C))

Trifluoromethanesulfonic acid 4-(2,4-dioxothiazolidin-5-ylidenemethyl)naphthalen-1-yl ester

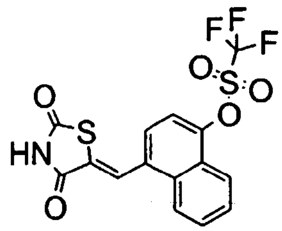

HPLC-MS (Method A): m/z: 404 (M+1); Rt = 4.96 min.

Preparation of starting material:

4-Hydroxy-1-naphthaldehyde (10 g, 58 mmol) was dissolved in pyridin (50 ml) and the mixture was cooled to 0-5 ˚C. With stirring, trifluoromethanesulfonic acid anhydride (11.7 ml, 70 mmol) was added drop-wise. After addition was complete, the mixture was allowed to warm up to room temperature, and diethyl ether (200 ml) was added. The mixture was washed with water (2 x 250 ml), hydrochloric acid (3N, 200 ml), and saturated aqueous sodium chloride (100 ml). After drying (MgSO4), filtration and concentration in vacuo, the residue was purified by column chromatography on silica gel eluting with a mixture of ethyl acetate and heptane (1:4). This afforded 8.35 g (47%) trifluoromethanesulfonic acid 4-formylnaphthalen-1-yl ester, mp 44-46.6 ˚C.

Example 216 (General procedure (C))

5-(4-Nitroindol-3-ylmethylene)-thiazolidine-2,4-dione

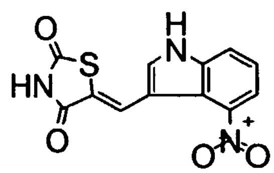

HPLC-MS (Method A): m/z: 290 (M+1); Rt = 3.14 min.

Example 217 (General procedure (C))

5-(3,5-Dibromo-4-hydroxy-benzylidene)thiazolidine-2,4-dione

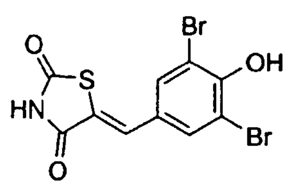

$^1$H NMR (DMSO-$d_6$): $\delta_H$ = 12.65 (broad,1H), 10.85 (broad,1H), 7.78 (s,2H), 7.70 (s,1H); HPLC-MS (Method A): m/z: 380 (M+1); Rt = 3.56 min.

Example 218 (General procedure (C))

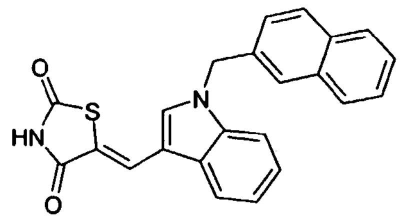

HPLC-MS (Method A): m/z: 385 (M+1); Rt = 5.08 min.

General procedure for preparation of starting materials for examples 218 - 221:

Indole-3-carbaldehyde (3.8 g, 26 mmol) was stirred with potassium hydroxide (1.7 g) in acetone (200 mL) at RT until a solution was obtained indicating full conversion to the indole potassium salt. Subsequently the solution was evaporated to dryness *in vacuo.* The residue was dissolved in acetone to give a solution containing 2.6 mmol/20 mL.

20 mL portions of this solution were mixed with equimolar amounts of arylmethylbromides in acetone (10 mL). The mixtures were stirred at RT for 4 days and subsequently evaporated to dryness and checked by HPLC-MS. The crude products, 1-benzylated indole-3-carbaldehydes, were used for the reaction with thiazolidine-2,4-dione using the general procedure C.

Example 219 (General procedure (C))

4-[3-(2,4-Dioxothiazolidin-5-ylidenemethyl)indol-1-ylmethyl]benzoic acid methyl ester

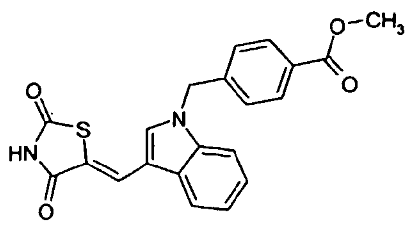

HPLC-MS (Method A): m/z: 393 (M+1); Rt = 4.60 min.

Example 220 (General procedure (C))

5-[1-(9,10-Dioxo-9,10-dihydroanthracen-2-ylmethyl)-1*H*-indol-3-ylmethylene]thiazolidine-2,4-dione

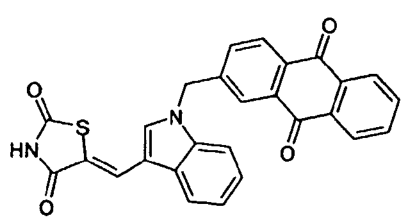

HPLC-MS (Method A): m/z: 465 (M+1); Rt = 5.02 min.

Example 221 (General procedure (C))

4'-[3-(2,4-Dioxothiazolidin-5-ylidenemethyl)indol-1-ylmethyl]biphenyl-2-carbonitrile

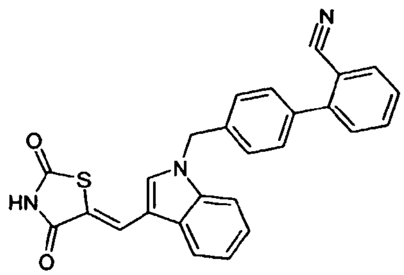

HPLC-MS (Method A): m/z: 458 (M+23); Rt = 4.81 min.

Example 222 (General procedure (C))

3-[3-(2,4-Dioxothiazolidin-5-ylidenemethyl)-2-methylindol-1-ylmethyl]benzonitrile.

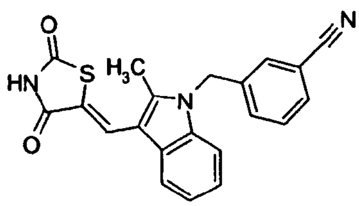

2-Methylindole-3-carbaldehyde (200 mg, 1.26 mmol) was added to a slurry of 3-bromomethylbenzenecarbonitrile (1.26 mmol) followed by sodium hydride, 60%, (1.26 mmol) in DMF (2 mL). The mixture was shaken for 16 hours, evaporated to dryness and washed with water and ethanol. The residue was treated with thiazolidine-2,4-dione following the general procedure C to afford the title compound (100 mg).
HPLC-MS (Method C): m/z: 374 (M+1); Rt. = 3.95 min.

Example 223 (General procedure (C))

5-(1-Benzyl-2-methylindol-3-ylmethylene)thiazolidine-2,4-dione.

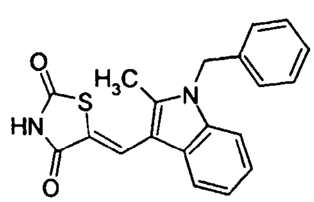

This compound was prepared in analogy with the compound described in example 222 from benzyl bromide and 2-methylindole-3-carbaldehyde, followed by reaction with thiazolidine-2,4-dione resulting in 50 mg of the title compound.
HPLC-MS (Method C): m/z: 349 (M+1); Rt. = 4.19 min.

Example 224

4-[3-(2,4-Dioxothiazolidin-5-ylidenemethyl)-2-methylindol-1-ylmethyl]benzoic acid methyl ester

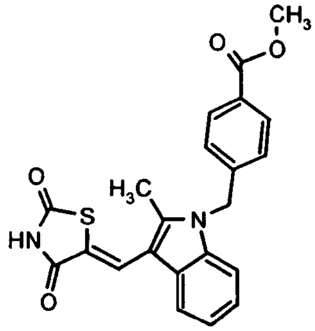

This compound was prepared in analogy with the compound described in example 222 from 4-(bromomethyl) benzoic acid methyl ester and 2-methylindole-3-carbaldehyde, followed by reaction with thiazolidine-2,4-dione. HPLC-MS (Method C): m/z: 407 (M+1); Rt.= 4.19 min.

Example 225 (General procedure (C))

5-(2-Chloro-1-methyl-1H-indol-3-ylmethylene)thiazolidine-2,4-dione

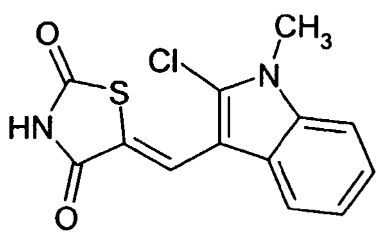

HPLC-MS (Method A): m/z: 293 (M+1); Rt = 4.10 min.

Example 226 (General procedure (C))

5-(4-Hydroxy-3,5-diiodo-benzylidene)-thiazolidine-2,4-dione

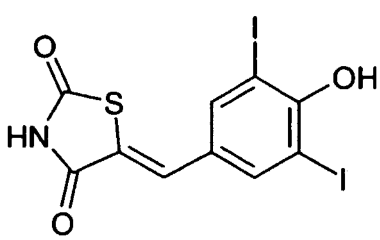

HPLC-MS (Method A): m/z: 474 (M+1); Rt = 6.61 min.

Example 227 (General procedure (C))

5-(4-Hydroxy-3-iodobenzylidene)thiazolidine-2,4-dione

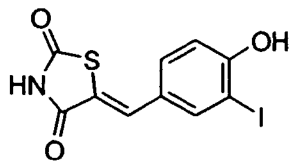

HPLC-MS (Method C): m/z: 348 (M+1); Rt. = 3.13 min
$^1$H-NMR: (DMSO-$d_6$): 11.5 (1H, broad); 7.95(1 H,d); 7.65(1 H,s); 7.45 (1H,dd); 7.01 (1H,dd); 3.4 (1H,broad).

Example 228 (General procedure (C))

5-(2,3,6-Trichlorobenzylidene)thiazolidine-2,4-dione

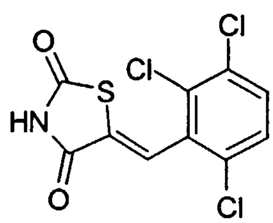

H PLC-MS (Method C): m/z: 309 (M+1); Rt.= 4.07 min

Example 229 (General procedure (C))

5-(2,6-Dichlorobenzylidene)thiazolidine-2,4-dione

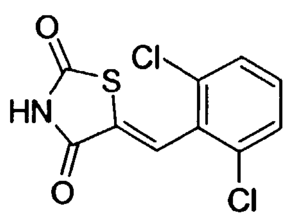

Mp. 152-154˚C.
HPLC-MS (Method C): m/z: 274 (M+1), Rt.= 3.70 min
$^1$H-NMR: (DMSO-$d_6$): 12.8 (1H, broad); 7.72 (1H,s); 7.60 (2H,d); 7.50 (1H,t).

Example 230 (General procedure (C))

5-[1-(2,6-Dichloro-4-trifluoromethylphenyl)-2,5-dimethyl-1*H*-pyrrol-3-ylmethylene]thiazolidine-2,4-dione

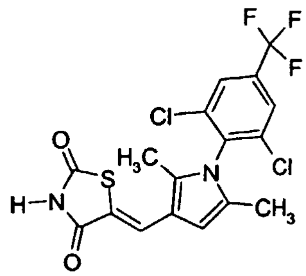

HPLC-MS (Method C): m/z: 436 (M+1); Rt. 4.81 min

Example 231 (General procedure (C))

5-[1-(3,5-Dichlorophenyl)-5-(4-methanesulfonylphenyl)-2-methyl-1*H*-pyrrol-3-ylmethylene]-thiazolidine-2,4-dione

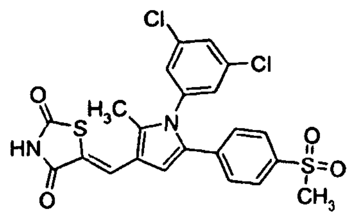

HPLC-MS (Method C): m/z: 508 (M+1); Rt. = 4.31 min

Example 232 (General procedure (C))

5-[1-(2,5-Dimethoxyphenyl)-5-(4-methanesulfonylphenyl)-2-methyl-1*H*-pyrrol-3-ylmethylene]-thiazolidine-2,4-dione

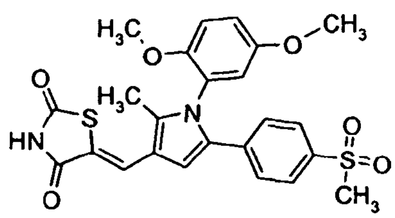

HPLC-MS (Method C): m/z: 499 (M+1); Rt. = 3.70 min

Example 233 (General procedure (C))

4-[3-(2,4-Dioxothiazolidin-5-ylidenemethyl)-2,5-dimethylpyrrol-1-yl]benzoic acid

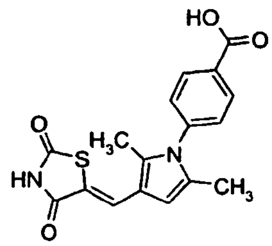

HPLC-MS (Method C): m/z:342 (M+1); Rt.= 3.19 min

Example 234 (General procedure (C))

5-(4-Hydroxy-2,6-dimethoxybenzylidene)thiazolidine-2,4-dione

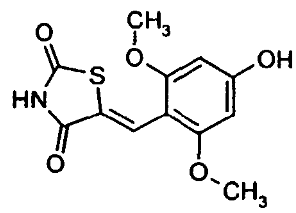

HPLC-MS (Method C): m/z:282(M+1); Rt.= 2.56, mp=331-333 ˚C

Example 235 (General procedure (C))

5-(2,6-Dimethylbenzylidene)thiazolidine-2,4-dione

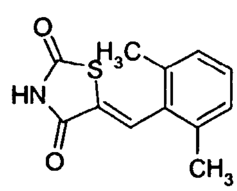

M.p: 104-105 ˚C
HPLC-MS (Method C): m/z: 234 (M+1); Rt.= 3.58 min,

Example 236 (General procedure (C))

5-(2,6-Dimethoxybenzylidene)thiazolidine-2,4-dione

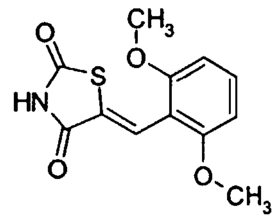

Mp: 241-242 ˚C
HPLC-MS (Method C): m/z: 266 (M+1); Rt.= 3.25 min;

Example 237 (General procedure (C))

5-[4-(2-Fluoro-6-nitrobenzyloxy)-2,6-dimethoxybenzylidene]thiazolidine-2,4-dione

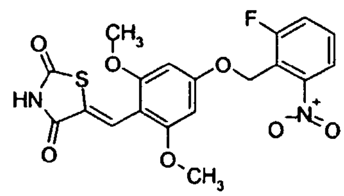

Mp: 255-256 ˚C
HPLC-MS (Method C): m/z: 435 (M+1), Rt 4.13 min,

Example 238 (General procedure (C))

5-Benzofuran-2-ylmethylenethiazolidine-2,4-dione

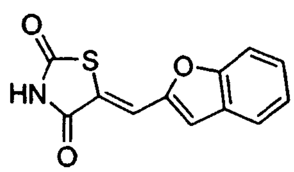

HPLC-MS (Method C): m/z:246 (M+1); Rt.= 3.65 min, mp = 265-266 ˚C .

Example 239 (General procedure (C))

5-[3-(4-Dimethylaminophenyl)allylidene]thiazolidine-2,4-dione

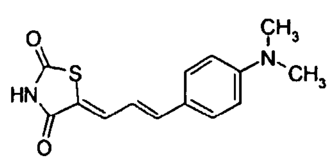

HPLC-MS (Method C): m/z:276(M+1); Rt.= 3.63, mp = 259-263 ˚C

$^1$H-NMR: (DMSO-$d_6$) δ= 12.3 (1H,broad); 7.46 (2H,d); 7.39 (1H,d); 7.11 (1H,d); 6.69 (2H,d); 6.59 (1 H, dd); 2.98 (3H,s).

Example 240 (General procedure (C))

5-(2-Methyl-3-phenylallylidene)thiazolidine-2,4-dione

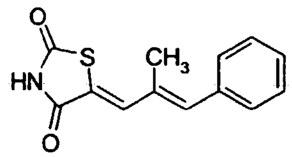

Mp: 203-210 ˚C
HPLC-MS (Method C): m/z: 246 (M+1); Rt = 3.79 min.

Example 241 (General procedure (C))

5-(2-Chloro-3-phenylallylidene)thiazolidine-2,4-dione

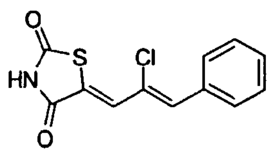

Mp: 251-254 ˚C
HPLC-MS (Method C): m/z: 266 (M+1; Rt= 3.90 min

Example 242 (General procedure (C))

5-(2-Oxo-1,2-dihydroquinolin-3-ylmethylene)thiazolidine-2,4-dione

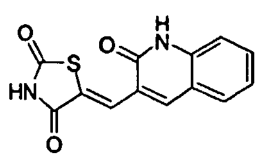

Mp: 338-347 ˚C
HPLC-MS (Method C): m/z: 273 (M+1); Rt. = 2.59 min.

Example 243 (General procedure (C))

5-(2,4,6-Tribromo-3-hydroxybenzylidene)thiazolidine-2,4-dione.

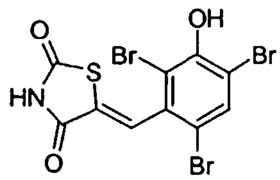

HPLC-MS (Method C): m/z: 459 (M+1);Rt.= 3.65 min.

Example 244 (General procedure (C))

5-(5-Bromo-2-methylindol-3-ylmethylene)thiazolidine-2,4-dione.

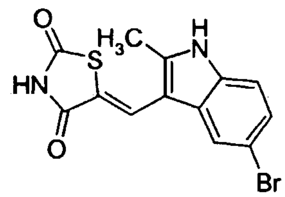

HPLC-MS (Method C): m/z: 339 (M+1); Rt = 3.37min.

Example 245 (General procedure (C))

5-(7-Bromo-2-methylindol-3-ylmethylene)thiazolidine-2,4-dione.

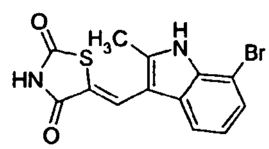

HPLC-MS (Method C): m/z: 319 (M+1); Rt = 3.48min.

Example 246 (General procedure (C))

5-(6-Bromoindol-3-ylmethylene)thiazolidine-2,4-dione.

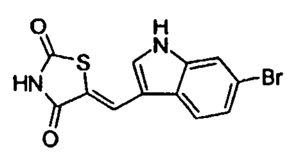

HPLC-MS (Method C): m/z: 325 (M+1); Rt = 3.54 min.

Example 247 (General procedure (C))

5-(8-Methyl-2-oxo-1,2-dihydroquinolin-3-ylmethylene)thiazolidine-2,4-dione.

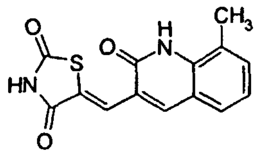

HPLC-MS (Method C): m/z: 287 (M+1); Rt = 2.86 min.

Example 248 (General procedure (C))

5-(6-Methoxy-2-oxo-1,2-dihydroquinolin-3-ylmethylene)thiazolidine-2,4-dione.

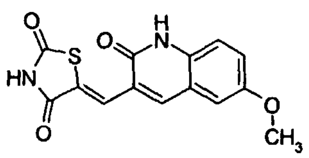

HPLC-MS (Method C): m/z: 303 (M+1); Rt = 2.65 min.

Example 249 (General procedure (C))

5-Quinolin-3-ylmethylenethiazolidine-2,4-dione.

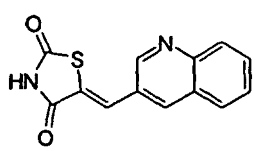

HPLC-MS (Method C): m/z: 257 (M+1); Rt = 2.77 min.

Example 250 (General procedure (C))

5-(8-Hydroxyquinolin-2-ylmethylene)thiazolidine-2,4-dione.

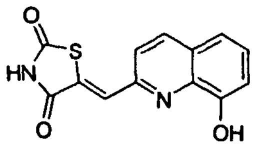

HPLC-MS (Method C): m/z: 273 (M+1); Rt = 3.44 min.

Example 251 (General procedure (C))

5-Quinolin-8-ylmethylenethiazolidine-2,4-dione.

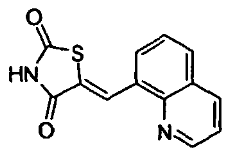

HPLC-MS (Method C): m/z: 257 (M+1); Rt = 3.15 min.

Example 252 (General procedure (C))

5-(1-Bromo-6-methoxynaphthalen-2-ylmethylene)thiazolidine-2,4-dione.

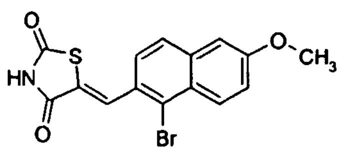

HPLC-MS (Method C): m/z: 366 (M+1); Rt = 4.44 min.

Example 253 (General procedure (C))

5-(6-Methyl-2-oxo-1,2-dihydroquinolin-3-ylmethylene)thiazolidine-2,4-dione.

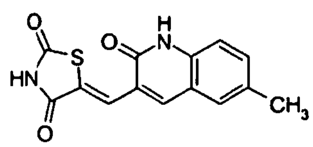

HPLC-MS (Method C): m/z: 287 (M+1); Rt. = 2.89 min.

Example 254 (General procedure (D))

5-(2,6-Dichloro-4-dibenzylaminobenzylidene)thiazolidine-2,4-dione.

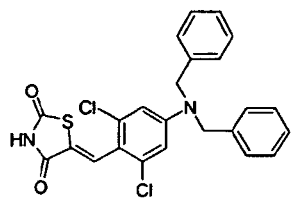

HPLC-MS (Method C): m/z: 469 (M+1); Rt = 5.35 min.

Example 255 (General Procedure (C))

7-(2,4-Dioxothiazolidin-5-ylidenemethyl)-4-methoxybenzofuran-2-carboxylic acid

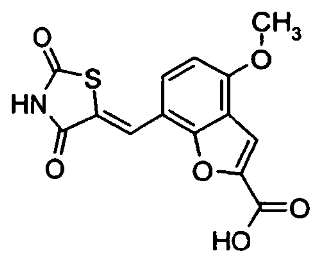

HPLC-MS (Method C): m/z: 320 (M+1); Rt = 2.71 mln.

Preparation of the intermediate, 7-formyl-4-methoxybenzofuran-2-carboxylic acid:

A mixture of 2-hydroxy-6-methoxybenzaldehyde (6.4 g, 42 mmol), ethyl bromoacetate (14.2 mL, 128 mmol) and potassium carbonate (26 g, 185 mmol) was heated to 130 ˚C. After 3 h the mixture was cooled to room temperature and acetone (100 mL) was added, the mixture was subsequently filtered and concentrated *in vacuo.* The residue was purified by column chromatography on silica gel eluting with a mixture of ethyl acetate and heptane (1:4). This afforded 7.5 g (55%) of ethyl 4-methoxybenzofuran-2-carboxylate.

A solution of ethyl 4-methoxybenzofuran-2-carboxylate (6.9 g, 31.3 mmol) in dichloromethane (70 ml) was cooled to 0 ˚C and a solution of titanium tetrachloride (13.08 g, 69 mmol) was added drop wise. After 10 minutes dichloromethoxymethane (3.958 g, 34 mmol) was added over 10 minutes. After addition, the mixture was warmed to room temperature for 18 hours and the mixture poured into hydrochloric acid (2N, 100 mL). The mixture was stirred for 0.5 hour and then extracted with a mixture of ethyl acetate and toluene (1:1). The organic phase was dried over $Na_2SO_4$ and concentrated *in vacuo.* The residue was purified by column chromatography on silica gel eluting with a mixture of ethyl acetate and heptane (1:4). This afforded 5.8 g (80%) of ethyl 7-formyl-4-methoxybenzofuran-2-carboxylate.

7-formyl-4-methoxybenzofuran-2-carboxylate (5.0 g, 21.5 mmol) and sodium carbonate (43 mmol) in water (100 mL) was refluxed until a clear solution appeared (about 0.5 hour). The solution was filtered and acidified to pH =1 with hydrochloric acid (2 N), the resulting product was filtered off and washed with ethyl acetate and ethanol and dried to afford 3.5 g (74%) of 7-formyl-4-methoxybenzofuran-2-carboxylic acid as a solid.
$^1$H NMR (DMSO-$d_6$): $\delta$ = 10.20 (s, 1H) ; 8.07 (d, 1H) ; 7.70 (s, 1H) ; 7.17 (d, 1H) ; 4.08 (s, 3H).

Example 256 (General Procedure (C))

5-(4-Methoxybenzofuran-7-ylmethylene)thiazolidine-2,4-dione

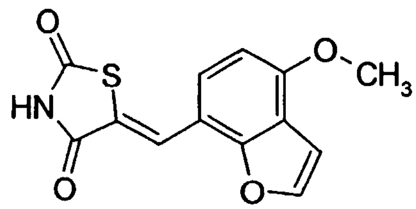

HPLC-MS (Method C): m/z: 267 (M+1); Rt = 3.30 min.

Preparation of the intermediate, 4-methoxybenzofuran-7-carbaldehyde:

A mixture of 7-formyl-4-methoxybenzofuran-2-carboxylic acid (3.0 g, 13.6 mmol) and Cu (0.6 g, 9.44 mmol) in quinoline (6 mL) was refluxed. After 0.5 h the mixture was cooled to room temperature and water (100 mL) and hydrochloric acid (10 N, 20 mL) were added. The mixture was extracted with a mixture of ethyl acetate and toluene (1:1), filtered through celite and the organic layer separated and washed with a sodium carbonate solution, dried over $Na_2SO_4$ and concentrated *in vacuo* to afford 1.5 g crude product. Column chromatography $SiO_2$, EtOAc/heptanes=1/4 gave 1.1 g (46%) of 4-methoxybenzofuran-7-carbaldehyde as a solid.
$^1$H NMR ($CDCl_3$): δ: 10.30 (s,1H) ; 7.85 (d,1H) ; 7.75 (d,1H) ; 6.98 (d,1H) ; 6.87 (d,1H) ; 4.10 (s,3H). HPLC-MS (Method C) :m/z: 177 (M+1); Rt. = 7.65 min.

Example 257 (General Procedure (C))

5-(4-Hydroxybenzofuran-7-ylmethylene)thiazolidine-2,4-dione

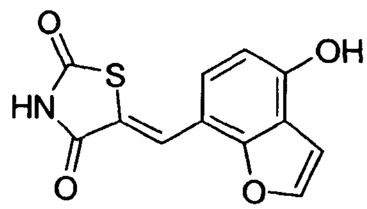

HPLC-MS (Method C): m/z: = 262 (M+1); Rt 2.45 min.

Preparation of the intermediate, 4-hydroxybenzofuran-7-carbaldehyde

A mixture of 4-methoxybenzofuran-7-carbaldehyde (1.6 g, 9.1 mmol) and pyridine hydrochloride (4.8 g, 41.7mmol) in quinoline (8 mL) was refluxed. After 8 h the mixture was cooled to room temperature and poured into water (100 mL) and hydrochloric acid (2 N) was added to pH = 2. The mixture was extracted with a mixture of ethyl acetate and toluene (1:1), washed with a sodium carbonate solution, dried with $Na_2SO_4$ and concentrated *in vacuo* to afford 0.8 g crude product. This was purified by column chromatography on silica gel, eluting with a mixture of ethyl acetate and heptane (1:3). This afforded 250 mg of 4-hydroxybenzofuran-7-carbaldehyde as a solid.
$^1$H NMR (DMSO-$d_6$): δ = 11.35 (s, broad,1H) ; 10.15 (s, 1H); 8.05 (d, 1H); 7.75 (d, 1H); 7.10 (d, 1H); 6.83 (d, 1H). HPLC-MS (Method C): m/z: 163 (M+1); Rt. = 6.36 min.

Example 258 (General Procedure (C))

5-(5-Bromo-2,3-dihydrobenzofuran-7-ylmethylene)thiazolidine-2,4-dione

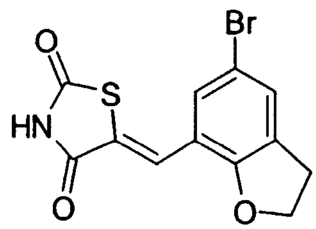

HPLC-MS (Method C): m/z: 328 (M+1); Rt = 3.66 min.

Preparation of the intermediate, 5-bromo-2,3-dihydrobenzofuran-7-carbaldehyde:

To a cooled (15 °C) stirred mixture dihydrobenzofuran (50.9 g, 0.424 mol) in acetic acid (500 mL), a solution of bromine (65.5 mL, 1.27 mol) in acetic acid (200 mL) was added drop wise over 1 hour. After stirring for 18 hours, a mixture of $Na_2S_2O_5$ (150 g) in water (250 mL) was added carefully, and the mixture was concentrated *in vacuo.* Water (200 mL) was added and the mixture was extracted with ethyl acetate containing 10% heptane, dried over $Na_2SO_4$ and concentrated *in vacuo* to give crude 5,7-dibromo-2,3-dihydrobenzofuran which was used as such for the following reaction steps. To a cooled solution (-78 °C) of crude 5,7-dibromo-2,3-dihydrobenzofuran (50.7 g, 0.182 mol) in THF (375 mL) a solution of n-BuLi (2.5 M, 80 mL, 0.200 mol) in hexane was added. After addition, the mixture was stirred for 20 min. DMF (16 mL) was then added drop wise at -78 °C. After addition, the mixture was stirred at room temperature for 3 h and then the mixture was poured into a mixture of ice water, (500 mL) and hydrochloric acid (10 N, 40 mL) and extracted with toluene, dried over $Na_2SO_4$ and concentrated *in vacuo.* Column chromatography on silica gel eluting with a mixture of ethyl acetate and heptane (1:4) afforede 23 g of 5-bromo-2,3-dihydrobenzofuran-7-carbaldehyde as a solid. $^1H$ NMR ($CDCl_3$): δ:10.18 (s,1H); 7.75 (d,1H) ;7.55 (d,1H) ; 4.80 (t,2H) ; 3.28 (t,2H).

Example 259 (General Procedure (C))

5-(4-Cyclohexylbenzylidene)thiazolidine-2,4-dione

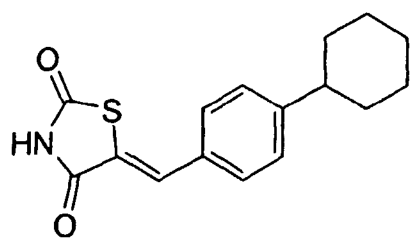

HPLC-MS (Method C): m/z: 288 (M+1); Rt = 5.03 min.

Preparation of the intermediate, 4-cyclohexylbenzaldehyde:

This compound was synthesized according to a modified literature procedure (J. Org. Chem., 37, No.24, (1972), 3972-3973).
Cyclohexylbenzene (112.5 g, 0.702 mol) and hexamethylenetetramine (99.3 g, 0.708 mol) were mixed in TFA (375 mL). The mixture was stirred under nitrogen at 90 °C for 3 days. After cooling to room temperature the red-brown mixture was poured into ice-water (3600 ml) and stirred for 1 hour. The solution was neutralized with $Na_2CO_3$ (2 M solution in water) and extracted with dichloromethane (2.5 L). The organic phase was dried ($Na_2SO_4$) and the solvent was removed *in vacuo.* The remaining red-brown oil was purified by fractional distillation to afford the title compound (51 g, 39%).
$^1H$ NMR ($CDCl_3$): δ 9.96 (s, 1H), 7.80 (d, 2H), 7.35 (d, 2H), 2.58 (m, 1H), 1.94-1.70 (m, 5 H), 1.51-1.17 (m, 5H)
Other ligands of the invention include

3',5'-Dichloro-4'-(2,4-dioxothiazolidin-5-ylidenemethyl)biphenyl-4-carboxylic acid:

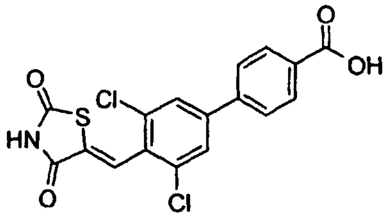

Example 260 (General procedure (C))

5-(1-Bromo-6-hydroxynaphthalen-2-ylmethylene)-thiazolidine-2,4-dione

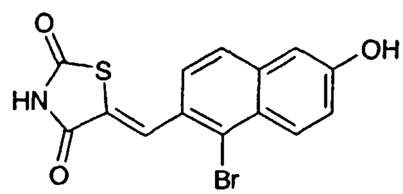

HPLC-MS (Method C): m/z = 350 (M+1); Rt. = 3.45 min.

Example 261 (General procedure (C))

5-[4-(2-Bromoethoxy)-naphthalen-1-ylmethylene]-thiazolidine-2,4-dione

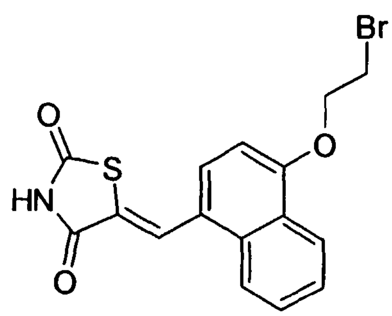

HPLC-MS (Method C): m/z = 380 (M+1); Rt = 3.52 min.

Example 262 (General procedure (C))

5-(2-Methyl-5-nitro-1H-indol-3-ylmethylene)-thiazolidine-2,4-dione

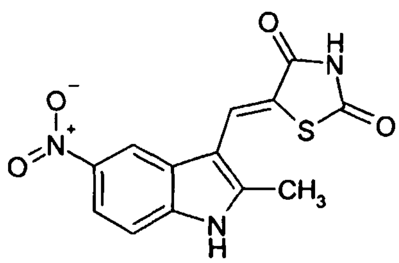

HPLC-MS (Method C): m/z = 304 (M+1); Rt = 2.95 min.

Example 263 (General procedure (C))

5-(4-Naphthalen-2-yl-thiazol-2-ylmethylene)-thiazolidine-2,4-dione

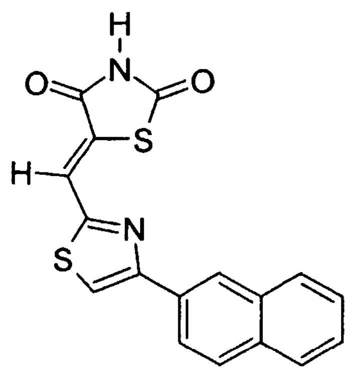

HPLC-MS (Method C): m/z = 339 (M+1); Rt.= 4.498 min.

Example 264 (General procedure (C))

5-[4-(4-Methoxy-naphthalen-1-yl)-thiazol-2-ylmethylene]-thiazolidine-2,4-dione

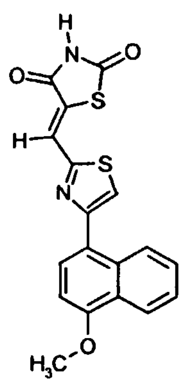

HPLC-MS (Method C): m/z = 369 (M+1); Rt.= 4.456 min.

Example 265 (General procedure (C))

5-(2-Pyridin-4-yl-1H-indol-3-ylmethylene)-thiazolidine-2,4-dione

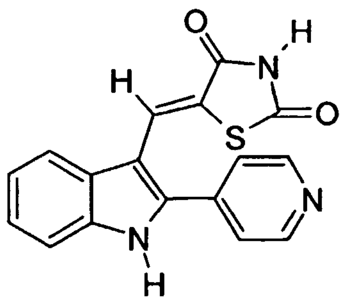

HPLC-MS (Method C): m/z = 322 (M+1); Rt. = 2.307 min.

Example 266 (General procedure (C))

5-[5-(4-Chlorophenyl)-1H-pyrazol-4-ylmethylene]-thiazolidine-2,4-dione

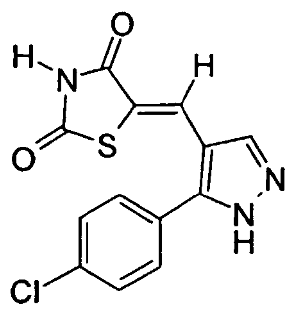

HPLC-MS (Method C): m/z = 306 (M+1); Rt.= 3.60 min.

Example 267 (General procedure (C))

5-[5-(2,5-Dimethylphenyl)-1H-pyrazol-4-ylmethylene]-thiazolidine-2,4-dione

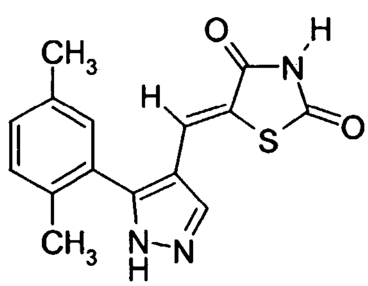

HPLC-MS (Method C): m/z = 300 (M+1); Rt. = 3.063 min.

Example 268 (General procedure (C))

5-(2-Phenyl-benzo[d]imidazo[2,1-b]thiazol-3-ylmethylene)-thiazolidine-2,4-dione

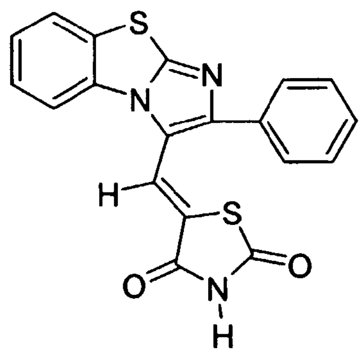

HPLC-MS (Method C): m/z = 378 (M+1); Rt = 3.90 min.

Example 269 (General procedure (C))

N-{4-[2-(2,4-Dioxothiazolidin-5-ylidenemethyl)-phenoxy]-phenyl}-acetamide

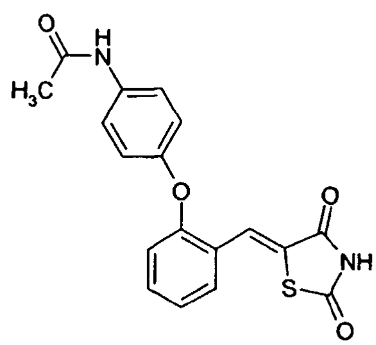

HPLC-MS (Method C): m/z = 355 (M+1); Rt 3.33 min.

Example 270 (General procedure (C))

5-(2-Phenyl-imidazo[1,2-a]pyridin-3-ylmethylene)-thiazolidine-2,4-dione

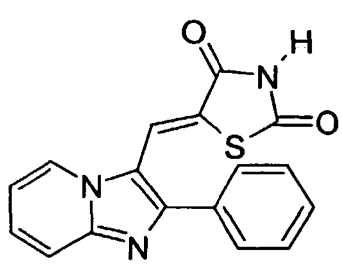

HPLC-MS (Method C): m/z = 322 (M+1); Rt. = 2.78 min.

Example 271 (General procedure (C))

5-(2-Naphthalen-2-yl-imidazo[1,2-a]pyridin-3-ylmethylene)-thiazolidine-2,4-dione

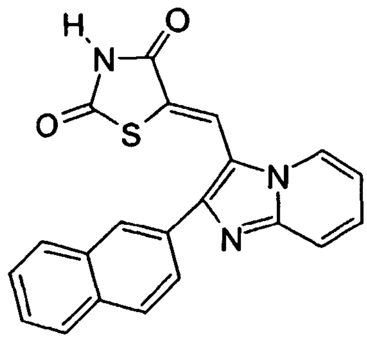

HPLC-MS (Method C): m/z = 372 (M+1); Rt. = 2.78 min.

Example 272 (General procedure (C))

5-[6-Bromo-2-(3-methoxyphenyl)-imidazo[1,2-a]pyridin-3-ylmethylene]-thiazolidine-2,4-dione

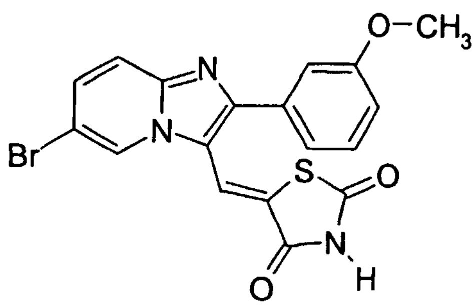

HPLC-MS (Method C): m/z = 431 (M+1); Rt.= 3.30 min.

Example 273 (General procedure (C))

5-(1,2,3,4-Tetrahydrophenanthren-9-ylmethylene)thiazolidine-2,4-dione

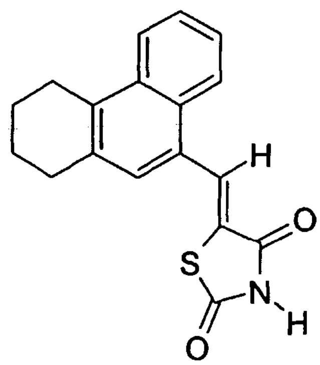

HPLC-MS (Method C): m/z = 310 (M+1); Rt.= 4.97 min.

Example 274 (General procedure (C))

5-(3,5,5,8,8-Pentamethyl-5,6,7,8-tetrahydro-naphthalen-2-ylmethylene)thiazolidine-2,4-dione

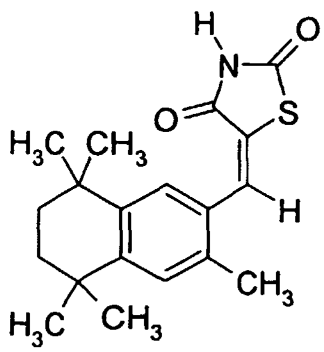

HPLC-MS (Method C): m/z = 330 (M+1); Rt.= 5.33 min.

Example 275 (General procedure (C))

5-[6-(2,4-Dichloro-phenyl)-imidazo[2,1-b]thiazol-5-ylmethylene]-thiazolidine-2,4-dione

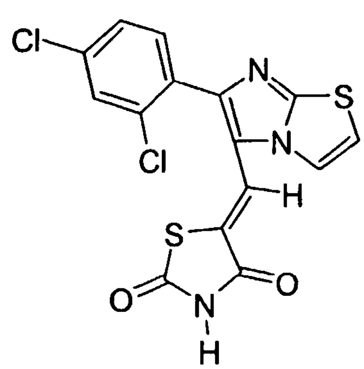

HPLC-MS (Method C): m/z = 396 (M+1); Rt. = 3.82 min.

Example 276 (General procedure (C))

5-(5-Bromobenzofuran-7-ylmethylene)-thiazolidine-2,4-dione

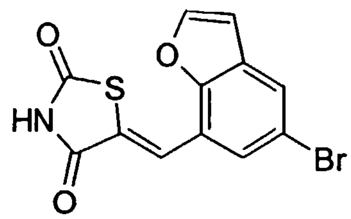

HPLC-MS (Method C): m/z = 324 (M+1); Rt. = 3.82 min.

Example 277 (General procedure (C))

4-[3-(2,4-Dioxothiazolidin-5-ylidenemethyl)-1,4-dimethylcarbazol-9-ylmethyl]-benzoic acid

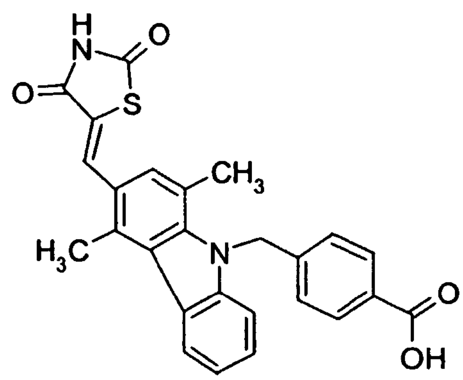

HPLC-MS (Method C): m/z = 457 (M+1); Rt = 4,23 min.

Preparation of intermediary aldehyde:

1,4 Dimethylcarbazol-3-carbaldehyde (0.68 g, 3.08 mmol) was dissolved in dry DMF (15 mL), NaH (diethyl ether washed) (0.162 g, 6.7 mol) was slowly added under nitrogen and the mixture was stirred for 1 hour at room temperature. 4-Bromomethylbenzoic acid (0.73 g, 3.4 mmol) was slowly added and the resulting slurry was heated to 40 ˚C for 16 hours. Water (5 mL) and hydrochloric acid (6N, 3 mL) were added. After stirring for 20 min at room temperature, the precipitate was filtered off and washed twice with acetone to afford after drying 0.38 g (34%) of 4-(3-formyl-1,4-dimethylcarbazol-9-ylmethyl)benzoic acid.
HPLC-MS (Method C): m/z = 358 (M+1), RT. = 4.15 min.

Example 278 (General procedure (C))

4-[7-(2,4-Dioxothiazolidin-5-ylidenemethyl)-benzofuran-5-yl]-benzoic acid

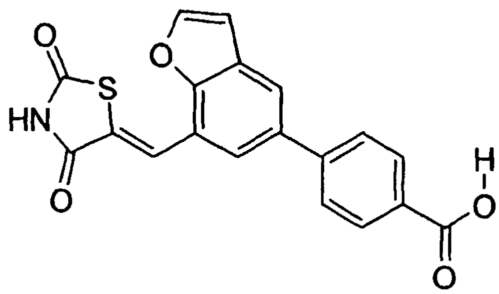

Starting aldehyde commercially available (Syncom BV, NL)
HPLC-MS (Method C): m/z = 366 (M+1); Rt. = 3.37 min.

Example 279 (General procedure (C))

4-[4-(2,4-Dioxothiazolidin-5-ylidenemethyl)-2-nitrophenoxy]-benzoic acid methyl ester

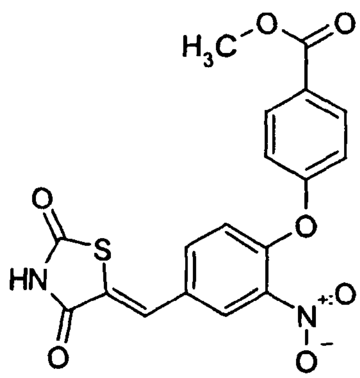

HPLC-MS (Method C): m/z = 401 (M+1); Rt. = 4.08 min.

Example 280 (General procedure (C))

3',5'-Dichloro-4'-(2,4-dioxothiazolidin-5-ylidenemethyl)-biphenyl-4-carboxylic acid

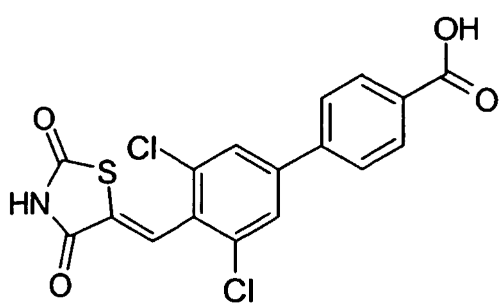

Starting aldehyde commercially available (Syncom BV, NL)
HPLC-MS (Method C): m/z = 394 (M+1); Rt. = 3.71 min.

Example 281 (General procedure (C))

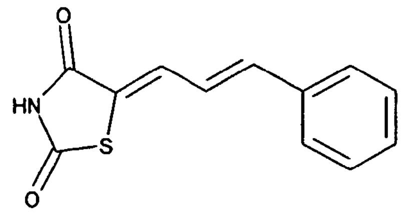

HPLC-MS (Method C): m/z = 232(M+1); Rt.= 3.6 min.

Example 282

5-(2-Methyl-1H-indol-3-ylmethyl)-thiazolidine-2,4-dione

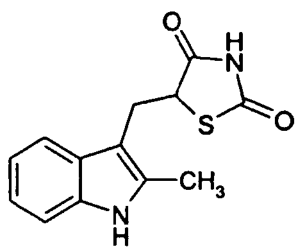

5-(2-Methyl-1H-indol-3-ylmethylene)thiazolidine-2,4-dione (prepared as described in example 187, 1.5 g, 5.8 mmol) was dissolved in pyridine (20 mL) and THF (50 mL), $LiBH_4$ (2 M in THF, 23.2 mmol) was slowly added with a syringe under cooling on ice. The mixture was heated to 85 °C for 2 days. After cooling, the mixture was acidified with concentrated hydrochloric acid to pH 1. The aquous layer was extracted 3 times with ethyl acetate, dried with $MgSO_4$ treated with activated carbon, filtered and the resulting filtrate was evaporated *in vacuo* to give 1.3 g (88%) of the title compound.
HPLC-MS (Method C): m/z = 261 (M+1); Rt. = 3.00 min.

Example 283

4-[4-(2,4-Dioxothiazolidin-5-ylmethyl)naphthalen-1-yloxy]butyric acid

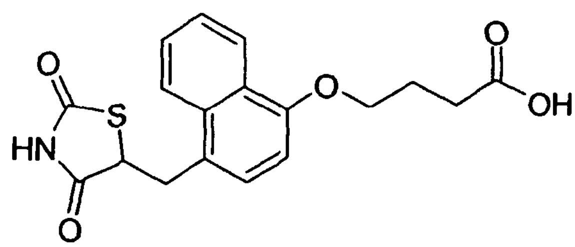

4-[4-(2,4-Dioxothiazolidin-5-ylidenemethyl)naphthalen-1-yloxy]butyric acid (4.98 g, 13.9 mmol, prepared as described in example 469) was dissolved in dry THF (50 mL) and added dry pyridine (50 mL) and, in portions, lithium borohydride (2.0 M, in THF, 14 mL). The resulting slurry was refluxed under nitrogen for 16 hours, added (after cooling) more lithium borohydride (2.0 M, in THF, 7 mL). The resulting mixture was refluxed under nitrogen for 16 hours. The

mixture was cooled and added more lithium borohydride (2.0 M, in THF, 5 mL). The resulting mixture was refluxed under nitrogen for 16 hours. After cooling to 5 °C, the mixture was added water (300 mL) and hydrochloric acid (150 mL). The solid was isolated by filtration, washed with water (3 x 500 mL) and dried. Recrystallization from acetonitrile (500 mL) afforded2.5 g of the title compound.
$^1$H-NMR (DMSO-$d_6$, selected peaks): δ = 3.42 (1H, dd), 3.90 (1H, dd), 4.16 (2H, "t"), 4.95 (1H, dd), 6.92 (1H, d), 7.31 (1H, d), 7.54 (1H, t), 7.62 (1H, t), 8.02 (1H, d), 8.23 (1H, d), 12.1 (1H, bs), 12.2 (1H, bs).
HPLC-MS (Method C): m/z = 382 (M+23); Rt = 3,23 min.

Example 284

5-Naphthalen-1-ylmethylthiazolidine-2,4-dione

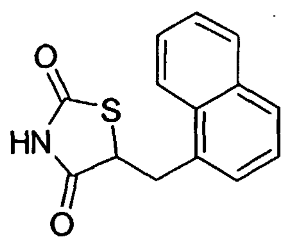

5-Naphthalen-1-ylmethylenethiazolidine-2,4-dione (1.08 g, 4.2 mmol, prepared as described in example 68) was dissolved in dry THF (15 mL) and added dry pyridine (15 mL) and, in portions, lithium borohydride (2.0 M, in THF, 4.6 mL). The resulting mixture was refluxed under nitrogen for 16 hours. After cooling to 5 °C, the mixture was added water (100 mL), and, in portions, concentrated hydrochloric acid (40 mL). More water (100 mL) was added, and the mixture was extracted with ethyl acetate (200 mL). The organic phase was washed with water (3 x 100 mL), dried and concentrated *in vacuo.* The residue was dissolved in ethyl acetate (50 mL) added activated carbon, filtered and concentrated in vacuo and dried to afford 0.82 g (75%) of the title compound.
$^1$H-NMR (DMSO-$d_6$): δ = 3.54 (1H, dd), 3.98 (1H, dd), 5.00 (1H, dd), 7.4-7.6 (4H, m), 7.87 (1H, d), 7.96 (1H, d), 8.11 (1H, d), 12.2 (1H, bs).
HPLC-MS (Method C): m/z = 258 (M+1); Rt = 3,638 min.

The following preferred compounds of the invention may be prepared according to procedures similar to those described in the three examples above:

| | | |
|---|---|---|
| Example 285 | 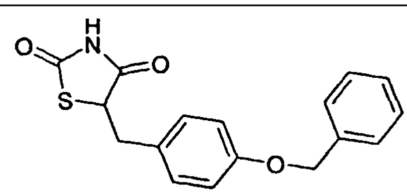 | |
| Example 286 | 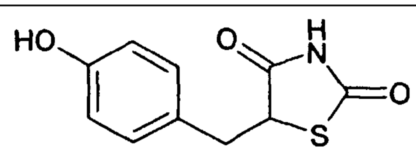 | |
| Example 287 | 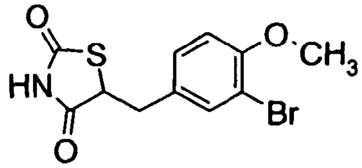 | |

(continued)

| | | |
|---|---|---|
| Example 288 | 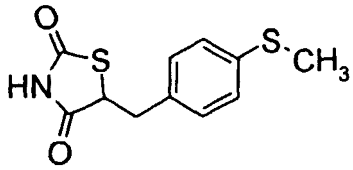 | |
| Example 289 | 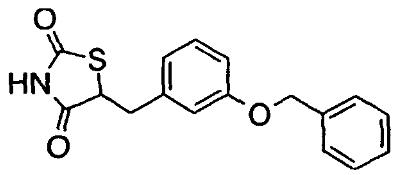 | |
| Example 290 | 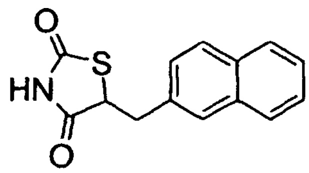 | |
| Example 291 | 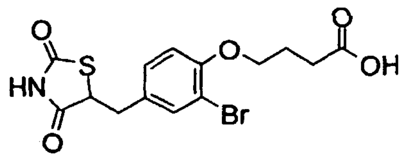 | |
| Example 292 | 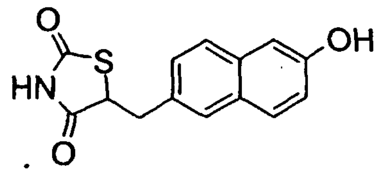 | |
| Example 293 | 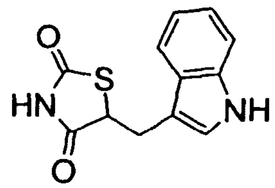 | |
| Example 294 | 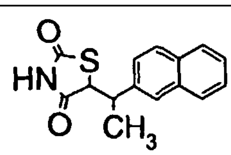 | |
| Example 295 | 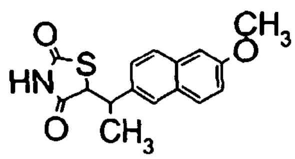 | |

(continued)

| | | |
|---|---|---|
| Example 296 | 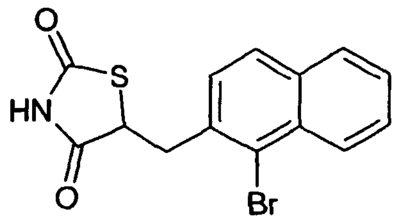 | |
| | | |
| Example 297 | 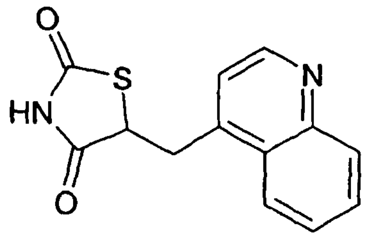 | |
| Example 298 | 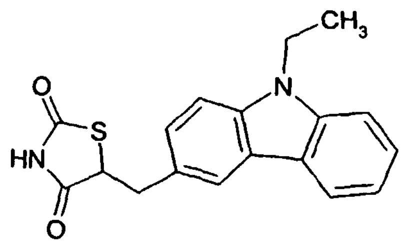 | |
| Example 299 | 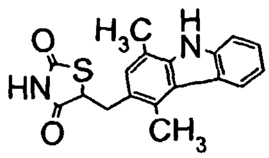 | |
| Example 300 | 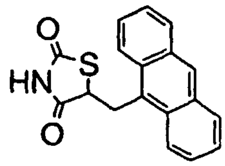 | |
| Example 301 | 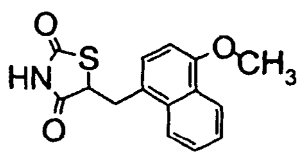 | |
| Example 302 | 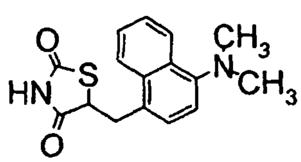 | |

(continued)

| | | |
|---|---|---|
| Example 303 | 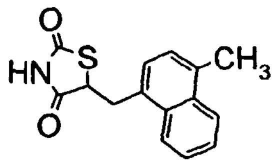 | |
| Example 304 | 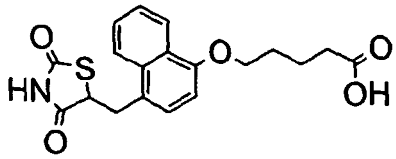 | |
| Example 305 | 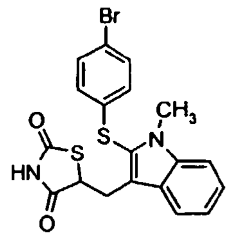 | |
| Example 306 | 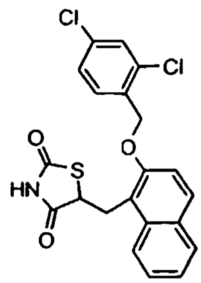 | |
| Example 307 | 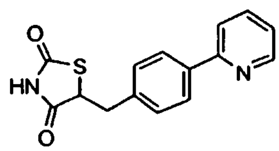 | |
| Example 308 | 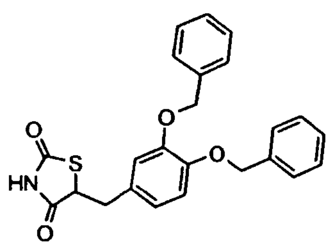 | |
| Example 309 | 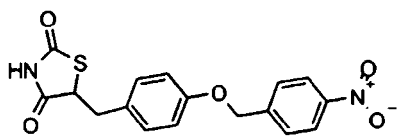 | |

(continued)

| | | |
|---|---|---|
| Example 310 | 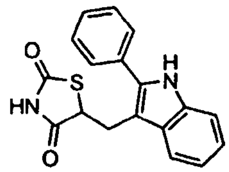 | |
| Example 311 | 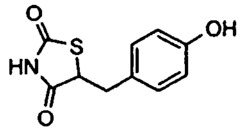 | |
| Example 312 | 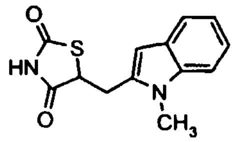 | |
| Example 313 | 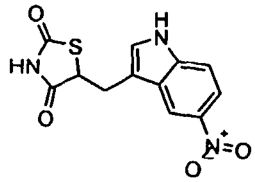 | |
| Example 314 | 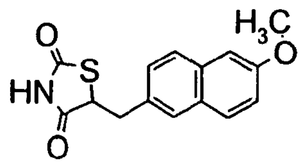 | |
| Example 315 | 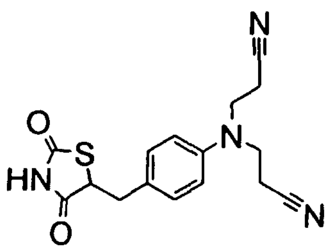 | |
| Example 316 | 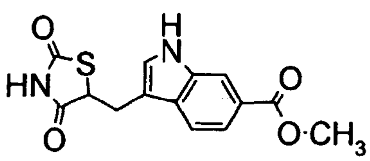 | |
| Example 317 | 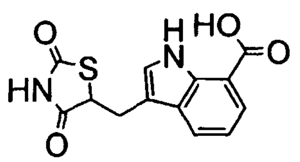 | |

(continued)

| | | |
|---|---|---|
| Example 318 | 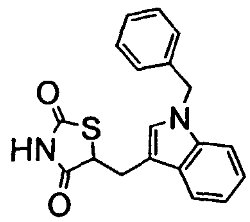 | |
| Example 319 | 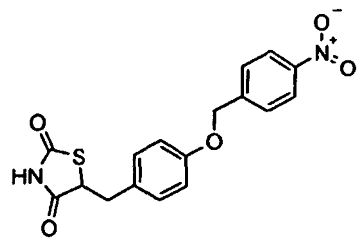 | |
| Example 320 | 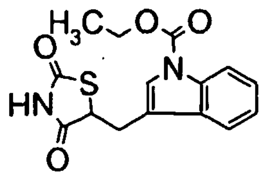 | |
| Example 321 | 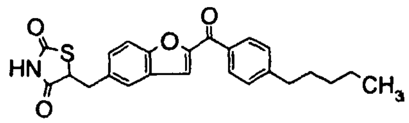 | |
| Example 322 | 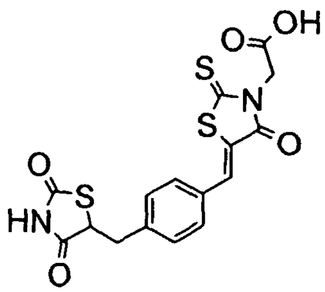 | |
| Example 323 | 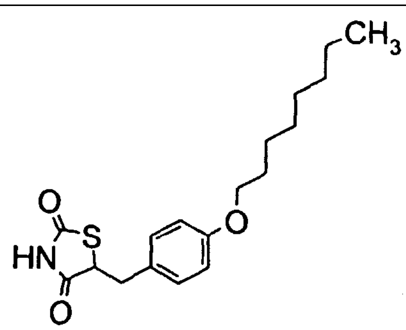 | |
| Example 324 | 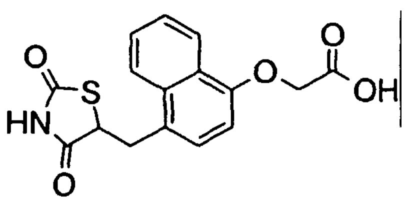 | |

(continued)

| | | |
|---|---|---|
| Example 325 | 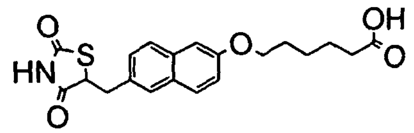 | |
| Example 326 | 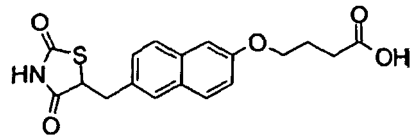 | |
| Example 327 | 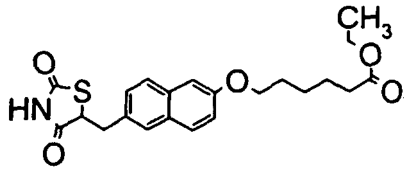 | |
| Example 328 | 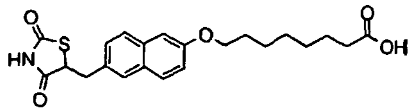 | |
| | | |
| Example 329 | 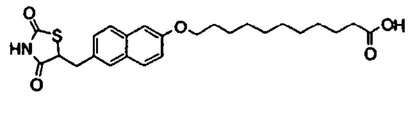 | |
| Example 330 | 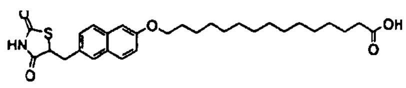 | |
| Example 331 | 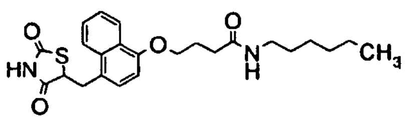 | |
| Example 332 | 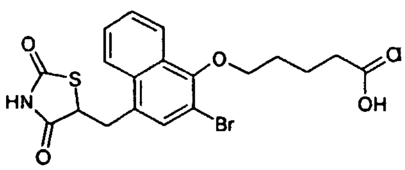 | |
| Example 333 | 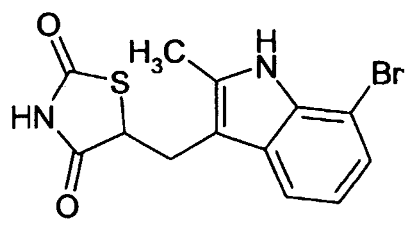 | |
| Example 334 | 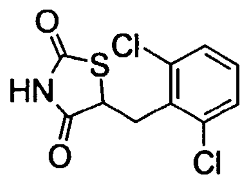 | |

(continued)

| | | |
|---|---|---|
| Example 335 | 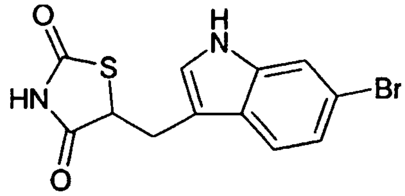 | |
| Example 336 | 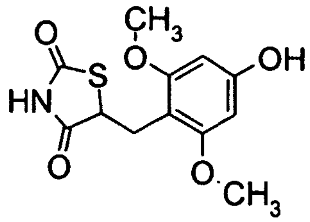 | |
| Example 337 | 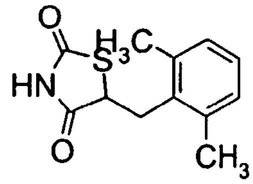 | |
| Example 338 | 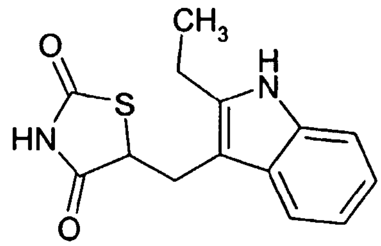 | |
| Example 339 | 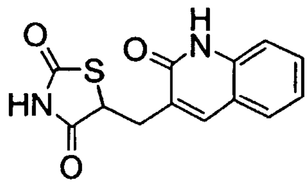 | |
| Example 340 | 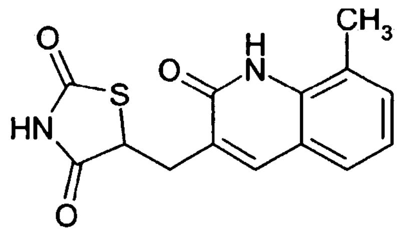 | |
| Example 341 | 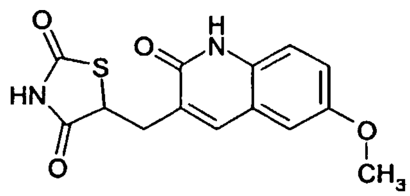 | |

(continued)

| | | |
|---|---|---|
| Example 342 | 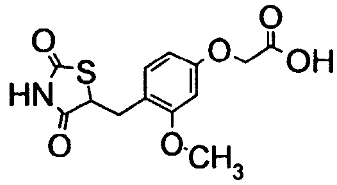 | |
| Example 343 | 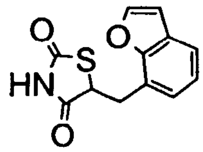 | |
| Example 344 | 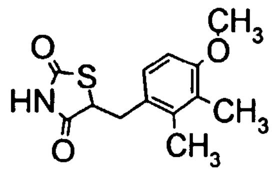 | |
| Example 345 | 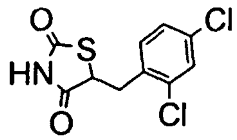 | |
| Example 346 | 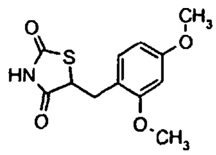 | |
| Example 347 | 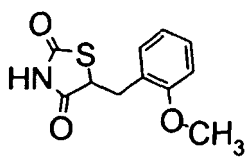 | |
| Example 348 | 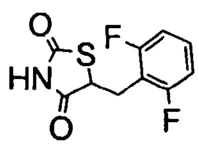 | |
| Example 349 | 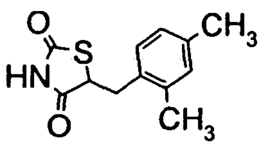 | |
| Example 350 | 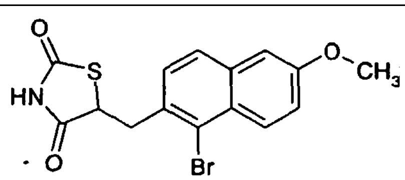 | |

(continued)

| | | |
|---|---|---|
| Example 351 | 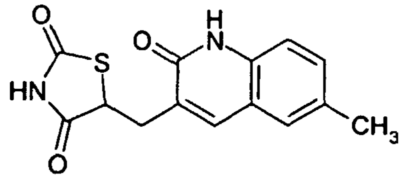 | |
| Example 352 | 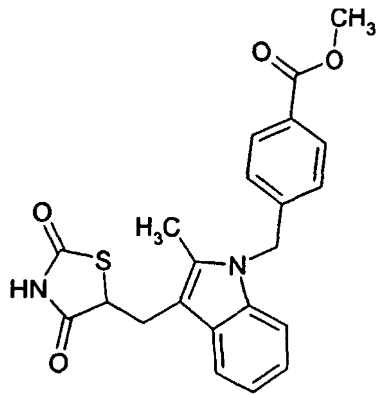 | |
| Example 353 | 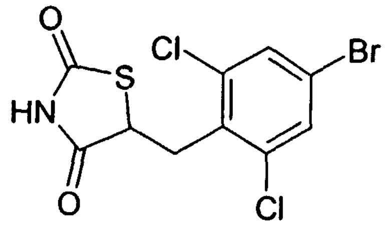 | |
| Example 354 | 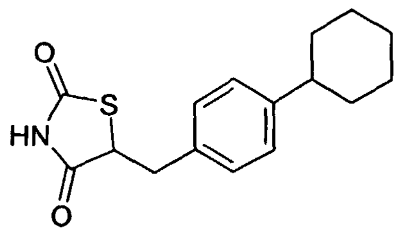 | |
| Example 355 | 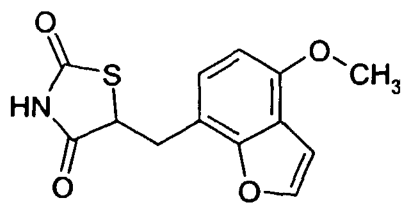 | |
| Example 356 | 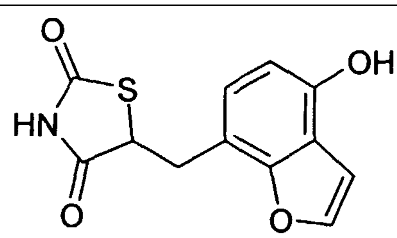 | |

(continued)

| | | |
|---|---|---|
| Example 357 | 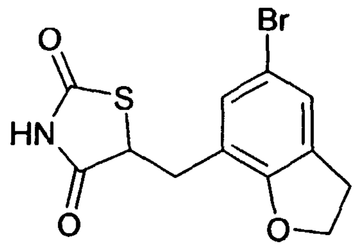 | |
| Example 358 | 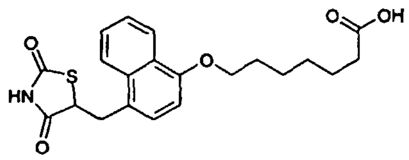 | |
| Example 359 | 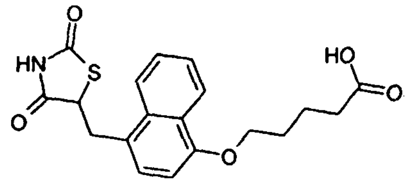 | |
| Example 360 | 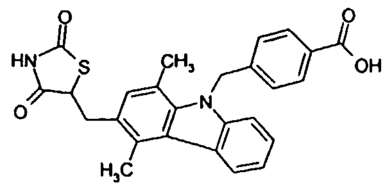 | |
| Example 361 | 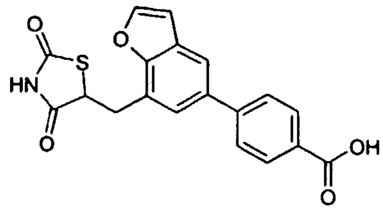 | |
| Example 362 | 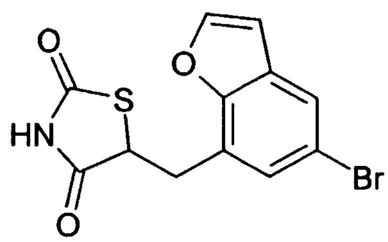 | |
| Example 363 | 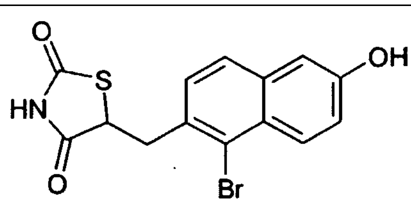 | |

(continued)

| | | |
|---|---|---|
| Example 364 | 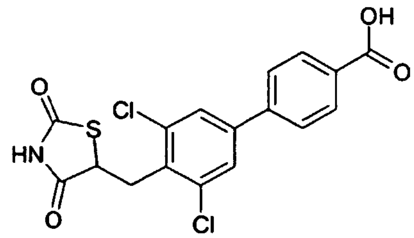 | |
| Example 365 | 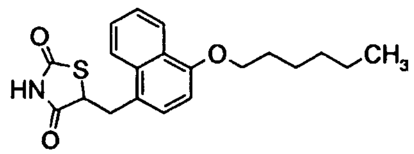 | |
| Example 366 | 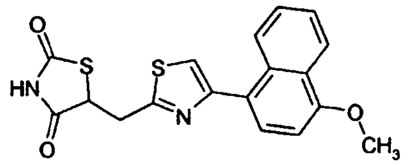 | |
| Example 367 | 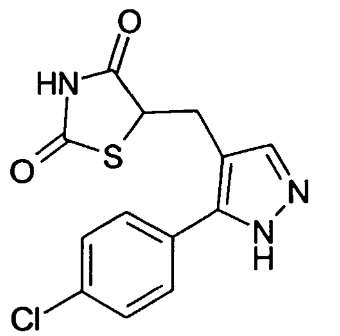 | |
| Example 368 | 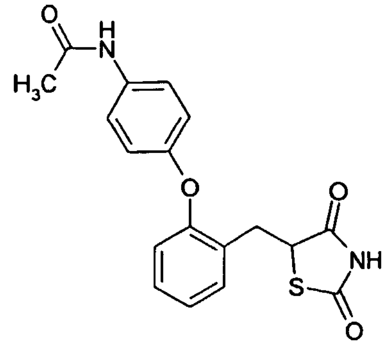 | |
| Example 369 | 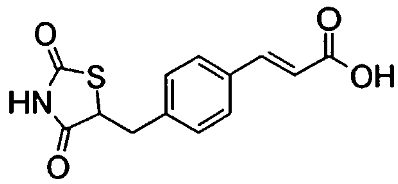 | |

(continued)

| | | |
|---|---|---|
| Example 370 | 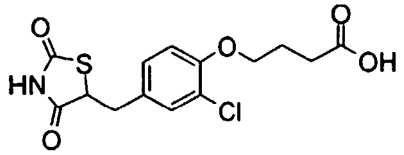 | |
| Example 371 | 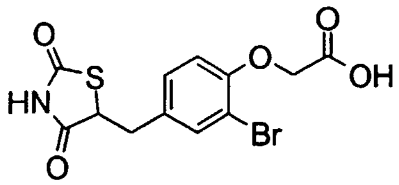 | |
| Example 372 | 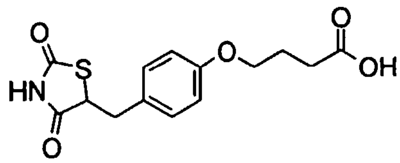 | |
| Example 373 | 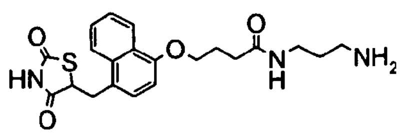 | |
| Example 374 | 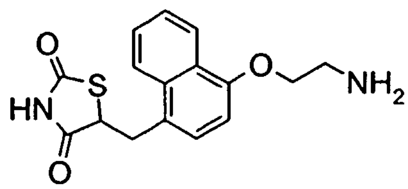 | |
| Example 375 | 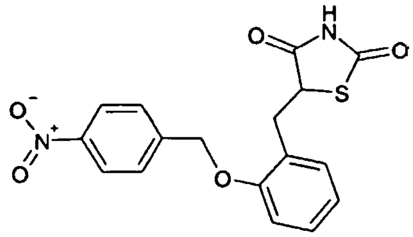 | |
| Example 376 | 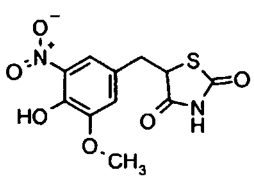 | |
| Example 377 | 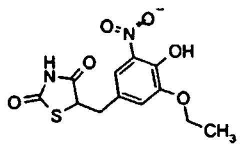 | |

(continued)

| | | |
|---|---|---|
| Example 378 | 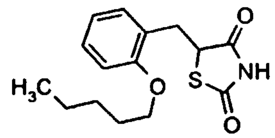 | |
| Example 379 | 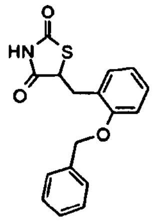 | |

The following compounds are commercially available and may be prepared using general procedures (B) and / or (C).

Example 380

5-(5-Bromo-1H-indol-3-ylmethylene)thiazolidine-2,4-dione

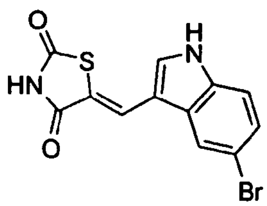

Example 381

5-Pyridin-4-ylmethylenethiazolidine-2,4-dione

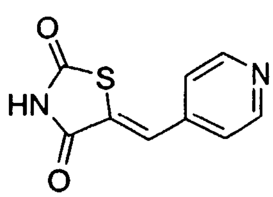

Example 382

5-(3-Bromo-4-methoxybenzylidene)thiazolidine-2,4-dione

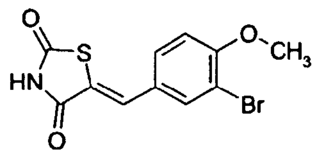

Example 383

5-(3-Nitrobenzylidene)thiazolidine-2,4-dione

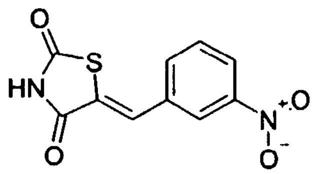

Example 384

5-Cyclohexylidene-1,3-thiazolidine-2,4-dione

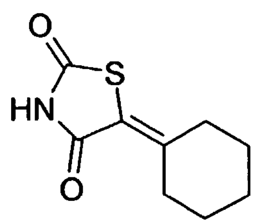

Example 385

5-(3,4-Dihydroxybenzylidene)thiazolidine-2,4-dione

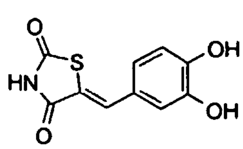

Example 386

5-(3-Ethoxy-4-hydroxybenzylidene)thiazolidine-2,4-dione

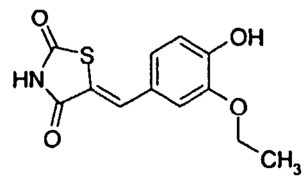

Example 387

5-(4-Hydroxy-3-methoxy-5-nitrobenzylidene)thiazolidine-2,4-dione

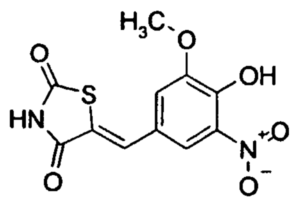

Example 388

5-(3-Ethoxy-4-hydroxybenzylidene)thiazolidine-2,4-dione

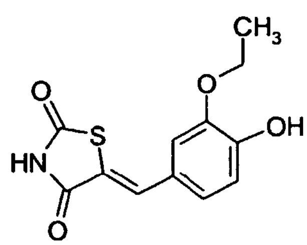

Example 389

5-(4-Hydroxy-3,5-dimethoxybenzylidene)thiazolidine-2,4-dione

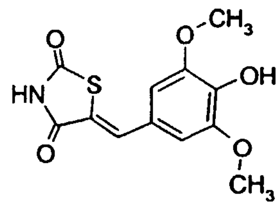

Example 390

5-(3-Bromo-5-ethoxy-4-hydroxybenzylidene)thiazolidine-2,4-dione

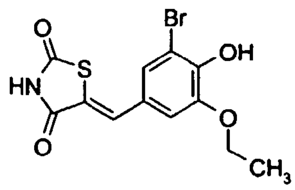

Example 391

5-(3-Ethoxy-4-hydroxy-5-nitrobenzylidene)thiazolidine-2,4-dione

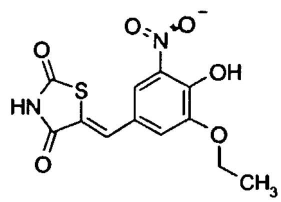

Example 392

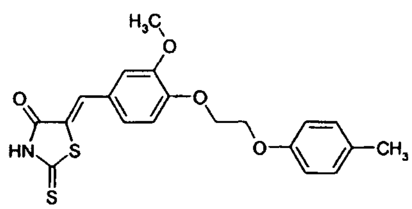

Example 393

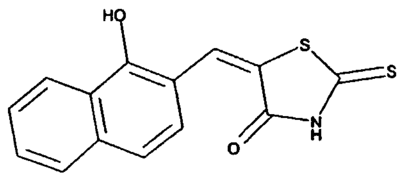

Example 394

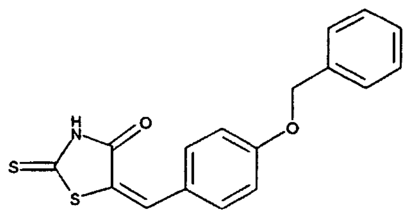

Example 395

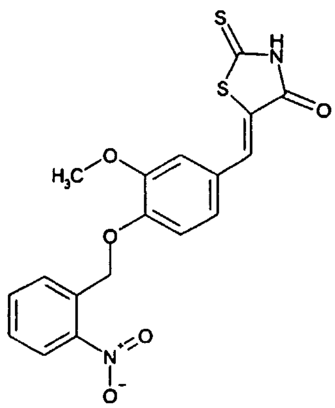

**Example 396**

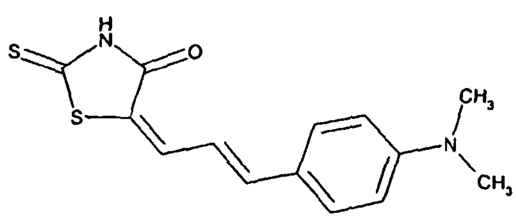

Example 397

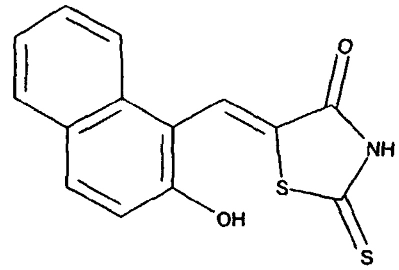

Example 398

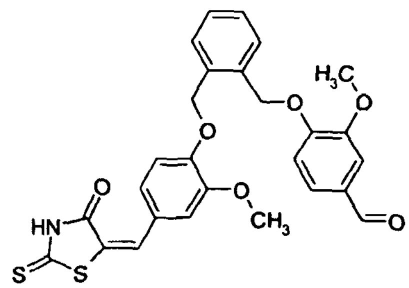

Example 399

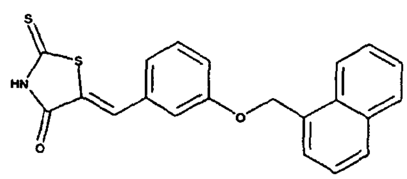

Example 400

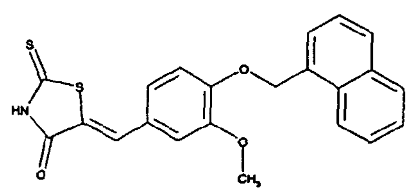

Example 401

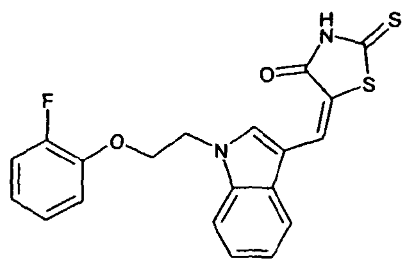

Example 402

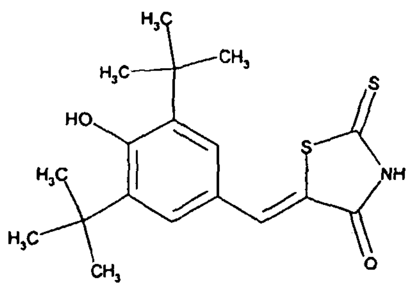

Example 403

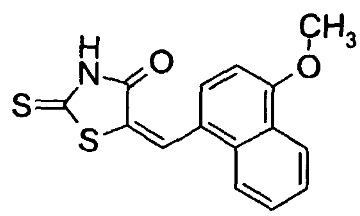

Example 404

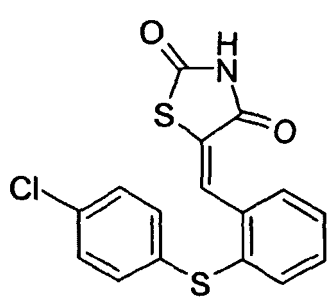

Example 405

5-(3-Hydroxy-5-methyl-phenylamino)-thiazolidine-2,4-dione

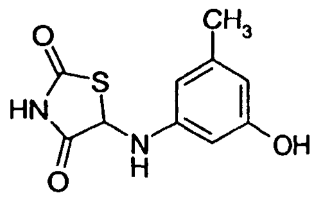

Example 406

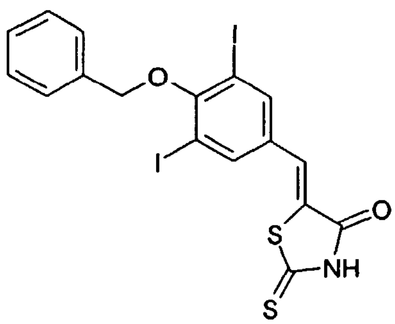

Example 407

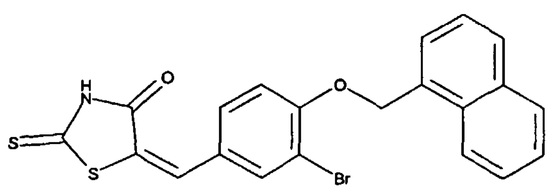

Example 408

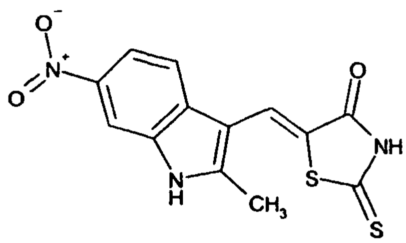

Example 409

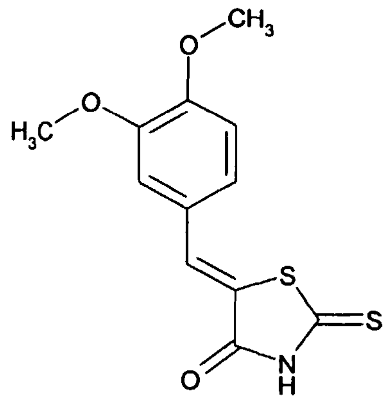

Example 410

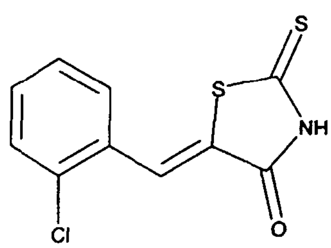

Example 411

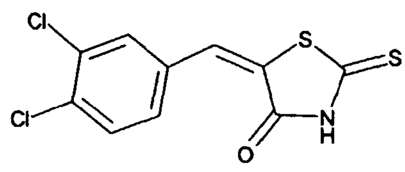

Example 412

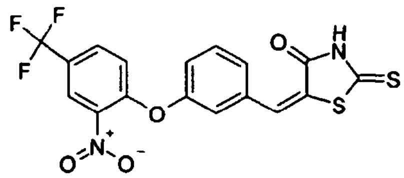

Example 413

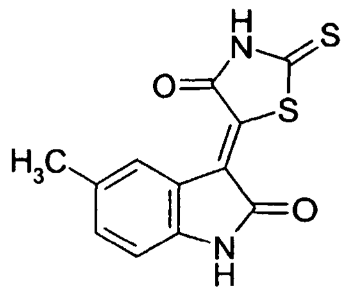

Example 414

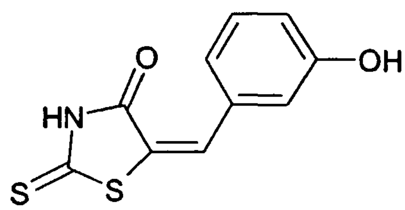

Example 415

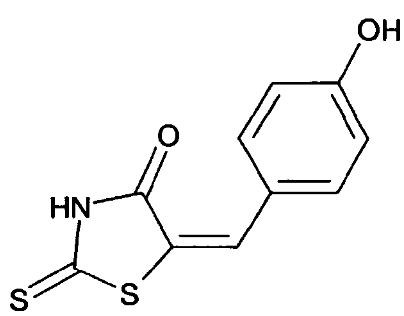

Example 416

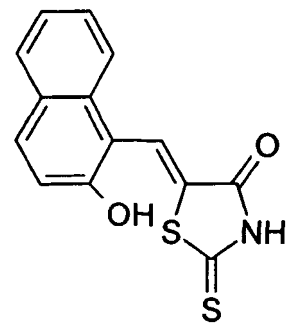

Example 417

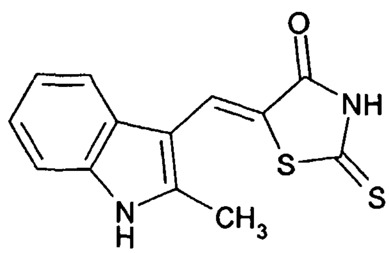

Example 418

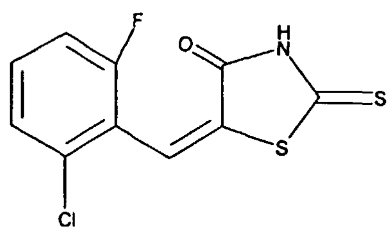

Example 419

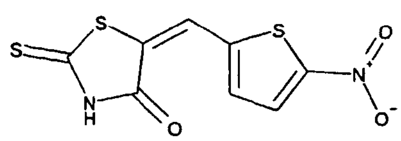

Example 420

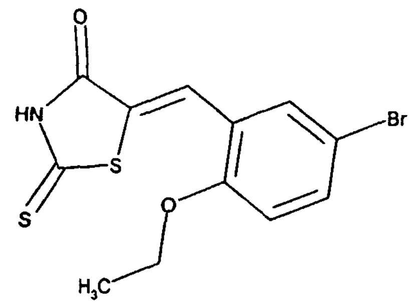

Example 421

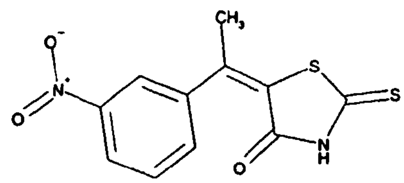

Example 422

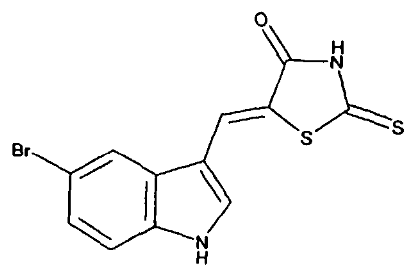

Example 423

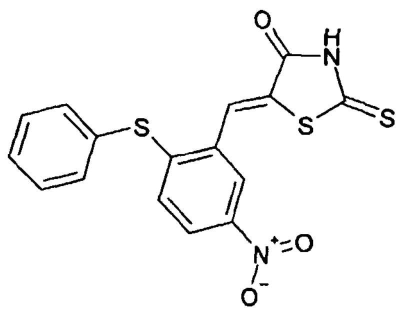

Example 424

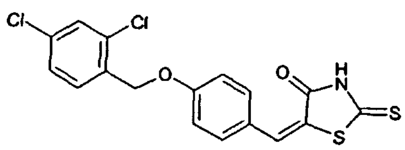

Example 425

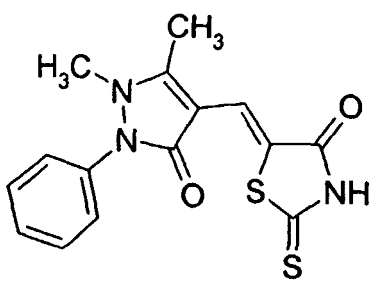

Example 426

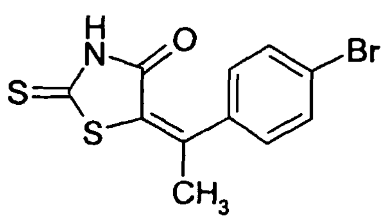

Example 427

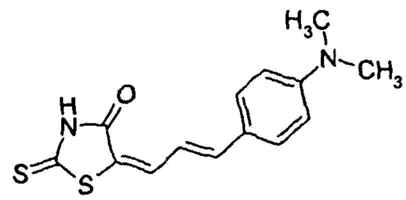

Example 428

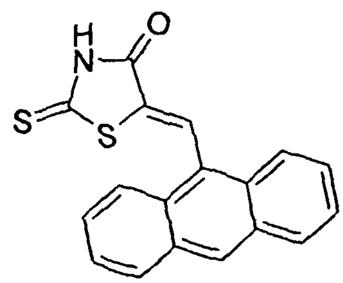

Example 429

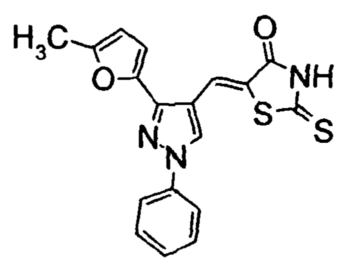

Example 430

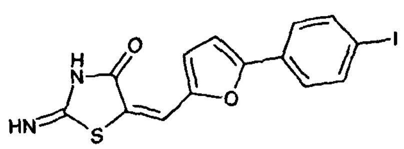

*Example 431*

5-(4-Diethylamino-2-methoxy-benzylidene)-imidazolidine-2,4-dione

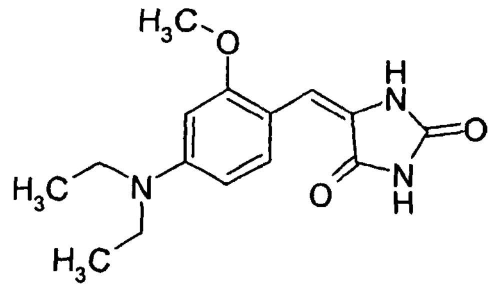

Example 432

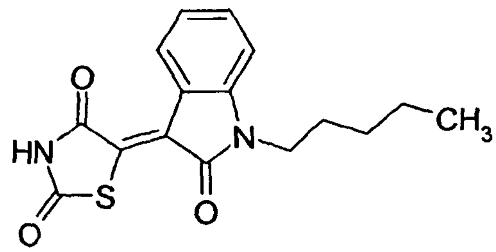

Example 433

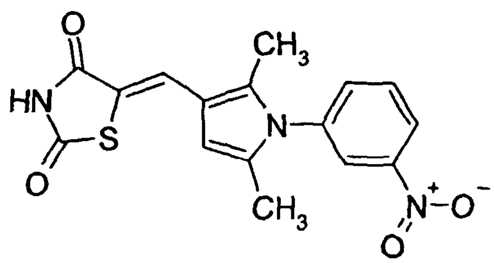

Example 434

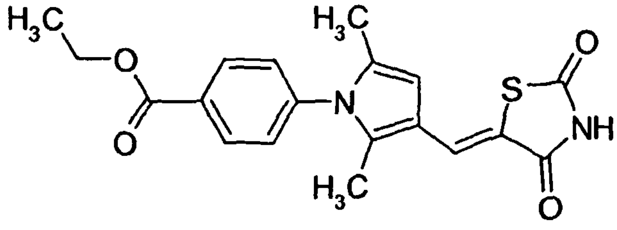

Example 435

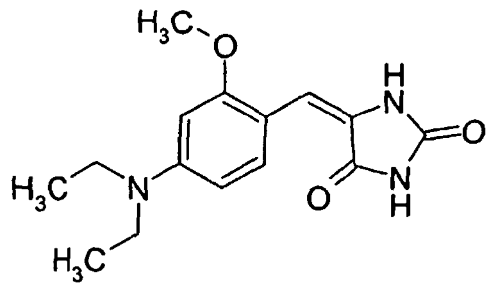

Example 436

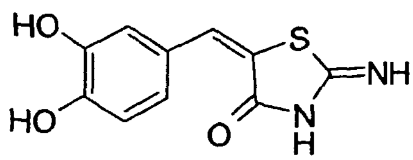

Example 437

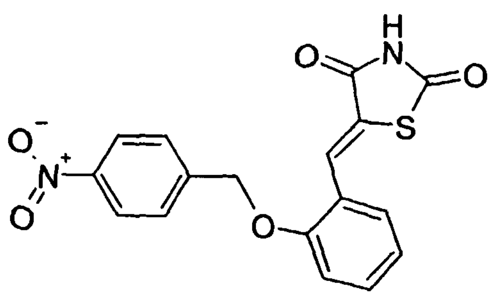

Example 438

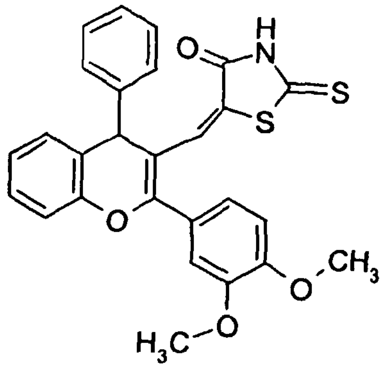

Example 439

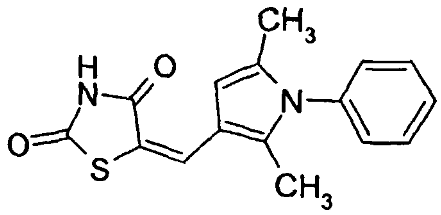

Example 440

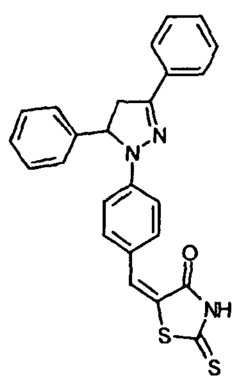

Example 441

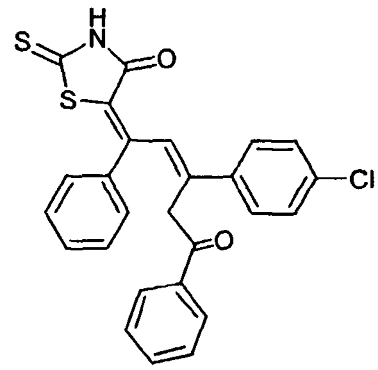

Example 442

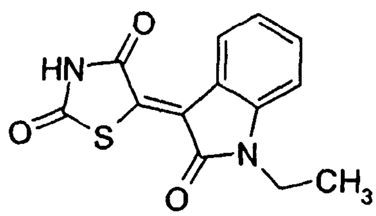

Example 443

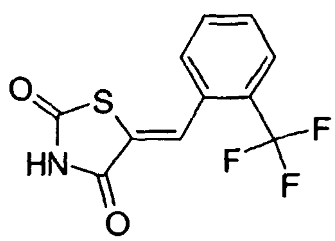

Example 444

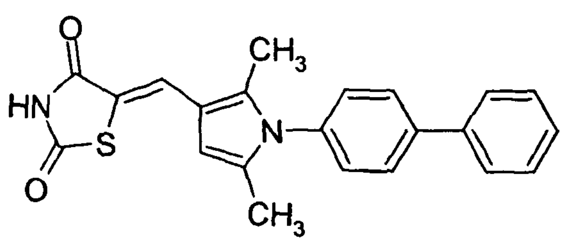

Example 445

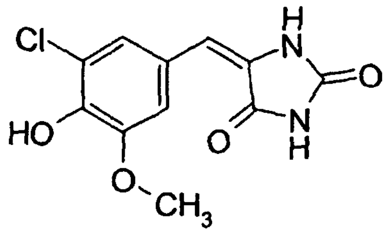

Example 446

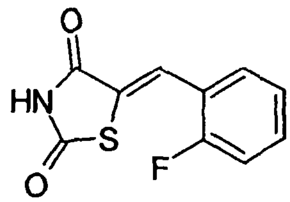

Example 447

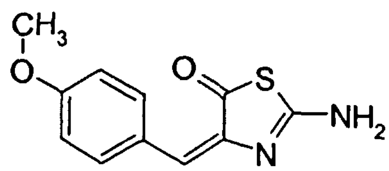

Example 448

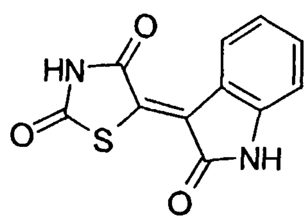

Example 449

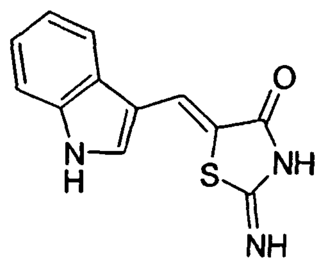

Example 450

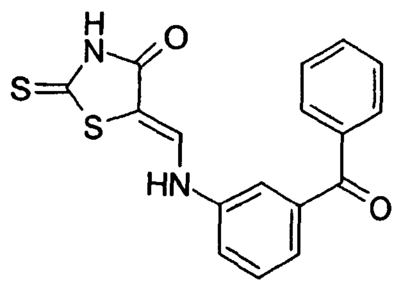

Example 451

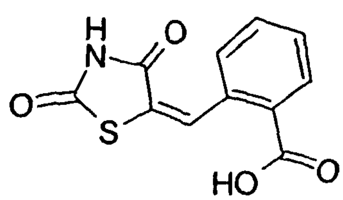

Example 452

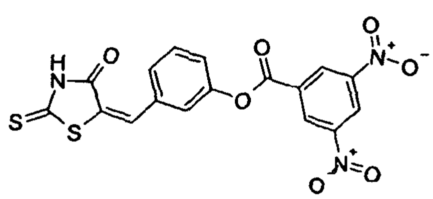

Example 453

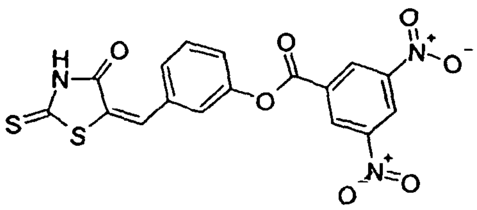

Example 454

5-(4-Diethylamino-benzylidene)-2-imino-thiazolidin-4-one

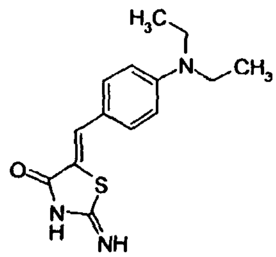

Example 455

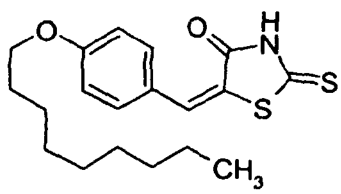

Example 456

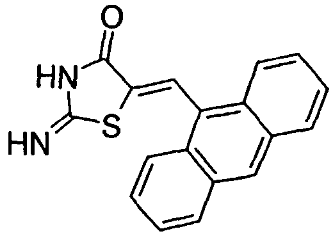

Example 457

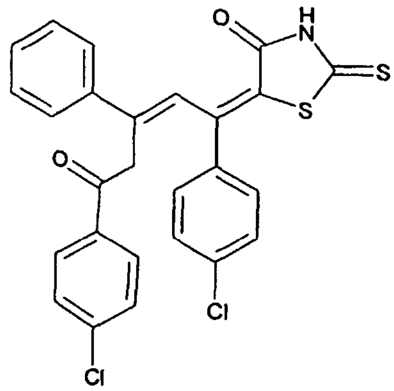

Example 458

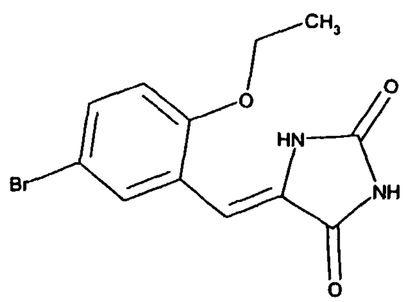

Example 459

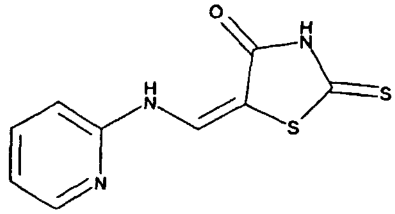

**General procedure (D) for preparation of compounds of general formula $I_3$:**

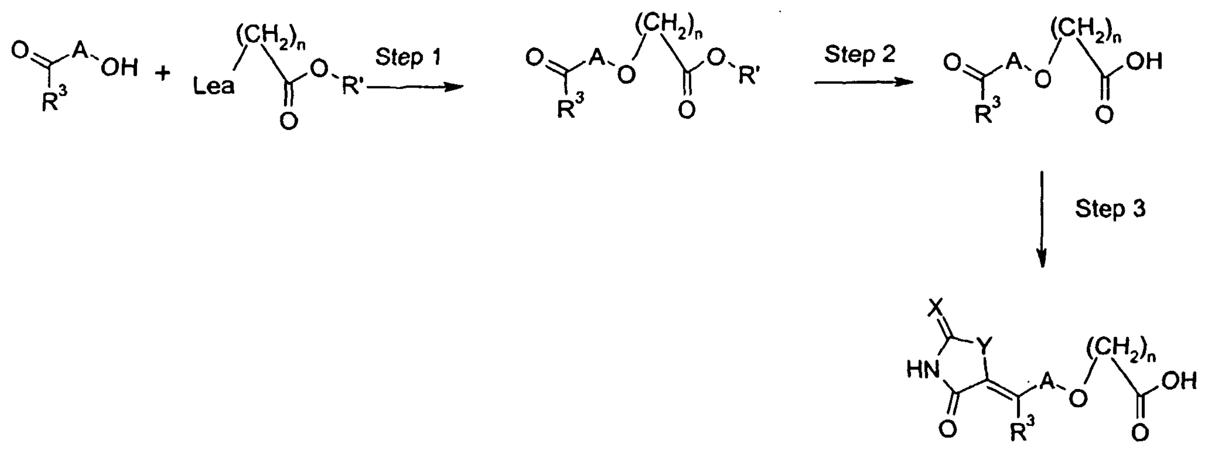


$I_3$

wherein X, Y, and $R^3$ are as defined above,
n is 1 or 3-20,
E is arylene or heterarylene (including up to four optional substituents, $R^{13}$, $R^{14}$, $R^{15}$, and $R^{15A}$ as defined above),
R' is a standard carboxylic acid protecting group, such as $C_1$-$C_6$-alkyl or benzyl and Lea is a leaving group, such as chloro, bromo, iodo, methanesulfonyloxy, toluenesulfonyloxy or the like.

Step 1 is an alkylation of a phenol moiety. The reaction is preformed by reacting $R^{10}$-C(=O)-E-OH with an ω-bromo-alkane-carboxylic acid ester (or a synthetic equivalent) in the presence of a base such as sodium or potassium carbonate, sodium or potassium hydroxide, sodium hydride, sodium or potassium alkoxide in a solvent, such as DMF, NMP, DMSO, acetone, acetonitrile, ethyl acetate or isopropyl acetate. The reaction is performed at 20 - 160 ˚C, usually at room temperature, but when the phenol moiety has one or more substituents heating to 50 ˚C or more can be beneficial, especially when the substituents are in the ortho position relatively to the phenol. This will readily be recognised by those skilled in the art.

Step 2 is a hydrolysis of the product from step 1.

Step 3 is similar to general procedure (B) and (C).

This general procedure (D) is further illustrated in the following examples:

Example 460 (General procedure (D))

4-[4-(2,4-Dioxothiazolidin-5-ylidenemethyl)phenoxy]butyric acid

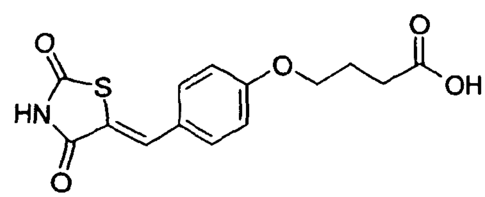

Step 1:

A mixture of 4-hydroxybenzaldehyde (9.21 g, 75 mmol), potassium carbonate (56 g, 410 mmol) and 4-bromobutyric acid ethyl ester (12.9 mL, 90 mmol) in *N*,*N*-dimethylformamide (250 mL) was stirred vigorously for 16 hours at room temperature. The mixture was filtered and concentrated *in vacuo* to afford 19.6 g (100%) of 4-(4-formylphenoxy) butyric acid ethyl ester as an oil. $^1$H-NMR (DMSO-$d_6$): δ 1.21 (3H, t), 2.05 (2H, p), 2.49 (2H, t), 4.12 (4H, m), 7.13 (2H, d), 7.87 (2H, d), 9.90 (1H, s). HPLC-MS (Method A): m/z = 237 (M+1); $R_t$ = 3.46 min.

Step 2:

4-(4-Formylphenoxy)butyric acid ethyl ester (19.6 g, 75 mmol) was dissolved in methanol (250 mL) and 1N sodium hydroxide (100 mL) was added and the resulting mixture was stirred at room temperature for 16 hours. The organic solvent was evaporated *in vacuo* (40 ˚C, 120 mBar) and the residue was acidified with 1N hydrochloric acid (110 mL). The mixture was filtered and washed with water and dried in vacuo to afford 14.3 g (91%) 4-(4-formylphenoxy) butyric acid as a solid. $^{1}$H-NMR (DMSO-$d_6$): δ 1.99 (2H, p), 2.42 (2H, t), 4.13 (2H, t), 7.14 (2H, d), 7.88 (2H, d), 9.90 (1H, s), 12.2 (1H, bs). HPLC-MS (Method A): m/z = 209 (M+1); $R_t$ = 2.19 min.

Step 3:

Thiazolidine-2,4-dione (3.55 g, 27.6 mmol), 4-(4-formylphenoxy)butyric acid (5.74 g, 27.6 mmol), anhydrous sodium acetate (11.3 g, 138 mmol) and acetic acid (100 mL) was refluxed for 16 h. After cooling, the mixture was filtered and washed with acetic acid and water. Drying in vacuo afforded 2.74 g (32%) of 4-[4-(2,4-dioxothiazolidin-5-ylidenemethyl)phenoxy]butyric acid as a solid.
$^{1}$H-NMR (DMSO-$d_6$): δ 1-97 (2H, p), 2.40 (2H, t), 4.07 (2H, t), 7.08 (2H, d), 7.56 (2H, d), 7.77 (1H, s), 12.2 (1H, bs), 12.5 (1H, bs); HPLC-MS (Method A): m/z: 308 (M+1); Rt= 2.89 min.

Example 461 (General procedure (D))

[3-(2,4-Dioxothiazolidin-5-ylidenemethyl)phenoxy]acetic acid

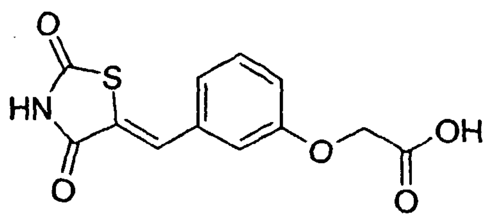

Step 3:

Thiazolidine-2,4-dione (3.9 g, 33 mmol), 3-formylphenoxyacetic acid (6.0 g, 33 mmol), anhydrous sodium acetate (13.6 g, 165 mmol) and acetic acid (100 mL) was refluxed for 16 h. After cooling, the mixture was filtered and washed with acetic acid and water. Drying in vacuo afforded 5.13 g (56%) of [3-(2,4-dioxothiazolidin-5-ylidenemethyl) phenoxy]acetic acid as a solid.
$^{1}$H-NMR (DMSO-$d_6$): δ 4.69 (2H, s), 6.95 (1H, dd), 7.09 (1H, t), 7.15 (1H, d), 7.39 (1H, t),7.53 (1H, s); HPLC-MS (Method A): m/z = 280 (M+1) (poor ionisation); $R_t$ = 2.49 min.

The compounds in the following examples were similarly prepared.

Example 462 (General procedure (D))

3-[4-(2,4-Dioxothiazolidin-5-ylidenemethyl)phenyl]acrylic acid

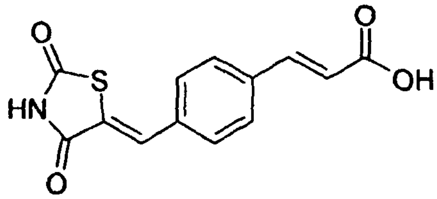

$^1$H-NMR (DMSO-$d_6$): δ 6.63 (1H, d), 7.59-7.64 (3H, m), 7.77 (1H, s), 7.83 (2H, m).

Example 463 (General procedure (D))

[4-(2,4-Dioxothiazolidin-5-ylidenemethyl)phenoxy]acetic acid

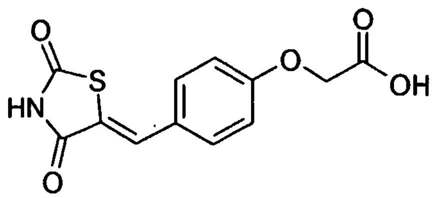

Triethylamine salt: $^1$H-NMR (DMSO-$d_6$): δ 4.27 (2H, s), 6.90 (2H, d), 7.26 (1H, s), 7.40 (2H, d).

Example 464 (General procedure (D))

4-(2,4-Dioxothiazolidin-5-ylidenemethyl)benzoic acid

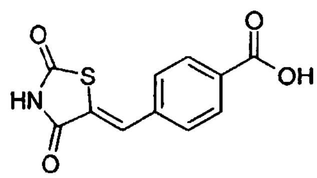

Example 465 (General procedure (D))

3-(2,4-Dioxothiazolidin-5-ylidenemethyl)benzoic acid

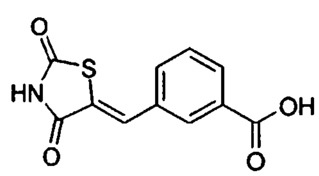

$^1$H-NMR (DMSO-$d_6$): δ 7.57 (1H, s), 7.60 (1H, t), 7.79 (1H, dt), 7.92 (1H, dt), 8.14 (1H, t).

Example 466 (General procedure (D))

4-[2-Chloro-4-(2,4-dioxothiazolidin-5-ylidenemethyl)phenoxy]butyric acid

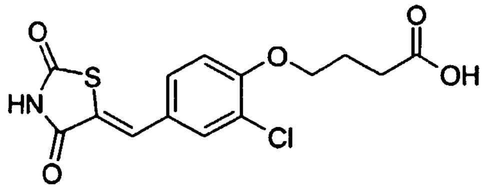

$^1$H-NMR (DMSO-$d_6$): δ 2.00 (2H, p), 2.45 (2H, t), 4.17 (2H, t), 7.31 (1H, d), 7.54 (1H, dd), 7.69 (1H, d), 7.74 (1H, s), 12.2 (1H, bs), 12.6 (1H, bs). HPLC-MS (Method A): m/z: 364 (M+23); Rt = 3.19 min.

Example 467 (General procedure (D))

4-[2-Bromo-4-(2,4-dioxothiazolidin-5-ylidenemethyl)phenoxy]butyric acid

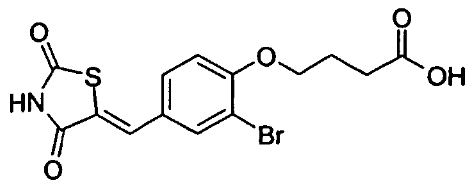

$^1$H-NMR (DMSO-$d_6$): δ 1.99 (2H, p), 2.46 (2H, t), 4.17 (2H, t), 7.28 (1H, d), 7.57 (1H, dd), 7.25 (1H, s), 7.85 (1H, d), 12.2 (1H, bs), 12.6 (1H, bs). HPLC-MS (Method A): m/z: 410 (M+23); Rt = 3.35 min.

Example 468 (General procedure (D))

4-[2-Bromo-4-(4-oxo-2-thioxothiazolidin-5-ylidenemethyl)phenoxy]butyric acid

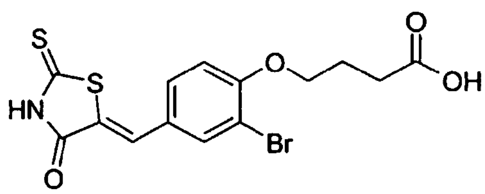

$^1$H-NMR (DMSO-$d_6$): δ 1.99 (2H, p), 2.45 (2H, t), 4.18 (2H, t), 7.28 (1H, d), 7.55 (1H, dd), 7.60 (1H, s), 7.86 (1H, d), 12.2 (1H, bs), 13.8 (1H, bs). HPLC-MS (Method A): m/z: 424 (M+23); Rt = 3.84 min.
HPLC-MS (Method A): m/z: 424 (M+23); Rt = 3,84 min

Example 469 (General procedure (D))

4-[4-(2,4-Dioxothiazolidin-5-ylidenemethyl)naphthalen-1-yloxy]butyric acid

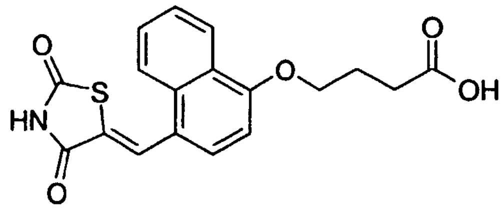

$^1$H-NMR (DMSO-$d_6$): δ 2.12 (2H, p), 2.5 (below DMSO), 4.28 (2H, t), 7.12 (1H, d), 7.6-7.7 (3H, m), 8.12 (1H, d), 8.31 (1H, d), 8.39 (1H, s), 12.2 (1H, bs), 12.6 (1H, bs). HPLC-MS (Method A): m/z: 380 (M+23); Rt = 3.76 min.

Example 470 (General procedure (D))

5-[4-(2,4-Dioxothiazolidin-5-ylidenemethyl)naphthalen-1-yloxy]pentanoic acid

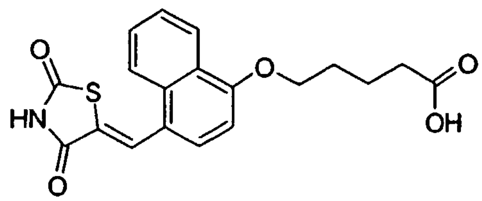

HPLC-MS (Method A): m/z: 394 (M+23); Rt = 3.62 min.
$^1$H-NMR (DMSO-$d_6$): δ 1.78 (2H, m), 1.90 (2H, m), 2.38 (2H, t), 4.27 (2H, t), 7.16 (1H, d), 7.6-7.75 (3H, m), 8.13 (1H, d), 8.28 (1H, d), 8.39 (1H, s), 12.1 (1H, bs), 12.6 (1H, bs).

Example 471

5-[2-Bromo-4-(2,4-dioxothiazolidin-5-ylidenemethyl)naphthalen-1-yloxy]pentanoic acid.

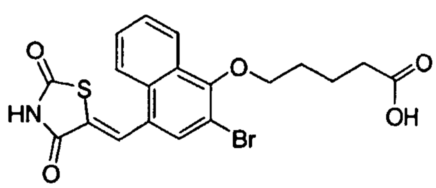

5-[4-(2,4-Dioxothiazolidin-5-ylidenemethyl)-naphthalen-1-yloxy]pentanoic acid (example 470, 185 mg, 0.5 mmol) was treated with an equimolar amount of bromine in acetic acid (10 mL). Stirring at RT for 14 days followed by evaporation to dryness afforded a mixture of the brominated compound and unchanged starting material. Purification by preparative HPLC on a C18 column using acetonitrile and water as eluent afforded 8 mg of the title compound.
HPLC-MS (Method C): m/z: 473 (M+23), Rt. = 3.77 min

Example 472

4-[2-Bromo-4-(2,4-dioxothiazolidin-5-ylidenemethyl)naphthalen-1-yloxy]butyric acid.

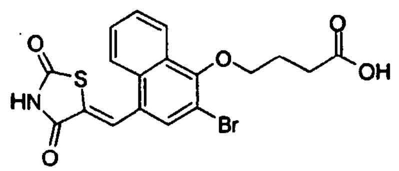

Starting with 4-[4-(2,4-dioxothiazolidin-5-ylidenemethyl)-naphthalen-1-yloxy]-butyric acid (example 469, 0.5 mmol) using the same method as in example 471 afforded 66 mg of the title compound.
HPLC-MS (Method C): m/z: 459 (M+23) ; Rt. = 3.59 min.

Example 473 (General procedure (D))

[2-Bromo-4-(2,4-dioxothiazolidin-5-ylidenemethyl)phenoxy]acetic acid

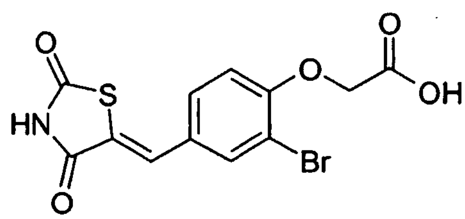

$^1$H-NMR (DMSO-$d_6$): δ 4.90 (2H, s), 7.12 (1H, d), 7.52 (1H, dd), 7.65 (1H, s) 7.84 (1H, d).HPLC-MS (Method A): m/z: not observed; Rt = 2.89 min.

Example 474 (General procedure (D))

4-[3-(2,4-Dioxothiazolidin-5-ylidenemethyl)phenoxy]butyric acid

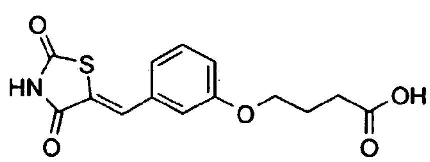

$^1$H-NMR (DMSO-$d_6$): δ 1.98 (2H, p), 2.42 (2H, t), 4.04 (2H, t), 7.05 (1H, dd), 7.15 (2H, m), 7.45 (1H, t), 7.77 (1H, s), 12.1 (1H, bs), 12.6 (1H, bs). HPLC-MS (Method A): m/z: 330 (M+23); Rt = 3.05 min.

Example 475 (General procedure (D))

[4-(2,4-Dioxothiazolidin-5-ylidenemethyl)-3-methoxyphenoxy]acetic acid

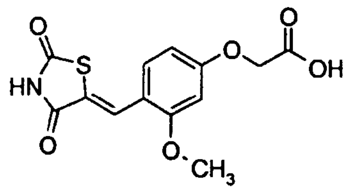

HPLC-MS (Method B): m/z: 310 (M+1); Rt = 3,43 min.

Example 476 (General procedure (D))

[4-(2,4-Dioxothiazolidin-5-ylidenemethyl)naphthalen-1-yloxy]acetic acid

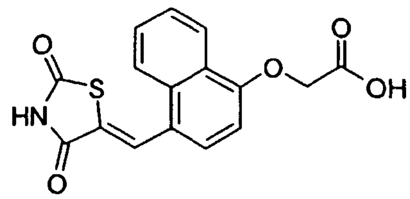

HPLC-MS (Method A): m/z: 330 (M+1); Rt = 3.25 min.

Example 477 (General procedure (D))

8-(2,4-Dioxothiazolidin-5-ylidenemethyl)naphthalene-l-carboxylic acid

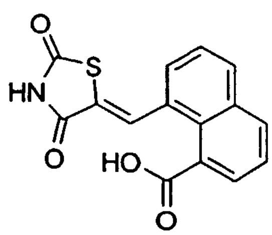

HPLC-MS (Method A): m/z: 299 (M+1); Rt = 2,49 min.

Example 478 (General procedure (D))

[3-(2,4-Dioxothiazolidin-5-ylidenemethyl)indol-1-yl]acetic acid

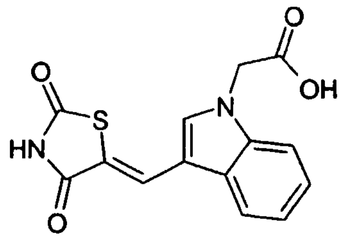

HPLC-MS (Method A): m/z: 303 (M+1); Rt = 2.90 min.

Preparation of starting material:

3-Formylindol (10 g, 69 mmol) was dissolved in N,N-dimethylformamide (100 mL) and under an atmosphere of nitrogenand with external cooling, keeping the temperature below 15 °C, sodium hydride (60% in mineral oil, 3.0 g, 76 mmol) was added in portions. Then a solution of ethyl bromoacetate (8.4 mL, 76 mmol) in *N,N*-dimethylformamide (15 mL) was added dropwise over 30 minutes and the resulting mixture was stirred at room temperature for 16 hours. The mixture was concentrated *in vacuo* and the residue was partitioned between water (300 mL) and ethyl acetate (2 x 150 mL). The combined organic extracts were washed with a saturated aqueous solution of ammonium chloride (100 mL), dried ($MgSO_4$) and concentrated in vacuo to afford 15.9 g (quant.) of (3-formylindol-1-yl)acetic acid ethyl ester as an oil.

$^1$H-NMR ($CDCl_3$): $\delta_H$ = 1.30 (3H, t), 4.23 (2H, q), 4.90 (2H, s), 7.3 (3H, m), 7.77 (1H, s), 8.32 (1H, d), 10.0 (1H, s).

(3-Fonnylindol-1-yl)acetic acid ethyl ester (15.9 g 69 mmol) was dissolved in 1,4-dioxane (100 mL) and 1 N sodium hydroxide (10 mL) was added and the resulting mixture was stirred at room temperature for 4 days. Water (500 mL) was added and the mixture was washed with diethyl ether (150 mL). The aqueous phase was acidified with 5N hydrochloric acid and extracted with ethyl acetate (250 + 150 mL). The combined organic extracts were dried ($MgSO_4$) and concentrated in vacuo to afford 10.3 g (73%) of (3-formylindol-1-yl)acetic acid as a solid.

$^1$H-NMR (DMSO-$d_6$): $\delta_H$ = 5.20 (2H, s), 7.3 (2H, m), 7.55 (1H, d), 8.12 (1H, d), 8.30 (1H, s), 9.95 (1H, s), 13.3 (1H, bs).

Example 479 (General procedure (D))

3-[3-(2,4-Dioxothiazolidin-5-ylidenemethyl)indol-1-yl]propionic acid

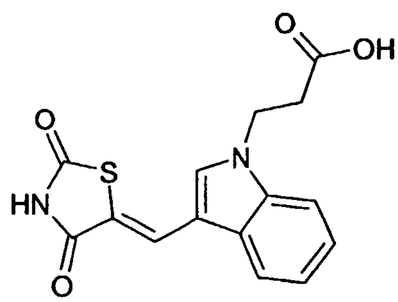

HPLC-MS (Method A): m/z: 317 (M+1); Rt = 3.08 min.

Preparation of starting material:

A mixture of 3-formylindol (10 g, 69 mmol), ethyl 3-bromopropionate (10.5 mL. 83 mmol) and potassium carbonate (28.5 g, 207 mmol) and acetonitrile (100 mL) was stirred vigorously at refux temperature for 2 days. After cooling, the mixture was filtered and the filtrate was concentrated *in vacuo* to afford 17.5 g (quant.) of 3-(3-formylindol-1-yl) propionic acid ethyl ester as a solid.

$^1$H-NMR (DMSO-$d_6$): $\delta_H$ = 1.10 (3H, t), 2.94 (2H, t), 4.02 (2H, q), 4.55 (2H, t), 7.3 (2H, m), 7.67 (1H, d), 8.12 (1H, d), 8.30 (1H, s), 9.90 (1H, s).

3-(3-Formylindol-1-yl)propionic acid ethyl ester (17.5 g 69 mmol) was hydrolysed as described above to afford 12.5 g (83%) of 3-(3-formylindol-1-yl)propionic acid as a solid.
$^{1}$H-NMR (DMSO-$d_6$): $\delta_H$ = 2.87 (2H, t), 4.50 (2H, t), 7.3 (2H, m), 7.68 (1H, d), 8.12 (1H, d), 8.31 (1H, s), 9.95 (1H, s), 12.5 (1H, bs).

Example 480 (General procedure (D))

{5-[4-(2,4-Dioxothiazolidin-5-ylidenemethyl)benzylidene]-4-oxo-2-thioxothiazolidin-3-yl}acetic acid

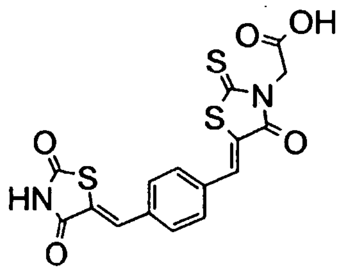

HPLC-MS (Method A): m/z: 429 (M+23); Rt = 3.89 min.

Example 481 (General procedure (D))

6-(2,4-Dioxothiazolidin-5-ylidenemethyl)naphthalen-2-yloxyoctanoic acid

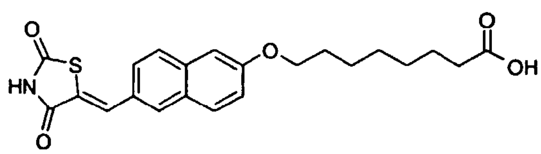

HPLC-MS (Method C): m/z: 436 (M+23); Rt.= 4.36 min
The intermediate aldehyde for this compound was prepared by a slightly modified procedure: 6-Hydroxynaphthalene-2-carbaldehyde (1.0 g, 5.8 mmol) was dissolved in DMF (10 mL) and sodium hydride 60% (278 mg) was added and the mixture stirred at RT for 15 min. 8-Bromooctanoic acid (0.37 g, 1.7 mmol) was converted to the sodium salt by addition of sodium hydride 60% and added to an aliquot (2.5 mL) of the above naphtholate solution and the resulting mixture was stirred at RT for 16 hours. Aqueous acetic acid (10 %) was added and the mixture was extracted 3 times with diethyl ether. The combined organic phases were dried with $MgSO_4$ and evaporated to dryness affording 300 mg of 8-(6-formylnaphthalen-2-yloxy)octanoic acid.
HPLC-MS (Method C): m/z 315 (M+1); Rt. = 4.24 min.

Example 482 (General procedure (D))

12-[6-(2,4-Dioxothiazolidin-5-ylidenemethyl)naphthalen-2-yloxy]dodecanoic acid.

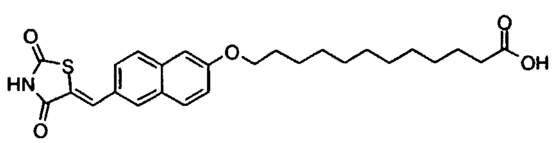

HPLC-MS (Method C): m/z: 492 (M+23); Rt.= 5.3 min.

The intermediate aldehyde was prepared similarly as described in example 481.

Example 483 (General procedure (D))

11-[6-(2,4-Dioxothiazolidin-5-ylidenemethyl)naphthalen-2-yloxy]undecanoic acid.

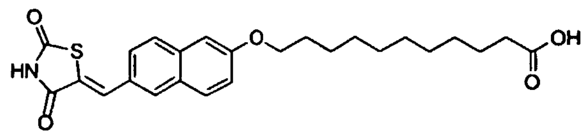

HPLC-MS (Method C): m/z:478 (M+23); Rt.= 5.17 min.

The intermediate aldehyde was prepared similarly as described in example 481.

Example 484 (General procedure (D))

15-[6-(2,4-Dioxothiazolidin-5-ylidenemethyl)naphthalen-2-yloxy]pentadecanoic acid.

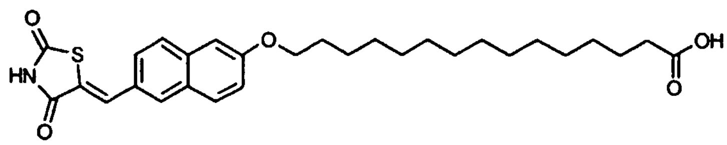

HPLC-MS (Method C): m/z: 534 (M+23); Rt.= 6.07 min.

The intermediate aldehyde was prepared similarly as described in example 481.

Example 485 (General procedure (D))

6-[6-(2,4-Dioxothiazolidin-5-ylidenemethyl)naphthalen-2-yloxy]hexanoic acid.

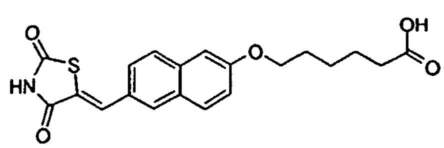

HPLC-MS (Method C): m/z: 408 (M+23); Rt.= 3.71 min.

Example 486 (General procedure (D))

4-[6-(2,4-Dioxothiazolidin-5-ylidenemethyl)naphthalen-2-yloxy]butyric acid.

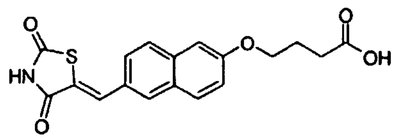

HPLC-MS (Method C): m/z: 380 (M+23); Rt.= 3.23 min.

Example 487 (General procedure (D))

6-[6-(2,4-Dioxothiazolidin-5-ylidenemethyl)naphthalen-2-yloxy]hexanoic acid ethyl ester.

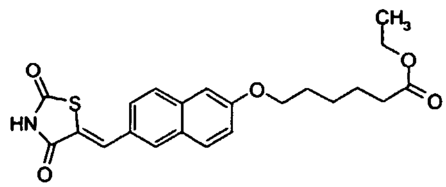

HPLC-MS (Method C): m/z: 436 (M+23); Rt.= 4.64 min.

Example 488 (General procedure (D))

4-[6-(2,4-Dioxothiazolidin-5-ylidenemethyl)naphthalen-2-yloxy]butyric acid ethyl ester.

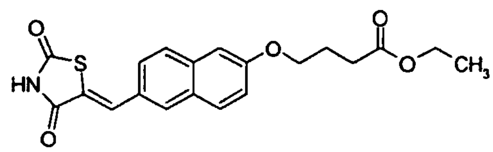

HPLC-MS (Method C): m/z: 408 (M+23); Rt.= 4.28 min.

Example 489 (General procedure (D))

2-{5-[4-(2,4-Dioxothiazolidin-5-ylidenemethyl)naphthalen-1-yloxy]pentyl}malonic acid

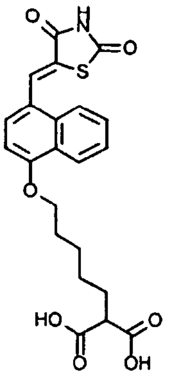

HPLC-MS (Method C): m/z = 444 (M+1); Rt = 3,84 min.

Example 490 (General procedure (D)

2-{5-[4-(2,4-Dioxothiazolidin-5-ylidenemethyl)naphthalen-1-yloxy]pentyl}malonic acid diethyl ester

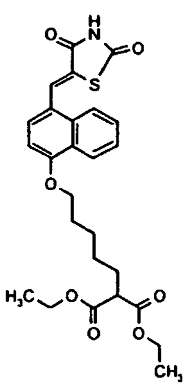

HPLC-MS (Method C): m/z = 500 (M+1); Rt = 5.18 min.

Example 491 (General procedure (D))

4-[4-(2,4,6-Trioxotetrahydropyrimidin-5-ylidenemethyl)naphthalen-1-yloxy]butyric acid

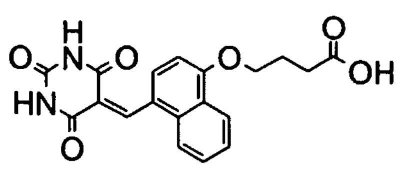

HPLC-MS (Method C): m/z = 369 (M+1); Rt = 2,68 min.

Example 492

N-(3-Aminopropyl)-4-[4-(2,4-dioxothiazolidin-5-ylidenemethyl)-naphthalen-1-yloxy]-butyramide

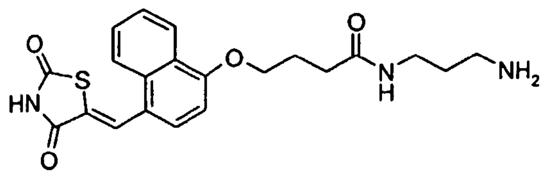

To a mixture of 4-[4-(2,4-dioxothiazolidin-5-ylidenemethyl)naphthalen-1-yloxy]butyric acid (example 469, 5.9 g, 16.5 mmol) and 1-hydroxybenzotriazole (3.35 g, 24.8 mmol) in DMF (60 mL) was added 1-ethyl-3-(3'-dimethylaminopropyl)carbodiimide hydrochloride (4.75 g, 24.8 mmol) and the resulting mixture was stirred at room temperature for 2 hours. *N*-(3-amino-propylcarbamic acid *tert*-butyl ester (3.45 g, 19.8 mmol) was added and the resulting mixture was stirred at room temperature for 16 hours. The mixture was concentrated *in vacuo* and ethyl acetate and dichloromethane were added to the residue. The mixture was filtered, washed with water and dried *in vacuo* to afford 4.98 g (59%) of (3-{4-[4-(2,4-dioxothiazolidin-5-ylidenemethyl)naphthalen-1-yloxy]butyrylamino}propyl)carbamic acid tert-butyl ester.
HPLC-MS (Method C): m/z: 515 (M+1); Rt = 3.79 min.

(3-{4-[4-(2,4-Dioxothiazolidin-5-ylidenemethyl)naphthalen-1-yloxy]butyrylamino}propyl)carbamic acid tert-butyl ester (4.9 g, 9.5 mmol) was added dichloromethane (50 mL) and trifluoroacetic acid (50 mL) and the resulting mixture was stirred at room temperature for 45 minutes. The mixture was concentrated *in vacuo* and co-evaporated with toluene. To the residue was added ethyl acetate (100 mL) and the mixture was filtered and dried *in vacuo* to afford the title compound as the trifluoroacetic acid salt.
HPLC-MS (Method C): m/z: 414 (M+1); Rt = 2,27 min.

Compounds of the invention includes:

| |
|---|
| Example 493 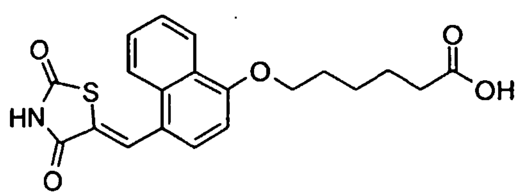 |
| Example 494 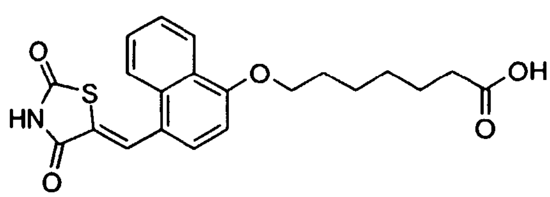 |
| Example 495 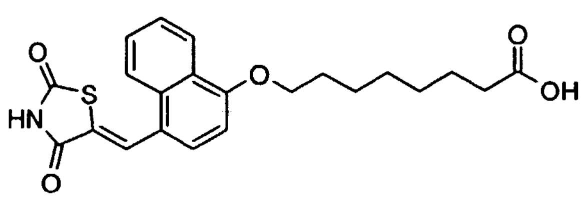 |

(continued)

| |
|---|
| Example 496<br>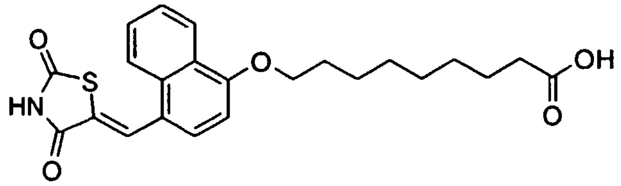 |
| Example 497<br>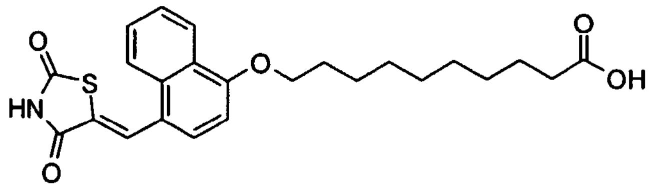 |
| Example 498<br>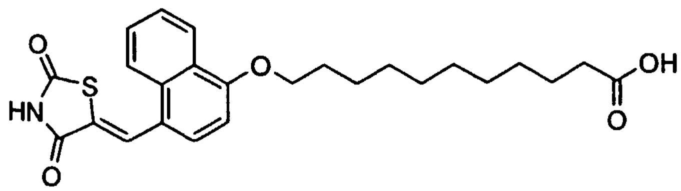 |
| Example 499<br>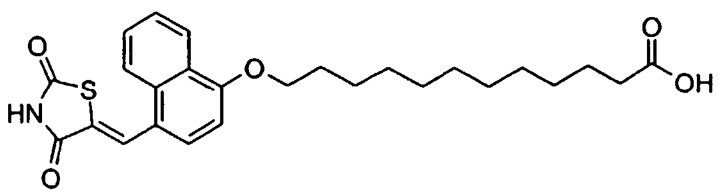 |
| Example 500<br>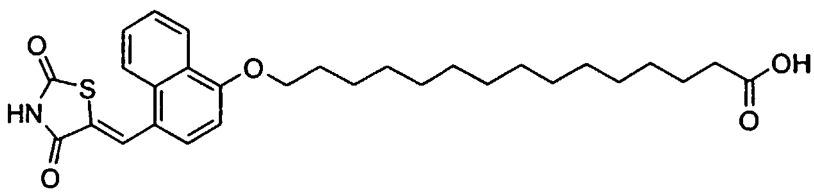 |
| Example 501<br>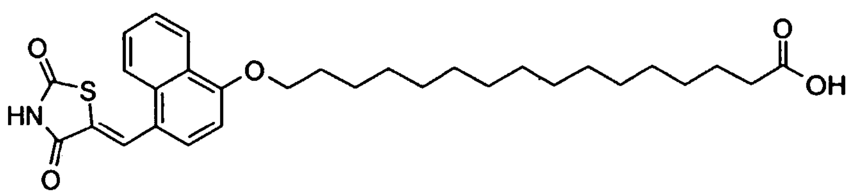 |

(continued)

| |
|---|
| Example 502<br>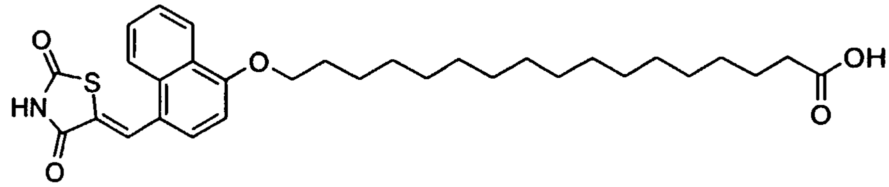 |
| Example 503<br>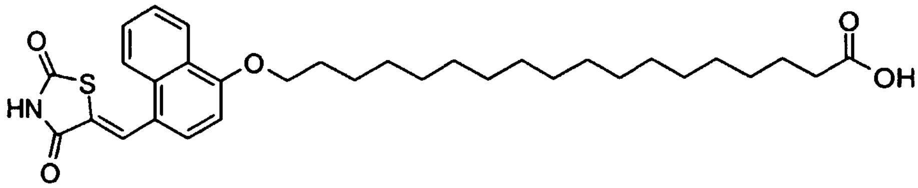 |

Example 504 (Prepared analogously to General Procedure (D))

2-{5-[4-(2,4-Thiazolidindion-5-ylidenemethyl)naphthalen-1-yloxy]pentyl}malonic acid

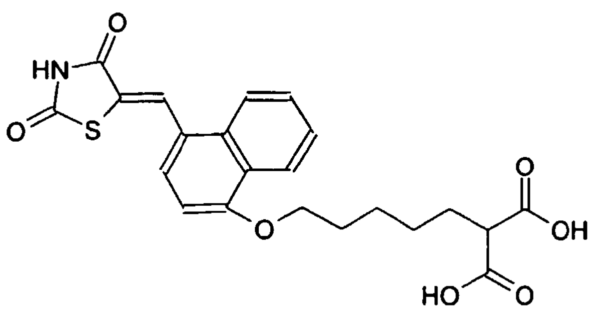

A solution of 4-hydroxy-1-naphtaldehyde (1.0 g, 5.81 mmol), 2-(5-bromopentyl)malonic acid diethyl ester (2.07 g, 6.68 mmol) and potassium carbonate (4.01 g, 29 mmol) in DMF (50 mL) was stirred at 100˚ C for 3 hours. The mixture was cooled and the salt was filtered off. The solvent was then removed under reduced pressure to afford 2.9 g of crude 2-[5-(4-formylnaphtalen-1-yloxy)pentyl]malonic acid diethyl ester which was used for the next reaction without further purification.

HPLC-MS (Method C): m/z: 401 (M+1); Rt = 5.16 min. $^{1}$H-NMR (DMSO-*d*6): δ = 1.18 (t, 6 H), 1,39 (m, 2 H), 1.55 (m, 2 H), 1.87 (m, 4 H), 3.48 (t, 1 H), 4.13 (m, 4 H), 4.27 (t, 2 H), 7.17 (d, 1 H), 7.64(t, 1 H), 7.75 (t, 1 H), 8.13 (d, 1 H), 8.29 (d, 1 H), 9.24 (d, 1 H), 10.19 (s, 1 H).

1.4 g (3.5 mmol) of crude 2-[5-(4-formylnaphtalen-1-yloxy)pentyl]malonic acid diethyl ester was treated with aqueous sodium hydroxide (1N, 8.75 mL, 8.75 mmol) and methanol (50 mL). The solution was stirred at 70˚ C for 5 hours and the mixture was concentrated under reduced pressure. Hydrochloric acid (6 N) was added until pH <2. The resulting slurry was stirred untill it solidified. The crystals were filtered off, washed with water and then dried *in vacuo* to afford 1.1 g (92%) of 2-[5-(4-formylnaphtalen-1-yloxy)pentyl]malonic acid. The product was used in the next step without further purification.

HPLC-MS (Method C): m/z: 345 (M+1); Rt = 3.52 min. $^{1}$H-NMR(DMSO-$d_6$): δ = 1,40 (m, 2 H), 1.55 (m, 2 H), 1.80 (m, 2 H), 1.90 (m, 2 H), 3.24 (t, 1 H), 4.29 (t, 2 H), 7.19 (d, 1 H), 7.64(t, 1 H), 7.75 (t, 1 H), 8.14 (d, 1 H), 8.30 (d, 1 H), 9.23 (d, 1 H), 10.18 (s, 1 H), 12.69 (s, 2 H).

To a solution of 2-[5-(4-formylnaphtalen-1-yloxy) pentyl]malonic acid (0.36 g, 1.05 mmol) in acetic acid (10 mL) was added 2,4-thiazolidindione (0.16 g,1.36 mmol) and piperidine (0.52 mL, 5.25 mmol). The solution was heated to 105˚C for 24 hours. After cooling to room temperature, the solvents were removed *in vacuo.* Water was added to the

residue. The precipitate was filtered off and washed with water. Recrystalisation from acetonitrile afforded 200 mg (43%) of the title compound as a solid.
HPLC-MS (Method C): m/z: 422 ($M-CO_2+Na$); Rt = 4.08 min. $^1H$-NMR(DMSO-$d_6$): $\delta$ = 1,41 (m, 2 H), 1.55 (m, 4 H), 1.88 (m, 2 H), 2.23 (t, 1 H), 4.24 (t, 2 H), 7.61-7.74 (m, 3 H), 8.12 (d, 1 H), 8.28 (d, 1 H), 8.38 (s, 1 H), 12.00 (s, 1 H), 12.59 (s, 2 H).

The following compounds are commercially available and may be prepared according to general procedure (D):

Example 505

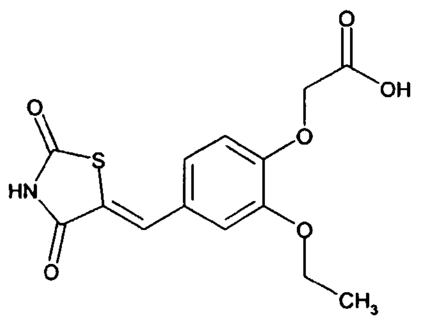

Example 506

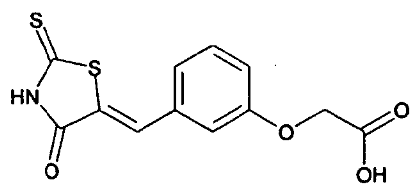

Example 507

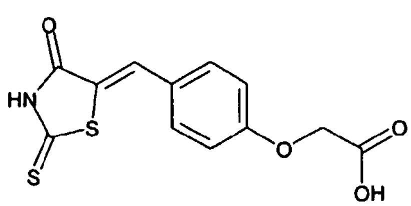

Example 508

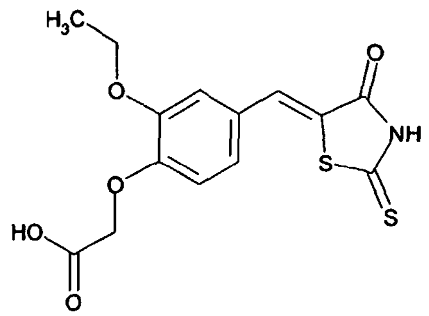

Example 509

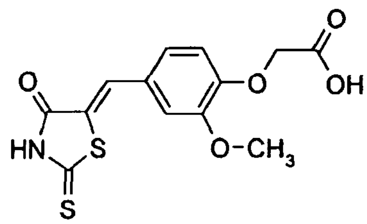

Example 510

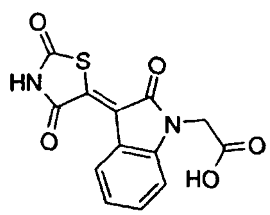

Example 511

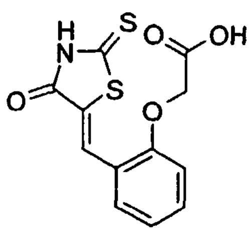

The following salicylic acid derivatives do all bind to the His B10 $Zn^{2+}$ site of the insulin hexamer:

Example 512

Salicylic acid

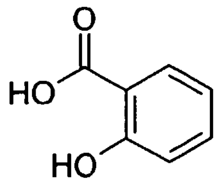

Example 513

Thiosalicylic acid (or: 2-Mercaptobenzoic acid)

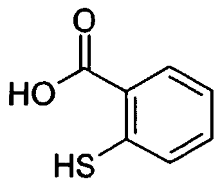

Example 514

2-Hydroxy-5-nitrobenzoic acid

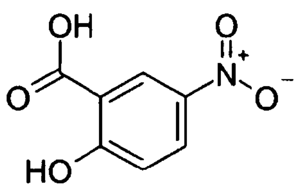

Example 515

3-Nitrosalicyclic acid

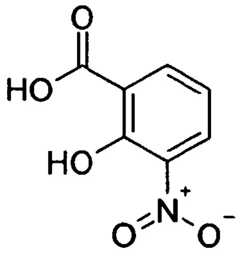

Example 516

5,5'-Methylenedisalicylic acid

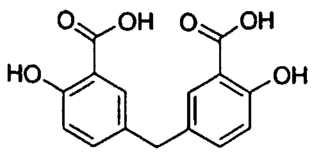

Example 517

2-Amino-5-trifluoromethylbenzoesyre

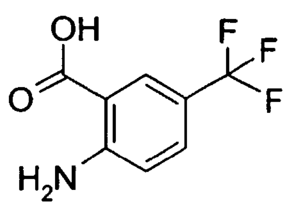

Example 518

2-Amino-4-chlorobenzoic acid

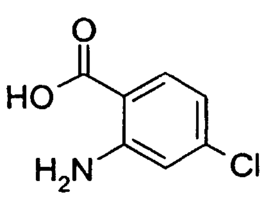

Example 519

2-Amino-5-methoxybenzoesyre

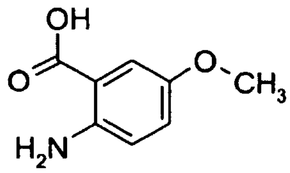

Example 520

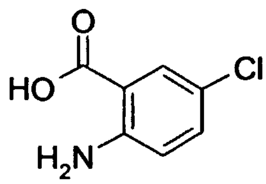

Example 521

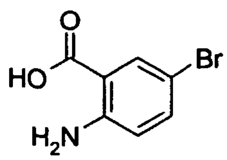

Example 522

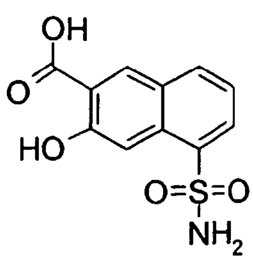

Example 523

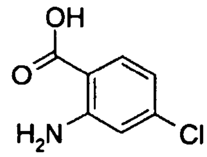

Example 524

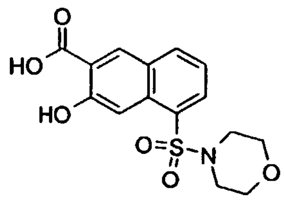

Example 525

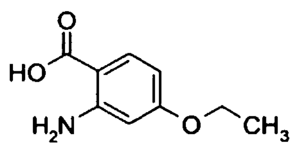

Example 526

5-Iodosalicylic acid

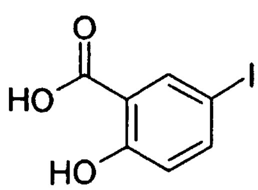

Example 527

5-Chlorosalicylic acid

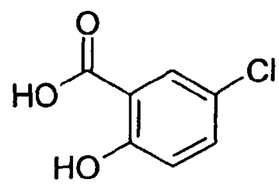

Example 528

1-Hydroxy-2-naphthoic acid

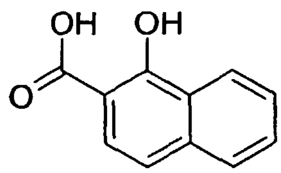

Example 529

3,5-Dihydroxy-2-naphthoic acid

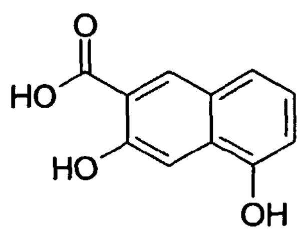

Example 530

3-Hydroxy-2-naphthoic acid

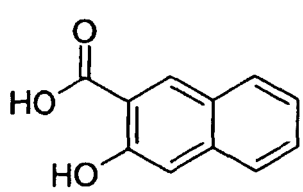

Example 531

3,7-Dihydroxy-2-naphthoic acid

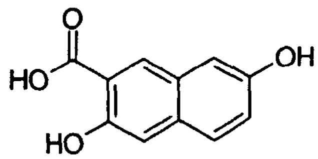

Example 532

2-Hydroxybenzo[a]carbazole-3-carboxylic acid

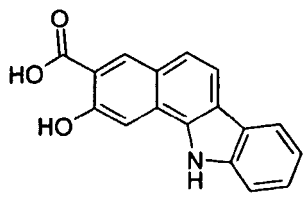

Example 533

7-Bromo-3-hydroxy-2-naphthoic acid

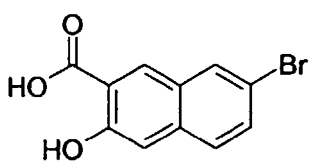

This compound was prepared according to Murphy et al., J. Med. Chem. 1990, 33, 171-8. HPLC-MS (Method A): m/z: 267 (M+1); Rt: = 3.78 min.

Example 534

1,6-Dibromo-2-hydroxynaphthalene-3-carboxylic acid

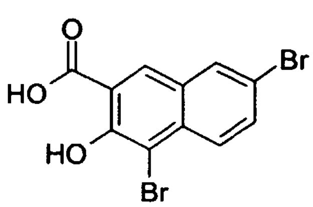

This compound was prepared according to Murphy et al., J. Med. Chem. 1990, 33, 171-8. HPLC-MS (Method A): m/z: 346 (M+1); Rt: = 4,19 min.

Example 535

7-Formyl-3-hydroxynaphthalene-2-carboxylic Acid

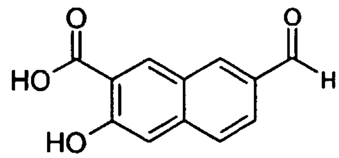

A solution of 7-bromo-3-hydroxynaphthalene-2-carboxylic acid (15.0 g, 56.2 mmol) (example 533) in tetrahydrofuran (100 mL) was added to a solution of lithium hydride (893 mg, 112 mmol) in tetrahydrofuran (350 mL). After 30 minutes stirring at room temperature, the resulting solution was heated to 50 °C for 2 minutes and then allowed to cool to ambient temperature over a period of 30 minutes. The mixture was cooled to -78 °C, and butyllithium (1.6 M in hexanes, 53 mL, 85 mmol) was added over a period of 15 minutes. N,N-Dimethylformamide (8.7 mL, 8.2 g, 112 mmol) was added after 90 minutes additional stirring. The cooling was discontinued, and the reaction mixture was stirred at room temperature for 17 hours before it was poured into 1 N hydrochloric acid (aq.) (750 mL). The organic solvents were evaporated in vacuo, and the resulting precipitate was filtered off and rinsed with water (3 x 100 mL) to yield the crude product (16.2 g). Purification on silica gel (dichloromethane /methanol / acetic acid = 90:9:1) furnished the title compound as a solid. $^1$H-NMR (DMSO-$d_6$) : δ 11.95 (1H, bs), 10.02 (1H, s), 8.61 (1H, s), 8.54 (1H, s), 7.80 (2H, bs), 7.24 (1H, s); HPLC-MS (Method (A)): m/z: 217 (M+1); Rt = 2.49 min.

Example 536

3-Hydroxy-7-methoxy-2-naphthoic acid

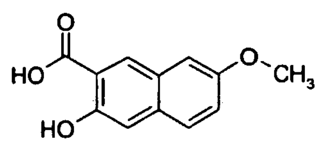

Example 537

4-Amino-2-hydroxybenzoic acid

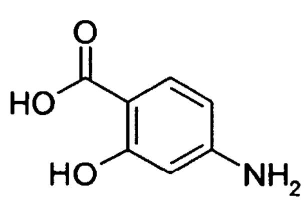

Example 538

5-Acetylamino-2-hydroxybenzoic acid

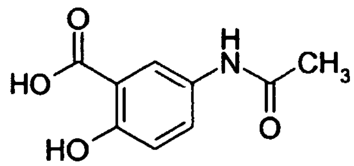

Example 539

2-Hydroxy-5-methoxybenzoic acid

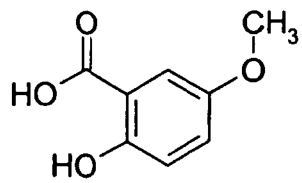

The following compounds were prepared as described below:

Example 540

4-Bromo-3-hydroxynaphthalene-2-carboxylic acid

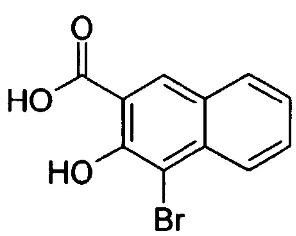

3-Hydroxynaphthalene-2-carboxylic acid (3.0 g, 15.9 mmol) was suspended in acetic acid (40 mL) and with vigorous stirring a solution of bromine (817 μL, 15.9 mmol) in acetic acid (10 mL) was added drop wise during 30 minutes. The suspension was stirred at room temperature for 1 hour, filtered and washed with water. Drying in vacuo afforded 3.74 g (88%) of 4-bromo-3-hydroxynaphthalene-2-carboxylic acid as a solid.
$^1$H-NMR (DMSO-$d_6$): δ 7.49 (1H, t), 7.75 (1H, t), 8.07 (2H, "t"), 8.64 (1H, s). The substitution pattern was confirmed by a COSY experiment, showing connectivities between the 3 (4 hydrogen) "triplets". HPLC-MS (Method A): m/z: 267 (M+1); Rt = 3.73 min.

Example 541

3-Hydroxy-4-iodonaphthalene-2-carboxylic acid

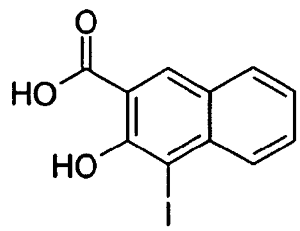

3-Hydroxynaphthalene-2-carboxylic acid (0.5 g, 2.7 mmol) was suspended in acetic acid (5 mL) and with stirring iodine monochloride (135 μL, 2.7 mml) was added. The suspension was stirred at room temperature for 1 hour, filtered and washed with water. Drying afforded 0.72 g (85%) of 4-iodo-3-hydroxynaphthalene-2-carboxylic acid as a solid.
$^1$H-NMR (DMSO-$d_6$): δ 7.47 (1H, t), 7.73 (1H, t), 7.98 (1H, d), 8.05 (1H, d), 8.66 (1H, s). HPLC-MS (Method A): m/z: 315 (M+1); Rt = 3.94 min.

Example 542

2-Hydroxy-5-[(4-methoxyphenylamino)methyl]benzoic acid

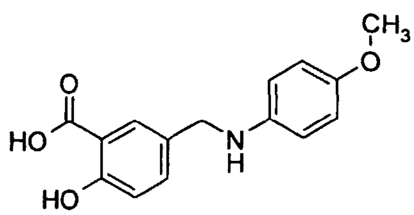

p-Anisidine (1.3 g, 10.6 mmol) was dissolved in methanol (20 mL) and 5-formylsalicylic acid (1.75 g, 10.6 mmol) was added and the resulting mixture was stirred at room temperature for 16 hours. The solid formed was isolated by filtration, re-dissolved in N-methyl pyrrolidone (20 mL) and methanol (2 mL). To the mixture was added sodium cyanoborohydride (1.2 g) and the mixture was heated to 70 °C for 3 hours. To the cooled mixture was added ethyl acetate (100 mL) and the mixture was extracted with water (100 mL) and saturated aqueous ammonium chloride (100 mL). The combined aqueous phases were concentrated *in vacuo* and a 2 g aliquot was purified by SepPac chromatography eluting with mixtures of aetonitrile and water containing 0.1% trifluoroacetic acid to afford the title compound.
HPLC-MS (Method A): m/z: 274 (M+1); Rt = 1.77 min.
$^1$H-NMR (methanol-$d_4$): δ 3.82 (3H, s), 4.45 (2H, s), 6.96 (1H, d), 7.03 (2H, d), 7.23 (2H, d), 7.45 (1H, dd), 7.92 (1H, d).

Example 543

2-Hydroxy-5-(4-methoxyphenylsulfamoyl)benzoic acid

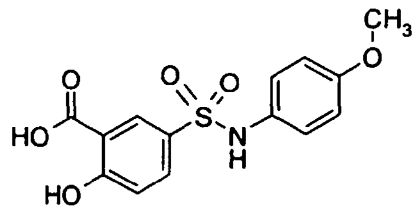

A solution of 5-chlrosulfonylsalicylic acid (0.96 g, 4.1 mmol) in dichloromethane (20 mL) and triethylamine (1.69 mL, 12.2 mmol) was added p-anisidine (0.49 g, 4.1 mmol) and the resulting mixture was stirred at room temperature for 16 hours. The mixture was added dichloromethane (50 mL) and was washed with water (2 x 100 mL). Drying ($MgSO_4$) of the organic phase and concentration *in vacuo* afforded 0.57 g crude product. Purification by column chromatography on silica gel eluting first with ethyl acetate:heptane (1:1) then with methanol afforded 0.1 g of *the title compound.*
HPLC-MS (Method A): m/z: 346 (M+23); Rt = 2.89 min.
$^1$H-NMR (DMSO-$d_6$): δ 3.67 (3H, s), 6.62 (1H, d), 6.77 (2H, d), 6.96 (2H, d), 7.40 (1H, dd), 8.05 (1H, d), 9.6 (1H, bs).

**General procedure (E) for preparation of compounds of general formula $I_4$:**

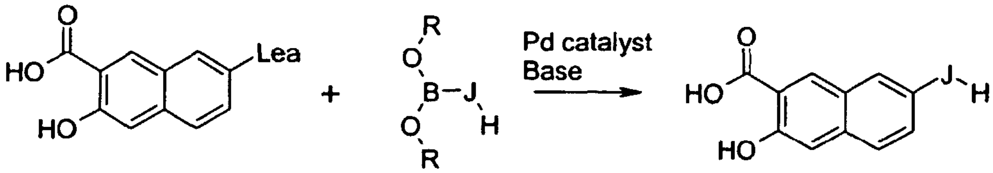


$I_4$

wherein Lea is a leaving group such as Cl, Br, I or $OSO_2CF_3$, R is hydrogen or $C_1$-$C_6$-alkyl, optionally the two R-groups may together form a 5-8 membered ring, a cyclic boronic acid ester, and J is as defined above.

An analogous chemical transformation has previously been described in the literature (Bumagin et al., Tetrahedron, 1997, 53, 14437-14450). The reaction is generally known as the Suzuki coupling reaction and is generally performed by reacting an aryl halide or triflate with an arylboronic acid or a heteroarylboronic acid in the presence of a palladium catalyst and a base such as sodium acetate, sodium carbonate or sodium hydroxide. The solvent can be water, acetone, DMF, NMP, HMPA, methanol, ethanol toluene or a mixture of two or more of these solvents. The reaction is performed at room temperature or at elevated temperature.

The general procedure (E) is further illustrated in the following example:

Example 544 (General Procedure (E))

7-(4-Acetylphenyl)-3-hydroxynaphthalene-2-carboxylic Acid

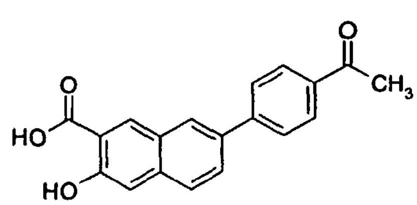

To 7-bromo-3-hydroxynaphthalene-2-carboxylic acid (100 mg, 0.37 mmol) (example 533) was added a solution of 4-acetylphenylboronic acid (92 mg, 0.56 mmol) in acetone (2.2 mL) followed by a solution of sodium carbonate (198 mg, 1.87 mmol) in water (3.3 mL). A suspension of palladium(II) acetate (4 mg, 0.02 mmol) in acetone (0.5 mL) was

filtered and added to the above solution. The mixture was purged with $N_2$ and stirred vigorously for 24 hours at room temperature. The reaction mixture was poured into 1 N hydrochloric acid (aq.) (60 mL) and the precipitate was filtered off and rinsed with water (3 x 40 mL). The crude product was dissolved in acetone (25 mL) and dried with magnesium sulfate (1 h). Filtration followed by concentration furnished the title compound as a solid (92 mg).
$^1$H-NMR (DMSO-$d_6$): δ 12.60 (1H, bs), 8.64 (1H, s), 8.42 (1H, s), 8.08 (2H, d), 7.97 (2H, d), 7.92 (2H, m), 7.33 (1H, s), 2.63 (3H, s); HPLC-MS (Method (A): m/z: 307 (M+1); Rt = 3.84 min.

The compounds in the following examples were prepared in a similar fashion. Optionally, the compounds can be further purified by recrystallization from e.g. ethanol or by chromatography.

Example 545 (General Procedure (E))

3-Hydroxy-7-(3-methoxyphenyl)naphthalene-2-carboxylic acid

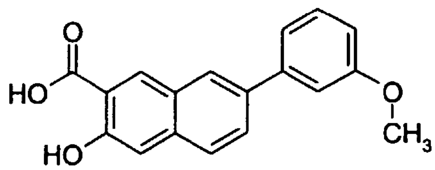

HPLC-MS (Method (A)): m/z: 295 (M+1); Rt = 4.60 min.

Example 546 (General Procedure (E))

3-Hydroxy-7-phenylnaphthalene-2-carboxylic acid

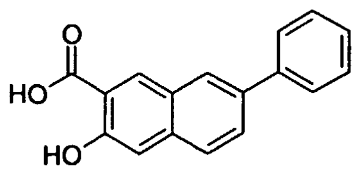

HPLC-MS (Method (A)): m/z: 265 (M+1); Rt = 4.6 min.

Example 547 (General Procedure (E))

3-Hydroxy-7-p-tolylnaphthalene-2-carboxylic acid

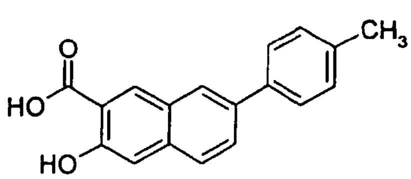

HPLC-MS (Method (A)): m/z: 279 (M+1); Rt = 4.95 min.

Example 548 (General Procedure (E))

7-(4-Formylphenyl)-3-hydroxynaphthalene-2-carboxylic acid

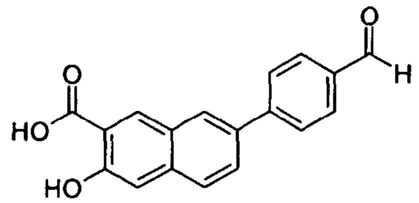

HPLC-MS (Method (A)): m/z: 293 (M+1); Rt = 4.4 min.

Example 549 (General Procedure (E))

6-Hydroxy-[1,2]binaphthalenyl-7-carboxylic acid

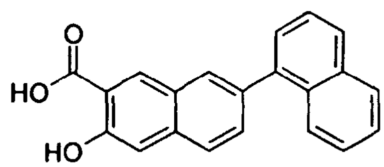

HPLC-MS (Method (A)): m/z: 315 (M+1); Rt = 5.17 min.

Example 550 (General Procedure (E))

7-(4-Carboxy-phenyl)-3-hydroxynaphthalene-2-carboxylic acid

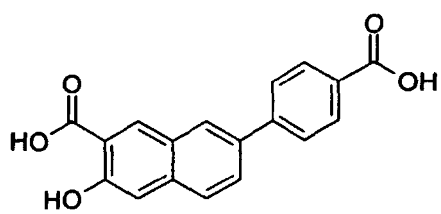

HPLC-MS (Method (A)): m/z: 309 (M+1); Rt = 3.60 min.

Example 551 (General Procedure (E))

7-Benzofuran-2-yl-3-hydroxynaphthalene-2-carboxylic acid

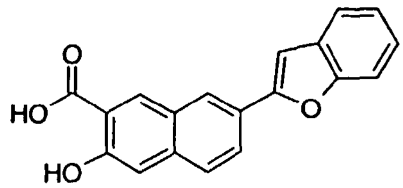

HPLC-MS (Method (A)): m/z: 305 (M+1); Rt = 4.97 min.

Example 552 (General Procedure (E))

3-Hydroxy-7-(4-methoxyphenyl)-naphthalene-2-carboxylic acid

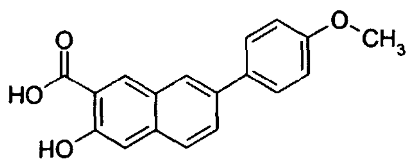

HPLC-MS (Method (A)): m/z: 295 (M+1); Rt = 4.68 min.

Example 553 (General Procedure (E))

7-(3-Ethoxyphenyl)-3-hydroxynaphthalene-2-carboxylic acid

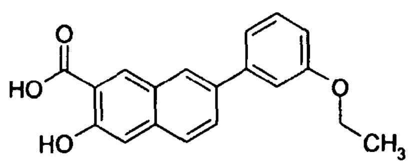

HPLC-MS (Method (A)): m/z: 309 (M+1); Rt = 4.89 min.

Example 554 (General Procedure (E))

7-Benzo[1,3]dioxol-5-yl-3-hydroxynaphthalene-2-carboxylic acid

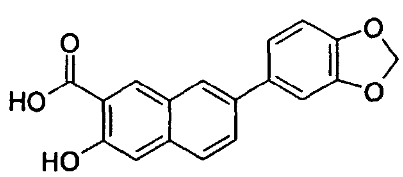

HPLC-MS (Method (A)): m/z: 309 (M+1); Rt = 5.61 min.

Example 555 (General Procedure (E))

7-Biphenyl-3-yl-3-hydroxynaphthalene-2-carboxylic acid

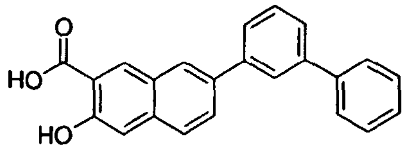

HPLC-MS (Method (A)): m/z: 341 (M+1); Rt = 5.45 min.

**General procedure (F) for preparation of compounds of general formula $I_5$:**

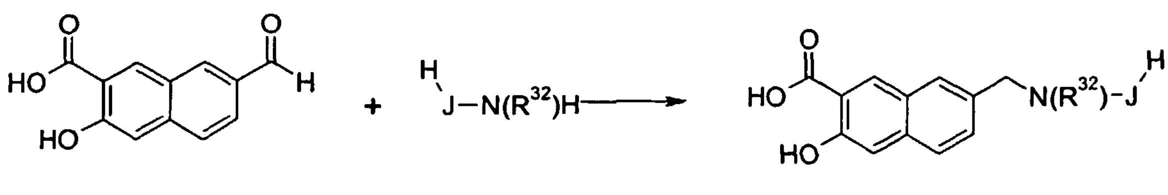

$I_5$

wherein $R^{30}$ is hydrogen or $C_1$-$C_6$-alkyl and T is as defined above

This general procedure (F) is further illustrated in the following example:

Example 556 (General procedure (F))

3-Hydroxy-7-[(4-(2-propyl)phenylamino)methyl]naphthalene-2-carboxylic Acid

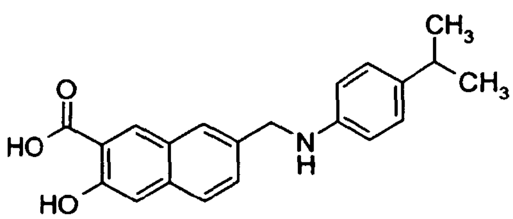

7-Formyl-3-hydroxynaphthalene-2-carboxylic acid (40 mg, 0.19 mmol) (example 535) was suspended in methanol (300 μL). Acetic acid (16 μL. 17 mg, 0.28 mmol) and 4-(2-propyl)aniline (40 μL, 40 mg, 0.30 mmol) were added consecutively, and the resulting mixture was stirred vigorously at room temperature for 2 hours. Sodium cyanoborohydride (1.0 M in tetrahydrofuran, 300 μL, 0.3 mmol) was added, and the stirring was continued for another 17 hours. The reaction mixture was poured into 6 N hydrochloric acid (aq.) (6 mL), and the precipitate was filtered off and rinsed with water (3 x 2 mL) to yield the title compound (40 mg) as its hydrochloride salt. No further purification was necessary. $^1$H-NMR (DMSO-$d_6$): δ 10.95 (1H, bs), 8.45 (1H, s), 7.96 (1H, s), 7.78 (1H, d), 7.62 (1H, d), 7.32 (1H, s), 7.13 (2H, bd), 6.98 (2H, bd), 4.48 (2H, s), 2.79 (1H, sept), 1.14 (6H, d); HPLC-MS (Method (A)): m/z: 336 (M+1); Rt = 3.92 min.

The compounds in the following examples were made using this general procedure (F).

Example 557 (General procedure (F))

7-{[(4-Bromophenyl)amino]methyl}-3-hydroxynaphthalene-2-carboxylic Acid

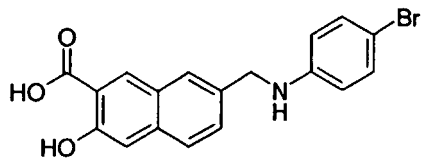

HPLC-MS (Method C): m/z: 372 (M+1); Rt = 4.31min.

Example 558 (General procedure (F))

7-{[(3,5-Dichlorophenyl)amino]methyl}-3-hydroxynaphthalene-2-carboxylic Acid

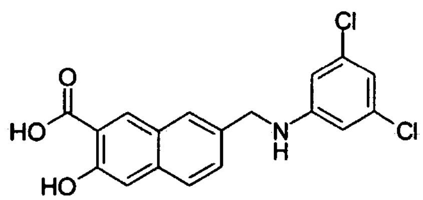

HPLC-MS (Method C): m/z: 362 (M+1); Rt = 4.75 min.

Example 559 (General procedure (F))

7-{[(Benzothiazol-6-yl)amino]methyl}-3-hydroxynaphthalene-2-carboxylic Acid

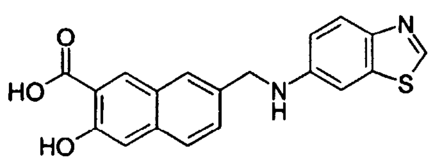

HPLC-MS (Method C): m/z: 351 (M+1); Rt = 3.43 min.

Example 560 (General procedure (F))

3-Hydroxy-7-{[(quinolin-6-yl)amino]methyl}naphthalene-2-carboxylic Acid

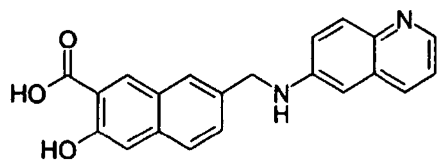

HPLC-MS (Method C): m/z: 345 (M+1); Rt = 2.26 min.

Example 561 (General procedure (F))

3-Hydroxy-7-{[(4-methoxyphenyl)amino]methyl}naphthalene-2-carboxylic Acid

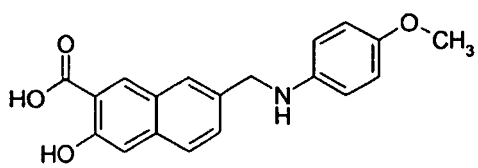

HPLC-MS (Method C): m/z: 324 (M+1); Rt = 2.57min.

Example 562 (General procedure (F))

7-{[(2,3-Dihydrobenzofuran-5-ylmethyl)amino]methyl}-3-hydroxynaphthalene-2-carboxylic Acid

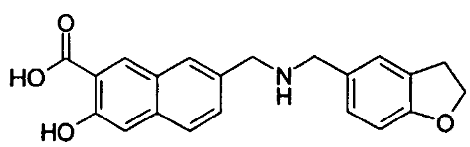

HPLC-MS (Method C): m/z: 350 (M+1); Rt = 2.22 min.

Example 563 (General procedure (F))

7-{[(4-Chlorobenzyl)amino]methyl}-3-hydroxynaphthalene-2-carboxylic Acid

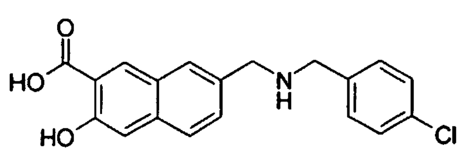

HPLC-MS (Method C): m/z: 342 (M+1); Rt = 2.45 min.

Example 564 (General procedure (F))

3-Hydroxy-7-{[(naphthalen-1-ylmethyl)amino]methyl}naphthalene-2-carboxylic Acid

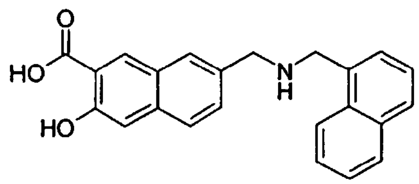

HPLC-MS (Method C): m/z: 357 (M+1); Rt = 2.63 min.

Example 565 (General procedure (F))

7-{[(Biphenyl-2-ylmethyl)amino]methyl}-3-hydroxynaphthalene-2-carboxylic Acid

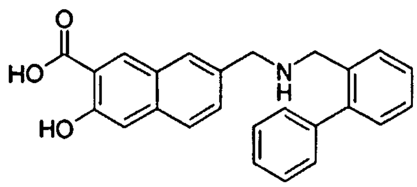

HPLC-MS (Method C): m/z: 384 (M+1); Rt = 2.90 min.

Example 566 (General procedure (F))

3-Hydroxy-7-{[(4-phenoxybenzyl)amino]methyl}naphthalene-2-carboxylic Acid

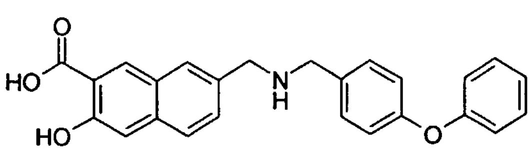

HPLC-MS (Method C): m/z: 400 (M+1); Rt = 3.15 min.

Example 567 (General procedure (F))

3-Hydroxy-7-{[(4-methoxybenzyl)amino]methyl}naphthalene-2-carboxylic Acid

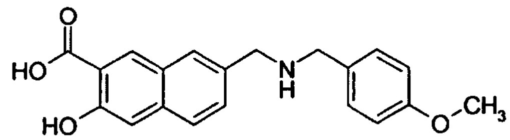

HPLC-MS (Method C): m/z: 338 (M+1); Rt = 2.32 min.

**General procedure (G) for preparation of compounds of general formula $I_6$:**

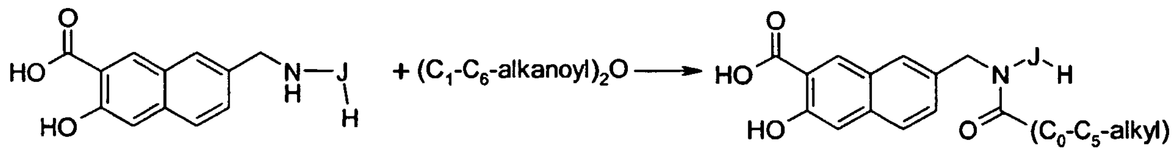

$I_6$

wherein J is as defined above and the moiety ($C_1$-$C_6$-alkanoyl)$_2$O is an anhydride.

The general procedure (G) is illustrated by the following example:

Example 568 (General procedure (G))

*N*-Acetyl-3-hydroxy-7-[(4-(2-propyl)phenylamino)methyl]naphthalene-2-carboxylic Acid

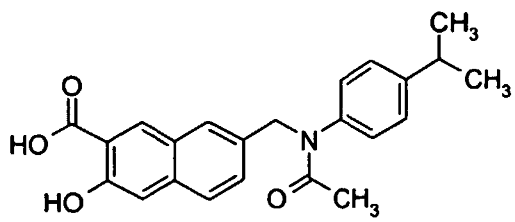

3-Hydroxy-7-[(4-(2-propyl)phenylamino)methyl]naphthalene-2-carboxylic acid (25 mg, 0.07 mmol) (example 556) was suspended in tetrahydrofuran (200 μL). A solution of sodium hydrogencarbonate (23 mg, 0.27 mmol) in water (200 μL) was added followed by acetic anhydride (14 μL, 15 mg, 0.15 mmol). The reaction mixture was stirred vigorously for 65 hours at room temperature before 6 N hydrochloric acid (4 mL) was added. The precipitate was filtered off and rinsed with water (3 x 1 mL) to yield the title compound (21 mg). No further purification was necessary.
$^1$H-NMR (DMSO-$d_6$): δ 10.96 (1H, bs), 8.48 (1H, s), 7.73 (1H, s), 7.72 (1H, d), 7.41 (1H, dd), 7.28 (1H, s), 7.23 (2H, d), 7.18 (2H, d), 4.96 (2H, s), 2.85 (1H, sept), 1.86 (3H, s), 1.15 (6H, d); HPLC-MS (Method (A)): m/z: 378 (M+1); Rt = 3.90 min.

The compounds in the following examples were prepared in a similar fashion.

Example 569 (General procedure (G))

*N*-Acetyl-7-{[(4-bromophenyl)amino]methyl}-3-hydroxynaphthalene-2-carboxylic Acid

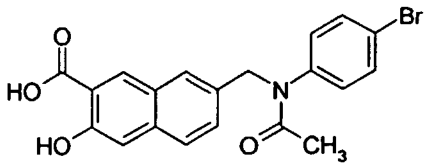

HPLC-MS (Method C): m/z: 414 (M+1); Rt = 3.76 min.

Example 570 (General procedure (G))

*N*-Acetyl-7-{[(2,3-dihydrobenzofuran-5-ylmethyl)amino]methyl}-3-hydroxynaphthalene-2-carboxylic Acid

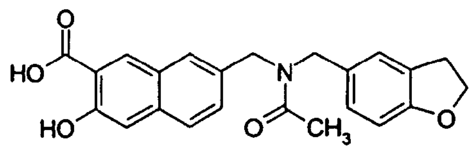

HPLC-MS (Method C): m/z: 392 (M+1); Rt = 3.26 min.

Example 571 (General procedure (G))

*N*-Acetyl-7-{[(4-chlorobenzyl)amino]methyl}-3-hydroxynaphthalene-2-carboxylic Acid

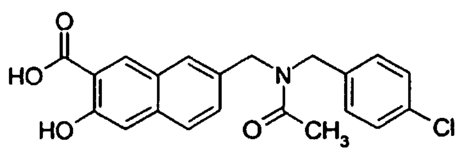

HPLC-MS (Method C): m/z: 384 (M+1); Rt = 3.67 min.

Compounds of the invention may also include tetrazoles:

Example 572

5-(3-(Naphthalen-2-yloxymethyl)-phenyl)-1*H*-tetrazole

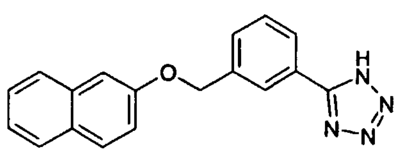

To a mixture of 2-naphthol (10 g, 0.07 mol) and potassium carbonate (10 g, 0.073 mol) in acetone (150 mL), alpha-bromo-m-tolunitril (13.6 g, 0.07 mol) was added in portions. The reaction mixture was stirred at reflux temperature for 2.5 hours. The cooled reaction mixture was filtered and evaporated in vacuo affording an oily residue (19 g) which was dissolved in diethyl ether (150 mL) and stirred with a mixture of active carbon and $MgSO_4$ for 16 hours. The mixture

was filtered and evaporated in vacuo affording crude 18.0 g (100 %) of 3-(naphthalen-2-yloxymethyl)-benzonitrile as a solid.

12 g of the above benzonitrile was recrystallised from ethanol (150 mL) affording 8.3 g (69 %) of 3-(naphthalen-2-yloxymethyl)-benzonitrile as a solid.

M.p. 60 - 61 ˚C.

Calculated for $C_{18}H_{13}NO$:

C, 83.37 %; H, 5.05 %; N, 5.40 %; Found

C, 83.51 %; H, 5.03 %; N, 5.38 %.

To a mixture of sodium azide (1.46 g, 22.5 mmol) and ammonium chloride (1.28 g, 24.0 mmol) in dry dimethylformamide (20 mL) under an atmosphere of nitrogen, 3-(naphthalen-2-yloxymethyl)-benzonitrile (3.9 g, 15 mmol) was added and the reaction mixture was stirred at 125 ˚C for 4 hours. The cooled reaction mixture was poured on to ice water (300 mL) and acidified to pH = 1 with 1 N hydrochloric acid. The precipitate was filtered off and washed with water, dried at 100 ˚C for 4 hours affording 4.2 g (93 %) of the title compound.

M.p. 200 - 202 ˚C.

Calculated for $C_{18}H_{14}N_4O$:

C, 71.51 %; H, 4.67 %; N, 18.54 %; Found

C. 72.11 %; H, 4.65 %; N, 17.43 %.

$^1H$ NMR (400 MHz, DMSO-$d_6$) $\delta_H$ 5.36 (s, 2H), 7.29 (dd, 1H), 7.36 (dt, 1H), 7.47 (m, 2H), 7.66 (t, 1H), 7.74 (d, 1H), 7.84 (m, 3H), 8.02 (d, 1H), 8.22 (s, 1H).

Example 573

N-(3-(Tetrazol-5-yl)phenyl)-2-naphtoic acid amide

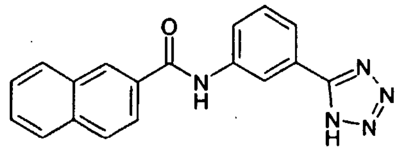

2-Naphtoic acid (10 g, 58 mmol) was dissolved in dichloromethane (100 mL) and N,N-dimethylformamide (0.2 mL) was added followed by thionyl chloride (5.1 ml, 70 mmol). The mixture was heated at reflux temperature for 2 hours. After cooling to room temperature, the mixture was added dropwise to a mixture of 3-aminobenzonitril (6.90 g, 58 mmol) and triethyl amine (10 mL) in dichloromethane (75 mL). The resulting mixture was stirred at room temperature for 30 minutes. Water (50 mL) was added and the volatiles was exaporated in vacuo. The resulting mixture was filtered and the filter cake was washed with water followed by heptane (2 x 25 mL). Drying in vacuo at 50 ˚C for 16 hours afforded 15.0 g (95 %) of N-(3-cyanophenyl)-2-naphtoic acid amide.

M.p. 138-140 ˚C

The above naphthoic acid amide (10 g, 37 mmol) was dissolved in N,N-dimethylformamide (200 mL) and sodium azide (2.63 g, 40 mmol) and ammonium chloride (2.16 g, 40 mmol) were added and the mixture heated at 125 ˚C for 6 hours. Sodium azide (1.2 g) and ammonium chloride (0.98 g) were added and the mixture heated at 125 ˚C for 16 hours. After cooling, the mixture was poured into water (1.5 l) and stirred at room temperature for 30 minutes. The solid formed was filtered off, washed with water and dried in vacuo at 50 ˚C for 3 days affording 9.69 g (84 %) of the title compound as a solid which could be further purified by treatment with ethanol at reflux temperature.

$^1H$ NMR (200 MHz, DMSO-$d_6$): $\delta_H$ 7.58-7.70 (m, 3H), 7.77 (d, 1H), 8.04-8.13 (m, 5H), 8.65 (d, 1H), 10.7 (s, 1H).

Calculated for $C_{18}H_{13}N_5O$, 0.75 $H_2O$:

C, 65.74 %; H, 4.44 %; N, 21.30 %. Found:

C, 65.58 %; H, 4.50 %; N, 21.05 %.

Example 574

5-[3-(Biphenyl-4-yloxymethyl)phenyl]-1*H*-tetrazole

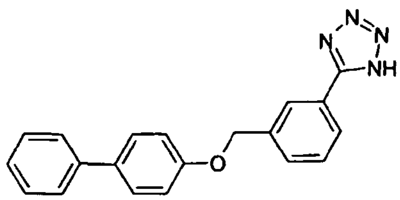

To a solution of 4-phenylphenol (10.0 g, 59 mmol) in dry N,N-dimethyl-formamide (45 mL) kept under an atmosphere of nitrogen, sodium hydride (2.82 g, 71 mmol, 60 % dispersion in oil) was added in portions and the reaction mixture was stirred until gas evolution ceased. A solution of m-cyanobenzyl bromide (13 g, 65 mmol) in dry N,N-dimethylformamide (45 mL) was added dropwise and the reaction mixture was stirred at room temperature for 18 hours. The reaction mixture was poured on to ice water (150 mL). The precipitate was filtered of and washed with 50 % ethanol (3 x 50 mL), ethanol (2 x 50 mL), diethyl ether (80 mL), and dried in vacuo at
50 ˚C for 18 hours affording crude 17.39 g of 3-(biphenyl-4-yloxymethyl)-benzonitrile as a solid.
$^1$H NMR (200 MHz, $CDCl_3$) $\delta_H$ 5.14 (s, 2H), 7.05 (m, 2H), 7.30 - 7.78 (m, 11H).

To a mixture of sodium azide (2.96 g, 45.6 mmol) and ammonium chloride (2.44 g, 45.6 mmol) in dry N,N-dimethylformamide (100 mL) under an atmosphere of nitrogen, 3-(biphenyl-4-yloxymethyl)-benzonitrile (10.0 g, 35.0 mmol) was added and the reaction mixture was stirred at 125 ˚C for 18 hours. The cooled reaction mixture was poured on to a mixture of 1N hydrochloric acid (60 mL) and ice water (500 mL). The precipitate was filtered off and washed with water (3 x 100 mL), 50 % ethanol (3 x 100 mL), ethanol (50 mL), diethyl ether (50 mL), ethanol (80 mL), and dried in vacuo at 50 ˚C for 18 hours affording 8.02 g (70 %) of the title compound.
$^1$H NMR (200 MHz, DMSO-$d_6$) $\delta_H$ 5.31 (s, 2H), 7.19 (m, 2H), 7.34 (m, 1H), 7.47 (m, 2H), 7.69 (m, 6H), 8.05 (dt, 1H), 8.24 (s, 1H).

Example 575

5-(3-Phenoxymethyl)-phenyl)-tetrazole

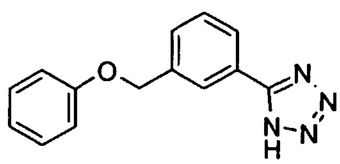

3-Bromomethylbenzonitrile (5.00 g, 25.5 mmol) was dissolved in N,N-dimethylformamide (50 mL), phenol (2.40 g, 25.5 mmol) and potassium carbonate (10.6 g, 77 mmol) were added. The mixture was stirred at room temperature for 16 hours. The mixture was poured into water (400 mL) and extracted with ethyl acetate (2 x 200 mL). The combined organic extracts were washed with water (2 x 100 mL), dried ($MgSO_4$) and evaporated in vacuo to afford 5.19 g (97 %) 3-(phenoxymethyl)benzonitrile as an oil.
TLC: $R_f$ = 0.38 (Ethyl acetate/heptane = 1:4)

The above benzonitrile (5.19 g, 24.8 mmol) was dissolved in N,N-dimethylformamide (100 mL) and sodium azide (1.93 g, 30 mmol) and ammonium chloride (1.59 g, 30 mmol) were added and the mixture was heated at 140 ˚C for 16 hours. After cooling, the mixture was poured into water (800 mL). The aqeous mixture was washed with ethyl acetate (200 mL). The pH of the aqueous phase was adjusted to 1 with 5 N hydrochloric acid and stirred at room temperature for 30 minutes. Filtration, washing with water and drying in vacuo at 50 ˚C afforded 2.06 g (33 %) of the title compound as a solid.
$^1$H NMR (200 MHz, $CDCl_3$ + DMSO-$d_6$) $\delta_H$ 5.05 (s, 2H), 6.88 (m, 3H), 7.21 (m, 2H), 7.51 (m, 2H), 7.96 (dt, 1H), 8.14 (s, 1H).

Example 576

5-[3-(Biphenyl-4-ylmethoxy)phenyl]-1H-tetrazole

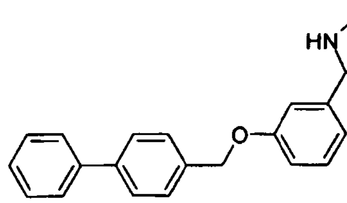

To a solution of 3-cyanophenol (5.0 g, 40.72 mmol) in dry N,N-dimethylformamide (100 mL) kept under an atmosphere of nitrogen, sodium hydride (2 g, 48.86 mmol, 60 % dispersion in oil) was added in portions and the reaction mixture was stirred until gas evolution ceased. p-Phenylbenzyl chloride (9.26 g, 44.79 mmol) and potassium iodide (0.2 g, 1.21 mmol) were added and the reaction mixture was stirred at room temperature for 60 hours. The reaction mixture was poured on to a mixture of saturated sodium carbonate (100 mL) and ice water (300 mL). The precipitate was filtered of and washed with water (3 x 100 mL), n-hexane (2 x 80 mL) and dried in vacuo at 50 ˚C for 18 hours affording 11.34 g (98 %) of 3-(biphenyl-4-ylmethoxy)-benzonitrile as a solid.

To a mixture of sodium azide (2.37 g, 36.45 mmol) and ammonium chloride (1.95 g, 36.45 mmol) in dry N,N-dimethylformamide (100 mL) under an atmosphere of nitrogen, 3-(biphenyl-4-ylmethoxy)-benzonitrile (8.0 g, 28.04 mmol) was added and the reaction mixture was stirred at 125 ˚C for 18 hours. To the cooled reaction mixture water (100 mL) was added and the reaction mixture stirred for 0.75 hour. The precipitate was filtered off and washed with water, 96 % ethanol (2 x 50 mL), and dried in vacuo at 50˚C for 18 hours affording 5.13 g (56 %) of the title compound.

$^1$H NMR (200 MHz, DMSO-$d_6$) $\delta_H$ 5.29 (s, 2H), 7.31 (dd, 1H), 7.37 - 7.77 (m, 12H).

Example 577

5-[4-(Biphenyl-4-ylmethoxy)-3-methoxyphenyl]-1H-tetrazol

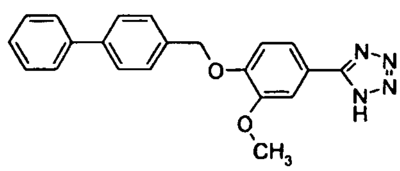

This compound was made similarly as described in example 576.

Example 578

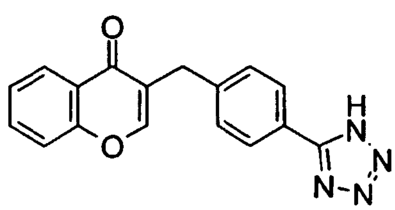

Example 579

5-(2-Naphtylmethyl)-1H-tetrazole

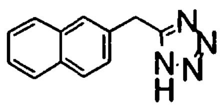

This compound was prepared similarly as described in example 572, step 2.

Example 580

5-(1-Naphtylmethyl)-1H-tetrazole

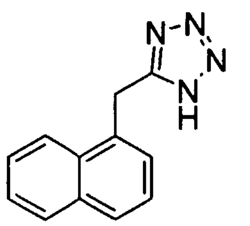

This compound was prepared similarly as described in example 572, step 2.

Example 581

5-[4-(Biphenyl-4-yloxymethyl)phenyl]-1H-tetrazole

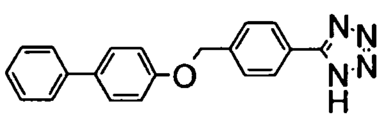

A solution of alpha-bromo-p-tolunitrile (5.00 g, 25.5 mmol), 4-phenylphenol (4.56 g, 26.8 mmol), and potassium carbonate (10.6 g, 76.5 mmol) in N,N-dimethylformamide (75 mL) was stirred vigorously for 16 hours at room temperature. Water (75 mL) was added and the mixture was stirred at room temperature for 1 hour. The precipitate was filtered off and washed with thoroughly with water. Drying in vacuo over night at 50 ˚C afforded 7.09 g (97 %) of 4-(biphenyl-4-yloxymethyl)benzonitrile as a solid.

The above benzonitrile (3.00 g, 10.5 mmol) was dissolved in N,N-dimethylformamide (50 mL), and sodium azide (1.03 g, 15.8 mmol) and ammonium chloride (0.84 g, 15.8 mmol) were added and the mixture was stirred 16 hours at 125 ˚C. The mixture was cooled to room temperature and water (50 mL) was added. The suspension was stirred overnight, filtered, washed with water and dried in vacuo at 50 ˚C for 3 days to give crude 3.07 g (89 %) of the title compound. From the mother liquor crystals were colected and washed with water, dried by suction to give 0.18 g (5 %) of the title compound as a solid.
$^1$H NMR (200 MHz, DMSO-$d_6$): $\delta_H$ 5.21 (s, 2H), 7.12 (d, 2H), 7.30 (t, 1H), 7.42 (t, 2H), 7.56-7.63 (m, 6H), 8.03 (d, 2H).
Calculated for $C_{20}H_{16}N_4O$, $2H_2O$:
C, 65.92 %; H, 5.53 %; N, 15.37 %. Found:
C, 65.65 %; H, 5.01 %; N, 14.92 %.

Example 582

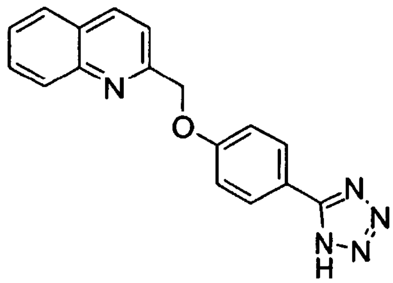

This compound was prepared similarly as described in example 576.

Example 583

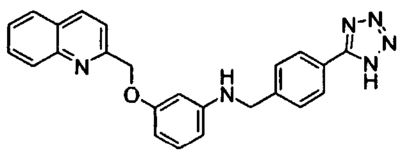

Example 584

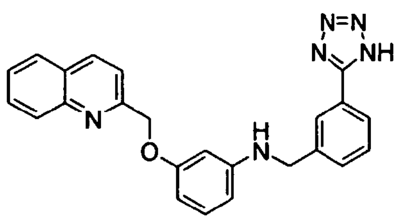

Example 585

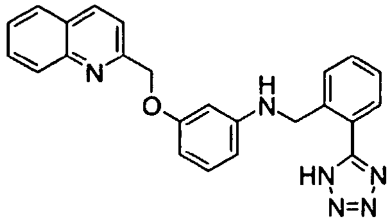

Example 586

5-(3-(Biphenyl-4-yloxymethyl)-benzyl)-1*H*-tetrazole

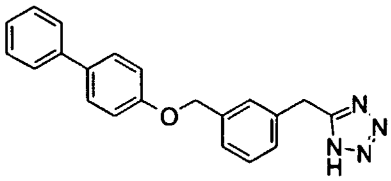

Example 587

5-(1-Naphthyl)-1*H*-tetrazole

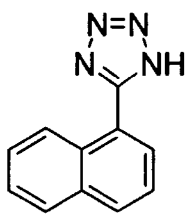

This compound was prepared similarly as described in example 572, step 2.

Example 588

5-[3-Methoxy-4-(4-methylsulfonylbenzyloxy)phenyl]-1*H*-tetrazole

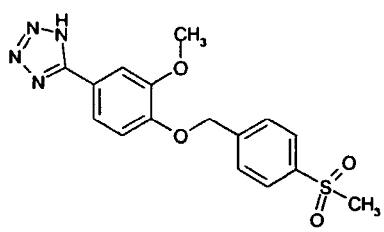

This compound was made similarly as described in example 576.

Example 589

5-(2-Naphthyl)-1*H*-tetrazole

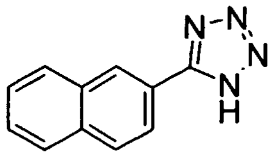

This compound was prepared similarly as described in example 572, step 2.

Example 590

2-Amino-N-(1*H*-tetrazol-5-yl)-benzamide

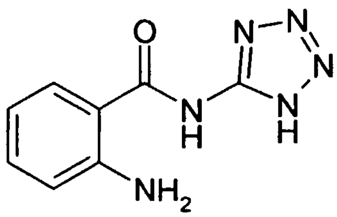

Example 591

5-(4-Hydroxy-3-methoxyphenyl)-1*H*-tetrazole

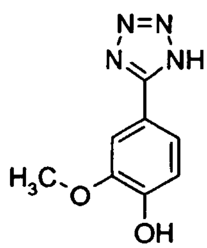

This compound was prepared similarly as described in example 572, step 2.

Example 592

4-(2H-Tetrazol-5-ylmethoxy)benzoic acid

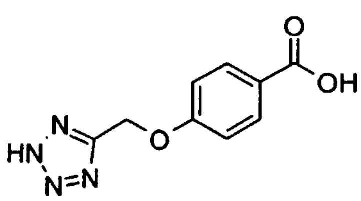

To a mixture of methyl 4-hydroxybenzoate (30.0 g, 0.20 mol), sodium iodide (30.0 g, 0.20 mol) and potassium

carbonate (27.6 g, 0.20 mol) in acetone (2000 mL) was added chloroacetonitrile (14.9 g , 0.20 mol). The mixture was stirred at RT for 3 days. Water was added and the mixture was acidified with 1N hydrochloric acid and the mixture was extracted with diethyl ether. The combined organic layers were dried over $Na_2SO_4$ and concentrated *in vacuo.* The residue was dissolved in acetone and chloroacetonitrile (6.04 g,0.08 mol), sodium iodide (12.0 g, 0.08 mol) and potassium carbonate (11.1 g, 0.08 mol) were added and the mixture was stirred for 16 hours at RT and at 60 °C. More chloroacetonitrile was added until the conversion was 97%. Water was added and the mixture was acidified with 1N hydrochloric acid and the mixture was extracted with diethyl ether. The combined organic layers were dried over $Na_2SO_4$ and concentrated *in vacuo* to afford methyl 4-cyanomethyloxybenzoate in quantitative yield. This compound was used without further purification in the following step.

A mixture of methyl 4-cyanomethyloxybenzoate (53.5 g,0.20 mol), sodium azide (16.9 g, 0.26 mol) and ammonium chloride (13.9 g, 0.26 mol) in DMF 1000 (mL) was refluxed overnight under $N_2$. After cooling, the mixture was concentrated *in vacuo.* The residue was suspended in cold water and extracted with ethyl acetate. The combined organic phases were washed with brine, dried over $Na_2SO_4$ and concentrated *in vacuo,* to afford methyl 4-(2*H*-tetrazol-5-ylmethoxy)benzoate. This compound was used as such in the following step.

Methyl 4-(2H-Tetrazol-5-ylmethoxy)-benzoate was refluxed in 3N sodium hydroxide. The reaction was followed by TLC (DCM:MeOH = 9:1). The reaction mixture was cooled, acidified and the product filtered off. The impure product was washed with DCM, dissolved in MeOH, filtered and purified by column chromatography on silica gel (DCM:MeOH = 9:1).The resulting product was recrystallised from DCM:MeOH=95:5. This was repeated until the product was pure. This afforded 13.82 g (30 %) of the title compound.

$^1$H-NMR (DMSO-$d_6$): 4.70 (2H, s), 7.48 (2H, d), 7.73 (2H, d), 13 (1H, bs).

Example 593

4-(2H-Tetrazol-5-ylmethylsulfanyl)benzoic acid

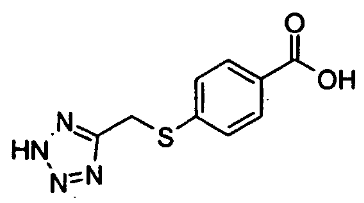

To a solution of sodium hydroxide (10.4 g, 0.26 mol) in degassed water (600 mL) was added 4-mercaptobenzoic acid (20.0 g, 0.13 mol). This solution was stirred for 30 minutes. To a solution of potassium carbonate (9.0 g, 65 mmol) in degassed water (400 mL) was added chloroacetonitrile (9.8 g, (0.13 mol) portion-wise. These two solutions were mixed and stirred for 48 hours at RT under $N_2$. The mixture was filtered and washed with heptane. The aqueous phase was acidified with 3N hydrochloric acid and the product was filtered off, washed with water and dried, affording 4-cyanomethylsulfanylbenzoic acid (27.2 g, 88%). This compound was used without further purification in the following step.

A mixture of 4-cyanomethylsulfanylbenzoic acid (27.2 g, 0.14 mol), sodium azide (11.8 g, 0,18 mol) and ammonium chloride (9.7 g, 0.18 mol) in DMF (1000 mL) was refluxed overnight under $N_2$. The mixture was concentrated *in vacuo.* The residue was suspended in cold water and extracted with diethyl ether. The combined organic phases were washed with brine, dried over $Na_2SO_4$ and concentrated *in vacuo.* Water was added and the precipitate was filtered off. The aqueous layer was concentrated *in vacuo,* water was added and the precipitate filtered off. The combined impure products were purified by column chromatography using DCM:MeOH = 9:1 as eluent, affording the title compound (5.2 g, 16%).

$^1$H-NMR (DMSO-$d_6$): 5.58 (2H, s), 7.15 (2H, d), 7.93 (2H, d), 12.7 (1H, bs).

Example 594

3-(2*H*-Tetrazol-5-yl)-9*H*-carbazole

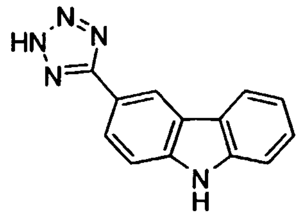

3-Bromo-9*H*-carbazole was prepared as described by Smith et al. in Tetrahedron 1992, 48, 7479-7488. A solution of 3-bromo-9*H*-carbazole (23.08 g, 0.094 mol) and cuprous cyanide (9.33 g, 0.103 mol) in *N*-methyl-pyrrolidone (300 ml) was heated at 200 ˚C for 5 h. The cooled reaction mixture was poured on to water (600 ml) and the precipitate was filtered off and washed with ethyl acetate (3 x 50 ml). The filtrate was extracted with ethyl acetate (3 x 250 ml) and the combined ethyl acetate extracts were washed with water (150 ml), brine (150 ml), dried ($MgSO_4$) and concentrated *in vacuo.* The residue was crystallised from heptanes and recrystallised from acetonitrile (70 ml) affording 7.16 g (40 %) of 3-cyano-9*H*-carbazole as a solid. M.p. 180 - 181 ˚C.

3-Cyano-9H-carbazole (5.77 g, 30 mmol) was dissolved in *N,N*-dimethylformamide (150 ml), and sodium azide (9.85 g, 152 mmol), ammonium chloride (8.04 g. 150 mmol) and lithium chloride (1.93 g, 46 mmol) were added and the mixture was stirred for 20 h at 125 ˚C. To the reaction mixture was added an additional portion of sodium azide (9.85 g, 152 mmol) and ammonium chloride (8.04 g, 150 mmol) and the reaction mixture was stirred for an additional 24 h at 125 ˚C. The cooled reaction mixture was poured on to water (500 ml). The suspension was stirred for 0.5 h, and the precipitate was filtered off and washed with water (3 x 200 ml) and dried *in vacuo* at 50 ˚C. The dried crude product was suspended in diethyl ether (500 ml) and stirred for 2 h, filtered off and washed with diethyl ether (2 x 200 ml) and dried *in vacuo* at 50 ˚C affording 5.79 g (82 %) of the title compound as a solid.

$^1$H-NMR (DMSO-$d_6$): δ 11.78 (1H, bs), 8.93 (1H, d), 8.23 (1H, d), 8.14 (1H, dd), 7.72 (1H, d), 7.60 (1H, d), 7.49 (1H, t), 7.28 (1H, t); HPLC-MS (Method C): m/z: 236 (M+1); Rt = 2.77 min.

The following commercially available tetrazoles do all bind to the His B10 $Zn^{2+}$ site of the insulin hexamer:

Example 595

5-(3-Tolyl)-1*H*-tetrazole

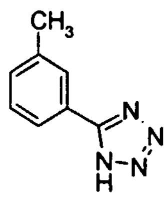

Example 596

5-(2-Bromophenyl)tetrazole

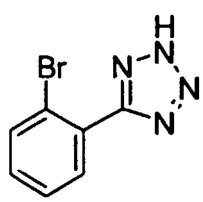

Example 597

5-(4-Ethoxalylamino-3-nitrophenyl)tetrazole

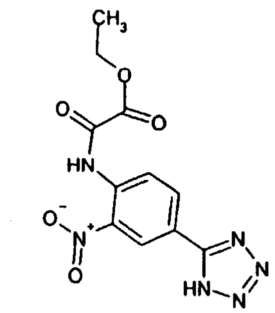

Example 598

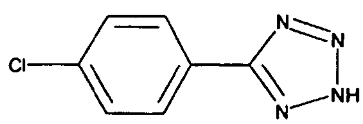

Example 599

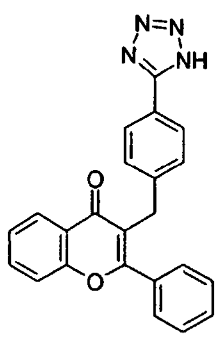

Example 600

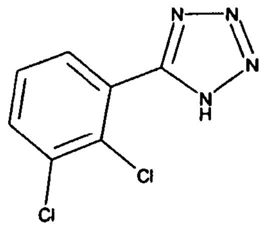

Example 601

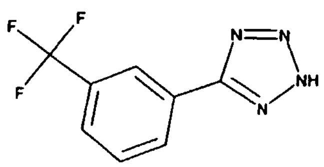

Example 602

Tetrazole

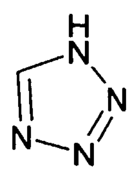

Example 603

5-Methyltetrazole

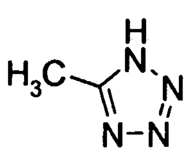

Example 604

5-Benzyl-2H-tetrazole

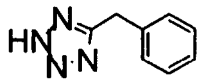

Example 605

4-(2H-Tetrazol-5-yl)benzoic acid

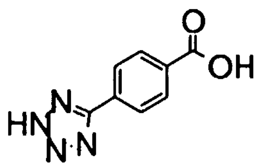

Example 606

5-Phenyl-2H-tetrazole

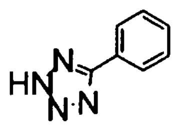

Example 607

5-(4-Chlorophenylsulfanylmethyl)-2H-tetrazole

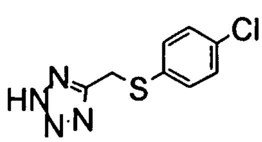

Example 608

5-(3-Benzyloxyphenyl)-2H-tetrazole

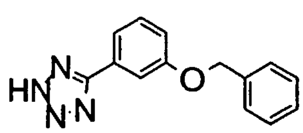

Example 609

2-Phenyl-6-(1H-tetrazol-5-yl)-chromen-4-one

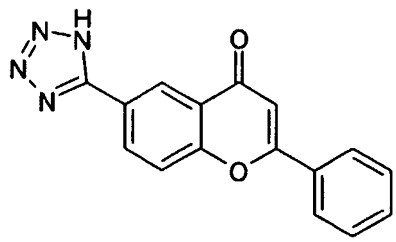

Example 610

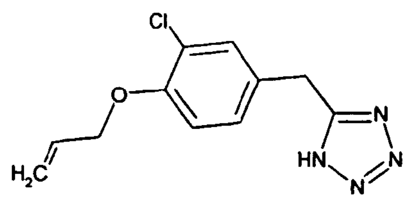

Example 611

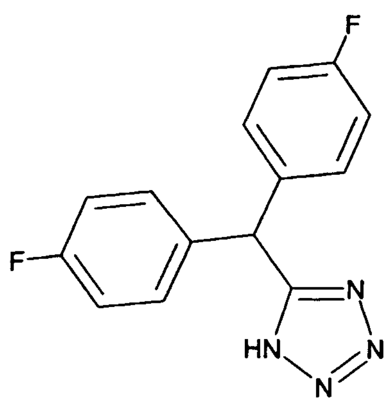

Example 612

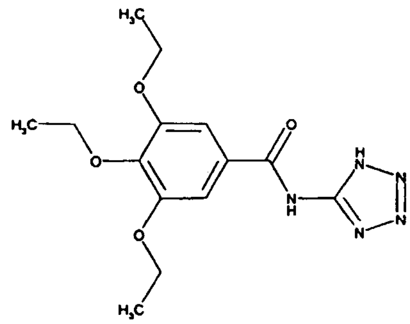

Example 613

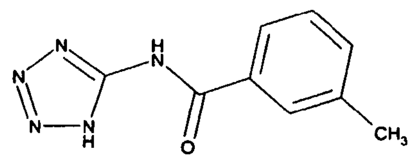

Example 614

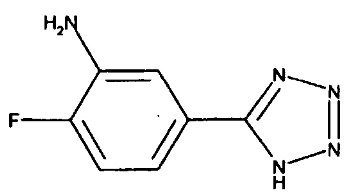

Example 615

5-(4-Bromo-phenyl)-1H-tetrazole

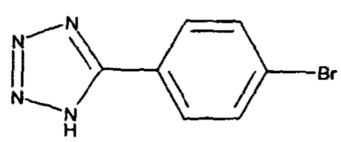

Example 616

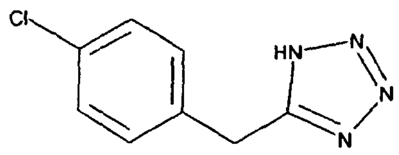

Example 617

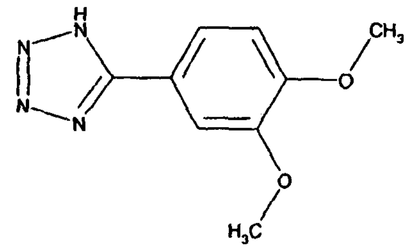

Example 618

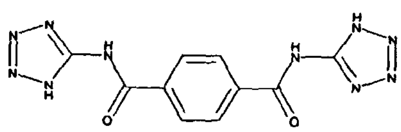

Example 619

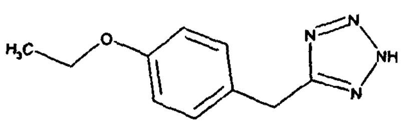

Example 620

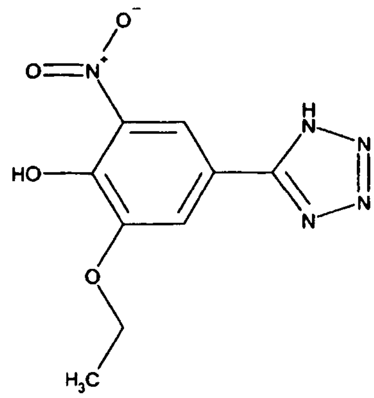

Example 621

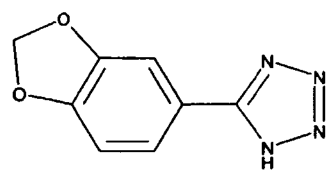

Example 622

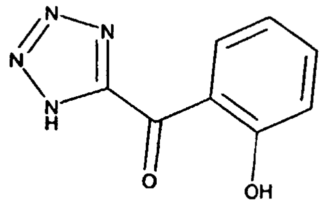

Example 623

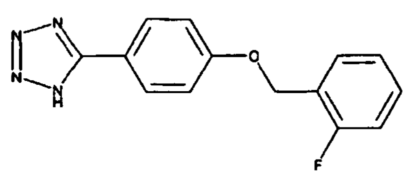

Example 624

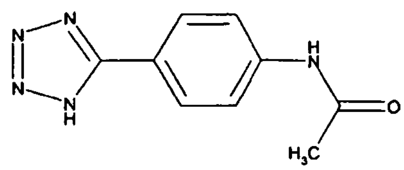

Example 625

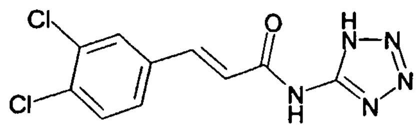

Example 626

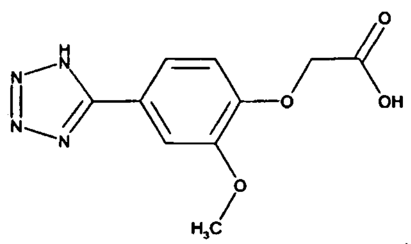

Example 627

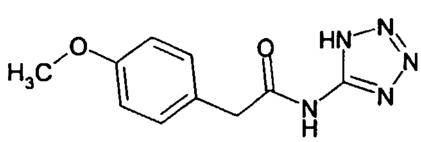

Example 628

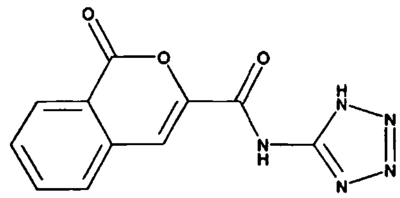

Example 629

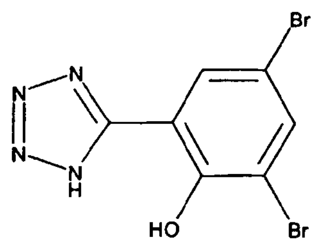

Example 630

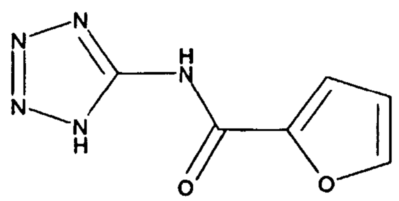

Example 631

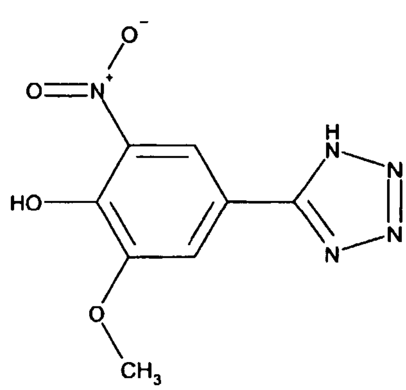

Example 632

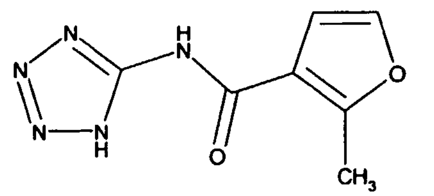

Example 633

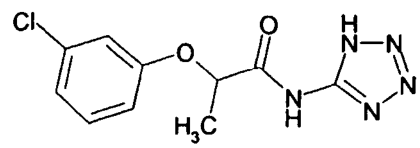

Example 634

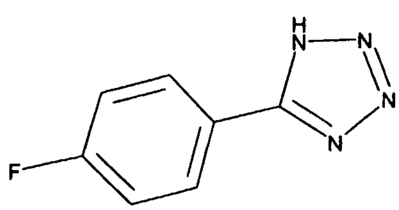

Example 635

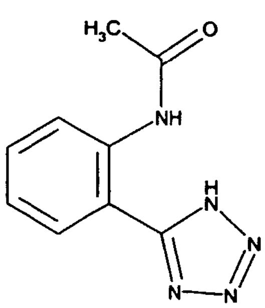

Example 636

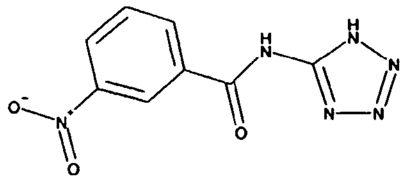

Example 637

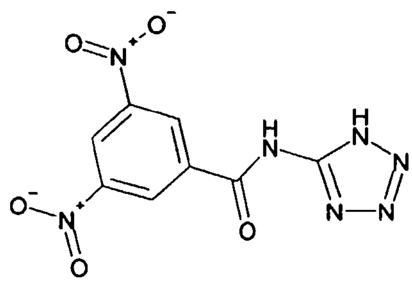

Example 638

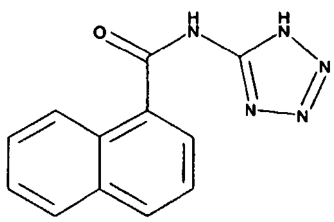

Example 639

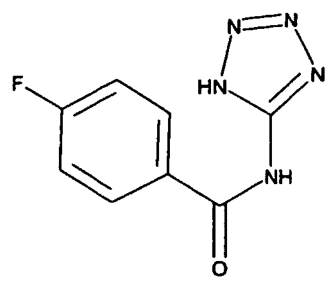

Example 640

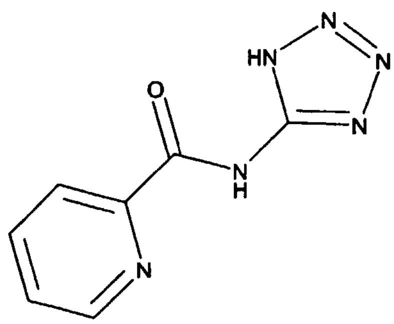

Example 641

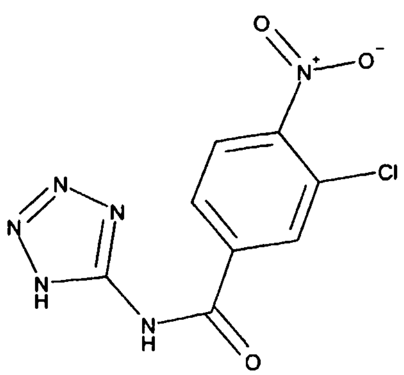

Example 642

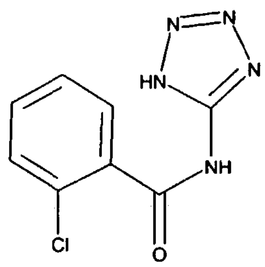

Example 643

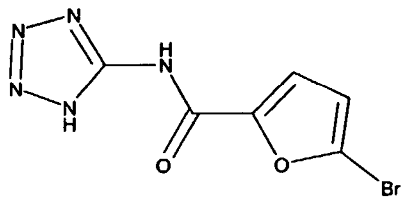

Example 644

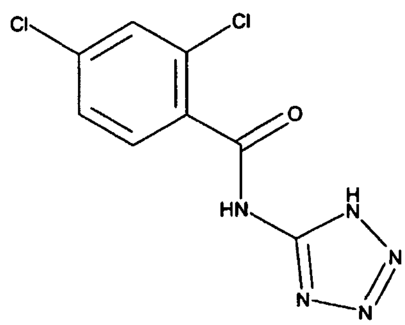

Example 645

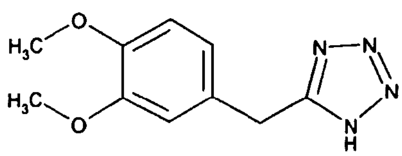

Example 646

5-(2,6-Dichlorobenzyl)-2*H*-tetrazole

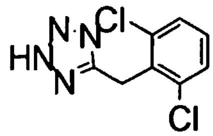

**General procedure (H) for preparation of compounds of general formula $I_7$:**

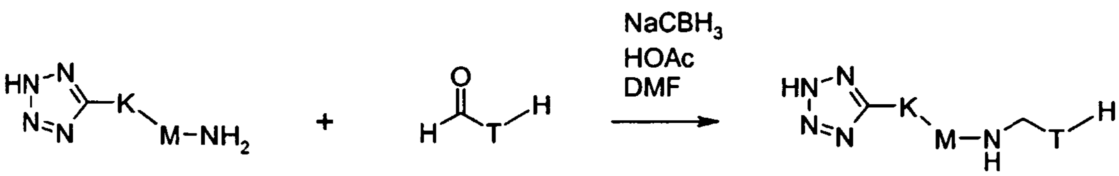


$I_7$

wherein K, M, and T are as defined above.

The reaction is generally known as a reductive alkylation reaction and is generally performed by stirring an aldehyde with an amine at low pH (by addition of an acid, such as acetic acid or formic acid) in a solvent such as THF, DMF, NMP, methanol, ethanol, DMSO, dichloromethane, 1,2-dichloroethane, trimethyl orthoformate, triethyl orthoformate, or a mixture of two or more of these. As reducing agent sodium cyano borohydride or sodium triacetoxy borohydride may be used. The reaction is performed between 20˚C and 120˚C, preferably at room temperature.

When the reductive alkylation is complete, the product is isolated by extraction, filtration, chromatography or other methods known to those skilled in the art.

The general procedure (H) is further illustrated in the following example 647:

Example 647 (General procedure (H))

Biphenyl-4-ylmethyl-[3-(2*H*-tetrazol-5-yl)phenyl]amine

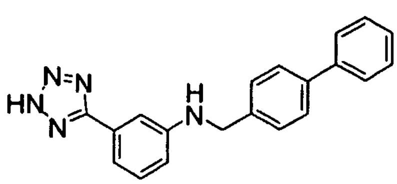

A solution of 5-(3-aminophenyl)-2*H*-tetrazole (example 874, 48 mg, 0.3 mmol) in DMF (250 μL) was mixed with a solution of 4-biphenylylcarbaldehyde (54 mg, 0.3 mmol) in DMF (250 μL) and acetic acid glacial (250 μL) was added to the mixture followed by a solution of sodium cyano borohydride (15 mg, 0.24 mmol) in methanol (250 μL). The resulting mixture was shaken at room temperature for 2 hours. Water (2 mL) was added to the mixture and the resulting mixture was shaken at room temperature for 16 hours. The mixture was centrifugated (6000 rpm, 10 minutes) and the supernatant was removed by a pipette. The residue was washed with water (3 mL), centrifugated (6000 rpm, 10 minutes) and the supernatant was removed by a pipette. The residue was dried *in vacuo* at 40 ˚C for 16 hours to afford the title

compound as a solid.
HPLC-MS (Method C): m/z: 328 (M+1), 350 (M+23); Rt = 4.09 min.

Example 648 (General procedure (H))

Benzyl-[3-(2*H*-tetrazol-5-yl)phenyl]amine

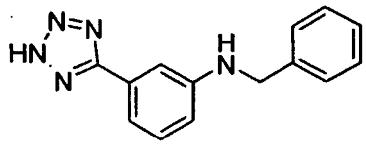

HPLC-MS (Method D): m/z: 252 (M+1); Rt = 3,74 min.

Example 649 (General procedure (H))

(4-Methoxybenzyl)-[3-(2*H*-tetrazol-5-yl)phenyl]amine

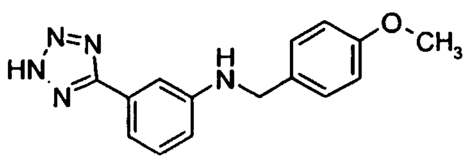

HPLC-MS (Method D): m/z: 282,2 (M+1); Rt = 3,57min.

Example 650 (General procedure (H))

4-{[3-(2*H*-Tetrazol-5-yl)phenylamino]methyl}phenol

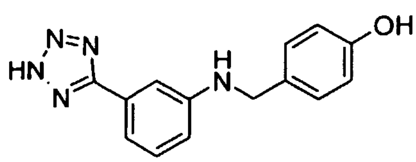

HPLC-MS (Method D): m/z: 268,4 (M+1); Rt = 2,64 min.

Example 651 (General procedure (H))

(4-Nitrobenzyl)-[3-(2*H*-tetrazol-5-yl)phenyl]amine

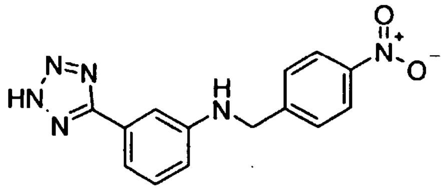

HPLC-MS (Method D): m/z: 297,4 (M+1); Rt = 3,94 min.

Example 652 (General procedure (H))

(4-Chlorobenzyl)-[3-(2*H*-tetrazol-5-yl)phenyl]amine

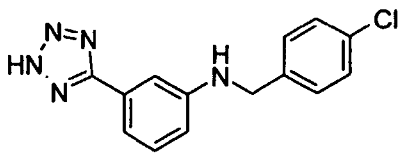

HPLC-MS (Method D): m/z: 287,2 (M+1); Rt = 4,30 min.

Example 653 (General procedure (H))

(2-Chlorobenzyl)-[3-(2*H*-tetrazol-5-yl)phenyl]amine

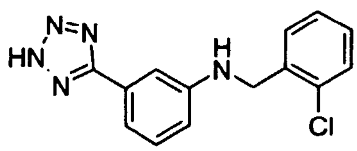

HPLC-MS (Method D): m/z: 286 (M+1); Rt = 4,40 min.

Example 654 (General procedure (H))

(4-Bromobenzyl)-[3-(2*H* tetrazol-5-yl)phenyl]amine

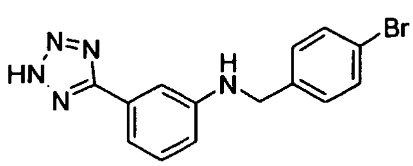

HPLC-MS (Method D): m/z:332 (M+1); Rt = 4,50 min.

Example 655 (General procedure (H))

(3-Benzyloxybenzyl)-[3-(2*H*-tetrazol-5-yl)phenyl]amine

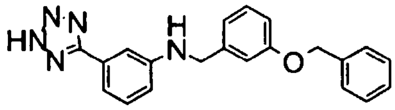

HPLC-MS (Method D): m/z: 358 (M+1); Rt = 4,94 min.

Example 656 (General procedure (H))

Naphthalen-1-ylmethyl-[3-(2*H*-tetrazol-5-yl)phenyl]amine

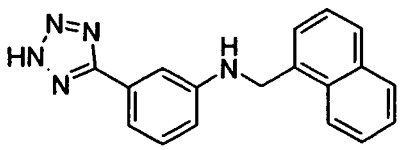

HPLC-MS (Method D): m/z: 302 (M+1); Rt = 4,70 min.

Example 657 (General procedure (H))

Naphthalen-2-ylmethyl-[3-(2*H*-tetrazol-5-yl)phenyl]amine

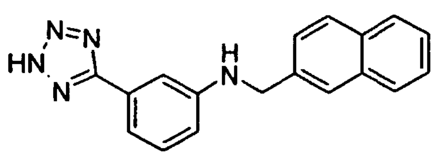

HPLC-MS (Method D): m/z: 302 (M+1); Rt = 4,60 min.

Example 658 (General procedure (H))

4-{[3-(2*H*-Tetrazol-5-yl)phenylamino]methyl}benzoic acid

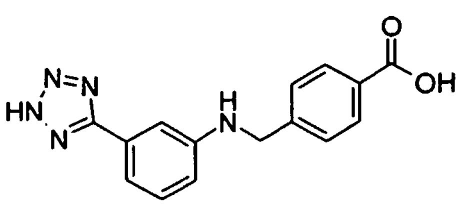

HPLC-MS (Method D): m/z: 296 (M+1); Rt = 3,24 min.

Example 659 (General procedure (H))

[3-(2*H*-Tetrazol-5-yl)-phenyl]-[3-(3-trifluoromethyl-phenoxy)benzyl]amine

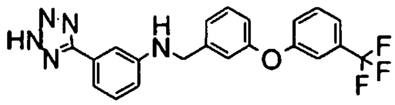

HPLC-MS (Method D): m/z: 412 (M+1); Rt = 5,54 min.

Example 660 (General procedure (H))

(3-Phenoxybenzyl)-[3-(2*H*-tetrazol-5-yl)phenyl]amine

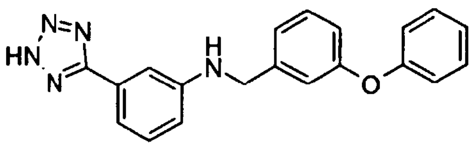

HPLC-MS (Method D): m/z: 344 (M+1); Rt = 5,04 min.

Example 661 (General procedure (H))

(4-Phenoxy-benzyl)-[3-(2*H*-tetrazol-5-yl)phenyl]amine

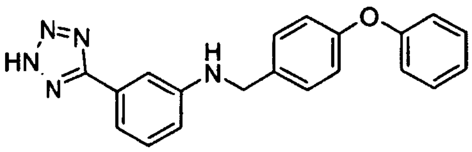

HPLC-MS (Method D): m/z: 344 (M+1); Rt = 5,00 min.

Example 662 (General procedure (H))

(4-{[3-(2*H*-Tetrazol-5-yl)phenylamino]methyl}phenoxy)acetic acid

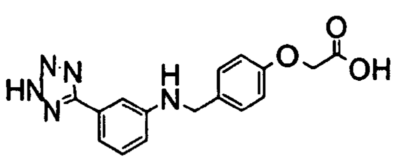

HPLC-MS (Method D): m/z: 326 (M+1); Rt = 3,10 min.

Example 663 (General procedure (H))

(4-Benzyloxybenzyl)-[3-(2*H*-tetrazol-5-yl)phenyl]amine

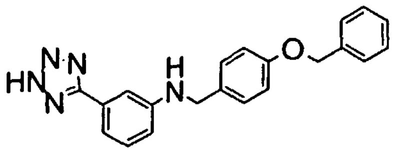

HPLC-MS (Method D): m/z: 358 (M+1); Rt = 4,97 min.

Example 664 (General procedure (H))

3-(4-{[3-(2*H*-Tetrazol-5-yl)phenylamino]methyl}phenyl)acrylic acid

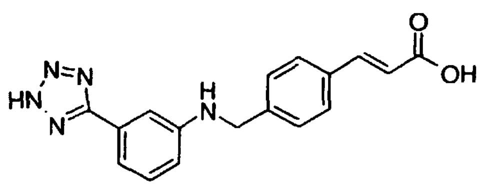

HPLC-MS (Method D): m/z: 322 (M+1); Rt = 3,60 min.

Example 665 (General procedure (H))

Dimethyl-(4-{[3-(2*H*-tetrazol-5-yl)phenylamino]methyl}naphthalen-1-yl)amine

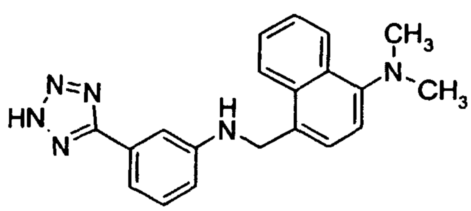

HPLC-MS (Method D): m/z: 345 (M+1); Rt = 3,07 min.

Example 666 (General procedure (H))

(4'-Methoxybiphenyl-4-ylmethyl)-[3-(2*H*-tetrazol-5-yl)phenyl]amine

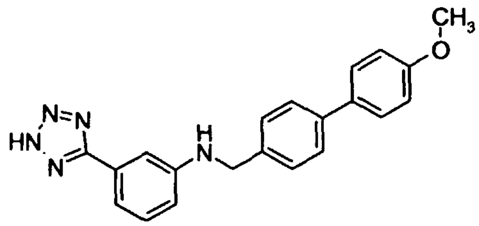

HPLC-MS (Method D): m/z: 358 (M+1); Rt = 4,97 min.

Example 667 (General procedure (H))

(2'-Chlorobiphenyl-4-ylmethyl)-[3-(2*H*-tetrazol-5-yl)phenyl]amine

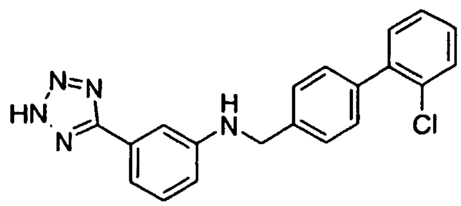

HPLC-MS (Method D): m/z: 362 (M+1); Rt = 5,27 min.

Example 668 (General procedure (H))

Benzyl-[4-(2*H*-tetrazol-5-yl)phenyl]amine

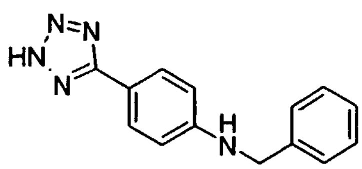

For preparation of starting material, see example 875.
HPLC-MS (Method D): m/z: 252 (M+1); Rt = 3,97 min.

Example 669 (General procedure (H))

(4-Methoxybenzyl)-[4-(2*H*-tetrazol-5-yl)phenyl]amine

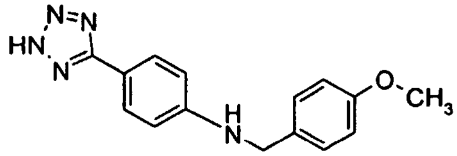

HPLC-MS (Method D): m/z: 282 (M+1); Rt = 3,94 min.

Example 670 (General procedure (H))

4-{[4-(2*H*-Tetrazol-5-yl)phenylamino]methyl}phenol

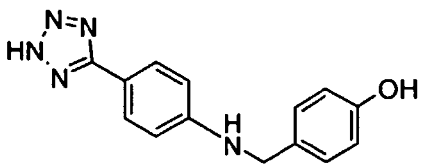

HPLC-MS (Method D): m/z: 268 (M+1); Rt = 3,14 min.

Example 671 (General procedure (H))

(4-Nitrobenzyl)-[4-(2*H*-tetrazol-5-yl)phenyl]amine

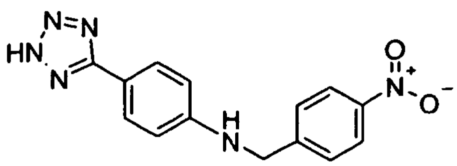

HPLC-MS (Method D): m/z: (M+1); Rt = 3,94 min.

Example 672 (General procedure (H))

(4-Chlorobenzyl)-[4-(2*H*-tetrazol-5-yl)phenyl]amine

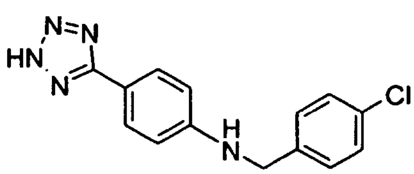

HPLC-MS (Method D): m/z: (M+1); Rt = 4,47 min.

Example 673 (General procedure (H))

(2-Chlorobenzyl)-[4-(2*H*-tetrazol-5-yl)phenyl]amine

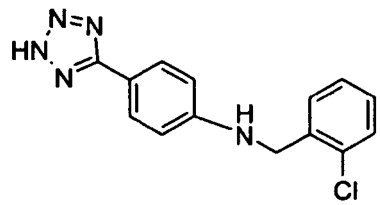

HPLC-MS (Method D): m/z: 286 (M+1); Rt = 4,37 min.

Example 674 (General procedure (H))

(4-Bromobenzyl)-[4-(2*H*-tetrazol-5-yl)phenyl]amine

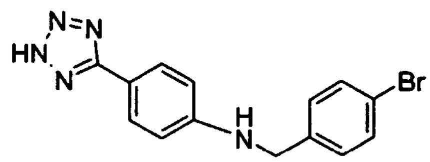

HPLC-MS (Method D): m/z: 331 (M+1); Rt = 4,57 min.

Example 675 (General procedure (H))

(3-Benzyloxybenzyl)-[4-(2*H*-tetrazol-5-yl)phenyl]amine

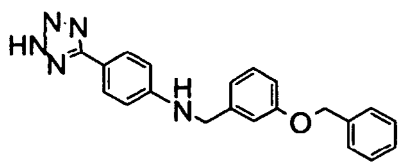

HPLC-MS (Method D): m/z: 358 (M+1); Rt = 5,07min.

Example 676 (General procedure (H))

Naphthalen-1-ylmethyl-[4-(2*H*-tetrazol-5-yl)phenyl]amine

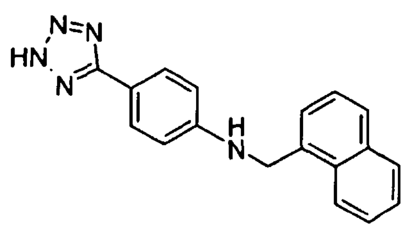

HPLC-MS (Method D): m/z: 302 (M+1); Rt = 4,70 min.

Example 677 (General procedure (H))

Naphthalen-2-ylmethyl-[4-(2*H*-tetrazol-5-yl)phenyl]amine

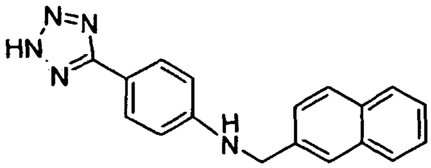

HPLC-MS (Method D): m/z: 302 (M+1); Rt = 4,70 min.

Example 678 (General procedure (H))

Biphenyl-4-ylmethyl-[4-(2*H*-tetrazol-5-yl)phenyl]amine

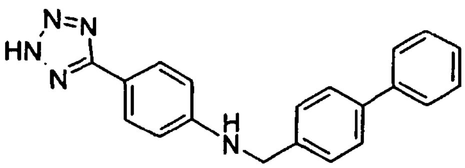

HPLC-MS (Method D): m/z: 328 (M+1); Rt = 5,07 min.

Example 679 (General procedure (H))

4-{[4-(2*H*-Tetrazol-5-yl)phenylamino]methyl}benzoic acid

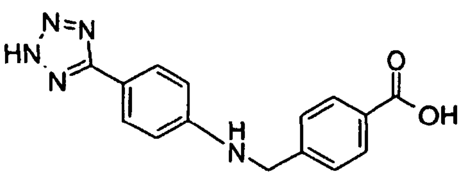

HPLC-MS (Method D): m/z: 296 (M+1); Rt = 3,34 min.

Example 680 (General procedure (H))

[4-(2*H*-Tetrazol-5-yl)phenyl]-[3-(3-trifluoromethylphenoxy)benzyl]amine

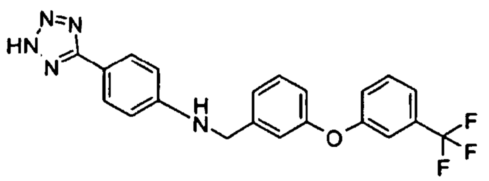

HPLC-MS (Method D): m/z: 412 (M+1); Rt = 5,54 min.

Example 681 (General procedure (H))

(3-Phenoxybenzyl)-[4-(2*H*-tetrazol-5-yl)phenyl]amine

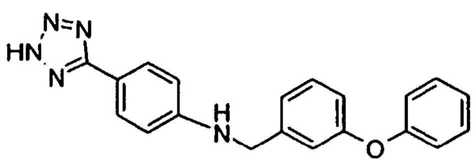

HPLC-MS (Method D): m/z: 344 (M+1); Rt = 5,07 min.

Example 682 (General procedure (H))

(4-Phenoxybenzyl)-[4-(2*H*-tetrazol-5-yl)-phenyl]-amine

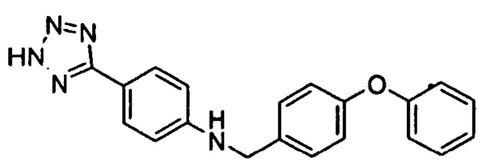

HPLC-MS (Method D): m/z: 344 (M+1); Rt = 5,03 min.

Example 683 (General procedure (H))

3-{[4-(2*H*-Tetrazol-5-yl)phenylamino]methyl}benzoic acid

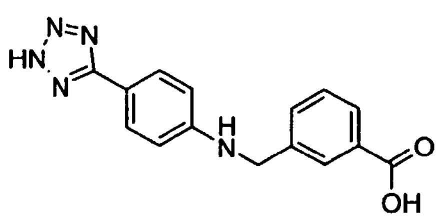

HPLC-MS (Method D): m/z: 286 (M+1); Rt = 3,47 min.

Example 684 (General procedure (H))

(4-{[4-(2*H*-Tetrazol-5-yl)phenylamino]methyl}phenoxy)acetic acid

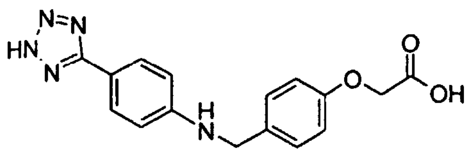

HPLC-MS (Method D): m/z: 326 (M+1); Rt = 3,40 min.

Example 685 (General procedure (H))

(4-Benzyloxybenzyl)-[4-(2*H*-tetrazol-5-yl)phenyl]amine

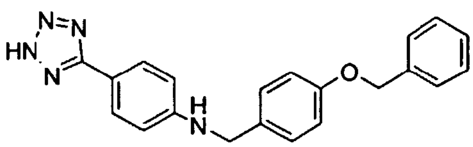

HPLC-MS (Method D): m/z: 358 (M+1); Rt = 5,14 min.

Example 686 (General procedure (H))

3-(4-{[4-(2*H*-Tetrazol-5-yl)phenylamino]methyl}phenyl)acrylic acid

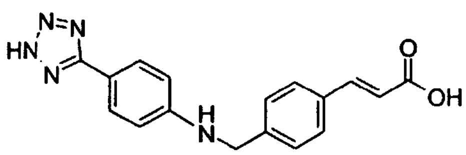

HPLC-MS (Method D): m/z: 322 (M+1); Rt = 3,66 min.

Example 687 (General procedure (H))

Dimethyl-(4-{[4-(2*H*-tetrazol-5-yl)phenylamino]methyl}naphthalen-1-yl)amine

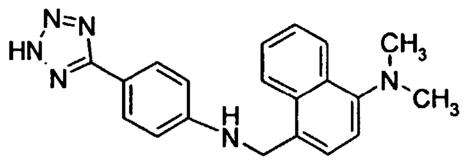

HPLC-MS (Method D): m/z: 345 (M+1); Rt = 3,10 min.

Example 688 (General procedure (H))

(4'-Methoxybiphenyl-4-ylmethyl)-[4-(2H-tetrazol-5-yl)phenyl]amine

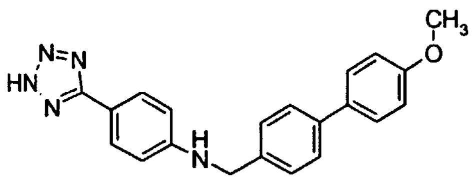

HPLC-MS (Method D): m/z: 358 (M+1); Rt = 5,04 min.

Example 689 (General procedure (H))

(2'-Chlorobiphenyl-4-ylmethyl)-[4-(2*H*-tetrazol-5-yl)-phenyl]-amine

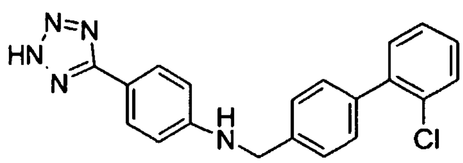

HPLC-MS (Method D): m/z: 362 (M+1); Rt = 5,30 min.

**General procedure (I) for preparation of compounds of general formula $I_8$:**

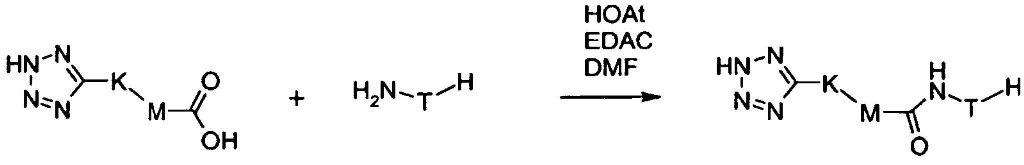


$I_8$

wherein K, M and T are as defined above.

This procedure is very similar to general procedure (A), the only difference being the carboxylic acid is containing a tetrazole moiety. When the acylation is complete, the product is isolated by extraction, filtration, chromatography or other methods known to those skilled in the art.

The general procedure (I) is further illustrated in the following example 690:

Example 690 (General procedure (I))

4-[4-(2*H*-Tetrazol-5-yl)benzoylamino]benzoic acid

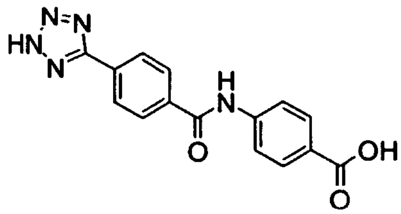

To a solution of 4-(2H-tetrazol-5-yl)benzoic acid (example 605, 4 mmol) and HOAt (4.2 mmol) in DMF (6 mL) was added 1-ethyl-3-(3'-dimethylaminopropyl)carbodiimide hydrochloride (4.2 mmol) and the resulting mixture was stirred at room temperature for 1 hour. An alquot of this HOAt-ester solution (0.45 mL) was mixed with 0.25 mL of a solution of 4-aminobenzoic acid (1.2 mmol in 1 mL DMF). (Anilines as hydrochlorides can also be utilised, a slight excess of triethylamine was added to the hydrochloride suspension in DMF prior to mixing with the HOAt-ester.) The resulting mixture was shaken for 3 days at room temperature. 1N hydrochloric acid (2 mL) was added and the mixture was shaken for 16 hours at room temperature. The solid was isolated by centrifugation (alternatively by filtration or extraction) and was washed with water (3 mL). Drying *in vacuo* at 40 °C for 2 days afforded the title compound.
HPLC-MS (Method D): m/z: 310 (M+1); Rt = 2.83 min.

Example 691 (General procedure (I))

3-[4-(2*H*-Tetrazol-5-yl)benzoylamino]benzoic acid

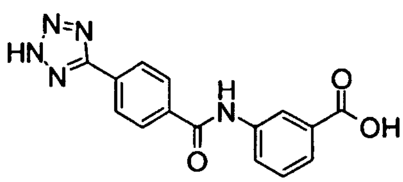

HPLC-MS (Method D): m/z: 310 (M+1); Rt = 2.89 min.

Example 692 (General procedure (I))

3-{4-[4-(2*H*-Tetrazol-5-yl)benzoylamino]phenyl}acrylic acid

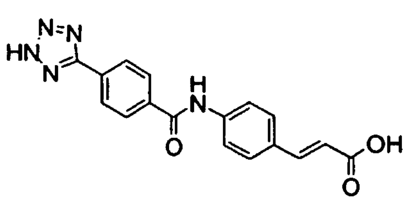

HPLC-MS (Method D): m/z: 336 (M+1); Rt = 3.10 min.

Example 693 (General procedure (I))

3-{4-[4-(2*H*-Tetrazol-5-yl)benzoylamino]phenyl}propionic acid

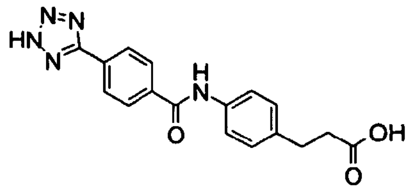

HPLC-MS (Method D): m/z: 338 (M+1); Rt = 2.97 min.

Example 694 (General procedure (I))

3-Methoxy-4-[4-(2*H*-tetrazol-5-yl)benzoylamino]benzoic acid

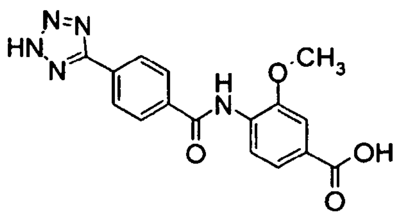

HPLC-MS (Method D): m/z: 340 (M+1); Rt = 3.03 min.

Example 695 (General procedure (I))

*N*-(4-Benzyloxyphenyl)-4-(2*H*-tetrazol-5-yl)benzamide

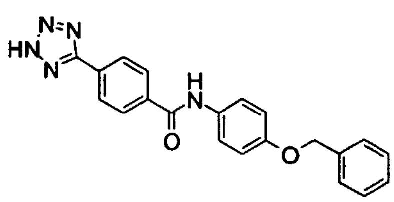

HPLC-MS (Method D): m/z: 372 (M+1); Rt = 4.47 min.

Example 696 (General procedure (I))

*N*-(4-Phenoxyphenyl)-4-(2*H*-tetrazol-5-yl)benzamide

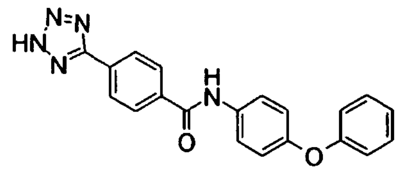

HPLC-MS (Method D): m/z: 358 (M+1); Rt = 4.50 min.

Example 697 (General procedure (I))

*N*-(9*H*-Fluoren-2-yl)-4-(2*H*-tetrazol-5-yl)benzamide

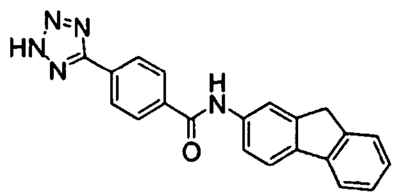

HPLC-MS (Method D): m/z: 354 (M+1); Rt = 4.60 min.

Example 698 (General procedure (I))

*N*-(9-Ethyl-9*H*-carbazol-2-yl)-4-(2*H*-tetrazol-5-yl)benzamide

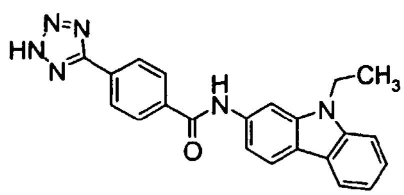

HPLC-MS (Method D): m/z: 383 (M+1); Rt = 4.60 min.

Example 699 (General procedure (I))

*N*-Phenyl-4-(2*H*-tetrazol-5-yl)benzamide

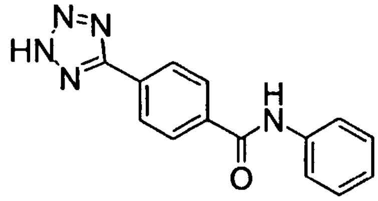

HPLC-MS (Method D): m/z: 266 (M+1); Rt = 3.23 min.

Example 700 (General procedure (I))

4-[4-(2*H*-Tetrazol-5-ylmethoxy)benzoylamino]benzoic acid

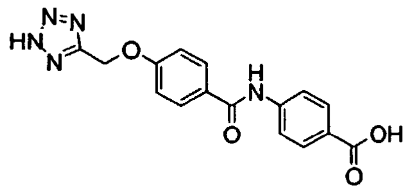

The starting material was prepared as described in example 592. HPLC-MS (Method D): m/z: 340 (M+1); Rt = 2.83 min.

Example 701 (General procedure (I))

3-[4-(2*H*-Tetrazol-5-ylmethoxy)benzoylamino]benzoic acid

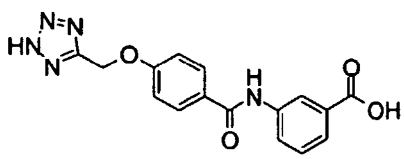

HPLC-MS (Method D): m/z: 340 (M+1); Rt = 2.90 min.

Example 702 (General procedure (I))

3-{4-[4-(2*H*-Tetrazol-5-ylmethoxy)benzoylamino]phenyl}acrylic acid

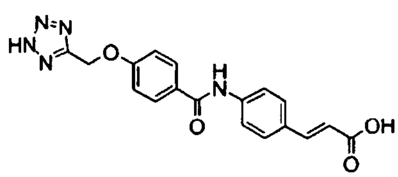

HPLC-MS (Method D): m/z: 366 (M+1); Rt = 3.07 min.

Example 703 (General procedure (I))

3-{4-[4-(2*H*-Tetrazol-5-ylmethoxy)benzoylamino]phenyl}propionic acid

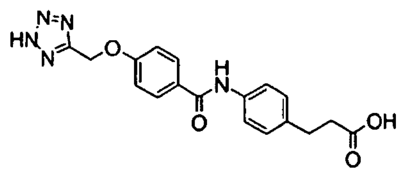

HPLC-MS (Method D): m/z: 368 (M+1); Rt = 2.97 min.

Example 704 (General procedure (I))

3-Methoxy-4-[4-(2*H*-tetrazol-5-ylmethoxy)benzoylamino]benzoic acid

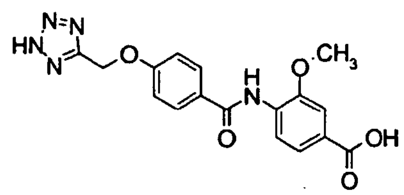

HPLC-MS (Method D): m/z: 370 (M+1); Rt = 3.07 min.

Example 705 (General procedure (I))

*N*-(4-Benzyloxyphenyl)-4-(2*H*-tetrazol-5-ylmethoxy)benzamide

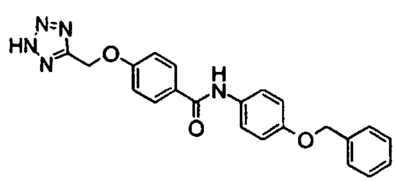

HPLC-MS (Method D): m/z: 402 (M+1); Rt = 4.43 min.

Example 706 (General procedure (I))

*N*-(4-Phenoxyphenyl)-4-(2*H*-tetrazol-5-ylmethoxy)benzamide

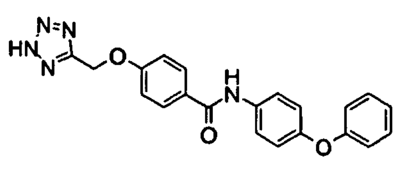

HPLC-MS (Method D): m/z: 388 (M+1); Rt = 4.50 min.

Example 707 (General procedure (I))

*N*-(9H-Fluoren-2-yl)-4-(2*H*-tetrazol-5-ylmethoxy)benzamide

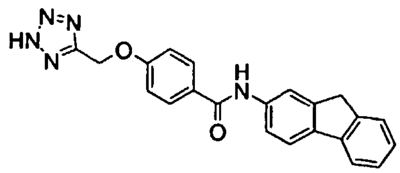

HPLC-MS (Method D): m/z: 384 (M+1); Rt = 4.57 min.

Example 708 (General procedure (I))

N-(9-Ethyl-9H-carbazol-2-yl)-4-(2*H*-tetrazol-5-ylmethoxy)benzamide

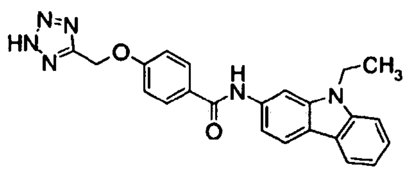

HPLC-MS (Method D): m/z: 413 (M+1); Rt = 4.57 min.

Example 709 (General procedure (I))

*N*-Phenyl-4-(2*H*-tetrazol-5-ylmethoxy)benzamide

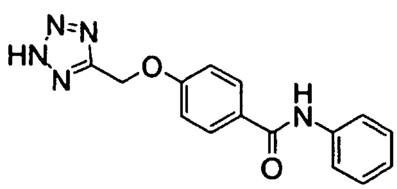

HPLC-MS (Method D): m/z: 296 (M+1); Rt = 3.23 min.

Example 710 (General procedure (I))

4-[4-(2*H*-Tetrazol-5-ylmethylsulfanyl)benzoylamino]benzoic acid

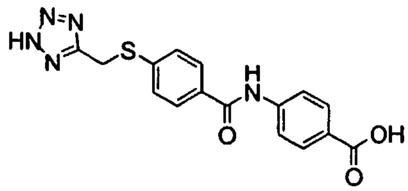

The starting material was prepared as described in example 593.
HPLC-MS (Method D): m/z: 356 (M+1); Rt = 2.93 min.

Example 711 (General procedure (I))

3-[4-(2*H*-Tetrazol-5-ylmethylsulfanyl)benzoylamino]benzoic acid

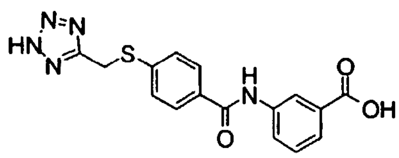

HPLC-MS (Method D): m/z: 356 (M+1); Rt = 3.00 min.

Example 712 (General procedure (I))

3-{4-[4-(2*H*-Tetrazol-5-ylmethylsulfanyl)benzoylamino]phenyl}acrylic acid

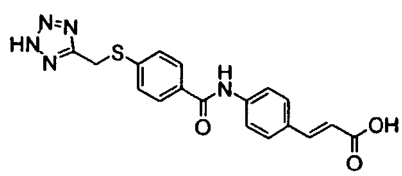

HPLC-MS (Method D): m/z: 382 (M+1); Rt = 3.26 min.

Example 713 (General procedure (I))

3-{4-[4-(2*H*-Tetrazol-5-ylmethylsulfanyl)benzoylamino]phenyl}propionic acid

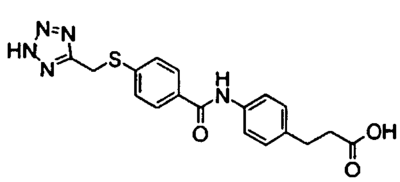

HPLC-MS (Method D): m/z: 384 (M+1); Rt = 3.10 min.

Example 714 (General procedure (I))

3-Methoxy-4-[4-(2*H*-tetrazol-5-ylmethylsulfanyl)benzoylamino]benzoic acid

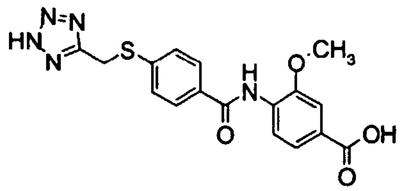

HPLC-MS (Method D): m/z: 386 (M+1); Rt = 3.20 min.

Example 715 (General procedure (I))

*N*-(4-Benzyloxyphenyl)-4-(2*H*-tetrazol-5-ylmethylsulfanyl)benzamide

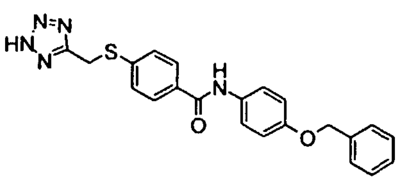

HPLC-MS (Method D): m/z: 418 (M+1); Rt = 4.57 min.

Example 716 (General procedure (I))

*N*-(4-Phenoxyphenyl)-4-(2*H*-tetrazol-5-ylmethylsulfanyl)benzamide

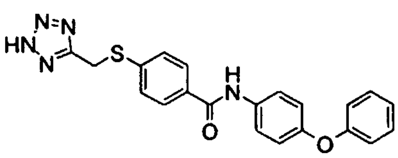

HPLC-MS (Method D): m/z: 404 (M+1); Rt = 4.60 min.

Example 717 (General procedure (I))

*N*-(9*H*-Fluoren-2-yl)-4-(2*H*-tetrazol-5-ylmethylsulfanyl)benzamide

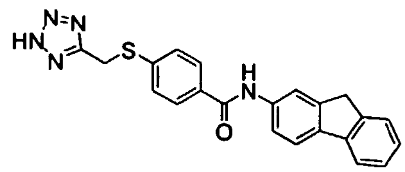

HPLC-MS (Method D): m/z: 400 (M+1); Rt = 4.67 min.

Example 718 (General procedure (I))

*N*-(9-Ethyl-9*H*-carbazol-2-yl)-4-(2*H*-tetrazol-5-ylmethylsulfanyl)benzamide

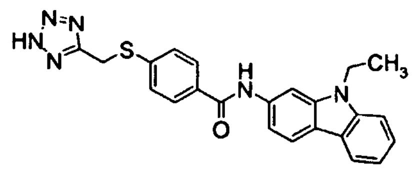

HPLC-MS (Method D): m/z: 429 (M+1); Rt = 4.67 min.

Example 719 (General procedure (I))

*N*-Phenyl-4-(2*H*-tetrazol-5-ylmethylsulfanyl)benzamide

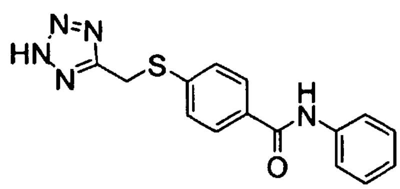

HPLC-MS (Method D): m/z: 312 (M+1); Rt = 3.40 min.

**General procedure (J) for solution phase preparation of amides of general formula $I_9$:**

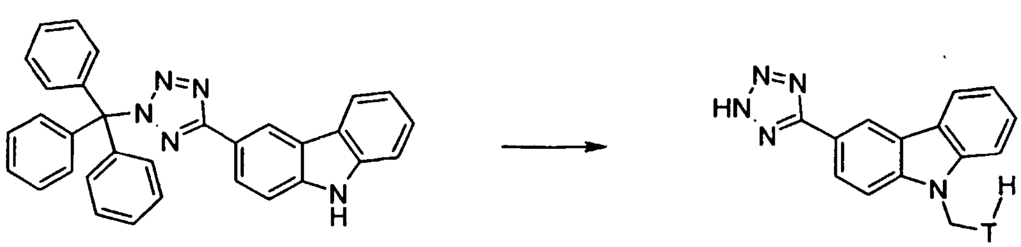

$I_9$

wherein T is as defined above.

This general procedure (J) is further illustrated in the following example.

Example 720 (General procedure (J)).

9-(3-Chlorobenzyl)-3-(2*H*-tetrazol-5-yl)-9*H*-carbazole

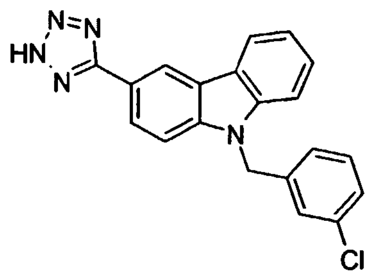

3-(2*H*-Tetrazol-5-yl)-9*H*-carbazole (example 594, 17 g, 72.26 mmol) was dissolved in *N*,*N*-dimethylformamide (150 mL). Triphenylmethyl chloride (21.153 g, 75.88 mmol) and triethylamine (20.14 mL, 14.62 g, 144.50 mmol) were added consecutively. The reaction mixture was stirred for 18 hours at room temperature, poured into water (1.5 L) and stirred for an additional 1 hour. The crude product was filtered off and dissolved in dichloromethane (500 mL). The organic phase was washed with water (2 x 250 mL) and dried with magnesium sulfate (1 h). Filtration followed by concentration yielded a solid which was triturated in heptanes (200 mL). Filtration furnished 3-[2-(triphenylmethyl)-2*H*-tetrazol-5-yl]-9*H*-carbazole (31.5 g) which was used without further purification.
$^1$H-NMR ($CDCl_3$): δ 8.87 (1H, d), 8.28 (1H, bs), 8.22 (1H, dd), 8.13 (1H, d), 7.49 (1H, d), 7.47-7.19 (18H, m); HPLC-MS (Method C): m/z: 243 (triphenylmethyl); Rt = 5.72 min.

3-[2-(Triphenylmethyl)-2*H*-tetrazol-5-yl]-9*H*-carbazole (200 mg, 0.42 mmol) was dissolved in methyl sulfoxide (1.5 mL). Sodium hydride (34 mg, 60 %, 0.85 mmol) was added, and the resulting suspension was stirred for 30 min at room temperature. 3-Chlorobenzyl chloride (85 μL, 108 mg, 0.67 mmol) was added, and the stirring was continued at 40°C for 18 hours. The reaction mixture was cooled to ambient temperature and poured into 0.1 N hydrochloric acid (aq.) (15 mL). The precipitated solid was filtered off and washed with water (3 x 10 mL) to furnish 9-(3-chlorobenzyl)-3-[2-(triphenylmethyl)-2*H*-tetrazol-5-yl]-9*H*-carbazole, which was dissolved in a mixture of tetrahydrofuran and 6 N hydrochloric acid (aq.) (9:1) (10 mL) and stirred at room temperature for 18 hours. The reaction mixture was poured into water (100 mL). The solid was filtered off and rinsed with water (3x10mL) and dichloromethane (3x10 mL) to yield the title compound (127 mg). No further purification was necessary.
$^1$H-NMR (DMSO-$d_6$): δ 8.89 (1H, d), 8.29 (1H, d), 8.12 (1H, dd), 7.90 (1H, d), 7.72 (1H, d), 7.53 (1H, t), 7.36-7.27 (4H, m), 7.08 (1H, bt), 5.78 (2H, s); HPLC-MS (Method B): m/z: 360 (M+1); Rt = 5.07 min.

The compounds in the following examples were prepared in a similar fashion. Optionally, the compounds can be further purified by recrystallization from e.g. aqueous sodium hydroxide (1 N) or by chromatography.

Example 721 (General Procedure (J)).

9-(4-Chlorobenzyl)-3-(2*H*-tetrazol-5-yl)-9*H*-carbazole

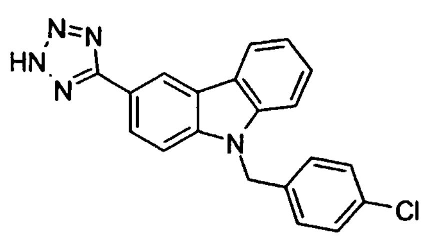

HPLC-MS (Method C): m/z: 360 (M+1); Rt = 4.31 min.

Example 722 (General Procedure (J)).

9-(4-Methylbenzyl)-3-(2*H*-tetrazol-5-yl)-9*H*-carbazole

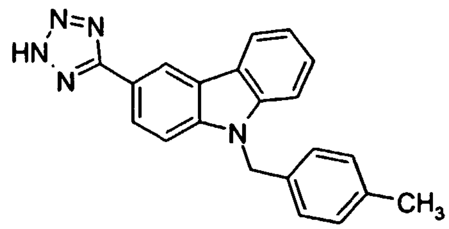

HPLC-MS (Method C): m/z: 340 (M+1); Rt = 4.26 min.

Example 723 (General Procedure (J)).

3-(2*H*-Tetrazol-5-yl)-9-(4-trifluoromethylbenzyl)-9*H*-carbazole

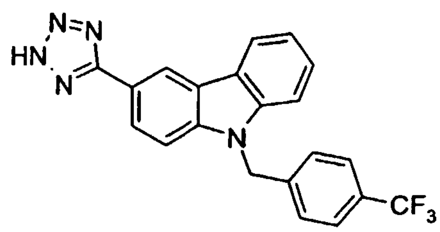

HPLC-MS (Method C): m/z: 394 (M+1); Rt = 4.40 min.

Example 724 (General Procedure (J)).

9-(4-Benzyloxybenzyl)-3-(2*H*-tetrazol-5-yl)-9*H*-carbazole

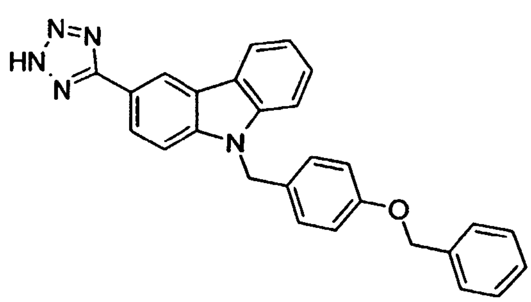

HPLC-MS (Method C): m/z: 432 (M+1); Rt = 4.70 min.

Example 725 (General Procedure (J)).

9-(3-Methylbenzyl)-3-(2*H*-tetrazol-5-yl)-9*H*-carbazole

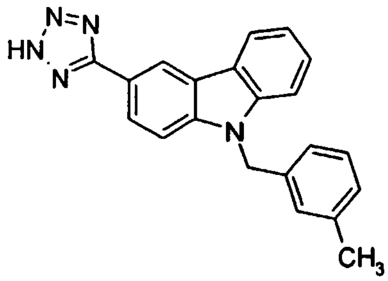

HPLC-MS (Method C): m/z: 340 (M+1); Rt = 4.25 min.

Example 726 (General Procedure (J)).

9-Benzyl-3-(2*H*-tetrazol-5-yl)-9*H*-carbazole

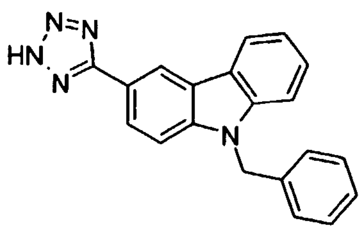

$^1$H-NMR (DMSO-$d_6$): δ 8.91 (1H, dd), 8.30 (1H, d), 8.13 (1H, dd), 7.90 (1H, d), 7.73 (1H, d), 7.53 (1H, t), 7.36-7.20 (6H, m), 5.77 (2H, s).

Example 727 (General Procedure (J)).

9-(4-Phenylbenzyl)-3-(2*H*-tetrazol-5-yl)-9*H*-carbazole

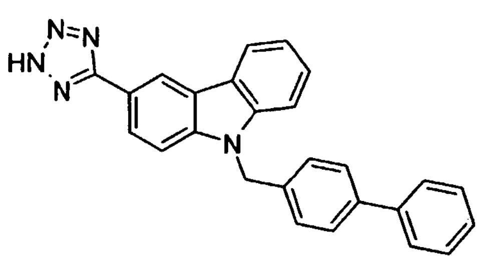

$^1$H-NMR (DMSO-$d_6$): δ 8.94 (1H, s), 8.33 (1H, d), 8.17 (1H, dd), 7.95 (1H, d), 7.77 (1H, d), 7.61-7.27 (11 H, m), 5.82 (2H, s).

Example 728 (General Procedure (J)).

9-(3-Methoxybenzyl)-3-(2*H*-tetrazol-5-yl)-9*H*-carbazole

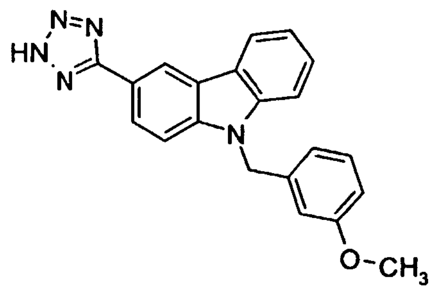

HPLC-MS (Method C): m/z: 356 (M+1); Rt = 3.99 min.

Example 729 (General Procedure (J)).

9-(Naphthalen-2-ylmethyl)-3-(2*H*-tetrazol-5-yl)-9*H*-carbazole

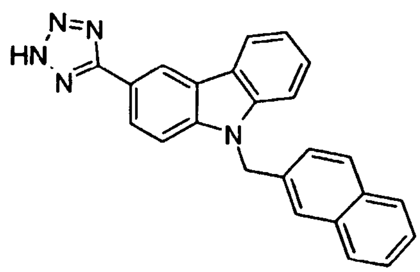

HPLC-MS (Method C): m/z: 376 (M+1); Rt = 4.48 min.

Example 730 (General Procedure (J)).

9-(3-Bromobenzyl)-3-(2*H*-tetrazol-5-yl)-9*H*-carbazole

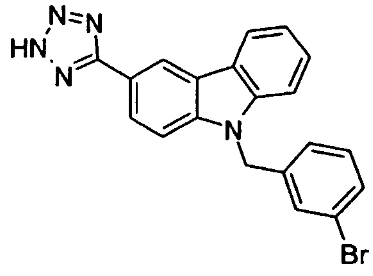

HPLC-MS (Method C): m/z: 404 (M+1); Rt = 4.33 min.

Example 731 (General Procedure (J)).

9-(Biphenyl-2-ylmethyl)-3-(2*H*-tetrazol-5-yl)-9*H*-carbazole

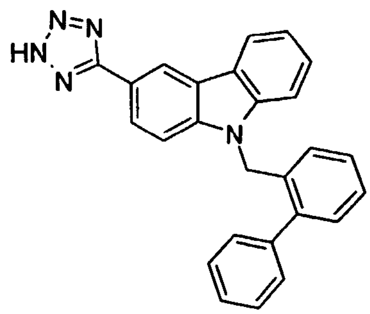

HPLC-MS (Method C): m/z: 402 (M+1); Rt = 4.80 min.

Example 732 (General Procedure (J)).

3-(2*H*-Tetrazol-5-yl)-9-[4-(1,2,3-thiadiazol-4-yl)benzyl]-9*H*-carbazole

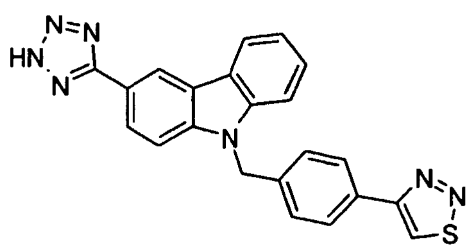

Example 733 (General Procedure (J)).

9-(2'-Cyanobiphenyl-4-ylmethyl)-3-(2*H* tetrazol-5-yl)-9*H*-carbazole

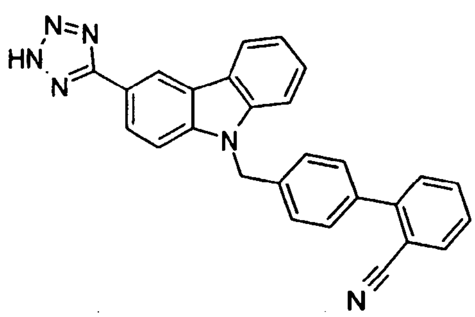

$^1$H-NMR (DMSO-$d_6$): δ 8.91 (1H, d), 8.31 (1H, d), 8.13 (1H, dd), 7.95 (1H, d), 7.92 (1H, d), 7.78 (1H, d), 7.75 (1H, dt), 7.60-7.47 (5H, m), 7.38-7.28 (3H, m), 5.86 (2H, s); HPLC-MS (Method C): m/z: 427 (M+1); Rt = 4.38 min.

Example 734 (General Procedure (J)).

9-(4-Iodobenzyl)-3-(2*H*-tetrazol-5-yl)-9*H* carbazole

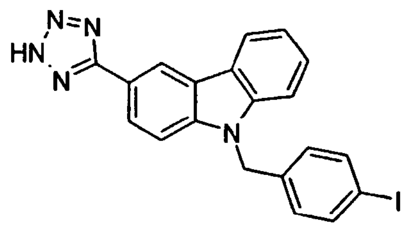

HPLC-MS (Method C): m/z: 452 (M+1); Rt = 4.37 min.

Example 735 (General Procedure (J)).

9-(3,5-Bis(trifluoromethyl)benzyl)-3-(2*H*-tetrazol-5-yl)-9*H*-carbazole

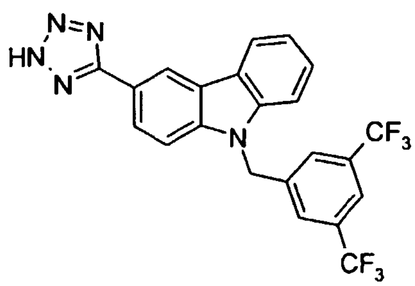

HPLC-MS (Method C): m/z: 462 (M+1); Rt = 4.70 min.

Example 736 (General Procedure (J)).

9-(4-Bromobenzyl)-3-(2*H*-tetrazol-5-yl)-9*H*-carbazole

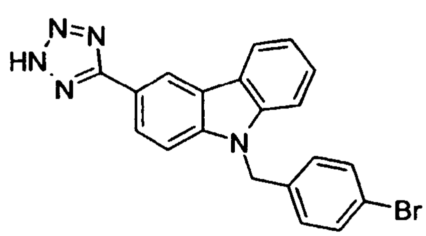

$^1$H-NMR (DMSO-$d_6$): δ 8.89 (1H, d), 8.29 (1H, d), 8.11 (1H, dd), 7.88 (1H, d), 7.70 (1H, d), 7.52 (1H, t), 7.49 (2H, d), 7.31 (1H, t), 7.14 (2H, d), 5.74 (2H, s); HPLC-MS (Method C): m/z: 404 (M+1); Rt = 4.40 min.

Example 737 (General Procedure (J)).

9-(Anthracen-9-ylmethyl)-3-(2*H*-tetrazol-5-yl)-9*H*-carbazole

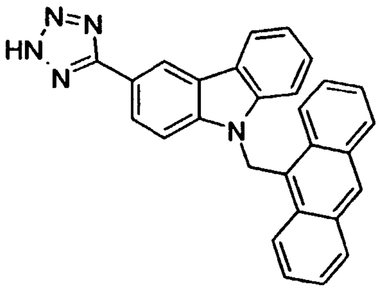

HPLC-MS (Method C): m/z: 426 (M+1); Rt = 4.78 min.

Example 738 (General Procedure (J)).

9-(4-Carboxybenzyl)-3-(2*H*-tetrazol-5-yl)-9*H*-carbazole

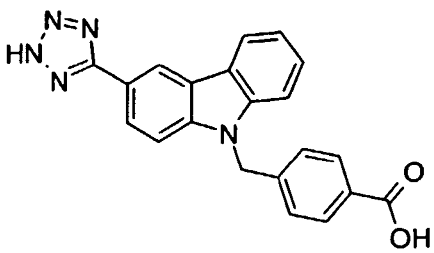

3.6 fold excess sodium hydride was used.
$^1$H-NMR (DMSO-$d_6$): δ 12.89 (1H, bs), 8.89 (1H, d), 8.30 (1H, d), 8.10 (1H, dd), 7.87 (1H, d), 7.86 (2H, d), 7.68 (1H, d), 7.51 (1H, t), 7.32 (1H, t), 7.27 (2H, d), 5.84 (2H, s); HPLC-MS (Method C): m/z: 370 (M+1); Rt = 3.37 min.

Example 739 (General Procedure (J)).

9-(2-Chlorobenzyl)-3-(2*H*-tetrazol-5-yl)-9*H*-carbazole

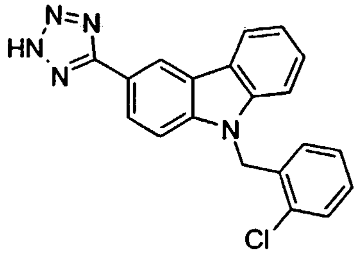

HPLC-MS (Method B): m/z: 360 (M+1); Rt = 5.30 min.

Example 740 (General Procedure (J)).

9-(4-Fluorobenzyl)-3-(2*H*-tetrazol-5-yl)-9*H*-carbazole

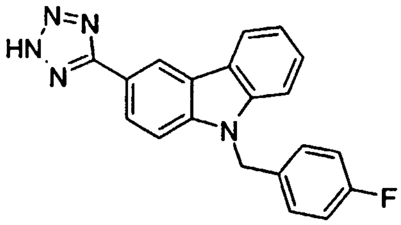

$^1$H-NMR (DMSO-$d_6$): δ 8.88 (1H, d), 8.28 (1H, d), 8.10 (1H, dd), 7.89 (1H, d), 7.72 (1H, d), 7.52 (1H, t), 7.31 (1H, t), 7.31-7.08 (4H, m), 5.74 (2H, s); HPLC-MS (Method C): m/z: 344 (M+1); Rt = 4.10 min.

Example 741 (General Procedure (J)).

9-(3-Fluorobenzyl)-3-(2*H*-tetrazol-5-yl)-9*H*-carbazole

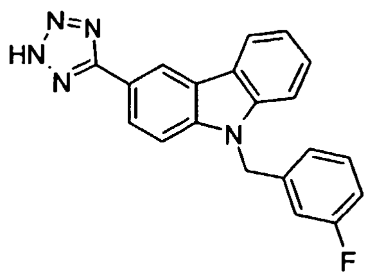

$^1$H-NMR (DMSO-$d_6$): δ8.89 (1H, d), 8.29 (1H, d), 8.12 (1H, dd), 7.90 (1H, d), 7.72 (1H, d), 7.53 (1H, t), 7.37-7.27 (2H, m), 7.12-7.02 (2H, m), 6.97 (1H, d), 5.78 (2H, s); HPLC-MS (Method C): m/z: 344 (M+1); Rt = 4.10 min.

Example 742 (General Procedure (J)).

9-(2-Iodobenzyl)-3-(2*H*-tetrazol-5-yl)-9*H*-carbazole

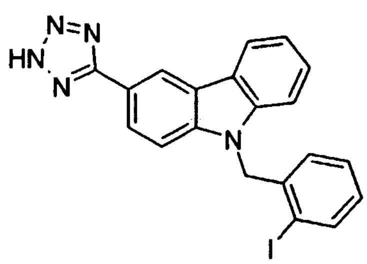

HPLC-MS (Method C): m/z: 452 (M+1); Rt = 4.58 min.

Example 743 (General Procedure (J)).

9-(3-Carboxybenzyl)-3-(2*H*-tetrazol-5-yl)-9*H*-carbazole

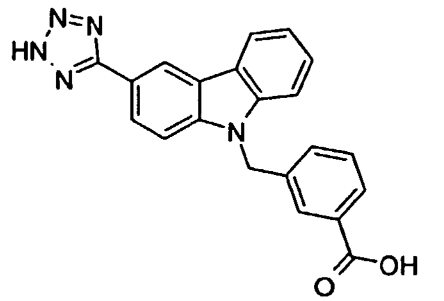

3.6 fold excess sodium hydride was used.
$^1$H-NMR (DMSO-$d_6$): δ 12.97 (1H, bs), 8.90 (1H, bs), 8.30 (1H, d), 8.12 (1H, bd), 7.89 (1H, d), 7.82 (1H, m), 7.77 (1H, bs), 7.71 (1H, d), 7.53 (1H, t), 7.46-7.41 (2H, m), 7.32 (1H, t), 5.84 (2H, s); HPLC-MS (Method C): m/z: 370 (M+1); Rt = 3.35 min.

Example 744 (General Procedure (J)).

9-[4-(2-Propyl)benzyl]-3-(2H-tetrazol-5-yl)-9H-carbazole

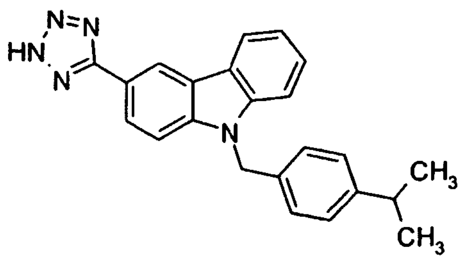

$^1$H-NMR (DMSO-$d_6$): δ 8.87 (1H, d), 8.27 (1H, d), 8.10 (1H, dd), 7.87 (1H, d), 7.71 (1H, d), 7.51 (1H, t), 7.31 (1H, t), 7.15 (2H, d), 7.12 (2H, d), 5.69 (2H, s), 2.80 (1H, sept), 1.12 (6H, d); HPLC-MS (Method C): m/z: 368 (M+1); Rt = 4.73 min.

Example 745 (General Procedure (J)).

9-(3,5-Dimethoxybenzyl)-3-(2*H*-tetrazol-5-yl)-9*H*-carbazole

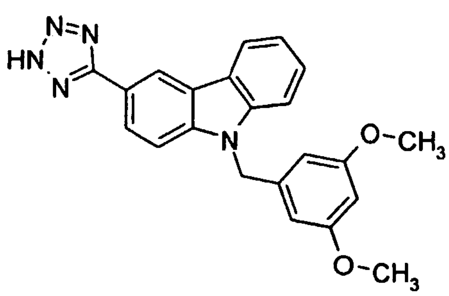

HPLC-MS (Method C): m/z: 386 (M+1); Rt = 4.03 min.

Example 746 (General Procedure (J)).

3-(2*H*-Tetrazol-5-yl)-9-(2,4,5-trifluorobenzyl)-9*H*-carbazole

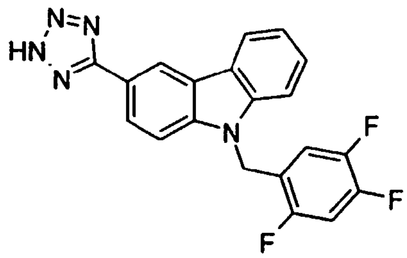

HPLC-MS (Method B): m/z: 380 (M+1); Rt = 5.00 min.

Example 747 (General Procedure (J)).

*N*-Methyl-*N*-phenyl-2-[3-(2*H*-tetrazol-5-yl)carbazol-9-yl]acetamide

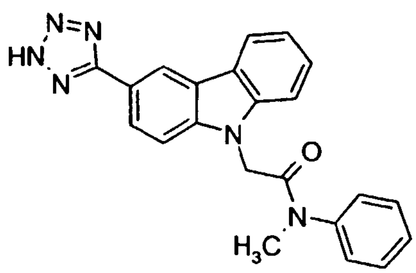

HPLC-MS (Method B): m/z: 383 (M+1); Rt = 4.30 min.

Example 748 (General Procedure (J)).

9-(4-Methoxybenzyl)-3-(2*H*-tetrazol-5-yl)-9*H*-carbazole

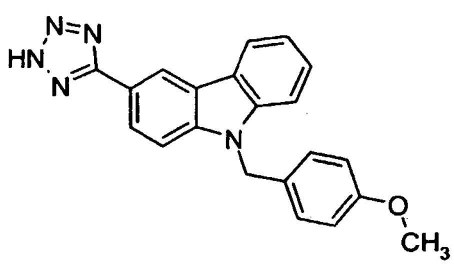

$^1$H-NMR (DMSO-$d_6$): δ 8.86 (1H, d), 8.26 (1H, d), 8.10 (1H, dd), 7.90 (1H, d), 7.73 (1H, d), 7.51 (1H, t), 7.30 (1H, t), 7.18 (2H, d), 6.84 (2H, d), 5.66 (2H, s), 3.67 (3H, s); HPLC-MS (Method B): m/z: 356 (M+1); Rt = 4.73 min.

Example 749 (General Procedure (J)).

9-(2-Methoxybenzyl)-3-(2*H*-tetrazol-5-yl)-9*H*-carbazole

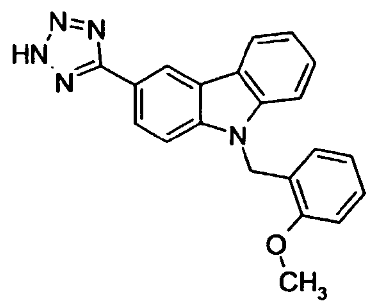

$^1$H-NMR (DMSO-$d_6$): δ 8.87 (1H, d), 8.27 (1H, d), 8.09 (1H, dd), 7.77 (1H, d), 7.60 (1H, d), 7.49 (1H, t), 7.29 (1H, t), 7.23 (1H, bt), 7.07 (1H, bd), 6.74 (1H, bt), 6.61 (1H, bd), 5.65 (2H, s), 3.88 (3H, s); HPLC-MS (Method B): m/z: 356 (M+1); Rt = 4.97 min.

Example 750 (General Procedure (J)).

9-(4-Cyanobenzyl)-3-(2*H*-tetrazol-5-yl)-9*H*-carbazole

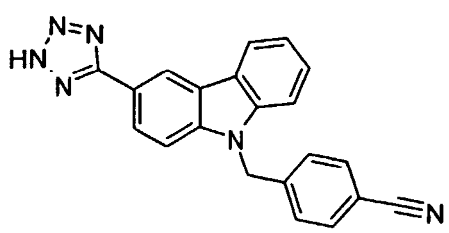

HPLC-MS (Method C): m/z: 351 (M+1); Rt = 3.74 min.

Example 751 (General Procedure (J)).

9-(3-Cyanobenzyl)-3-(2*H*-tetrazol-5-yl)-9*H*-carbazole

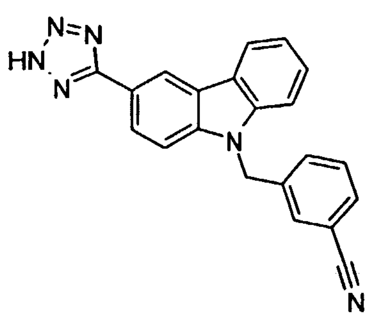

HPLC-MS (Method C): m/z: 351 (M+1); Rt = 3.73 min.

Example 752 (General Procedure (J)).

9-(5-Chloro-2-methoxybenzyl)-3-(2*H*-tetrazol-5-yl)-9*H*-carbazole

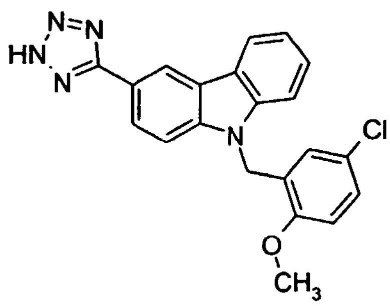

$^1$H-NMR (DMSO-$d_6$): δ 8.87 (1H, d), 8.35 (1H, d), 8.10 (1H, dd), 7.73 (1H, d), 7.59 (1H, d), 7.49 (1H, t), 7.29 (1H, t), 7.27 (1H, dd), 7.11 (1H, d), 6.51 (1H, d), 5.63 (2H, s), 3.88 (3H, s); HPLC-MS (Method C): m/z: 390 (M+1); Rt = 4.37 min.

Example 753 (General Procedure (J)).

*N*-Phenyl-2-[3-(2*H*-tetrazol-5-yl)carbazol-9-yl]acetamide

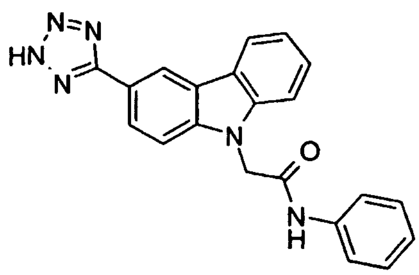

$^1$H-NMR (DMSO-$d_6$): δ 10.54 (1H, s), 8.87 (1H, bs), 8.27 (1H, d), 8.12 (1H, bd), 7.83 (1H, d), 7.66 (1H, d), 7.61 (2H, d), 7.53 (1H, t), 7.32 (1H, t), 7.32 (2H, t), 7.07 (1H, t), 5.36 (2H, s); HPLC-MS (Method C): m/z: 369 (M+1); Rt = 3.44 min.

Example 754 (General Procedure (J)).

*N*-Butyl-2-[3-(2*H*-tetrazol-5-yl)carbazol-9-yl]acetamide

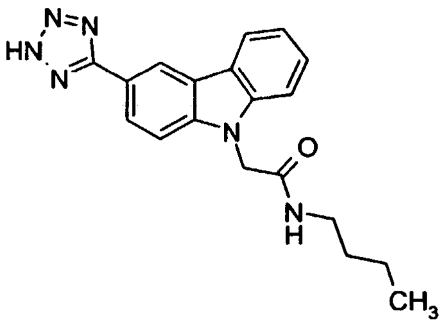

$^1$H-NMR (DMSO-$d_6$): δ 8.85 (1H, d), 8.31 (1H, t), 8.25 (1H, d), 8.10 (1H, dd), 7.75 (1H, d), 7.58 (1H, d), 7.52 (1H, t), 7.30 (1H, t), 5.09 (2H, s), 3.11 (2H, q), 1.42 (2H, quint), 1.30 (2H, sext), 0.87 (3H, t); HPLC-MS (Method C): m/z: 349 (M+1); Rt = 3.20 min.

Example 755 (General Procedure (J)).

9-(2,4-Dichlorobenzyl)-3-(2*H*-tetrazol-5-yl)-9*H*-carbazole

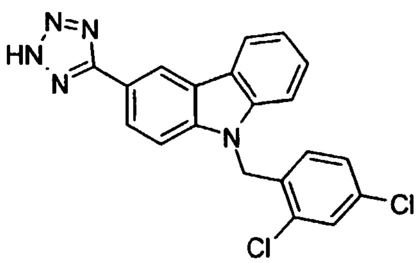

$^1$H-NMR (DMSO-$d_6$): δ 8.92 (1H, d), 8.32 (1H, d), 8.09 (1H, dd), 7.76 (1H, d), 7.74 (1H, d), 7.58 (1H, d), 7.51 (1H, t), 7.33 (1H, t), 7.23 (1H, dd), 6.42 (1H, d), 5.80 (2H, s); HPLC-MS (Method B): m/z: 394 (M+1); Rt = 5.87 min.

Example 756 (General Procedure (J)).

9-(2-Methylbenzyl)-3-(2*H*-tetrazol-5-yl)-9*H*-carbazole

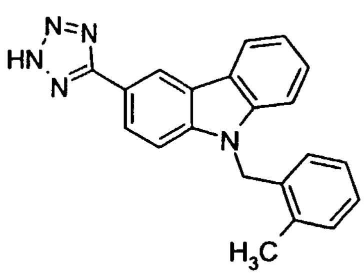

$^1$H-NMR (DMSO-$d_6$): δ 8.92 (1H, d), 8.32 (1H, d), 8.08 (1H, dd), 7.72 (1H, d), 7.55 (1H, d), 7.48 (1H, t), 7.32 (1H, t), 7.26 (1H, d), 7.12 (1H, t), 6.92 (1H, t), 6.17 (1H, d), 5.73 (2H, s), 2.46 (3H, s); HPLC-MS (Method B): m/z: 340 (M+1); Rt = 5.30 min.

Example 757 (General Procedure (J)).

9-(3-Nitrobenzyl)-3-(2*H*-tetrazol-5-yl)-9*H*-carbazole

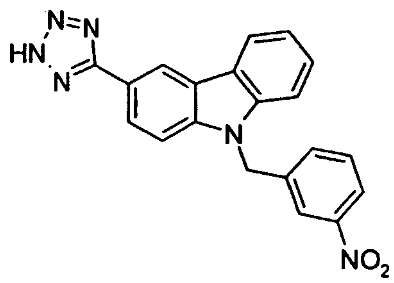

HPLC-MS (Method C): m/z: 371 (M+1); Rt = 3.78 min.

Example 758 (General Procedure (J)).

9-(3,4-Dichlorobenzyl)3-(2*H*-tetrazol-5-yl)9*H*-carbazole

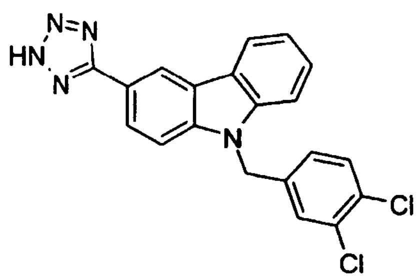

HPLC-MS (Method B): m/z: 394 (M+1); Rt = 5.62 min.

Example 759 (General Procedure (J)).

9-(2,4-Difluorobenzyl)-3-(2*H*-tetrazol-5-yl)-9*H*-carbazole

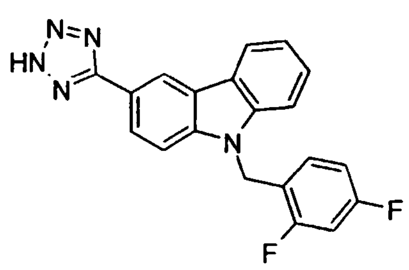

$^1$H-NMR (DMSO-$d_6$): δ 88.89 (1H, d), 8.29 (1H, d), 8.11 (1H, dd), 7.88 (1H, d), 7.69 (1H, d), 7.52 (1H, t), 7.36-7.24 (2H, m), 7.06-6.91 (2H, m), 5.78 (2H, s); HPLC-MS (Method B): m/z: 362 (M+1); Rt = 5.17 min.

Example 760 (General Procedure (J)).

9-(3,5-Difluorobenzyl)-3-(2*H*-tetrazol-5-yl)-9*H*-carbazole

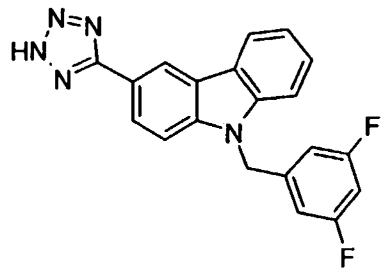

$^1$H-NMR (DMSO-$d_6$): δ 8.90 (1H, bs), 8.31 (1H, d), 8.13 (1H, bd), 7.90 (1H, d), 7.73 (1H, d), 7.54 (1H, t), 7.34 (1H, t), 7.14 (1H, t), 6.87 (2H, bd), 5.80 (2H, s); HPLC-MS (Method B): m/z: 362 (M+1); Rt = 5.17 min.

Example 761 (General Procedure (J)).

9-(3,4-Difluorobenzyl)-3-(2*H*-tetrazol-5-yl)-9*H*-carbazole

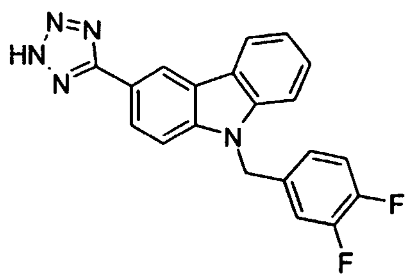

$^1$H-NMR (DMSO-$d_6$): δ 8.89 (1H, bs), 8.29 (1H, d), 8.12 (1H, bd), 7.92 (1H, d), 7.74 (1H, d), 7.54 (1H, t), 7.42-7.25 (3H, m), 6.97 (1H, bm), 5.75 (2H, s); HPLC-MS (Method B): m/z: 362 (M+1); Rt = 5.17 min.

Example 762 (General Procedure (J)).

9-(3-Iodobenzyl)-3-(2*H*-tetrazol-5-yl)-9*H*-carbazole

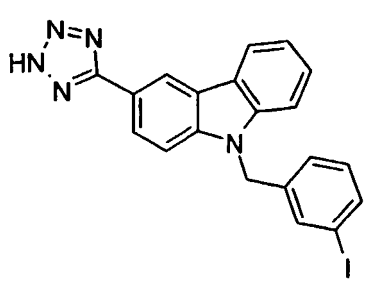

HPLC-MS (Method B): m/z: 452 (M+1); Rt = 5.50 min.

Example 763 (General Procedure (J)).

3-(2*H*-Tetrazol-5-yl)-9-[3-(trifluoromethyl)benzyl]-9*H*-carbazole

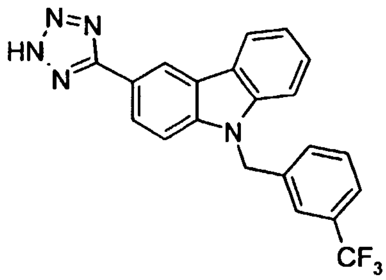

$^1$H-NMR (DMSO-$d_6$): δ 8.89 (1H, d), 8.30 (1H, d), 8.11 (1H, dd), 7.90 (1H, d), 7.72 (1H, d), 7.67 (1H, bs), 7.62 (1H, bd), 7.53 (1H, t), 7.50 (1H, bt), 7.33 (1H, bd), 7.32 (1H, t), 5.87 (2H, s); HPLC-MS (Method B): m/z: 394 (M+1); Rt = 5.40 min.

Example 764 (General Procedure (J)).

*N*-(4-Carboxyphenyl)-2-[3-(2*H*-tetrazol-5-yl)carbazol-9-yl]acetamide

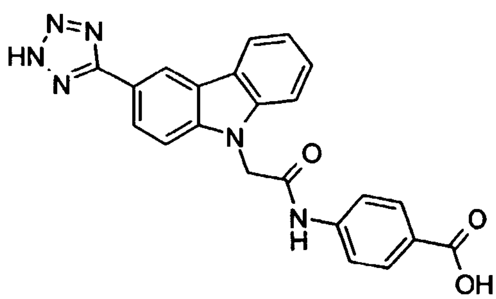

3.6 fold excess sodium hydride was used.
HPLC-MS (Method B): m/z: 413 (M+1); Rt = 3.92 min.

Example 765 (General Procedure (J)).

*N*-(2-Propyl)-2-[3-(2*H*-tetrazol-5-yl)carbazol-9-yl]acetamide

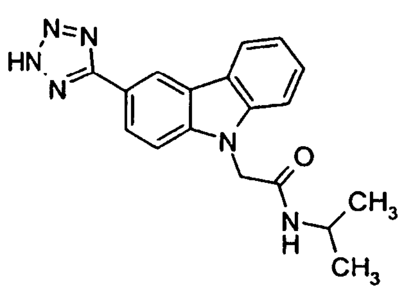

HPLC-MS (Method B): m/z: 335 (M+1); Rt = 3.70 min.

Example 766 (General Procedure (J)).

*N*-Benzyl-*N*-phenyl-2-[3-(2*H*-tetrazol-5-yl)carbazol-9-yl]acetamide

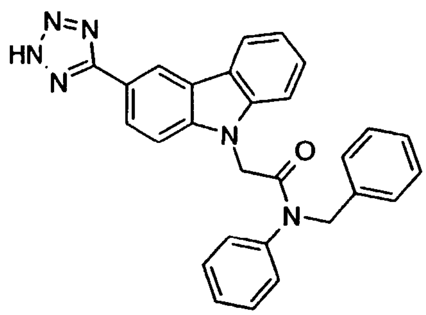

HPLC-MS (Method B): m/z: 459 (M+1); Rt = 5.37 min.

Example 767 (General Procedure (J)).

*N*-[4-(2-Methyl-2-propyl)phenyl]-2-[3-(2*H*-tetrazol-5-yl)carbazol-9-yl]acetamide

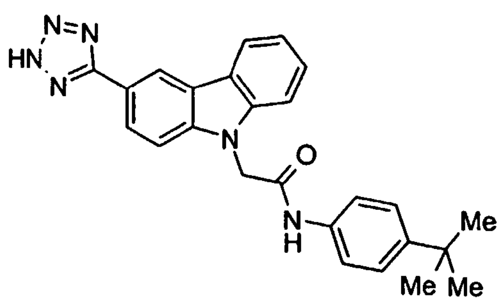

HPLC-MS (Method B): m/z: 425 (M+1); Rt = 5.35 min.

Example 768 (General Procedure (J)).

*N*-Phenethyl-2-[3-(2*H*-tetrazol-5-yl)carbazol-9-yl]acetamide

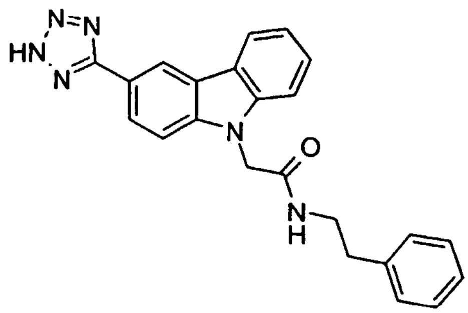

HPLC-MS (Method C): m/z: 397 (M+1); Rt = 3.43 min.

Example 769 (General Procedure (J)).

3-(2*H*-Tetrazol-5-yl)-9-[2-(trifluoromethyl)benzyl]-9*H*-carbazole

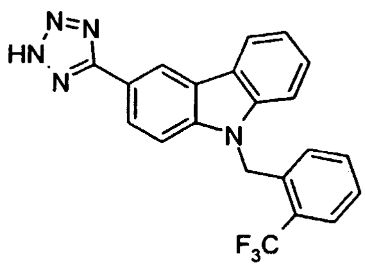

HPLC-MS (Method C): m/z: 394 (M+1); Rt = 4.44 min.

Example 770 (General Procedure (J)).

9-[2-Fluoro-6-(trifluoromethyl)benzyl]-3-(2*H*-tetrazol-5-yl)-9*H*-carbazole

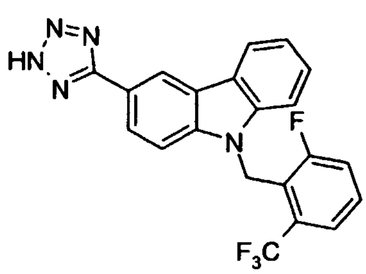

HPLC-MS (Method C): m/z: 412 (M+1); Rt = 4.21 min.

Example 771 (General Procedure (J)).

9-[2,4-Bis(trifluoromethyl)benzyl)]-3-(2*H*-tetrazol-5-yl)-9*H*-carbazole

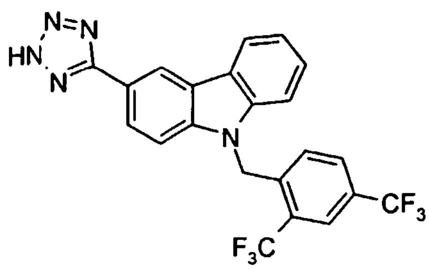

HPLC-MS (Method C): m/z: 462 (M+1); Rt = 4.82 min.

Example 772 (General Procedure (J)).

3-(2*H*-Tetrazol-5-yl)-9-(2,4,6-trimethylbenzyl)-9*H*-carbazole

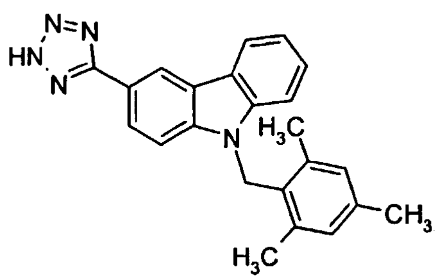

HPLC-MS (Method C): m/z: 368 (M+1); Rt = 4.59 min.

Example 773 (General Procedure (J)).

9-(2,3,5,6-Tetramethylbenzyl)-3-(2*H*-tetrazol-5-yl)-9*H*-carbazole

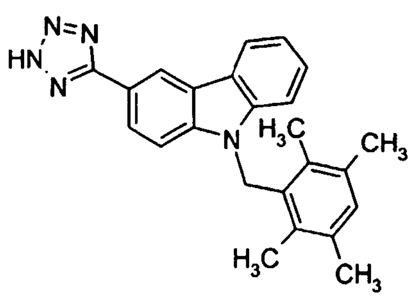

HPLC-MS (Method C): m/z: 382 (M+1); Rt = 4.47 min.

Example 774 (General Procedure (J)).

9-[(Naphthalen-1-yl)methyl]-3-(2*H*-tetrazol-5-yl)-9*H*-carbazole

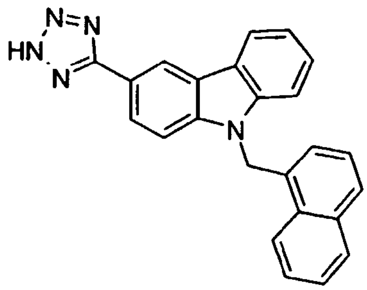

HPLC-MS (Method C): m/z: 376 (M+1); Rt = 4.43 min.

Example 775 (General Procedure (J)).

9-[Bis(4-fluorophenyl)methyl]-3-(2*H*-tetrazol-5-yl)-9*H*-carbazole

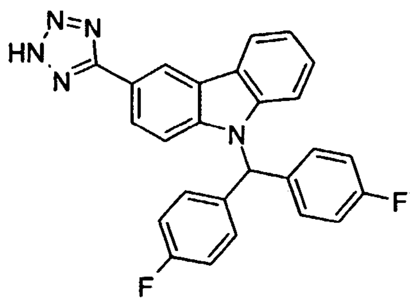

HPLC-MS (Method C): m/z: 438 (M+1); Rt = 4.60 min.

Example 776 (General Procedure (J)).

9-(2-Bromobenzyl)-3-(2*H*-tetrazol-5-yl)-9*H*-carbazole

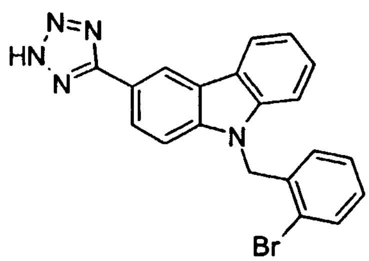

HPLC-MS (Method C): m/z: 404 (M+1); Rt = 4.50 min.

Example 777 (General Procedure (J)).

9-(2-Fluorobenzyl)-3-(2*H*-tetrazol-5-yl)-9*H*-carbazole

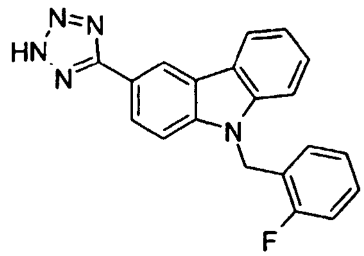

HPLC-MS (Method C): m/z: 344 (M+1); Rt = 4.09 min.

Example 778 (General Procedure (J)).

9-(4-Carboxy-2-methylbenzyl)-3-(2*H*-tetrazol-5-yl)-9*H*-carbazole

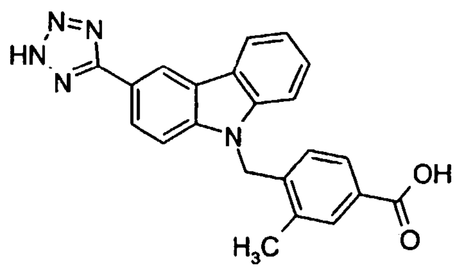

In this preparation, a 3.6-fold excess of sodium hydride was used.
HPLC-MS (Method C): m/z: 384 (M+1); Rt = 3.56 min.

Example 779 (General Procedure (J)).

9-(2-Phenylethyl)-3-(2*H*-tetrazol-5-yl)-9*H*-carbazole

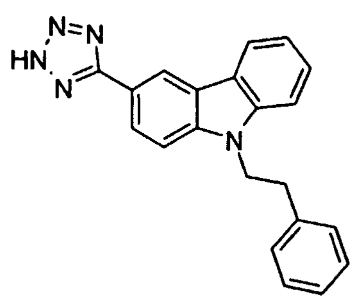

HPLC-MS (Method C): m/z: 340 (M+1); Rt = 4.08 min.

Example 780 (General Procedure (J)).

9-[2-Fluoro-5-(trifluoromethyl)benzyl]-3-(2*H*-tetrazol-5-yl)-9*H*-carbazole

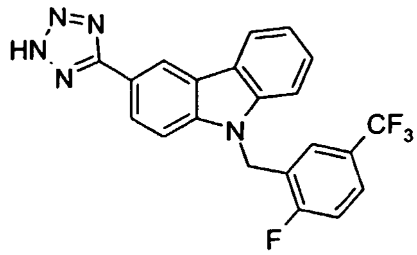

HPLC-MS (Method C): m/z: 412 (M+1); Rt = 4.34 min.

Example 781 (General Procedure (J)).

9-(4-Carboxy-2-fluorobenzyl)-3-(2*H* tetrazol-5-yl)-9*H*-carbazole

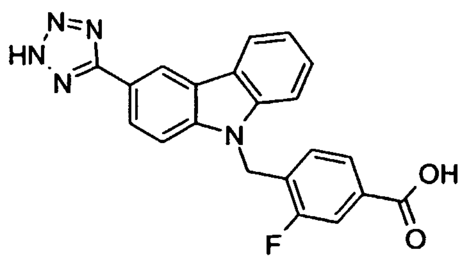

3-Fluoro-4-methylbenzoic acid (3.0 g, 19.5 mmol) and benzoyl peroxide (0.18 g, 0.74 mmol) were suspended in benzene. The mixture was purged with $N_2$ and heated to reflux. N-Bromosuccinimide (3.47 g, 19.5 mmol) was added portionwise, and reflux was maintained for 18 hours. The reaction mixture was concentrated, and the residue was washed with water (20 mL) at 70 ˚C for 1 hour. The crude product was isolated by filtration and washed with additional water (2 x 10 mL). The dry product was recrystallized from heptanes. Filtration furnished 4-bromomethyl-3-fluorobenzoic acid (1.92 g) which was used in the following step according to General Procedure (J).
In this preparation, a 3.6-fold excess of sodium hydride was used.
HPLC-MS (Method C): m/z: 388 (M+1); Rt = 3.49 min.

Example 782 (General Procedure (J)).

5-{4-[[(3-(2*H*-Tetrazol-5-yl)carbazol-9-yl)methyl]naphthalen-1-yl]oxy}pentanoic Acid

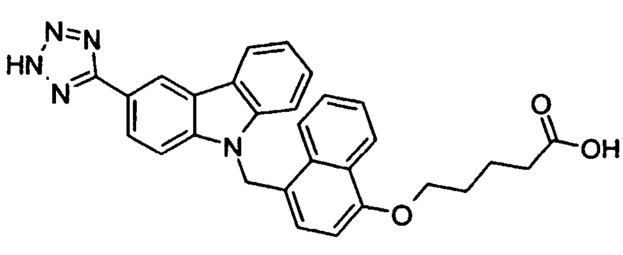

5-[(4-Formylnaphthalen-1-yl)oxy]pentanoic acid intermediate obtained in example 470(3.0 g, 11.0 mmol) was dissolved in a mixture of methanol and tetrahydrofuran (9:1) (100 mL), and sodium borohydride (1.67 g, 44.1 mmol) was added portionwise at ambient temperature. After 30 minutes, the reaction mixture was concentrated to 50 mL and added to hydrochloric acid (0.1 N, 500 mL). Additional hydrochloric acid (1 N, 40 mL) was added, and 5-[(4-hydroxymethyl-naphthalen-1-yl)oxy]pentanoic acid (2.90 g) was collected by filtration. To the crude product was added concentrated

hydrochloric acid (100 mL), and the suspension was stirred vigorously for 48 hours at room temperature. The crude product was filtered off and washed with water, until the pH was essentially neutral. The material was washed with heptanes to furnish 5-[(4-chloromethylnaphthalen-1-yl)oxy]pentanoic acid (3.0 g) which was used in the following step according to General Procedure (J).

In this preparation, a 3.6-fold excess of sodium hydride was used.
HPLC-MS (Method C): m/z: 492 (M+1); Rt = 4.27 min.

Example 783 (General procedure (J))

9-(2,3-Difluorobenzyl)-3-(2H-tetrazol-5-yl)-9H-carbazole

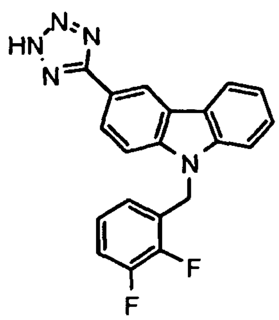

HPLC-MS (Method C): m/z= 362 (M+1); Rt = 4.13 min.

Example 784 (General procedure (J))

9-(2,5-Difluorobenzyl)-3-(2H-tetrazol-5-yl)-9H-carbazole

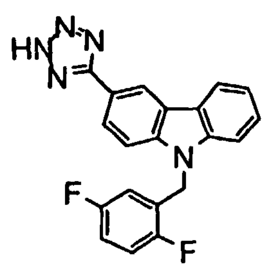

HPLC-MS (Method C): m/z = 362 (M+1); Rt = 4.08 min.

Example 785 (General procedure (J))

9-Pentafluorophenylmethyl-3-(2H-tetrazol-5-yl)-9H-carbazole

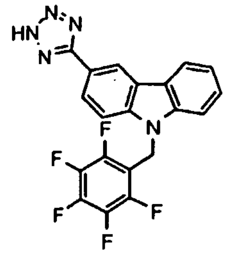

HPLC-MS (Method C): m/z = 416 (M+1); Rt = 4.32 min.

Example 786 (General procedure (J))

9-(2,6-Difluorobenzyl)-3-(2H-tetrazol-5-yl)-9H-carbazole

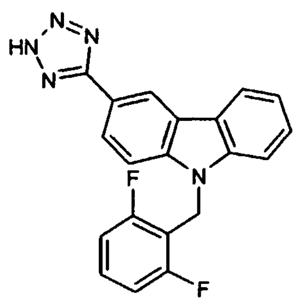

HPLC-MS (Method C): m/z = 362 (M+1); Rt = 3.77 min.

Further compounds of the invention that may be prepared according to general procedure (J), and includes:

| | | |
|---|---|---|
| Example 787 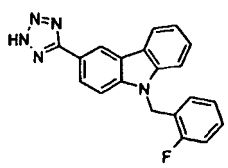 | Example 788 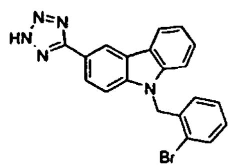 | Example 789 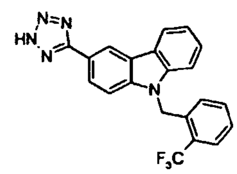 |
| Example 790 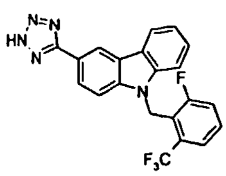 | Example 791 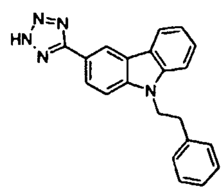 | Example 792 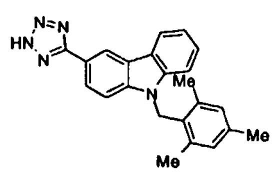 |
| Example 793 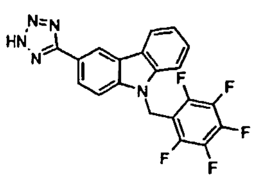 | Example 794 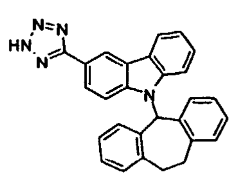 | Example 795 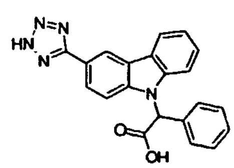 |

(continued)

| | | |
|---|---|---|
| Example 796 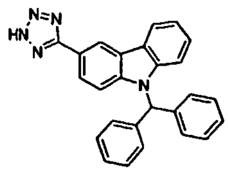 | Example 797 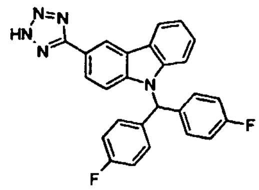 | Example 798 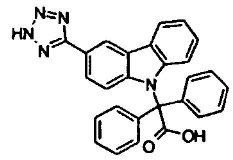 |
| Example 799 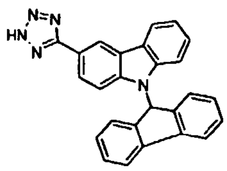 | | |

The following compounds of the invention may be prepared eg. from 9-(4-bromobenzyl)-3-(2*H*-tetrazol-5-yl)-9*H*-carbazole (example 736) or from 9-(3-bromobenzyl)-3-(2*H*-tetrazol-5-yl)-9*H*-carbazole (example 730) and aryl boronic acids *via* the Suzuki coupling reaction eg as described in Littke, Dai & Fu J. Am. Chem. Soc., 2000, 122, 4020-8 (or references cited therein), or using the methodology described in general procedure (E), optionally changing the palladium catalyst to bis(tri-*tert*-butylphosphine)palladium (0).

| | | |
|---|---|---|
| Example 800 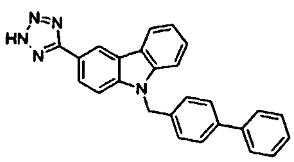 | Example 801 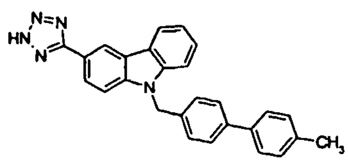 | Example 802 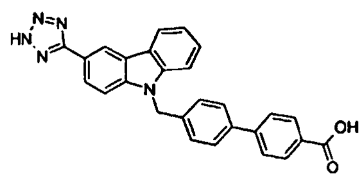 |
| Example 803 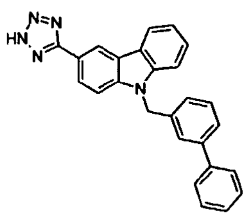 | Example 804 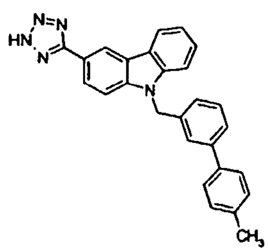 | Example 805 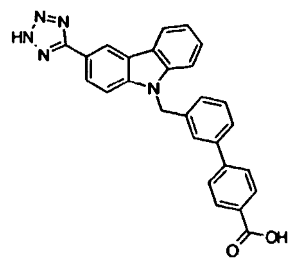 |

**General procedure (K) for preparation of compounds of general formula $I_{10}$:**

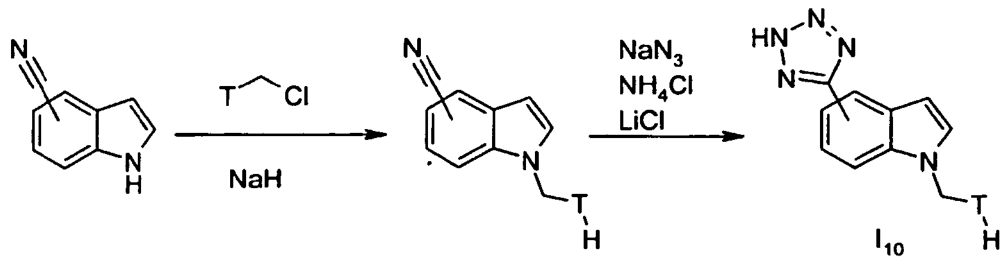

$I_{10}$

wherein T is as defined above.

The general procedure (K) is further illustrated by the following example:

Example 806 (General procedure (K)).

1-Benzyl-5-(2*H*-tetrazol-5-yl)-1*H*-indole

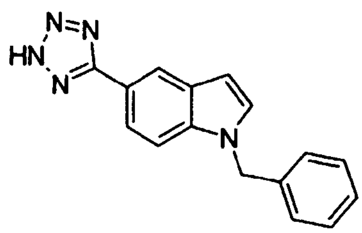

5-Cyanoindole (1.0 g, 7.0 mmol) was dissolved in *N,N*-dimethylformamide (14 mL) and cooled in an ice-water bath. Sodium hydride (0.31 g, 60 %, 7.8 mmol) was added, and the resulting suspension was stirred for 30 min. Benzyl chloride (0.85 mL, 0.94 g, 7.4 mmol) was added, and the cooling was discontinued. The stirring was continued for 65 hours at room temperature. Water (150 mL) was added, and the mixture was extracted with ethyl acetate (3 x 25 mL). The combined organic phases were washed with brine (30 mL) and dried with sodium sulfate (1 hour). Filtration and concentration yielded the crude material. Purification by flash chromatography on silica gel eluting with ethyl acetate/heptanes = 1:3 afforded 1.60 g 1-benzyl-1*H*-indole-5-carbonitrile.

HPLC-MS (Method C): m/z: 233 (M+1); Rt = 4.17 min.

1-Benzyl-1*H*-indole-5-carbonitrile was transformed into 1-benzyl-5-(2*H*-tetrazol-5-yl)-1*H*-indole by the method described in general procedure (J) and in example 594. Purification was done by flash chromatography on silica gel eluting with dichloromethane/methanol =9:1.

HPLC-MS (Method C): m/z: 276 (M+1); Rt = 3.35 min.

The compounds in the following examples were prepared by the same procedure.

Example 807 (General procedure (K)).

1-(4-Bromobenzyl)-5-(2*H*-tetrazol-5-yl)-1*H*-indole

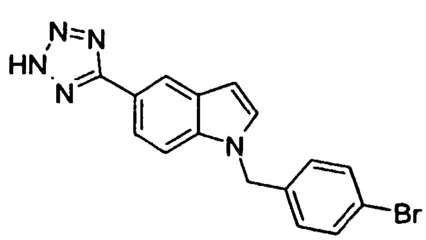

HPLC-MS (Method C): m/z: 354 (M+1); Rt = 3.80 min.

Example 808 (General procedure (K)).

1-(4-Phenylbenzyl)-5-(2*H*-tetrazol-5-yl)-1*H*-indole

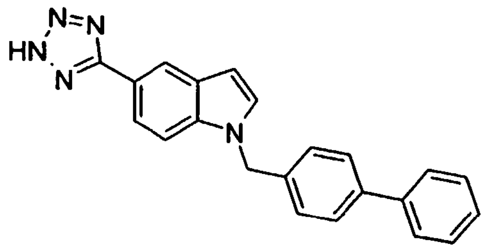

$^1$H-NMR (200 MHz, DMSO-$d_6$): δ = 5.52 (2H, s), 6.70 (1H, d), 7.3-7.45 (6H, m), 7.6 (4H, m), 7.7-7.8 (2H, m), 7.85(1H, dd), 8.35 (1H, d).
Calculated for $C_{22}H_{17}N_5$, $H_2O$:
73.32% C; 5.03% H; 19.43% N. Found:
73.81% C; 4.90% H; 19.31% N.

Example 809

4'-[5-(2H-Tetrazol-5-yl)indol-1-ylmethyl]biphenyl-4-carboxylic acid

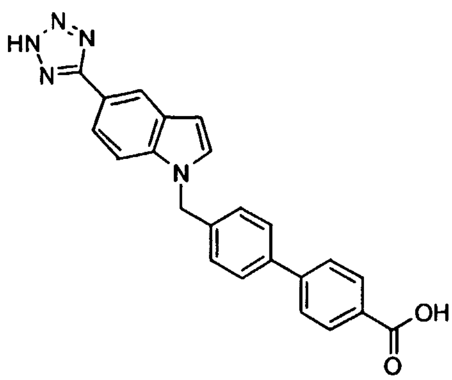

5-(2H-Tetrazol-5-yl)-1H-indole (Syncom BV, Groningen, NL) (1.66g, 8.9 mmol) was treated with trityl chloride (2.5 g, 8.9 mmol) and triethyl amine (2.5 mL, 17.9 mmol) in DMF(25 mL) by stirring at RT overnight. The resulting mixture was treated with water. The gel was isolated, dissolved in methanol, treated with activated carbon; filtered and evaporated to dryness in *vacuo.* This afforded 3.6 g (94%) of crude 5-(2-trityl-2*H*-tetrazol-5-yl)-1*H*-indole.
HPLC-MS (Method C): m/z = 450 (M+23); Rt. = 5.32 min.

4-Methylphenylbenzoic acid (5 g, 23.5 mmol) was mixed with $CCl_4$ (100 mL) and under an atmosphere of nitrogen, the slurry was added *N*-Bromosuccinimide (4.19 g, 23.55 mmol) and dibenzoyl peroxide (0.228 g, 0.94 mmol). The mixture was subsequently heated to reflux for 0.5 hour. After cooling, DCM and water (each 30 mL) were added. The resulting precipitate was isolated, washed with water and a small amount of methanol. The solid was dried *in vacuo* to afford 5.27 g (77%) of 4'-bromomethylbiphenyl-4-carboxylic acid.
HPLC-MS (Method C): m/z = 291 (M+1); Rt. = 3.96 min.

5-(2-Trityl-2*H*-tetrazol-5-yl)-1*H*-indole (3.6 g, 8.4 mmol) was dissolved in DMF (100 mL). Under nitrogen, NaH (60 % suspension in mineral oil, 34 mmol) was added slowly. 4'-Bromomethylbiphenyl-4-carboxylic acid (2.7 g, 9.2 mmol) was added over 5 minutes and the resulting slurry was heated at 40 ˚C for 16 hours. The mixture was poured

into water (100mL) and the precipitate was isolated by filtration and treated with THF/6N HCl (9/1) (70 mL) at room temperature for 16 hours. The mixture was subsequently evaporated to dryness *in vacuo,* the residue was treated with water and the solid was isolated by filtration and washed thoroughly 3 times with DCM. The solid was dissolved in hot THF (400 mL) treated with activated carbon and filtered. The filtrate was evaporated *in vacuo* to dryness. This afforded 1.6 g (50%) of the title compound.
HPLC-MS (Method C): m/z = 396 (M+1); Rt. = 3.51 min.

Example 810 (General procedure (K)).

5-(2*H*-Tetrazol-5-yl)-1*H*-indole

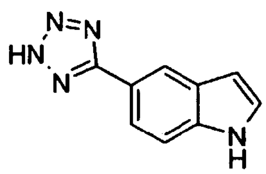

5-(2*H*-Tetrazol-5-yl)-1*H*-indole was prepared from 5-cyanoindole according to the method described in example 594.
HPLC-MS (Method C): m/z: 186 (M+1); Rt= 1.68 min.

Example 811 (General procedure (K)).

1-Benzyl-4-(2*H*-tetrazol-5-yl)-1*H*-indole

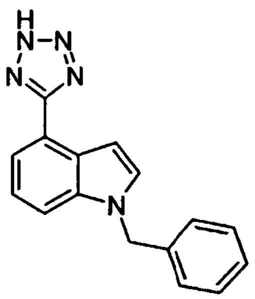

1-Benzyl-1*H*-indole-4-carbonitrile was prepared from 4-cyanoindole according to the method described in example 806.
HPLC-MS (Method C): m/z: 233 (M+1); Rt = 4.24 min.
1-Benzyl-4-(2*H*-tetrazol-5-yl)-1*H*-indole was prepared from 1-benryl-1*H*-indole-4-carbonitrile according to the method described in example 594.
HPLC-MS (Method C): m/z: 276 (M+1); Rt = 3.44 min.

**General procedure (L) for preparation of compounds of general formula $I_{11}$:**

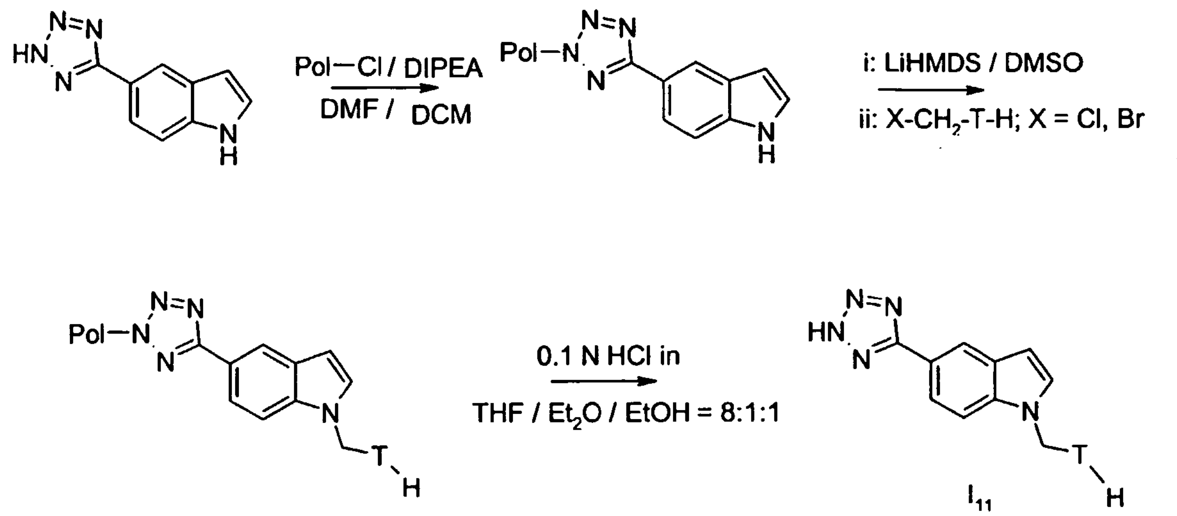


$I_{11}$

wherein T is as defined above and
Pol- is a polystyrene resin loaded with a 2-chlorotrityl linker, graphically shown below:

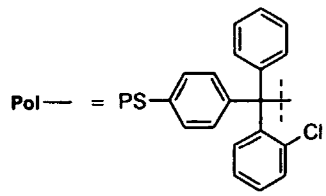

This general procedure (L) is further illustrated by the following example:

Example 812 (General procedure (L)).

5-(2*H*-Tetrazol-5-yl)-1-[3-(trifluoromethyl)benzyl]-1*H*-indole

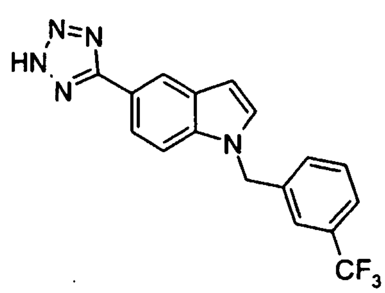

2-Chlorotritylchloride resin (100 mg, 0.114 mmol active chloride) was swelled in dichloromethane (2 mL) for 30 min. The solvent was drained, and a solution of 5-(2*H*-tetrazol-5-yl)-1*H*-indole (example 810) (63 mg, 0.34 mmol) in a mixture of *N,N*-dimethylformamide, dichloromethane and N,N-di(2-propyl)ethylamine (DIPEA) (5:5:2) (1.1 mL) was added. The reaction mixture was shaken at room temperature for 20 hours. The solvent was removed by filtration, and the resin was washed consecutively with *N,N*-dimethylformamide (2 x 4 mL), dichloromethane (6 x 4 mL) and methyl sulfoxide (2 x 4 mL). Methyl sulfoxide (1 mL) was added, followed by the addition of a solution of lithium bis(trimethylsilyl) amide in tetrahydrofuran (1.0 M, 0.57 mL, 0.57 mmol). The mixture was shaken for 30 min at room temperature, before

3-(trifluoromethyl)benzyl bromide (273 mg, 1.14 mmol) was added as a solution in methyl sulfoxide (0.2 mL). The reaction mixture was shaken for 20 hours at room temperature. The drained resin was washed consecutively with methyl sulfoxide (2 x 4 mL), dichloromethane (2 x 4 mL), methanol (2 x 4 mL), dichloromethane (2 x 4 mL) and tetrahydrofuran (4 mL). The resin was treated with a solution of hydrogen chloride in tetrahydrofuran, ethyl ether and ethanol = 8:1:1 (0.1 M, 3 mL) for 6 hours at room temperature. The resin was drained and the filtrate was concentrated *in vacuo*. The crude product was re-suspended in dichloromethane (1.5 mL) and concentrated three times to afford the title compound (35 mg). No further purification was necessary.
HPLC-MS (Method B): m/z: 344 (M+1); Rt = 4.35 min.
$^1$H-NMR (DMSO-$d_6$): δ 8.29 (1H, s), 7.80 (1H, dd), 7.72 (2H, m), 7.64 (2H, bs), 7.56 (1H, t), 7.48 (1H, d), 6.70 (1H, d), 5.62 (2H, s).

The compounds in the following examples were prepared in a similar fashion. Optionally, the compounds can be further purified by recrystallization or by chromatography.

Example 813 (General procedure (L)).

1-(4-Chlorobenzyl)-5-(2*H*-tetrazol-5-yl)-1*H*-indole

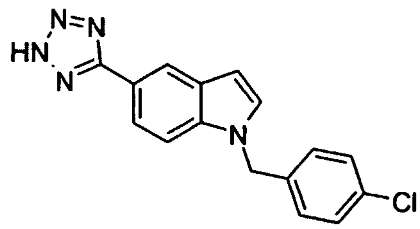

HPLC-MS (Method B): m/z: 310 (M+1); Rt = 4.11 min.

Example 814 (General procedure (L)).

1-(2-Chlorobenzyl)-5-(2*H*-tetrazol-5-yl)-1*H*-indote

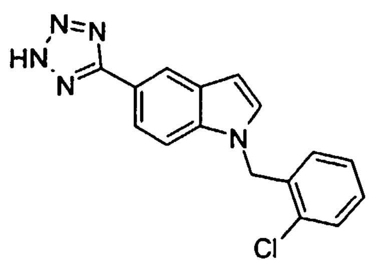

HPLC-MS (Method B): m/z: 310 (M+1); Rt = 4.05 min.

Example 815 (General procedure (L)).

1-(4-Methoxybenzyl)-5-(2*H*-tetrazol-5-yl)-1*H*-indole

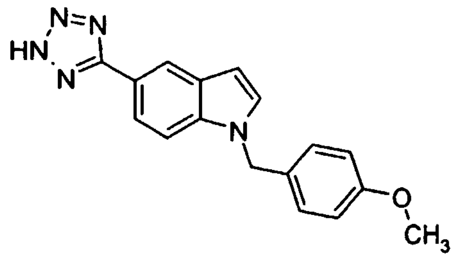

HPLC-MS (Method B): m/z: 306 (M+1); Rt = 3.68 min.

Example 816 (General procedure (L)).

1-(4-Methylbenzyl)-5-(2*H*-tetrazol-5-yl)-1*H*-indole

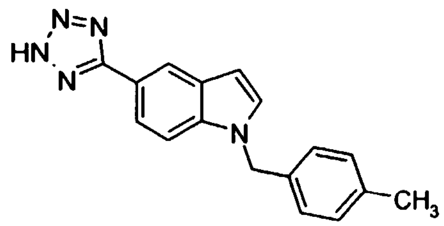

HPLC-MS (Method B): m/z: 290 (M+1); Rt = 3.98 min.

Example 817 (General procedure (L)).

5-(2*H*-Tetrazol-5-yl)-1-[4-(trifluoromethyl)benzyl]-1*H*-indole

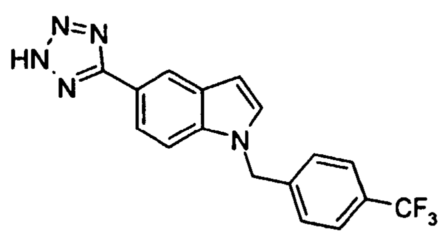

HPLC-MS (Method B): m/z: 344 (M+1); Rt = 4.18 min.

Example 818 (General procedure (L)).

1-(3-Chlorobenzyl)-5-(2*H*-tetrazol-5-yl)-1*H*-indole

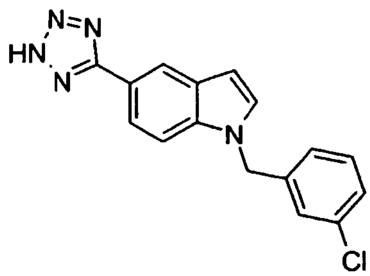

HPLC-MS (Method B): m/z: 310 (M+1); Rt = 4.01 min.

Example 819 (General procedure (L)).

1-(3-Methylbenzyl)-5-(2*H*-tetrazol-5-yl)-1*H*-indole

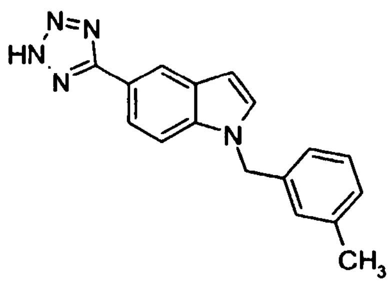

HPLC-MS (Method B): m/z: 290 (M+1); Rt = 3.98 min.

Example 820 (General procedure (L)).

1-(2,4-Dichlorobenzyl)-5-(2*H*-tetrazol-5-yl)-1*H*-indole

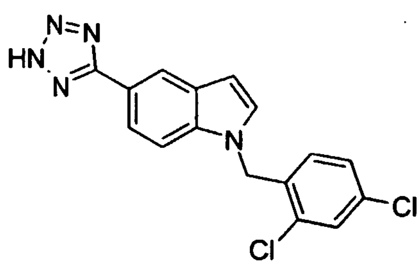

HPLC-MS (Method B): m/z: 344 (M+1); Rt = 4.41 min.

Example 821 (General procedure (L)).

1-(3-Methoxybenzyl)-5-(2*H*-tetrazol-5-yl)-1*H*-indole

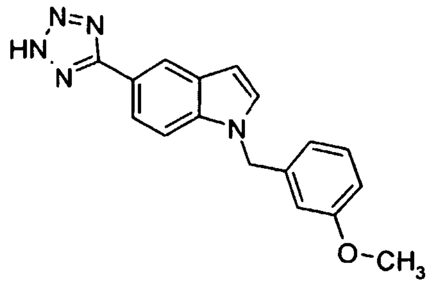

HPLC-MS (Method B): m/z: 306 (M+1); Rt = 3.64 min.

Example 822 (General procedure (L)).

1-(4-Fluorobenryl)-5-(2*H*-tetrazol-5-yl)-1*H*-indole

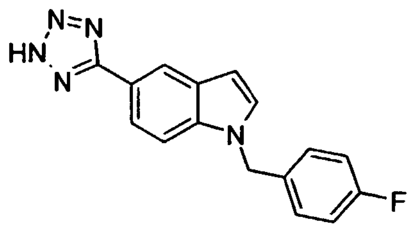

HPLC-MS (Method B): m/z: 294 (M+1); Rt = 3.71 min.

Example 823 (General procedure (L)).

1-(3-Fluorobenzyl)-5-(2*H*-tetrazol-5-yl)-1*H*-indole

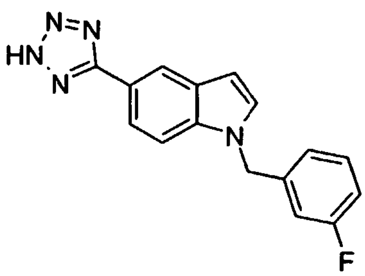

HPLC-MS (Method B): m/z: 294 (M+1); Rt = 3.68 min.

Example 824 (General procedure (L)).

1-(2-Iodobenzyl)-5-(2*H*-tetrazol-5-yl)-1*H*-indole

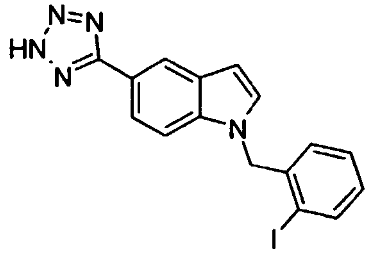

HPLC-MS (Method B): m/z: 402 (M+1); Rt = 4.11 min.

Example 825 (General procedure (L)).

1-[(Naphthalen-2-yl)methyl]-5-(2*H*-tetrazol-5-yl)-1*H*-indole

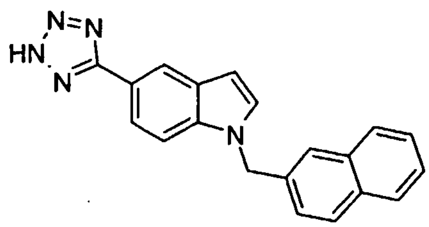

HPLC-MS (Method B): m/z: 326 (M+1); Rt = 4.18 min.

Example 826 (General procedure (L)).

1-(3-Bromobenzyl)-5-(2*H*-tetrazol-5-yl)-1*H*-indole

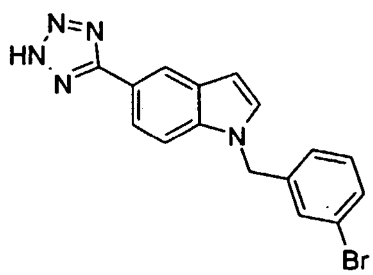

HPLC-MS (Method B): m/z: 354 (M+1); Rt = 4.08 min.

Example 827 (General procedure (L)).

1-(4-Carboxybenzyl)-5-(2*H*-tetrazol-5-yl)-1*H*-indole

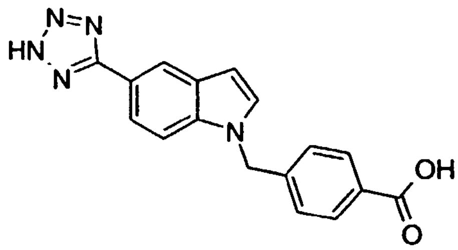

In this preparation, a larger excess of lithium bis(trimethylsilyl)amide in tetrahydrofuran (1.0 M, 1.7 mL, 1.7 mmol) was used.
HPLC-MS (Method B): m/z: 320 (M+1); Rt = 2.84 min.

Example 828 (General procedure (L)).

1-(3-Carboxybenzyl)-5-(2*H*-tetrazol-5-yl)-1*H*-indole

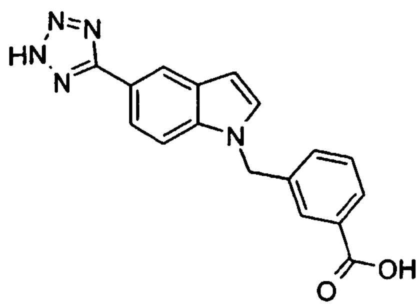

In this preparation, a larger excess of lithium bis(trimethylsilyl)amide in tetrahydrofuran (1.0 M, 1.7 mL, 1.7 mmol) was used.
HPLC-MS (Method B): m/z: 320 (M+1); Rt = 2.91 min.

Example 829 (General procedure (L)).

1-(2,4-Difluorobenzyl)-5-(2*H* tetrazol-5-yl)-1*H*-indole

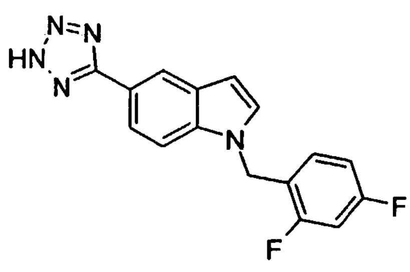

HPLC-MS (Method B): m/z: 312 (M+1); Rt = 3.78 min.

Example 830 (General procedure (L)).

1-(3,5-Difluorobenzyl)-5-(2*H*-tetrazol-5-yl)-1*H*-indole

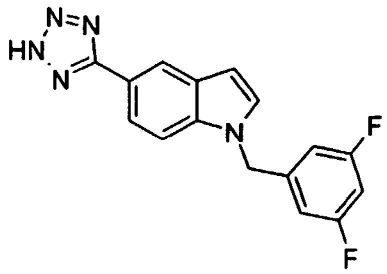

HPLC-MS (Method B): m/z: 312 (M+1); Rt = 3.78 min.

Example 831 (General procedure (L)).

1-(3,4-Difluorobenzyl)-5-(2*H*-tetrazol-5-yl)-1*H*-indole

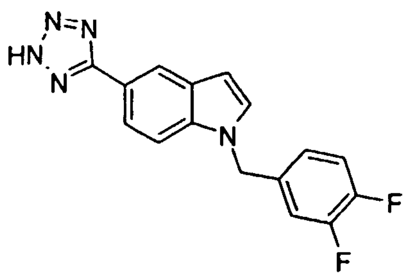

HPLC-MS (Method B): m/z: 312 (M+1); Rt = 3.81 min.

Example 832 (General procedure (L)).

1-[4-(2-Propyl)benzyl]-5-(2*H*-tetrazol-5-yl)-1*H*-indole

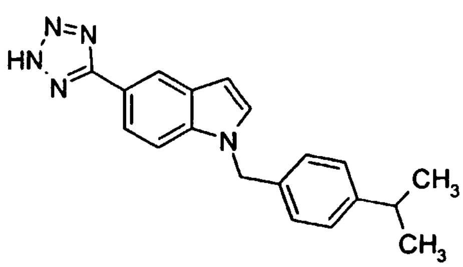

HPLC-MS (Method B): m/z: 318 (M+1); Rt = 4.61 min.

Example 833 (General procedure (L)).

1-(3,5-Dimethoxybenzyl)-5-(2*H*-tetrazol-5-yl)-1*H*-indole

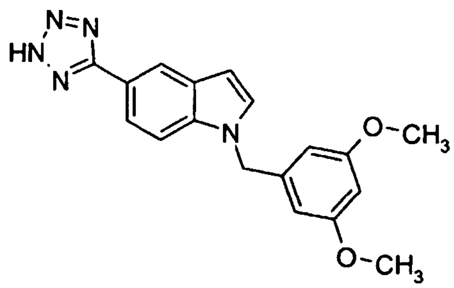

HPLC-MS (Method B): m/z: 336 (M+1); Rt = 3.68 min.

Example 834 (General procedure (L)).

1-(2'-Cyanobiphenyl-4-ylmethyl)-5-(2*H*-tetrazol-5-yl)-1*H*-indole

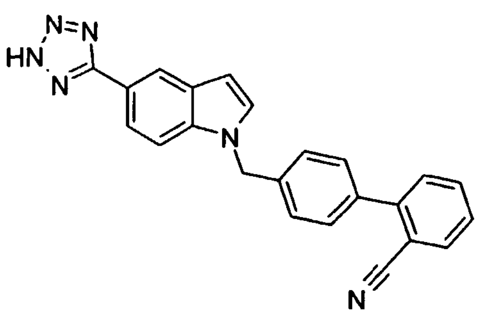

HPLC-MS (Method B): m/z: 377 (M+1); Rt = 4.11 min.

Example 835 (General procedure (L)).

1-(2-Methylbenzyl)-5-(2*H*-tetrazol-5-yl)-1*H*-indole

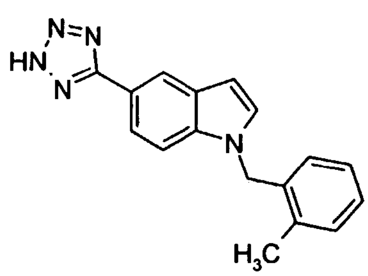

HPLC-MS (Method B): m/z: 290 (M+1); Rt = 3.98 min.

Further compounds of the invention that may be prepared according to general procedure (K) and/or (L) includes:

| | | |
|---|---|---|
| Example 836 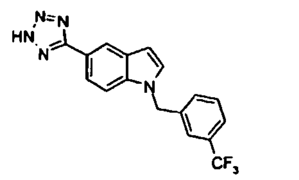 | Example 837 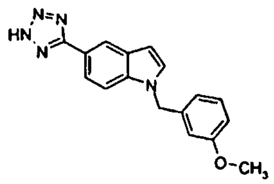 | Example 838 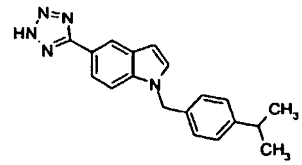 |
| Example 839 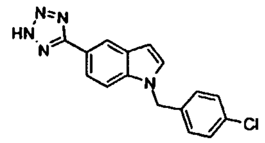 | Example 840 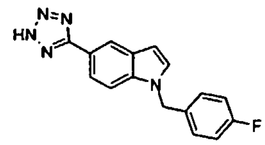 | Example 841 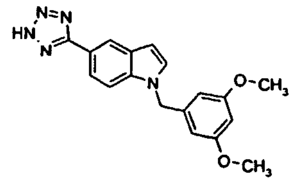 |
| Example 842 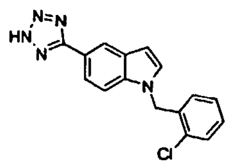 | Example 843 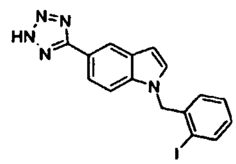 | Example 844 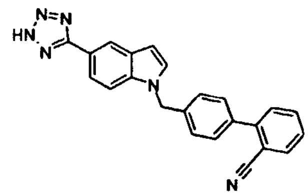 |
| Example 845 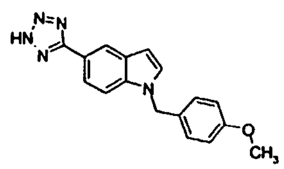 | Example 846 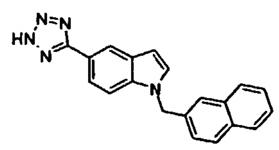 | Example 847 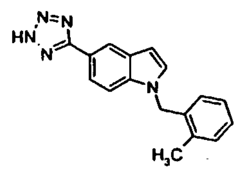 |
| Example 848 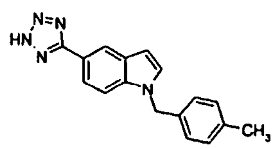 | Example 849 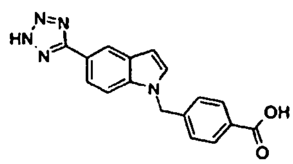 | Example 850 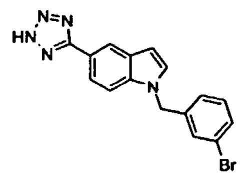 |
| Example 851 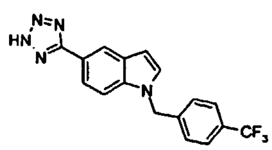 | Example 852 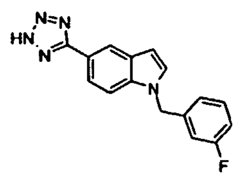 | Example 853 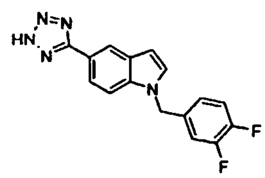 |
| Example 854 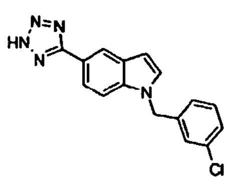 | Example 855 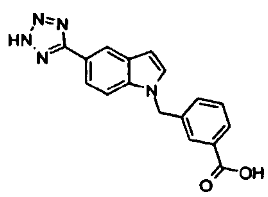 | Example 856 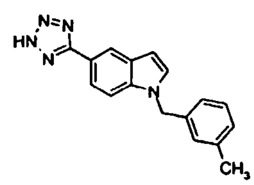 |

(continued)

| Example 857 | Example 858 | Example 859 |
|---|---|---|
| 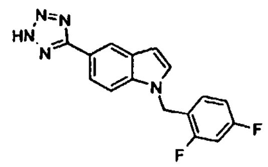 | 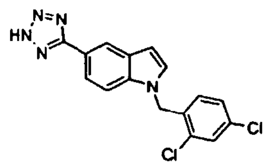 | 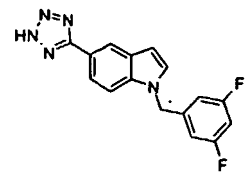 |

The following compounds of the invention may be prepared eg. from 1-(4-bromobenzyl)-5-(2*H*-tetrazol-5-yl)-1*H*-indole (example 807) or from the analogue 1-(3-bromobenzyl)-5-(2*H*-tetrazol-5-yl)-1*H*-indole and aryl boronic acids *via* the Suzuki coupling reaction eg as described in Littke, Dai & Fu J. Am. Chem. Soc., 2000, 122, 4020-8 (or references cited therein), or using the methodology described in general procedure (E), optionally changing the palladium catalyst to bis(tri-*tert*-butylphosphine)palladium (0).

| | Example 860 | Example 861 |
|---|---|---|
| | 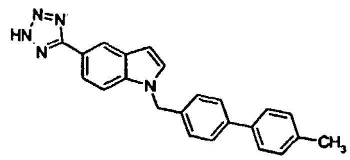 | 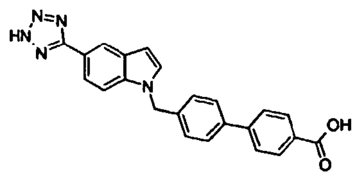 |
| Example 862 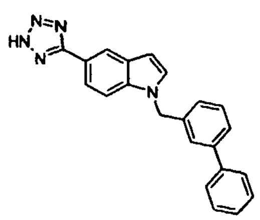 | Example 863 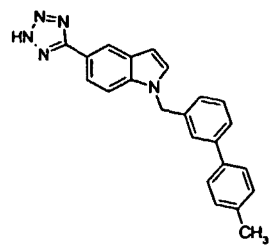 | Example 864 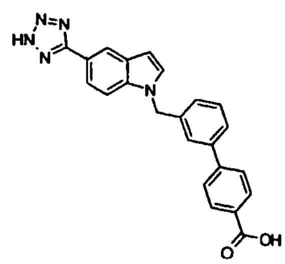 |

**General procedure (M) for preparation of compounds of general formula $I_{12}$:**

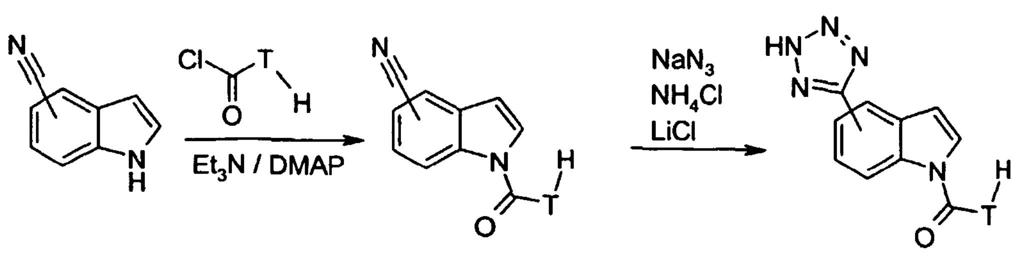


$I_{12}$

wherein T is as defined above.

The general procedure (M) is further illustrated by the following example:

Example 865 (General procedure (M)).

1-Benzoyl-5-(2*H*-tetrazol-5-yl)-1*H*-indole

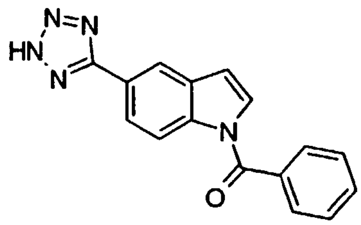

To a solution of 5-cyanoindole (1.0 g, 7.0 mmol) in dichloromethane (8 mL) was added 4-(dimethylamino) pyridine (0.171 g, 1.4 mmol), triethylamine (1.96 mL, 1.42 g, 14 mmol) and benzoyl chloride (0.89 mL, 1.08 g, 7.7 mmol). The resulting mixture was stirred for 18 hours at room temperature. The mixture was diluted with dichloromethane (80 mL) and washed consecutively with a saturated solution of sodium hydrogencarbonate (40 mL) and brine (40 mL). The organic phase was dried with magnesium sulfate (1 hour). Filtration and concentration furnished the crude material which was purified by flash chromatography on silica gel, eluting with ethyl acetate/heptanes = 2:3. 1-Benzoyl-1*H*-indole-5-carbonitrile was obtained as a solid.
HPLC-MS (Method C): m/z: 247 (M+1); Rt = 4.07 min.
1-Benzoyl-1*H*-indole-5-carbonitrile was transformed into 1-benzoyl-5-(2*H*-tetrazol-5-yl)-1*H*-indole by the method described in example 594.
HPLC (Method C): Rt = 1.68 min.
The compound in the following example was prepared by the same procedure.

Example 866 (General procedure (M)).

1-Benzoyl-4-(2*H*-tetrazol-5-yl)-1*H*-indole

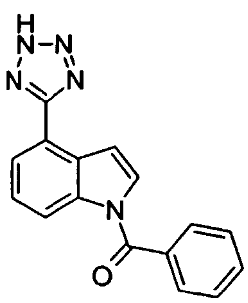

1-Benzoyl-1*H*-indole-4-carbonitrile was prepared from 4-cyanoindole according to the method described in example 865.
HPLC-MS (Method C): m/z: 247 (M+1); Rt = 4.24 min.
1-Benzoyl-4-(2*H*-tetrazol-5-yl)-1*H*-indole was prepared from 1-benzoyl-1*H*-indole-4-carbonitrile according to the method described in example 594.
HPLC (Method C): Rt = 1.56 min.

Example 867 (General procedure (M))

(2-Fluoro-3-trifluoromethylphenyl)-[5-(2H-tetrazol-5-yl)-indol-1-yl]-methanone

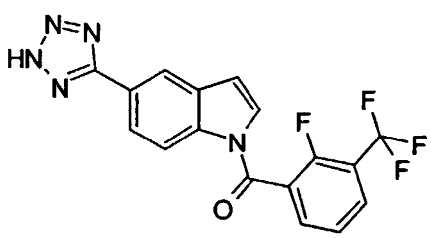

HPLC-MS (Method B): m/z = 376 (M+1); Rt = 4.32 min.

Example 868 (General procedure (M))

(4-Methoxyphenyl)-[5-(2H-tetrazol-5-yl)-indol-1-yl]-methanone

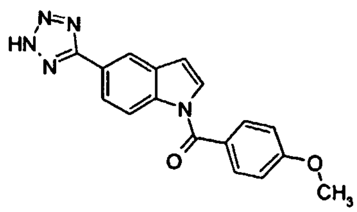

HPLC-MS (Method B): m/z = 320 (M+1); Rt = 3.70 min.

Example 869 (General procedure (M))

(3-Nitrophenyl)-[5-(2H-tetrazol-5-yl)-indol-1-yl]-methanone

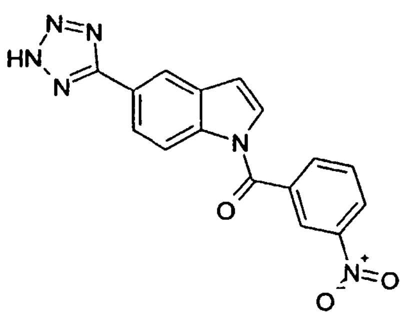

HPLC-MS (Method B): m/z = 335 (M+1); Rt = 3.72 min.

Example 870 (General procedure (M))

(4-Nitrophenyl)-[5-(2H-tetrazol-5-yl)-indol-1-yl]-methanone

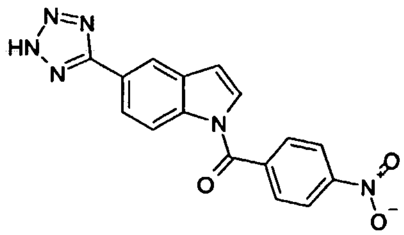

HPLC-MS (Method B): m/z = 335 (M+1); Rt = 3.71 min.

Example 871 (General procedure (M))

Naphthalen-2-yl-[5-(2H-tetrazol-5-yl)-indol-1-yl]-methanone

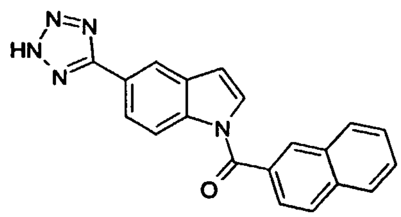

HPLC-MS (Method C): m/z = 340 (M+1); Rt = 4.25 min.

Example 872 (General procedure (M))

(2,3-Difluorophenyl)-[5-(2H-tetrazol-5-yl)-indol-1-yl]-methanone

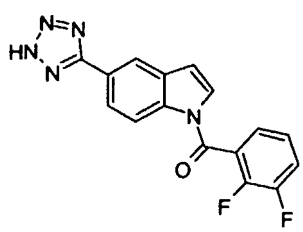

HPLC-MS (Method B: m/z = 326 (M+1); Rt = 3.85 min.

The following known and commercially available compounds do all bind to the His B10 $Zn^{2+}$ site of the insulin hexamer:

Example 873

1-(4-Fluorophenyl)-5-(2*H*-tetrazol-5-yl)-1*H*-indole

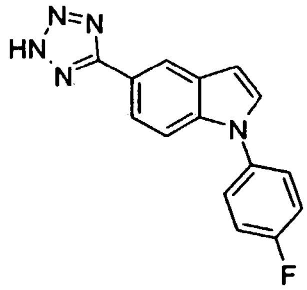

Example 874

1-Amino-3-(2*H*-tetrazol-5-yl)benzene

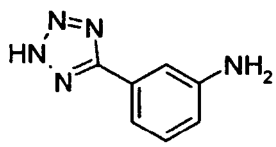

Example 875

1-Amino-4-(2*H*-tetrazol-5-yl)benzene

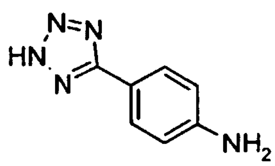

A mixture of 4-aminobenzonitrile (10 g, 84.6 mmol), sodium azide (16.5 g, 254 mmol) and ammonium chloride (13.6 g, 254 mmol) in DMF was heated at 125 ˚C for 16 hours. The cooled mixture was filtered and the filtrate was concentrated *in vacuo.* The residue was added water (200 mL) and diethyl ether (200 mL) which resulted in crystallisation. The mixture was filtered and the solid was dried *in vacuo* at 40 ˚C for 16 hours to afford 5-(4-aminophenyl)-2*H*-tetrazole. $^1$H NMR DMSO-$d_6$): δ = 5.7 (3H, bs), 6.69 (2H, d), 7.69 (2H, d). HPLC-MS (Method C): m/z: 162 (M+1); Rt = 0,55 min.

Example 8761-Nitro-4-(2*H*-tetrazol-5-yl)benzene

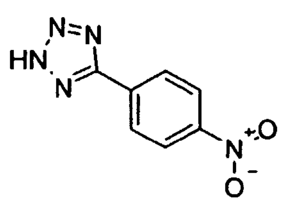

Example 8771-Bromo-4-(2*H*-tetrazol-5-yl)benzene

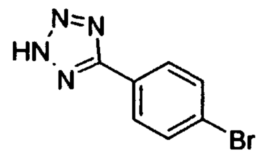

**General procedure (N) for solution phase preparation of amides of general formula $I_{13}$:**

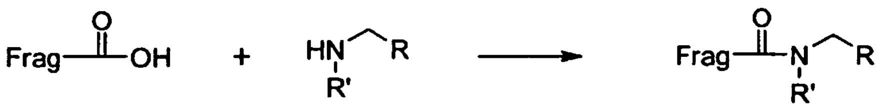

$I_{13}$

wherein Frag is any fragment carrying a carboxylic acid group, **R** is hydrogen, optionally substituted aryl or $C_{1-8}$-alkyl and **R'** is hydrogen or $C_{1-4}$-alkyl.

Frag-$CO_2H$ may be prepared eg by general procedure (D) or by other similar procedures described herein, or may be commercially available.

The procedure is further illustrated in the following example 878:

Example 878 (General procedure (N))

*N*-(4-Chlorobenzyl)-2-[3-(2,4-dioxothiazolidin-5-ylidenemethyl)-1*H*-indol-1-yl]acetamide

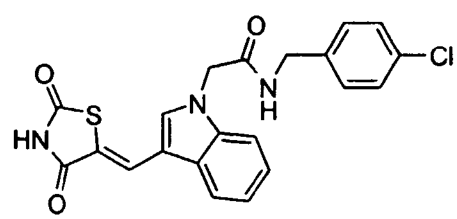

[3-(2,4-Dioxothiazolidin-5-ylidenemethyl)indol-1-yl]acetic acid (example 478, 90.7 mg, 0.3 mmol) was dissolved in NMP (1 mL) and added to a mixture of 1-ethyl-3-(3-dimethylaminopropyl)carbodiimide, hydrochloride (86.4 mg, 0.45 mmol) and 1-hydroxybenzotriazol (68.8 mg, 0.45 mmol) in NMP (1 mL). The resulting mixture was shaken at RT for 2 h. 4-Chlorobenzylamine (51 mg, 0.36 mmol) and DIPEA (46.4 mg, 0.36 mmol) in NMP (1 mL) were added to the mixture and the resulting mixture shaken at RT for 2 days. Subsequently ethyl acetate (10 mL) was added and the resulting mixture washed with 2x10 mL water followed by saturated ammonium chloride (5 mL). The organic phase was evaporated to dryness giving 75 mg (57%) of the title compound.
HPLC-MS (Method C): m/z: 426 (M+1); Rt. = 3.79 min.

Example 879 (General procedure (N))

*N*-(4-Chlorobenzyl)-4-[2-chloro-4-(2,4-dioxothiazolidin-5-ylidenemethyl)phenoxy]butyramide

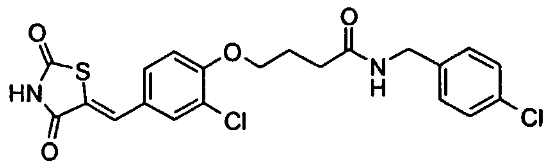

HPLC-MS (Method A): m/z: 465 (M+1); Rt = 4.35 min.

Example 880 (General procedure (N))

*N*-(4-Chlorobenzyl)-4-[4-(2,4-dioxothiazolidin-5-ylidenemethyl)phenoxy]butyramide

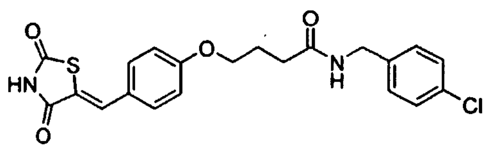

HPLC-MS (Method A): m/z: 431 (M+1); Rt = 3.68 min.

Example 881 (General procedure (N))

2-[2-Bromo-4-(2,4-dioxothiazolidin-5-ylidenemethyl)phenoxy]-*N*-(4-chlorobenzyl)acetamide

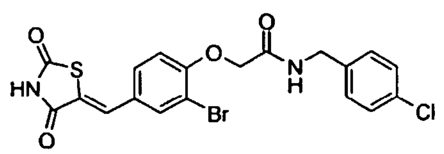

HPLC-MS (Method A): m/z: 483 (M+1); Rt = 4.06 min.

Example 882 (General procedure (N))

N-(4-Chlorobenzyl)-2-[3-(2,4-dioxothiazolidin-5-ylidenemethyl)phenoxy]acetamide

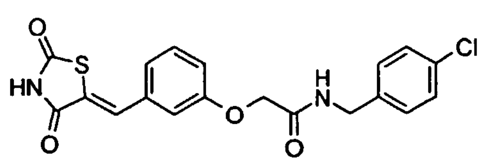

HPLC-MS (Method A): m/z: 403 (M+1); Rt = 4.03 min.

Example 883 (General procedure (N))

*N*-(4-Chlorobenzyl)-3-[4-(2,4-dioxothiazolidin-5-ylidenemethyl)phenyl]acrylamide

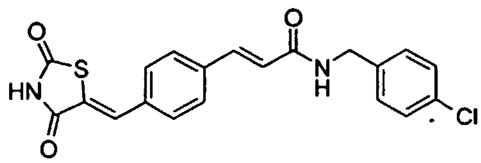

HPLC-MS (Method A): m/z: 399 (M+1); Rt = 3.82.

Example 884 (General procedure (N))

*N*-(4-Chlorobenzyl)-4-[3-(2,4-dioxothiazolidin-5-ylidenemethyl)phenoxy]butyramide

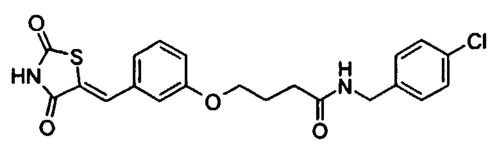

HPLC-MS (Method A): m/z: 431 (M+1); Rt = 3.84 min.

Example 885 (General procedure (N))

4-[2-Bromo-4-(2,4-dioxothiazolidin-5-ylidenemethyl)phenoxy]-*N*-(4-chlorobenzyl)butyramide

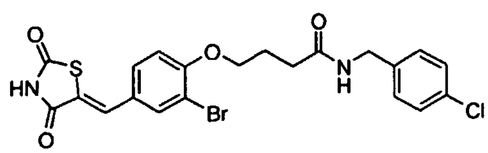

HPLC-MS (Method A): m/z: 511 (M+1); Rt = 4.05 min.

Example 886 (General procedure (N))

4-[2-Bromo-4-(4-oxo-2-thioxothiazolidin-5-ylidenemethyl)-phenoxy]-N-(4-chlorobenzyl)-butyramide

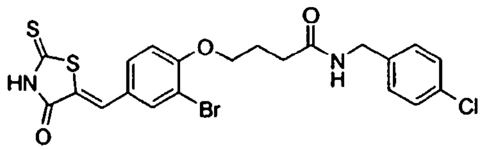

HPLC-MS (Method A): m/z: 527 (M+1); Rt = 4.77 min.

Example 887 (General procedure (N))

*N*-(4-Chlorobenzyl)-2-[4-(2,4-dioxothiazolidin-5-ylidenemethyl)naphthalen-1-yloxy]acetamide

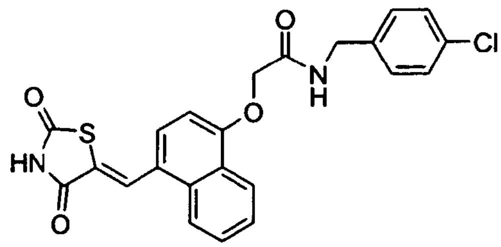

HPLC-MS (Method C): m/z: 431 (M+1); Rt. = 4.03 min.

Example 888 (General procedure (N))

*N*-(4-Chlorobenzyl)-3-[3-(2,4-dioxothiazolidin-5-ylidenemethyl)-1*H*-indol-1-yl]propionamide

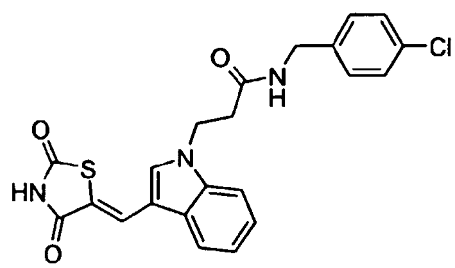

HPLC-MS (Method C): m/z: 440 (M+1); Rt. = 3.57 min.

Example 889 (General procedure (N))

*N*-(4-Chlorobenzyl)-4-[4-(2,4-dioxothiazolidin-5-ylldenemethyl)naphthalen-1-yloxy]butyramide

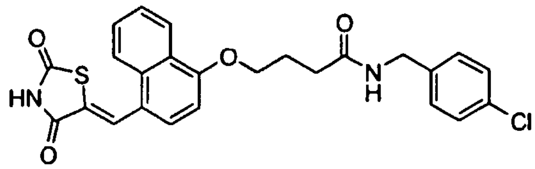

HPLC-MS (Method C): m/z: 481 (M+1); Rt = 4.08 min.

Example 890 (General procedure (N))

4-[4-(2,4-Dioxothiazolidin-5-ylidenemethyl)-naphthalen-1-yloxy]-*N*-hexylbutyramide

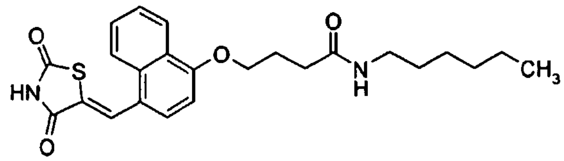

HPLC-MS (Method C): m/z: 441 (M+1); Rt = 4.31 min.

Example 891 (General Procedure (N))

4-({[3-(2,4-Dioxothiazolidin-5-ylidenemethyl)indole-7-carbony]amino}methyl)benzoic acid methyl ester

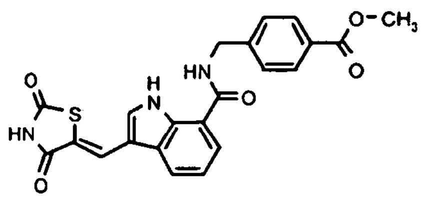

HPLC-MS (Method C): m/z: 436 (M+1); Rt.= 3.55 min.

Example 892 (General procedure (N))

*N*-(4-Chlorobenzyl)-4-[3-(2*H*-tetrazol-5-yl)carbazol-9-ylmethyl]benzamide

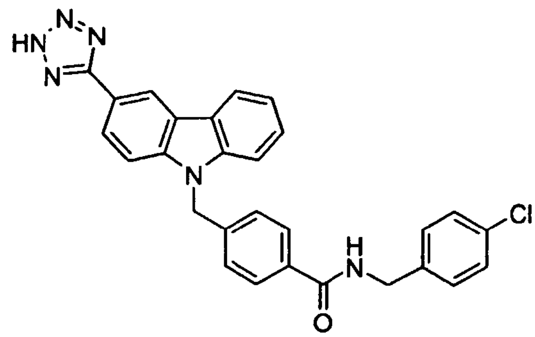

HPLC-MS (Method C): m/z:493 (M+1); Rt = 4.19 min.

Example 893 (General procedure (N))

*N*-(4-Chlorobenzyl)-3-[3-(2*H*-tetrazol-5-yl)carbazol-9-ylmethyl]benzamide

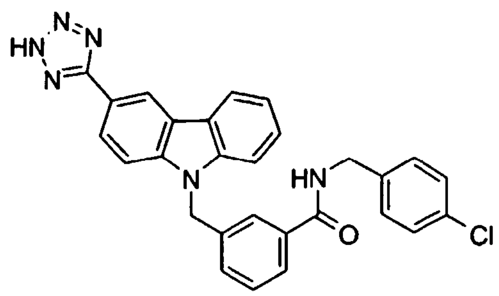

HPLC-MS (Method C): m/z: 493 (M+1); Rt = 4.20 min.

Example 894 (General Procedure (N))

*N*-(4-Chlorobenzyl)-3-methyl-4-[3-(2*H*-tetrazol-5-yl)-carbazol-9-ylmethyl]benzamide

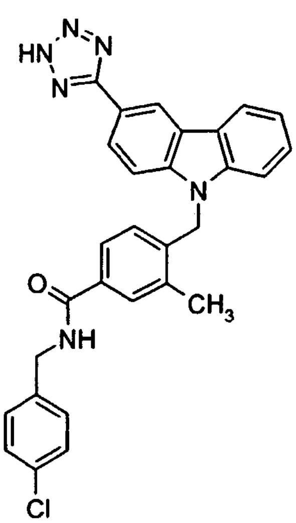

HPLC-MS (Method C): m/z: 507 (M+1); Rt = 4.37min.

Example 895 (General procedure (N))

5-{2-[4-(2,4-Dioxothiazolidin-5-ylidenemethyl)-naphthalen-1-yloxy]-acetylamino}-isophthalic acid dimethyl ester

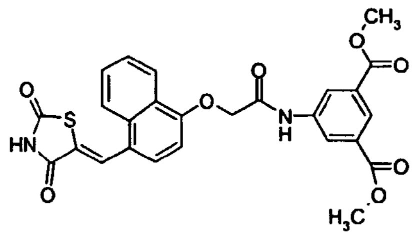

HPLC-MS (Method C): m/z = 521 (M+1); Rt. = 4.57 min.

Example 896 (General procedure (N))

5-{2-[4-(2,4-Dioxothiazolidin-5-ylidenemethyl)-naphthalen-1-yloxy]-acetylamino}-isophthalic acid

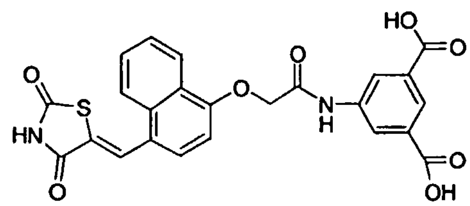

HPLC-MS (Method C): m/z = 515 (M+23); Rt. = 3.09 min.

Example 897 (General procedure (N))

5-(3-{2-[4-(2,4-Dioxothiazolidin-5-ylidenemethyl)-naphthalen-1-yloxy]-ethyl}-ureido)-isophthalic acid monomethyl ester

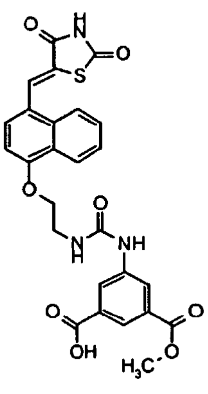

HPLC-MS (Method C): m/z = 536 (M+1); Rt = 3,58 min.

Example 898 (General Procedure (N)).

2-{4-[3-(2H-Tetrazol-5-yl)carbazol-9-ylmethyl]benzoylamino}succinic acid dimethyl ester

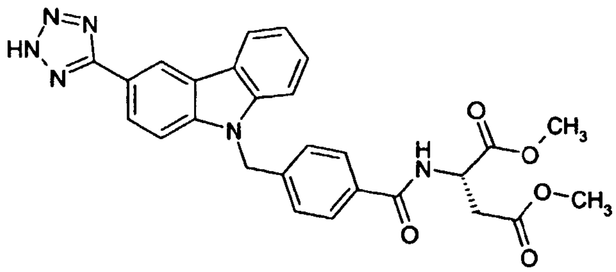

4-[3-(1H-Tetrazol-5-yl)carbazol-9-ylmethyl]benzoic acid (2.00 g, 5.41 mmol), 1-hydroxybenzotriazole (1.46 g, 10.8 mmol) and N,N-di(2-propyl)ethylamine (4.72 mL, 3.50 g, 27.1 mmol) were dissolved in dry N,N-dimethylformamide (60 mL). The mixture was cooled in an ice-water bath, and 1-[3-(dimethylamino)propyl]-3-ethylcarbodiimide hydrochloride (1.45 g, 7.56 mmol) and (S)-aminosuccinic acid dimethyl ester hydrochloride (1.28 g, 6.48 mmol) were added. The cooling was discontinued, and the reaction mixture was stirred at room temperature for 18 hours before it was poured into hydrochloric acid (0.1 N, 600 mL). The solid was collected by filtration and washed with water (2 X 25 mL) to furnish the title compound.
HPLC-MS (Method C): m/z: 513 (M+1); Rt = 3.65 min.
$^1$H-NMR (DMSO-$d_6$): δ 8.90 (1H, d), 8.86 (1H, d), 8.29 (1H, d), 8.11 (1H, dd), 7.87 (1H, d), 7.75 (2H, d), 7.69 (1H, d), 7.51 (1H, t), 7.32 (1H, t), 7.28 (2H, d), 5.82 (2H, s), 4.79 (1H, m), 3.61 (3H, s), 3.58 (3H, s), 2.92 (1H, dd), 2.78 (1H, dd).

Example 899 (General Procedure (N)).

2-{4-[3-(2H-Tetrazol-5-yl)carbazol-9-ylmethyl]benzoylamino}succinic acid

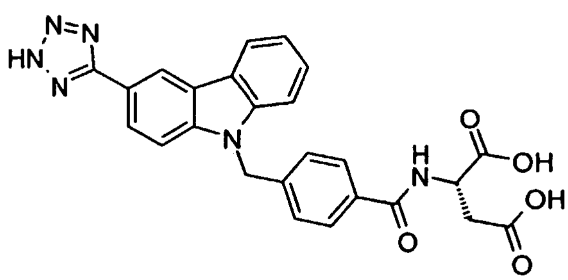

2-{4-[3-(2H-Tetrazol-5-yl)carbazol-9-ytmethyl]benzoylamino}succinic acid dimethyl ester (1.20 g, 2.34 mmol) was dissolved in tetrahydrofuran (30 mL). Aqueous sodium hydroxide (1 N, 14 mL) was added, and the resulting mixture was stirred at room temperature for 18 hours. The reaction mixture was poured into hydrochloric acid (0.1 N, 500 mL). The solid was collected by filtration and washed with water (2 X 25 mL) and diethyl ether (2 X 25 mL) to furnish the title compound.
HPLC-MS (Method C): m/z: 485 (M+1); Rt = 2.94 min.
$^1$H-NMR (DMSO-$d_6$): δ 12.44 (2H, s (br)), 8.90 (1H, d), 8.68 (1H, d), 8.29 (1H, d), 8.11 (1H, dd), 7.87 (1H, d), 7.75 (2H, d), 7.68 (1H, d), 7.52 (1H, t), 7.32 (1H, t), 7.27 (2H, d), 5.82 (2H, s), 4.70 (1H, m), 2.81 (1H, dd), 2.65 (1H, dd).
The compounds in the following examples were prepared in a similar fashion.

Example 900 (General procedure (N))

2-{4-[3-(2H-Tetrazol-5-yl)-carbazol-9-ylmethyl]-benzoylamino}-succinic acid dimethyl ester

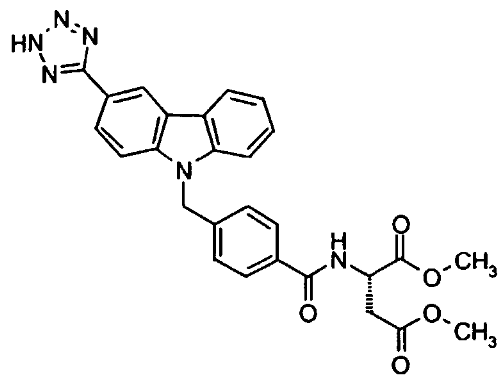

HPLC-MS (Method C): m/z = 513 (M+1); Rt = 3.65min.

Example 901 (General procedure (N))

2-{4-[3-(2H-Tetrazol-5-yl)carbazol-9-ylmethyl]benzoylamino}pentanedioic acid dimethyl ester

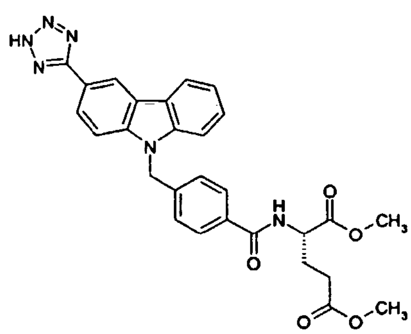

HPLC-MS (Method C): m/z = 527 (M+1); Rt = 3.57min.

Example 902 (General procedure (N))

(Methoxycarbonylmethyl-{4-[3-(2H-tetrazol-5-yl)-carbazol-9-ylmethyl]-benzoyl}-amino)-acetic acid methyl ester

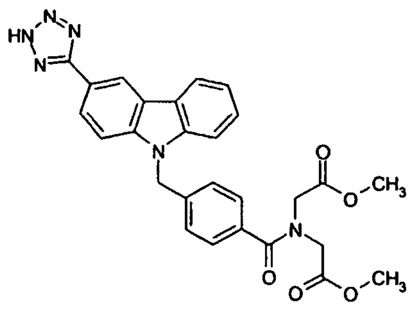

HPLC-MS (Method C): m/z = 513 (M+1); Rt = 3,55min.

Example 903 (General procedure (N))

2-{4-[3-(2H-Tetrazol-5-yl)carbazol-9-ylmethyl]benzoylamino}pentanedioic acid

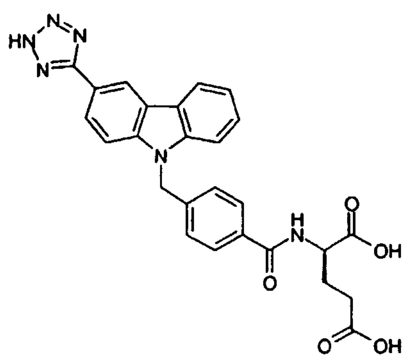

HPLC-MS (Method C): m/z = 499 (M+1); Rt = 2.87min.

Example 904 (General procedure (N))

(Ethoxycarbonylmethyl-{4-[3-(2H-tetrazol-5-yl)-carbazol-9-ylmethyl]-benzoyl}-amino)-acetic acid ethyl ester

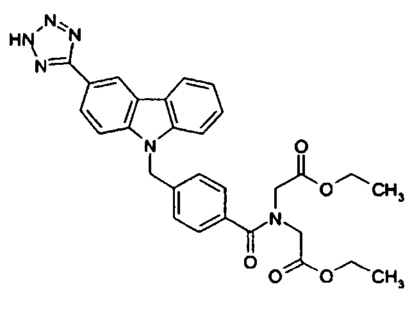

HPLC-MS (Method C): m/z = 541 (M+1); Rt = 3.91 min.

Example 905 (General procedure (N))

3-(3-{4-[4-(2,4-Dioxo-thiazolidin-5-ylidenemethyl)-naphthalen-1-yloxy]-butyrylamino}-propylamino)-hexanedioic acid dimethyl ester

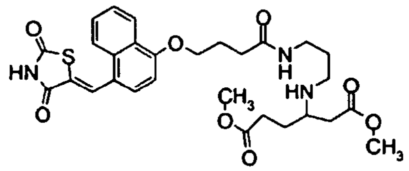

HPLC-MS (Method C: m/z = 585 (M+1); Rt = 2,81 min.

Example 906 (General procedure (N))

3-(3-{4-[4-(2,4-Dioxo-thiazolidin-5-ylidenemethyl)-naphthalen-1-yloxy]-butyrylamino}-propylamino)-hexanedioic acid

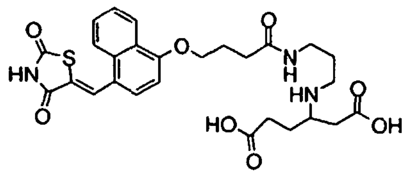

HPLC-MS (Method C): m/z = 554 (M-3); Rt = 3,19 min.

Example 907 (General procedure (N))

(Carboxymethyl-{4-[3-(2H-tetrazol-5-yl)-carbazol-9-ylmethyl]-benzoyl}-amino)-acetic acid

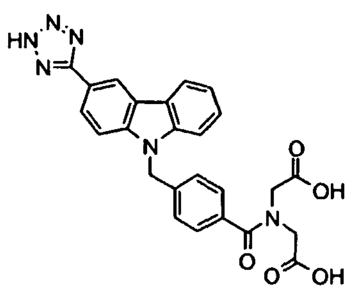

HPLC-MS (Method C): m/z = 485 (M+1); Rt = 3.04 min.

Example 908 (General procedure (N))

4-(3-{4-[4-(2,4-Dioxothiazolidin-5-ylidenemethyl)-naphthalen-1-yloxy]-butyrylamino}-propylamino)-cyclohexane-1,3-dicarboxylic acid dimethyl ester

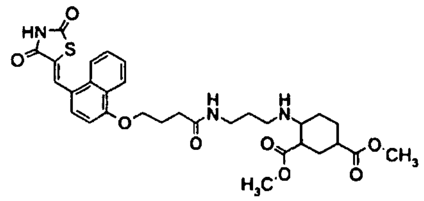

HPLC-MS (Method C): m/z = 612 (M+1); Rt = 3,24 min.

Example 909 (General procedure (N))

2-{3-[3-(2H-Tetrazol-5-yl)carbazol-9-ylmethyl]benzoylamino}pentanedioic acid dimethyl ester

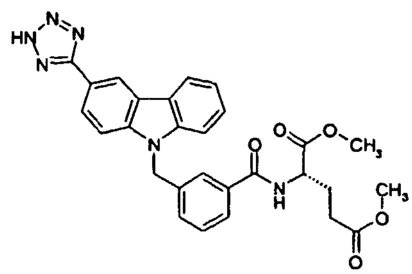

HPLC-MS (Method C): m/z = 527 (M+1); Rt = 3.65min.

Example 910 (General procedure (N))

2-{3-[3-(2H-Tetrazol-5-yl)carbazol-9-ylmethyl]benzoylamino}pentanedioic acid dimethyl ester

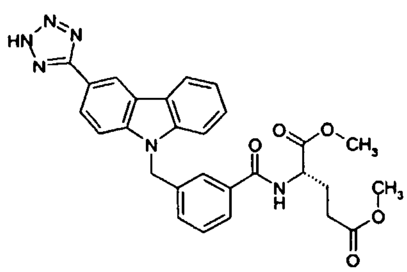

HPLC-MS (Method C): m/z = 527 (M+1); Rt = 3.65min.

Example 911 (General procedure (N))

2-{3-[3-(2H-Tetrazol-5-yl)carbazol-9-ylmethyl]benzoylamino}pentanedioic acid dimethyl ester

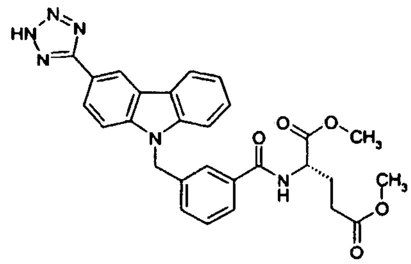

HPLC-MS (Method C): m/z = 527 (M+1); Rt = 3.65min.

Example 912 (General procedure (N))

2-{3-[3-(2H-Tetrazol-5-yl)carbazol-9-ylmethyl]benzoylamino}pentanedioic acid

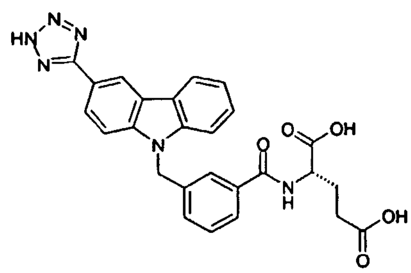

HPLC-MS (Method C): m/z = 499 (M+1); Rt = 3.00 min.

Example 913 (General procedure (N))

(Methoxycarbonylmethyl-{3-[3-(2H-tetrazol-5-yl)carbazol-9-ylmethyl]benzoyl}amino)acetic acid methyl ester

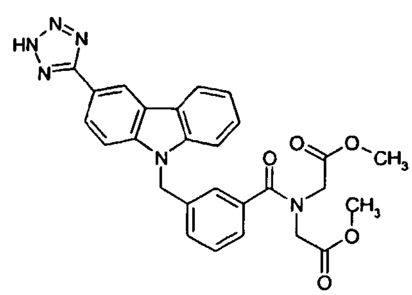

$^1$H-NMR (DMSO-$d_6$): δ 8.88 (1H, d), 8.29 (1H, d), 8.10 (1H, dd), 7.85 (1H, d), 7.67 (1H, d), 7.52 (1H, t), 7.39 (1H, t), 7.30 (2H, m), 7.17 (2H, m), 5.79 (2H, s), 4.17 (2H, s), 4.02 (2H, s), 3.62 (3H, s), 3.49 (3H, s).

Example 914 (General procedure (N))

2-(3-[3-(2H-Tetrazol-5-yl)carbazol-9-ylmethyl]benzoylamino}succinic acid dimethyl ester

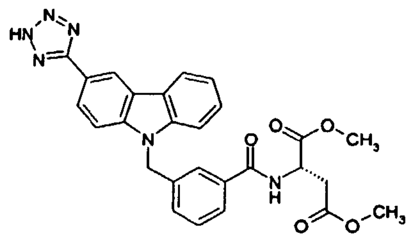

HPLC-MS (Method C): m/z = 513 (M+1); Rt = 3.70 min.

Example 915 (General procedure (N))

2-{3-[3-(2H-Tetrazol-5-yl)-carbazol-9-ylmethyl]-benzoylamino}-succinic acid

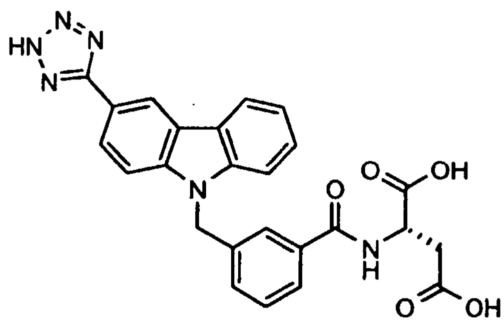

HPLC-MS (Method C): m/z = 485 (M+1); Rt = 2.96 min.

Example 916 (General procedure (N))

(Carboxymethyl-{3-[3-(2H-tetrazol-5-yl)carbazol-9-ylmethyl]benzoyl}amino)acetic acid

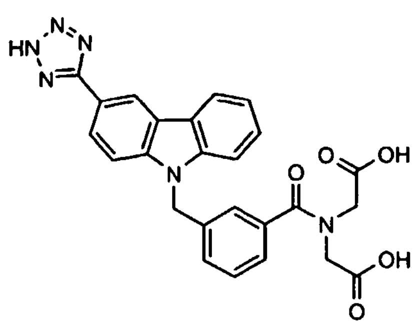

HPLC-MS (Method C): m/z = 485 (M+1); Rt = 2.87 min.

Example 917 (General procedure (N))

4-(4-(3-Carboxy-propylcarbamoyl)4-{4-[3-(2H-tetrazol-5-yl)carbazol-9-ylmethyl]-benzoylamino}-butyrylamino)-butyric acid

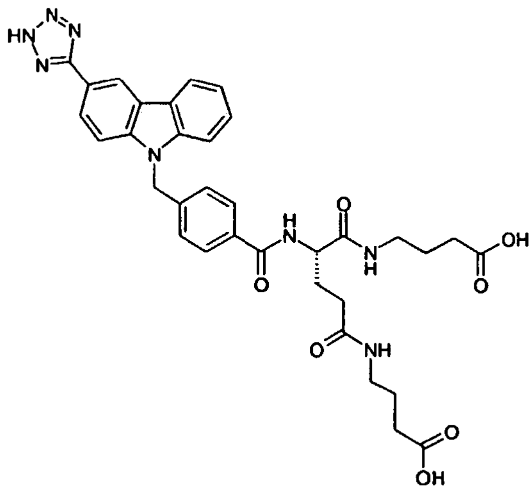

The title compound was prepared by coupling of (S)-2-{4-[3-(2H-tetrazol-5-yl)carbazol-9-ylmethyl]benzoylamino}pentanedioic acid bis-(2,5-dioxopyrrolidin-1-yl) ester (prepared from (S)-2-{4-[3-(2H-tetrazol-5-yl)carbazol-9-ylmethyl]benzoylamino}pentanedioic acid by essentially the same procedure as described for the synthesis of 4-[3-(2H-tetrazol-5-yl)carbazol-9-ylmethyl]benzoic acid 2,5-dioxopyrrolidin-1-yl ester) with 4-aminobutyric acid according to the procedure described for the preparation of 4-{4-[3-(2H-tetrazol-5-yl)carbazol-9-ylmethyl]benzoylamino}butyric acid. HPLC-MS (Method C): m/z: 669 (M+1); Rt = 2.84 min.

Example 918 (General procedure (N))

[2-(2-{4-[3-(2H-Tetrazol-5-yl)-carbazol-9-ylmethyl]benzoylamino}ethoxy)ethoxy]acetic acid

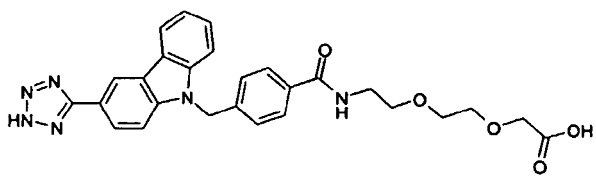

HPLC-MS (Method C): m/z: 515 (M+1); Rt = 3.10 min.

Example 919 (General procedure (N))

2-{4-[3-(2H-Tetrazol-5-yl)-carbazol-9-ylmethyl]-benzoylamino}-pentanedioic acid di-tert-butyl ester

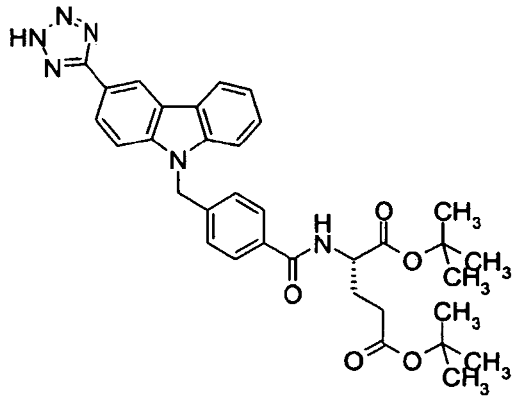

HPLC-MS (Method C): m/z = 611 (M+1); Rt = 4.64 min.

Example 920 (General Procedure (N)).

4-{4-[3-(2H-Tetrazol-5-yl)carbazol-9-ylmethyl]benzoylamino}butyric Acid

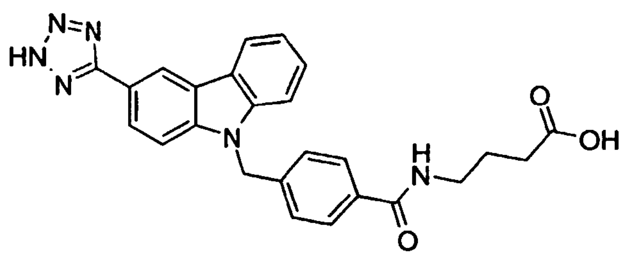

HPLC-MS (Method C): m/z: 455 (M+1); Rt = 3.13 min.

Example 921 (General Procedure (N)).

[2-(2-{4-[3-(2H-Tetrazol-5-yl)carbazol-9-ylmethyl]benzoylamino}ethoxy)ethoxy]acetic acid

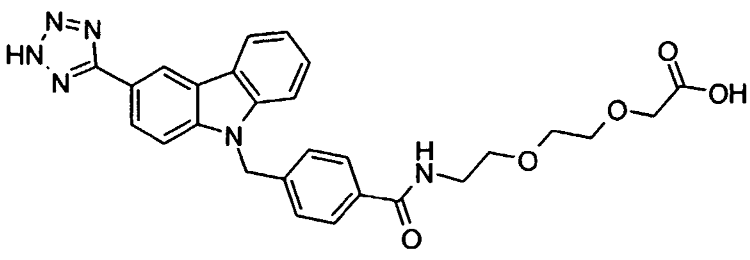

The title compound was prepared by coupling of 4-[3-(2H-tetrazol-5-yl)carbazol-9-ylmethyl]benzoic acid 2,5-dioxopyrrolidin-1-yl ester with [2-(2-aminoethoxy)ethoxy]acetic acid (prepared from [2-[2-(Fmoc-amino)ethoxy]ethoxy] acetic acid by treatment with PS-Trisamine resin in DMF).
HPLC-MS (Method C): m/z: 515 (M+1); Rt = 3.10 min.
The commercially available compounds in the following examples do all bind to the HisB10 $Zn^{2+}$site:

Example 922

1-(4-Bromo-3-methylphenyl)-1,4-dihydrotetrazole-5-thione

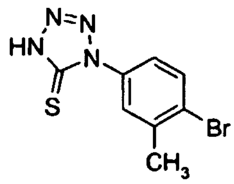

Example 923

1-(4-Iodophenyl)-1,4-dihydrotetrazole-5-thione

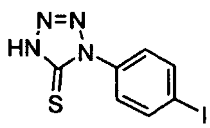

Example 924

1-(2,4,5-Trichlorophenyl)-1*H*-tetrazole-5-thiol

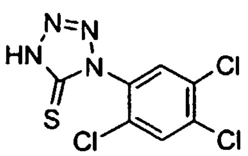

Example 925

1-(2,6-Dimethylphenyl)-1,4-dihydrotetrazole-5-thione

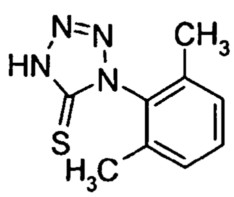

Example 926

1-(2,4,6-Trimethylphenyl)-1,4-dihydrotetrazole-5-thione

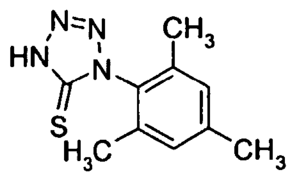

Example 927

1-(4-Dimethylaminophenyl)-1*H*-tetrazole-5-thiol

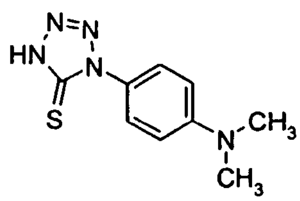

Example 928

1-(3,4-Dichlorophenyl)-1,4-dihydro-1*H*-tetrazole-5-thione

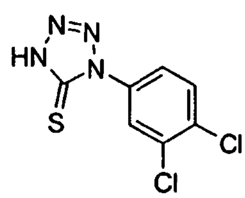

Example 929

1-(4-Propylphenyl)-1,4-dihydro-1H-tetrazole-5-thione

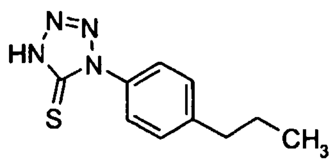

Example 930

1-(3-Chlorophenyl)-1,4-dihydro-1*H*-tetrazole-5-thione

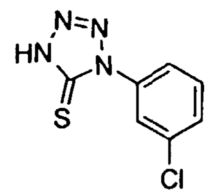

Example 931

1-(2-Fluorophenyl)-1,4-dihydro-1H-tetrazole-5-thione

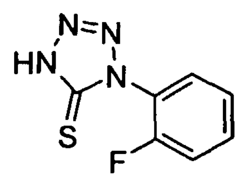

Example 932

1-(2,4-Dichlorophenyl)-1,4-dihydro-1*H*-tetrazole-5-thione

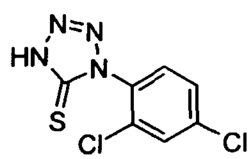

Example 933

1-(4-Trifluoromethoxyphenyl)-1,4-dihydro-1*H*-tetrazole-5-thione

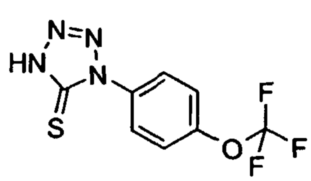

Example 934

N-[4-(5-Mercaptotetrazol-1-yl)-phenyl]-acetamide

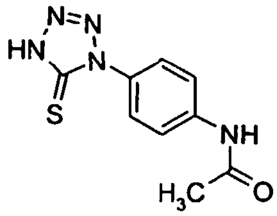

Example 935

1-(4-Chlorophenyl)-1,4-dihydrotetrazole-5-thione

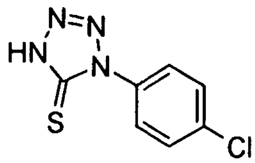

Example 936

1-(4-Methoxyphenyl)-1,4-dihydrotetrazole-5-thione

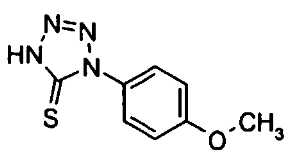

Example 937

1-(3-Fluoro-4-pyrrolidin-1-ylphenyl)-1,4-dihydrotetrazole-5-thione

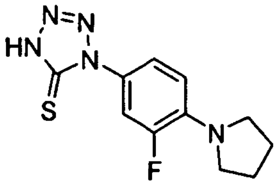

Example 938

*N*-[3-(5-Mercaptotetrazol-1-yl)phenyl]acetamide

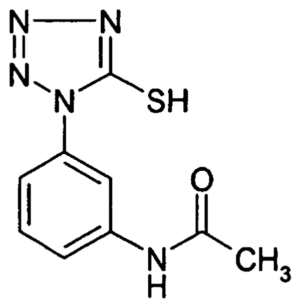

Example 939

1-(4-Hydroxyphenyl)-5-mercaptotetrazole

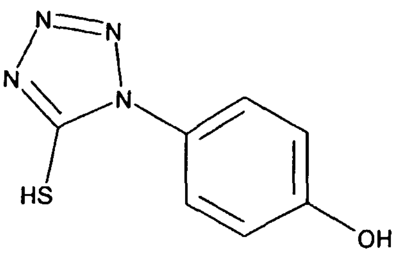

Example 940

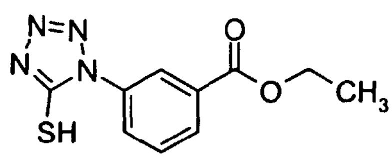

Preparation of 1-aryl-1,4-dihydrotetrazole-5-thiones (or the tautomeric 1-aryltetrazole-5-thiols) is described in the literature (eg. by Kauer & Sheppard, J. Org. Chem., 32, 3580-92 (1967)) and is generally performed eg. by reaction of aryl-isothiocyanates with sodium azide followed by acidification

1-Aryl-1,4-dihydrotetrazole-5-thiones with a carboxylic acid tethered to the aryl group may be prepared as shown in the following scheme:

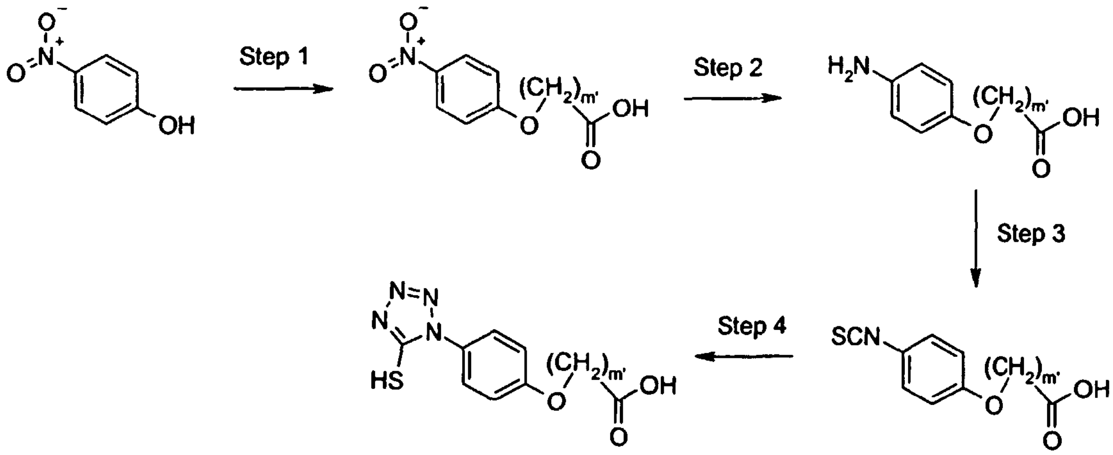


Step 1 is a phenol alkylation and is very similar to steps 1 and 2 of general procedure (D) and may also be prepared similarly as described in example 481.

Step 2 is a reduction of the nitro group. $SnCl_2$, $H_2$ over Pd/C and many other procedures known to those skilled in the art may be utilised.

Step 3 is formation of an arylisothiocyanate from the corresponding aniline. As reagents $CS_2$, $CSCl_2$, or other reagents known to those skilled in the art, may be utilised.

Step 4 is a conversion to mercaptotetrazole as described above.

Compounds of the invention include:

| | |
|---|---|
| Example 941 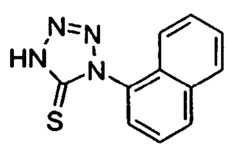 | Example 942 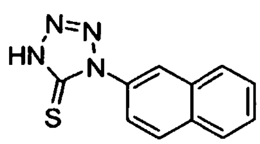 |
| Example 943 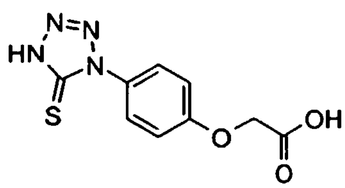 | Example 944 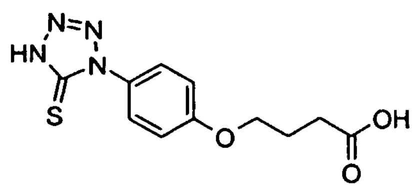 |
| Example 945 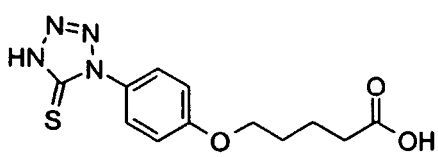 | Example 946 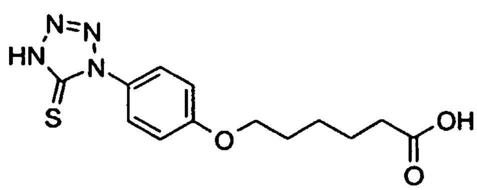 |
| Example 947 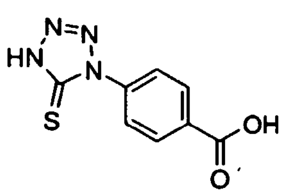 | |

Example 948

4-(4-Hydroxyphenyl)-1*H*-[1,2,3]triazole-5-carbonitrile

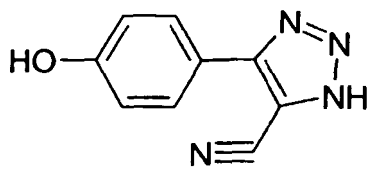

Phenylsulphonyl acetonitrile (2.0 g, 11.04 mmol) was mixed with 4-hydroxybenzaldehyde (1.35 g, 11.04 mmol) in DMF (10 mL) and toluene (20 mL). The mixture was refluxed for 3 hours and subsequently evaporated to dryness *in vacuo.* The residue was treated with diethyl ether and toluene. The solid formed was filtered to afford 2.08 g (66%) of 2-benzenesulfonyl-3-(4-hydroxyphenyl)acrylonitrile.
HPLC-MS (Method C): m/z: 286 (M+1); Rt. = 3.56 min.

A mixture of 2-benzenesulfonyl-3-(4-hydroxyphenyl)acrylonitrile (2.08 g, 7.3 mmol) and sodium azide (0.47g, 7.3 mmol) in DMF (50 mL) was heated at reflux temperature 2 hours. After cooling, the mixture was poured on ice. The mixture was evaporated in vacuo to almost dryness and toluene was added. After filtration, the organic phase was evaporated *in vacuo.* The residue was purified by silicagel chromatography eluting with a mixture of ethyl acetate and heptane (1:2). This afforded 1.2 g (76%) of the title compound.
1H NMR (DMSO-$d_6$): 10.2 (broad,1H); 7.74 (d,2H); 6.99 (d,2H); 3.6-3.2 (broad, 1H).
HPLC-MS (Method C) m/z: = 187 (M+1); Rt. = 1.93 min

**General procedure (O) for preparation of compounds of general formula $I_{14}$:**

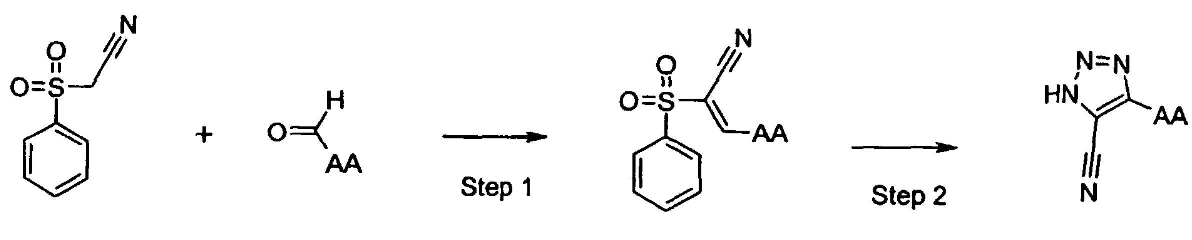


$I_{14}$

wherein
AA is as defined above,

Steps 1 and 2 are described in the literature (eg Beck & Gûnther, Chem. Ber., 106, 2758-66 (1973))

Step 1 is a Knoevenagel condensation of the aldehyde AA-CHO with phenylsulfonylacetonitrile and step 2 is a reaction of the vinylsulfonyl compound obtained in step 1 with sodium azide. This reaction is usually performed in DMF at 90 -110 °C.

This general procedure is further illustrated in the following example 949:

Example 949 (General Procedure (O))

[4-(5-Cyano-1*H*-[1,2,3]triazol-4-yl)phenoxy]acetic acid

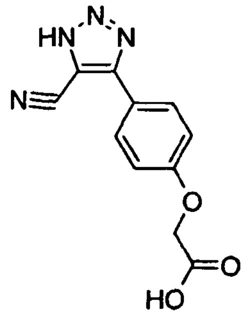

Phenylsulphonylacetonitrile (0.1 g, 0.55 mmol) was mixed with 4-formylphenoxyactic acid (0.099 g, 0.55 mmol) in DMF (3 mL) and heated to 110 ˚C for 3 h and subsequently cooled to RT. Sodium azide (0.036 g, 0.55 mmol) was added and the resulting mixture was heated to 110 ˚C for 3 h and cooled to RT. The mixture was poured into water (20 mL) and centrifuged. The supernatant was discarded, ethanol (5 mL) was added and the mixture was centrifuged again. After discarding the supernatant, the residue was dried *in vacuo* to afford 50 mg (37%) of [4-(5-Cyano-1*H*[1,2,3]triazol-4-yl)phenoxy]acetic acid.
HPLC-MS (Method C): m/z: 245 (M+1) Rt. 2.19 min.

Example 950 (General Procedure (O))

5-(Naphthalen-1-yl)-3*H*-[1,2,3]triazole-4-carbonitrile

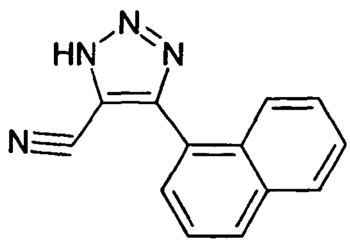

HPLC-MS (Method C): m/z: 221 (M+1); Rt. 3.43 min.

Example 951 (General Procedure (O))

5-(Naphthalen-2-yl)-3*H*-[1,2,3]triazole-4-carbonitrile

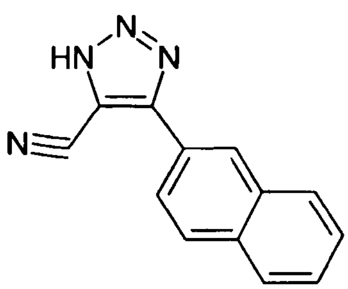

HPLC-MS (Method C): m/z: 221 (M+1); Rt = 3.66 min.

Example 952 (General procedure (O))

4-[3-(5-Cyano-[1,2,3]triazol-4-yl)-1,4-dimethylcarbazol-9-ylmethyl]-benzoic acid

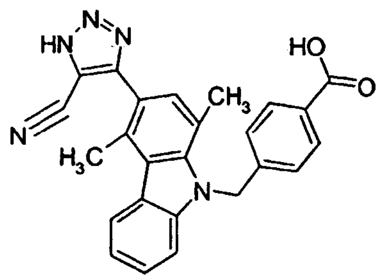

HPLC-MS (Method C): m/z = 422 (M+1); Rt = 3.85 min.

Preparation of intermediary aldehyde:

1,4 Dimethylcarbazol-3-carbaldehyde (0.68 g, 3.08 mmol) was dissolved in dry DMF (15 mL), NaH (diethyl ether washed) (0.162 g, 6.7 mol) was slowly added under nitrogen and the mixture was stirred for 1 hour at room temperature. 4-Bromomethylbenzoic acid (0.73 g, 3.4 mmol) was slowly added and the resulting slurry was heated to 40 °C for 16 hours. Water (5 mL) and hydrochloric acid (6N, 3 mL) were added. After stirring for 20 min at room temperature, the precipitate was filtered off and washed twice with acetone to afford after drying 0.38 g (34%) of 4-(3-formyl-1,4-dimethylcarbazol-9-ylmethyl)benzoic acid.
HPLC-MS (Method C) : m/z = 358 (M+1), RT. = 4.15 min.

Example 953 (General Procedure (O))

5-(Anthracen-9-yl)-3*H*-[1,2,3]triazole-4-carbonitrile

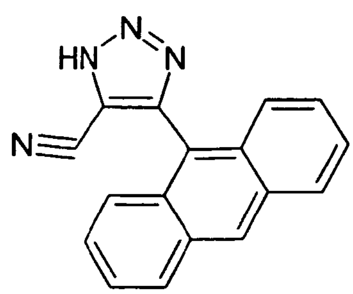

HPLC-MS (Method C): m/z: 271 (M+1); Rt = 3.87 min.

Example 954 (General Procedure (O))

5-(4-Methoxynaphthalen-1-yl)-3*H*-[1,2,3]triazole-4-carbonitrile

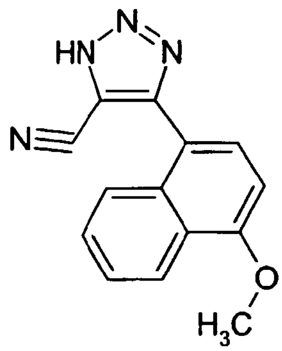

HPLC-MS (Method C): m/z: 251 (M+1); Rt = 3.57 min.

Example 955 (General Procedure (O))

5-(1,4-Dimethyl-9*H*-carbazol-3-yl)-3*H*-[1,2,3]triazole-4-carbonitrile

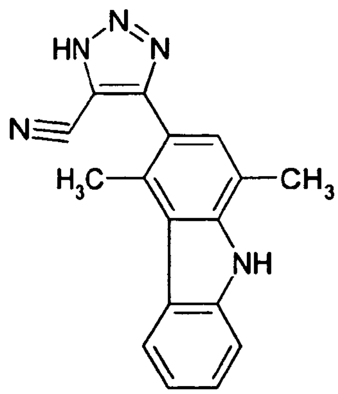

HPLC-MS (Method C): m/z: 288 (M+1); Rt = 3.67 min.

Example 956 (General procedure (O))

5-(4'-Methoxybiphenyl-4-yl)-3H-[1,2,3]triazole-4-carbonitrile

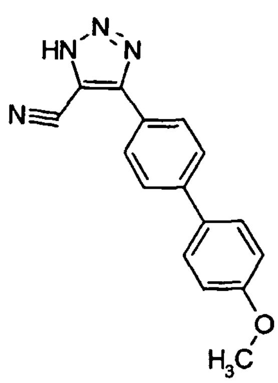

HPLC-MS (Method C): m/z = 277 (M+1); Rt = 3.60 min.

Example 957 (General procedure (O)) .

5-(4-Styrylphenyl)-3H-[1,2,3]triazole-4-carbonitrile

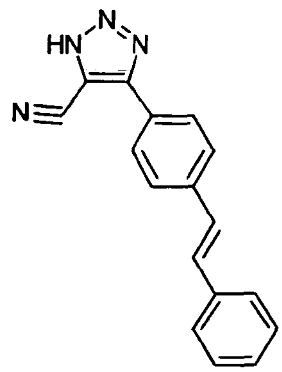

HPLC-MS (Method C): m/z = 273 (M+1); Rt = 4.12 min.

Example 958 (General procedure (O))

5-(2,6-Dichloro-4-dibenzylaminophenyl)-3H-[1,2,3]triazole-4-carbonitrile

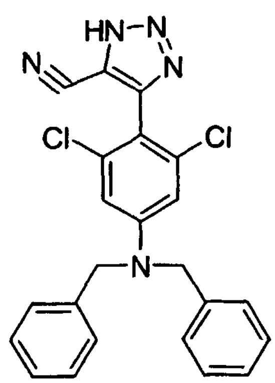

HPLC-MS (Method C): m/z = 434 (M+1); Rt = 4.64 min.

Example 959 (General procedure (O))

5-(1-Bromonaphthalen-2-yl)-3H-[1,2,3]triazole-4-carbonitrile

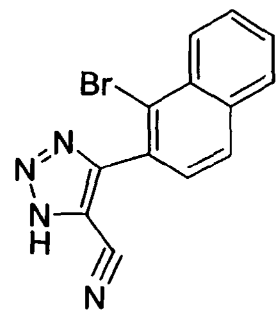

HPLC-MS (Method C: m/z = 300 (M+1); Rt. = 3.79 min.

Example 960

4-(4-Bromophenyl)-1H-[1,2,3]triazole-5-carbonitrile

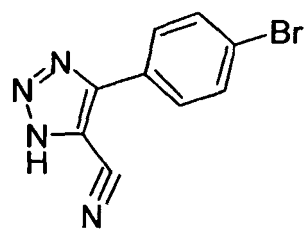

This compound is commercially available (MENAI).

Example 961

N-[4-(5-Cyano-1H-[1,2,3]triazol-4-yl)-phenyl]-acetamide

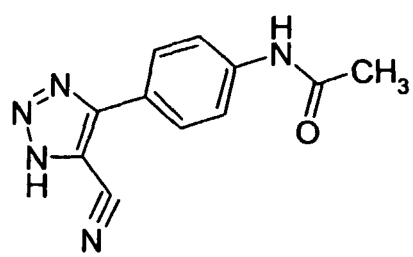

This compound is commercially available (MENAI).

Example 962 (General procedure (O))

5-(4'-Chlorobiphenyl-4-yl)-3H-[1,2,3]triazole-4-carbonitrile

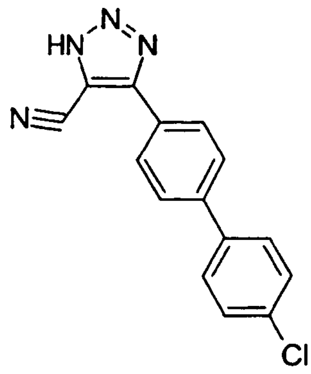

HPLC-MS (Method C): m/z = 281 (M+1); Rt = 4.22 min.

The compounds in the following examples are commercially available and may be prepared using a similar methodology:

Example 963

4-(4-Trifluoromethoxyphenyl)-1*H*-[1,2,3]triazole-5-carbonitrile

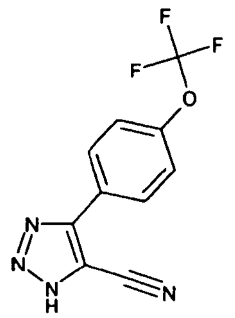

Example 964

4-Benzo[1,3]dioxol-5-yl-1*H*-[1,2,3]triazole-5-carbonitrile

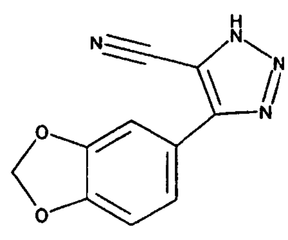

Example 965

4-(3-Trifluoromethylphenyl)-1*H*-[1,2,3]triazole-5-carbonitrile

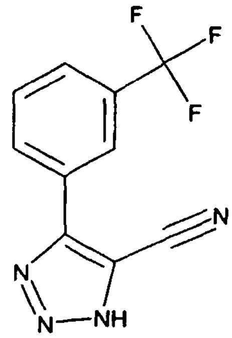

Example 966

4-Pyridin-3-yl-1*H*-[1,2,3]triazole-5-carbonitrile

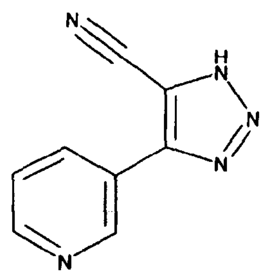

Example 967

4-(2,6-Dichlorophenyl)-1*H*-[1,2,3]triazole-5-carbonitrile

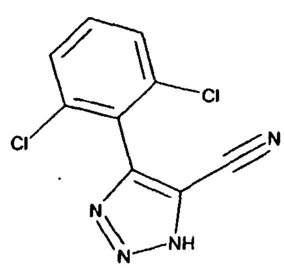

Example 968

4-Thiophen-2-yl-1*H*-[1,2,3]triazole-5-carbonitrile

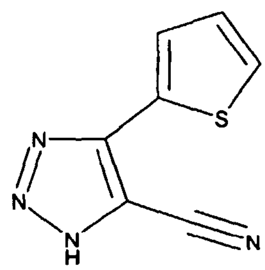

Example 969

3,5-Dimethylisoxazole-4-carboxylic acid 4-(5-cyano-1*H*-[1,2,3]triazol-4-yl)phenyl ester

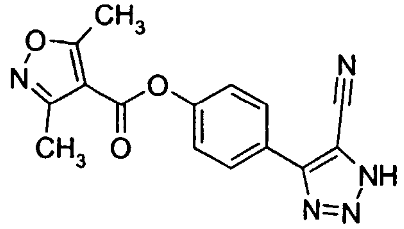

Example 970

3,3-Dimethyl-butyric acid 4-(5-cyano-1*H*-[1,2,3]triazol-4-yl)phenyl ester

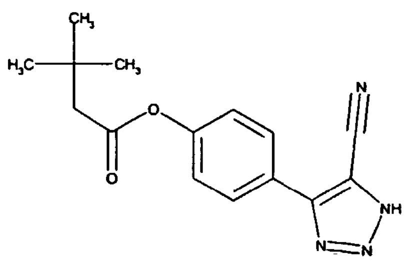

Example 971

4-Methyl-[1,2,3]thiadiazole-5-carboxylic acid 4-(5-cyano-1*H*-[1,2,3]triazol-4-yl)phenyl ester

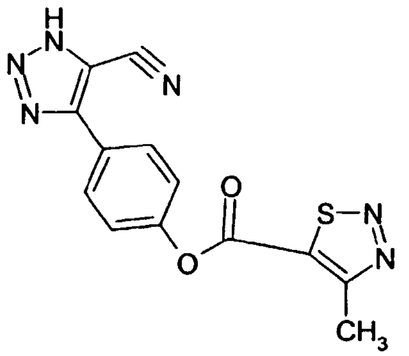

Example 972

4-Chlorobenzoic acid 4-(5-cyano-1*H*-[1,2,3]triazol-4-yl)phenyl ester

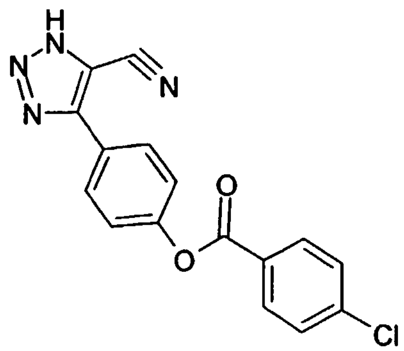

Example 973

4-(3-Phenoxyphenyl)-1*H*-[1,2,3]triazole-5-carbonitrile

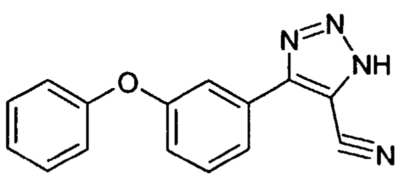

Example 974

4-(5-Bromo-2-methoxyphenyl)-1*H*-[1,2,3]triazole-5-carbonitrile

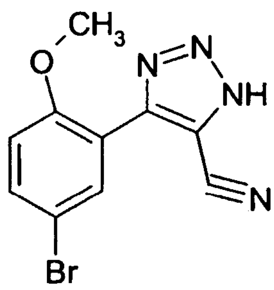

Example 975

4-(2-Chloro-6-fluorophenyl)-1*H*-[1,2,3]triazole-5-carbonitrile

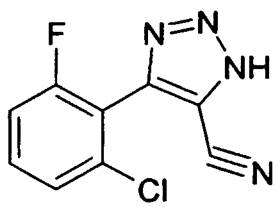

The following cyanotriazoles are also compounds of the invention:

4-(2-Chloro-6-fluorophenyl)-1*H*-[1,2,3]triazole-5-carbonitrile.
Terephthalic acid mono[4-(5-cyano-1*H*-[1,2,3]triazol-4-yl)phenyl] ester.
*N*-[4-(5-cyano-1*H*-[1,2,3]triazol-4-yl)-phenyl]terephthalamic acid
4-(4-Octyloxyphenyl)-1*H*-[1,2,3]triazole-5-carbonitrile
4-(4-Styrylphenyl)-1*H*-[1,2,3]triazole-5-carbonitrile.
4-(4'-Trifluoromethylbiphenyl-4-yl)-1*H*-[1,2,3]triazole-5-carbonitrile.
4-(4'-Chlorobiphenyl-4-yl)-1*H*-[1,2,3]triazole-5-carbonitrile.
4-(4'-Methoxybiphenyl-4-yl)-1*H*-[1,2,3]triazole-5-carbonitrile.
4-(1-Naphthyl)-1*H*-[1,2,3]triazole-5-carbonitrile.
4-(9-Anthranyl)-1*H*-[1,2,3]triazole-5-carbonitrile.
4-(4-Methoxy-1-naphthyl)-1*H*-[1,2,3]triazole-5-carbonitrile.
4-(4-Aminophenyl)-1*H*-[1,2,3]triazole-5-carbonitrile.
4-(2-Naphthyl)-1*H*-[1,2,3]triazole-5-carbonitrile.

**General procedure (P) for preparation of compounds of general formula $I_{15}$:**

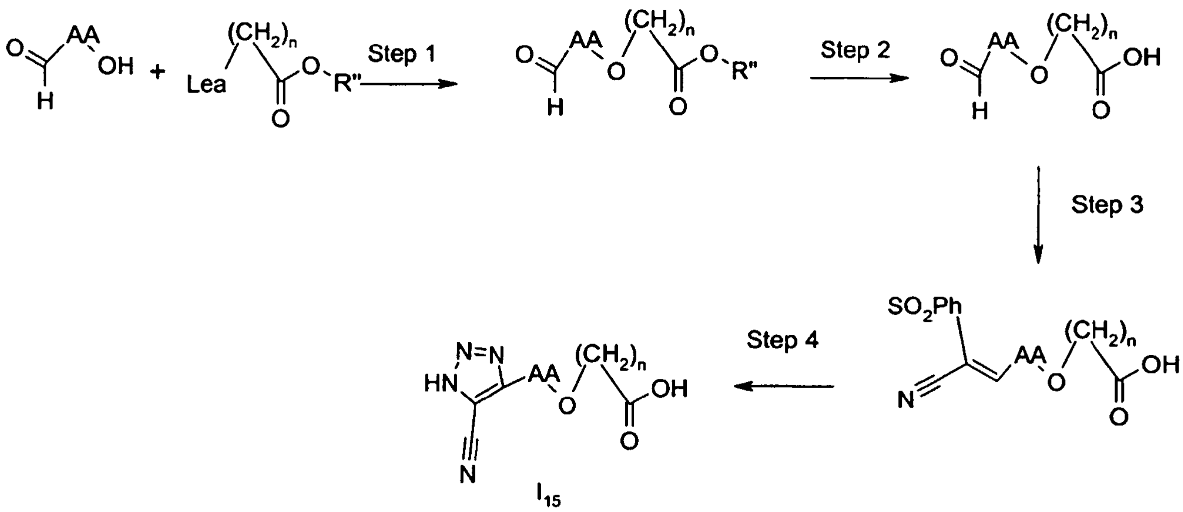


$I_{15}$

wherein

n is 1 or 3-20,
AA is as defined above,
R" is a standard carboxylic acid protecting group, such as $C_1$-$C_6$-alkyl or benzyl and Lea is a leaving group, such as chloro, bromo, iodo, methanesulfonyloxy, toluenesulfonyloxy or the like.

This procedure is very similar to general procedure (D), steps 1 and 2 are identical.

Steps 3 and 4 are described in the literature (eg Beck & Gûnther, Chem. Ber., 106, 2758-66 (1973))

Step 3 is a Knoevenagel condensation of the aldehyde obtained in step 2 with phenylsulfonylacetonitrile and step 4 is a reaction of the vinylsulfonyl compound obtained in step 3 with sodium azide. This reaction is usually performed in DMF at 90-110 ˚C.

This General procedure (P) is further illustrated in the following two examples

Example 976 (General procedure (P))

5-[6-(5-Cyano-1H-[1,2,3]triazol-4-yl)-naphthalen-2-yloxy]-pentanoic acid ethyl ester

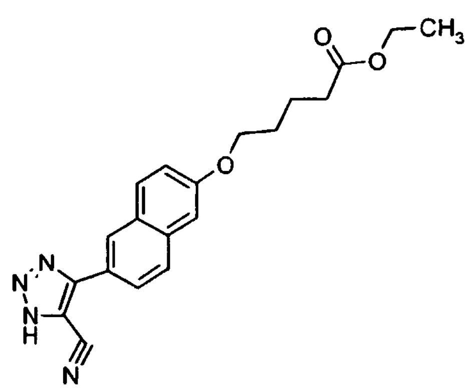

6-Hydroxynaphthalene-2-carbaldehyde (Syncom BV. NL, 15.5 g, 90 mmol) and $K_2CO_3$ (62.2 g, 450 mmol) were mixed in DMF (300mL) and stirred at room temperature for 1 hour. Ethyl 5-bromovalerate (21.65 g, 103.5 mmol) was added and the mixture was stirred at room temperature for 16 hours. Activated carbon was added and the mixture

was filtered. The filtrate was evaporated to dryness *in vacuo* to afford 28.4 g of crude 5-(6-formylnaphthalen-2-yloxy) pentanoic acid ethyl ester, which was used without further purification.
HPLC-MS (Method C): m/z = 301 (M+1); Rt. = 4.39 min.

5-(6-Formylnaphthalen-2-yloxy)pentanoic acid ethyl ester (28.4 g, 94.5 mmol), phenylsulfonylacetonitrile (20.6 g, 113.5 mmol), and piperidine (0.94 mL) were dissolved in DMF (200 mL) and the mixture was heated at 50 ˚C for 16 hours. The resulting mixture was evaporated to dryness *in vacuo* and the residue was dried for 16 hours at 40 ˚C *in vacuo.* The solid was recrystallised from 2-propanol (800 mL) and dried again as described above. This afforded 35 g (80%) of 5-[6-(2-benzenesulfonyl-2-cyanovinyl)naphthalen-2-yloxy]pentanoic acid ethyl ester.
HPLC-MS (Method C): m/z = 486 (M+23); Rt. = 5.09 min.

5-[6-(2-Benzenesulfonyl-2-cyanovinyl)naphthalen-2-yloxy]pentanoic acid ethyl ester (35 g, 74.6 mmol) and sodium azide (4.9 g, 75.6 mmol) were dissolved in DMF (100 mL) and stirred for 16 hours at 50 ˚C. The mixture was evaporated to dryness *in vacuo,* redissolved in THF /ethanol and a small amount of precipitate was filtered off. The resulting filtrate was poured into water (2.5 L). Filtration afforded after drying 24.5 g (88%) of 5-[6-(5-cyano-1*H*-[1,2,3] triazol-4-yl)naphthalen-2-yloxy]pentanoic acid ethyl ester (24.5 g, 88%).
HPLC-MS (Method C): m/z = 365 (M+1); Rt. = 4.36 min.

Example 977 (General procedure (B))

5-[6-(5-Cyano-1H-[1,2,3]triazol-4-yl)-naphthalen-2-yloxy]-pentanoic acid

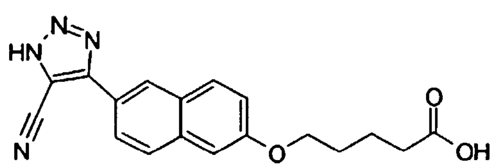

5-[6-(5-Cyano-1*H*-[1,2,3]triazol-4-yl)naphthalen-2-yloxy]pentanoicacid ethyl ester (24.5 g, 67.4 mmol) was dissolved in THF (150 mL) and mixed with sodium hydroxide (8.1 g, 202 mmol) dissolved in water (50 mL). The mixture was stirred for 2 days and the volatiles were evaporated *in vacuo.* The resulting aqueous solution was poured into a mixture of water (1 L) and hydrochloric acid (1N, 250 mL). The solid was isolated by filtration, dissolved in sodium hydroxide (1N, 200 mL), and the solution was washed with DCM and then ethyl acetate, the aquous layer was acidified with hydrochloric acid (12N). The precipitate was isolated by filtration, dissolved in THF / diethyl ether, the solution was treated with $MgSO_4$ and activated carbon, filtrated and evaporated *in vacuo* to almost dryness followed by precipitation by addition of pentane (1L). This afforded after drying *in vacuo* 17.2 g ( 76%) of the title compound.
HPLC-MS (Method C): m/z = 337 (M+1); Rt. = 3.49 min.

Example 978 (General procedure (P))

6-[6-(5-Cyano-1H-[1,2,3]triazol-4-yl)naphthalen-2-yloxy]hexanoic acid

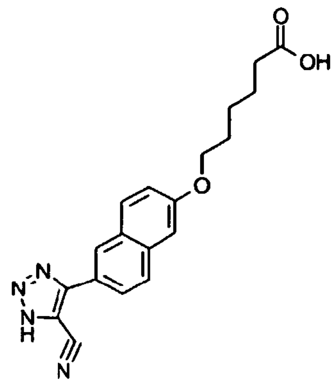

HPLC-MS (Method C): m/z = 351 (M+1); Rt = 3.68 min.

Example 979 (General procedure (P))

11-[6-(5-Cyano-1H-[1,2,3]triazol-4-yl)-naphthalen-2-yloxy]-undecanoic acid

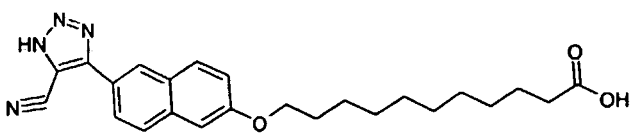

HPLC-MS (Method C): m/z = 443 (M+23); Rt = 4.92 min.

Example 980 (General procedure (P))

2-{3-[6-(5-Cyano-1H-[1,2,3]triazol-4-yl)-naphthalen-2-yloxy]-propyl}-malonic acid diethyl ester

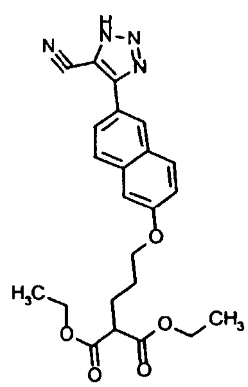

HPLC-MS (Method C): m/z = 465 (M+1); Rt. = 4.95 min.

Example 981 (General procedure (P))

2-{5-[6-(5-Cyano-1H-[1,2,3]triazol-4-yl)-naphthalen-2-yloxy]-pentyl}-malonic acid diethyl ester

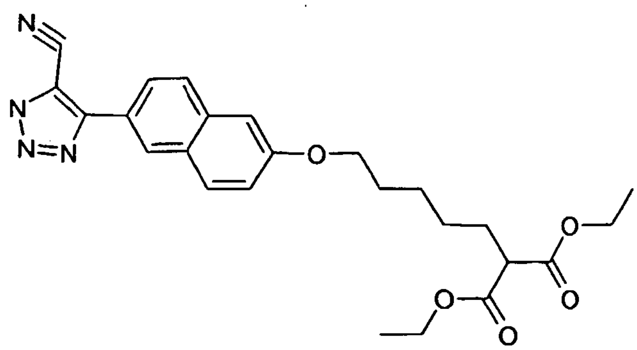

HPLC-MS (Method C): m/z = 465 (M+1); Rt. = 4.95 min.

Example 982 (General procedure (P))

2-{3-[6-(5-Cyano-1H-[1,2,3]triazol-4-yl)-naphthalen-2-yloxy]-propyl}-malonic acid

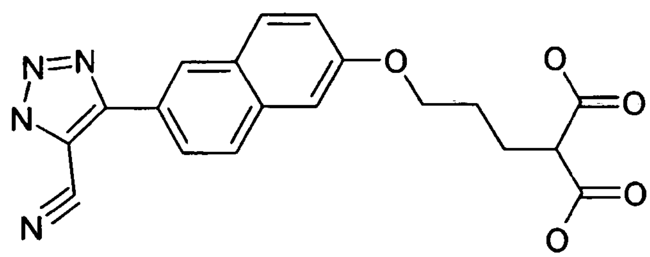

HPLC-MS (Method C): m/z = 381 (M+1); Rt. = 3.12 min.

Example 983 (General procedure (P))

2-{5-[6-(5-Cyano-1H-[1,2,3]triazol-4-yl)-naphthalen-2-yloxy]-pentyl}-malonic acid

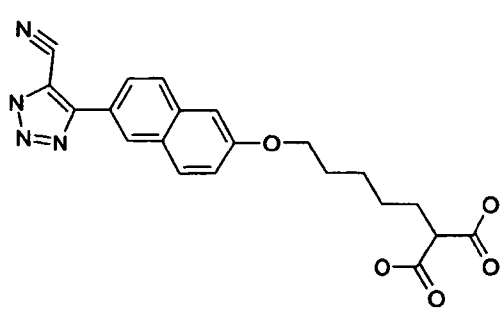

HPLC-MS (Method C): m/z 0 409 (M+1); Rt. = 3.51 min.

Example 984 (General procedure (P))

4-[4-(5-Cyano-1H-[1,2,3]triazol-4-yl)-phenoxy]butyric acid

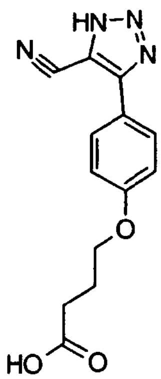

HPLC-MS (Method C): m/z = 273 (M+1); Rt = 2.44 min.

The following compounds may be prepared according to this general procedure (P):

4-(4-(5-Cyano-1*H*-[1,2,3]triazol-4-yl)phenoxy)butyric acid:

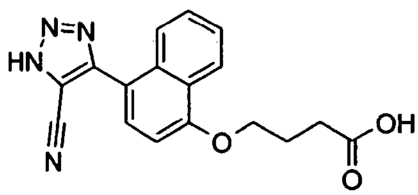

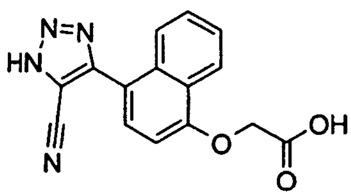

2-(4-(5-Cyano-1*H*-[1,2,3]triazol-4-yl)phenoxy)acetic acid:

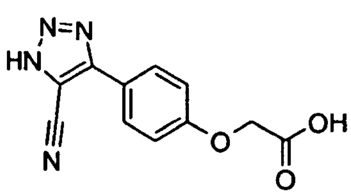

4-(4-(5-Cyano-1*H*-[1,2,3]triazol-4-yl)phenoxy)butyric acid ethyl ester
5-(4-(5-Cyano-1*H*-[1,2,3]triazol-4-yl)phenoxy)pentanoic acid
8-(4-(5-Cyano-1*H*-[1,2,3]triazol-4-yl)phenoxy)octanoic acid
10-(4-(5-Cyano-1*H*-[1,2,3]triazol-4-yl)phenoxy)decanoic acid

12-(4-(5-Cyano-1*H*-[1,2,3]triazol-4-yl)phenoxy)dodecanoic acid

**General procedure (R) for preparation of compounds of general formula $I_{12}$:**

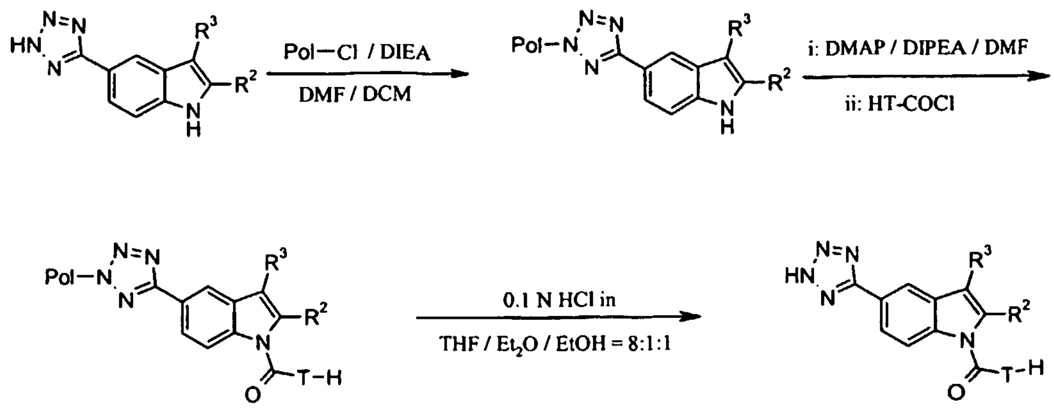


$I_{12}$

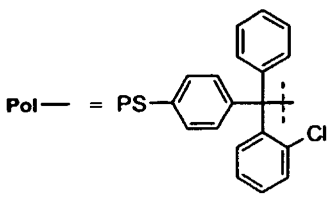

wherein T is as defined above and $R^2$ and $R^3$ are hydrogen, aryl or lower alkyl, both optionally substituted.

The general procedure (R) is further illustrated by the following example:

Example 985 (General procedure (R))

Phenyl-[3-(2H-tetrazol-5-yl)-carbazol-9-yl]-methanone

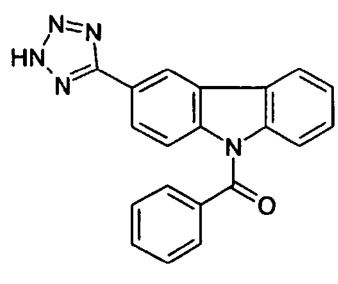

2-Chlorotritylchloride resin (100 mg, 0.114 mmol active chloride) was swelled in dichloromethane (4 mL) for 30 minutes. The solvent was drained, and a solution of 3-(2H-tetrazol-5-yl)-9H-carbazole (80 mg, 0.34 mmol) in a mixture of N,N-dimethylformamide /dichloromethane / N,N-di(2-propyl)ethylamine (5:5:1) (3 mL) was added. The reaction mixture was shaken at room temperature for 20 hours. The solvent was removed by filtration, and the resin was washed thoroughly with N,N-dimethylformamide (2 x 4 mL) and dichloromethane (6 x 4 mL). A solution of 4-(dimethylamino)pyridine (14 mg, 0.11 mmol) and N,N-di(2-propyl)ethylamine (0.23 mL, 171 mg, 1.32 mmol) in N,N-dimethylformamide (2 mL) was added followed by benzoyl chloride (0.13 mL, 157 mg, 1.12 mmol). The mixture was shaken for 48 hours at room

temperature. The drained resin was washed consecutively with dichloromethane (2 x 4 mL), methanol (2 x 4 mL) and tetrahydrofuran (4 mL). The resin was treated for 2 hours at room temperature with a solution of dry hydrogen chloride in tetrahydrofuran / ethyl ether / ethanol = 8:1:1 (0.1 M, 3 mL). The reaction mixture was drained and concentrated. The crude product was stripped with dichloromethane (1.5 mL) three times to yield the title compound.
HPLC-MS (Method C): m/z: 340 (M+1); Rt = 3.68 min.
$^1$H-NMR (DMSO-$d_6$): δ 8.91 (1H, s), 8.34 (1H, d), 8.05 (1H, d), 7.78 (3H, m), 7.63 (3H, m), 7.46 (2H, m), 7.33 (1H, dd).

The compounds in the following examples were prepared in a similar fashion.

Example 986 (General procedure (R))

Phenyl-[5-(2H-tetrazol-5-yl)-indol-1-yl]-methanone

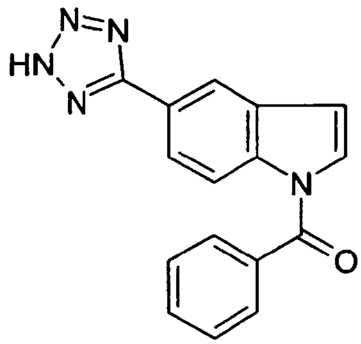

HPLC-MS (Method C): m/z: 290 (M+1); Rt = 3.04 min.
$^1$H-NMR (DMSO-$d_6$): δ 8.46 (1H, d), 8.42 (1H, d), 8.08 (1H, dd), 7.82 (2H, d), 7.74 (1H, t), 7.64 (2H, t), 7.55 (1H, d), 6.93 (1H, d).

Example 987 (General procedure (R))

(2,3-Difluorophenyl)-[5-(2H-tetrazol-5-yl)-indol-1-yl]-methanone

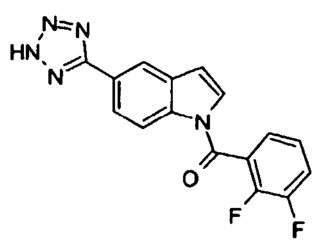

HPLC-MS (Method B): m/z = 326 (M+1); Rt = 3.85 min.

Example 988 (General procedure (R))

(2-Fluoro-3-trifluoromethylphenyl)-[5-(2H-tetrazol-5-yl)-indol-1-yl]-methanone

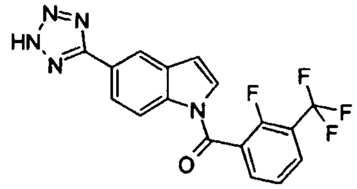

HPLC-MS (Method B): m/z = 376 (M+1); Rt = 4.32 min.

Example 989 (General procedure (R))

(3-Nitrophenyl)-[5-(2H-tetrazol-5-yl)-indol-1-yl]-methanone

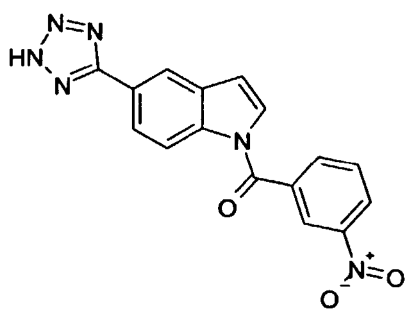

HPLC-MS (Method B): m/z = 335 (M+1); Rt = 3.72 min.

Example 990 (General procedure (R))

(4-Nitrophenyl)-[5-(2H-tetrazol-5-yl)-indol-1-yl]-methanone

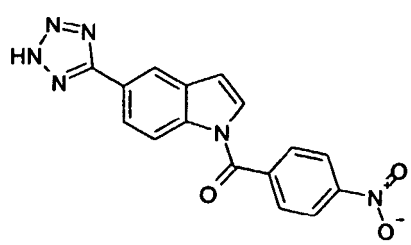

HPLC-MS (Method B): m/z = 335 (M+1); Rt = 3.71 min.

Example 991 (General procedure (R))

Naphthalen-2-yl-[5-(2H-tetrazol-5-yl)-indol-1-yl]-methanone

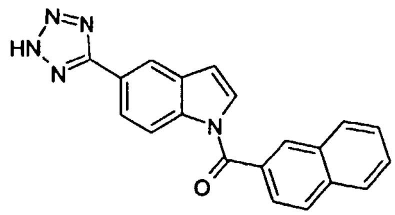

HPLC-MS (Method C): m/z = 340 (M+1); Rt = 4.25 min.

Example 992 (General procedure (R))

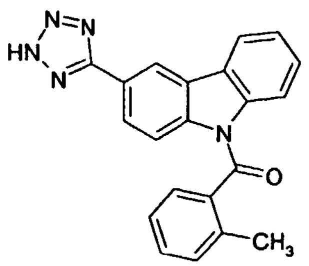

HPLC-MS (Method C): m/z: 354 (M+1); Rt = 3.91 min.

Example 993 (General procedure (R))

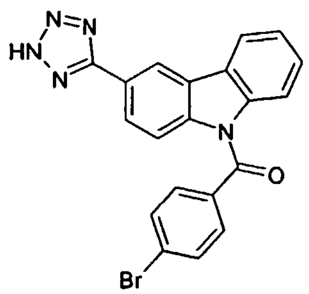

HPLC-MS (Method C): m/z: 418 (M+1); Rt = 4.39 min.

Example 994 (General procedure (R))

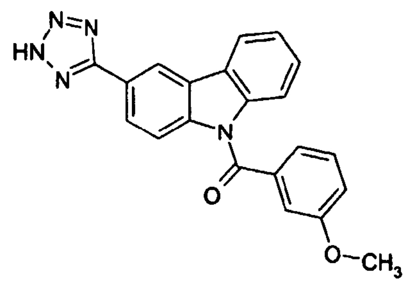

HPLC-MS (Method C): m/z: 370 (M+1); Rt = 4.01 min.

Example 995 (General procedure (R))

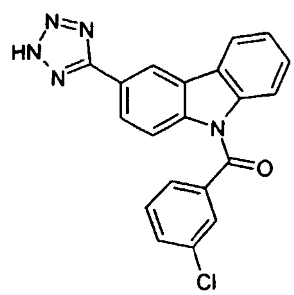

HPLC-MS (Method C): m/z: 374 (M+1); Rt = 4.28 min.

Example 996 (General procedure (R))

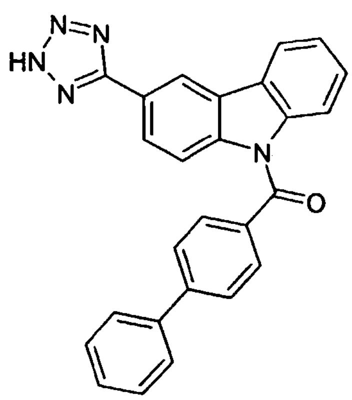

HPLC-MS (Method C): m/z: 416 (M+1); Rt = 4.55 min.

Example 997 (General procedure (R))

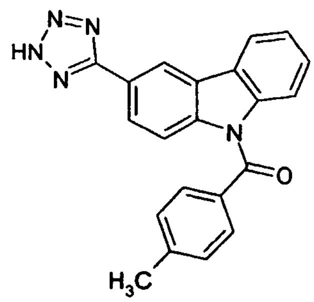

HPLC-MS (Method C): m/z: 354 (M+1); Rt = 4.22 min.

Example 998 (General procedure (R))

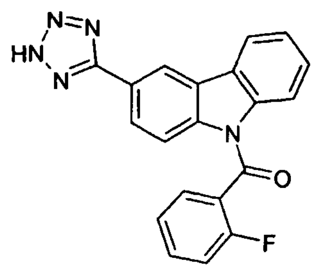

HPLC-MS (Method C): m/z: 358 (M+1); Rt = 3.91 min.

Example 999 (General procedure (R))

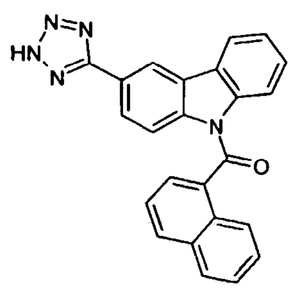

HPLC-MS (Method C): m/z: 390 (M+1); Rt = 4.38 min.

Example 1000 (General procedure (R))

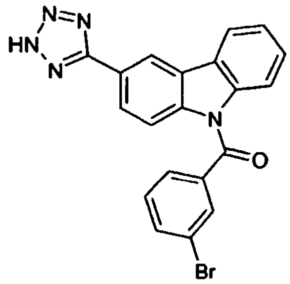

HPLC-MS (Method C): m/z: 418 (M+1); Rt = 4.36 min.

Example 1001 (General procedure (R))

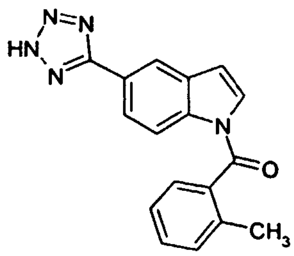

HPLC-MS (Method C): m/z: 304 (M+1); Rt = 3.32 min.

Example 1002 (General procedure (R))

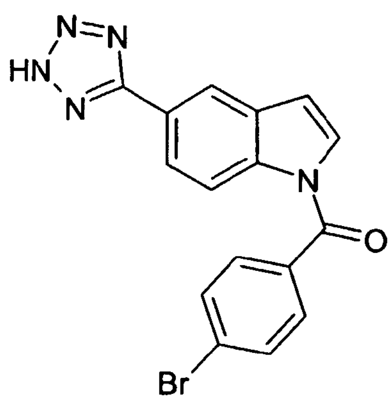

HPLC-MS (Method C): m/z: 368 (M+1); Rt = 3.84 min.

Example 1003 (General procedure (R))

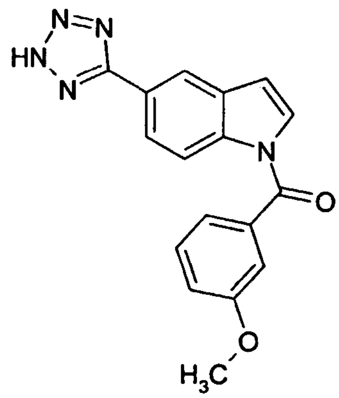

HPLC-MS (Method C): m/z: 320 (M+1); Rt = 3.44 min.

Example 1004 (General procedure (R))

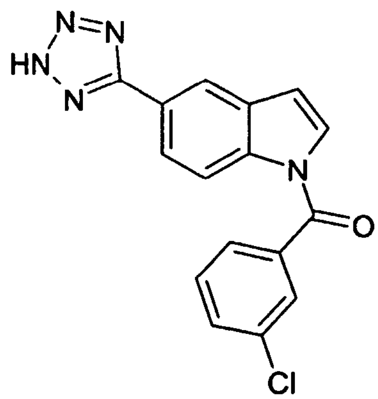

HPLC-MS (Method C): m/z: 324 (M+1); Rt = 3.73 min.

Example 1005 (General procedure (R))

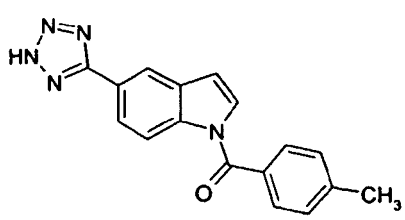

HPLC-MS (Method C): m/z: 304 (M+1); Rt = 3.64 min.

Example 1006 (General procedure (R))

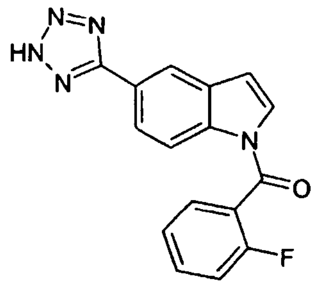

HPLC-MS (Method A): m/z: 308 (M+1); Rt = 3.61 min.

Example 1007 (General procedure (R))

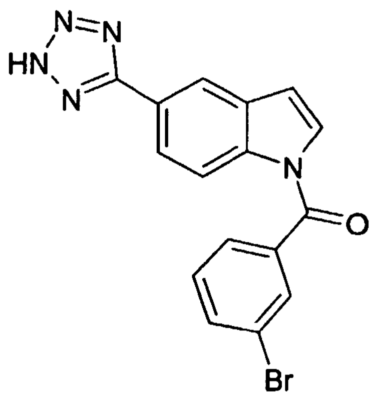

HPLC-MS (Method C): m/z: 368 (M+1); Rt = 3.77 min.

Example 1008 (General procedure (R))

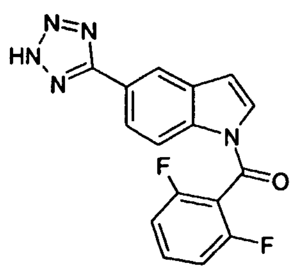

HPLC-MS (Method A): (sciex) m/z: 326 (M+1); Rt = 3.73 min.
HPLC-MS (Method C): m/z: 326 (M+1); Rt = 3.37 min.

Example 1009 (General procedure (R))

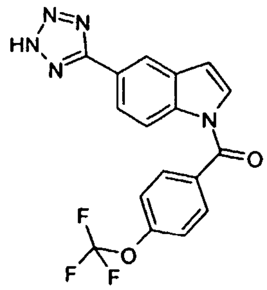

HPLC-MS (Method C): m/z: 374 (M+1); Rt = 4.03 min.

Example 1010 Characterization of ligand effects on physical stability of formulations by the Thioflavine T fluorescence assay.

Low physical stability of insulin formulations may lead to amyloid fibril formation, which is observed as well-ordered, thread-like macromolecular structures in the sample eventually resulting in gel formation. This has traditionally been measured by visual inspection of the sample. However, the application of a small molecule indicator probe is much more preferable. Thioflavin T is such a probe and has a distinct fluorescence signature when binding to fibrils (or rather β-sheet rich proteins) [Naiki et al. (1989) Anal. Biochem. 177, 244-249; LeVine (1999) Methods. Enzymol. 309, 274-284]. Its application to insulin fibrillation has recently been validated [Nielsen et al. (2001) Biochemistry 40, 6036-6046].
The time course for fibril formation can be described by a sigmoidal curve with the following expression:

$$F = f_i + m_i t + \frac{f_f + m_f t}{1 + e^{-[(t-t_0)/\tau]}} \quad \text{Eq.(2)}$$

Here, $F$ is the ThT fluorescence at the time $t$. The constant $t_0$ is the time needed to reach 50% of maximum fluorescence. The minimum and maximum fluorescence is denoted $f_i$ and $f_f$, respectively, and the expressions $m_i t$ and $m_f t$ describe the linear development of the bottom and top base lines. The two important parameters describing fibril formation are the lag-time calculated by $t_0$ - 2τ and the apparent rate constant $k_{app}$ = 1/τ.

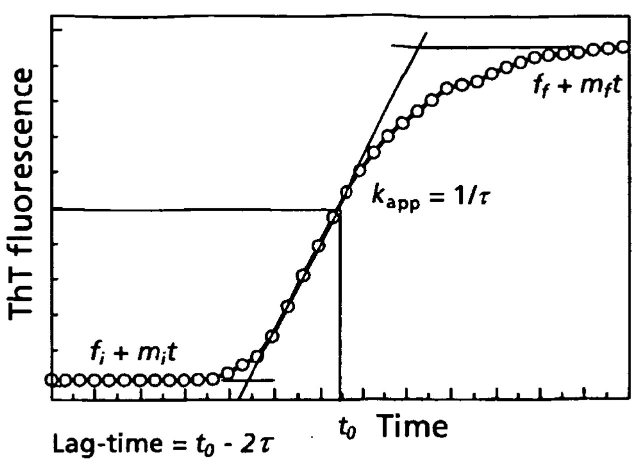


Formation of a partially folded intermediate of the protein is suggested as a general initiating mechanism for fibrillation. Few of those intermediates nucleate to form a template onto which further intermediates may assembly and the fibrillation is initiated. The lag-time corresponds to the interval in which the critical mass of nucleus is built up and the apparent rate constant is the rate with which the fibril itself is formed.
In accordance with this mechanism, insulin needs to dissociate to its monomeric form before a partially folded intermediate

may be formed. Keeping insulin on a multimeric form may therefore result in increased physical stability. Ligands binding to the insulin hexamer zinc site should stabilize the hexameric form and draw the equilibrium even further away from the monomeric form. Hence, an increased physical stability could be achieved.

Sample preparation

Insulin formulations were prepared freshly before each assay from appropriate stock solutions. Typical final concentrations were 0.6 mM human insulin or insulin aspart analogue, 0.2 mM ZnAc, 30 mM phenol, 10 mM Tris pH 8. ThT was added from a 1 mM stock solution in water to a final concentration of 1 μM. The formulations were typically prepared in double concentration and mixed with an equal volume of test compound in appropriate concentration in 4% DMSO, 10 mM Tris pH 8.
Alternatively, insulin aspart formulations (100 U/ml) from the production line were used directly. ThT was added to 1 μM and DMSO containing test compound in appropriate concentration to 2%.
Sample aliquots of 200 μl were placed in a 96 well microtiter plate (Packard OptiPlate™-96, white polystyrene). Usually, eight replica of each sample (corresponding to one test compound concentration) was placed in one column of wells. The plate was sealed with Scotch Pad (Qiagen).
Control experiments for possible test compound quenching of the ThT emission were carried out using human insulin without $Zn^{2+}$ and phenol i.e. in a non-hexameric configuration. Hence, the fibrillation process as well as the ThT emission should be unaffected by the presence of test compound, unless it quenched the ThT signal.

Incubation and fluorescence measurement

Temperature incubation, shaking and measurement of the ThT fluorescence were done in a Fluoroskan Ascent FL fluorescence platereader (Thermo Labsystems). Temperature setting is possible up till 45 ˚C, but usually sat at 30 ˚C. Heating was initiated at first measurement. The orbital shaking is selectable up till 1200 rpm, but adjusted to 960 rpm in all the presented data with an amplitude of 1 mm.
Fluorescence measurement was done using excitation trough a 444 nm filter and measurement of emission through a 485 nm filter. Each run was initiated by a measurement and intervals between measurements were usually 20 min. The plate was shaken and heated as adjusted between each measurement. The assay time was regulated by the number of measurements and the interval in between. Usually the plate was measured 46 times with 20 min between, i.e. over 15 hours.

Data handling

The measurement points were saved in Microsoft Excel format for further processing and curve drawing and fitting was performed using GraphPad Prism. The background emission from ThT in the absence of fibrils was negligible. Some test compounds had background fluorescence under the applied experimental conditions. This was eliminated by subtracting the mean value of the first measurement from the data set for this test compound. The data points are shown with standard deviation.
The data set may be fitted to Eq. (2). However, since the stabilizing effect of the test compounds/ligands were so significant that a full sigmoid curve was not obtained during the usual assay time, curve fitting to such a data set would be imprecise and hence meaningless.
Only data obtained in the same experiment (i.e. samples on the same plate) are presented in the same graph.

Examples & Results

The various ligands are shown below with structure and affinity towards the zinc site as measured by the TZD-assay described in "Analytical Methods".

| Reference | Example | Compound | $K_d$(app) (nM) |
|---|---|---|---|
| A | 533 | 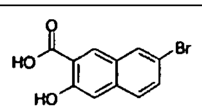 | 383 |

(continued)

| Reference | Example | Compound | $K_d$(app) (nM) |
|---|---|---|---|
| C | 462 | 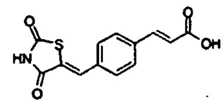 | 58 |
| D | 738 | 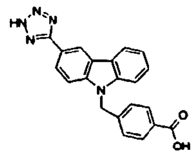 | 171 |
| E | 68 | 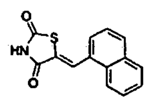 | 23 |
| F | 756 | 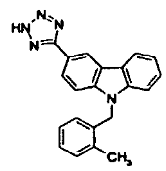 | 3 |
| G | 1 | 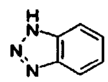 | 3879 |
| H | 76 | 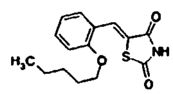 | 82 |
| I | 759 | 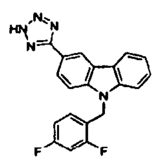 | 23 |

The ThT assays of various combinations of insulin formulations and ligands are shown in Fig. 1 - 8.

Addition of ligands improves the physical stability of insulin formulations. This holds for human insulin formulations (see Fig 1) as well as insulin aspart formulations (rest of data set).

The improved stability can be obtained by using various compound classes as zinc site anchor, e.g. benzothriazoles (G, Fig 1), naphthosalicylic acids (A, Fig 2), thiazolidine-diones (E, Figs 4, 7; C, Fig 2; H, Figs 6, 8) and tetrazoles (D, Fig 3; F, Fig 8; I, Fig 5).

Increased affinity of the ligand results in higher stability of the formulation. Compare the effect of the weakest binding ligand in 2 mM (G, Fig. 1) with the effect on an insulin aspart formulation of 0,5 mM E (Fig. 4). Also compare the effects of similar concentrations of A and C (Fig 2); and of D (fig. 3) and E (Fig 4) on insulin aspart formulations.

Increasing the concentration of ligand tends to improve the stabilization (see Figs. 1, 3, 4, 5, 6). In some instants, more pronounced effects are seen with the ligand in slight molar excess to the zinc sites, see Figs. 4, 6, whereas it seems to plateau around the stoichiometric concentration in other instances (Figs. 3, 5).

Of the presented ligands, A, C, D, E, F were tested in a disappearance assay for the effect on release of insulin aspart from a subcutaneous inject site. Surprisingly, the ligands had no effect on the insulin aspart disappearance. In a very limited way, this can be mimicked in the ThT assay by increasing the assay temperature to 37 ˚C (see Figs. 7, 8). The stabilizing effect is somewhat attenuated, e.g. compare E at 30 ˚C (Fig 4) and 37 ˚C (Fig 7), and H (Fig 6 and 8). The ligand with highest affinity (F) has the most stabilizing effect at 37 ˚C (Fig. 8).

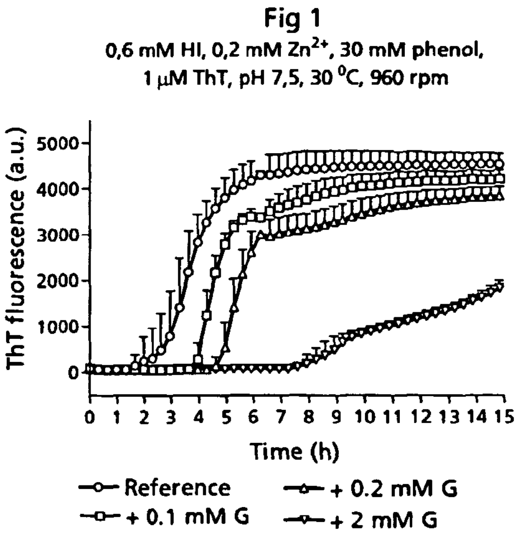
Fig 1
0,6 mM HI, 0,2 mM Zn2+, 30 mM phenol, 1 μM ThT, pH 7,5, 30 °C, 960 rpm
ThT fluorescence (a.u.)
Time (h)
Reference
+ 0.1 mM G
+ 0.2 mM G
+ 2 mM G

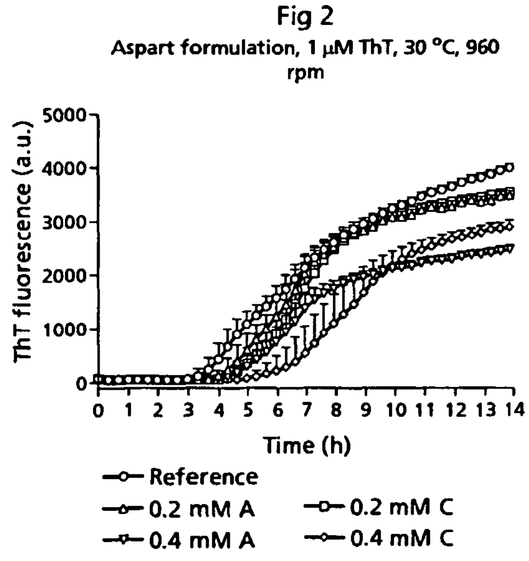
Fig 2
Aspart formulation, 1 μM ThT, 30 °C, 960 rpm
ThT fluorescence (a.u.)
Time (h)
Reference
0.2 mM A
0.4 mM A
0.2 mM C
0.4 mM C

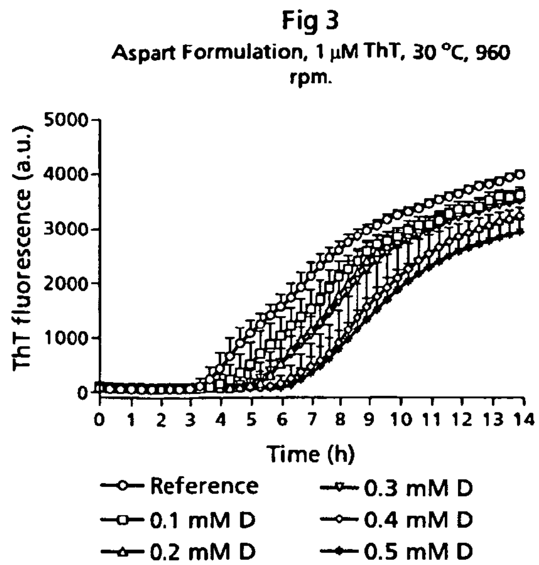
Fig 3
Aspart Formulation, 1 μM ThT, 30 °C, 960 rpm.
ThT fluorescence (a.u.)
Time (h)
Reference
0.1 mM D
0.2 mM D
0.3 mM D
0.4 mM D
0.5 mM D

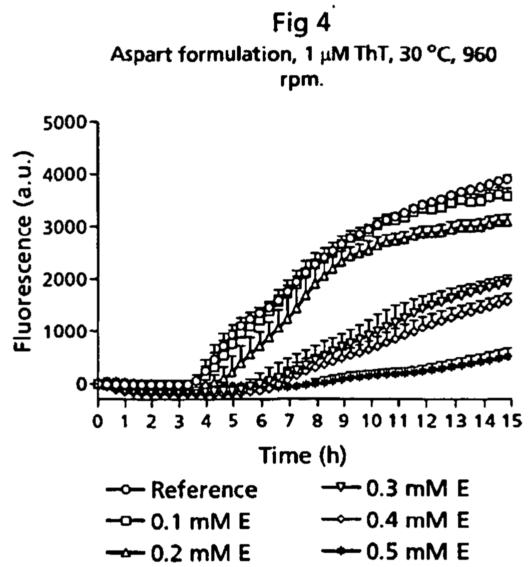
Fig 4
Aspart formulation, 1 μM ThT, 30 °C, 960 rpm.
Fluorescence (a.u.)
Time (h)
Reference
0.1 mM E
0.2 mM E
0.3 mM E
0.4 mM E
0.5 mM E

Fig 5
Aspart formulation, 1 μM ThT, pH 8, 30 °C, 960 rpm.

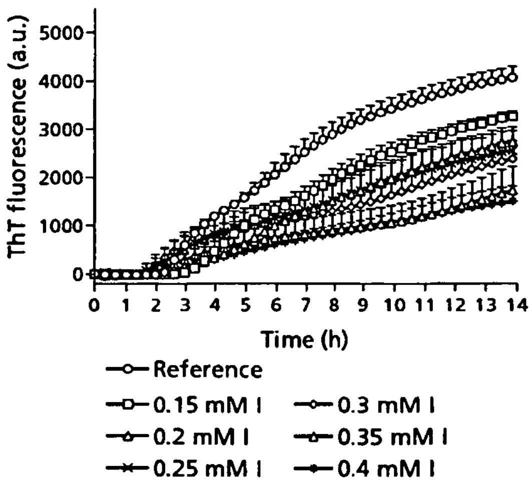


Fig 6
Aspart formulation, 1 μM ThT, pH 8, 30 °C, 960 rpm.

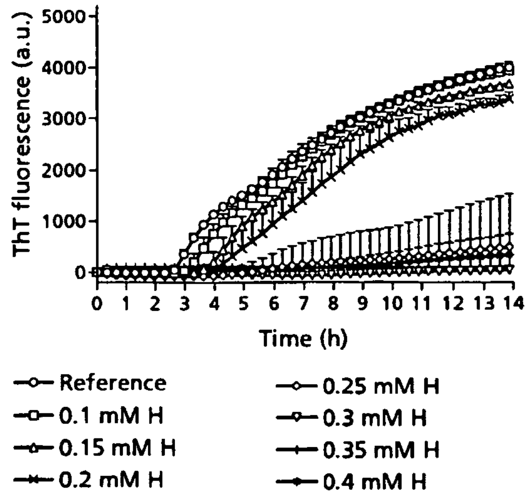


Fig 7
Aspart formulation , 1 μM ThT, 37 °C, 960 rpm

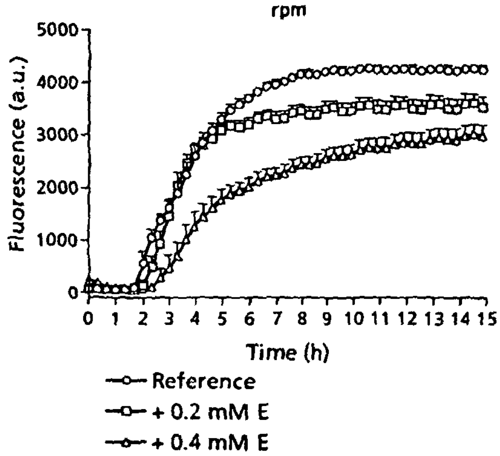


Fig 8
Aspart formulation, 1 μM ThT, 37 °C, 960 rpm.

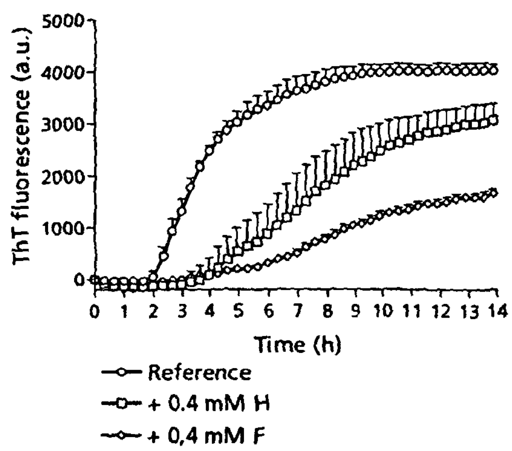


Example 1011 Retention of fast absorption characteristics of formulations stabilized by addition of $His^{B10}$ $Zn^{2+}$-site ligands.

Formulations of the present invention were characterized by the disappearance rate from the subcutaneous depot following injection in pigs. Formulations of B28 Asp human insulin containing A14Tyr($^{125}$I) B28 Asp human insulin were followed with an external y-counter (Ribel et al., The pig as a model for subcutaneous absorption in man. In: Serrano-Ritos & Lefebre (Eds.): Diabetes (1985) proceedings ot the 12th congress of the international diabetes federation, Madrid. Spain, 1985 (Excerpta Medica, Amsterdam (1986) 891-896. Formulations of Insulin Aspart (0.6 mM, U100) containing 0.3 mM $Zn^{2+}$, 30 mM phenol, 2 mM phosphate buffer, and 1.6% glycerol, pH 7.4, were compared with the corresponding formulations containing 0.3 mM of the ligands shown below: where $T_{50\%}$ is the time when 50% ot the A14Tyr($^{125}$I) 828 Asp insulin has disappeared from the site of injection and $K_d$ is the affinity of the ligand as measured by the TZD-assay described in "Analytical methods" below. It is evident that the stabilizing ligands do not affect the fast absorption properties of the formulations

| | Example # | Compound | T50% | $K_d$ (app) (nM) |
|---|---|---|---|---|
| reference | | | 1.3± 0.3 | |
| A | 533 | 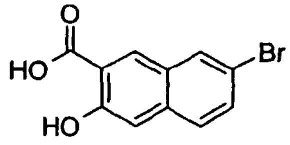 | 1.0±0.23 | 383 |
| B | 476 | 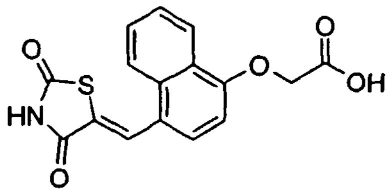 | 1.1± 0.37 | 68 |
| C | 462 | 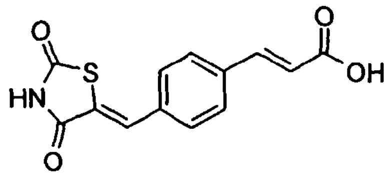 | 0.9±0.19 | 58 |
| D | 738 | 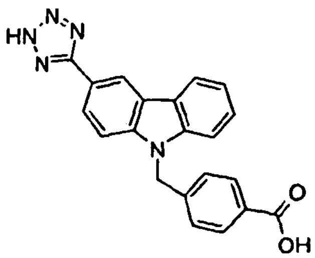 | 1.3±0.36 | 171 |
| E | 68 | 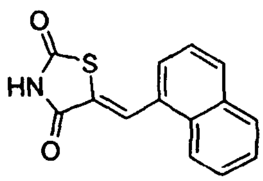 | 1.2±0.41 | 23 |
| F | 756 | 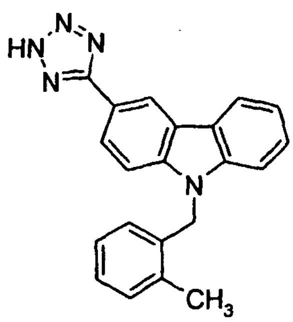 | 1.2± 0.27 | 3 |

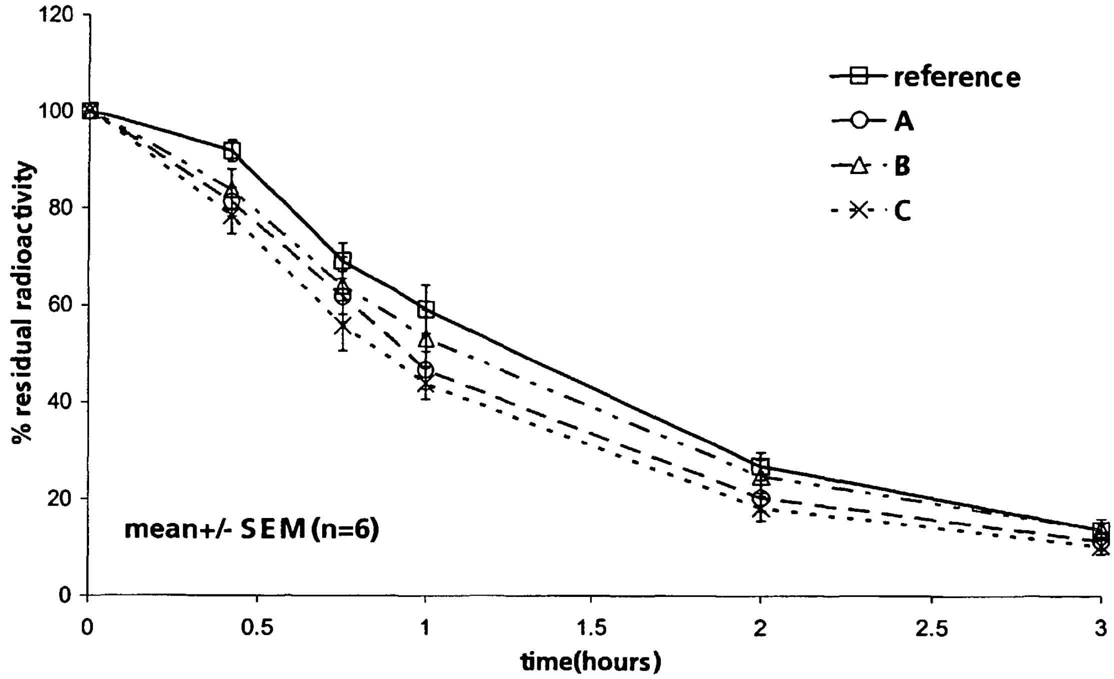


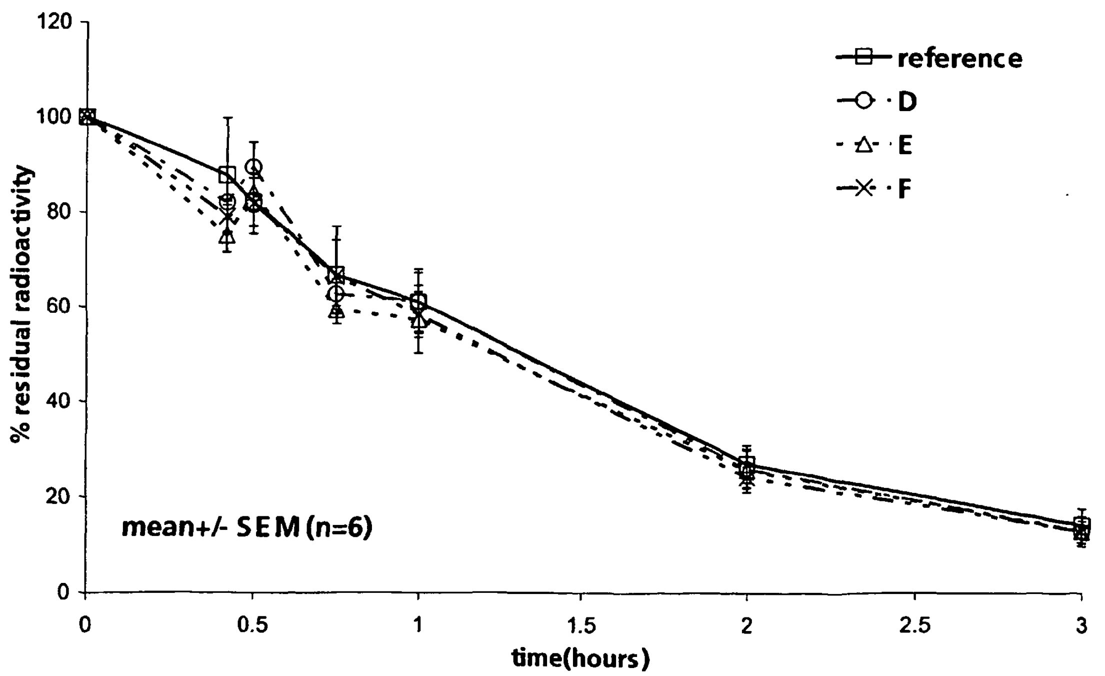


**Figure 9**

**Example 1012**

Reference experiment

The chemical stability of insulin formulations of the invention was characterised by HPLC (RPC, reverse phase chromatography and SEC, size exclusion chromatography). As reference, insulin formulated without ligands of the invention but with 0.3 % DMSO was also investigated and shown below:

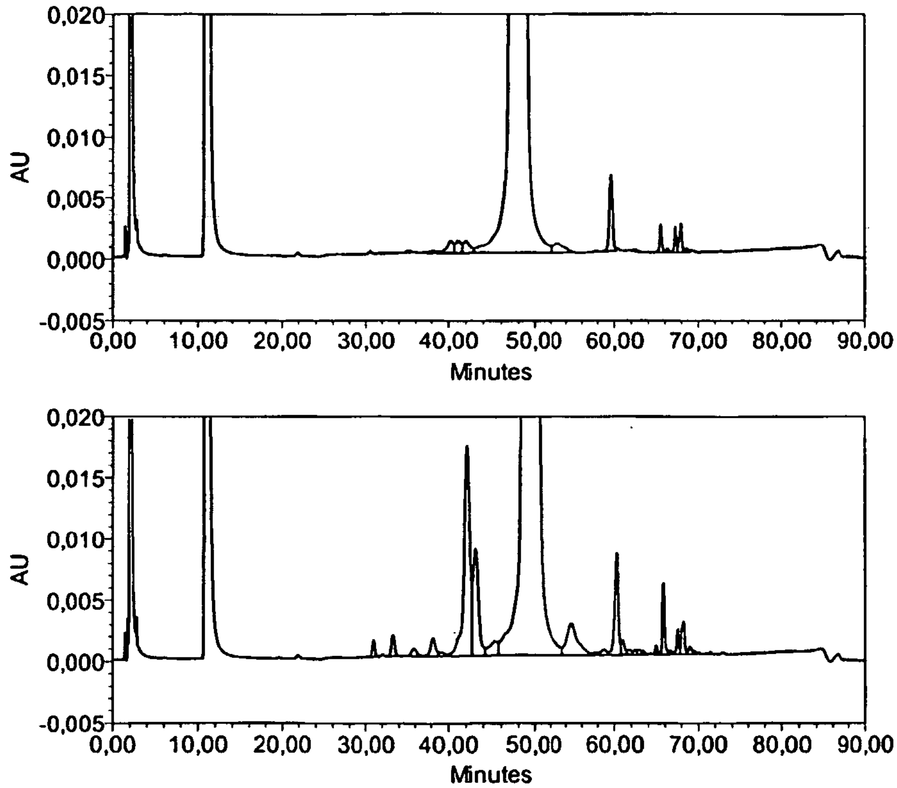


**Figure 10**

**Fig 10.** Reverse phase chromatography of formulated human insulin with 3 $Zn^{2+}$ per hexamer, 30 mM phenol, 150 mM mannitol, 3 mM phosphoric acid, sodium hydroxide to pH 7.4 and 0.3 % DMSO corresponding to 3 ligands per hexamer at start (upper panel) and after storage for 2 weeks at 37 ˚C (lower panel): Preservatives before 20 min., "hydrophilic derivatives" (desamido-insulins) 20 min to main top insulin, "hydrophobic derivatives 1" main top to 64 min., and "hydrophobic derivatives 2" (insulin dimers) after 64 min.

**Storage** in HPLC 1 ml vials at 45 ˚C (5 d), 37 ˚C (2 w), 30 ˚C (6 w), 25 ˚C (10 w), 15 ˚C (30 w) gave about the same increase of transformation products correlating to an increase in reactions constants of a factor 3-4 per 10 degree..

**RPC** (reverse phase chromatography) on Waters SymmetryShield $RP_8$ column, 150x4.6 mm and 3.5 μm, eluted by **A**: 0.2 M sodium sulfate + 0.04 M sodium phosphate pH 7.2 + 10 % acetonitrile and isocratically (i) or a gradient (g) of **B**: 70 % acetonitrile [minutes/%B(i/g): 0/19, 21/24(i)(sudden change), 51/24(i), 81/39(g), 81.1/0(i), 82.3/19(i)] at flow of 0.9 mL/min and 30 ˚C.

**SEC** (size exclusion chromatography) on Waters insulin HMWP column, 300x7.8 mm, eluted by 15:20:65 of acetic acid: acetonitrile: arginine 1 g/L at flow of 1 mL/min and ambient temperature.

| % formed/year | **RPC** | | | **SEC** |
|---|---|---|---|---|
| | hphil | hphob1 | hphob2 | dimer |
| 45˚C | 153.3 | 47.5 | 23.4 | 21.9 |
| 37˚C | 57.2 | 15.6 | 5.20 | 6.24 |
| 30˚C | 24.5 | 4.16 | 1.30 | 2.08 |
| 25˚C | 14.2 | 2.13 | 0.68 | 1.09 |
| 15˚C | 4.12 | 0.43 | 0.22 | 0.31 |

(continued)

| % formed/year | RPC | | | SEC |
|---|---|---|---|---|
| | hphil | hphob1 | hphob2 | dimer |
| 5°predicted | (1.001) | (0.066) | (0.027) | (0.052) |

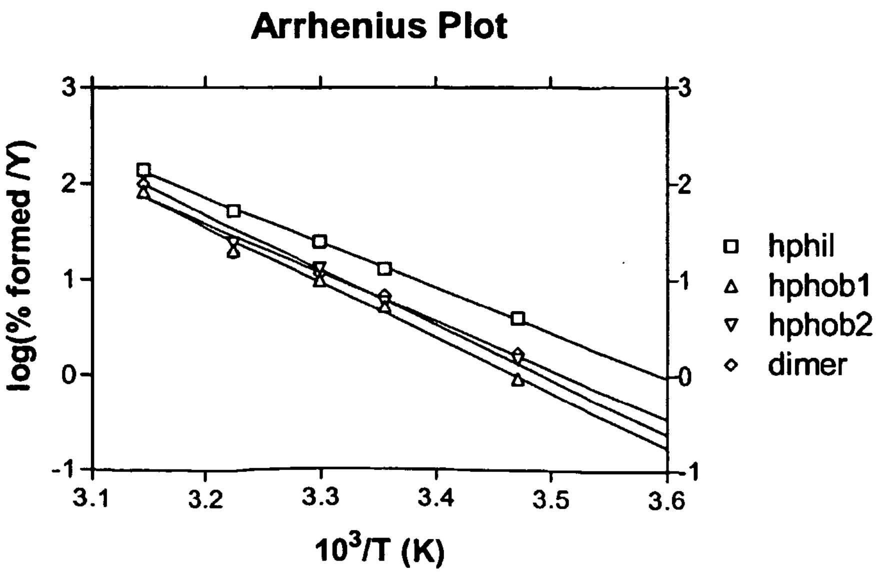


The chemical stability of insulin formulations of the invention was likewise characterised by HPLC (RPC, reverse phase chromatography and SEC, size exclusion chromatography). Compared to the reference the formulations of the invention were shown to be more chemically stable.

**Example 1013**

Chemical stability of insulin formulated with the compound of example 533 , 7-bromo-3-hydroxy-2-naphthoic acid:

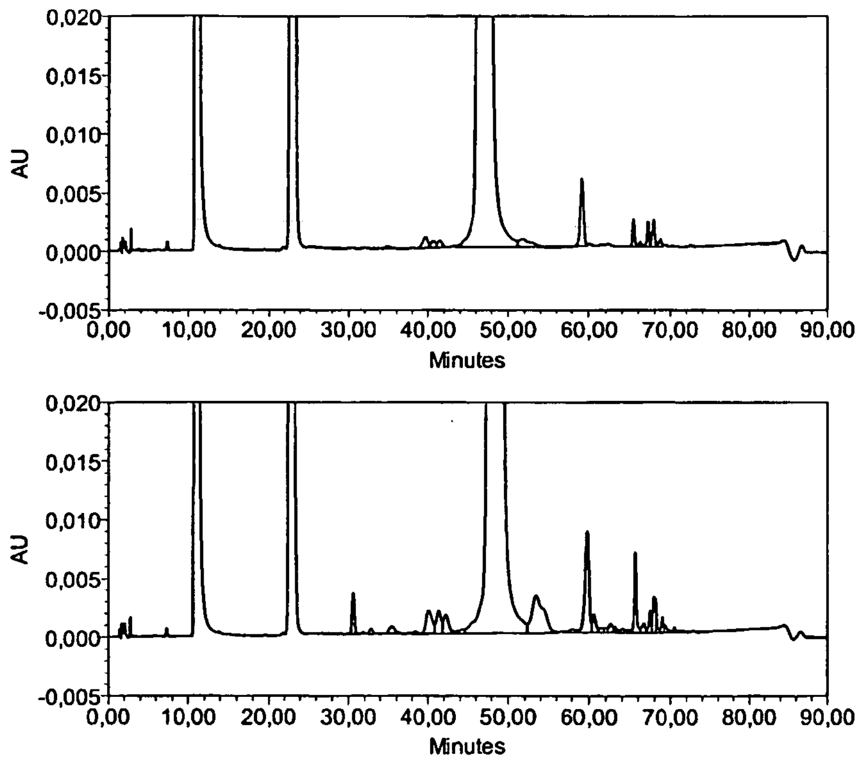


**Figure 11**

**Fig 11.** Reverse phase chromatography of formulated human insulin as described for the reference example and added 3 ligands of Example 533 and 3 $Zn^{2+}$ per hexamer at start (upper panel) and after storage for 2 weeks at 37 ˚C (lower panel): Preservatives before 20 min., "hydrophilic derivatives" (desamido-insulins) 20 min to main top insulin, "hydrophobic derivatives 1" main top to 64 min., and "hydrophobic derivatives 2" (insulin dimers) after 64 min.

**Storage** in HPLC 1 ml vials at 45 ˚C (5 d), 37 ˚C (2 w), 30 ˚C (6 w), 25 ˚C (10 w), 15 ˚C (30 w) will give about the same increase in transformation products correlating to an increase in reaction constants of a factor 3-4 per 10 degrees...

**RPC** (reverse phase chromatography) on Waters SymmetryShield $RP_8$ column, 150x4.6 mm and 3.5 μm, eluted by **A**: 0.2 M sodium sulfate + 0.04 M sodium phosphate pH 7.2 + 10 % acetonitrile and isocratically (i) or a gradient (g) of **B**: 70 % acetonitrile [minutes/%B(i/g): 0/19, 21/24(i)(sudden change), 51/24(i), 81/39(g), 81.1/0(i), 82.3/19(i)] at flow of 0.9 mL/min and 30 ˚C.

**SEC** (size exclusion chromatography) on Waters insulin HMWP column, 300x7.8 mm, eluted by 15:20:65 of acetic acid: acetonitrile:arginine 1 g/L at flow of 1 mL/min and ambient temperature.

| % formed/year | **RPC** | | | **SEC** |
|---|---|---|---|---|
| | hphil | hphob1 | hphob2 | dimer |
| 45˚C | 30.7 | 62.1 | 23.4 | 26.3 |
| 37˚C | 12.5 | 22.4 | 7.02 | 7.02 |
| 30˚C | 6.76 | 6.42 | 2.25 | 2.86 |
| 25˚C | 4.06 | 5.67 | 1.46 | 1.61 |
| 15˚C | 1.80 | 0.85 | 0.42 | 0.45 |
| 5˚predicted | (0.55) | (0.19) | (0.079) | (0.087) |

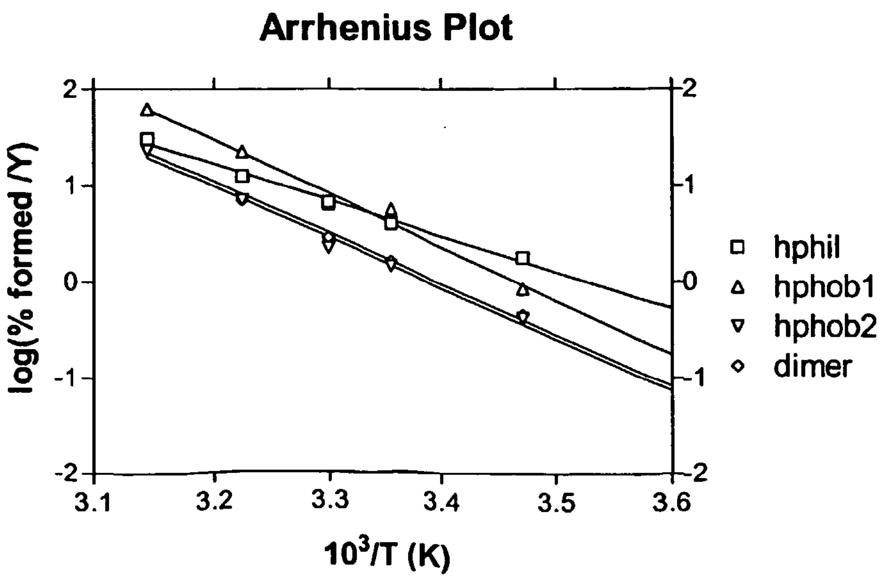


**Example 1014**

Chemical stability of insulin formulated with the compound of Example 462, 3-[4-(2,4-dioxothiazolidin-5-ylidenemethyl) phenyl]acrylic acid:

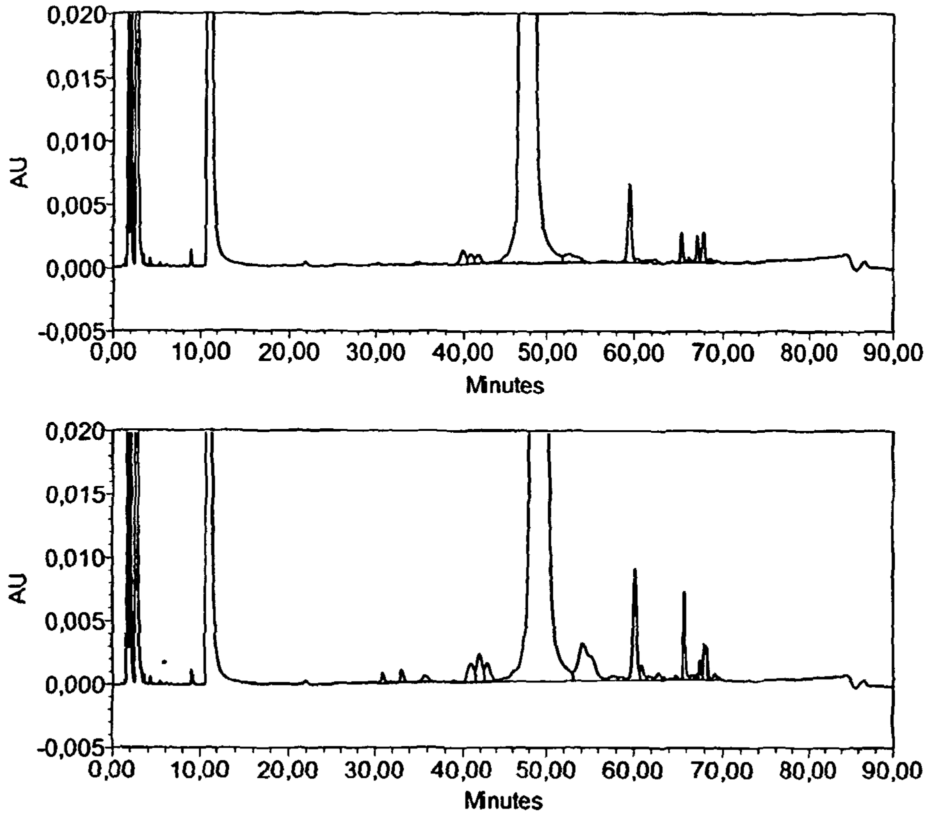


**Figure 12**

**Fig 12.** Reverse phase chromatography of formulated human insulin as described for the reference example and added 3 ligands of example 462 and 3 $Zn^{2+}$ per hexamer at start (upper panel) and after storage for 2 weeks at 37 ˚C (lower panel): Preservatives before 20 min., "hydrophilic derivatives" (desamido-insulins) 20 min to main top insulin, "hydrophobic derivatives 1" main top to 64 min., and "hydrophobic derivatives 2" (insulin dimers) after 64 min.

**Storage** in HPLC 1 ml vials at 45 ˚C (5 d), 37 ˚C (2 w), 30 ˚C (6 w), 25 ˚C (10 w), 15 ˚C (30 w) will give about the same increase in transformation products correlating to an increase in reaction constants of a factor 3-4 per 10 degrees.

**RPC** (reverse phase chromatography) on Waters SymmetryShield $RP_8$ column, 150x4.6 mm and 3.5 μm, eluted by **A:** 0.2 M sodium sulfate + 0.04 M sodium phosphate pH 7.2 + 10 % acetonitrile and isocratically (i) or a gradient (g) of **B:** 70 % acetonitrile [minutes/%B(i/g): 0/19, 21/24(i)(sudden change), 51/24(i), 81/39(g), 81.1/0(i), 82.3/19(i)] at flow of 0.9 mUmin and 30 ˚C.

**SEC** (size exclusion chromatography) on Waters insulin HMWP column, 300x7.8 mm, eluted by 15:20:65 of acetic acid: acetonitrile:arginine 1 g/L at flow of 1 mL/min and ambient temperature.

| % formed/year | RPC | | | SEC |
|---|---|---|---|---|
| | hphil | hphob1 | hphob2 | Dimer |
| 45˚C | 20.4 | 43.8 | 17.5 | 21.2 |
| 37˚C | 9.88 | 19.5 | 4.68 | 6.24 |
| 30˚C | 4.07 | 4.42 | 1.65 | 2.17 |
| 25˚C | 2.60 | 6.14 | 0.78 | 1.09 |

(continued)

| % formed/year | RPC | | | SEC |
|---|---|---|---|---|
| | hphil | hphob1 | hphob2 | Dimer |
| 15°C | 1.02 | 1.28 | 0.38 | 0.28 |
| 5°predicted | (0.293) | (0.336) | (0.062) | (0.048) |

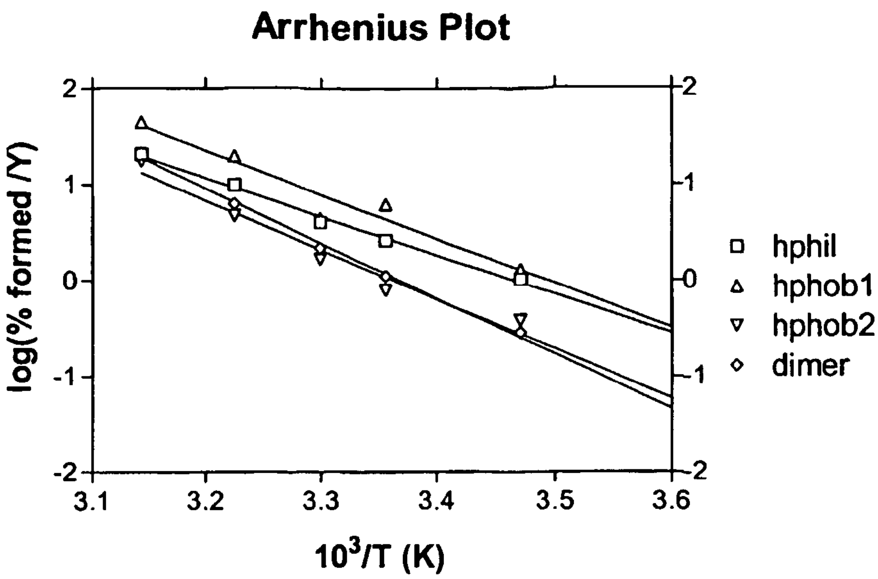


**Example 1015**

Chemical stability of insulin formulated with the compound of example 461 , [3-(2,4-Dioxothiazolidin-5-ylidenemethyl) phenoxy]acetic acid:

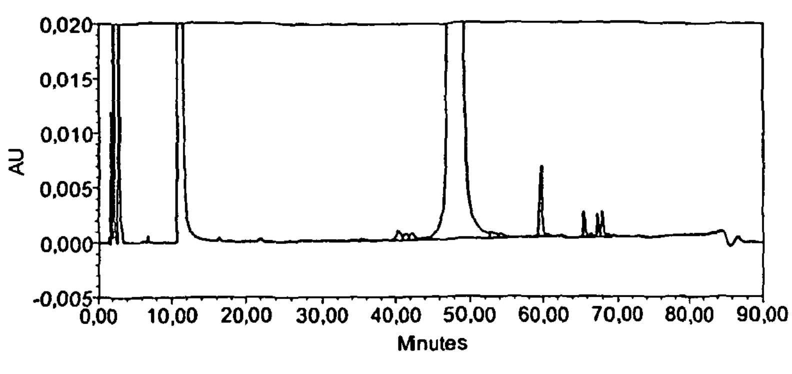

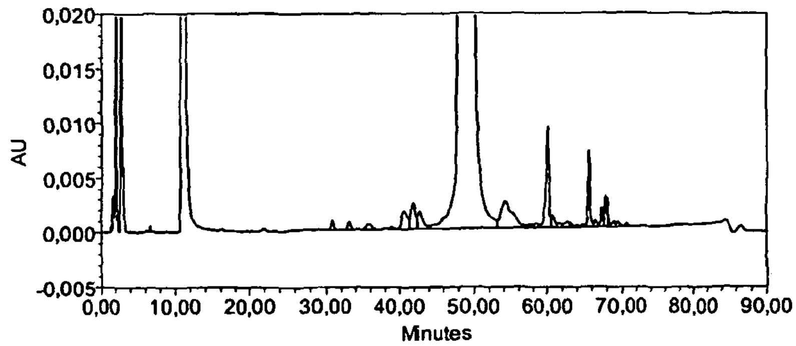


**Fig 13.** Reverse phase chromatography of formulated human insulin added 3 ligands (#) and 3 Zn per hexamer at start and storage of 2 w 37 ˚C: Preservatives before 20 min., "hydrophilic derivatives" (desamido-insulins) 20 min to main top insulin, "hydrophobic derivatives 1" main top to 64 min., and "hydrophobic derivatives 2" (insulin dimers) after 64 min.

**Storage** in HPLC 1 ml vials at 45 ˚C (5 d), 37 ˚C (2 w), 30˚C (6 w), 25˚C (10 w), 15 ˚C (30 w) will give about the same increase of 0.7 % hfil, 0.6 % hfob1, 0.3 % hfob2 and 0.3 % dimer solution 1, correlating to $Q_{10}$ of 3 below 30 ˚C and 4 at higher temperature for ref..

**RPC** (reverse phase chromatography) on Waters SymmetryShield $RP_8$ column, 150x4.6 mm and 3.5 μm, eluted by **A**: 0.2 M sodium sulfate + 0.04 M sodium phosphate pH 7.2 + 10 % acetonitrile and isocratically (i) or a gradient (g) of **B**: 70 % acetonitrile [minutes/%B(i/g): 0/19, 21/24(i)(sudden change), 51/24(i), 81/39(g), 81.1/0(i), 82.3/19(i)] at flow of 0.9 mL/min and 30 ˚C.

**SEC** (size exclusion chromatography) on Waters insulin HMWP column, 300x7.8 mm, eluted by 15:20:65 of acetic acid: acetonitrile:arginine 1 g/L at flow of 1 mL/min and ambient temperature.

| % formed/year | **RPC** | | | **SEC** |
|---|---|---|---|---|
| | hphil | hphob1 | hphob2 | dimer |
| 45˚C | 29.9 | 52.6 | 23.4 | 23.4 |
| 37˚C | 11.2 | 20.0 | 7.02 | 6.50 |
| 30˚C | 5.12 | 7.11 | 1.99 | 2.34 |
| 25˚C | 2.86 | 4.78 | 0.99 | 1.20 |
| 15˚C | 1.14 | 0.67 | 0.29 | 0.33 |
| 5˚predicted | (0.287) | (0.153) | (0.044) | (0.057) |

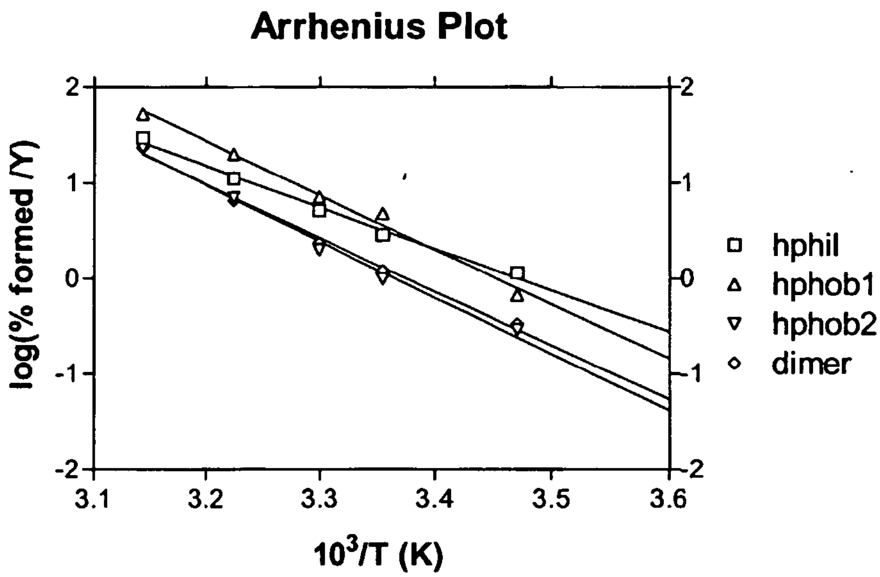


**Example 1016**

Chemical stability of insulin formulated with the compound of example 70 , 5-(4-Diethylaminobenzylidene)thiazolidine-2,4-dione

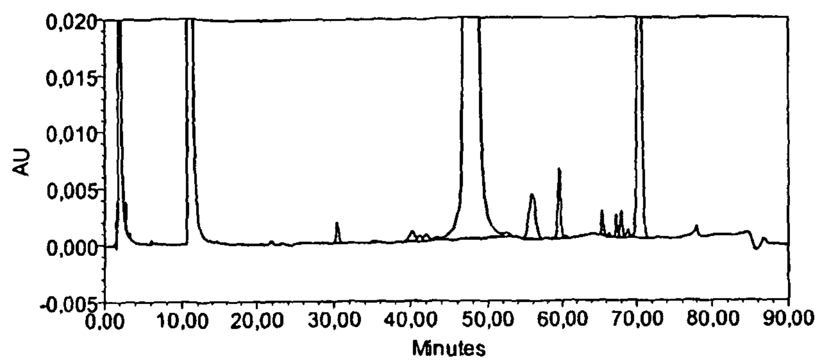


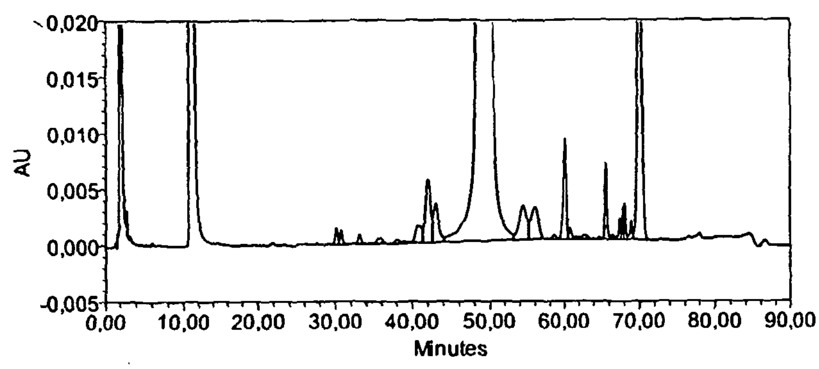

**Fig 14.** Reverse phase chromatography of formulated human insulin added 3 ligands (#) and 3 Zn per hexamer at start and storage of 2 w 37 ˚C: Preservatives before 20 min., "hydrophilic derivatives" (desamido-insulins) 20 min to main top insulin, "hydrophobic derivatives 1" main top to 64 min., and "hydrophobic derivatives 2" (insulin dimers) after 64 min.

**Storage** in HPLC 1 ml vials at 45 ˚C (5 d), 37 ˚C (2 w), 30 ˚C (6 w), 25 ˚C (10 w), 15 ˚C (30 w) will give about the same increase of 0.7 % hfil, 0.6 % hfob1, 0.3 % hfob2 and 0.3 % dimer solution 1, correlating to $Q_{10}$ of 3 below 30 ˚C and 4 at higher temperature for ref..

**RPC** (reverse phase chromatography) on Waters SymmetryShield $RP_8$ column, 150x4.6 mm and 3.5 μm, eluted by **A**: 0.2 M sodium sulfate + 0.04 M sodium phosphate pH 7.2 + 10 % acetonitrile and isocratically (i) or a gradient (g) of **B**: 70 % acetonitrile [minutes/%B(i/g): 0/19, 21/24(i)(sudden change), 51/24(i), 81/39(g), 81.1/0(i), 82.3/19(i)] at flow of 0.9 mL/min and 30 ˚C.

**SEC** (size exclusion chromatography) on Waters insulin HMWP column, 300x7.8 mm, eluted by 15:20:65 of acetic acid: acetonitrile:arginine 1 g/L at flow of 1 mL/min and ambient temperature.

| % formed/year | RPC | | | SEC |
|---|---|---|---|---|
| | hphil | hphob1 | hphob2 | dimer |
| 45˚C | 56.2 | 78.8 | 27.0 | 29.2 |
| 37˚C | 23.7 | 14.8 | 6.50 | 8.84 |
| 30˚C | 12.1 | 6.94 | 3.03 | 3.55 |
| 25˚C | 6.08 | 4.99 | 0.94 | 2.24 |
| 15˚C | 1.95 | 1.09 | 0.42 | 0.69 |
| 5˚predicted | (0.54) | (0.22) | (0.064) | (0.15) |

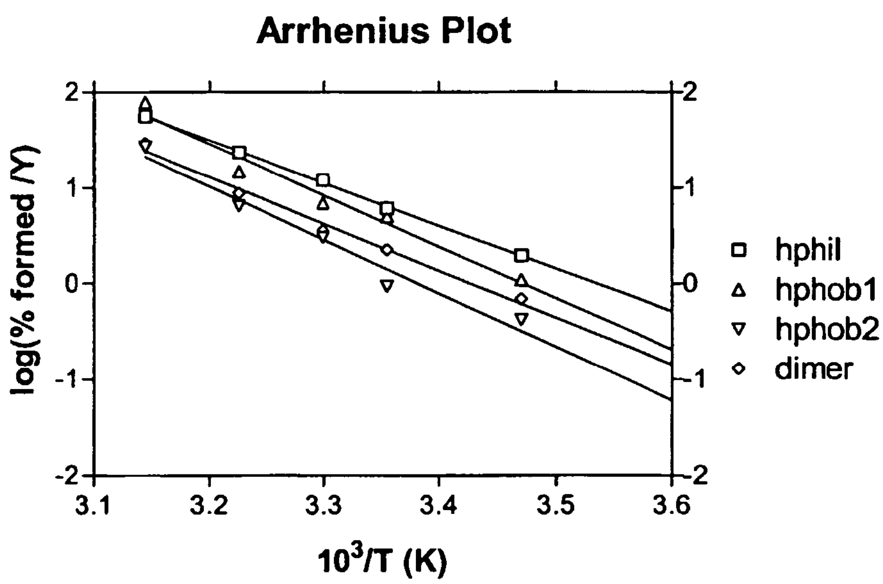


## ANALYTICAL METHODS

*Assays to quantify the binding affinity of ligands to the metal site of the insulin $R_6$ hexamers:*

**4H3N-assay:**

The binding affinity of ligands to the metal site of insulin $R_6$ hexamers are measured in a UV/vis based displacement assay. The UV/vis spectrum of 3-hydroxy-4-nitro benzoic acid (4H3N) which is a known ligand for the metal

site of insulin $R_6$ shows a shift in absorption maximum upon displacement from the metal site to the solution (Huang et al., 1997, Biochemistry 36, 9878-9888). Titration of a ligand to a solution of insulin $R_6$ hexamers with 4H3N mounted in the metal site allows the binding affinity of these ligands to be determined following the reduction of absorption at 444 nm. A stock solution with the following composition 0.2 mM human insulin, 0.067 mM Zn-acetate, 40 mM phenol, 0.101 mM 4H3N is prepared in a 10mL quantum as described below. Buffer is always 50mM tris buffer adjusted to pH=8.0 with $NaOH/ClO_4^-$.

1000 μL of 2.0mM human insulin in buffer
66.7 μL of 10mM Zn-acetate in buffer
800 μL of 500mM phenol in $H_2O$
201 μL of 4H3N in $H_2O$
7.93 ml buffer

The ligand is dissolved in DMSO to a concentration of 20 mM.
The ligand solution is titrated to a cuvette containing 2 mL stock solution and after each addition the UV/vis spectrum is measured. The titration points are listed in Table 3 below.

**Table 3**

| ligand addition (μl) | ligand conc. (mM) | dilution factor |
|---|---|---|
| 1 | 0.010 | 1.0005 |
| 1 | 0.020 | 1.0010 |
| 1 | 0.030 | 1.0015 |
| 2 | 0.050 | 1.0025 |
| 5 | 0.100 | 1.0050 |
| 10 | 0.198 | 1.0100 |
| 20 | 0.392 | 1.0200 |
| 20 | 0.583 | 1.0300 |
| 20 | 0.769 | 1.0400 |
| 20 | 0.952 | 1.0500 |

The UV/vis spectra resulting from a titration of the compound 3-hydroxy-2-naphthoic acid is shown in Figure 5. Inserted in the upper right corner is the absorbance at 444nm vs. the concentration of ligand.
The following equation is fitted to these datapoints to determine the two parameters $K_D$(obs), the observed dissociation constant, and $abs_{max}$ the absorbance at maximal ligand concentration.

$$abs([ligand]_{free}) = (abs_{max} * [ligand]_{free}) / (K_D(obs) + [ligand]_{free})$$

The observed dissociation constant is recalculated to obtain the apparent dissociation constant

$$K_D(app) = K_D(obs) / (1+[4H3N]/K_{4H3N})$$

The value of $K_{4H3N}$=50 μM is taken from Huang et al., 1997, Biochemistry 36, 9878-9888.

**TZD-assay:**

The binding affinity of ligands to the metal site of insulin $R_6$ hexamers are measured in a fluorescense based displacement assay. The fluorescence of 5-(4-dimethylaminobenzylidene)thiazolidine-2,4-dione (TZD) which is a ligand for the metal site of insulin $R_6$ is quenched upon displacement from the metal site to the solution. Titration of a ligand to a stock solution of insulin $R_6$ hexamers with this compound mounted in the metal site allows the binding affinity of

these ligands to be determined measuring the fluorescence at 455nm upon excitation at 410nm.

Preparation

Stock solution: 0.02 mM human insulin, 0.007 mM Zn-acetate, 40 mM phenol, 0.01 mM TZD in 50mM tris buffer adjusted to pH=8.0 with $NaOH/ClO_4^-$ .
The ligand is dissolved in DMSO to a concentration of 5 mM and added in aliquots to the stock solution to final concentrations of 0-250 □M.

Measurements

Fluorescence measurements were carried out on a Perkin Elmer Spectrofluorometer LS50B.The main absorption band was excited at 410 nm and emission was detected at 455 nm. The resolution was 10 nm and 2.5 nm for excitation and emission, respectively.

Data analysis

This equation is fitted to the datapoints

$$\Delta F(455nm)) = \Delta F_{max} * [ligand]_{free}/( K_D(app) * ( 1+[TZD]/K_{TZD} )+ [ligand]_{free}))$$

$K_D$(app) is the apparent dissociation constant and $F_{max}$ is the fluorescence at maximal ligand concentration. The value of $K_{TZD}$ is measured separately to 230 nM

Two different fitting-procedures can be used. One in which both parameters, $K_D$(app) and $F_{max}$, are adjusted to best fit the data and a second in which the value of $F_{max}$ is fixed ($F_{max}$=1) and only $K_D$(app) is adjusted. The given data are from the second fitting procedure. The Solver module of Microsoft Excel can be used to generate the fits from the data points.

## Claims

**1.** A pharmaceutical composition comprising insulin and a zinc-binding ligand which reversibly binds to a $His^{B10}$ $Zn^{2+}$ site of an insulin hexamer, wherein the ligand is selected from the group consisting of benzotriazoles, 3-hydroxy 2-napthoic acids, salicylic acids, tetrazoles, thiazolidinediones, 5-mercaptotetrazoles, or 4-cyano-1,2,3-triazoles, or any enantiomer, diastereomer, including a racemic mixture, tautomer as well as a salt thereof with a pharmaceutically acceptable acid or base.

**2.** A pharmaceutical composition according to claim 1 wherein the zinc-binding ligand is

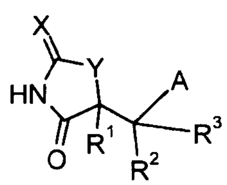 or 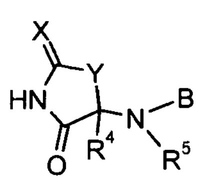 or 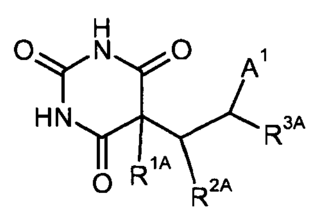

wherein

X is =O, =S or =NH
Y is -S-, -O- or -NH-
$R^1$, $R^{1A}$ and $R^4$ are independently selected from hydrogen or $C_1$-$C_6$-alkyl,
$R^2$ and $R^{2A}$ are hydrogen or $C_1$-$C_6$-alkyl or aryl, $R^1$ and $R^2$ may optionally be combined to form a double bond, $R^{1A}$ and $R^{2A}$ may optionally be combined to form a double bond,
$R^3$, $R^{3A}$ and $R^5$ are independently selected from hydrogen, halogen, aryl optionally substituted with one or more substituents independently selected from $R^{16}$, $C_1$-$C_6$-alkyl, or $-C(O)NR^{11}R^{12}$,

A, $A^1$ and B are independently selected from $C_1$-$C_6$-alkyl, aryl, aryl-$C_1$-$C_6$-alkyl, -$NR^{11}$-aryl, aryl-$C_2$-$C_6$-alkenyl or heteroaryl, wherein the alkyl or alkenyl is optionally substituted with one or more substituents independently selected from $R^6$ and the aryl or heteroaryl is optionally substituted with up to four substituents $R^7$, $R^8$, $R^9$, and $R^{10}$, A and $R^3$ may be connected through one or two valence bonds, B and $R^5$ may be connected through one or two valence bonds,
$R^6$ is independently selected from halogen, -CN, -$CF_3$, -$OCF_3$, aryl, -COOH and -$NH_2$,
$R^7$, $R^8$, $R^9$ and $R^{10}$ are independently selected from

- hydrogen, halogen, -CN, -$CH_2$CN, -$CHF_2$, -$CF_3$, -$OCF_3$, -$OCHF_2$, -$OCH_2CF_3$, -$OCF_2CHF_2$, -$S(O)_2CF_3$, -$OS(O)_2CF_3$, -$SCF_3$, -$NO_2$, -$OR^{11}$, -$NR^{11}R^{12}$, -$SR^{11}$, -$NR^{11}S(O)_2R^{12}$, -$S(O)_2NR^{11}R^{12}$, -$S(O)NR^{11}R^{12}$, -S (O)$R^{11}$, -$S(O)_2R^{11}$, -$OS(O)_2$ $R^{11}$, -C(O)$NR^{11}R^{12}$, -OC(O)$NR^{11}R^{12}$, -$NR^{11}C(O)R^{12}$, -$CH_2C(O)NR^{11}R^{12}$, -$OC_1$-$C_6$-alkyl-C(O)$NR^{11}R^{12}$, -$CH_2OR^{11}$, -$CH_2OC(O)R^{11}$, -$CH_2NR^{11}R^{12}$, -OC(O)$R^{11}$, -$OC_1$-$C_{15}$-alkyl-C(O) $OR^{11}$, -$OC_1$-$C_6$-alkyl-$OR^{11}$, -$SC_1$-$C_6$-alkyl-C(O)$OR^{11}$ , -$C_2$-$C_6$-alkenyl-C(=O)$OR^{11}$, -$NR^{11}$-C (=O)-$C_1$-$C_6$-alkyl-C(=O)$OR^{11}$, -$NR^{11}$-C(=O)-$C_1$-$C_6$-alkenyl-C(=O)$OR^{11}$, -C(O)$OR^{11}$, C(O)$R^{11}$, or -$C_2$-$C_6$-alkenyl-C(=O)$R^{11}$, =O, or-$C_2$-$C_6$-alkenyl-C(=O)-$NR^{11}R^{12}$,
- $C_1$-$C_6$-alkyl, $C_2$-$C_6$-alkenyl or $C_2$-$C_6$-alkynyl, each of which may optionally be substituted with one or more substituents independently selected from $R^{13}$,
- aryl, aryloxy, aryloxycarbonyl, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, heteroaryl-$C_2$-$C_6$-alkenyl, heteroaryl-$C_2$-$C_6$-alkynyl, or $C_3$-$C_6$ cycloalkyl,

of which each cyclic moiety may optionally be substituted with one or more substituents independently selected from $R^{14}$,
$R^{11}$ and $R^{12}$ are independently selected from hydrogen, OH, $C_1$-$C_{20}$-alkyl, aryl-$C_1$-$C_6$-alkyl or aryl, wherein the alkyl groups may optionally be substituted with one or more substituents independently selected from $R^{15}$, and the aryl groups may optionally be substituted one or more substituents independently selected from $R^{16}$; $R^{11}$ and $R^{12}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds,
$R^{13}$ is independently selected from halogen, -CN, -$CF_3$, -$OCF_3$, -$OR^{11}$, -C(O)$OR^{11}$, -$NR^{11}R^{12}$, and -C(O) $NR^{11}R^{12}$,
$R^{14}$ is independently selected from halogen, -C(O)$OR^{11}$, -$CH_2C(O)OR^{11}$, -$CH_2OR^{11}$, -CN, - $CF_3$, -$OCF_3$, -$NO_2$, -$OR^{11}$, -$NR^{11}R^{12}$, -$NR^{11}C(O)R^{11}$, -$S(O)_2R^{11}$, aryl and $C_1$-$C_6$-alkyl,
$R^{15}$ is independently selected from halogen, -CN, -$CF_3$, =O, -$OCF_3$, -$OC_1$-$C_6$-alkyl, -C(O)$OC_1$-$C_6$-alkyl, -COOH and -$NH_2$,
$R^{16}$ is independently selected from halogen, -C(O)$OC_1$-$C_6$-alkyl, -COOH, -CN, -$CF_3$, -$OCF_3$, - $NO_2$, -OH, -$OC_1$-$C_6$-alkyl, -$NH_2$, C(=O) or $C_1$-$C_6$-alkyl, or any enantiomer, diastereomer, including a racemic mixture, tautomer as well as a salt thereof with a pharmaceutically acceptable acid or base.

**3.** A pharmaceutical composition according to claim 2 wherein X is =O or =S.

**4.** A pharmaceutical composition according to claim 3 wherein X is =O.

**5.** A pharmaceutical composition according to claim 3 wherein X is =S.

**6.** A pharmaceutical composition according to any one of the claims 2 to 5 wherein Y is -O- or -S-.

**7.** A pharmaceutical composition according to claim 6 wherein Y is -O-.

**8.** A pharmaceutical composition according to claim 6 wherein Y is -NH-.

**9.** A pharmaceutical composition according to claim 6 wherein Y is -S-.

**10.** A pharmaceutical composition according to any one of the claims 2 to 9 wherein A is aryl optionally substituted with up to four substituents, $R^7$, $R^8$, $R^9$, and $R^{10}$ which may be the same or different.

**11.** A pharmaceutical composition according to claim 10 wherein A is selected from ArG1 optionally substituted with up to four substituents, $R^7$, $R^8$, $R^9$, and $R^{10}$ which may be the same or different.

**12.** A pharmaceutical composition according to claim 11 wherein A is phenyl or naphtyl optionally substituted with up to four substituents, $R^7$, $R^8$, $R^9$, and $R^{10}$ which may be the same or different.

**13.** A pharmaceutical composition according to claim 12 wherein A is

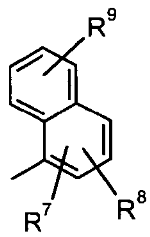

or

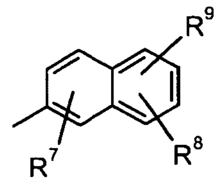

**14.** A pharmaceutical composition according to claim 12 wherein A is phenyl.

**15.** A pharmaceutical composition according to any one of the claims 2 to 9 wherein A is heteroaryl optionally substituted with up to four substituents, $R^7$, $R^8$, $R^9$, and $R^{10}$ which may be the same or different.

**16.** A pharmaceutical composition according to claim 15 wherein A is selected from Het1 optionally substituted with up to four substituents, $R^7$, $R^8$, $R^9$, and $R^{10}$ which may be the same or different.

**17.** A pharmaceutical composition according to claim 16 wherein A is selected from Het2 optionally substituted with up to four substituents, $R^7$, $R^8$, $R^9$, and $R^{10}$ which may be the same or different.

**18.** A pharmaceutical composition according to claim 17 wherein A is selected from Het3 optionally substituted with up to four substituents, $R^7$, $R^8$, $R^9$, and $R^{10}$ which may be the same or different.

**19.** A pharmaceutical composition according to claim 18 wherein A is selected from the group consisting of indolyl, benzofuranyl, quinolyl, furyl, thienyl, or pyrrolyl, wherein each heteroaryl may optionally substituted with up to four substituents, $R^7$, $R^8$, $R^9$, and $R^{10}$ which may be the same or different.

**20.** A pharmaceutical composition according to claim 18 wherein A is benzofuranyl optionally substituted with up to four substituents $R^7$, $R^8$, $R^9$, and $R^{10}$ which may be the same or different.

**21.** A pharmaceutical composition according to claim 20 wherein A is

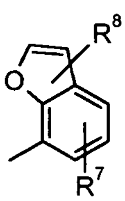

or

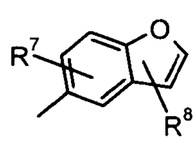

or

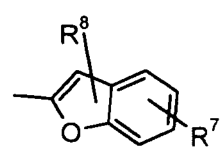

**22.** A pharmaceutical composition according to claim 18 wherein A is carbazolyl optionally substituted with up to four substituents $R^7$, $R^8$, $R^9$, and $R^{10}$ which may be the same or different.

**23.** A pharmaceutical composition according to claim 22 wherein A is

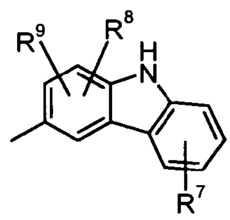

**24.** A pharmaceutical composition according to claim 18 wherein A is quinolyl optionally substituted with up to four substituents $R^7$, $R^8$, $R^9$, and $R^{10}$ which may be the same or different.

**25.** A pharmaceutical composition according to claim 24 wherein A is

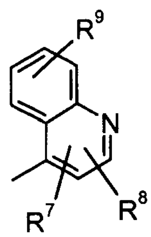

or

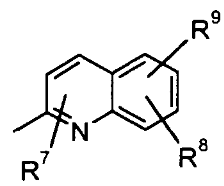

**26.** A pharmaceutical composition according to claim 18 wherein A is indolyl optionally substituted with up to four substituents $R^7$, $R^8$, $R^9$, and $R^{10}$ which may be the same or different.

**27.** A pharmaceutical composition according to claim 26 wherein A is

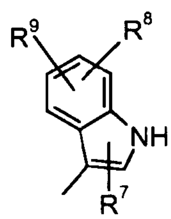

or

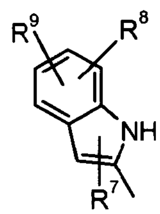

**28.** A pharmaceutical composition according to any one of the claims 2 to 27 wherein $R^1$ is hydrogen.

**29.** A pharmaceutical composition according to any one of the claims 2 to 28 wherein $R^2$ is hydrogen.

**30.** A pharmaceutical composition according to any one of the claims 2 to 27 wherein $R^1$ and $R^2$ are combined to form a double bond.

**31.** A pharmaceutical composition according to any one of the claims 2 to 30 wherein $R^3$ is $C_1$-$C_6$-alkyl, halogen, or $C(O)NR^{16}R^{17}$.

**32.** A pharmaceutical composition according to claim 31 wherein $R^3$ is $C_1$-$C_6$-alkyl or $C(O)NR^{16}R^{17}$.

**33.** A pharmaceutical composition according to claim 32 wherein $R^3$ is methyl.

**34.** A pharmaceutical composition according to any one of the claims 2 to 9 wherein B is phenyl optionally substituted with up to four substituents, $R^7$, $R^8$, $R^9$, and $R^{10}$ which may be the same or different.

**35.** A pharmaceutical composition according to any one of the claims 2 to 9 or 34 wherein $R^4$ is hydrogen.

**36.** A pharmaceutical composition according to any one of the claims 2 to 9 or 34 to 35 wherein $R^5$ is hydrogen.

**37.** A pharmaceutical composition according to any one of the claims 2 to 36 wherein $R^6$ is aryl.

**38.** A pharmaceutical composition according to claim 37 wherein $R^6$ is phenyl.

**39.** A pharmaceutical composition according to any one of the claims 2 to 38 wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-NR^{11}S(O)_2R^{12}$, $-S(O)_2NR^{11}R^{12}$, $-S(O)NR^{11}R^{12}$, $-S(O)R^{11}$, $-S(O)_2R^{11}$, $-OS(O)_2 R^{11}$, $-NR^{11}C(O)R^{12}$, $-CH_2OR^{11}$,$-CH_2OC(O)R^{11}$, $-CH_2NR^{11}R^{12}$, $-OC(O)R^{11}$, $-OC_1$-$C_6$-alkyl-$C(O)OR^{11}$, $-OC_1$-$C_6$-alkyl-$C(O)NR^{11}R^{12}$, $-OC_1$-$C_6$-alkyl-$OR^{11}$ $-SC_1$-$C_6$-alkyl-$C(O)OR^{11}$, $-C_2$-$C_6$-alkenyl-$C(=O)OR^{11}$, $-C(O)OR^{11}$, or-$C_2$-$C_6$-alkenyl-$C(=O)R^{11}$,
- $C_1$-$C_6$-alkyl, $C_2$-$C_6$-alkenyl or $C_2$-$C_6$-alkynyl, which may each optionally be substituted with one or more substituents independently selected from $R^{13}$
- aryl, aryloxy, aroyl, arylsulfanyl, aryl-$C_1$-$C_6$-alkoxy, aryl-$C_1$-$C_6$-alkyl, aryl-$C_2$-$C_6$-alkenyl, aroyl-$C_2$-$C_6$-alkenyl, aryl-$C_2$-$C_6$-alkynyl, heteroaryl, heteroaryl-$C_1$-$C_6$-alkyl, wherein each of the cyclic moieties optionally may be substituted with one or more substituents independently selected from $R^{14}$

**40.** A pharmaceutical composition according to claim 39 wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2 R^{11}$,$-CH_2OC(O)R^{11}$, $-OC(O)R^{11}$, $-OC_1$-$C_6$-alkyl-$C(O)OR^{11}$, $-OC_1$-$C_6$-alkyl-$OR^{11}$, $-SC_1$-$C_6$-alkyl-$C(O)OR^{11}$, $-C(O)OR^{11}$, or $-C_2$-$C_6$-alkenyl-$C(=O)R^{11}$,
- $C_1$-$C_6$-alkyl or $C_1$-$C_6$-alkenyl which may each optionally be substituted with one or more substituents independently selected from $R^{13}$
- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$

**41.** A pharmaceutical composition according to claim 40 wherein $R^7$, $R^8$, $R^9$ and $R^{10}$ are independently selected from

- hydrogen, halogen, $-NO_2$, $-OR^{11}$, $-NR^{11}R^{12}$, $-SR^{11}$, $-S(O)_2R^{11}$, $-OS(O)_2 R^{11}$, - $CH^2OC(O)R^{11}$, $-OC(O)R^{11}$, $-OC_1$-$C_6$-alkyl-$C(O)OR^{11}$, $-OC_1$-$C_6$-alkyl-$OR^{11}$, $-SC_1$-$C_6$-alkyl-$C(O)OR^{11}$, $-C(O)OR^{11}$, or $-C_2$-$C_6$-alkenyl-$C(=O)R^{11}$,
- $C_1$-$C_6$-alkyl or $C_1$-$C_6$- which may each optionally be substituted with one or more substituents independently selected from $R^{13}$
- aryl, aryloxy, aroyl, aryl-$C_1$-$C_6$-alkoxy, aryl-$C_1$-$C_6$-alkyl, heteroaryl,

of which each of the cyclic moieties optionally may be substituted with one or more substituents independently selected from $R^{14}$.

**42.** A pharmaceutical composition according to claim 41 wherein $R^7$, $R^8$, $R^9$ and $R^{10}$ are independently selected from

- hydrogen, halogen, $-OR^{11}$, $-OC_1$-$C_6$-alkyl-$C(O)OR^{11}$, or-$C(O)OR^{11}$,
- $C_1$-$C_6$-alkyl which may each optionally be substituted with one or more substituents independently selected from $R^{13}$
- aryl, aryloxy, aryl-$C_1$-$C_6$-alkoxy,

of which each of the cyclic moieties optionally may be substituted with one or more substituents independently selected from $R^{14}$.

**43.** A pharmaceutical composition according to claim 42 wherein $R^7$, $R^8$, $R^9$ and $R^{10}$ are independently selected from

- hydrogen, halogen, $-OR^{11}$, $-OC_1$-$C_6$-alkyl-$C(O)OR^{11}$, or $-C(O)OR^{11}$,
- $C_1$-$C_6$-alkyl which may each optionally be substituted with one or more substituents independently selected from $R^{13}$
- ArG1, ArG1oxy, ArG1-$C_1$-$C_6$-alkoxy,

of which each of the cyclic moieties optionally may be substituted with one or more substituents independently selected from $R^{14}$.

**44.** A pharmaceutical composition according to claim 43 wherein $R^7$, $R^8$, $R^9$ and $R^{10}$ are independently selected from

- hydrogen, halogen, $-OR^{11}$, $-OC_1-C_6$-alkyl-$C(O)OR^{11}$, or-$C(O)OR^{11}$,
- $C_1-C_6$-alkyl which may optionally be substituted with one or more substituents independently selected from $R^{13}$
- phenyl, phenyloxy, phenyl-$C_1-C_6$-alkoxy, wherein each of the cyclic moieties optionally may be substituted with one or more substituents independently selected from $R^{14}$.

**45.** A pharmaceutical composition according to any one of the claims 2 to 44 wherein $R^{11}$ and $R^{12}$ are independently selected from hydrogen, $C_1-C_{20}$-alkyl, aryl or aryl-$C_1-C_6$-alkyl, wherein the alkyl groups may optionally be substituted with one or more substituents independently selected from $R^{15}$, and the aryl groups may optionally be substituted one or more substituents independently selected from $R^{16}$; $R^{11}$ and $R^{12}$ when attached to the same nitrogen atom may form a 3 to 8 membered heterocyclic ring with the said nitrogen atom, the heterocyclic ring optionally containing one or two further heteroatoms selected from nitrogen, oxygen and sulphur, and optionally containing one or two double bonds.

**46.** A pharmaceutical composition according to claim 45 wherein $R^{11}$ and $R^{12}$ are independently selected from hydrogen, $C_1-C_{20}$-alkyl, aryl or aryl-$C_1-C_6$-alkyl, wherein the alkyl groups may optionally be substituted with one or more substituents independently selected from $R^{15}$, and the aryl groups may optionally be substituted one or more substituents independently selected from $R^{16}$.

**47.** A pharmaceutical composition according to claim 46 wherein $R^{11}$ and $R^{12}$ are independently selected from phenyl or phenyl-$C_1-C_6$-alkyl.

**48.** A pharmaceutical composition according to claim 46 wherein one or both of $R^{11}$ and $R^{12}$ are methyl.

**49.** A pharmaceutical composition according to any one of the claims 2 to 48 wherein $R^{13}$ is independently selected from halogen, $CF_3$, $OR^{11}$ or $NR^{11}R^{12}$.

**50.** A pharmaceutical composition according to claim 49 wherein $R^{13}$ is independently selected from halogen or $OR^{11}$.

**51.** A pharmaceutical composition according to claim 50 wherein $R^{13}$ is $OR^{11}$.

**52.** A pharmaceutical composition according to any one of the claims 2 to 51 wherein $R^{14}$ is independently selected from halogen, $-C(O)OR^{11}$, -CN, $-CF_3$, $-OR^{11}$, $S(O)_2R^{11}$, and $C_1-C_6$-alkyl.

**53.** A pharmaceutical composition according to claim 52 wherein $R^{14}$ is independently selected from halogen, $-C(O)OR^{11}$, or $-OR^{11}$.

**54.** A pharmaceutical composition according to any one of the claims 2 to 53 wherein $R^{15}$ is independently selected from halogen, -CN, $-CF_3$, $-C(O)OC_1-C_6$-alkyl, and -COOH.

**55.** A pharmaceutical composition according to claim 54 wherein $R^{15}$ is independently selected from halogen or $-C(O)OC_1-C_6$-alkyl.

**56.** A pharmaceutical composition according to any one of the claims 2 to 55 wherein $R^{16}$ is independently selected from halogen, $-C(O)OC_1-C_6$-alkyl, -COOH, $-NO_2$, $-OC_1-C_6$-alkyl, $-NH_2$, C(=O) or $C_1-C_6$-alkyl.

**57.** A pharmaceutical composition according to claim 56 wherein $R^{16}$ is independently selected from halogen, $-C(O)OC_1-C_6$-alkyl, -COOH, $-NO_2$, or $C_1-C_6$-alkyl.

**58.** A pharmaceutical composition according to any one of the claims 1 to 57 wherein the insulin is rapid acting insulin.

**59.** A pharmaceutical composition according to any one of the claims 1 to 58 wherein the insulin is selected from the group consisting of human insulin, an analogue thereof, a derivative thereof, and combinations of any of these.

**60.** A pharmaceutical composition according to claim 59 wherein the insulin is an analogue of human insulin selected from the group consisting of

i.An analogue wherein position B28 is Asp, Lys, Leu, Val, or Ala and position B29 is Lys or Pro; and
ii. des (B28-B30), des (B27) or des(B30) human insulin.

**61.** A pharmaceutical composition according to claim 60, wherein the insulin is an analogue of human insulin wherein position B28 is Asp or Lys, and position B29 is Lys or Pro.

**62.** A pharmaceutical composition according to claim 60 wherein the insulin is des(B30) human insulin.

**63.** A pharmaceutical composition according to claim 60 wherein the insulin is is an analogue of human insulin wherein position B3 is Lys and position B29 is Glu or Asp.

**64.** A pharmaceutical composition according to claim 59 wherein the insulin is a derivative of human insulin having one or more lipophilic substituents.

**65.** A pharmaceutical composition according to claim 64 wherein the insulin derivative is selected from the group consisting of B29-$N^{\varepsilon}$-myristoyl-des(B30) human insulin, B29-$N^{\varepsilon}$-palmitoyl-des(B30) human insulin, B29-$N^{\varepsilon}$-myristoyl human insulin, B29-$N^{\varepsilon}$-palmitoyl human insulin, B28-$N^{\varepsilon}$-myristoyl $Lys^{B28}$ $Pro^{B29}$ human insulin, B28-$N^{\varepsilon}$-palmitoyl $Lys^{B28}$ $Pro^{B29}$ human insulin, B30-$N^{\varepsilon}$-myristoyl-$Thr^{B29}Lys^{B30}$ human insulin, B30-$N^{\varepsilon}$-palmitoyl-$Thr^{B29}Lys^{B30}$ human insulin, B29-$N^{\varepsilon}$-(N-palmitoyl-γ-glutamyl)-des(B30) human insulin, B29-$N^{\varepsilon}$-(N-lithocholyl-γ-glutamyl)-des(B30) human insulin, B29-$N^{\varepsilon}$-(ω-carboxyheptadecanoyl)-des(B30) human insulin and B29-$N^{\varepsilon}$-(ω-carboxyheptadecanoyl) human insulin.

**66.** A pharmaceutical composition according to claim 65 wherein the insulin derivative is B29-$N^{\varepsilon}$-myristoyl-des(B30) human insulin.

**67.** A pharmaceutical composition according to any one of the claims 1 to 66 comprising 2-6 moles $zinc^{2+}$ ions per mole insulin.

**68.** A pharmaceutical composition according to claim 67 comprising 2-3 moles $zinc^{2+}$ ions per mole insulin.

**69.** A pharmaceutical composition according to any one of the claims 1 to 68 further comprising at least 3 molecules of a phenolic compound per insulin hexamer.

**70.** A pharmaceutical composition according to any one of the claims 1 to 69 further comprising an isotonicity agent.

**71.** A pharmaceutical composition according to any one of the claims 1 to 70 further comprising a buffer substance.

**72.** A method of stabilising an insulin composition comprising adding a zinc-binding ligand according to any one of the claims 1 to 57 to the insulin composition.

## Patentansprüche

**1.** Arzneimittel, umfassend Insulin und einen zinkbindenden Liganden, der reversibel an eine $His^{B10}$-$Zn^{2+}$-Stelle eines Insulinhexamers bindet, wobei der Ligand ausgewählt ist aus der Gruppe, bestehend aus Benzotriazolen, 3-Hydroxy-2-naphthoesäuren, Salicylsäuren, Tetrazolen, Thiazolidindionen, 5-Mercaptotetrazolen oder 4-Cyano-1,2,3-triazolen, oder einem Enantiomeren, Diastereomeren, einschließlich eines racemischen Gemischs, Tautomeren, sowie einem Salz davon mit einer pharmazeutisch verträglichen Säure oder Base.

**2.** Arzneimittel nach Anspruch 1, wobei der zinkbindende Ligand

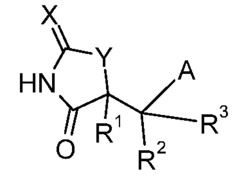 oder 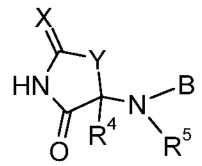 oder 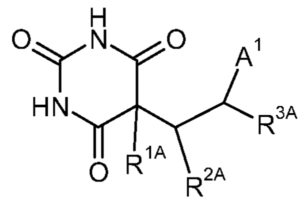

wobei

X =O, =S oder =NH ist,
-S-, -O- oder -NH- ist,
$R^1$, $R^{1A}$ und $R^4$ unabhängig ausgewählt sind aus Wasserstoff oder $C_1$-$C_6$-Alkyl,
$R^2$ und $R^{2A}$ Wasserstoff oder $C_1$-$C_6$-Alkyl oder Aryl sind, $R^1$ und $R^2$ wahlweise unter Bildung einer Doppelbindung kombiniert werden können, $R^{1A}$ und $R^{2A}$ wahlweise unter Bildung einer Doppelbindung kombiniert werden können,
$R^3$, $R^{3A}$ und $R^5$ unabhängig ausgewählt sind aus Wasserstoff, Halogen, Aryl, wahlweise substituiert mit einem oder mehreren Substituenten, ausgewählt aus $R^{16}$, $C_1$-$C_6$-Alkyl oder -C(O)$NR^{11}R^{12}$,
A, $A^1$ und B unabhängig ausgewählt sind aus $C_1$-$C_6$-Alkyl, Aryl, Aryl-$C_1$-$C_6$-alkyl, -$NR^{11}$-aryl, Aryl-$C_2$-$C_6$-alkenyl oder Heteroaryl, wobei das Alkyl oder Alkenyl wahlweise substituiert ist mit einem oder mehreren Substituenten, ausgewählt aus $R^6$ und das Aryl oder Heteroaryl wahlweise substituiert ist mit bis zu vier Substituenten $R^7$, $R^8$, $R^9$ und $R^{10}$,
A und $R^3$ durch eine oder zwei Valenzbindungen verbunden sein können, B und $R^5$ durch eine oder zwei Valenzbindungen verbunden sein können,
$R^6$ unabhängig ausgewählt ist aus Halogen, -CN, -$CF_3$, -$OCF_3$, Aryl, -COOH und -$NH_2$,
$R^7$, $R^8$, $R^9$ und $R^{10}$ unabhängig ausgewählt sind aus

- Wasserstoff, Halogen, -CN, -$CH_2$CN, -$CHF_2$, -$CF_3$, -$OCF_3$, -$OCHF_2$, -$OCH_2CF_3$, -$OCF_2CHF_2$, -S(O)$_2CF_3$, -OS(O)$_2CF_3$, -$SCF_3$, -$NO_2$, -$OR^{11}$, -$NR^{11}R^{12}$, -$SR^{11}$, -$NR^{11}$S(O)$_2R^{12}$, -S(O)$_2NR^{11}R^{12}$, -S(O)$NR^{11}R^{12}$, -S (O)$R^{11}$, -S(O)$_2R^{11}$, -OS(O)$_2$ $R^{11}$, -C(O)$NR^{11}R^{12}$, -OC(O)$NR^{11}R^{12}$, -$NR^{11}$C(O)$R^{12}$, -$CH_2$C(O)$NR^{11}R^{12}$, -O$C_1$-$C_6$-Alkyl-C(O)$NR^{11}R^{12}$, -$CH_2OR^{11}$, -$CH_2$OC(O)$R^{11}$, -$CH_2NR^{11}R^{12}$, -OC(O)$R^{11}$, -O$C_1$-$C_{15}$-Alkyl-C(O) $OR^{11}$, -O$C_1$-$C_6$-Alkyl-$OR^{11}$, -S$C_1$-$C_6$-Alkyl-C(O)$OR^{11}$, -$C_2$-$C_6$-Alkenyl-C(=O)$OR^{11}$, -$NR^{11}$-C(=O)-$C_1$-$C_6$-Alkyl-C(=O)$OR^{11}$, -$NR^{11}$-C(=O)-$C_1$-$C_6$-Alkenyl-C(=O)$OR^{11}$ , -C(O)$OR^{11}$, C(O)$R^{11}$ oder -$C_2$-$C_6$-Alkenyl-C (=O)$R^{11}$, =O oder -$C_2$-$C_6$-Alkenyl-C(=O)-$NR^{11}R^{12}$,
- $C_1$-$C_6$-Alkyl, $C_2$-$C_6$-Alkenyl oder $C_2$-$C_6$-Alkinyl, wobei jedes davon wahlweise substituiert sein kann mit einem oder mehrere Substituenten, unabhängig ausgewählt aus $R^{13}$,
- Aryl, Aryloxy, Aryloxycarbonyl, Aroyl, Arylsulfanyl, Aryl-$C_1$-$C_6$-alkoxy, Aryl-$C_1$-$C_6$-alkyl, Aryl-$C_2$-$C_6$-alkenyl, Aroyl-$C_2$-$C_6$-alkenyl, Aryl-$C_2$-$C_6$-alkinyl, Heteroaryl, Heteroaryl-$C_1$-$C_6$-alkyl, Heteroaryl-$C_2$-$C_6$-alkenyl, Heteroaryl-$C_2$-$C_6$-alkinyl oder $C_3$-$C_6$-Cycloalkyl,

wobei jede cyclische Einheit davon wahlweise substituiert sein kann mit einem oder mehreren Substituenten, unabhängig ausgewählt aus $R^{14}$,
$R^{11}$ und $R^{12}$ unabhängig ausgewählt sind aus Wasserstoff, OH, $C_1$-$C_{20}$-Alkyl, Aryl-$C_1$-$C_6$-alkyl oder Aryl, wobei die Alkylgruppen wahlweise substituiert sein können mit einem oder mehreren Substituenten, ausgewählt aus $R^{15}$, und die Arylgruppen wahlweise substituiert sein können mit einem oder mehreren Substituenten, ausgewählt aus $R^{16}$; $R^{11}$ und $R^{12}$, falls sie am selben Stickstoffatom angelagert sind einen 2- bis 8-gliedrigen heterocyclischen Ring mit dem Stickstoffatom bilden können, wobei der heterocyclische Ring wahlweise ein oder zwei weitere Heteroatome, ausgewählt aus Stickstoff, Sauerstoff und Schwefel, und wahlweise eine oder zwei Doppelbindungen enthält,
$R^{13}$ unabhängig ausgewählt ist aus Halogen, -CN, -$CF_3$, -$OCF_3$, -$OR^{11}$, -C(O)$OR^{11}$, -$NR^{11}R^{12}$ und -C(O) $NR^{11}R^{12}$,
$R^{14}$ unabhängig ausgewählt ist aus Halogen, -C(O)$OR^{11}$, -$CH_2$C(O)$OR^{11}$, -$CH_2OR^{11}$, -CN, -$CF_3$, -$OCF_3$, -$NO_2$, -$OR^{11}$, -$NR^{11}R^{12}$, -$NR^{11}$C(O)$R^{11}$, -S(O)$_2R^{11}$ Aryl und $C_1$-$C_6$-Alkyl,
$R^{15}$ unabhängig ausgewählt ist aus Halogen, -CN, -$CF_3$, =O, -$OCF_3$, -O$C_1$-$C_6$-Alkyl, -C(O)O$C_1$-$C_6$-Alkyl, -COOH und -$NH_2$,
$R^{16}$ unabhängig ausgewählt ist aus Halogen, -C(O)O$C_1$-$C_6$-Alkyl, -COOH, -CN, -$CF_3$, -$OCF_3$, -$NO_2$, -OH,

$-OC_1-C_6$-Alkyl, $-NH_2$, C(=O) oder $C_1-C_6$-Alkyl, oder ein beliebiges Enantiomer, Diastereomer, einschließlich eines racemischen Gemischs, Tautomer, sowie ein Salz davon mit einer pharmazeutisch verträglichen Säure oder Base ist.

**3.** Arzneimittel nach Anspruch 2, wobei X =O oder =S ist.

**4.** Arzneimittel nach Anspruch 3, wobei X =O ist.

**5.** Arzneimittel nach Anspruch 3, wobei X =S ist.

**6.** Arzneimittel nach einem der Ansprüche 2 bis 5, wobei Y -O- oder -S- ist.

**7.** Arzneimittel nach Anspruch 6, wobei Y -O- ist.

**8.** Arzneimittel nach Anspruch 6, wobei Y -NH- ist.

**9.** Arzneimittel nach Anspruch 6, wobei Y -S- ist.

**10.** Arzneimittel nach einem der Ansprüche 2 bis 9, wobei A Aryl ist, wahlweise substituiert mit bis zu vier Substituenten $R^7$, $R^8$, $R^9$ und $R^{10}$, die gleich oder verschieden sein können.

**11.** Arzneimittel nach Anspruch 10, wobei A ausgewählt ist aus ArG1, wahlweise substituiert mit bis zu vier Substituenten $R^7$, $R^8$, $R^9$ und $R^{10}$, die gleich oder verschieden sein können.

**12.** Arzneimittel nach Anspruch 11, wobei A Phenyl oder Naphthyl ist, wahlweise substituiert mit bis zu vier Substituenten $R^7$, $R^8$, $R^9$ und $R^{10}$, die gleich oder verschieden sein können.

**13.** Arzneimittel nach Anspruch 12, wobei A

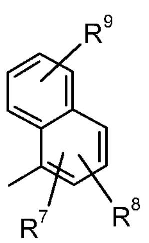 oder 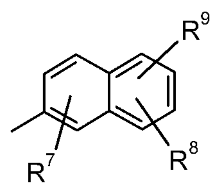

ist.

**14.** Arzneimittel nach Anspruch 12, wobei A Phenyl ist

**15.** Arzneimittel nach einem der Ansprüche 2 bis 9, wobei A Heteroaryl ist, wahlweise substituiert mit bis zu vier Substituenten $R^7$, $R^8$, $R^9$ und $R^{10}$, die gleich oder verschieden sein können.

**16.** Arzneimittel nach Anspruch 15, wobei A ausgewählt ist aus Het1, wahlweise substituiert mit bis zu vier Substituenten $R^7$, $R^8$, $R^9$, und $R^{10}$, die gleich oder verschieden sein können.

**17.** Arzneimittel nach Anspruch 16, wobei A ausgewählt ist aus Het2, wahlweise substituiert mit bis zu vier Substituenten $R^7$, $R^8$, $R^9$, und $R^{10}$, die gleich oder verschieden sein können.

**18.** Arzneimittel nach Anspruch 17, wobei A ausgewählt ist aus Het3, wahlweise substituiert mit bis zu vier Substituenten $R^7$, $R^8$, $R^9$, und $R^{10}$, die gleich oder verschieden sein können.

**19.** Arzneimittel nach Anspruch 18, wobei A ausgewählt ist aus der Gruppe, bestehend aus Indolyl, Benzofuranyl,

Chinolyl, Furyl, Thienyl oder Pyrrolyl, wobei jedes Heteroaryl wahlweise substituiert sein kann mit bis zu vier Substituenten $R^7$, $R^8$, $R^9$, und $R^{10}$, die gleich oder verschieden sein können.

**20.** Arzneimittel nach Anspruch 18, wobei A Benzofuranyl ist, wahlweise substituiert mit bis zu vier Substituenten $R^7$, $R^8$, $R^9$, und $R^{10}$, die gleich oder verschieden sein können.

**21.** Arzneimittel nach Anspruch 20, wobei A

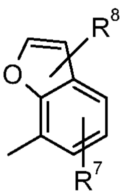

oder

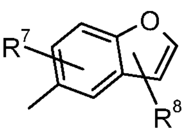

oder

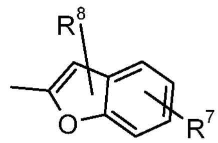

ist.

**22.** Arzneimittel nach Anspruch 18, wobei A Carbazolyl ist, wahlweise substituiert mit bis zu vier Substituenten $R^7$, $R^8$, $R^9$, und $R^{10}$, die gleich oder verschieden sein können.

**23.** Arzneimittel nach Anspruch 22, wobei A

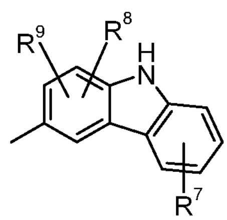

ist.

**24.** Arzneimittel nach Anspruch 18, wobei A Chinolyl ist, wahlweise substituiert mit bis zu vier Substituenten $R^7$, $R^8$, $R^9$, und $R^{10}$, die gleich oder verschieden sein können.

**25.** Arzneimittel nach Anspruch 24, wobei A

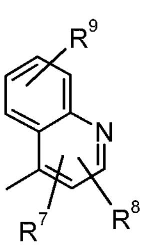

oder

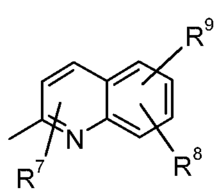

ist.

**26.** Arzneimittel nach Anspruch 18, wobei A Indolyl ist, wahlweise substituiert mit bis zu vier Substituenten $R^7$, $R^8$, $R^9$, und $R^{10}$, die gleich oder verschieden sein können.

**27.** Arzneimittel nach Anspruch 26, wobei A

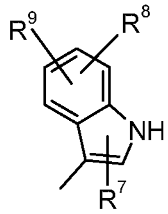

oder

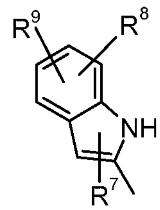

ist.

**28.** Arzneimittel nach einem der Ansprüche 2 bis 27, wobei $R^1$ Wasserstoff ist.

**29.** Arzneimittel nach einem der Ansprüche 2 bis 28, wobei $R^2$ Wasserstoff ist.

**30.** Arzneimittel nach einem der Ansprüche 2 bis 27, wobei $R^1$ und $R^2$ unter Bildung einer Doppelbindung kombiniert sind.

**31.** Arzneimittel nach einem der Ansprüche 2 bis 30, wobei $R^3$ $C_1$-$C_6$-Alkyl, Halogen oder $C(O)NR^{16}R^{17}$ ist.

**32.** Arzneimittel nach Anspruch 31, wobei $R^3$ $C_1$-$C_6$-alkyl oder $C(O)NR^{16}R^{17}$ ist.

**33.** Arzneimittel nach Anspruch 32, wobei $R^3$ Methyl ist.

**34.** Arzneimittel nach einem der Ansprüche 2 bis 9, wobei B Phenyl ist, wahlweise substituiert mit bis zu vier Substituenten $R^7$, $R^8$, $R^9$, und $R^{10}$, die gleich oder verschieden sein können.

**35.** Arzneimittel nach einem der Ansprüche 2 bis 9 oder 34, wobei $R^4$ Wasserstoff ist.

**36.** Arzneimittel nach einem der Ansprüche 2 bis 9 oder 34 bis 35, wobei $R^5$ Wasserstoff ist.

**37.** Arzneimittel nach einem der Ansprüche 2 bis 36, wobei $R^6$ Aryl ist.

**38.** Arzneimittel nach Anspruch 37, wobei $R^6$ Phenyl ist.

**39.** Arzneimittel nach einem der Ansprüche 2 bis 38, wobei $R^7$, $R^8$ $R^9$ und $R^{10}$ unabhängig ausgewählt sind aus

- Wasserstoff Halogen, $-NO_2$, $-OR^{11}$, $-NR^{11}R^{12}$, $-SR^{11}$, $-NR^{11}S(O)_2R^{12}$, $-S(O)_2NR^{11}R^{12}$, $-S(O)NR^{11}R^{12}$, $-S(O)R^{11}$, $-S(O)_2R^{11}$, $-OS(O)_2\ R^{11}$, $-NR^{11}C(O)R^{12}$, $-CH_2OR^{11}$, , $-CH_2OC(O)R^{11}$, $-CH_2NR^{11}R^{12}$, $-OC(O)R^{11}$, $-OC_1$-$C_6$-Alkyl-$C(O)OR^{11}$, $-OC_1$-$C_6$-Alkyl-$C(O)NR^{11}R^{12}$, $-OC_1$-$C_6$-Alkyl-$OR^{11}$, $-SC_1$-$C_6$-Alkyl-$C(O)OR^{11}$, $-C_2$-$C_6$-Alkenyl-$C(=O)OR^{11}$, $-C(O)OR^{11}$ oder $-C_2$-$C_6$-Alkenyl-$C(=O)R^{11}$,
- $C_1$-$C_6$-Alkyl, $C_2$-$C_6$-Alkenyl oder $C_2$-$C_6$-Alkinyl, wobei jedes davon wahlweise substituiert sein kann mit einem oder mehreren Substituenten, unabhängig ausgewählt aus $R^{13}$,
- Aryl, Aryloxy, Aroyl, Arylsulfanyl, Aryl-$C_1$-$C_6$-alkoxy, Aryl-$C_1$-$C_6$-alkyl, Aryl-$C_2$-$C_6$-alkenyl, Aroyl-$C_2$-$C_6$-alkenyl, Aryl-$C_2$-$C_6$-alkinyl, Heteroaryl, Heteroaryl-$C_1$-$C_6$-alkyl, wobei jede der cyclischen Einheiten wahlweise substituiert sein kann mit einem oder mehreren Substituenten, unabhängig ausgewählt aus $R^{14}$,

**40.** Arzneimittel nach Anspruch 39, wobei $R^7$, $R^8$, $R^9$ und $R^{10}$ unabhängig ausgewählt sind aus

- Wasserstoff, Halogen, $-NO_2$, $-OR^{11}$, $-NR^{11}R^{12}$, $-SR^{11}$, $-S(O)_2R^{11}$, $-OS(O)_2\ R^{11}$, $-CH_2OC(O)R^{11}$, $-OC(O)R^{11}$, $-OC_1$-$C_6$-Alkyl-$C(O)OR^{11}$, $-OC_1$-$C_6$-Alkyl-$OR^{11}$, $-SC_1$-$C_6$-Alkyl-$C(O)OR^{11}$, $-C(O)OR^{11}$ oder -C2-$C_6$-Alkenyl-$C(=O)R^{11}$,
- $C_1$-$C_6$-Alkyl oder $C_1$-$C_6$-Alkenyl, wobei jedes davon wahlweise substituiert sein kann mit einem oder mehreren Substituenten, unabhängig ausgewählt aus $R^{13}$,
- Aryl, Aryloxy, Aroyl, Aryl-$C_1$-$C_6$-alkoxy, Aryl-$C_1$-$C_6$-alkyl, Heteroaryl,

wobei jede der cyclischen Einheiten davon wahlweise substituiert sein kann mit einem oder mehreren Substituenten, unabhängig ausgewählt aus $R^{14}$.

**41.** Arzneimittel nach Anspruch 40, wobei $R^7$, $R^8$, $R^9$ und $R^{10}$ unabhängig ausgewählt sind aus

- Wasserstoff, Halogen, $-NO_2$, $-OR^{11}$, $-NR^{11}R^{12}$, $-SR^{11}$, $-S(O)_2R^{11}$, $-OS(O)_2 R^{11}$, $-CH_2OC(O)R^{11}$, $-OC(O)R^{11}$, $-OC_1$-$C_6$-Alkyl-$C(O)OR^{11}$, $-OC_1$-$C_6$-Alkyl-$OR^{11}$, $-SC_1$-$C_6$-Alkyl-$C(O)OR^{11}$, $-C(O)OR^{11}$ oder $-C_2$-$C_6$-Alkenyl-C (=O)$R^{11}$,
- $C_1$-$C_6$-Alkyl oder $C_1$-$C_6$-, wobei jedes davon wahlweise substituiert sein kann mit einem oder mehreren Substituenten, unabhängig ausgewählt aus $R^{13}$,
- Aryl, Aryloxy, Aroyl, Aryl-$C_1$-$C_6$-Alkoxy, Aryl-$C_1$-$C_6$-alkyl, Heteroaryl,

wobei jede der cyclischen Einheiten davon wahlweise substituiert sein kann mit einem oder mehreren Substituenten, unabhängig ausgewählt aus $R^{14}$.

**42.** Arzneimittel nach Anspruch 41, wobei $R^7$, $R^8$, $R^9$ und $R^{10}$ unabhängig ausgewählt sind aus

- Wasserstoff, Halogen, $-OR^{11}$, $-OC_1$-$C_6$-Alkyl-$C(O)OR^{11}$ oder $-C(O)OR^{11}$,
- $C_1$-$C_6$-Alkyl, wobei jedes davon wahlweise substituiert sein kann mit einem oder mehreren Substituenten, unabhängig ausgewählt aus $R^{13}$,
- Aryl, Aryloxy, Aryl-$C_1$-$C_6$-alkoxy,

wobei jede der cyclischen Einheiten davon wahlweise substituiert sein kann mit einem oder mehreren Substituenten, unabhängig ausgewählt aus $R^{14}$.

**43.** Arzneimittel nach Anspruch 42, wobei $R^7$, $R^8$, $R^9$ und $R^{10}$ unabhängig ausgewählt sind aus

- Wasserstoff, Halogen, $-OR^{11}$, $-OC_1$-$C_6$-Alkyl-$C(O)OR^{11}$, oder $-C(O)OR^{11}$,
- $C_1$-$C_6$-Alkyl, wobei jedes davon wahlweise substituiert sein kann mit einem oder mehreren Substituenten, unabhängig ausgewählt aus $R^{13}$,
- ArG1, ArGloxy, ArG1-$C_1$-$C_6$-alkoxy,

wobei jede der cyclischen Einheiten davon wahlweise substituiert sein kann mit einem oder mehreren Substituenten, unabhängig ausgewählt aus $R^{14}$.

**44.** Arzneimittel nach Anspruch 43, wobei $R^7$, $R^8$, $R^9$ und $R^{10}$ unabhängig ausgewählt sind aus

- Wasserstoff, Halogen, $-OR^{11}$, $-OC_1$-$C_6$-Alkyl-$C(O)OR^{11}$ oder $-C(O)OR^{11}$,
- $C_1$-$C_6$-Alkyl, das wahlweise substituiert sein kann mit einem oder mehreren Substituenten, ausgewählt aus $R^{13}$,
- Phenyl, Phenyloxy, Phenyl-$C_1$-$C_6$-alkoxy, wobei jede der cyclischen Einheiten wahlweise substituiert sein kann mit einem oder mehreren Substituenten, unabhängig ausgewählt aus $R^{14}$.

**45.** Arzneimittel nach einem der Ansprüche 2 bis 44, wobei $R^{11}$ und $R^{12}$ unabhängig ausgewählt sind aus Wasserstoff, $C_1$-$C_{20}$-Alkyl, Aryl oder Aryl-$C_1$-$C_6$-alkyl, wobei die Alkylgruppen wahlweise substituiert sein können mit einem oder mehreren Substituenten, ausgewählt aus $R^{15}$, und die Arylgruppen wahlweise substituiert sein können mit einem oder mehreren Substituenten, ausgewählt aus $R^{16}$; $R^{11}$ und $R^{12}$, falls sie am selben Stickstoffatom angelagert sind, einen 3- bis 8-gliedrigen, heterocyclischen Ring mit dem Stickstoffatom bilden können, wobei der heterocyclische Ring wahlweise ein oder zwei weitere Heteroatome, ausgewählt aus Stickstoff, Sauerstoff und Schwefel, und wahlweise eine oder zwei Doppelbindungen enthält.

**46.** Arzneimittel nach Anspruch 45, wobei $R^{11}$ und $R^{12}$ unabhängig ausgewählt sind aus Wasserstoff, $C_1$-$C_{20}$-Alkyl, Aryl oder Aryl-$C_1$-$C_6$-alkyl, wobei die Alkylgruppen wahlweise substituiert sein können mit einem oder mehreren Substituenten, ausgewählt aus $R^{15}$, und die Arylgruppen wahlweise substituiert sein können mit einem oder mehreren Substituenten, ausgewählt aus $R^{16}$.

**47.** Arzneimittel nach Anspruch 46, wobei $R^{11}$ und $R^{12}$ unabhängig ausgewählt sind aus Phenyl oder Phenyl-$C_1$-$C_6$-Alkyl.

**48.** Arzneimittel nach Anspruch 46, wobei einer oder beide der Reste $R^{11}$ und $R^{12}$ Methyl sind.

**49.** Arzneimittel nach einem der Ansprüche 2 bis 48, wobei $R^{13}$ unabhängig ausgewählt ist aus Halogen, $CF_3$, $OR^{11}$ oder $NR^{11}R^{12}$.

**50.** Arzneimittel nach Anspruch 49, wobei $R^{13}$ unabhängig ausgewählt ist aus Halogen oder $OR^{11}$.

**51.** Arzneimittel nach Anspruch 50, wobei $R^{13}$ $OR^{11}$ ist.

**52.** Arzneimittel nach einem der Ansprüche 2 bis 51, wobei $R^{14}$ unabhängig ausgewählt ist aus Halogen, $-C(O)OR^{11}$, -CN, $-CF_3$, $-OR^{11}$, $S(O)_2R^{11}$ und $C_1$-$C_6$-Alkyl.

**53.** Arzneimittel nach Anspruch 52 wobei $R^{14}$ unabhängig ausgewählt ist aus Halogen, $-C(O)OR^{11}$ oder $-OR^{11}$.

**54.** Arzneimittel nach einem der Ansprüche 2 bis 53, wobei $R^{15}$ unabhängig ausgewählt ist aus Halogen, -CN, $-CF_3$, $-C(O)OC_1$-$C_6$-alkyl und -COOH.

**55.** Arzneimittel nach Anspruch 54, wobei $R^{15}$ unabhängig ausgewählt ist aus Halogen oder $-C(O)OC_1$-$C_6$-alkyl.

**56.** Arzneimittel nach einem der Ansprüche 2 bis 55, wobei $R^{16}$ unabhängig ausgewählt ist aus Halogen, $-C(O)OC_1$-$C_6$-Alkyl, -COOH, $-NO_2$, $-OC_1$-$C_6$-Alkyl, $-NH_2$, C(=O) oder $C_1$-$C_6$-Alkyl.

**57.** Arzneimittel nach Anspruch 56, wobei $R^{16}$ unabhängig ausgewählt ist aus Halogen, $-C(O)OC_1$-$C_6$-Alkyl, -COOH, $-NO_2$, oder $C_1$-$C_6$-Alkyl.

**58.** Arzneimittel nach einem der Ansprüche 1 bis 57, wobei das Insulin ein schnell wirkendes Insulin ist.

**59.** Arzneimittel nach einem der Ansprüche 1 bis 58, wobei das Insulin ausgewählt ist aus der Gruppe, bestehend aus Humaninsulin, einem Analogon davon, einem Derivat davon und Kombinationen von beliebigen derselben.

**60.** Arzneimittel nach Anspruch 59, wobei das Insulin ein Analogon von Humaninsulin ist, ausgewählt aus der Gruppe, bestehend aus

i. einem Analogon, wobei Position B28 Asp, Lys, Leu, Val oder Ala ist und Position B29 Lys oder Pro ist; und
ii. des(B28-B30)-, des(B27)- oder des(B30)-Humaninsulin.

**61.** Arzneimittel nach Anspruch 60, wobei das Insulin ein Analogon von Humaninsulin ist, wobei Position B28 Asp oder Lys ist und Position B29 Lys oder Pro ist.

**62.** Arzneimittel nach Anspruch 60, wobei das Insulin des(B30)-Humaninsulin ist.

**63.** Arzneimittel nach Anspruch 60, wobei das Insulin ein Analogon von Humaninsulin ist, wobei Position B3 Lys ist und Position B29 Glu oder Asp ist.

**64.** Arzneimittel nach Anspruch 59, wobei das Insulin ein Derivat von Humaninsulin mit einem oder mehreren lipophilen Substituenten ist.

**65.** Arzneimittel nach Anspruch 64, wobei das Insulinderivat ausgewählt ist aus der Gruppe, bestehend aus B29-$N^{\varepsilon}$-Myristoyl-des(B30)-Humaninsulin, B29-$N^{\varepsilon}$-Palmitoyl-des(B30)-Humaninsulin, B29-$N^{\varepsilon}$-Myristoyl-Humaninsulin, B29-$N^{\varepsilon}$-Palmitoyl-Humaninsulin, B28-$N^{\varepsilon}$-Myristoyl-$Lys^{B28}$-$Pro^{B29}$ -Humaninsulin, B28-$N^{\varepsilon}$-Palmitoyl-$Lys^{B28}$ $Pro^{B29}$-Humaninsulin, B30-$N^{\varepsilon}$-Myristoyl-$Thr^{B29}Lys^{B30}$-Humaninsulin, B30-$N^{\varepsilon}$-Palmitoyl-$Thr^{B29}Lys^{B30}$-Humaninsulin, B29-$N^{\varepsilon}$-(N-Palmitoyl-y-glutamyl)-des(B30)-Humaninsulin, B29-$N^{\varepsilon}$-(N-Lithocholyl-$\gamma$-glutamyl)-des(B30)-Humaninsulin, B29-$N^{\varepsilon}$-($\omega$-Carboxyheptadecanoyl)-des(B30)-Humaninsulin und B29-$N^{\varepsilon}$-($\omega$-Carboxyheptadecanoyl)-Humaninsulin.

**66.** Arzneimittel nach Anspruch 65, wobei das Insulinderivat B29-$N^{\varepsilon}$-Myristoyl-des(B30)-Humaninsulin ist.

**67.** Arzneimittel nach einem der Ansprüche 1 bis 66, umfassend 2-6 mol $Zink^{2+}$-Ionen pro mol Insulin.

**68.** Arzneimittel nach Anspruch 67, umfassend 2-3 mol $Zink^{2+}$-Ionen pro Mol Insulin.

**69.** Arzneimittel nach einem der Ansprüche 1 bis 68, ferner umfassend mindestens 3 Moleküle einer Phenolverbindung pro Insulinhexamer.

**70.** Arzneimittel nach einem der Ansprüche 1 bis 69, ferner umfassend ein Isotoniemittel.

**71.** Arzneimittel nach einem der Ansprüche 1 bis 70, ferner umfassend eine Puffersubstanz.

**72.** Verfahren zum Stabilisieren einer Insulinzusammensetzung, umfassend das Zusetzen eines zinkbindenden Liganden nach einem der Ansprüche 1 bis 57 zu der Insulinzusammensetzung.

**Revendications**

**1.** Composition pharmaceutique comprenant de l'insuline et un ligand se liant au zinc, qui se lie d'une manière réversible à un site $His^{B10}Zn^{2+}$ d'un hexamère de l'insuline, où le ligand est choisi dans l'ensemble consistant en les benzotriazoles, les acides 3-hydroxy-2-naphtoïques, les acides salicyliques, les tétrazoles, le thiazolidinediones, les 5-mercaptotétrazoles ou les 4-cyano-1,2,3-triazoles, ou tout énantiomère, diastéréoisomère, y compris un mélange racémique, un tautomère, ainsi qu'un sel de ces derniers avec un acide ou une base acceptable d'un point de vue pharmaceutique.

**2.** Composition pharmaceutique selon la revendication 1, dans laquelle le ligand se liant au zinc est

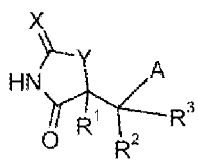 or 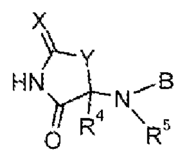 or 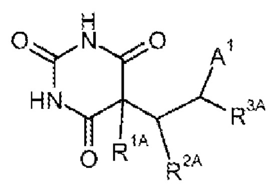

(or = ou)
où

X est =O, =S ou =NH,
Y est -S-, -O- ou -NH-
$R^1$, $R^{1A}$ et $R^4$ sont choisis d'une manière indépendante les uns des autres parmi l'atome d'hydrogène ou les groupes alkyle en $C_1$-$C_6$,
$R^2$ et $R^{2A}$ sont des atomes d'hydrogène ou des groupes alkyle en $C_1$-$C_6$ ou aryle, $R^1$ et $R^2$ peuvent en option être combinés pour former une double liaison, $R^{1A}$ et $R^{2A}$ peuvent en option être combinés pour former une double liaison,
$R^3$, $R^{3A}$ et $R^5$ sont choisis d'une manière indépendante les uns des autres chacun parmi l'atome d'hydrogène, les groupes halogéno, aryle en option substitué par un ou plusieurs substituants choisis d'une manière indépendante les uns des autres parmi $R^{16}$, les radicaux alkyle en $C_1$-$C_6$ ou -C(O)$NR^{11}R^{12}$,
A, $A^1$ et B sont choisis d'une manière indépendante les uns des autres parmi les radicaux alkyle en $C_1$-$C_6$, aryle, aryl (alkyle en $C_1$-$C_6$), -$NR^{11}$-aryle, aryl(alcényle en $C_2$-$C_6$) ou hétéroaryle, où les radicaux alkyle ou alcényle sont en option substitués par un ou plusieurs substituants choisis d'une manière indépendante parmi les radicaux $R^6$, et les radicaux aryle ou hétéroaryle sont en option substitués par jusqu'à quatre substituants $R^7$, $R^8$, $R^9$ et $R^{10}$,
A et $R^3$ peuvent être reliés par l'intermédiaire d'une ou deux liaisons de valence, B et $R^5$ peuvent être reliés par une ou deux liaisons de valence,
les radicaux $R^6$ sont choisis d'une manière indépendante les uns des autres parmi groupes halogéno, -CN, -$CF_3$, -$OCF_3$, aryle, -COOH et -$NH_2$,
$R^7$ $R^8$, $R^9$ et $R^{10}$ sont choisis d'une manière indépendante parmi

- l'atome d'hydrogène, les groupes halogéno, les radicaux -CN, -$CH_2$CN, -$CHF_2$, -$CF_3$, -$OCF_3$, -$OCHF_2$, -$OCH_2CF_3$, -$OCF_2CHF_2$, -S(O)$_2CF_3$, -OS(O)$_2CF_3$, -$SCF_3$, -$NO_2$, -$OR^{11}$, $NR^{11}R^{12}$, -$SR^{11}$, -$NR^{11}$S(O)$_2R^{12}$, -S(O)$_2NR^{11}R^{12}$, -S(O)$NR^{11}R^{12}$, -S(O)$R^{11}$, -S(O)$_2R^{11}$, -OS(O)$_2R^{11}$, -C(O)$NR^{11}R^{12}$, -OC(O)$NR^{11}R^{12}$, -$NR^{11}$C(O)$R^{12}$, -$CH_2$C (O) $NR^{11}R^{12}$, -O(alkyle en $C_1$-$C_6$)-C(O)$NR^{11}R^{12}$, -$CH_2OR^{11}$, -$CH_2$OC(O)$R^{11}$,

$-CH_2NR^{11}R^{12}$, -OC (O) $R^{11}$, -O(alkyle en $C_1$-$C_{15}$)-C(O)$OR^{11}$, -O(alkyle en $C_1$-$C_6$) -$OR^{11}$, -S(alkyle en $C_1$-$C_6$)-C(O)$OR^{11}$, -(alcényle en $C_2$-$C_6$)-C (=O) $OR^{11}$, -$NR^{11}$-C(=O)-(alkyle en $C_1$-$C_6$)-C (=O)$OR^{11}$, -$NR^{11}$-C (=O)-(alcényle en $C_1$-$C_6$)-C(=O)$OR^{11}$, -C(O)$OR^{11}$, C(O)$R^{11}$ ou - (alcényle en $C_2$-$C_6$)-C (=O)$R^{11}$, = O, ou -(alcényle en $C_2$-$C_6$) -C (=O) -$NR^{11}R^{12}$,

- les radicaux alkyle en $C_1$-$C_6$, alcényle en $C_2$-$C_6$ ou alcynyle en $C_2$-$C_6$, dont chacun peut en option être substitué par un ou plusieurs substituants choisis d'une manière indépendante les uns des autres parmi $R^{13}$,
- les radicaux aryle, aryloxy, aryloxycarbonyle, aroyle, arylsulfanyle, aryl(alcoxy en $C_1$-$C_6$), aryl-(alkyle en $C_1$-$C_6$), aryl(alcényle en $C_2$-$C_6$), aroyl-(alcényle en $C_2$-$C_6$), aryl (alcynyle en $C_2$-$C_6$), hétéroaryle, hétéroaryl (alkyle en $C_1$-$C_6$), hétéroaryl-(alcényle en $C_2$-$C_6$), hétéroaryl(alcynyle en $C_2$-$C_6$) ou cycloalkyle en $C_3$-$C_6$,

dont chaque fragment cyclique peut en option être substitué par un ou plusieurs substituants choisis d'une manière indépendante les uns des autres parmi $R^{14}$,

$R^{11}$ et $R^{12}$ sont choisis d'une manière indépendante l'un de l'autre parmi l'atome d'hydrogène, OH, les radicaux alkyle en $C_1$-$C_{20}$, aryl(alkyle en $C_1$-$C_6$) ou aryle, où les groupes alkyle peuvent en option être substitués par un ou plusieurs substituants choisis d'une manière indépendante parmi $R^{15}$, et les groupes aryle peuvent en option être substitués par un ou plusieurs substituants choisis d'une manière indépendante parmi $R^{16}$ ; $R^{11}$ et $R^{12}$, quand ils sont fixés au même atome d'azote, peuvent former un noyau hétérocyclique à 3 à 8 chaînons avec ledit atome d'azote, le noyau hétérocyclique contenant en option un ou deux hétéroatomes supplémentaires choisis parmi l'azote, l'oxygène et le soufre, et contenant en option une ou deux doubles liaisons,

$R^{13}$ est choisi d'une manière indépendante parmi les groupes halogéno, -CN, $-CF_3$, $-OCF_3$, $-OR^{11}$, -C(O)$OR^{11}$, $-NR^{11}R^{12}$ et -C(O) $NR^{11}R^{12}$,

$R^{14}$ est choisi d'une manière indépendante parmi les groupes halogéno, -C(O)$OR^{11}$, $-CH_2C(O)OR^{11}$, $-CH_2OR^{11}$, -CN, $-CF_3$, $-OCF_3$, $-NO_2$, $-OR^{11}$, $-NR^{11}R^{12}$, $-NR^{11}C(O)R^{11}$, $-S(O)_2R^{11}$, aryle et alkyle en $C_1$-$C_6$,

$R^{15}$ est choisi d'une manière indépendante parmi les atomes d'hydrogène, les radicaux -CN, $-CF_3$, =0, $-OCF_3$, -O(alkyle en $C_1$-$C_6$), -C(O)O(alkyle en $C_1$-$C_6$), -COOH et $-NH_2$,

$R^{16}$ est choisi d'une manière indépendante parmi les groupes halogéno, les radicaux -C(O)O(alkyle en $C_1$-$C_6$), -COOH, -CN, $-CF_3$, $-OCF_3$, $-NO_2$, -OH, -O(alkyle en $C_1$-$C_6$), $-NH_2$, C(=O) ou alkyle en $C_1$-$C_6$, ou tout énantiomère, diastéréoisomère, y compris un mélange racémique, un tautomère, ainsi qu'un sel de ceux-ci avec un acide ou une base acceptable d'un point de vue pharmaceutique.

**3.** Composition pharmaceutique selon la revendication 2, dans laquelle X est =0 ou =S.

**4.** Composition pharmaceutique selon la revendication 3, dans laquelle X est =0.

**5.** Composition pharmaceutique selon la revendication 3, dans laquelle X est =S.

**6.** Composition pharmaceutique selon l'une quelconque des revendications 2 à 5, dans laquelle Y est -O- ou -S-.

**7.** Composition pharmaceutique selon la revendication 6, dans laquelle Y est -O-.

**8.** Composition pharmaceutique selon la revendication 6, dans laquelle Y est -NH-.

**9.** Composition pharmaceutique selon la revendication 6, dans laquelle Y est -S-.

**10.** Composition pharmaceutique selon l'une quelconque des revendications 2 à 9, dans laquelle A est un radical aryle en option substitué par jusqu'à quatre substituants, $R^7$, $R^8$, $R^9$ et $R^{10}$, qui peuvent être identiques ou différents.

**11.** Composition pharmaceutique selon la revendication 10, dans laquelle A est choisi parmi ArG1, en option substitué par jusqu'à quatre substituants $R^7$, $R^8$, $R^9$ et $R^{10}$, qui peuvent être identiques ou différents.

**12.** Composition pharmaceutique selon la revendication 11, dans laquelle A est le groupe phényle ou naphtyle, en option substitué par jusqu'à quatre substituants $R^7$, $R^8$, $R^9$ et $R^{10}$, qui peuvent être identiques ou différents.

**13.** Composition pharmaceutique selon la revendication 12, dans laquelle A est

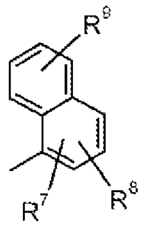

or

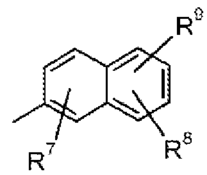

(or = ou)

**14.** Composition pharmaceutique selon la revendication 12, dans laquelle A est le groupe phényle.

**15.** Composition pharmaceutique selon l'une quelconque des revendications 2 à 9, dans laquelle A est un radical hétéroaryle en option substitué par jusqu'à quatre substituants $R^7$, $R^8$, $R^9$ et $R^{10}$, qui peuvent être identiques ou différents.

**16.** Composition pharmaceutique selon la revendication 15, dans laquelle A est choisi parmi Het1, en option substitué par jusqu'à quatre substituants $R^7$, $R^8$, $R^9$ et $R^{10}$, qui peuvent être identiques ou différents.

**17.** Composition pharmaceutique selon la revendication 16, dans laquelle A est choisi parmi Het2, en option substitué par jusqu'à quatre substituants $R^7$, $R^8$, $R^9$ et $R^{10}$, qui peuvent être identiques ou différents.

**18.** Composition pharmaceutique selon la revendication 17, dans laquelle A est choisi parmi Het3, en option substitué par jusqu'à quatre substituants $R^7$, $R^8$, $R^9$ et $R^{10}$, qui peuvent être identiques ou différents.

**19.** Composition pharmaceutique selon la revendication 18, dans laquelle A est choisi dans l'ensemble consistant en les groupes indolyle, benzofurannyle, quinoléyle, furyle, thiényle ou pyrrolyle, où chaque radical hétéroaryle peut en option être substitué par jusqu'à quatre substituants $R^7$, $R^8$, $R^9$ et $R^{10}$, qui peuvent être identiques ou différents.

**20.** Composition pharmaceutique selon la revendication 18, dans laquelle A est le groupe benzofurannyle, en option substitué par jusqu'à quatre substituants $R^7$, $R^8$, $R^9$ et $R^{10}$, qui peuvent être identiques ou différents.

**21.** Composition pharmaceutique selon la revendication 20, dans laquelle A est

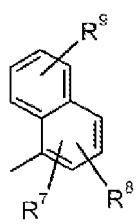

or

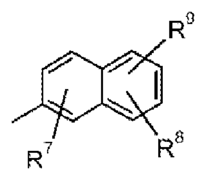

(or = ou)

**22.** Composition pharmaceutique selon la revendication 18, dans laquelle A est le groupe carbazolyle, en option substitué par jusqu'à quatre substituants $R^7$, $R^8$, $R^9$ et $R^{10}$, qui peuvent être identiques ou différents.

**23.** Composition pharmaceutique selon la revendication 22, dans laquelle A est

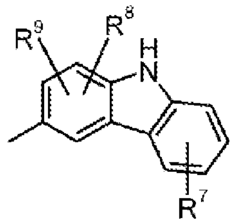

**24.** Composition pharmaceutique selon la revendication 18, dans laquelle A est le groupe quinoléyle, en option substitué par jusqu'à quatre substituants $R^7$, $R^8$, $R^9$ et $R^{10}$, qui peuvent être identiques ou différents.

**25.** Composition pharmaceutique selon la revendication 24, dans laquelle A est

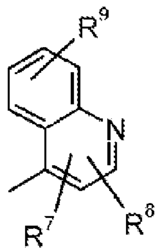

or

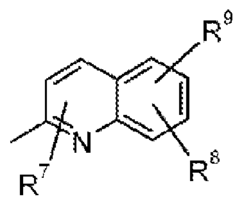

(or = ou)

**26.** Composition pharmaceutique selon la revendication 18, dans laquelle A est le groupe indolyle, en option substitué par jusqu'à quatre substituants $R^7$, $R^8$, $R^9$ et $R^{10}$, qui peuvent être identiques ou différents.

**27.** Composition pharmaceutique selon la revendication 26, dans laquelle A est

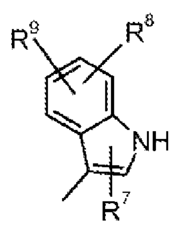

or

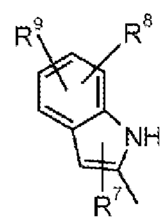

(or = ou)

**28.** Composition pharmaceutique selon l'une quelconque des revendications 2 à 27, dans laquelle $R^1$ est un atome d'hydrogène.

**29.** Composition pharmaceutique selon l'une quelconque des revendications 2 à 28, dans laquelle $R^2$ est un atome d'hydrogène.

**30.** Composition pharmaceutique selon l'une quelconque des revendications 2 à 27, dans laquelle $R^1$ et $R^2$ sont combinés pour former une double liaison.

**31.** Composition pharmaceutique selon l'une quelconque des revendications 2 à 30, dans laquelle $R^3$ est un radical alkyle en $C_1$-$C_6$, un atome d'halogène ou $C(O)NR^{16}R^{17}$.

**32.** Composition pharmaceutique selon la revendication 31, dans laquelle $R^3$ est un radical alkyle en $C_1$-$C_6$ ou $C(O)NR^{16}R^{17}$.

**33.** Composition pharmaceutique selon la revendication 32, dans laquelle $R^3$ est le groupe méthyle.

**34.** Composition pharmaceutique selon l'une quelconque des revendications 2 à 9, dans laquelle B est le groupe phényle, en option substitué par jusqu'à quatre substituants $R^7$, $R^8$, $R^9$ et $R^{10}$, qui peuvent être identiques ou différents.

**35.** Composition pharmaceutique selon l'une quelconque des revendications 2 à 9 ou 34, dans laquelle $R^4$ est un atome d'hydrogène.

**36.** Composition pharmaceutique selon l'une quelconque des revendications 2 à 9 ou 34 à 35, dans laquelle $R^5$ est un atome d'hydrogène.

**37.** Composition pharmaceutique selon l'une quelconque des revendications 2 à 36, dans laquelle $R^6$ est un radical aryle.

**38.** Composition pharmaceutique selon la revendication 37, dans laquelle $R^6$ est le groupe phényle.

**39.** Composition pharmaceutique selon l'une quelconque des revendications 2 à 38, dans laquelle $R^7$, $R^8$, $R^9$ et $R^{10}$ sont choisis d'une manière indépendante les uns des autres parmi

- l'atome d'hydrogène, les groupes halogéno, $-NO_2$, $-OR^{11}$, $-NR^{11}R^{12}$, $-SR^{11}$, $-NR^{11}S(O)_2R^{12}$, $-S(O)_2NR^{11}R^{12}$, $-S(O)NR^{11}R^{12}$, $-S(O)R^{11}$, $-S(O)_2R^{11}$, $-OS (O)_2R^{11}$, $-NR^{11}C(O)R^{12}$, $-CH_2OR^{11}$, $-CH_2OC (O) R^{11}$, $-CH_2NR^{11}R^{12}$, $-OC(O)R^{11}$, -O(alkyle en $C_1$-$C_6$)-$C(O)OR^{11}$, -O(alkyle en $C_1$-$C_6$)-$C(O)NR^{11}R^{12}$, -O(alkyle en $C_1$-$C_6$)-$OR^{11}$, -S (alkyle en $C_1$-$C_6$)-$C(O)OR^{11}$, -(alcényle en $C_2$-$C_6$)-$C(=O)OR^{11}$, $-C(O)OR^{11}$ ou - (alcényle en $C_2$-$C_6$)-$C(=O)R^{11}$,
- les radicaux alkyle en $C_1$-$C_6$, alcényle en $C_2$-$C_6$ ou alcynyle en $C_2$-$C_6$, qui peuvent chacun en option être substitués par un ou plusieurs substituants choisis d'une manière indépendante parmi $R^{13}$,
- les radicaux aryle, aryloxy, aroyle, arylsulfanyle, aryl(alcoxy en $C_1$-$C_6$), aryl (alkyle en $C_1$-$C_6$), aryl-(alcényle en $C_2$-$C_6$), aroyl (alcényle en $C_2$-$C_6$), aryl-(alcynyle en $C_2$-$C_6$), hétéroaryle, hétéroaryl(alkyle en $C_1$-$C_6$), où chacun des fragments cycliques peut en option être substitué par un ou plusieurs substituants choisis d'une manière indépendante parmi $R^{14}$.

**40.** Composition pharmaceutique selon la revendication 39, dans laquelle $R^7$, $R^8$, $R^9$ et $R^{10}$ sont choisis d'une manière indépendante parmi

- l'atome d'hydrogène, les groupes halogéno, les radicaux $-NO_2$, $-OR^{11}$, $-NR^{11}R^{12}$, $-SR^{11}$, $-S(O)_2R^{11}$, $-OS(O)_2R^{11}$, $-CH_2OC(O)R^{11}$, $-OC(O)R^{11}$, -0(alkyle en $C_1$-$C_6$)-$C(O)OR^{11}$, -O(alkyle en $C_1$-$C_6$)-$OR^{11}$, -S(alkyle en $C_1$-$C_6$)-C (O)$OR^{11}$, $-C(O)OR^{11}$ ou - (alcényle en $C_2$-$C_6$) - $C(=O)R^{11}$,
- les radicaux alkyle en $C_1$-$C_6$ ou alcényle en $C_1$-$C_6$, qui peuvent chacun en option être substitués par un ou plusieurs substituants choisis d'une manière indépendante parmi $R^{13}$,
- les radicaux aryle, aryloxy, aroyle, aryl(alcoxy en $C_1$-$C_6$), aryl (alkyle en $C_1$-$C_6$), hétéroaryle,

dont chacun des fragments cycliques peut en option être substitué par un ou plusieurs substituants choisis d'une manière indépendante parmi $R^{14}$.

**41.** Composition pharmaceutique selon la revendication 40, dans laquelle $R^7$, $R^8$, $R^9$ et $R^{10}$ sont choisis d'une manière indépendante parmi

- l'atome d'hydrogène, les groupes halogéno, les radicaux $-NO_2$, $-OR^{11}$, $-NR^{11}R^{12}$, $-SR^{11}$, $-S(O)_2R^{11}$, -OS $(O)_2R^{11}$, $-CH_2OC(O)R^{11}$, $-OC(O)R^{11}$, -O(alkyle en $C_1$-$C_6$)-$C(O)OR^{11}$, -O(alkyle en $C_1$-$C_6$)-$OR^{11}$, -S(alkyle en $C_1$-$C_6$)-$C(O)OR^{11}$, $-C(O)OR^{11}$ ou -(alcényle en $C_2$-$C_6$)-$C(=O)R^{11}$,
- les radicaux alkyle en $C_1$-$C_6$ ou ... en $C_1$-$C_6$, qui peuvent chacun en option être substitués par un ou plusieurs substituants choisis d'une manière indépendante parmi les radicaux $R^{13}$,
- les radicaux aryle, aryloxy, aroyle, aryl(alcoxy en $C_1$-$C_6$), aryl(alkyle en $C_1$-$C_6$), hétéroaryle,

dont chacun des fragments cycliques peut en option être substitué par un ou plusieurs substituants choisis d'une manière indépendante parmi les radicaux $R^{14}$.

**42.** Composition pharmaceutique selon la revendication 41, dans laquelle $R^7$, $R^8$, $R^9$ et $R^{10}$ sont choisis d'une manière indépendante parmi

- l'atome d'hydrogène, les groupes halogéno, les radicaux $-OR^{11}$, -O (alkyle en $C_1$-$C_6$) -C (O) $OR^{11}$ ou -C(O) $OR^{11}$,
- les radicaux alkyle en $C_1$-$C_6$, qui peuvent chacun en option être substitués par un ou plusieurs substituants choisis d'une manière indépendante parmi les radicaux $R^{13}$,
- les radicaux aryle, aryloxy, aryl(alcoxy en $C_1$-$C_6$),

dont chacun des fragments cycliques peut en option être substitué par un ou plusieurs substituants choisis d'une manière indépendante parmi les radicaux $R^{14}$.

**43.** Composition pharmaceutique selon la revendication 42, dans laquelle $R^7$, $R^8$, $R^9$ et $R^{10}$ sont choisis d'une manière indépendante parmi

- l'atome d'hydrogène, les groupes halogéno, les radicaux $-OR^{11}$, -O(alkyle en $C_1$-$C_6$) -C(O)$OR^{11}$, ou -C(O)$OR^{11}$,
- les radicaux alkyle en $C_1$-$C_6$, qui peuvent chacun en option être substitués par un ou plusieurs substituants choisis d'une manière indépendante parmi les radicaux $R^{13}$,
- les radicaux ArG1, ArGloxy, ArG1-(alcoxy en $C_1$-$C_6$),

dont chacun des fragments cycliques peut en option être substitué par un ou plusieurs substituants choisis d'une manière indépendante parmi les radicaux $R^{14}$.

**44.** Composition pharmaceutique selon la revendication 43, dans laquelle $R^7$, $R^8$, $R^9$ et $R^{10}$ sont choisis d'une manière indépendante parmi

- l'atome d'hydrogène, les groupes halogéno, les radicaux $-OR^{11}$, -O(alkyle en $C_1$-$C_6$)-C (O)$OR^{11}$ ou -C(O)$OR^{11}$,
- les radicaux alkyle en $C_1$-$C_6$, qui peuvent en option être substitués par un ou plusieurs substituants choisis d'une manière indépendante parmi $R^{13}$,
- les radicaux phényle, phényloxy, phényl(alcoxy en $C_1$-$C_6$), où chacun des fragments cycliques peut en option être substitué par un ou plusieurs substituants choisis d'une manière indépendante parmi $R^{14}$.

**45.** Composition pharmaceutique selon l'une quelconque des revendications 2 à 44, dans laquelle $R^{11}$ et $R^{12}$ sont choisis d'une manière indépendante parmi l'atome d'hydrogène, les radicaux alkyle en $C_1$-$C_{20}$, aryle ou aryl(alkyle en $C_1$-$C_6$), où les groupes alkyle peuvent en option être substitués par un ou plusieurs substituants choisis d'une manière indépendante parmi $R^{15}$, et les groupes aryle peuvent en option être substitués par un ou plusieurs substituants choisis d'une manière indépendante parmi $R^{16}$ ; $R^{11}$ et $R^{12}$, quand ils sont fixés au même atome d'azote, peuvent former un noyau hétérocyclique à 3 à 8 chaînons avec ledit atome d'azote, le noyau hétérocyclique contenant en option un ou deux hétéroatomes supplémentaires choisis parmi l'azote, l'oxygène et le soufre, et contenant en option une ou deux doubles liaisons.

**46.** Composition pharmaceutique selon la revendication 45, dans laquelle $R^{11}$ et $R^{12}$ sont choisis d'une manière indépendante parmi l'atome d'hydrogène, les radicaux alkyle en $C_1$-$C_{20}$, aryle ou aryl(alkyle en $C_1$-$C_6$), où les groupes alkyle peuvent en option être substitués par un ou plusieurs substituants choisis d'une manière indépendante parmi $R^{15}$, et les groupes aryle peuvent en option être substitués par un ou plusieurs substituants choisis d'une manière indépendante parmi $R^{16}$.

**47.** Composition pharmaceutique selon la revendication 46, dans laquelle $R^{11}$ et $R^{12}$ sont choisis d'une manière indépendante parmi le groupe phényle ou les radicaux phényl(alkyle en $C_1$-$C_6$).

**48.** Composition pharmaceutique selon la revendication 46, dans laquelle l'un des radicaux $R^{11}$ et $R^{12}$, ou les deux, sont des groupes méthyle.

**49.** Composition pharmaceutique selon l'une quelconque des revendications 2 à 48, dans laquelle $R^{13}$ est choisi d'une manière indépendante parmi les groupes halogéno, $CF_3$, $OR^{11}$ ou $NR^{11}R^{12}$.

**50.** Composition pharmaceutique selon la revendication 49, dans laquelle $R^{13}$ est choisi d'une manière indépendante parmi les groupes halogéno ou $OR^{11}$.

**51.** Composition pharmaceutique selon la revendication 50, dans laquelle $R^{13}$ est $OR^{11}$.

**52.** Composition pharmaceutique selon l'une quelconque des revendications 2 à 51, dans laquelle $R^{14}$ est choisi d'une manière indépendante parmi les groupes halogéno, $-C(O)OR^{11}$, -CN, $-CF_3$, $-OR^{11}$, $-S(O)_2R^{11}$ et les radicaux alkyle en $C_1$-$C_6$.

**53.** Composition pharmaceutique selon la revendication 52, dans laquelle $R^{14}$ est choisi d'une manière indépendante parmi les groupes halogéno, -C (O) $OR^{11}$, ou $-OR^{11}$.

**54.** Composition pharmaceutique selon l'une quelconque des revendications 2 à 53, dans laquelle $R^{15}$ est choisi d'une manière indépendante parmi les groupes halogéno, -CN, $-CF_3$, les radicaux -C(O)O(alkyle en $C_1$-$C_6$) et -COOH.

**55.** Composition pharmaceutique selon la revendication 54, dans laquelle $R^{15}$ est choisi d'une manière indépendante parmi les groupes halogéno ou les groupes -C(O)O(alkyle en $C_1$-$C_6$) .

**56.** Composition pharmaceutique selon l'une quelconque des revendications 2 à 55, dans laquelle $R^{16}$ est choisi d'une manière indépendante parmi les groupes halogéno, les radicaux -C(O)O(alkyle en $C_1$-$C_6$), -COOH, $-NO_2$, -O(alkyle en $C_1$-$C_6$), $-NH_2$, C(=O) ou alkyle en $C_1$-$C_6$.

**57.** Composition pharmaceutique selon la revendication 56, dans laquelle $R^{16}$ est choisi d'une manière indépendante parmi les groupes halogéno, les radicaux -C(O)O(alkyle en $C_1$-$C_6$), -COOH, $-NO_2$ ou alkyle en $C_1$-$C_6$.

**58.** Composition pharmaceutique selon l'une quelconque des revendications 1 à 57, dans laquelle l'insuline est une insuline d'action rapide.

**59.** Composition pharmaceutique selon l'une quelconque des revendications 1 à 58, dans laquelle l'insuline est choisie dans le groupe consistant en l'insuline humaine, un analogue de cette dernière, un dérivé de cette dernière ou des combinaisons de membres quelconques de ce groupe.

**60.** Composition pharmaceutique selon la revendication 59, dans laquelle l'insuline est un analogue de l'insuline humaine, choisi dans le groupe consistant en

i. un analogue dans lequel la position B28 est Asp, Lys, Leu, Val ou Ala, et la position B29 est Lys ou Pro ; et
ii. l'insuline humaine des (B28-B30), des (B27) ou des (B30).

**61.** Composition pharmaceutique selon la revendication 60, dans laquelle l'insuline est un analogue de l'insuline humaine dans laquelle la position B28 est Asp ou Lys, et la position B29 est Lys ou Pro.

**62.** Composition pharmaceutique selon la revendication 60, dans laquelle l'insuline est une insuline humaine des (B30).

**63.** Composition pharmaceutique selon la revendication 60, dans laquelle l'insuline est un analogue de l'insuline humaine dans lequel la position B3 est Lys et la position B29 est Glu ou Asp.

**64.** Composition pharmaceutique selon la revendication 59, dans laquelle l'insuline est un dérivé de l'insuline humaine ayant un ou plusieurs substituants lipophiles.

**65.** Composition pharmaceutique selon la revendication 64, dans laquelle le dérivé de l'insuline est choisi dans le groupe consistant en l'insuline humaine B29-$N^{\varepsilon}$-myristoyl-des(B30), l'insuline humaine B29-$N^{\varepsilon}$-palmitoyl-des(B30), l'insuline humaine B29-$N^{\varepsilon}$-myristoyle, l'insuline humaine B29-$N^{\varepsilon}$-palmitoyle, l'insuline humaine B28-$N^{\varepsilon}$-myristoyl-$Lys^{B28}$ $Pro^{B29}$, l'insuline humaine B28-$N^{\varepsilon}$-palmitoyl-$Lys^{B28}$ $Pro^{B29}$, l'insuline humaine B30-$N^{\varepsilon}$-myristoyl-$Thr^{B29}$ $Lys^{B30}$, l'insuline humaine B30-$N^{\varepsilon}$-palmitoyl-$Thr^{B29}$ $Lys^{B30}$, l'insuline humaine B29-$N^{\varepsilon}$-(N-palmitoyl-γ-glutamyl) des (B30), l'insuline humaine B29-$N^{\varepsilon}$-(N-lithocholyl-γ-glutamyl)des (B30), l'insuline humaine B29-$N^{\varepsilon}$-(ω-carboxyheptadécanoyl)-des(B30), et l'insuline humaine B29-$N^{\varepsilon}$-(ω-carboxyheptadécanoyle).

**66.** Composition pharmaceutique selon la revendication 65, dans laquelle le dérivé de l'insuline est l'insuline humaine B29-$N^{\varepsilon}$-myristoyl-des(B30).

**67.** Composition pharmaceutique selon l'une quelconque des revendications 1 à 66, comprenant 2-6 mol d'ions $zinc^{2+}$ par mole d'insuline.

**68.** Composition pharmaceutique selon la revendication 67, comprenant 2-3 mol d'ions $zinc^{2+}$ par mole d'insuline.

**69.** Composition pharmaceutique selon l'une quelconque des revendications 1 à 68, comprenant en outre au moins 3 molécules d'un composé phénolique par hexamère de l'insuline.

**70.** Composition pharmaceutique selon l'une quelconque des revendications 1 à 69, comprenant en outre un agent d'isotonicité.

**71.** Composition pharmaceutique selon l'une quelconque des revendications 1 à 70, comprenant en outre une substance tampon.

**72.** Procédé pour stabiliser une composition d'insuline, comprenant l'addition, à la composition d'insuline, d'un ligand se liant au zinc, selon l'une quelconque des revendications 1 à 57.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5830999 A
- US 5957895 A
- US 5858001 A
- US 4468221 A
- US 6074369 A
- US 5527288 A

### Non-patent literature cited in the description


- *J. Pharm. Sci.,* 1977, vol. 66, 2
- **LOHRAY et al.** *J. Med. Chem.,* 1999, vol. 42, 2569-81
- *J. Org. Chem.,* 1972, vol. 37 (24), 3972-3973
- **MURPHY et al.** *J. Med. Chem.,* 1990, vol. 33, 171-8
- **BUMAGIN et al.** *Tetrahedron,* 1997, vol. 53, 14437-14450
- **SMITH et al.** *Tetrahedron,* 1992, vol. 48, 7479-7488
- **LITTKE ; DAI ; FU.** *J. Am. Chem. Soc,* 2000, vol. 122, 4020-8
- **KAUER ; SHEPPARD.** *J. Org. Chem.,* 1967, vol. 32, 3580-92
- **BECK ; GÛNTHER.** *Chem. Ber.,* 1973, vol. 106, 2758-66
- **NAIKI et al.** *Anal. Biochem.,* 1989, vol. 177, 244-249
- **LEVINE.** *Methods. Enzymol.,* 1999, vol. 309, 274-284
- **NIELSEN et al.** *Biochemistry,* 2001, vol. 40, 6036-6046
- The pig as a model for subcutaneous absorption in man. **RIBEL et al.** Diabetes (1985) proceedings ot the 12th congress of the international diabetes federation. 1985, 891-896
- **HUANG et al.** *Biochemistry,* 1997, vol. 36, 9878-9888